# EUROPEAN PATENT APPLICATION

(11) **EP 2 765 198 A2**
(43) Date of publication of application: **13.08.2014**
(21) Application number: 13198548.3
(22) Date of filing: 10.09.2008
(51) Int. Cl.: C12N 15/74, C12N 15/00, C12N 1/21, C07K 14/19, C07K 14/02, C12P 21/06

(54) **Isoprenoid production by genetically modified chloroplasts**

(30) Priority: 11.09.2007 US 971418 P; 11.09.2007 US 971412 P; 02.06.2008 US 130892
(62) Divisional of application: 08799404.2
(71) Applicant: Sapphire Energy, Inc., San Diego, CA 92121 (US); THE SCRIPPS RESEARCH INSTITUTE, La Jolla, CA 92037 (US)
(72) Inventor: Mendez, Michael, San Diego, CA California 92130 (US); Mayfield, Stephen, Cardiff-by-the-Sea, CA California 92007 (US); O'Neill, Bryan, Carlsbad, CA 92011 (US); Poon, Yan, Pasadena, CA California 91106 (US); Lee, Philip, Las Cruces, New Mexico 99007 (US); Behnke, Craig, Aaron, San Diego, CA 92104 (US); Fang, Su-Chiung, Tainan County, Taiwan ROC 74146 Taiwan (TW)
(74) Representative: Kiddle, Simon John

(57) **Abstract**

The present invention provides compositions and methods for producing products, such as isoprenoids, which can be used for multiple purposes (e.g., fuel, fuel feedstocks, fragrances and insecticides), using photosynthetic organisms. One aspect of the present invention provides a vector comprising a nucleic acid encoding an enzyme that produces an isoprenoid with two phosphates and a promoter configured for expression of the nucleic acid in a chloroplast of a non-vascular, photosynthetic organism and does not comprise the entire genome of a chloroplast.

## Description

### CROSS-REFERENCE TO RELATED APPLICATIONS

This application claims priority to and benefit of U.S. Provisional Application Nos. 60/971,418 (filed September 11, 2007), 60/971,412 (filed September 11, 2007), and 61/130,892 (filed June 2, 2008), which applications are incorporated herein by reference.

### INCORPORATION BY REFERENCE

All publications and patent applications mentioned in this specification are herein incorporated by reference to the same extent as if each individual publication or patent application was specifically and individually indicated to be incorporated by reference.

### BACKGROUND OF THE INVENTION

Fuel products, such as oil, petrochemicals, and other substances useful for the production of petrochemicals are increasingly in demand. Much of today's fuel products are generated from fossil fuels, which are not considered renewable energy sources, as they are the result of organic material being covered by successive layers of sediment over the course of millions of years. There is also a growing desire to lessen dependence on imported crude oil. Public awareness regarding pollution and environmental hazards has also increased. As a result, there has been a growing interest and need for alternative methods to produce fuel products. Thus, there exists a pressing need for alternative methods to develop fuel products that are renewable, sustainable, and less harmful to the environment.

### SUMMARY OF THE INVENTION

The present invention relates to compositions and methods for creating products, such as isoprenoids, which can be used for multiple purposes (e.g., fuel, fuel feedstocks, fragrances and insecticides), using photosynthetic organisms. The compositions include expression vector comprising one or more nucleotide sequences that initiate, increase, or effect the production of a product in a non-vascular, photosynthetic organism.

In some instances, such nucleotide sequence(s) encode one or more polypeptides in the mevalonate pathway (MVA pathway). Examples of polypeptides in the MVA pathway include, but are not limited to, thiolase, HMG-CoA synthase, HMG-CoA reductase, mevalonate kinase, phosphemevalonate kinase, or mevalonate-5-pyrophosphate decarboxylase. In other embodiments, the nucleotide sequence encodes a polypeptide in the non-mevalonate pathway (MEP pathway). The polypeptide may be DOXP synthase, DOXP reductase, 4-diphosphocytidyl-2-C-methyl-D-erythritol synthase, 4-diphophocytidyl-2-C-methyl-D-erythritol kinase, 2-C-methyl-D-erythritol 2,4,-cyclodiphosphate synthase, HMB-PP synthase, HMB-PP reductase, and DOXP reductoisomerase.

One aspect of the present invention provides a vector comprising a nucleic acid encoding an enzyme that produces an isoprenoid with two phosphates and a promoter configured for expression of the nucleic acid in a chloroplast of a non-vascular, photosynthetic organism (NVPO) and does not comprise the entire genome of a chloroplast. In practice, insertion of the vector into a chloroplast genome may not disrupt photosynthetic capability of the chloroplast(s). In other instances, the vectors of the present invention further comprise a nucleic acid sequence which facilitates homologous recombination with a chloroplast genome. In some instances, an isoprenoid produced by an enzyme encoded on a vecor as disclosed herein is GPP, IPP, FPP, GGPP or DMAPP. In some vectors disclosed herein, a second nucleic acid encoding a second enzyme which modifies an isoprenoid with two phosphates is also present on the vector. Specific examples of a second enzyme which may be encoded by the vectors of the present invention include, but are not limited to, botyrococcene synthase, limonene synthase, cineole synthase, pinene synthase, camphene synthase, sabinene synthase, myrcene synthase, abietadiene synthase, taxadiene synthase, FPP synthase, bisabolene synthase, diapophytoene desaturase, diapophytoene synthase, GPP synthase, IPP isomerase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner-13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase. The vectors may also contain a selectable marker. Specific vectors of the present invention are capable of stable transformation in microalga. In some embodiments, the algal species is *C*. *reinhardtii, D. salina, H. pluvalis, S. dimorphus, D. viridis,* or *D. tertiolecta.* A nucleic nucleic acid encoding an enzyme may be biased for a nonvascular photosynthetic microorganism nuclear and/or chloroplast expression. Sequences encoding the enzymes useful in the present invention may be any of the sequences specifically disclosed herein (e.g., the sequences in Tables 5-8), or sequences with 60, 65, 70, 75, 80, 85, 90, 95 or higher identity thereto. Some vectors of the present invention comprise any of the sequences specifically disclosed herein (e.g., the sequences in Tables 5-8), or sequences with 60, 65, 70, 75, 80, 85, 90, 95 or higher identity thereto.

Another vector disclosed herein comprises a nucleic acid encoding an enzyme that produces an isoprenoid with two phosphates; and a nucleic acid encoding a chloroplast targeting molecule for targeting the enzyme to a chloroplast. Such vectors may further comprise a selectable marker, a nucleic acid sequence which facilitates homologous recombination with a chloroplast or nuclear genome genome or a combination of these features. In some instances, the enzyme encoded by the nucleic acid produces GPP, IPP, FPP, GGPP or DMAPP. Such vectors disclosed herein may further comprise a nucleic acid encoding a second enzyme which modifies an isoprenoid with two phosphates and a nucleic acid encoding a chloroplast targeting molecule for targeting the second enzyme to a chloroplast. Specific examples of a second enzyme which may be encoded by the vectors of the present invention include, but are not limited to, botyrococcene synthase, limonene synthase, cineole synthase, pinene synthase, camphene synthase, sabinene synthase, myrcene synthase, abietadiene synthase, taxadiene synthase, FPP synthase, bisabolene synthase, diapophytoene desaturase, diapophytoene synthase, GPP synthase, IPP isomerase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner-13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase. Nucleic acid(s) encoding an enzyme for use in the present invention may be codon biased for an NVPO. Specific vectors of the present invention are capable of stable transformation in microalga. In some embodiments, the algal species is *C*. *reinhardtii, D. salina, H. pluvalis, S. dimorphus, D. viridis,* or *D. tertiolecta.* Sequences encoding the enzymes useful in the present invention may be any of the sequences specifically disclosed herein (e.g., the sequences in Table 5-8), or sequences with 60, 65, 70, 75, 80, 85, 90, 95 or higher identity thereto. Some vectors of the present invention comprise any of the sequences specifically disclosed herein (e.g., the sequences in Tables 5-8), or sequences with 60, 65, 70, 75, 80, 85, 90, 95 or higher identity thereto.

The present disclosure also provides a host cell comprising; 1) a vector comprising a nucleic acid encoding an enzyme that produces an isoprenoid with two phosphates and a promoter configured for expression of the nucleic acid in a chloroplast of an NVPO and does not comprise the entire genome of a chloroplast; or 2) a vector comprising a nucleic acid encoding an enzyme that produces an isoprenoid with two phosphates and a nucleic acid encoding a chloroplast targeting molecule for targeting an enzyme to a chloroplast. The host cell may be homoplasmic for one or more of the nucleic acids present on a vector. Some examples of the host cells contemplated herein include cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. Some specific examples of host cells include the algal species *C*. *reinhardtii, D. salina, H. pluvalis, S. dimorphus, D. viridis,* or *D. tertiolecta.* In some instances, chlorophyll levels are sufficient for the host cell to be photoautotrophic following transformation. In other instances, the host cell may produce at least one naturally occurring isoprenoid at levels greater than a wild-type strain of the same organism.

In another embodiment of the present invention, a host cell is provided which comprises at least two copies of a nucleotide sequence described herein (e.g., in Tables 5-8), or a nucleotide sequence having at least 70% identity to any of these sequences. The host cell may be a non-vascular photosynthetic organism, particularly *C*. *reinhardtii, D. salina, H. pluvalis, S. dimorphus, D. viridis,* or *D. tertiolecta.* In some instances, the host cell is homoplasmic for the nucleotide sequence. The present disclosure also provides a genetically modified chloroplast containing a vector comprising a nucleic acid encoding an enzyme that produces an isoprenoid with two phosphates and a promoter configured for expression of the nucleic acid in a chloroplast of a non-vascular, photosynthetic organism (NVPO).

Also provided herein, is a method for producing an isoprenoid-containing composition comprising the steps of transforming a chloroplast of a non-vascular, photosynthetic organism with a nucleic acid encoding an enzyme that produces an isoprenoid with two phosphates and collecting at least one isoprenoid produced by the transformed NVPO. Such methods may further comprise growing the organism in an aqueous environment, wherein CO₂ is supplied to the organism. CO₂ provided may be at least partially derived from a burned fossil fuel and/or may be at least partially derived from flue gas. Such methods may include production of GPP, IPP, FPP, GGPP or DMAPP. In some instances, the collection process may comprise one or more of the following: harvesting a transformed NVPO, harvesting an isoprenoid from a cell medium; mechanically disrupting a transformed organism and; chemically disrupting an organism. A microalga may be utilized in some aspects of this invention, and the microalga may be C. *reinhardtii, D. salina, H. pluvalis, S. dimorphus, D. viridis,* or *D. tertiolecta.*

Another method for producing an isoprenoid is also described wherein the method comprises the steps of transforming the chloroplast of a non-vascular, photosynthetic organism to produce said isoprenoid, wherein said organism is not transformed with isoprene synthase or a methyl-butenol synthase; and (b) collecting said isoprenoid. This method may further comprise growing the organism in an aqueous environment, wherein CO₂ is supplied to the organism. The CO₂ may be at least partially derived from a burned fossil fuel and/or flue gas. Such methods may include production of GPP, IPP, FPP, GGPP or DMAPP. A chloroplast of this method may be transformed with a nucleic acid encoding botyrococcene synthase, limonene synthase, cineole synthase, pinene synthase, camphene synthase, sabinene synthase, myrcene synthase, abietadiene synthase, taxadiene synthase, bisabolene synthase, diapophytoene desaturase, diapophytoene synthase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner-13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase. In some instances, the collection process may comprise one or more of the following: harvesting a transformed NVPO, harvesting an isoprenoid from a cell medium; mechanically disrupting a transformed organism and; chemically disrupting an organism. A microalga may be utilized in some aspects of this invention, and the microalga may be *C*. *reinhardtii, D. salina, H. pluvalis, S. dimorphus, D. viridis,* or *D. tertiolecta.*

A further embodiment of the present invention provides an organism with a genetically modified chloroplast wherein the chloroplast comprises a nucleic acid encoding an isoprenoid producing enzyme and wherein the organism can grow in a high saline environment. Such an organism may be an NVPO, specifically *D. salina, D. viridis,* or *D. tertiolecta.* In such embodiments, that high saline environment may comprise about 0.5-4.0 molar sodium chloride. Also provided is a method for preparing an isoprenoid comprising transforming an organism with a nucleic acid to increase or initiate production of the isoprenoid, wherein the organism is grown in a high-saline environment; and collecting the isoprenoid. Such an organism may be an NVPO, specifically *D. salina, D. viridis,* or *D. tertiolecta.* In such embodiments, that high saline environment may comprise about 0.5-4.0 molar sodium chloride. In some instances, the transforming step is a chloroplast transformation. In other instances, the collecting step comprises one or more of the following steps: (a) harvesting said transformed organism; (b) harvesting said isoprenoid from a cell medium; (c) mechanically disrupting said organism; or (d) chemically disrupting said organism.

The disclosure herein also provides a vector comprising a heterologous nucleic acid encoding one or more isoprenoid producing enzymes; and a promoter configured for expression of said nucleic acid in a photosynthetic bacteria. In some instances, the photosynthetic bacteria is a cyanobacterial species and may be a member of the genera *Synechocystis, Synechococcus,* and/or *Athrospira.* A host cell comprising such a vector is provided. A host cell may be a cyanobacterial species and may be a member of the genera *Synechocystis, Synechococcus,* and/or *Athrospira.*

The present disclosure further provides a vector comprising: a first nucleic acid encoding a protein, a second nucleic acid encoding a selectable marker, wherein the first and second nucleic acids comprise one open reading frame, and a promoter configured for expression of said first and second nucleic acids in a non-vascular, photosynthetic organism. In some instances, the protein is an isoprenoid producing enzyme or a biomass degrading enzyme. In other instances, the isoprenoid producing enzyme is botyrococcene synthase, limonene synthase, cineole synthase, pinene synthase, camphene synthase, sabinene synthase, myrcene synthase, abietadiene synthase, taxadiene synthase, FPP synthase, bisabolene synthase, diapophytoene desaturase, diapophytoene synthase, GPP synthase, IPP isomerase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase. In still other instances, the biomass degrading enzyme is exo-β-glucanase, endo-β-glucanase, β-glucosidase, endoxylanase, or ligninase. Some of the vectors described herein comprise a first or second nucleic acid in which at least one codon is optimized for expression in the nucleus of a non-vascular, photosynthetic organism is present.

In some instances, vectors described herein comprise a third nucleic acid encoding a cleavage moiety in-frame with said first and second nucleic acids. The cleavage moiety may be a self-cleaving protease and may specifically be a functional portion of the A2 region from foot and mouth disease virus. In other instances, the cleavage moiety is capable of being cleaved by a protease naturally produced by said organism. Also described herein are vectors comprising regulatory elements including an HSP70 promoter, a functional portion of HSP70 promoter, rbcS2 5' upstream translated region (UTR), a functional portion of rbcS2 5' UTR, or a combination thereof. In some instances, the regulatory element is derived from the organism to be transformed. Still other vectors comprise a fourth nucleic acid encoding a secretion signal in-frame with the first, second and/or third nucleic acids. A secretion signal useful in the present vectors is a C. *reinhardtii* carbonic anhydrase secretion signal. Vectors of the invention may be useful in mulitple NVPOs, including photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. In some instances, the vector is capable of stable transformation in *C. reinhardtii, D. salina, H. pluvalis, S. dimorphus, D. viridis, D. tertiolecta,* a bacterium of the genus *Synechocystis,* a bacterium of the genus *Synechococcus,* or a bacterium of the genus *Athrospira.* The vectors described herein may comprise any nucleotide sequence described herein (e.g., in Tables 5-8), or a nucleotide sequence having at least 70% identity to any of these sequences. Still other vectors comprise a fifth nucleic acid in-frame with said first, second, third and/or fourth nucleic acids, where the fifth nucleic acid encodes a tag. The encoded tag may be an epitope tag or a metal affinity tag.

The present disclosure also provides a host cell comprising a vector, wherein the vector comprises a first nucleic acid encoding a protein, a second nucleic acid encoding a selectable marker, wherein the first and second nucleic acids comprise one open reading frame, a promoter configured for expression of the first and second nucleic acids in a non-vascular, photosynthetic organism and optionally one or more additional nucleic acids encoding a cleavage moiety, a secretion signal, a tag or a combination thereof, wherein the one or more additional nucleic acids are in-frame with said first and second nucleic acids. The host cell may be a photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, or phytoplankton. In some instances, the host cell is *C*. *reinhardtii, D. salina, H. pluvalis, S. dimorphus, D. viridis, D. tertiolecta,* a bacterium of the genus *Synechocystis,* a bacterium of the genus *Synechococcus,* or a bacterium of the genus *Athrospira.* In other instances, the vector is stably integrated into the nuclear genome of said host cell.

Further provided herein is a method of producing a protein in a non-vascular photosynthetic organism, comprising:growing said organism, wherein the organism comprises an exogenous nucleic acid comprising a single open reading frame, wherein the open reading frame comprises a first nucleic acid encoding a protein and a second nucleic acid encoding a selectable marker, and wherein the orgnaism further comprises a promoter configured for expression of said open reading frame in said organism, thereby producing said protein. In some instances, the protein is an isoprenoid producing enzyme or a biomass degrading enzyme. In other instances, the open reading frame comprises at least one codon optimized for expression in the nucleus of a non-vascular, photosynthetic organism. An open reading frame may further comprise a third nucleic acid encoding a cleavage moiety in-frame with the first and second nucleic acids. The cleavage moiety may be a self-cleaving protease, and in a particular embodiment may be a functional portion of the A2 region from foot and mouth disease virus. In other embodiments, a cleavage moiety is capable of being cleaved by a protease naturally produced by said organism. An open reading frame may further comprise a fourth nucleic acid encoding a secretion signal in-frame with all other nucleic acids comprising the open reading frame. In one embodiment, the secretion signal is a C. *reinhardtii* carbonic anhydrase secretion signal. As disclosed herein, the organism useful for such a method may be C. *reinhardtii, D. salina, H. pluvalis, S. dimorphus, D. viridis, D. tertiolecta,* a bacterium of the genus *Synechocystis,* the genus *Synechococcus,* or the genus *Athrospira.* An open reading frame for use in this method may further comprise a fifth nucleic acid encoding a tag in-frame with all other nucleic acids comprising the open reading frame. The tag may be an epitope tag or a metal affinity tag.

Further disclosed herein is a host cell comprising a fusion protein, wherein the fusion protein comprises a first nucleic acid encoding a protein and a second nucleic acid encoding a selectable marker and wherein the host cell is a non-vascular photosynthetic organism. The host cell may be *C*. *reinhardtii, D. salina, H. pluvalis,* a bacterium of the genus *Synechocystis,* the genus *Synechococcus,* or the genus *Athrospira.* In some instances, the vector is stably integrated into a nuclear genome of the host cell. A fusion protein may further comprise a cleavage moiety, a secretion signal, a tag, or a combination thereof. A cleavage moiety may be a self-cleaving protease, such as a functional portion of the A2 region from foot and mouth disease virus. Alternately, a cleavage moiety may capable of being cleaved by a protease naturally produced by said organism. One secretion signal which may be utilized is a *C*. *reinhardtii* carbonic anhydrase secretion signal. In fusion proteins comprising a tag, the tag may be an epitope tag or a metal affinity tag.

Provided herein is a method of producing a transgenic non-vascular photosynthetic organism expressing a protein of interest under selective conditions, where the method comprises the step of: transforming the organism with a nucleic acid comprising a single open reading frame, wherein the open reading frame encodes a fusion protein comprising said protein of interest and a selectable marker; wherein the organism is capable of expressing the selectable marker under environmental conditions which require expression of the selectable marker for continued viability of the organism, thereby resulting in expression of said protein of interest. In some instances, the protein is an isoprenoid producing enzyme or a biomass degrading enzyme. A fusion protein may further comprise a cleavage moiety, a secretion signal, a tag, or a combination thereof. A cleavage moiety may be a self-cleaving protease, such as a functional portion of the A2 region from foot and mouth disease virus. Alternately, a cleavage moiety may capable of being cleaved by a protease naturally produced by said organism. One secretion signal which may be utilized is a *C*. *reinhardtii* carbonic anhydrase secretion signal. In fusion proteins comprising a tag, the tag may be an epitope tag or a metal affinity tag.

Further provided by the disclosure herein is a method of increasing phytol production in a non-vascular photosynthetic organism, comprising the step of transforming the organism with a nucleic acid which results in an increase in production of phytol by the organism above a level produced by the organism not containing said nucleic acid. In some instances, the nucleic acid encodes a GPP synthase, a FPP synthase, a geranylgeranyl reductase, a chlorophyllidohydrolase, or a pyrophosphatase. In some embodiments, a transformation step may comprise a chloroplast transformation. In still other embodiments, the enzyme is endogenous to the organism or is homologous to an endogenous enzyme of the organism or is exogenous to the organism. In some instances, the enzyme is overexpressed. Expression of the enzyme may be regulated by an inducible promoter. The disclosed method may further comprise transformation of the organism with a nucleic acid which results in production of dimethylallyl alcohol, isopentyl alcohol, geraniol, farnesol or geranylgeraniol. In some instances, the organism used in practicing the method is a photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. The organism may be *C*. *reinhardtii, D. salina, H. pluvalis, S. dimorphus, D. viridis,* or *D. tertiolecta.*

Also provided herein, is a host cell comprising an introduced nucleic acid, wherein the nucleic acid results in an increase in production of phytol by the host cell above a level produced by a host cell not containing the nucleic acid, wherein the host cell is a non-vascular photosynthetic organism. In some embodiments, the host cell can grow in a high-saline environment, for example, the host cell may be , *D. viridis,* or *D. tertiolecta.* In some instances, the high-saline environment comprises 0.5-4.0 molar sodium chloride. In some host cells, the nucleic acid is present in a chloroplast. In still other host cells, the nucleic acid encodes an enzyme selected from the group consisting of a GPP synthase, a FPP synthase, a geranylgeranyl reductase, a chlorophyllidohydrolase, and a pyrophosphatase. The host cell may further comprise a nucleic acid which results in production of dimethylallyl alcohol, isopentyl alcohol, geraniol, farnesol or geranylgeraniol. The host cell may be a photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. In some instances, the host cell is *C. reinhardtii, D. salina, H. pluvalis, S. dimorphus, D. viridis,* or *D. tertiolecta.*

Another method disclosed herein provides a method of producing phytol in a non-vascular photosynthetic organism, comprising the steps of transforming the organism with a nucleic acid which results in an increase in production of phytol by the organism above a level produced under given environmental conditions; and collecting the phytol from the organism. In some instances of this method, the nucleic acid encodes a GPP synthase, a FPP synthase, a geranylgeranyl reductase, a chlorophyllidohydrolase, or a pyrophosphatase. In still other instances, the transformation is a chloroplast transformation. In still other embodiments, the enzyme is endogenous to the organism or is homologous to an endogenous enzyme of the organism or is exogenous to the organism. In some instances, the enzyme is overexpressed. Expression of the enzyme may be regulated by an inducible promoter. The disclosed method may further comprise transformation of the organism with a nucleic acid which results in production of dimethylallyl alcohol, isopentyl alcohol, geraniol, farnesol or geranylgeraniol. In some instances, the organism used in practicing the method is a photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. The organism may be *C*. *reinhardtii, D. salina, H. pluvalis, S. dimorphus, D. viridis,* or *D. tertiolecta.*

Further provided herein is a composition comprising at least 3% phytol and at least a trace amount of a cellular portion of a genetically modified non-vascular photosynthetic organism. In some instances, the genetically modified organism is modified by an endogenous, heterologous, or exogenous GPP synthase, FPP synthase, geranylgeranyl reductase, chlorophyllidohydrolase, or pyrophosphatase. In other instances, a chloroplast of the organism is genetically modified. The disclosed compositions may further comprise dimethylallyl alcohol, isopentyl alcohol, geraniol, farnesol or geranylgeraniol. In some instances, the cellular portion present in the composition is a from a photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. In other instances, the organism may be *C*. *reinhardtii, D. salina, H. pluvalis, S. dimorphus, D. viridis,* or *D. tertiolecta.*

In some instances, such nucleotide sequence(s) encode one or more polypeptides that function in isoprenoid synthetic pathway. Examples of polypeptides in the isoprenoid biosynthetic pathway include synthases such as C5, C10, C15, C20, C30, and C40 synthases. More specific examples of polypeptides in the isoprenoid pathway limonene synthase, 1,8 cineole synthase, α-pinene synthase, camphene synthase, (+)-sabinene synthase, myrcene synthase, abietadiene synthase, taxadiene synthase, farnesyl pyrophosphate synthase, amorphadiene synthase, (E)-α-bisabolene synthase, diapophytoene synthase, or diapophytoene desaturase. In other embodiments, the synthase is β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, γ-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.

Any of the nucleotides sequences contemplated herein can include one or more heterologous sequences and/or one or more homologous sequences.

In some instances, the products produced can be naturally produced by the organism that is transformed. In other instances, the products are not naturally produced by the organism that is transformed.

In some instances, a product (e.g. fuel, fuel feedstock, fragrance, insecticide) is a hydrocarbon-rich molecule, e.g. an isoprenoid. An isoprenoid (classified by the number of isoprene units) can be a hemiterpene, monoterpene, sesquiterpene, diterpene, triterpene, or tetraterpene. In specific embodiments, the isoprenoid may be a naturally occurring isoprenoid, such as a steroid or carotenoid. Subclasses of carotenoids include carotenes and xanthophylls. Some isoprenoids are pure hydrocarbons (e.g. limonene) and others are hydrocarbon derivatives (e.g. cineole).

Any of the nucleotide sequences herein can further include codons biased for expression of the nucleotide sequences in the organism transformed. In some instances, codons in the nucleotide sequences are A/T rich in a third nucleotide position of the codons. For example, at least 50% of the third nucleotide position of the codons may be A or T. In other instances, the codons are G/C rich, for example at least 50% of the third nucleotide positions of the codons may be G or C.

The nucleotide sequences herein can be adapted for chloroplast expression. For example, a nucleotide sequence herein can comprise a chloroplast specific promoter or chloroplast specific regulatory control region. The nucleotide sequences can also be adapted for nuclear expression. For example, a nucleotide sequence can comprise a nuclear specific promoter or nuclear specific regulatory control regions. The nuclear sequences can encode a protein with a targeting sequence that encodes a chloroplast targeting protein (e.g., a chloroplast transit peptide), or a signal peptide that directs a protein to the endomembrane system for deposition in the endoplasmic reticulum or plasma membrane.

Fuel products are produced by altering the enzymatic content of the cell to increase the biosynthesis of specific fuel molecules. For example, nucleotide sequences encoding biosynthetic enzymes can be introduced into the chloroplast of a photosynthetic organism. Nucleotide sequences encoding fuel biosynthetic enzymes can also be introduced into the nuclear genome of the photosynthetic organisms. Nucleotide sequences introduced into the nuclear genome can direct accumulation of the biosynthetic enzyme in the cytoplasm of the cell, or may direct accumulation of the biosynthetic enzyme in the chloroplast of the photosynthetic organism.

Any of the nucleotide sequences herein may further comprise a regulatory control sequence. Regulatory control sequences can include one or more of the following: a promoter, an intron, an exon, processing elements, 3' untranslated region, 5' untranslated region, RNA stability elements, or translational enhancers A promoter may be one or more of the following: a promoter adapted for expression in the organism, an algal promoter, a chloroplast promoter, and a nuclear promoter, any of which may be a native or synthetic promoters. A regulatory control sequence can be inducible or autoregulatable. A regulatory control sequence can include autologous and/or heterologous sequences. In some cases, control sequences can be flanked by a first homologous sequence and a second homologous sequence. The first and second homologous sequences can each be at least 500 nucleotides in length. The homologous sequences can allow for either homologous recombination or can act to insulate the heterologous sequence to facilitate gene expression.

In some instances, a nucleotide sequence may allow for secretion of the product (e.g., a protein) from the cell. In these cases, the nucleotide sequences herein may encode a protein that enhances or initiates or increases the rate of secretion of a product from an organism to the external environment.

The present invention also contemplates organisms transformed with the one or more nucleotide sequences or expression vectors herein. Such organisms are preferably photosynthetic and can be, e.g., unicellular or mutlicellular. For example, such organisms can be multicellular or unicellular algae or cyanobacteria. Some examples of algae contemplated herein include rhodophyta, chlorophyta, heterokontophyta, tribophyta, glaucophyta, chlorarachniophytes, euglenoids, haptophyta, cryptomonads, dinoflagellata, and phytoplankton.

Any of the organisms contemplated herein can be transiently or stably transformed with one or more of the expression vectors described herein. Preferably, the production of the product by the organism does not render the organism unviable.

The present invention also contemplates methods for producing a fuel product. The method can include transforming a non-vascular, photosynthetic organism with an expression vector, growing the organism; and collecting the fuel product produced by the organism. The expression vector can encode a protein that alters the biosynthetic pathway of a photosynthetic organism to allow for increased production or accumulation of a fuel molecule. The expression vector might also encode regulatory elements that alter a native enzyme in a biosynthetic pathway to allow for increased fuel production or accumulation. The vector may also encode a protein or regulatory elements that allows for secretion or increased secretion of a fuel molecule.

The present invention also provides a business method comprising providing a carbon credit to a party growing a genetically modified non-vascular, photosynthetic organism adapted to produce a fuel product. The organism may be any of the ones described herein. In some embodiments, the carbon credit is exchanged for one or more of the following: a substantially liquid monetary instrument, commitment of at least one of present and future business opportunity, a legal grant regarding an intellectual property right, government tax subsidy, access to purchasers of a given market; or use of a carbon emission process not comprising growing the organism. The carbon credit may be substantially received directly from a regulatory agency. Alternatively, the carbon credit is substantially received directly from an administrative entity. The carbon credit may be regulated by at least one entity selected from the group consisting of: a city, county, state, provincial, national, regional, multi-national, and international sovereign entity.

### BRIEF DESCRIPTION OF THE FIGURES

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 is a representation of a naturally occurring enzyme pathway in C. *reinhardtii.*
Figure 2 is a representation of one example of a modification of enzyme pathway in C. *reinhardtii.*
Figure 3, panels A-D provide a schematic representation of nucleic acid constructs of the present invention.
Figure 4, panels A-D show PCR and Western analysis of *C. reinhardtii* transformed with FPP synthase and bisabolene synthase.
Figure 5 shows gas chromatography - mass spectrometry analysis of *C. reinhardtii* transformed with FPP synthase and bisabolene synthase.
Figure 6, panels A-E show PCR and Western analysis of *C. reinhardtii* transformed with FPP synthase and squalene synthase.
Figure 7 shows gas chromatography - mass spectrometry analysis of *C. reinhardtii* transformed with FPP synthase and squalene synthase.
Figure 8 shows Western analysis of *C. reinhardtii* transformed with limonene synthase.
Figure 9 shows gas chromatography - mass spectrometry analysis of *C. reinhardtii* transformed with limonene synthase.
Figure 10, panels A-C show PCR and Western analysis of *C. reinhardtii* transformed with GPP synthase.
Figure 11 shows PCR and Western analysis of *C. reinhardtii* transformed with FPP synthase and zingiberene synthase.
Figure 12 shows Western analysis of *C. reinhardtii* transformed with FPP synthase and sesquiterpene synthase.
Figure 13 shows gas chromatography - mass spectrometry analysis of phytol production in *C. reinhardtii* transformed with FPP synthase and sesquiterpene synthase.
Figure 14 shows Western analysis of *E. coli* transformed with FPP synthase and sesquiterpene synthase.
Figure 15 shows gas chromatography - mass spectrometry analysis of FPP and sesquiterpene production in *E*. *coli* transformed with FPP synthase and sesquiterpene synthase.
Figure 16 is a graphic representation of nucleic acid constructs of the present invention.
Figure 17 shows Western analysis of *C. reinhardtii* expressing xylanase 2 from the nucleus.
Figure 18 shows a comparison of xylanase activity from exogenous enzymes expressed in the nucleus and chloroplast of *C. reinhardtii.*
Figure 19 shows Western analysis of *C*. *reinhardtii* expressing endoglucanase from the nucleus.
Figure 20 shows Western analysis of *C. reinhardtii* expressing CBH1 from the nucleus.

### DETAILED DESCRIPTION OF THE INVENTION

The present invention relates to compositions and methods for creating product(s) using one or more photosynthetic organisms. In some instances, the photosynthetic organisms are non-vascular organisms (e.g., cyanobacteria, algae). As detailed herein, a non-vascular photosynthetic organism (NVPO) may be transformed with exogenous, heterologous or autologous nucleic acids which encode one or more enzymes that effect the production of the product(s) of the invention. For example, an NVPO (e.g., *C. reinhardtii*) may be transformed with one or more nucleic acids encoding enzyme(s) (e.g., bisabolene synthase, sesquiterpene synthase) which effect the production of a desired product (e.g., bisabolene, squalene). The product(s) produced may be naturally, or not naturally, produced by the photosynthetic organism. When naturally produced, production may be enhanced by introduction of the nucleic acids of the present invention. For example, transformation of an NVPO with one or more nucleic acids encoding enzymes which effect the production of a desired product (e.g., zingiberene, bisabolene), may result in increased production of another product (e.g., phytol). In still other instances, multiple products may be produced by a transformed NVPO and the multiple products may be naturally occurring, non-naturally occurring, or a combination thereof. The compositions of the present invention may comprise mixtures of naturally and non-naturally occurring products in a ratio of 1:0.1, 1:0.2, 1:0.3, 1:0.4, 1:0.5, 1: 0.6, 1:0.7, 1:0.8, 1:0.9, 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100 or higher.

The products which are produced by the methods of the present invention include hydrocarbons and hydrocarbon derivatives. In certain aspects, the hydrocarbon and/or derivative is an isoprenoid (or terpenoid). The isoprenoids contemplated by the present invention may contain any number of carbon atoms, with isoprenoids containing five to fifty carbon atoms being exemplary. An isoprenoid of the present invention may be one naturally produced by the NVPO prior to transformation (e.g., phytol), or may be produced only after insertion of an exogenous nucleic acid (e.g., zingiberene). A product of the present invention may also contain one or more non-naturally produced isoprenoids in addition to one or more naturally produced isoprenoids. Additionally, the products are produced intracellularly and may be sequestered in the organism. Thus, collection of the product may involve disruption of one or more cells of the organism(s) of the present invention and/or collection of the product from the environment surrounding the organism(s). Collection of the product(s) of the present invention may involve collecting all or part of a liquid environment in which the cells are grown, isolating the cells from the liquid environment, and disrupting the cells prior to or following isolation from the growth environment, or a combination of these.

The collected product may be purified (e.g., refined) following collection. The product may be utilized in the form in which it is collected, or may be altered prior to, or after collection. For example, where the product is a sesquiterpene (C15), the sesquiterpene may be hydrogenated, cracked, or otherwise modified, resulting in a compound with a different number of carbon atoms. In some instances, alteration of the product may yield a fuel product (e.g., octane, butane).

### Organisms

Examples of organisms that can be transformed using the compositions and methods herein include vascular and non-vascular organisms. The organism can be prokaroytic or eukaroytic. The organism can be unicellular or multicellular.

Examples of non-vascular photosynthetic organisms include bryophtyes, such as marchantiophytes or anthocerotophytes. In some instances, the organism is a cyanobacteria. In some instances, the organism is algae (e.g., macroalgae or microalgae). The algae can be unicellular or multicellular algae. In some instances, the organism is a rhodophyte, chlorophyte, heterokontophyte, tribophyte, glaucophyte, chlorarachniophyte, euglenoid, haptophyte, cryptomonad, dinoflagellum, or phytoplankton. For example, the microalgae *Chlamydomonas reinhardtii* may be transformed with a vector, or a linearized portion therof, encoding limonene synthase to produce limonene.

The methods of the present invention are exemplified using the microalga, *C. reinhardtii.* The use of microalgae to express a polypeptide or protein complex according to a method of the invention provides the advantage that large populations of the microalgae can be grown, including commercially (Cyanotech Corp.; Kailua-Kona HI), thus allowing for production and, if desired, isolation of large amounts of a desired product. However, the ability to express, for example, functional mammalian polypeptides, including protein complexes, in the chloroplasts of any plant allows for production of crops of such plants and, therefore, the ability to conveniently produce large amounts of the polypeptides. Accordingly, the methods of the invention can be practiced using any plant having chloroplasts, including, for example, microalga and macroalgae, for example, marine algae and seaweeds, as well as plants that grow in soil.

The term "plant" is used broadly herein to refer to a eukaryotic organism containing plastids, particularly chloroplasts, and includes any such organism at any stage of development, or to part of a plant, including a plant cutting, a plant cell, a plant cell culture, a plant organ, a plant seed, or a plantlet. A plant cell is the structural and physiological unit of the plant, comprising a protoplast and a cell wall. A plant cell can be in the form of an isolated single cell or a cultured cell, or can be part of higher organized unit, for example, a plant tissue, plant organ, or plant. Thus, a plant cell can be a protoplast, a gamete producing cell, or a cell or collection of cells that can regenerate into a whole plant. As such, a seed, which comprises multiple plant cells and is capable of regenerating into a whole plant, is considered plant cell for purposes of this disclosure. A plant tissue or plant organ can be a seed, protoplast, callus, or any other groups of plant cells that is organized into a structural or functional unit. Particularly useful parts of a plant include harvestable parts and parts useful for propagation of progeny plants. A harvestable part of a plant can be any useful part of a plant, for example, flowers, pollen, seedlings, tubers, leaves, stems, fruit, seeds, roots, and the like. A part of a plant useful for propagation includes, for example, seeds, fruits, cuttings, seedlings, tubers, rootstocks, and the like.

A method of the invention can generate a plant containing chloroplasts that are genetically modified to contain a stably integrated polynucleotide (Hager and Bock, Appl. Microbiol. Biotechnol. 54:302-310, 2000). Accordingly, the present invention further provides a transgenic (transplastomic) plant, e.g. *C. reinhardtii,* which comprises one or more chloroplasts containing a polynucleotide encoding one or more heterologous polypeptides, including polypeptides that can specifically associate to form a functional protein complex. A photosynthetic organism of the present invention comprises at least one host cell that is modified to generate a product.

### Vectors, Transformation and Methods.

The organisms/host cells herein can be transformed to modify the production and/or secretion of a product(s) with an expression vector, or a linearized portion therof, for example, to increase production and/or secretion of a product(s). The product(s) can be naturally or not naturally produced by the organism.

The expression vector, or a linearized portion therof, can encode one or more homologous or heterologous nucleotide sequences (derived from the host organism or from a different organism) and/or one or more autologous nucleotide sequences (derived from the same organism) and/or those that encode homologous or heterologous polypeptides. Examples of heterologous nucleotide sequences that can be transformed into an algal host cell include genes from bacteria, fungi, plants, photosynthetic bacteria or other algae. Examples of autologous nucleotide sequences that can be transformed into an algal host cell include isoprenoid producing genes, including genes which encode for proteins which produce isoprenoids with two phosphates (e.g., GPP synthase, FPP synthase), endogenous promoters and 5' UTRs from the psbA, atpA, or rbcL genes. In some instances, a heterolgous sequence is flanked by two autologous sequences or homologous sequences. Homologous sequences include those that have at least 50%, 60%, 70%, 80%, or 90% homology to the sequence in the host cell. In some instances, a homologous sequence is flanked by two autologous sequences. The first and second homologous sequences enable recombination of the heterologous sequence into the genome of the host organism. The first and second homologous sequences can be at least 100, 200, 300, 400, or 500 nucleotides in length.

The expression vector may comprise nucleotide sequences that are codon biased for expression in the organism being transformed. The skilled artisan is well aware of the "codon-bias" exhibited by a specific host cell in usage of nucleotide codons to specify a given amino acid. Without being bound by theory, by using a host cell's preferred codons, the rate of translation may be greater. Therefore, when synthesizing a gene for improved expression in a host cell, it may be desirable to design the gene such that its frequency of codon usage approaches the frequency of preferred codon usage of the host cell. In some organisms, codon bias differs between the nuclear genome and organelle genomes, thus, codon optimization or biasing may be performed for the target genome (e.g., nuclear codon biased, chloroplast codon biased). The codons of the present invention are generally A/T rich, for example, A/T rich in the third nucleotide position of the codons. Typically, the A/T rich codon bias is used for algae. In some embodiments, at least 50% of the third nucleotide position of the codons are A or T. In other embodiments, at least 60%, 70%, 80%, 90%, or 99% of the third nucleotide position of the codons are A or T.

One approach to construction of a genetically manipulated strain of alga involves transformation with a nucleic acid which encodes a gene of interest, typically an enzyme capable of converting a precursor into a fuel product or precursor of a fuel product. In some embodiments, a transformation may introduce nucleic acids into any plastid of the host alga cell (e.g., chloroplast). Transformed cells are typically plated on selective media following introduction of exogenous nucleic acids. This method may also comprise several steps for screening. Initially, a screen of primary transformants is typically conducted to determine which clones have proper insertion of the exogenous nucleic acids. Clones which show the proper integration may be propagated and re-screened to ensure genetic stability. Such methodology ensures that the transformants contain the genes of interest. In many instances, such screening is performed by polymerase chain reaction (PCR); however, any other appropriate technique known in the art may be utilized. Many different methods of PCR are known in the art (e.g., nested PCR, real time PCR). For any given screen, one of skill in the art will recognize that PCR components may be varied to achieve optimal screening results. For example, magnesium concentration may need to be adjusted upwards when PCR is performed on disrupted alga cells to which EDTA (which chelates magnesium) is added to chelate toxic metals. In such instances, magnesium concentration may need to be adjusted upward, or downward (compared to the standard concentration in commercially available PCR kits) by 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 mM. Thus, after adjusting, final magnesium concentration in a PCR reaction may be, for example 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5 mM or higher. Particular examples are utilized in the examples described herein; however, one of skill in the art will recognize that other PCR techniques may be substituted for the particular protocols described. Following screening for clones with proper integration of exogenous nucleic acids, typically clones are screened for the presence of the encoded protein. Protein expression screening typically is performed by Western blot analysis and/or enzyme activity assays.

A recombinant nucleic acid molecule useful in a method of the invention can be contained in a vector. Furthermore, where the method is performed using a second (or more) recombinant nucleic acid molecules, the second recombinant nucleic acid molecule also can be contained in a vector, which can, but need not, be the same vector as that containing the first recombinant nucleic acid molecule. The vector can be any vector useful for introducing a polynucleotide into a chloroplast and, preferably, includes a nucleotide sequence of chloroplast genomic DNA that is sufficient to undergo homologous recombination with chloroplast genomic DNA, for example, a nucleotide sequence comprising about 400 to 1500 or more substantially contiguous nucleotides of chloroplast genomic DNA. Chloroplast vectors and methods for selecting regions of a chloroplast genome for use as a vector are well known (see, for example, Bock, J. Mol. Biol. 312:425-438, 2001; see, also, Staub and Maliga, Plant Cell 4:39-45, 1992; Kavanagh et al., Genetics 152:1111-1122, 1999, each of which is incorporated herein by reference).

In some instances, such vectors include promoters. Promoters useful for the present invention may come from any source (e.g., viral, bacterial, fungal, protist, animal). The promoters contemplated herein can be specific to photosynthetic organisms, non-vascular photosynthetic organisms, and vascular photosynthetic organisms (e.g., algae, flowering plants). As used herein, the term "non-vascular photosynthetic organism," refers to any macroscopic or microscopic organism, including, but not limited to, algae, cyanobacteria and photosynthetic bacteria, which does not have a vascular system such as that found in higher plants. In some instances, the nucleic acids above are inserted into a vector that comprises a promoter of a photosynthetic organism, e.g., algae. The promoter can be a promoter for expression in a chloroplast and/or other plastid. In some instances, the nucleic acids are chloroplast based. Examples of promoters contemplated for insertion of any of the nucleic acids herein into the chloroplast include those disclosed in US Application No. 2004/0014174. The promoter can be a constitutive promoter or an inducible promoter. A promoter typically includes necessary nucleic acid sequences near the start site of transcription, (e.g., a TATA element).

A "constitutive" promoter is a promoter that is active under most environmental and developmental conditions. An "inducible" promoter is a promoter that is active under environmental or developmental regulation. Examples of inducible promoters/regulatory elements include, for example, a nitrate-inducible promoter (Bock et al, Plant MoI. Biol. 17:9 (1991)), or a light-inducible promoter, (Feinbaum et al, MoI Gen. Genet. 226:449 (1991); Lam and Chua, Science 248:471 (1990)), or a heat responsive promoter (Muller et al., Gene 111: 165-73 (1992)).

The entire chloroplast genome of C. *reinhardtii* is available to the public on the world wide web, at the URL "biology.duke.edu/chlamy_genome/- chloro.html" (see "view complete genome as text file" link and "maps of the chloroplast genome" link), each of which is incorporated herein by reference (J. Maul, J. W. Lilly, and D. B. Stem, unpublished results; revised Jan. 28, 2002; to be published as GenBank Acc. No. AF396929). Generally, the nucleotide sequence of the chloroplast genomic DNA is selected such that it is not a portion of a gene, including a regulatory sequence or coding sequence, particularly a gene that, if disrupted due to the homologous recombination event, would produce a deleterious effect with respect to the chloroplast, for example, for replication of the chloroplast genome, or to a plant cell containing the chloroplast. In this respect, the website containing the C. *reinhardtii* chloroplast genome sequence also provides maps showing coding and non-coding regions of the chloroplast genome, thus facilitating selection of a sequence useful for constructing a vector of the invention. For example, the chloroplast vector, p322, is a clone extending from the Eco (Eco RI) site at about position 143.1 kb to the Xho (Xho I) site at about position 148.5 kb (see, world wide web, at the URL "biology.duke.edu/chlamy_genome/chloro.html", and clicking on "maps of the chloroplast genome" link, and "140-150 kb" link; also accessible directly on world wide web at URL "biology.duke.edu/chlam- y/chloro/chloro140.html").

A vector utilized in the practice of the invention also can contain one or more additional nucleotide sequences that confer desirable characteristics on the vector, including, for example, sequences such as cloning sites that facilitate manipulation of the vector, regulatory elements that direct replication of the vector or transcription of nucleotide sequences contain therein, sequences that encode a selectable marker, and the like. As such, the vector can contain, for example, one or more cloning sites such as a multiple cloning site, which can, but need not, be positioned such that a heterologous polynucleotide can be inserted into the vector and operatively linked to a desired element. The vector also can contain a prokaryote origin of replication (ori), for example, an E. coli ori or a cosmid ori, thus allowing passage of the vector in a prokaryote host cell, as well as in a plant chloroplast, as desired.

A regulatory element, as the term is used herein, broadly refers to a nucleotide sequence that regulates the transcription or translation of a polynucleotide or the localization of a polypeptide to which it is operatively linked. Examples include, but are not limited to, an RBS, a promoter, enhancer, transcription terminator, an initiation (start) codon, a splicing signal for intron excision and maintenance of a correct reading frame, a STOP codon, an amber or ochre codon, and an IRES. Additionally, a cell compartmentalization signal (i.e., a sequence that targets a polypeptide to the cytosol, nucleus, chloroplast membrane or cell membrane). In some aspects of the present invention, a cell compartmentalization signal (e.g., a chloroplast targeting sequence) may be ligated to a gene and/or transcript, such that tranlation of the gene occurs in the chloroplast. In other aspects, a cell compartmentalization signal may be ligated to a gene such that, following translation of the gene, the protein is transported to the chloroplast. Such signals are well known in the art and have been widely reported. *See, e.g.,* U.S. Pat. No. 5,776,689; Quinn et al., J. Biol. Chem. 1999; 274(20): 14444-54; von Heijne et al., Eur. J. Biochem. 1989; 180(3): 535-45.

A vector, or a linearized portion therof, may include a nucleotide sequence encoding a reporter polypeptide or other selectable marker. The term "reporter" or "selectable marker" refers to a polynucleotide (or encoded polypeptide) that confers a detectable phenotype. A reporter generally encodes a detectable polypeptide, for example, a green fluorescent protein or an enzyme such as luciferase, which, when contacted with an appropriate agent (a particular wavelength of light or luciferin, respectively) generates a signal that can be detected by eye or using appropriate instrumentation (Giacomin, Plant Sci. 116:59-72, 1996; Scikantha, J. Bacteriol. 178:121, 1996; Gerdes, FEBS Lett. 389:44-47, 1996; see, also, Jefferson, EMBO J. 6:3901-3907, 1997, fl-glucuronidase). A selectable marker generally is a molecule that, when present or expressed in a cell, provides a selective advantage (or disadvantage) to the cell containing the marker, for example, the ability to grow in the presence of an agent that otherwise would kill the cell.

A selectable marker can provide a means to obtain prokaryotic cells or plant cells or both that express the marker and, therefore, can be useful as a component of a vector of the invention (see, for example, Bock, supra, 2001). One class of selectable markers are native or modified genes which restore a biological or physiological function to a host cell (e.g., restores photosynthetic capability, restores a metabolic pathway). Other examples of selectable markers include, but are not limited to, those that confer antimetabolite resistance, for example, dihydrofolate reductase, which confers resistance to methotrexate (Reiss, Plant Physiol. (Life Sci. Adv.) 13:143-149, 1994); neomycin phosphotransferase, which confers resistance to the aminoglycosides neomycin, kanamycin and paromycin (Herrera-Estrella, EMBO J. 2:987-995, 1983), hygro, which confers resistance to hygromycin (Marsh, Gene 32:481-485, 1984), trpB, which allows cells to utilize indole in place of tryptophan; hisD, which allows cells to utilize histinol in place of histidine (Hartman, Proc. Natl. Acad. Sci., USA 85:8047, 1988); mannose-6-phosphate isomerase which allows cells to utilize mannose (WO 94/20627); ornithine decarboxylase, which confers resistance to the ornithine decarboxylase inhibitor, 2-(difluoromethyl)-DL-ornithine (DFMO; McConlogue, 1987, In: Current Communications in Molecular Biology, Cold Spring Harbor Laboratory ed.); and deaminase from *Aspergillus terreus,* which confers resistance to Blasticidin S (Tamura, Biosci. Biotechnol. Biochem. 59:2336-2338, 1995). Additional selectable markers include those that confer herbicide resistance, for example, phosphinothricin acetyltransferase gene, which confers resistance to phosphinothricin (White et al., Nucl. Acids Res. 18:1062, 1990; Spencer et al., Theor. Appl. Genet. 79:625-631, 1990), a mutant EPSPV-synthase, which confers glyphosate resistance (Hinchee et al., BioTechnology 91:915-922, 1998), a mutant acetolactate synthase, which confers imidazolione or sulfonylurea resistance (Lee et al., EMBO J. 7:1241-1248, 1988), a mutant psbA, which confers resistance to atrazine (Smeda et al., Plant Physiol. 103:911-917, 1993), or a mutant protoporphyrinogen oxidase (see U.S. Pat. No. 5,767,373), or other markers conferring resistance to an herbicide such as glufosinate. Selectable markers include polynucleotides that confer dihydrofolate reductase (DHFR) or neomycin resistance for eukaryotic cells and tetracycline; ampicillin resistance for prokaryotes such as *E. coli;* and bleomycin, gentamycin, glyphosate, hygromycin, kanamycin, methotrexate, phleomycin, phosphinotricin, spectinomycin, streptomycin, sulfonamide and sulfonylurea resistance in plants (see, for example, Maliga et al., Methods in Plant Molecular Biology, Cold Spring Harbor Laboratory Press, 1995, page 39).

Reporter genes have been successfully used in chloroplasts of higher plants, and high levels of recombinant protein expression have been reported. In addition, reporter genes have been used in the chloroplast of C. *reinhardtii,* but, in most cases very low amounts of protein were produced. Reporter genes greatly enhance the ability to monitor gene expression in a number of biological organisms. In chloroplasts of higher plants, β-glucuronidase (uidA, Staub and Maliga, EMBO J. 12:601-606, 1993), neomycin phosphotransferase (nptII, Carrer et al., Mol. Gen. Genet. 241:49-56, 1993), adenosyl-3-adenyltransf- erase (aadA, Svab and Maliga, Proc. Natl. Acad. Sci., USA 90:913-917, 1993), and the *Aequorea victoria* GFP (Sidorov et al., Plant J. 19:209-216, 1999) have been used as reporter genes (Heifetz, Biochemie 82:655-666, 2000). Each of these genes has attributes that make them useful reporters of chloroplast gene expression, such as ease of analysis, sensitivity, or the ability to examine expression in situ. Based upon these studies, other heterologous proteins have been expressed in the chloroplasts of higher plants such as *Bacillus thuringiensis* Cry toxins, conferring resistance to insect herbivores (Kota et al., Proc. Natl. Acad. Sci., USA 96:1840-1845, 1999), or human somatotropin (Staub et al., Nat. Biotechnol. 18:333-338, 2000), a potential biopharmaceutical. Several reporter genes have been expressed in the chloroplast of the eukaryotic green alga, *C. reinhardtii,* including aadA (Goldschmidt-Clermont, Nucl. Acids Res. 19:4083-4089 1991; Zerges and Rochaix, Mol. Cell Biol. 14:5268-5277, 1994), uidA (Sakamoto et al., Proc. Natl. Acad. Sci., USA 90:477-501, 19933, Ishikura et al., J. Biosci. Bioeng. 87:307-314 1999), Renilla luciferase (Minko et al., Mol. Gen. Genet. 262:421-425, 1999) and the amino glycoside phosphotransferase from *Acinetobacter baumanii,* aphA6 (Bateman and Purton, Mol. Gen. Genet 263:404-410, 2000).

The vectors described herein may contain modified genes and/or open reading frames containing one or more recombinantly produced features. For example, a gene encoding a protein of interest may be tagged with a useful molecular marker. In some instances, the tag may be an epitope tag or a tag polypeptide. Generally, epitope tags comprise a sufficient number of amino acid residues to provide an epitope against which an antibody can be made, yet is short enough such that it does not interfere with activity of the polypeptide to which it is fused. Preferably a tag is also fairly unique so that an antibody raised to the tag does not substantially cross-react with other epitopes (e.g., a FLAG tag). Othe appropriate tags may be used, for example, affinity tags. Affinity tags are appended to proteins so that they can be purified from their crude biological source using an affinity technique. Examples of such tags include, but are not limited to, chitin binding protein (CBP), maltose binding protein (MBP), glutathione-s-transferase (GST) and metal affinity tags (e.g., pol(His). Positioning of tags at the C- and/or N-terminal may be determined based on, for example, protein function. One of skill in the art will recognize that selection of an appropriate tag will be based on multiple factors, including the intended use, the target protein, cost, etc.

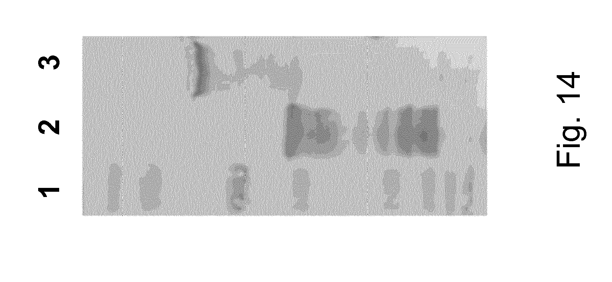

Another example of a modification which may be made to a gene encoding a protein of the present invention is the addition of a cleavage moiety. Typically, the cleavage moiety is a polypeptide of appropriate length to be targeted by a protease. The protease may be naturally occurring in the organism which is intended to be the host for the vectors of the present invention. For example, where the target host is C. *reinhardtii,* a protein may be engineered to contain an amino acid region recognized by membrane-bound proteases (*see, e.g.,* Hoober et al., Plant Physiol. 1992 July; 99(3): 932-937) or a ClpP protease (NCBI # 3053). In other instances, a self-cleaving protease, such as the A2 region (or a functional portion thereof) of Foot and Mouth Disease Virus may be utilized. Halpin, et al., Plant J., 1999; 17(4), 453-459. Typically, cleavage moieties will be utilized for vectors of the present invention which contain fusion proteins. For example, in some instances a vector may comprise a single open reading frame which encodes a fusion protein, a cleavage moiety may be inserted between the sequences encoding portions of the fusion protein (e.g., see **FIG. 14A****).**

Still another modification which may be made to a gene encoding a protein of the present invention is the addition of a secretion signal. Protein secretion is typically conferred by a hydrophobic secretion signal usually located at the N-terminal of the polypeptide which targets the protein to the endoplasmic reticulum and, eventually, the cell membrane. Secretion signals allow for the production and secretion of recombinant proteins in numerous hosts, including NVPOs. One example of a secretion signal which may be utilized in the present invention is the signal from the C. *reinhardtii* carbonic anhydrase protein. Toguri, et al., Eur. J. Biochem. 1986; 158, 443-450. Many such signals are known in the art, and the selection of an appropriate signal depends on, for example, the host cell and protein folding.

In some instances, the vectors of the present invention will contain elements such as an *E*. *coli* or *S*. *cerevisiae* origin of replication. Such features, combined with appropriate selectable markers, allows for the vector to be "shuttled" between the target host cell and the bacterial and/or yeast cell. The ability to passage a shuttle vector of the invention in a secondary host may allow for more convenient manipulation of the features of the vector. For example, a reaction mixture containing the vector and putative inserted polynucleotides of interest can be transformed into prokaryote host cells such as *E. coli,* amplified and collected using routine methods, and examined to identify vectors containing an insert or construct of interest. If desired, the vector can be further manipulated, for example, by performing site directed mutagenesis of the inserted polynucleotide, then again amplifying and selecting vectors having a mutated polynucleotide of interest. A shuttle vector then can be introduced into plant cell chloroplasts, wherein a polypeptide of interest can be expressed and, if desired, isolated according to a method of the invention.

A polynucleotide or recombinant nucleic acid molecule of the invention, can be introduced into plant chloroplasts or nucleus using any method known in the art. A polynucleotide can be introduced into a cell by a variety of methods, which are well known in the art and selected, in part, based on the particular host cell. For example, the polynucleotide can be introduced into a plant cell using a direct gene transfer method such as electroporation or microprojectile mediated (biolistic) transformation using a particle gun, or the "glass bead method," or by pollen-mediated transformation, liposome-mediated transformation, transformation using wounded or enzyme-degraded immature embryos, or wounded or enzyme-degraded embryogenic callus (Potrykus, Ann. Rev. Plant. Physiol. Plant Mol. Biol. 42:205-225, 1991).

The term "exogenous" is used herein in a comparative sense to indicate that a nucleotide sequence (or polypeptide) being referred to is from a source other than a reference source, or is linked to a second nucleotide sequence (or polypeptide) with which it is not normally associated, or is modified such that it is in a form that is not normally associated with a reference material. For example, a polynucleotide encoding an enzyme is heterologous with respect to a nucleotide sequence of a plant chloroplast, as are the components of a recombinant nucleic acid molecule comprising, for example, a first nucleotide sequence operatively linked to a second nucleotide sequence, as is a mutated polynucleotide introduced into a chloroplast where the mutant polynucleotide is not normally found in the chloroplast.

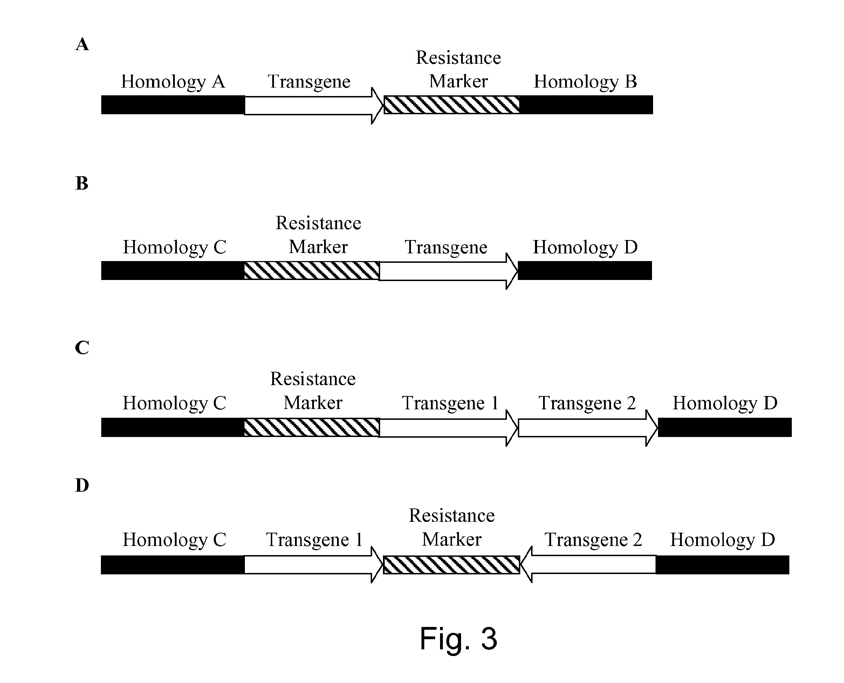

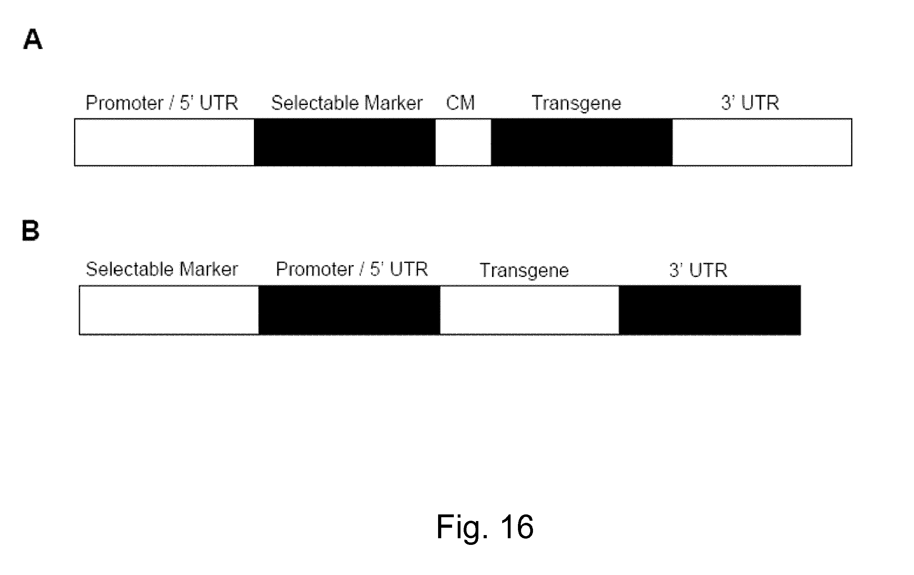

Plastid transformation is a method for introducing a polynucleotide into a plant cell chloroplast (see U.S. Pat. Nos. 5,451,513, 5,545,817, and 5,545,818; WO 95/16783; McBride et al., Proc. Natl. Acad. Sci., USA 91:7301-7305, 1994). In some embodiments, chloroplast transformation involves introducing regions of chloroplast DNA flanking a desired nucleotide sequence, allowing for homologous recombination of the exogenous DNA into the target chloroplast genome. The description herein provides that host cells may be transformed with vectors. One of skill in the art will recognize that such transformation includes transformation with circular or linearized vectors, or linearized portions of a vector. Thus, a host cell comprising a vector may contain the entire vector in the cell (in either circular or linear form), or may contain a linearized portion of a vector of the present invention (e.g., constructs graphically depicted in Figures 3 and 16). In some instances one to 1.5 kb flanking nucleotide sequences of chloroplast genomic DNA may be used. Using this method, point mutations in the chloroplast 16S rRNA and rps12 genes, which confer resistance to spectinomycin and streptomycin, can be utilized as selectable markers for transformation (Svab et al., Proc. Natl. Acad. Sci., USA 87:8526-8530, 1990), and can result in stable homoplasmic transformants, at a frequency of approximately one per 100 bombardments of target leaves.

Microprojectile mediated transformation also can be used to introduce a polynucleotide into a plant cell chloroplast (Klein et al., Nature 327:70-73, 1987). This method utilizes microprojectiles such as gold or tungsten, which are coated with the desired polynucleotide by precipitation with calcium chloride, spermidine or polyethylene glycol. The microprojectile particles are accelerated at high speed into a plant tissue using a device such as the BIOLISTIC PD-1000 particle gun (BioRad; Hercules Calif.). Methods for the transformation using biolistic methods are well known in the art (see, e.g.; Christou, Trends in Plant Science 1:423-431, 1996). Microprojectile mediated transformation has been used, for example, to generate a variety of transgenic plant species, including cotton, tobacco, corn, hybrid poplar and papaya. Important cereal crops such as wheat, oat, barley, sorghum and rice also have been transformed using microprojectile mediated delivery (Duan et al., Nature Biotech. 14:494-498, 1996; Shimamoto, Curr. Opin. Biotech. 5:158-162, 1994). The transformation of most dicotyledonous plants is possible with the methods described above. Transformation of monocotyledonous plants also can be transformed using, for example, biolistic methods as described above, protoplast transformation, electroporation of partially permeabilized cells, introduction of DNA using glass fibers, the glass bead agitation method, and the like.

Transformation frequency may be increased by replacement of recessive rRNA or r-protein antibiotic resistance genes with a dominant selectable marker, including, but not limited to the bacterial aadA gene (Svab and Maliga, Proc. Natl. Acad. Sci., USA 90:913-917, 1993). Approximately 15 to 20 cell division cycles following transformation are generally required to reach a homoplastidic state. It is apparent to one of skill in the art that a chloroplast may contain multiple copies of its genome, and therefore, the term "homoplasmic" or "homoplasmy" refers to the state where all copies of a particular locus of interest are substantially identical. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein.

A method of the invention can be performed by introducing a recombinant nucleic acid molecule into a chloroplast, wherein the recombinant nucleic acid molecule includes a first polynucleotide, which encodes at least one polypeptide (i.e., 1, 2, 3, 4, or more). In some embodiments, a polypeptide is operatively linked to a second, third, fourth, fifth, sixth, seventh, eighth, ninth, tenth and/or subsequent polypeptide. For example, several enzymes in a hydrocarbon production pathway may be linked, either directly or indirectly, such that products produced by one enzyme in the pathway, once produced, are in close proximity to the next enzyme in the pathway.

For transformation of chloroplasts, one major benefit of the present invention is the utilization of a recombinant nucleic acid construct which contains both a selectable marker and one or more genes of interest. Typically, transformation of chloroplasts is performed by co-transformation of chloroplasts with two constructs: one containing a selectable marker and a second containing the gene(s) of interest. Screening of such transformants is laborious and time consuming for multiple reasons. First, the time required to grow some transformed organisms is lengthy. Second, transformants must be screened both for presence of the selectable marker and for the presence of the gene(s) of interest. Typically, secondary screening for the gene(s) of interest is performed by Southern blot (see, e.g. PCT/US2007/072465).

In chloroplasts, regulation of gene expression generally occurs after transcription, and often during translation initiation. This regulation is dependent upon the chloroplast translational apparatus, as well as nuclear-encoded regulatory factors (see Barkan and Goldschmidt-Clermont, Biochemie 82:559-572, 2000; Zerges, Biochemie 82:583-601, 2000). The chloroplast translational apparatus generally resembles that in bacteria; chloroplasts contain 70S ribosomes; have mRNAs that lack 5' caps and generally do not contain 3' poly-adenylated tails (Harris et al., Microbiol. Rev. 58:700-754, 1994); and translation is inhibited in chloroplasts and in bacteria by selective agents such as chloramphenicol.

Some methods of the present invention take advantage of proper positioning of a ribosome binding sequence (RBS) with respect to a coding sequence. It has previously been noted that such placement of an RBS results in robust translation in plant chloroplasts (see U.S. Application 2004/0014174, incorporated herein by reference), and that polypeptides that an advantage of expressing polypeptides in chloroplasts is that the polypeptides do not proceed through cellular compartments typically traversed by polypeptides expressed from a nuclear gene and, therefore, are not subject to certain post-translational modifications such as glycosylation. As such, the polypeptides and protein complexes produced by some methods of the invention can be expected to be produced without such post-translational modification.

The term "polynucleotide" or "nucleotide sequence" or "nucleic acid molecule" is used broadly herein to mean a sequence of two or more deoxyribonucleotides or ribonucleotides that are linked together by a phosphodiester bond. As such, the terms include RNA and DNA, which can be a gene or a portion thereof, a cDNA, a synthetic polydeoxyribonucleic acid sequence, or the like, and can be single stranded or double stranded, as well as a DNA/RNA hybrid. Furthermore, the terms as used herein include naturally occurring nucleic acid molecules, which can be isolated from a cell, as well as synthetic polynucleotides, which can be prepared, for example, by methods of chemical synthesis or by enzymatic methods such as by the polymerase chain reaction (PCR). It should be recognized that the different terms are used only for convenience of discussion so as to distinguish, for example, different components of a composition, except that the term "synthetic polynucleotide" as used herein refers to a polynucleotide that has been modified to reflect chloroplast codon usage.

In general, the nucleotides comprising a polynucleotide are naturally occurring deoxyribonucleotides, such as adenine, cytosine, guanine or thymine linked to 2'-deoxyribose, or ribonucleotides such as adenine, cytosine, guanine or uracil linked to ribose. Depending on the use, however, a polynucleotide also can contain nucleotide analogs, including non-naturally occurring synthetic nucleotides or modified naturally occurring nucleotides. Nucleotide analogs are well known in the art and commercially available, as are polynucleotides containing such nucleotide analogs (Lin et al., Nucl. Acids Res. 22:5220-5234, 1994; Jellinek et al., Biochemistry 34:11363-11372, 1995; Pagratis et al., Nature Biotechnol. 15:68-73, 1997). Generally, a phosphodiester bond links the nucleotides of a polynucleotide of the present invention; however other bonds, including a thiodiester bond, a phosphorothioate bond, a peptide-like bond and any other bond known in the art may be utilized to produce synthetic polynucleotides (Tam et al., Nucl. Acids Res. 22:977-986, 1994; Ecker and Crooke, BioTechnology 13:351360, 1995).

Any of the products described herein can be prepared by transforming an organism to cause the production and/or secretion by such organism of the product. An organism is considered to be a photosynthetic organism even if a transformation event destroys or diminishes the photosynthetic capability of the transformed organism (e.g., exogenous nucleic acid is inserted into a gene encoding a protein required for photosynthesis).

### Fusion Protein Vectors.

In some embodiments of the present invention, a host NVPO nuclear or plastid genome will be targeted for transformation with a construct comprising a fusion protein. Any of the vectors or linearized portions thereof described herein can be modified for nuclear or plastid transformation by incorporating appropriate signals (e.g., a host-cell nuclear origin of replication or plastid origin of replication) or appropriate flanking homology regions (for homologous recombination with the target genome). Some constructs of the present invention are graphically represented in **FIG. 14****.** The construct shown in **FIG. 14A** comprises at least one regulatory element ("Promoter / 5' UTR" and/or "3'UTR") and an open reading frame (i.e., a fusion protein) comprising at least two elements ("Selectable Marker" and "Transgene"). In some instances, one or more of the regulatory elements may be endogenous to the organism to be transformed (e.g., the 5' and 3' regulatory elements are the flanking homology sections directing insertion of the construct into the target genome). In this figure, the promoter is operably linked to the open reading frame, thus driving expression of the fusion protein. One potential advantage to using such an approach is to prevent recombination events, lowered expression or other effects which might lead to deletion or lowered expression of the transgene (i.e., an enzyme producing an isoprenoid). Additionally, by creating a fusion protein comprising a selectable marker and a transgene, insertion sites may be conserved to create multiply-tranformed strains. However, in some instances, a fusion protein may comprise two or more transgenes (e.g., an **FPP** synthase and a zingiberene synthase).

Also shown in **FIG. 14A** is the inclusion of an optional cleavage moiety ("CM"), in-frame with the selectable marker and the transgene. As described above, this cleavage moiety, when present, may allow for cleavage of the gene of interest from the selectable marker. Typically, cleavage at the cleavage moiety will result in two functional proteins (e.g., the selectable marker and the transgene). Cleavage may result in a portion of the cleavage moiety remaining on one, both or neither of the flanking polypeptides. Cleavage may occur after or during translation. Thus, in some embodiments, a fusion protein consisting of the selectable marker, the transgene and the intervening cleavage moiety may be present in a host cell. In other embodiments, the fusion protein is cleaved prior to translation of the full-length fusion protein transcript.

Other modifications may be made to a sequence encoding a fusion protein (or any of the other proteins and classes of proteins described herein). One example is a linker polypeptide. Such polypeptides may provide spatial separation of the proteins of interest, allow for proper folding of the portions of a fusion protein, and/or result from recombinant construction of the fusion protein. In another example, a secretion signal may be fused in-frame with one or more portions of the fusion protein (e.g., the transgene, the selectable marker or both). Typically, a secretion signal will be utilized for fusion proteins targeted for expression in a nuclear genome. In other instances, one or more tags may be fused in-frame with one or more portions of the fusion protein. For example, a nucleotide sequence encoding a poly(His) tag may be ligated in-frame with the sequence encoding the transgene and a nucleotide sequence encoding a FLAG tag is ligated in-frame with the sequence encoding the selectable marker. In still other instances, a nucleic acid encoding a secretion signal may be attached in-frame with a portion of the fusion protein. In some instances, a secretion signal will be attached to the transgene. As is apparent, multiple combinations of selectable markers, transgenes, cleavage moieties, signal sequences and/or tags may be combined into a single open reading frame, based on the need of a practitioner. Thus, the simplified versions of the constructs shown in **FIG. 14** are not meant to be limiting on the scope of the constructs of the present invention. One of skill in the art will also recognize that such combinations of components may also be utilized in constructs which are used to transform the chloroplast.

**FIG. 14B** shows another approach to nuclear transformation in which the transforming construct contains a selectable marker and a transgene under control of different regulatory elements. Although not indicated, such constructs may contain cleavage moieties, secretion signals, and/or tags as described above.

### Nucleic acids, Proteins and Enzymes.

The vectors and other nucleic acids disclosed herein can encode polypeptide(s) that promote the production of intermediates, products, precursors, and derivatives of the products described herein. For example, the vectors can encode polypeptide(s) that promote the production of intermediates, products, precursors, and derivatives in the isoprenoid pathway.

The enzymes utilized in practicing the present invention may be encoded by nucleotide sequences derived from any organism, including bacteria, plants, fungi and animals. In some instances, the enzymes are isoprenoid producing enzymes. As used herein, an "isoprenoid producing enzyme" is a naturally or non-naturally occurring enzyme which produces or increases production of an isoprenoid. In some instances, an isoprenoid producing enzyme produces isoprenoids with two phosphate groups (e.g., GPP synthase, FPP synthase, DMAPP synthase). In other instances, isoprenoid producing enzymes produce isoprenoids with zero, one, three or more phosphates or may produce isoprenoids with other functional groups. Non-limiting examples of such enzymes and their sources are shown in **Table 1.** Polynucleotides encoding enzymes and other proteins useful in the present invention may be isolated and/or synthesized by any means known in the art, including, but not limited to cloning, sub-cloning, and PCR.

**Table 1. Examples of Synthases for Use in the Present Invention.**

| **Synthase** | **Source** | **NCBI protein ID** |
|---|---|---|
| Limonene | *M. spicata* | 2ONH_A |
| Cineole | *S. officinalis* | AAC26016 |
| Pinene | *A. grandis* | AAK83564 |
| Camphene | *A. grandis* | AAB70707 |
| Sabinene | *S. officinalis* | AAC26018 |
| Myrcene | *A. grandis* | AAB71084 |
| Abietadiene | *A. grandis* | Q38710 |
| Taxadiene | *T. brevifolia* | AAK83566 |
| FPP | *G. gallus* | P08836 |
| Amorphadiene | *A. annua* | AAF61439 |
| Bisabolene | *A. grandis* | 081086 |
| Diapophytoene | *S. aureus* | |
| Diapophytoene desaturase | *S. aureus* | |
| GPPS-LSU | *M. spicata* | AAF08793 |
| GPPS-SSU | *M. spicata* | AAF08792 |
| GPPS | *A. thaliana* | CAC16849 |
| GPPS | *C. reinhardtii* | EDP05515 |
| FPP | *E. coli* | NP_414955 |
| FPP | *A. thaliana* | NP_199588 |
| FPP | *A. thaliana* | NP_193452 |
| FPP | *C. reinhardtii* | EDP03194 |
| IPP isomerase | *E. coli* | NP_417365 |
| IPP isomerase | *H. pluvialis* | ABB80114 |
| Limonene | *L. angustifolia* | ABB73044 |
| Monoterpene | *S. lycopersicum* | AAX69064 |
| Terpinolene | *O. basilicum* | AAV63792 |
| Myrcene | *O. basilicum* | AAV63791 |
| Zingiberene | *O. basilicum* | AAV63788 |
| Myrcene | *Q*. *ilex* | CAC41012 |
| Myrcene | *P. abies* | AAS47696 |
| Myrcene, ocimene | *A. thaliana* | NP_179998 |
| Myrcene, ocimene | *A. thaliana* | NP_567511 |
| Sesquiterpene | *Z. mays*; B73 | AAS88571 |
| Sesquiterpene | *A. thaliana* | NP_199276 |
| Sesquiterpene | *A. thaliana* | NP_193064 |
| Sesquiterpene | *A. thaliana* | NP_193066 |
| Curcumene | *P. cablin* | AAS86319 |
| Farnesene | *M domestica* | AAX19772 |
| Farnesene | *C*. *sativus* | AAU05951 |
| Farnesene | *C. junos* | AAK54279 |
| Farnesene | *P. abies* | AAS47697 |
| Bisabolene | *P. abies* | AAS47689 |
| Sesquiterpene | *A. thaliana* | NP_197784 |
| Sesquiterpene | *A. thaliana* | NP_175313 |
| GPP Chimera | | |
| GPPS-LSU+SSU fusion | | |
| Geranylgeranyl reductase | *A. thaliana* | NP_177587 |
| Geranylgeranyl reductase | *C. reinhardtii* | EDP09986 |
| Chlorophyllidohydrolase | *C. reinhardtii* | EDP01364 |
| Chlorophyllidohydrolase | *A. thaliana* | NP_564094 |
| Chlorophyllidohydrolase | *A. thaliana* | NP_199199 |
| Phosphatase | *S. cerevisiae* | AAB64930 |
| FPP A118W | *G. gallus* | |

The synthase may also be botryococcene synthase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, γ-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.

The vectors of the present invention may be capable of stable transformation of multiple photosynthetic organisms, including, but not limited to, photosynthetic bacteria (including cyanobacteria), cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. Other vectors of the present invention are capable of stable transformation of *C*. *reinhardtii, D. salina, H. pluvalis, S. dimorphus, D. viridis,* or *D. tertiolecta.*

A vector herein may encode polypeptide(s) having a role in the mevalonate pathway, such as, for example, thiolase, HMG-CoA synthase, HMG-CoA reductase, mevalonate kinase, phosphemevalonate kinase, and mevalonate-5-pyrophosphate decarboxylase. In other embodiments, the polypeptides are enzymes in the non-mevalonate pathway, such as DOXP synthase, DOXP reductase, 4-diphosphocytidyl-2-C-methyl-D-erythritol synthase, 4-diphophocytidyl-2-C-methyl-D-erythritol kinase, 2-C-methyl-D-erythritol 2,4,-cyclodiphosphate synthase, HMB-PP synthase, HMB-PP reductase, or DOXP reductoisomerase.

In other instances, a vector may comprise a nucleotide sequence encoding a polypeptide in an isoprenoid pathway, such as, for example, a synthase-encoding sequence. The synthase may be a C10, C15, C20, C30, or C40 synthase. In some embodiments, the synthase is limonene synthase, 1,8 cineole synthase, α-pinene synthase, camphene synthase, (+)-sabinene synthase, myrcene synthase, abietadiene synthase, taxadiene synthase, farnesyl pyrophosphate synthase, amorphadiene synthase, (E)-α-bisabolene synthase, diapophytoene synthase, or diapophytoene desaturase. Non-limiting examples of synthases and their amino acid sequences are shown in **Table 2.**

**Table 2. Protein sequences of synthases**

| **SEQ ID NO** | **Synthesized AA Seq** | **Enzyme** |
|---|---|---|
| 1 | MVPRRSGNYNPSRWDVNFIQSLLSDYKEDKHVIRASELVTLVKMELEKETDQI RQLELIDDLQRMGLSDHFQNEFKEILSSIYLDHHYYKNPFPKEERDLYSTSLAF RLLREHGFQVAQEVFDSFKNEEGEFKESLSDDTRGLLQLYEASFLLTEGETTLE SAREFATKFLEEKVNEGGVDGDLLTRIAYSLDIPLHWRIKRPNAPVWIEWYRK RPDMNPVVLELAILDLNIVQAQFQEELKESFRWWRNTGFVEKLPFARDRLVE CYFWNTGIIEPRQHASARIMMGKVNALITVIDDIYDVYGTLEELEQFTDLIRRW DINSIDQLPDYMQLCFLALNNFVDDTSYDVMKEKGVNVIPYLRQSWVDLADK YMVEARWFYGGHKPSLEEYLENSWQSISGPCMLTHIFFRVTDSFTKETVDSLY KYHDLVRWSSFVLRLADDLGTSVEEVSRGDVPKSLQCYMSDYNASEAEARK HVKWLIAEVWKKMNAERVSKDSPFGKDFIGCAVDLGRMAQLMYHNGDGHG TQHPIIHQQMTRTLFEPFAGTGENLYFQGSGGGGSDYKDDDDKGTG | Limonene synthase |
| 2 | MVPRRTGGYQPTLWDFSTIQLFDSEYKEEKHLMRAAGMIAQVNMLLQEEVD SIQRLELIDDLRRLGISCHFDREIVEILNSKYYTNNEIDESDLYSTALRFKLLRQY DFSVSQEVFDCFKNDKGTDFKPSLVDDTRGLLQLYEASFLSAQGEETLHLARD FATKFLHKRVLVDKDINLLSSIERALELPTHWRVQMPNARSFIDAYKRRPDMN PTVLELAKLDFNMVQAQFQQELKEASRWWNSTGLVHELPFVRDRIVECYYW TTGVVERREHGYERIMLTKINALVTTIDDVFDIYGTLEELQLFTTAIQRWDIES MKQLPPYMQICYLALFNFVNEMAYDTLRDKGFNSTPYLRKAWVDLVESYLIE AKWYYMGHKPSLEEYMKNSWISIGGIPILSHLFFRLTDSIEEEDAESMHKYHDI VRASCTILRLADDMGTSLDEVERGDVPKSVQCYMNEKNASEEEAREHVRSLI DQTWKMMNKEMMTSSFSKYFVQVSANLARMAQWIYQHESDGFGMQHSLV NKMLRGLLFDRYEGTGENLYFQGSGGGGSDYKDDDDKGTG | Cineole synthase |
| 3 | MVPRRMGDFHSNLWDDDVIQSLPTAYEEKSYLERAEKLIGEVENMFNSMSLE DGELMSPLNDLIQRLWIVDSLGRLGIHRHFKDEIKSALDYVYSYWGENGIGCG RESAVTDLNSTALGFRTLRLHGYPVSSDVFKAFKGQNGQFSCSENIQTDEEIRG VLNLFRASLIAFPGEKIMDEAEIFSTKYLKEALQKIPVSSLSREIGDVLEYGWHT YLPRLEARNYIHVFGQDTENTKSYVKSKKLLELAKLEFNIFQSLQKRELESLVR WWKESGFPEMTFCRHRHVEYYTLASCIAFEPQHSGFRLGFAKTCHLITVLDD MYDTFGTVDELELFTATMKRWDPSSIDCLPEYMKGVYIAVYDTVNEMAREA EEAQGRDTLTYAREAWEAYIDSYMQEARWIATGYLPSFDEYYENGKVSCGH RISALQPILTMDIPFPDHILKEVDFPSKLNDLACAILRLRGDTRCYKADRARGEE ASSISCYMKDNPGVSEEDALDHINAMISDVIKGLNWELLKPDINVPISAKKHAF DIARAFHYGYKYRDGYSVANVETKSLVTRTLLESVPLGTGENLYFQGSGGGG SDYKDDDDKGTG | Pinene synthase |
| 4 | MVPRRVGNYHSNLWDDDFIQSLISTPYGAPDYRERADRLIGEVKDIMFNFKSL EDGGNDLLQRLLLVDDVERLGIDRHFKKEIKTALDYVNSYWNEKGIGCGRES VVTDLNSTALGLRTLRLHGYTVSSDVLNVFKDKNGQFSSTANIQIEGEIRGVL NLFRASLVAFPGEKVMDEAETFSTKYLREALQKIPASSILSLEIRDVLEYGWHT NLPRLEARNYMDVFGQHTKNKNAAEKLLELAKLEFNIFHSLQERELKHVSRW WKDSGSPEMTFCRHRHVEYYALASCIAFEPQHSGFRLGFTKMSHLITVLDDM YDVFGTVDELELFTATIKRWDPSAMECLPEYMKGVYMMVYHTVNEMARVA EKAQGRDTLNYARQAWEACFDSYMQEAKWIATGYLPTFEEYLENGKVSSAH RPCALQPILTLDIPFPDHILKEVDFPSKLNDLICIILRLRGDTRCYKADRARGEEA SSISCYMKDNPGLTEEDALNHINFMIRDAIRELNWELLKPDNSVPITSKKHAFD ISRVWHHGYRYRDGYSFANVETKSLVMRTVIEPVPLGTGENLYFQGSGGGGS DYKDDDDKGTG | Camphene synthase |
| 5 | MVPRRSGDYQPSLWDFNYIQSLNTPYKEQRHFNRQAELIMQVRMLLKVKME AIQQLELIDDLQYLGLSYFFQDEIKQILSSIHNEPRYFHNNDLYFTALGFRILRQ HGFNVSEDVFDCFKIEKCSDFNANLAQDTKGMLQLYEASFLLREGEDTLELAR RFSTRSLREKFDEGGDEIDEDLSSWIRHSLDLPLHWRVQGLEARWFLDAYARR PDMNPLIFKLAKLNFNIVQATYQEELKDISRWWNSSCLAEKLPFVRDRIVECFF WAIAAFEPHQYSYQRKMAAVIITFITIIDDVYDVYGTIEELELLTDMIRRWDNK SISQLPYYMQVCYLALYNFVSERAYDILKDQHFNSIPYLQRSWVSLVEGYLKE AYWYYNGYKPSLEEYLNNAKISISAPTIISQLYFTLANSIDETAIESLYQYHNIL YLSGTILRLADDLGTSQHELERGDVPKAIQCYMNDTNASEREAVEHVKFLIRE AWKEMNTVTTASDCPFTDDLVAAAANLARAAQFIYLDGDGHGVQHSEIHQQ MGGLLFQPYVGTGENLYFQGSGGGGSDYKDDDDKGTG | Sabinene synthase |
| 6 | MVPRRIGDYHSNIWDDDFIQSLSTPYGEPSYQERAERLIVEVKKIFNSMYLDDG RLMSSFNDLMQRLWIVDSVERLGIARHFKNEITSALDYVFRYWEENGIGCGRD SIVTDLNSTALGFRTLRLHGYTVSPEVLKAFQDQNGQFVCSPGQTEGEIRSVLN LYRASLIAFPGEKVMEEAEIFSTRYLKEALQKIPVSALSQEIKFVMEYGWHTNL PRLEARNYIDTLEKDTSAWLNKNAGKKLLELAKLEFNIFNSLQQKELQYLLR WWKESDLPKLTFARHRHVEFYTLASCIAIDPKHSAFRLGFAKMCHLVTVLDDI YDTFGTIDELELFTSAIKRWNSSEIEHLPEYMKCVYMVVFETVNELTREAEKT QGRNTLNYVRKAWEAYFDSYMEEAKWISNGYLPMFEEYHENGKVSSAYRV ATLQPILTLNAWLPDYILKGIDFPSRFNDLASSFLRLRGDTRCYKADRDRGEEA SCISCYMKDNPGSTEEDALNHINAMVNDIIKELNWELLRSNDNIPMLAKKHAF DITRALHHLYIYRDGFSVANKETKKLVMETLLESMLFGTGENLYFQGSGGGG SDYKDDDDKGTG | Myrcene synthase |
| 7 | MVPQSAEKNDSLSSSTLVKREFPPGFWKDDLIDSLTSSHKVAASDEKRIETLIS EIKNMFRCMGYGETNPSAYDTAWVARIPAVDGSDNPHFPETVEWILQNQLKD GSWGEGFYFLAYDRILATLACIITLTLWRTGETQVQKGIEFFRTQAGKMEDEA DSHRPSGFEIVFPAMLKEAKILGLDLPYDLPFLKQIIEKREAKLKRIPTDVLYAL PTTLLYSLEGLQEIVDWQKIMKLQSKDGSFLSSPASTAAVFMRTGNKKCLDFL NFVLKKFGNHVPCHYPLDLFERLWAVDTVERLGIDRHFKEEIKEALDYVYSH WDERGIGWARENPVPDIDDTAMGLRILRLHGYNVSSDVLKTFRDENGEFFCFL GQTQRGVTDMLNVNRCSHVSFPGETIMEEAKLCTERYLRNALENVDAFDKW AFKKNIRGEVEYALKYPWHKSMPRLEARSYIENYGPDDVWLGKTVYMMPYI SNEKYLELAKLDFNKVQSIHQTELQDLRRWWKSSGFTDLNFTRERVTEIYFSP ASFIFEPEFSKCREVYTKTSNFTVILDDLYDAHGSLDDLKLFTESVKRWDLSLV DQMPQQMKICFVGFYNTFNDIAKEGRERQGRDVLGYIQNVWKVQLEAYTKE AEWSEAKYVPSFNEYIENASVSIALGTVVLISALFTGEVLTDEVLSKIDRESRFL QLMGLTGRLVNDTKTYQAERGQGEVASAIQCYMKDHPKISEEEALQHVYSV MENALEELNREFVNNKIPDIYKRLVFETARIMQLFYMQGDGLTLSHDMEIKEH VKNCLFQPVAGTGENLYFQGSGGGGSDYKDDDDKGTG | Abietadiene synthase |
| 8 | MVPSSSTGTSKVVSETSSTIVDDIPRLSANYHGDLWHHNVIQTLETPFRESSTY QERADELVVKIKDMFNALGDGDISPSAYDTAWVARVATISSDGSEKPRFPQAL NWVFNNQLQDGSWGIESHFSLCDRLLNTTNSVIALSVWKTGHSQVQQGAEFI AENLRLLNEEDELSPDFQIIFPALLQKAKALGINLPYDLPFIKYLSTTREARLTD VSAAADNIPANMLNALEGLEEVIDWNKIMRFQSKDGSFLSSPASTACVLMNT GDEKCFTFLNNLLDKFGGCVPCMYSIDLLERLSLVDNIEHLGIGRHFKQEIKGA LDYVYRHWSERGIGWGRDSLVPDLNTTALGLRTLRMHGYNVSSDVLNNFKD ENGRFFSSAGQTHVELRSVVNLFRASDLAFPDERAMDDARKFAEPYLREALA TKISTNTKLFKEIEYVVEYPWHMSIPRLEARSYIDSYDDNYVWQRKTLYRMPS LSNSKCLELAKLDFNIVQSLHQEELKLLTRWWKESGMADINFTRHRVAEVYF SSATFEPEYSATRIAFTKIGCLQVLFDDMADIFATLDELKSFTEGVKRWDTSLL HEIPECMQTCFKVWFKLMEEVNNDVVKVQGRDMLAHIRKPWELYFNCYVQE REWLEAGYIPTFEEYLKTYAISVGLGPCTLQPILLMGELVKDDVVEKVHYPSN MFELVSLSWRLTNDTKTYQAEKVRGQQASGIACYMKDNPGATEEDAIKHICR VVDRALKEASFEYFKPSNDIPMGCKSFIFNLRLCVQIFYKFIDGYGIANEEIKDY IRKVYIDPIQVGTGENLYFQGSGGGGSDYKDDDDKGTG | Taxadiene synthase |
| 9 | MVPHKFTGVNAKFQQPALRNLSPVVVEREREEFVGFFPQIVRDLTEDGIGHPE VGDAVARLKEVLQYNAPGGKCNRGLTVVAAYRELSGPGQKDAESLRCALAV GWCIELFQAFFLVADDIMDQSLTRRGQLCWYKKEGVGLDAINDSFLLESSVY RVLKKYCRQRPYYVHLLELFLQTAYQTELGQMLDLITAPVSKVDLSHFSEERY KAIVKYKTAFYSFYLPVAAAMYMVGIDSKEEHENAKAILLEMGEYFQIQDDY LDCFGDPALTGKVGTDIQDNKCSWLVVQCLQRVTPEQRQLLEDNYGRKEPEK VAKVKELYEAVGMRAAFQQYEESSYRRLQELIEKHSNRLPKEIFLGLAQKIYK RQKGTGENLYFQGSGGGGSDYKDDDDKGTG | FPP synthase |
| 10 | MVPSLTEEKPIRPIANFPPSIWGDQFLIYEKQVEQGVEQIVNDLKKEVRQLLKE ALDIPMKHANLLKLIDEIQRLGIPYHFEREIDHALQCIYETYGDNWNGDRSSL WFRLMRKQGYYVTCDVFNNYKDKNGAFKQSLANDVEGLLELYEATSMRVP GEIILEDALGFTRSRLSIMTKDAFSTNPALFTEIQRALKQPLWKRLPRIEAAQYI PFYQQQDSHNKTLLKLAKLEFNLLQSLHKEELSHVCKWWKAFDIKKNAPCLR DRIVECYFWGLGSGYEPQYSRARVFFTKAVAVITLIDDTYDAYGTYEELKIFTE AVERWSITCLDTLPEYMKPIYKLFMDTYTEMEEFLAKEGRTDLFNCGKEFVKE FVRNLMVEAKWANEGHIPTTEEHDPVVIITGGANLLTTTCYLGMSDIFTKESV EWAVSAPPLFRYSGILGRRLNDLMTHKAEQERKHSSSSLESYMKEYNVNEEY AQTLIYKEVEDVWKDINREYLTTKNIPRPLLMAVIYLCQFLEVQYAGKDNFTR MGDEYKHLIKSLLVYPMSIGTGENLYFQGSGGGGSDYKDDDDKGTG | Amorpha diene synthase |
| 12 | MVPAGVSAVSKVSSLVCDLSSTSGLIRRTANPHPNVWGYDLVHSLKSPYIDSS YRERAEVLVSEIKAMLNPAITGDGESMITPSAYDTAWVARVPAIDGSARPQFP QTVDWILKNQLKDGSWGIQSHFLLSDRLLATLSCVLVLLKWNVGDLQVEQGI EFIKSNLELVKDETDQDSLVTDFEIIFPSLLREAQSLRLGLPYDLPYIHLLQTKR QERLAKLSREEIYAVPSPLLYSLEGIQDIVEWERIMEVQSQDGSFLSSPASTACV FMHTGDAKCLEFLNSVMIKFGNFVPCLYPVDLLERLLIVDNIVRLGIYRHFEKE IKEALDYVYRHWNERGIGWGRLNPIADLETTALGFRLLRLHRYNVSPAIFDNF KDANGKFICSTGQFNKDVASMLNLYRASQLAFPGENILDEAKSFATKYLREAL EKSETSSAWNNKQNLSQEIKYALKTSWHASVPRVEAKRYCQVYRPDYARIAK CVYKLPYVNNEKFLELGKLDFNIIQSIHQEEMKNVTSWFRDSGLPLFTFARERP LEFYFLVAAGTYEPQYAKCRFLFTKVACLQTVLDDMYDTYGTLDELKLFTEA VRRWDLSFTENLPDYMKLCYQIYYDIVHEVAWEAEKEQGRELVSFFRKGWE DYLLGYYEEAEWLAAEYVPTLDEYIKNGITSIGQRILLLSGVLIMDGQLLSQEA LEKVDYPGRRVLTELNSLISRLADDTKTYKAEKARGELASSIECYMKDHPECT EEEALDHIYSILEPAVKELTREFLKPDDVPFACKKMLFEETRVTMVIFKDGDGF GVSKLEVKDHIKECLIEPLPLGTGENLYFQGSGGGGSDYKDDDDKGTG | Bisabolene synthase |
| 13 | MVPTMMNMNFKYCHKIMKKHSKSFSYAFDLLPEDQRKAVWAIYAVCRKIDD SIDVYGDIQFLNQIKEDIQSIEKYPYEHHHFQSDRRIMMALQHVAQHKNIAFQS FYNLIDTVYKDQHFTMFETDAELFGYCYGVAGTVGEVLTPILSDHETHQTYD VARRLGESLQLINILRDVGEDFDNERIYFSKQRLKQYEVDIAEVYQNGVNNHY IDLWEYYAAIAEKDFQDVMDQIKVFSIEAQPIIELAARIYIEILDEVRQANYTLH ERVFVDKRKKAKLFHENKGTGENLYFQGSGGGGSDYKDDDDKGTG | Diapophytoene synthase |
| 14 | MVPKIAVIGAGVTGLAAAARIASQGHEVTIFEKNNNVGGRMNQLKKDGFTFD MGPTIVMMPDVYKDVFTACGKNYEDYIELRQLRYIYDVYFDHDDRITVPTDL AELQQMLESIEPGSTHGFMSFLTDVYKKYEIARRYFLERTYRKPSDFYNMTSL VQGAKLKTLNHADQLIEHYIDNEKIQKLLAFQTLYIGIDPKRGPSLYSIIPMIEM MFGVHFIKGGMYGMAQGLAQLNKDLGVNIELNAEIEQIIIDPKFKRADAIKVN GDIRKFDKILCTADFPSVAESLMPDFAPIKKYPPHKIADLDYSCSAFLMYIGIDI DVTDQVRLHNVIFSDDFRGNIEEIFEGRLSYDPSIYVYVPAVADKSLAPEGKTG IYVLMPTPELKTGSGIDWSDEALTQQIKEIIYRKLATIEVFEDIKSHIVSETIFTPN DFEQTYHAKFGSAFGLMPTLAQSNYYRPQNVSRDYKDLYFAGASTHPGAGVP IVLTSAKITVDEMIKDIERGVGTGENLYFQGSGGGGSDYKDDDDKGTG | Diapophytoene desaturase |
| 15 | MVPAFDFDGYMLRKAKSVNKALEAAVQMKEPLKIHESMRYSLLAGGKRVRP MLCIAACELVGGDESTAMPAACAVEMIHTMSLMHDDLPCMDNDDLRRGKPT NHMAFGESVAVLAGDALLSFAFEHVAAATKGAPPERIVRVLGELAVSIGSEGL VAGQVVDVCSEGMAEVGLDHLEFIHHHKTAALLQGSVVLGAILGGGKEEEV AKLRKFANCIGLLFQVVDDILDVTKSSKELGKTAGKDLVADKTTYPKLIGVEK SKEFADRLNREAQEQLLHFHPHRAAPLIALANYIAYRDNGTGENLYFQGSGG GGSDYKDDDDKGTG | GPPS-LSU synthase |
| 16 | MVPSQPYWAAIEADIERYLKKSITIRPPETVFGPMHHLTFAAPATAASTLCLAA CELVGGDRSQAMAAAAAIHLVHAAAYVHEHLPLTDGSRPVSKPAIQHKYGPN VELLTGDGIVPFGFELLAGSVDPARTDDPDRILRVIIEISRAGGPEGMISGLHRE EEIVDGNTSLDFIEYVCKKKYGEMHACGAACGAILGGAAEEEIQKLRNFGLYQ GTLRGMMEMKNSHQLIDENIIGKLKELALEELGGFHGKNAELMSSLVAEPSLY AAGTGENLYFQGSGGGGSDYKDDDDKGTG | GPPS-SSU synthase |
| 17 | MVPLLSNKLREMVLAEVPKLASAAEYFFKRGVQGKQFRSTILLLMATALDVR VPEALIGESTDIVTSELRVRQRGIAEITEMIHVASLLHDDVLDDADTRRGVGSL NVVMGNKMSVLAGDFLLSRACGALAALKNTEVVALLATAVEHLVTGETMEI TSSTEQRYSMDYYMQKTYYKTASLISNSCKAVAVLTGQTAEVAVLAFEYGRN LGLAFQLIDDILDFTGTSASLGKGSLSDIRHGVITAPILFAMEEFPQLREVVDQV EKDPRNVDIALEYLGKSKGIQRARELAMEHANLAAAAIGSLPETDNEDVKRSR RALIDLTHRVITRNKGTGENLYFQGSGGGGSDYKDDDDKGTG | GPPS |
| 18 | MVPVVSERLRHSVTTGIPALKTAAEYFFRRGIEGKRLRPTLALLMSSALSPAAP SPEYLQVDTRPAAEHPHEMRRRQQRLAEIAELIHVASLLHDDVIDDAQTRRGV LSLNTSVGNKTAILAGDFLLARASVTLASLRNSEIVELMSQVLEHLVSGEIMQ MTATSEQLLDLEHYLAKTYCKTASLMANSSRSVAVLAGAAPEVCDMAWSYG RHLGIAFQVVDDLLDLTGSSSVLGKPALNDMRSGLATAPVLFAAQEEPALQA LILRRFKHDGDVTKAMSLIERTQGLRRAEELAAQHAKAAADMIRCLPTAQSD HAEIAREALIQITHRVLTRKKGTGENLYFQGSGGGGSDYKDDDDKGTG | GPPS |
| 19 | MVPDFPQQLEACVKQANQALSRFIAPLPFQNTPVVETMQYGALLGGKRLRPF LVYATGHMFGVSTNTLDAPAAAVECIHAYSLIHDDLPAMDDDDLRRGLPTCH VKFGEANAILAGDALQTLAFSILSDADMPEVSDRDRISMISELASASGIAGMCG GQALDLDAEGKHVPLDALERIHRHKTGALIRAAVRLGALSAGDKGRRALPVL DKYAESIGLAFQVQDDILDVVGDTATLGKRQGADQQLGKSTYPALLGLEQAR KKARDLIDDARQSLKQLAEQSLDTSALEALADYIIQRNKGTGENLYFQGSGGG GSDYKDDDDKGTG | FPP synthase |
| 20 | MVPSVSCCCRNLGKTIKKAIPSHHLHLRSLGGSLYRRRIQSSSMETDLKSTFLN VYSVLKSDLLHDPSFEFTNESRLWVDRMLDYNVRGGKLNRGLSVVDSFKLLK QGNDLTEQEVFLSCALGWCIEWLQAYFLVLDDIMDNSVTRRGQPCWFRVPQ VGMVAINDGILLRNHIHRILKKHFRDKPYYVDLVDLFNEVELQTACGQMIDLI TTFEGEKDLAKYSLSIHRRIVQYKTAYYSFYLPVACALLMAGENLENHIDVKN VLVDMGIYFQVQDDYLDCFADPETLGKIGTDIEDFKCSWLVVKALERCSEEQT KILYENYGKPDPSNVAKVKDLYKELDLEGVFMEYESKSYEKLTGAIEGHQSK AIQAVLKSFLAKIYKRQKGTGENLYFQGSGGGGSDYKDDDDKGTG | FPP synthase |
| 21 | MVPADLKSTFLDVYSVLKSDLLQDPSFEFTHESRQWLERMLDYNVRGGKLNR GLSVVDSYKLLKQGQDLTEKETFLSCALGWCIEWLQAYFLVLDDIMDNSVTR RGQPCWFRKPKVGMIAINDGILLRNHIHRILKKHFREMPYYVDLVDLFNEVEF QTACGQMIDLITTFDGEKDLSKYSLQIHRRIVEYKTAYYSFYLPVACALLMAG ENLENHTDVKTVLVDMGIYFQVQDDYLDCFADPETLGKIGTDIEDFKCSWLV VKALERCSEEQTKILYENYGKAEPSNVAKVKALYKELDLEGAFMEYEKESYE KLTKLIEAHQSKAIQAVLKSFLAKIYKRQKGTGENLYFQGSGGGGSDYKDDD DKGTG | FPP synthase |
| 22 | MVPSGEPTPKKMKATYVHDRENFTKVYETLRDELLNDDCLSPAGSPQAQAA QEWFKEVNDYNVPGGKLNRGMAVYDVLASVKGPDGLSEDEVFKANALGWC IEWLQAFFLVADDIMDGSITRRGQPCWYKQPKVGMIACNDYILLECCIYSILKR HFRGHAAYAQLMDLFHETTFQTSHGQLLDLTTAPIGSVDLSKYTEDNYLRIVT YKTAYYSFYLPVACGMVLAGITDPAAFDLAKNICVEMGQYFQIQDDYLDCYG DPEVIGKIGTDIEDNKCSWLVCTALKIATEEQKEVIKANYGHKEAESVAAIKAL YVELGIEQRFKDYEAASYAKLEGTISEQTLLPKAVFTSLLAKIYKRKKGTGENL YFQGSGGGGSDYKDDDDKGTG | FPP synthase |
| 23 | MVPQTEHVILLNAQGVPTGTLEKYAAHTADTRLHLAFSSWLFNAKGQLLVTR RALSKKAWPGVWTNSVCGHPQLGESNEDAVIRRCRYELGVEITPPESIYPDFR YRATDPSGIVENEVCPVFAARTTSALQINDDEVMDYQWCDLADVLHGIDATP WAFSPWMVMQATNREARKRLSAFTQLKGTGENLYFQGSGGGGSDYKDDDD KGTG | IPP isomerase |
| 24 | MVPLRSLLRGLTHIPRVNSAQQPSCAHARLQFKLRSMQLLAENRTDHMRGAS TWAGGQSQDELMLKDECILVDADDNITGHASKLECHKFLPHQPAGLLHRAFS VFLFDDQGRLLLQQRARSKITFPSVWANTCCSHPLHGQTPDEVDQQSQVADG TVPGAKAAAIRKLEHELGIPAHQLPASAFRFLTRLHYCAADVQPAATQSALW GEHEMDYILFIRANVTLAPNPDEVDEVRYVTQEELRQMMQPDNGLQWSPWF RIIAARFLERWWADLDAALNTDKHEDWGTVHHINEAGTGENLYFQGSGGGG SDYKDDDDKGTG | IPP isomerase |
| 25 | MVPRRSGNYNPTAWDFNYIQSLDNQYKKERYSTRHAELTVQVKKLLEEEME AVQKLELIEDLKNLGISYPFKDNIQQILNQIYNEHKCCHNSEVEEKDLYFTALR FRLLRQQGFEVSQEVFDHFKNEKGTDFKPNLADDTKGLLQLYEASFLLREAED TLELARQFSTKLLQKKVDENGDDKIEDNLLLWIRRSLELPLHWRVQRLEARGF LDAYVRRPDMNPIVFELAKLDFNITQATQQEELKDLSRWWNSTGLAEKLPFA RDRVVESYFWAMGTFEPHQYGYQRELVAKIIALATVVDDVYDVYGTLEELEL FTDAIRRWDRESIDQLPYYMQLCFLTVNNFVFELAHDVLKDKSFNCLPHLQRS WLDLAEAYLVEAKWYHSRYTPSLEEYLNIARVSVTCPTIVSQMYFALPIPIEKP VIEIMYKYHDILYLSGMLLRLPDDLGTASFELKRGDVQKAVQCYMKERNVPE NEAREHVKFLIREASKQINTAMATDCPFTEDFAVAAANLGRVANFVYVDGDG FGVQHSKIYEQIGTLMFEPYPGTGENLYFQGSGGGGSDYKDDDDKGTG | Limonene synthase |
| 26 | MVPRRSGNYKPTMWDFQFIQSVNNLYAGDKYMERFDEVKKEMKKNLMMM VEGLIEELDVKLELIDNLERLGVSYHFKNEIMQILKSVHQQITCRDNSLYSTAL KFRLLRQHGFHISQDIFNDFKDMNGNVKQSICNDTKGLLELYEASFLSTECETT LKNFTEAHLKNYVYINHSCGDQYNNIMMELVVHALELPRHWMMPRLETRW YISIYERMPNANPLLLELAKLDFNIVQATHQQDLKSLSRWWKNMCLAEKLSFS RNRLVENLFWAVGTNFEPQHSYFRRLITKIIVFVGIIDDIYDVYGKLDELELFTL AVQRWDTKAMEDLPYYMQVCYLALINTTNDVAYEVLRKHNINVLPYLTKSW TDLCKSYLQEARWYYNGYKPSLEEYMDNGWISIAVPMVLAHALFLVTDPITK EALESLTNYPDIIRCSATIFRLNDDLGTSSDELKRGDVPKSIQCYMNEKGVSEE EAREHIRFLIKETWKFMNTAHHKEKSLFCETFVEIAKNIATTAHCMYLKGDSH GIQNTDVKNSISNILFHPIIIGTGENLYFQGSGGGGSDYKDDDDKGTG | Monoterp ene synthase |
| 27 | MVPRRSGNYEPSAWDFNYLQSLNNYHHKEERYLRRQADLIEKVKMILKEEK MEALQQLELIDDLRNLGLSYCFDDQINHILTTIYNQHSCFHYHEAATSEEANLY FTALGFRLLREHGFKVSQEVFDRFKNEKGTDFRPDLVDDTQGLLQLYEASFLL REGEDTLEFARQFATKFLQKKVEEKMIEEENLLSWTLHSLELPLHWRIQRLEA KWFLDAYASRPDMNPIIFELAKLEFNIAQALQQEELKDLSRWWNDTGIAEKLP FARDRIVESHYWAIGTLEPYQYRYQRSLIAKIIALTTVVDDVYDVYGTLDELQ LFTDAIRRWDIESINQLPSYMQLCYLAIYNFVSELAYDIFRDKGFNSLPYLHKS WLDLVEAYFQEAKWYHSGYTPSLEQYLNIAQISVASPAILSQIYFTMAGSIDKP VIESMYKYRHILNLSGILLRLPDDLGTASDELGRGDLAKAMQCYMKERNVSE EEARDHVRFLNREVSKQMNPARAADDCPFTDDFVVAAANLGRVADFMYVE GDGLGLQYPAIHQHMAELLFHPYAGTGENLYFQGSGGGGSDYKDDDDKGTG | Terpinolene synthase |
| 28 | MVPRRSGNYQPSAWDFNYIQSLNNNHSKEERHLERKAKLIEEVKMLLEQEMA AVQQLELIEDLKNLGLSYLFQDEIKIILNSIYNHHKCFHNNHEQCIHVNSDLYF VALGFRLFRQHGFKVSQEVFDCFKNEEGSDFSANLADDTKGLLQLYEASYLV TEDEDTLEMARQFSTKILQKKVEEKMIEKENLLSWTLHSLELPLHWRIQRLEA KWFLDAYASRPDMNPIIFELAKLEFNIAQALQQEELKDLSRWWNDTGIAEKLP FARDRIVESHYWAIGTLEPYQYRYQRSLIAKIIALTTVVDDVYDVYGTLDELQ LFTDAIRRWDIESINQLPSYMQLCYLAIYNFVSELAYDIFRDKGFNSLPYLHKS WLDLVEAYFVEAKWFHDGYTPTLEEYLNNSKITIICPAIVSEIYFAFANSIDKTE VESIYKYHDILYLSGMLARLPDDLGTSSFEMKRGDVAKAIQCYMKEHNASEE EAREHIRFLMREAWKHMNTAAAADDCPFESDLVVGAASLGRVANFVYVEGD GFGVQHSKIHQQMAELLFYPYQGTGENLYFQGSGGGGSDYKDDDDKGTG | Myrcene synthase |
| 29 | MVPRRSANYQASIWDDNFIQSLASPYAGEKYAEKAEKLKTEVKTMIDQTRDE LKQLELIDNLQRLGICHHFQDLTKKILQKIYGEERNGDHQHYKEKGLHFTALR FRILRQDGYHVPQDVFSSFMNKAGDFEESLSKDTKGLVSLYEASYLSMEGETI LDMAKDFSSHHLHKMVEDATDKRVANQIIHSLEMPLHRRVQKLEAIWFIQFY ECGSDANPTLVELAKLDFNMVQATYQEELKRLSRWYEETGLQEKLSFARHRL AEAFLWSMGIIPEGHFGYGRMHLMKIGAYITLLDDIYDVYGTLEELQVLTEIIE RWDINLLDQLPEYMQIFFLYMFNSTNELAYEILRDQGINVISNLKGLWVELSQ CYFKEATWFHNGYTPTTEEYLNVACISASGPVILFSGYFTTTNPINKHELQSLE RHAHSLSMILRLADDLGTSSDEMKRGDVPKAIQCFMNDTGCCEEEARQHVKR LIDAEWKKMNKDILMEKPFKNFCPTAMNLGRISMSFYEHGDGYGGPHSDTKK KMVSLFVQPMNITIGTGENLYFQGSGGGGSDYKDDDDKGTG | Zingiberene synthase |
| 30 | MVPRRSANYQPSIWNHDYIESLRIEYVGETCTRQINVLKEQVRMMLHKVVNP LEQLELIEILQRLGLSYHFEEEIKRILDGVYNNDHGGDTWKAENLYATALKFR LLRQHGYSVSQEVFNSFKDERGSFKACLCEDTKGMLSLYEASFFLIEGENILEE ARDFSTKHLEEYVKQNKEKNLATLVNHSLEFPLHWRMPRLEARWFINIYRHN QDVNPILLEFAELDFNIVQAAHQADLKQVSTWWKSTGLVENLSFARDRPVEN FFWTVGLIFQPQFGYCRRMFTKVFALITTIDDVYDVYGTLDELELFTDVVERW DINAMDQLPDYMKICFLTLHNSVNEMALDTMKEQRFHIIKYLKKAWVDLCR YYLVEAKWYSNKYRPSLQEYIENAWISIGAPTILVHAYFFVTNPITKEALDCLE EYPNIIRWSSIIARLADDLGTSTDELKRGDVPKAIQCYMNETGASEEGAREYIK YLISATWKKMNKDRAASSPFSHIFIEIALNLARMAQCLYQHGDGHGLGNRET KDRILSLLIQPIPLNKDGTGENLYFQGSGGGGSDYKDDDDKGTG | Myrcene synthase |
| 31 | MVPRRIGDYHSNLWNDDFIQSLTTPYGAPSYIERADRLISEVKEMFNRMCMED GELMSPLNDLIQRLWTVDSVERLGIDRHFKNEIKASLDYVYSYWNEKGIGCGR QSVVTDLNSTALGLRILRQHGYTVSSEVLKVFEEENGQFACSPSQTEGEIRSFL NLYRASLIAFPGEKVMEEAQIFSSRYLKEAVQKIPVSGLSREIGDVLEYGWHTN LPRWEARNYMDVFGQDTNTSFNKNKMQYMNTEKILQLVKLEFNIFHSLQQRE LQCLLRWWKESGLPQLTFARHRHVEFYTLASCIACEPKHSAFRLGFAKMCHL VTVLDDVYDTFGKMDELELFTAAVKRWDLSETERLPEYMKGLYVVVFETVN ELAQEAEKTQGRNTLNYVRKAWEAYFDSYMKEAEWISTGYLPTFEEYCENG KVSSAYRVAALQPILTLDVQLPDDILKGIDFPSRFNDLASSFLRLRGDTRCYEA DRARGEEASCISCYMKDNPGSTEEDALNHINAMINDIIRELNWEFLKPDSNIPM PARKHAFDITRALHHLYIYRDGFSVANKETKNLVEKTLLESMLFGTGENLYFQ GSGGGGSDYKDDDDKGTG | Myrcene synthase |
| 32 | MVPRRSANYQPSRWDHHHLLSVENKFAKDKRVRERDLLKEKVRKMLNDEQ KTYLDQLEFIDDLQKLGVSYHFEAEIDNILTSSYKKDRTNIQESDLHATALEFR LFRQHGFNVSEDVFDVFMENCGKFDRDDIYGLISLYEASYLSTKLDKNLQIFIR PFATQQLRDFVDTHSNEDFGSCDMVEIVVQALDMPYYWQMRRLSTRWYIDV YGKRQNYKNLVVVEFAKIDFNIVQAIHQEELKNVSSWWMETGLGKQLYFAR DRIVENYFWTIGQIQEPQYGYVRQTMTKINALLTTIDDIYDIYGTLEELQLFTV AFENWDINRLDELPEYMRLCFLVIYNEVNSIACEILRTKNINVIPFLKKSWTDV SKAYLVEAKWYKSGHKPNLEEYMQNARISISSPTIFVHFYCVFSDQLSIQVLET LSQHQQNVVRCSSSVFRLANDLVTSPDELARGDVCKSIQCYMSETGASEDKA RSHVRQMINDLWDEMNYEKMAHSSSILHHDFMETVINLARMSQCMYQYGD GHGSPEKAKIVDRVMSLLFNPIPLDGTGENLYFQGSGGGGSDYKDDDDKGTG | Myrcene, ocimene synthase |
| 33 | MVPRRSANYQPSLWQHEYLLSLGNTYVKEDNVERVTLLKQEVSKMLNETEG LLEQLELIDTLQRLGVSYHFEQEIKKTLTNVHVKNVRAHKNRIDRNRWGDLY ATALEFRLLRQHGFSIAQDVFDGNIGVDLDDKDIKGILSLYEASYLSTRIDTKL KESIYYTTKRLRKFVEVNKNETKSYTLRRMVIHALEMPYHRRVGRLEARWYI EVYGERHDMNPILLELAKLDFNFVQAIHQDELKSLSSWWSKTGLTKHLDFVR DRITEGYFSSVGVMYEPEFAYHRQMLTKVFMLITTIDDIYDIYGTLEELQLFTTI VEKWDVNRLEELPNYMKLCFLCLVNEINQIGYFVLRDKGFNVIPYLKESWAD MCTTFLKEAKWYKSGYKPNFEEYMQNGWISSSVPTILLHLFCLLSDQTLDILG SYNHSVVRSSATILRLANDLATSSEELARGDTMKSVQCHMHETGASEAESRA YIQGIIGVAWDDLNMEKKSCRLHQGFLEAAANLGRVAQCVYQYGDGHGCPD KAKTVNHVRSLLVHPLPLNGTGENLYFQGSGGGGSDYKDDDDKGTG | Myrcene, ocimene synthase |
| 34 | MVPASPPAHRSSKAADEELPKASSTFHPSLWGSFFLTYQPPTAPQRANMKERA EVLRERVRKVLKGSTTDQLPETVNLILTLQRLGLGYYYENEIDKLLHQIYSNSD YNVKDLNLVSQRFYLLRKNGYDVPSDVFLSFKTEEGGFACAAADTRSLLSLY NAAYLRKHGEEVLDEAISSTRLRLQDLLGRLLPESPFAKEVSSSLRTPLFRRVGI LEARNYIPIYETEATRNEAVLELAKLNFNLQQLDFCEELKHCSAWWNEMIAKS KLTFVRDRIVEEYFWMNGACYDPPYSLSRIILTKITGLITIIDDMFDTHGTTEDC MKFAEAFGRWDESAIHLLPEYMKDFYILMLETFQSFEDALGPEKSYRVLYLK QAMERLVELYSKEIKWRDDDYVPTMSEHLQVSAETIATIALTCSAYAGMGDM SIRKETFEWALSFPQFIRTFGSFVRLSNDVVSTKREQTKDHSPSTVHCYMKEHG TTMDDACEKIKELIEDSWKDMLEQSLALKGLPKVVPQLVFDFSRTTDNMYRD RDALTSSEALKEMIQLLFVEPIPEGTGENLYFQGSGGGGSDYKDDDDKGTG | Sesquiterpene synthase |
| 35 | MVPEALGNFDYESYTNFTKLPSSQWGDQFLKFSIADSDFDVLEREIEVLKPKV RENIFVSSSTDKDAMKKTILSIHFLDSLGLSYHFEKEIEESLKHAFEKIEDLIADE NKLHTISTIFRVFRTYGYYMSSDVFKIFKGDDGKFKESLIEDVKGMLSFYEAVH FGTTTDHILDEALSFTLNHLESLATGRRASPPHISKLIQNALHIPQHRNIQALVA REYISFYEHEEDHDETLLKLAKLNFKFLQLHYFQELKTITMWWTKLDHTSNLP PNFRERTVETWFAALMMYFEPQFSLGRIMSAKLYLVITFLDDACDTYGSISEV ESLADCLERWDPDYMENLQGHMKTAFKFVMYLFKEYEEILRSQGRSFVLEK MIEEFKIIARKNLELVKWARGGHVPSFDEYIESGGAEIGTYATIACSIMGLGEIG KKEAFEWLISRPKLVRILGAKTRLMDDIADFEEDMEKGYTANALNYYMNEHG VTKEEASRELEKMNGDMNKIVNEECLKITTMPRRILMQSVNYARSLDVLYTA DDVYNHREGKLKEYMRLLLVDPILLGTGENLYFQGSGGGGSDYKDDDDKGT G | Sesquiterpene synthase |
| 36 | MVPESQTTFKYESLAFTKLSHCQWTDYFLSVPIDESELDVITREIDILKPEVMEL LSSQGDDETSKRKVLLIQLLLSLGLAFHFENEIKNILEHAFRKIDDITGDEKDLS TISIMFRVFRTYGHNLPSSVFKRFTGDDGKFQQSLTEDAKGILSLYEAAHLGTT TDYILDEALKFTSSHLKSLLAGGTCRPHILRLIRNTLYLPQRWNMEAVIAREYI SFYEQEEDHDKMLLRLAKLNFKLLQLHYIKELKSFIKWWMELGLTSKWPSQF RERIVEAWLAGLMMYFEPQFSGGRVIAAKFNYLLTILDDACDHYFSIHELTRL VACVERWSPDGIDTLEDISRSVFKLMLDVFDDIGKGVRSEGSSYHLKEMLEEL NTLVRANLDLVKWARGIQTAGKEAYEWVRSRPRLIKSLAAKGRLMDDITDFD SDMSNGFAANAINYYMKQFVVTKEEAILECQRMIVDINKTINEELLKTTSVPG RVLKQALNFGRLLELLYTKSDDIYNCSEGKLKEYIVTLLIDPIRLGTGENLYFQ GSGGGGSDYKDDDDKGTG | Sesquiterpene synthase |
| 37 | MVPESQTKFDYESLAFTKLSHSQWTDYFLSVPIDDSELDAITREIDIIKPEVRKL LSSKGDDETSKRKVLLIQSLLSLGLAFHFENEIKDILEDAFRRIDDITGDENDLS TISIMFRVFRTYGHNLPSSVFKRFTGDDGKFERSLTEDAKGILSLYEAAHLGTT TDYILDEALEFTSSHLKSLLVGGMCRPHILRLIRNTLYLPQRWNMEAVIAREYI SFYEQEEDHDKMLLRLAKLNFKLLQLHYIKELKTFIKWWMELGLTSKWPSQF RERIVEAWLAGLMMYFEPQFSGGRVIAAKFNYLLTILDDACDHYFSIPELTRL VDCVERWNHDGIHTLEDISRIIFKLALDVFDDIGRGVRSKGCSYYLKEMLEEL KILVRANLDLVKWARGNQLPSFEEHVEVGGIALTTYATLMYSFVGMGEAVG KEAYEWVRSRPRLIKSLAAKGRLMDDITDFEVKIINLFFDLLLFVFGTGENLYF QGSGGGGSDYKDDDDKGTG | Sesquiterpene synthase |
| 38 | MVPAAFTANAVDMRPPVITIHPRSKDIFSQFSLDDKLQKQYAQGIEALKEEAR SMLMAAKSAKVMILIDTLERLGLGYHFEKEIEEKLEAIYKKEDGDDYDLFTTA LRFRLLRQHQRRVPCSVFDKFMNKEGKFEEEPLISDVEGLLSLYDAAYLQIHG EHILQEALIFTTHHLTRIEPQLDDHSPLKLKLNRALEFPFYREIPIIYAHFYISVYE RDDSRDEVLLKMAKLSYNFLQNLYKKELSQLSRWWNKLELIPNLPYIRDSVA GAYLWAVALYFEPQYSDVRMAIAKLIQIAAAVDDTYDNYATIREAQLLTEAL ERLNVHEIDTLPDYMKIVYRFVMSWSEDFERDATIKEQMLATPYFKAEMKKL GRAYNQELKWVMERQLPSFEEYMKNSEITSGVYIMFTVISPYLNSATQKNIDW LLSQPRLASSTAIVMRCCNDLGSNQRESKGGEVMTSLDCYMKQHGASKQETI SKFKLIIEDEWKNLNEEWAATTCLPKVMVEIFRNYARIAGFCYKNNGDAYTSP KIVQQCFDALFVNPLRIGTGENLYFQGSGGGGSDYKDDDDKGTG | Curcumene synthase |
| 39 | MVPEFRVHLQADNEQKIFQNQMKPEPEASYLINQRRSANYKPNIWKNDFLDQ SLISKYDGDEYRKLSEKLIEEVKIYISAETMDLVAKLELIDSVRKLGLANLFEKE IKEALDSIAAIESDNLGTRDDLYGTALHFKILRQHGYKVSQDIFGRFMDEKGTL ENHHFAHLKGMLELFEASNLGFEGEDILDEAKASLTLALRDSGHICYPDSNLS RDVVHSLELPSHRRVQWFDVKWQINAYEKDICRVNATLLELAKLNFNVVQA QLQKNLREASRWWANLGFADNLKFARDRLVECFSCAVGVAFEPEHSSFRICL TKVINLVLIIDDVYDIYGSEEELKHFTNAVDRWDSRETEQLPECMKMCFQVLY NTTCEIAREIEEENGWNQVLPQLTKVWADFCKALLVEAEWYNKSHIPTLEEYL RNGCISSSVSVLLVHSFFSITHEGTKEMADFLHKNEDLLYNISLIVRLNNDLGTS AAEQERGDSPSSIVCYMREVNASEETARKNIKGMIDNAWKKVNGKCFTTNQV PFLSSFMNNATNMARVAHSLYKDGDGFGDQEKGPRTHILSLLFQPLVNGTGE NLYFQGSGGGGSDYKDDDDKGTG | Farnesene synthase |
| 40 | MVPSSNVSAIPNSFELIRRSAQFQASVWGDYFLSYHSLPPEKGNKVMEKQTEE LKEEIKMELVSTTKDEPEKLRLIDLIQRLGVCYHFENEINNILQQLHHITITSEKN GDDNPYNMTLCFRLLRQQGYNVSSEPFDRFRGKWESSYDNNVEELLSLYEAS QLRMQGEEALDEAFCFATAQLEAIVQDPTTDPMVAAEIRQALKWPMYKNLPR LKARHHIGLYSEKPWRNESLLNFAKMDFNKLQNLHQTEIAYISKWWDDYGFA EKLSFARNRIVEGYFFALGIFFEPQLLTARLIMTKVIAIGSMLDDIYDVYGTFEE LKLLTLALERWDKSETKQLPNYMKMYYEALLDVFEEIEQEMSQKETETTPYCI HHMKEATKELGRVFLVEATWCKEGYTPKVEEYLDIALISFGHKLLMVTALLG MGSHMATQQIVQWITSMPNILKASAVICRLMNDIVSHKFEQERGHVASAIECY MEQNHLSEYEALIALRKQIDDLWKDMVENYCAVITEDEVPRGVLMRVLNLTR LFNVIYKDGDGYTQSHGSTKAHIKSLLVDSVPLGTGENLYFQGSGGGGSDYK DDDDKGTG | Farnesene synthase |
| 41 | MVPKDMSIPLLAAVSSSTEETVRPIADFHPTLWGNHFLKSAADVETIDAATQE QHAALKQEVRRMITTTANKLAQKLHMIDAVQRLGVAYHFEKEIEDELGKVSH DLDSDDLYVVSLRFRLFRQQGVKISCDVFDKFKDDEGKFKESLINDIRGMLSL YEAAYLAIRGEDILDEAIVFTTTHLKSVISISDHSHANSNLAEQIRHSLQIPLRKA AARLEARYFLDIYSRDDLHDETLLKFAKLDFNILQAAHQKEASIMTRWWNDL GFPKKVPYARDRIIETYIWMLLGVSYEPNLAFGRIFASKVVCMITTIDDTFDAY GTFEELTLFTEAVTRWDIGLIDTLPEYMKFIVKALLDIYREAEEELAKEGRSYGI PYAKQMMQELIILYFTEAKWLYKGYVPTFDEYKSVALRSIGLRTLAVASFVDL GDFIATKDNFECILKNAKSLKATETIGRLMDDIAGYKFEQKRGHNPSAVECYK NQHGVSEEEAVKELLLEVANSWKDINEELLNPTTVPLPMLQRLLYFARSGHFI YDDGHDRYTHSLMMKRQVALLLTEPLAIGTGENLYFQGSGGGGSDYKDDDD KGTG | Farnesene synthase |
| 42 | MVPDLAVEIAMDLAVDDVERRVGDYHSNLWDDDFIQSLSTPYGASSYRERAE RLVGEVKEMFTSISIEDGELTSDLLQRLWMVDNVERLGISRHFENEIKAAIDYV YSYWSDKGIVRGRDSAVPDLNSIALGFRTLRLHGYTVSSDVFKVFQDRKGEFA CSAIPTEGDIKGVLNLLRASYIAFPGEKVMEKAQTFAATYLKEALQKIQVSSLS REIEYVLEYGWLTNFPRLEARNYIDVFGEEICPYFKKPCIMVDKLLELAKLEFN LFHSLQQTELKHVSRWWKDSGFSQLTFTRHRHVEFYTLASCIAIEPKHSAFRL GFAKVCYLGIVLDDIYDTFGKMKELELFTAAIKRWDPSTTECLPEYMKGVYM AFYNCVNELALQAEKTQGRDMLNYARKAWEALFDAFLEEAKWISSGYLPTF EEYLENGKVSFGYRAATLQPILTLDIPLPLHILQQIDFPSRFNDLASSILRLRGDI CGYQAERSRGEEASSISCYMKDNPGSTEEDALSHINAMISDNINELNWELLKP NSNVPISSKKHAFDILRAFYHLYKYRDGFSIAKIETKNLVMRTVLEPVPMGTGE NLYFQGSGGGGSDYKDDDDKGTG | Farnesene synthase |
| 43 | MVPTSVSVESGTVSCLSSNNLIRRTANPHPNIWGYDFVHSLKSPYTHDSSYRER AETLISEIKVMLGGGELMMTPSAYDTAWVARVPSIDGSACPQFPQTVEWILKN QLKDGSWGTESHFLLSDRLLATLSCVLALLKWKVADVQVEQGIEFIKRNLQAI KDERDQDSLVTDFEIIFPSLLKEAQSLNLGLPYDLPYIRLLQTKRQERLANLSM DKIHGGTLLSSLEGIQDIVEWETIMDVQSQDGSFLSSPASTACVFMHTGDMKC LDFLNNVLTKFGSSVPCLYPVDLLERLLIVDNVERLGIDRHFEKEIKEALDYVY RHWNDRGIGWGRLSPIADLETTALGFRLLRLHRYNVSPVVLDNFKDADGEFF CSTGQFNKDVASMLSLYRASQLAFPEESILDEAKSFSTQYLREALEKSETFSSW NHRQSLSEEIKYALKTSWHASVPRVEAKRYCQVYRQDYAHLAKSVYKLPKV NNEKILELAKLDFNIIQSIHQKEMKNVTSWFRDSGLPLFTFARERPLEFYFLIAG GTYEPQYAKCRFLFTKVACLQTVLDDMYDTYGTPSELKLFTEAVRRWDLSFT ENLPDYMKLCYKIYYDIVHEVAWEVEKEQGRELVSFFRKGWEDYLLGYYEE AEWLAAEYVPTLDEYIKNGITSIGQRILLLSGVLIMEGQLLSQEALEKVDYPGR RVLTELNSLISRLADDTKTYKAEKARGELASSIECYMKDHPGCQEEEALNHIY GILEPAVKELTREFLKADHVPFPCKKMLFDETRVTMVIFKDGDGFGISKLEVK DHIKECLIEPLPLGTGENLYFQGSGGGGSDYKDDDDKGTG | Bisabolene synthase |
| 44 | MVPGSEVNRPLADFPANIWEDPLTSFSKSDLGTETFKEKHSTLKEAVKEAFMS SKANPIENIKFIDALCRLGVSYHFEKDIVEQLDKSFDCLDFPQMVRQEGCDLYT VGIIFQVFRQFGFKLSADVFEKFKDENGKFKGHLVTDAYGMLSLYEAAQWGT HGEDIIDEALAFSRSHLEEISSRSSPHLAIRIKNALKHPYHKGISRIETRQYISYYE EEESCDPTLLEFAKIDFNLLQILHREELACVTRWHHEMEFKSKVTYTRHRITEA YLWSLGTYFEPQYSQARVITTMALILFTALDDMYDAYGTMEELELFTDAMDE WLPVVPDEIPIPDSMKFIYNVTVEFYDKLDEELEKEGRSGCGFHLKKSLQKTA NGYMQEAKWLKKDYIATFDEYKENAILSSGYYALIAMTFVRMTDVAKLDAF EWLSSHPKIRVASEIISRFTDDISSYEFEHKREHVATGIDCYMQQFGVSKERAV EVMGNIVSDAWKDLNQELMRPHVFPFPLLMRVLNLSRVIDVFYRYQDAYTNP KLLKEHIVSLLIETIPIGTGENLYFQGSGGGSDYKDDDDKGTG | Sesquiterpene synthase |
| 45 | MVPEAIRVFGLKLGSKLSIHSQTNAFPAFKLSRFPLTSFPGKHAHLDPLKATTH PLAFDGEENNREFKNLGPSEWGHQFLSAHVDLSEMDALEREIEALKPKVRDM LISSESSKKKILFLYLLVSLGLAYHFEDEIKESLEDGLQKIEEMMASEDDLRFKG DNGKFKECLAKDAKGILSLYEAAHMGTTTDYILDEALSFTLTYMESLAASGTC KINLSRRIRKALDQPQHKNMEIIVAMKYIQFYEEEEDCDKTLLKFAKLNFKFLQ LHYLQELKILSKWYKDQDFKSKLPPYFRDRLVECHFASLTCFEPKYARARIFLS KIFTVQIFIDDTCDRYASLGEVESLADTIERWDPDDHAMDGLPDYLKSVVKFV FNTFQEFERKCKRSLRINLQVAKWVKAGHLPSFDEYLDVAGLELAISFTFAGIL MGMENVCKPEAYEWLKSRDKLVRGVITKVRLLNDIFGYEDDMRRGYVTNSI NCYKKQYGVTEEEAIRKLHQIVADGEKMMNEEFLKPINVPYQVPKVVILDTL RAANVSYEKDDEFTRPGEHLKNCITSIYFDLGTGENLYFQGSGGGGSDYKDD DDKGTG | Sesquiterpene synthase |
| 46 | MVPTTTLSSNLNSQFMQVYETLKSELIHDPLFEFDDDSRQWVERMIDYTVPGG KMVRGYSVVDSYQLLKGEELTEEEAFLACALGWCTEWFQAFILLHDDMMDG SHTRRGQPCWFRLPEVGAVAINDGVLLRNHVHRILKKHFQGKAYYVHLVDLF NETEFQTISGQMIDLITTLVGEKDLSKYSLSIHRRIVQYKTAYYSFYLPVACALL MFGEDLDKHVEVKNVLVEMGTYFQVQDDYLDCFGAPEVIGKIGTDIEDFKCS WLVVKALELANEEQKKTLHENYGKKDPASVAKVKEVYHTLNLQAVFEDYE ATSYKKLITSIENHPSKAVQAVLKSFLGKIYKRQKGTGENLYFQGSGGGGSDY KDDDDKGTG | GPP Chimera synthase |
| 47 | MVPSQPYWAAIEADIERYLKKSITIRPPETVFGPMHHLTFAAPATAASTLCLAA CELVGGDRSQAMAAAAIHLVHAAAYVHEHLPLTDGSRPVSKPAIQHKYGPN VELLTGDGIVPFGFELLAGSVDPARTDDPDRILRVIIEISRAGGPEGMISGLHRE EEIVDGNTSLDFIEYVCKKKYGEMHACGAACGAILGGAAEEEIQKLRNFGLYQ GTLRGMMEMKNSHQLIDENIIGKLKELALEELGGFHGKNAELMSSLVAEPSLY AASSNNLGIEGRFDFDGYMLRKAKSVNKALEAAVQMKEPLKIHESMRYSLLA GGKRVRPMLCIAACELVGGDESTAMPAACAVEMIHTMSLMHDDLPCMDND DLRRGKPTNHMAFGESVAVLAGDALLSFAFEHVAAATKGAPPERIVRVLGEL AVSIGSEGLVAGQVVDVCSEGMAEVGLDHLEFIHHHKTAALLQGSVVLGAIL GGGKEEEVAKLRKFANCIGLLFQVVDDILDVTKSSKELGKTAGKDLVADKTT YPKLIGVEKSKEFADRLNREAQEQLLHFHPHRAAPLIALANYIAYRDNGTGEN LYFQGSGGGGSDYKDDDDKGTG | GPPS-LSU+SS U fusion |
| 48 | MVPVTAARATPKLSNRKLRVAVIGGGPAGGAAAETLAQGGIETILIERKMDN CKPCGGAIPLCMVGEFNLPLDIIDRRVTKMKMISPSNIAVDIGRTLKEHEYIGM VRREVLDAYLRERAEKSGATVINGLFLKMDHPENWDSPYTLHYTEYDGKTG ATGTKKTMEVDAVIGADGANSRVAKSIDAGDYDYAIAFQERIRIPDEKMTYY EDLAEMYVGDDVSPDFYGWVFPKCDHVAVGTGTVTHKGDIKKFQLATRNRA KDKILGGKIIRVEAHPIPEHPRPRRLSKRVALVGDAAGYVTKCSGEGIYFAAKS GRMCAEAIVEGSQNGKKMIDEGDLRKYLEKWDKTYLPTYRVLDVLQKVFYR SNPAREAFVEMCNDEYVQKMTFDSYLYKRVAPGSPLEDIKLAVNTIGSLVRA NALRREIEKLSVGTGENLYFQGSGGGGSDYKDDDDKGTG | Geranyl geranyl reductase |
| 49 | MVPVAVIGGGPSGACAAETLAKGGVETFLLERKLDNCKPCGGAIPLCMVEEF DLPMEIIDRRVTKMKMISPSNREVDVGKTLSETEWIGMCRREVFDDYLRNRA QKLGANIVNGLFMRSEQQSAEGPFTIHYNSYEDGSKMGKPATLEVDMIIGADG ANSRIAKEIDAGEYDYAIAFQERIRIPDDKMKYYENLAEMYVGDDVSPDFYG WVFPKYDHVAVGTGTVVNKTAIKQYQQATRDRSKVKTEGGKIIRVEAHPIPE HPRPRRCKGRVALVGDAAGYVTKCSGEGIYFAAKSGRMAAEAIVEGSANGT KMCGEDAIRVYLDKWDRKYWTTYKVLDILQKVFYRSNPAREAFVELCEDSY VQKMTFDSYLYKTVVPGNPLDDVKLLVRTVSSILRSNALRSVNSKSVNVSFGS KANEERVMAAGTGENLYFQGSGGGGSDYKDDDDKGTG | Geranyl-geranyl reductase |
| 50 | MVPAMAVPLDVVITYPSSGAAAYPVLVMYNGFQAKAPWYRGIVDHVSSWG YTVVQYTNGGLFPIVVDRVELTYLEPLLTWLETQSADAKSPLYGRADVSRLG TMGHSRGGKLAALQFAGRTDVSGCVLFDPVDGSPMTPESADYPSATKALAA AGRSAGLVGAAITGSCNPVGQNYPKFWGALAPGSWQMVLSQAGHMQFART GNPFLDWSLDRLCGRGTMMSSDVITYSAAFTVAWFEGIFRPAQSQMGISNFKT WANTQVAARSITFDIKPMQSPQGTGENLYFQGSGGGGSDYKDDDDKGTG | Chloroph yllido-hydrolase |
| 51 | MVPAPPKPVRITCPTVAGTYPVVLFFHGFYLRNYFYSDVLNHIASHGYILVAP QLCKLLPPGGQVEVDDAGSVINWASENLKAHLPTSVNANGKYTSLVGHSRGG KTAFAVALGHAATLDPSITFSALIGIDPVAGTNKYIRTDPHILTYKPESFELDIPV AVVGTGLGPKWNNVMPPCAPTDLNHEEFYKECKATKAHFVAADYGHMDML DDDLPGFVGFMAGCMCKNGQRKKSEMRSFVGGIVVAFLKYSLWGEKAEIRLI VKDPSVSPAKLDPSPELEEASGIFVGTGENLYFQGSGGGGSDYKDDDDKGTG | Chloro-phyllido-hydrolase |
| 52 | MVPATPVEEGDYPVVMLLHGYLLYNSFYSQLMLHVSSHGFILIAPQLYSIAGP DTMDEIKSTAEIMDWLSVGLNHFLPAQVTPNLSKFALSGHSRGGKTAFAVAL KKFGYSSNLKISTLIGIDPVDGTGKGKQTPPPVLAYLPNSFDLDKTPILVIGSGL GETARNPLFPPCAPPGVNHREFFRECQGPAWHFVAKDYGHLDMLDDDTKGIR GKSSYCLCKNGEERRPMRRFVGGLVVSFLKAYLEGDDRELVKIKDGCHEDVP VEIQEFEVIMGTGENLYFQGSGGGGSDYKDDDDKGTG | Chloro-phyllido-hydrolase |
| 53 | MVPSHKKKNVIFFVTDGMGPASLSMARSFNQHVNDLPIDDILTLDEHFIGSSRT RSSDSLVTDSAAGATAFACALKSYNGAIGVDPHHRPCGTVLEAAKLAGYLTG LVVTTRITDATPASFSSHVDYRWQEDLIATHQLGEYPLGRVVDLLMGGGRSH FYPQGEKASPYGHHGARKDGRDLIDEAQSNGWQYVGDRKNFDSLLKSHGEN VTLPFLGLFADNDIPFEIDRDEKEYPSLKEQVKVALGALEKASNEDKDSNGFFL MVEGSRIDHAGHQNDPASQVREVLAFDEAFQYVLEFAENSDTETVLVSTSDH ETGGLVTSRQVTASYPQYVWYPQVLANATHSGEFLKRKLVDFVHEHKGASS KIENFIKHEILEKDLGIYDYTDSDLETLIHLDDNANAIQDKLNDMVSFRAQIGW TTHGHSAVDVNIYAYANKKATWSYVLNNLQGNHENTEVGQFLENFLELNLN EVTDLIRDTKHTSDFDATEIASEVQHYDEYYHELTNGTGENLYFQGSGGGGSD YKDDDDKGTG | Phosphatase |
| 54 | MVPHKFTGVNAKFQQPALRNLSPVVVEREREEFVGFFPQIVRDLTEDGIGHPE VGDAVARLKEVLQYNAPGGKCNRGLTVVAAYRELSGPGQKDAESLRCALAV GWCIELFQAFFLVWDDIMDQSLTRRGQLCWYKKEGVGLDAINDSFLLESSVY RVLKKYCRQRPYYVHLLELFLQTAYQTELGQMLDLITAPVSKVDLSHFSEERY KAIVKYKTAFYSFYLPVAAAMYMVGIDSKEEHENAKAILLEMGEYFQIQDDY LDCFGDPALTGKVGTDIQDNKCSWLVVQCLQRVTPEQRQLLEDNYGRKEPEK VAKVKELYEAVGMRAAFQQYEESSYRRLQELIEKHSNRLPKEIFLGLAQKIYK RQKGTGENLYFQGSGGGGSDYKDDDDKGTG | FPP A118W |

One or more codons of an encoding polynucleotide can be biased to reflect chloroplast and/or nuclear codon usage. Most amino acids are encoded by two or more different (degenerate) codons, and it is well recognized that various organisms utilize certain codons in preference to others. Such preferential codon usage, which also is utilized in chloroplasts, is referred to herein as "chloroplast codon usage". The codon bias of *Chlamydomonas reinhardtii* has been reported. *See* U.S. Application 2004/0014174. Examples of nucleic acids encoding isoprenoid biosynthetic enzymes which are biased for expression in C. reinhardtii are provided in Tables 5-8. Percent identity to the native sequence (in the organism from which the sequence was isolated) may be about 50%, about 60%, about 70%, about 80%, about 90% or higher. Some vectors of the present invention comprise one or more of the nucleic provided in Table 5 and/or nucleic acids with about 70% identity thereto.

One example of an algorithm that is suitable for determining percent sequence identity or sequence similarity between nucleic acid or polypeptide sequences is the BLAST algorithm, which is described, e.g., in Altschul et al., J. Mol. Biol. 215:403-410 (1990). Software for performing BLAST analysis is publicly available through the National Center for Biotechnology Information.The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915). In addition to calculating percent sequence identity, the BLAST algorithm also can perform a statistical analysis of the similarity between two sequences (*see*, *e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

The term "biased," when used in reference to a codon, means that the sequence of a codon in a polynucleotide has been changed such that the codon is one that is used preferentially in the target which the bias is for, e.g., alga cells, chloroplasts. A polynucleotide that is biased for chloroplast codon usage can be synthesized de novo, or can be genetically modified using routine recombinant DNA techniques, for example, by a site directed mutagenesis method, to change one or more codons such that they are biased for chloroplast codon usage. Chloroplast codon bias can be variously skewed in different plants, including, for example, in alga chloroplasts as compared to tobacco. Generally, the chloroplast codon bias selected reflects chloroplast codon usage of the plant which is being transformed with the nucleic acids of the present invention. For example, where C. *reinhardtii* is the host, the chloroplast codon usage is biased to reflect alga chloroplast codon usage (about 74.6% AT bias in the third codon position).

One method of the invention can be performed using a polynucleotide that encodes a first polypeptide and at least a second polypeptide. As such, the polynucleotide can encode, for example, a first polypeptide and a second polypeptide; a first polypeptide, a second polypeptide, and a third polypeptide; etc. Furthermore, any or all of the encoded polypeptides can be the same or different. The polypeptides expressed in chloroplasts of the microalga *C. reinhardtii* may be assembled to form functional polypeptides and protein complexes. As such, a method of the invention provides a means to produce functional protein complexes, including, for example, dimers, trimers, and tetramers, wherein the subunits of the complexes can be the same or different (e.g., homodimers or heterodimers, respectively).

The term "recombinant nucleic acid molecule" is used herein to refer to a polynucleotide that is manipulated by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise an encoding nucleotide sequence and a regulatory element. A recombinant nucleic acid molecule also can be based on, but manipulated so as to be different, from a naturally occurring polynucleotide, (e.g. biased for chloroplast codon usage, insertion of a restriction enzyme site, insertion of a promoter, insertion of an origin of replication). A recombinant nucleic acid molecule may further contain a peptide tag (e.g., His-6 tag), which can facilitate identification of expression of the polypeptide in a cell. Additional tags include, for example: a FLAG epitope, a c-myc epitope; biotin; and glutathione S-transferase. Such tags can be detected by any method known in the art (e.g., anti-tag antibodies, streptavidin). Such tags may also be used to isolate the operatively linked polypeptide(s), for example by affinity chromatography.

A polynucleotide comprising naturally occurring nucleotides and phosphodiester bonds can be chemically synthesized or can be produced using recombinant DNA methods, using an appropriate polynucleotide as a template. In comparison, a polynucleotide comprising nucleotide analogs or covalent bonds other than phosphodiester bonds generally are chemically synthesized, although an enzyme such as T7 polymerase can incorporate certain types of nucleotide analogs into a polynucleotide and, therefore, can be used to produce such a polynucleotide recombinantly from an appropriate template (Jellinek et al., supra, 1995). Polynucleotides useful for practicing a method of the present invention may be isolated from any organism.

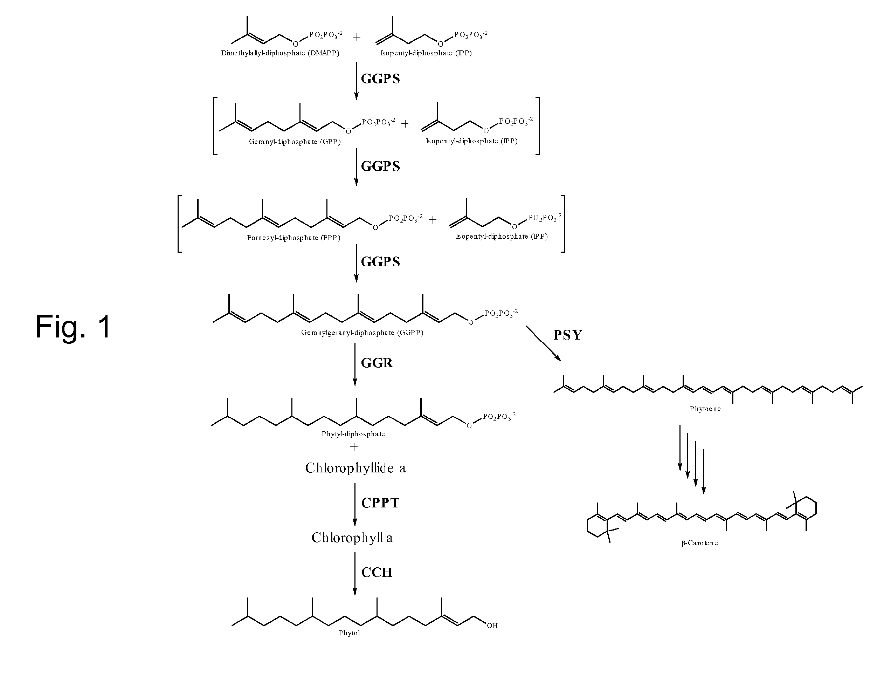

The invention may take advantage of naturally occurring product production pathways in an NVPO. An example of such a pathway (for the production of phytol and β-carotene) is shown in **FIG. 1****.** One of skill in the art will recognize that this isoprenoid production pathway is provided merely by way of example to further illustrate one embodiment.

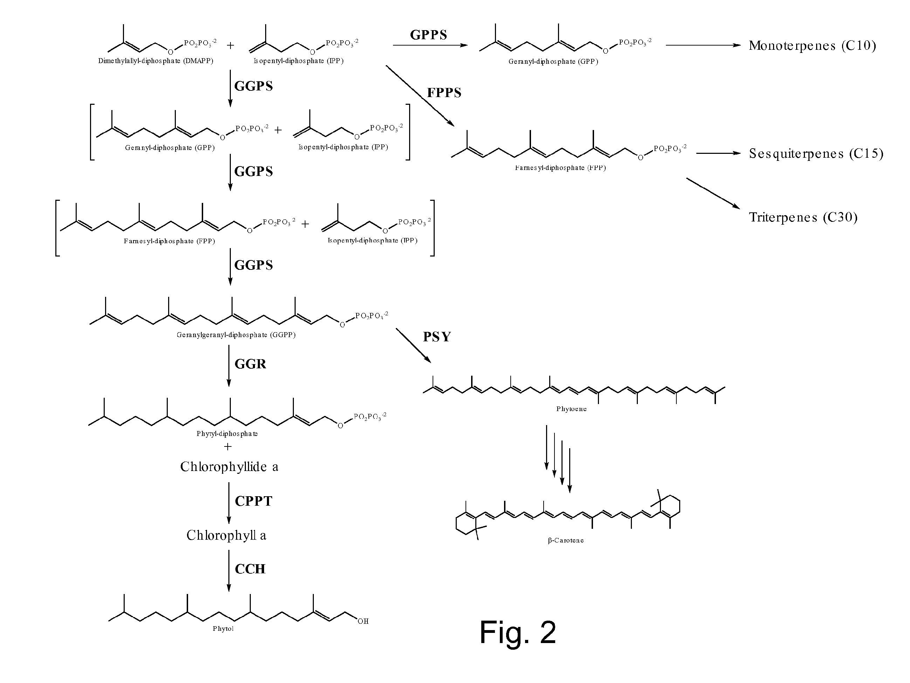

One aspect of the present invention is to modify the phytol/β-carotene pathway to produce non-naturally occurring products and/or increase the production of naturally occurring products. **FIG. 2** illustrates one potential for modification of the pathway illustrated in **FIG. 1****.** By inserting an exogenous geranyl-diphosphate synthase (GPPS) and/or farsenyl-diphosphate synthase (FPPS), the production of GPP and FPP can be increased. For example, a host organism (e.g., C. *reinhardtii, D. salina)* can be transformed with any of the sequences encoding GPP or FPP synthases listed in Tables 5 or 7 (e.g., SEQ ID NOs. 82, 87-94, 118, and/or 180-191). Furthermore, as exemplified in the examples below, introduction of a GPPS or FPPS may be accompanied by the insertion of an exogenous gene encoding an enzyme (e.g., limonene synthase, zingiberene synthase, chlorophyllohydrolase) which leads to the production of isoprenoids of interest (e.g., monoterpenes, sesquiterpenes, and triterpenes) which are not naturally produced by the NVPO. A non-limiting list of enzymes which may be used to transform NVPOs - alone, or in combination - is provided in Tables 5-8.

Insertion of genes encoding enzymes of the present invention may lead to increased production of a naturally occurring isoprenoid (e.g., GPP, FPP, phytol, phytoene, β-carotene). For example, production of naturally occurring isoprenoids (e.g., GPP, FPP, phytoene) may be increased by: 1) introducing extra copies of an endogenous or exogenous gene encoding a synthetic enzyme which produces the isoprenoid; 2) introducing a regulatory element (e.g., constitutive promoter, inducible promoter) to control expression of a naturally occurring synthetic enzyme; and/or 3) introduction of an exogenous nucleic acid which increases production of a naturally occurring isoprenoid through an indirect route (e.g., an exogenous GPPS may increase the intracellular concentration of GPP, providing more substrate for a phytol/chlorophyll synthesis pathway).

Thus, production of certain naturally occurring isoprenoids may be increased. For purposes of illustration only, the isoprenoid, phytol, is naturally produced by a number of NVPOs, including *C*. *reinhardtii.* Generally, the amount of phytol in wild type strains of *C*. *reinhardtii* is less than 1% by weight. The present disclosure provides for several mechanisms which may increase production of phytol. In one example, a regulatory element which drives constitutive or inducible expression of an endogenous gene (e.g., GPP synthase) may be introduced into a genome of the organism to express the gene at a higher level than that which is achieved by the naturally occuring regulatory elements. Alternately, one or more exogenous isoprenoid synthases may be introduced into a genome of the organism. Such synthases may be homologous or non-homologous to the target NVPO (e.g. a GPP synthase from a related organism, an FPP synthase). Alternately, exogenous enzymes (e.g., phosphatases, pyrophosphatases) may be introduced into the target NVPO. Such enzymes may act on naturally occuring substrates (e.g., GGPP, phytyl-diphosphate) or may act on substrates produced by other exogenous genes introduced into the host NVPO. In some instances, exogenous enzymes may produce the isoprenoid of interest (e.g., phytol) or may produce a precursor for an enzyme which then acts to produce the isoprenoid of interest. In still another approach, an enzyme may be introduced or upregulated which causes the degradation of a product produced by the host NVPO - either naturally or as the result of an introduced gene - thereby producing the isoprenoid of interest. For example, a chlorophyllidohydrolase may be introduced into the host cell to promote degradation of chlorophyll into phytol.

Utilizing such approaches, a modified NVPO may comprise about 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, or more. Where desired, phytol can be collected from modified NVPOs and concentrated to about 5%, 10%, 15%, 20%, 25%, 30%, 35%, 40%, 45%, 50%, 55%, 60%, 65%, 70%, 76%, 80%, 85%, 90%, 95%, or higher. In some instances, compositions comprising phytol collected from an NVPO of the present invention may also comprise portions of the cells of the NVPO (e.g., cell wall material, cell membrane material, proteins, carbohydrates, nucleic acids, etc.).

Pathways utilized for the present invention may involve enzymes present in the cytosol, in a plastid (e.g., chloroplast), or both. Exogenous nucleic acids encoding the enzymes of embodiments of the invention may be introduced into a host cell, such that the enzyme encoded is active in the cytosol or in a plastid, or both. In some embodiments, a naturally occurring enzyme which is present in one intracellular compartment (e.g., in the cytosol) may be expressed in a different intracellular locale (e.g., in the chloroplast), or in both the naturally occurring and non-naturally occurring locales following transformation of the host cell.

To illustrate this concept, and merely by way of example, a non-vascular photosynthetic microalga species can be genetically engineered to produce an isoprenoid, such as limonene (a molecule of high value in the specialty chemical and petrochemical industries). Limonene is a monoterpene that is a pure hydrocarbon, only composed of hydrogen and carbon atoms. Limonene is not naturally produced in the species, *Chlamydomonas rheinhardii.* Production of limonene in these microalgae can be achieved by engineering the microalgae to express the heterologous enzyme limonene synthase in the chloroplast. Limonene synthase can convert the terpene precursor geranyl pyrophosphate into limonene. Unlike limonene, geranyl pyrophosphate is naturally present in the chloroplast of microalgae. The expression of the limonene synthase can be accomplished by inserting the heterologous gene encoding limonene synthase into the chloroplast genome of the microalgae. The modified strain of microalgae is then made homoplasmic to ensure that the limonene gene will be stably maintained in the chloroplast genome of all descendents. A microalga is homoplasmic for a gene when the inserted gene is present in all copies of the chloroplast genome. It is apparent to one of skill in the art that a chloroplast may contain multiple copies of its genome, and therefore, the term "homoplasmic" or "homoplasmy" refers to the state where all copies of a particular locus of interest are substantially identical. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein.

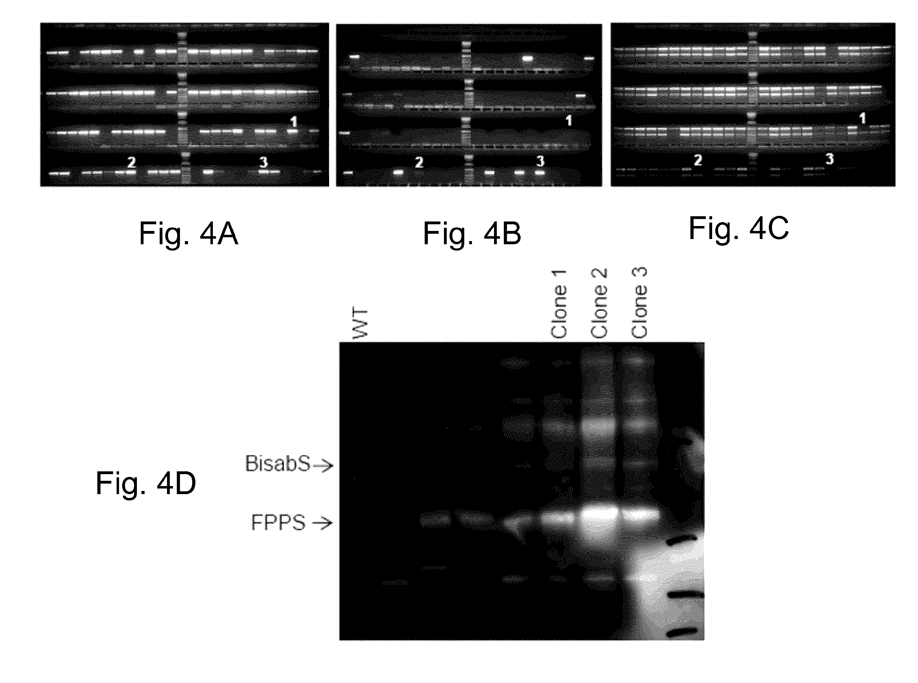

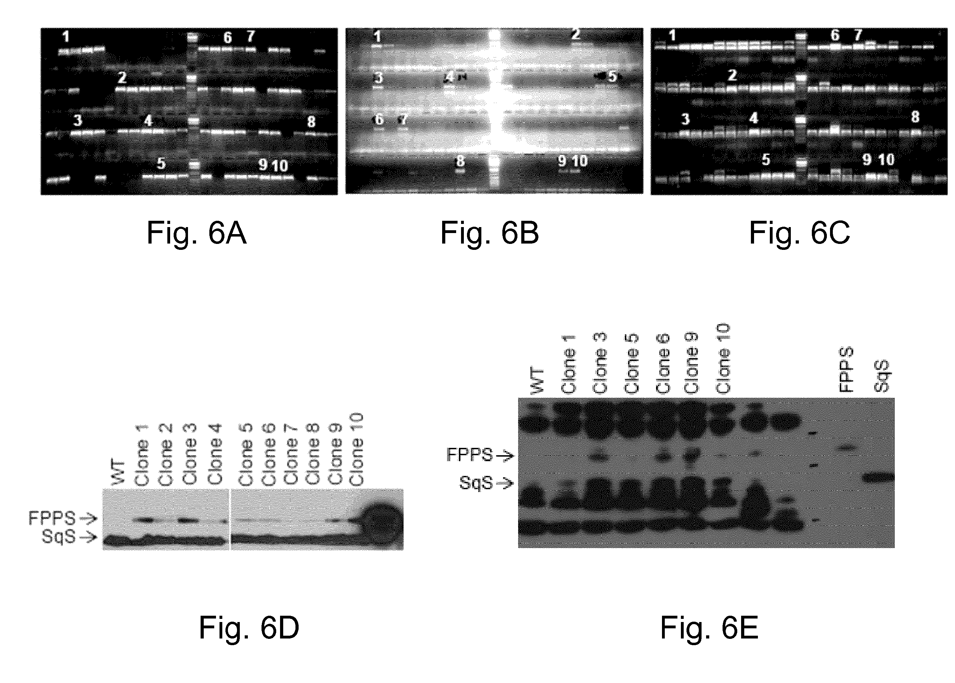

Briefly, the process of determining plasmic state of an organism of the present invention involves screening transformants for the presence of exogenous nucleic acids and the absence of wild-type nucleic acids at a given locus of interest. Such approaches are utilized in Examples 1 and 2 below (**Figs. 4A-C** and **6A-C****).**

Any of the vectors herein (e.g. including any of the expression vectors described above) can comprise a nucleotide sequence(s) encoding a protein or polypeptide that allows or improves secretion of a product produced by a transformed organism. The protein or polypeptide molecule affects, increases, upregulates, or modulates the secretion of a product from a host cell or organism.

An expression vector may comprise a sequence encoding a protein or polypeptide that allows or improves secretion of a product molecule and a nucleotide sequence encoding a synthase from an isoprenoid pathway.

### Selectable markers.

In some instances, a heterologous sequence in an expression vector encodes a dominant selectable marker for selection of the transformed organisms. Thus, organisms that have been subjected to transformation can be cultured in the presence of a compound (e.g., to which a dominant selectable marker confers resistance) that allows for the dominant selection of transformed host cells. Examples of compounds to which selectable markers confer resistance when expressed in the host organism include metabolic inhibitors (i.e., compounds that inhibit algal metabolism), such as antibiotics, fungicides, algicides, bactericides, and herbicides. Functionally, such compounds may be toxic to the cell or otherwise inhibit metabolism by functioning as protein or nucleic acid binding agents. For example, such compounds can inhibit translation, transcription, enzyme function, cell growth, cell division and/or microtubule formation. Dominant selectable markers suitable for use in the present invention can be selected from any known or subsequently identified selectable markers, including markers derived from fungal and bacterial sources (see e.g. US Pat. No. 5,661,017). In other embodiments, wild-type homologous genes that complement auxotrophic mutant strains may also be used as selectable marker systems, for example in some green algae (see e.g. Kindle et al., J. Cell Biol., 109:2589-2601 (1989) which discusses the transformation of a nitrate reductase deficient mutant of *Chlamydomonas reinhardtii* with a gene encoding nitrate reductase).

### Regulatory control sequences.

Any of the expression vectors herein can further comprise a regulatory control sequence. A regulatory control sequence may include for example, promoter(s), operator(s), repressor(s), enhancer(s), transcription termination sequence(s), sequence(s) that regulate translation, or other regulatory control sequence(s) that are compatible with the host cell and control the expression of the nucleic acid molecules of the present invention. In some cases, a regulatory control sequence includes transcription control sequence(s) that are able to control, modulate, or effect the initiation, elongation, and/or termination of transcription. For example, a regulatory control sequence can increase transcription and translation rate and/or efficiency of a gene or gene product in an organism, wherein expression of the gene or gene product is upregulated resulting (directly or indirectly) in the increased production, secretion, or both, of a product described herein. The regulatory control sequence may also result in the increase of production, secretion, or both, of a product by increasing the stability of a gene or gene product.

A regulatory control sequence can be autologous or heterologous, and if heterologous, may be homologous. The regulatory control sequence may encode one or more polypeptides which are enzymes that promote expression and production of products. For example, a heterologous regulatory control sequence may be derived from another species of the same genus of the organism (e.g., another algal species) and encode a synthase in an algae. In another example, an autologous regulatory control sequence can be derived from an organism in which an expression vector is to be expressed.

Depending on the application, regulatory control sequences can be used that effect inducible or constitutive expression. The algal regulatory control sequences can be used, and can be of nuclear, viral, extrachromosomal, mitochondrial, or chloroplastic origin.

Suitable regulatory control sequences include those naturally associated with the nucleotide sequence to be expressed (for example, an algal promoter operably linked with an algal-derived nucleotide sequence in nature). Suitable regulatory control sequences include regulatory control sequences not naturally associated with the nucleic acid molecule to be expressed (for example, an algal promoter of one species operatively linked to an nucleotide sequence of another organism or algal species). The latter regulatory control sequences can be a sequence that controls expression of another gene within the same species (i.e., autologous) or can be derived from a different organism or species (i.e., heterologous).

To determine whether a putative regulatory control sequence is suitable, the putative regulatory control sequence is linked to a nucleic acid molecule typically encodes a protein that produces an easily detectable signal. The construction may then be introduced into an alga or other organism by standard techniques and expression thereof is monitored. For example, if the nucleic acid molecule encodes a dominant selectable marker, the alga or organism to be used is tested for the ability to grow in the presence of a compound for which the marker provides resistance.

In some cases, a regulatory control sequence is a promoter, such as a promoter adapted for expression of a nucleotide sequence in a non-vascular, photosynthetic organism. For example, the promoter may be an algal promoter, for example as described in U.S. Publ. Appl. Nos. 2006/0234368 and 2004/0014174, and in Hallmann, Transgenic Plant J. 1:81-98(2007). The promoter may be a chloroplast specific promoter or a nuclear promoter. The promoter may an EF1-α gene promoter or a D promoter. In some embodiments, the synthase is operably linked to the EF1-α gene promoter. In other embodiments, the synthase is operably linked to the D promoter.

A regulatory control sequences herein can be found in a variety of locations, including for example, coding and non-coding regions, 5' untranslated regions (e.g., regions upstream from the coding region), and 3' untranslated regions (e.g., regions downstream from the coding region). Thus, in some instances an autologous or heterologous nucleotide sequence can include one or more 3' or 5' untranslated regions, one or more introns, or one or more exons.

For example, in some embodiments, a regulatory control sequence can comprise a Cyclotella cryptica acetyl-CoA carboxylase 5' untranslated regulatory control sequence or a Cyclotella cryptica acetyl-CoA carboxylase 3'-untranslated regulatory control sequence (U.S. Pat. No. 5,661,017).

A regulatory control sequence may also encode chimeric or fusion polypeptides, such as protein AB, or SAA, that promotes expression of heterologous nucleotide sequences and proteins. Other regulatory control sequences include autologous intron sequences that may promote translation of a heterologous sequence.

The regulatory control sequences used in any of the expression vectors herein may be inducible. Inducible regulatory control sequences, such as promoters, can be inducible by light, for example. Regulatory control sequences may also be autoregulatable. Examples of autoregulatable regulatory control sequences include those that are autoregulated by, for example, endogenous ATP levels or by the product produced by the organism. In some instances, the regulatory control sequences may be inducible by an exogenous agent. Other inducible elements are well known in the art and may be adapted for use in the present invention.

Various combinations of the regulatory control sequences described herein may be embodied by the present invention and combined with other features of the present invention. In some cases, an expression vector comprises one or more regulatory control sequences operatively linked to a nucleotide sequence encoding a polypeptide. Such sequences may, for example, upregulate secretion, production, or both, of a product described herein. In some cases, an expression vector comprises one or more regulatory control sequences operatively linked to a nucleotide sequence encoding a polypeptide that effects, for example, upregulates secretion, production, or both, of a product.

### Expression.

Chloroplasts are a productive organelle of photosynthetic organisms and a site of large of amounts of protein synthesis. Any of the expression vectors herein may be selectively adapted for chloroplast expression. A number of chloroplast promoters from higher plants have been described in Kung and Lin, Nucleic Acids Res. 13: 7543-7549 (1985). Gene products may be expressed from the expression vector in the chloroplast. Gene products encoded by expression vectors may also be targeted to the chloroplast by chloroplast targeting sequences. For example, targeting an expression vector or the gene product(s) encoded by an expression vector to the chloroplast may further enhance the effects provided by the regulatory control sequences and sequence(s) encoding a protein or peptide that allows or improves secretion of a fuel molecule.

Various combinations of the chloroplast targeting described herein may be embodied by the present invention and combined with other features of the present invention. For example, a nucleotide sequence encoding a terpene synthase may be operably linked to a nucleotide sequence encoding a chloroplast targeting sequence. A host cell may be transformed with an expression vector encoding limonene synthase targeted to the chloroplast, and thus, may produce more limonene synthase as compared to a host cell transformed with an expression vector encoding limonene synthase but not a chloroplast targeting sequence. The increased limonene synthase expression may produce more of the limonene in comparison to the host cell that produces less. Tables 5 and 7 provide examples of nucleic acids encoding isoprenoid producing enzymes useful in the present invention. Tables 6 and 8 provide these nucleic acid sequences with the addition of restriction enzyme sites. The sequences in Tables 5-8 are also codon-biased for expression in C. reinhardtii. Such sites, as will be readily apparent, can be used to integrate the nucleic acids into a vector.

In yet another example, an expression vector comprising a nucleotide sequence encoding an enzyme that produces a product (e.g. fuel product, fragrance product, insecticide product) not naturally produced by the organism by using precursors that are naturally produced by the organism as substrates, is targeted to the chloroplast. By targeting the enzyme to the chloroplast, production of the product may be increased in comparison to a host cell wherein the enzyme is expressed, but not targeted to the chloroplast. Without being bound by theory, this may be due to increased precursors being produced in the chloroplast and thus, more product may be produced by the enzyme encoded by the introduced nucleotide sequence.

### Products.

Examples of products contemplated herein include hydrocarbon products and hydrocarbon derivative products. A hydrocarbon product is one that consists of only hydrogen molecules and carbon molecules. A hydrocarbon derivative product is a hydrocarbon product with one or more heteroatoms, wherein the heteroatom is any atom that is not hydrogen or carbon. Examples of heteroatoms include, but not limited to, nitrogen, oxygen, sulfur, and phosphorus. Some products are hydrocarbon-rich, wherein as least 50%, 60%, 70%, 80%, 90%, or 95% of the product by weight is made up carbon and hydrogen.

Examples of hydrocarbon and hydrocarbon derivative products that can be produced using the compositions and methods herein include terpenes, and their derivatives, terpenoids. A terpene is a molecule made of isoprene (C5) units and is not necessarily a pure a hydrocarbon. Terpenes are typically derived from isoprene units. Isoprene units are five-carbon units (C5). Terpenes are hydrocarbons that can be modified (e.g. oxidized, methyl groups removed, etc.) or its carbon skeleton rearranged, to form derivatives of terpenes, such as isoprenoids.

Isoprenoids (also known as terpenoids) are derived from isoprene subunits but are modified, such as by the addition of heteroatoms such as oxygen, by carbon skeleton rearrangement, and by alkylation. Isoprenoids generally have a number of carbon atoms which is evenly divisible by five, but this is not a requirement as "irregular" terpenoids are known. Carotenoids, such as carotenes and xanthophylls, are an example of a isoprenoid as a useful product. A steroid is another example of a terpenoid. Examples of isoprenoids include, but are not limited to, hemiterpenes (C5), monoterpenes (C10), sesquiterpenes (C15), diterpenes (C20), triterpenes (C30), tetraterpenes (C40), and polyterpenes (Cₙ, wherein "n" is equal to or greater than 45). Other examples of isoprenoids include, but are not limited to, limonene, 1, 8-cineole, α-pinene, camphene, (+)-sabinene, myrcene, abietadiene, taxadiene, farnesyl pyrophosphate, amorphadiene, (E)-α-bisabolene, zingiberene, or diapophytoene, and their derivatives.

Isoprenoid precursors are thought to be generated by two pathways. The mevalonate pathway, or HMG-CoA reductase pathway, generates dimethylallyl pyrophosphate (DMAPP) and isopentyl pyrophosphate (IPP), the common C5 precursor for isoprenoids. The non-mevalonate pathway is an alternative pathway to form DMAPP and IPP. The DMAPP and IPP may be condensed to form geranyl-diphosphate (GPP), or other precursors, such as farnesyl-diphosphate (FPP), geranylgeranyl-diphosphate (GGPP), from which higher isoprenes are formed.

Examples of products which can include the isoprenoids of the present invention include, but are not limited to, fuel products, fragrance products, and insecticide products. In some instances, a product may be used directly. In other instances, the product may be used as a "feedstock" to produce another product. For example, where the product is an isoprenoid, the isoprenoid may be hydrogenated and "cracked" to produce a shorter chain hydrocarbon (e.g., farnesene is hydrogenated to produce farnesane which is then cracked to produce propane, butane, octane, or other fuel products).

The products produced by the present invention may be naturally, or non-naturally (e.g., as a result of transformation) produced by the host cell(s) and/or organism(s) transformed. The product may also be a novel molecule not present in nature. For example, products naturally produced in algae may be terpenes such as carotenoids (e.g. beta-carotene). Examples of products not naturally produced by algae may include a non-native terpene such as limonene. The host cell may be genetically modified, for example by transformation with a sequence to encourage the secretion of limonene.

### Fuel products

Examples of fuel products include petrochemical products and their precursors and all other substances that may be useful in the petrochemical industry. Fuel products include, for example, petroleum products, precursors of petroleum, as well as petrochemicals and precursors thereof. The fuel or fuel products may be used in a combustor such as a boiler, kiln, dryer or furnace. Other examples of combustors are internal combustion engines such as vehicle engines or generators, including gasoline engines, diesel engines, jet engines, and others. Fuel products may also be used to produce plastics, resins, fibers, elastomers, lubricants, and gels.

Fuel products can include small alkanes (for example, 1 to approximately 4 carbons) such as methane, ethane, propane, or butane, which may be used for heating (such as in cooking) or making plastics. Fuel products may also include molecules with a carbon backbone of approximately 5 to approximately 9 carbon atoms, such as naptha or ligroin, or their precursors. Other fuel products may be about 5 to about 12 carbon atoms or cycloalkanes used as gasoline or motor fuel. Molecules and aromatics of approximately 10 to approximately 18 carbons, such as kerosene, or its precursors, may also be fuel products. Fuel products may also include molecules, or their precursors, with more than 12 carbons, such as used for lubricating oil. Other fuel products include heavy gas or fuel oil, or their precursors, typically containing alkanes, cycloalkanes, and aromatics of approximately 20 to approximately 70 carbons. Fuel products also includes other residuals that can be derived from or found in crude oil, such as coke, asphalt, tar, and waxes, generally containing multiple rings with about 70 or more carbons, and their precursors.

The various fuel products may be further refined to a final product for an end user by a number of processes. Refining can occur by fractional distillation. For example, a mixture of fuel products, such as a mix of different hydrocarbons with different various chain lengths may be separated into various components by fractional distillation.

Refining may also include any one or more of the following steps; cracking, unifying, or altering the fuel product. Large fuel products, such as large hydrocarbons (e.g. ≥ C 10), may be broken down into smaller fragments by cracking. Cracking may be performed by heat or high pressure, such as by steam, visbreaking, or coking. Fuel products may also be refined by visbreaking, for example reducing the viscosity of heavy oils. Refining may also include coking, wherein a heavy, almost pure carbon residue is produced. Cracking may also be performed by catalytic means to enhance the rate of the cracking reaction by using catalysts such as, but not limited to, zeolite, aluminum hydrosilicate, bauxite, or silica-alumina. Catalysis may be by fluid catalytic cracking, whereby a hot catalyst, such as zeolite, is used to catalyze cracking reactions. Catalysis may also be performed by hydrocracking, where lower temperatures are generally used in comparison to fluid catalytic cracking. Hydrocracking typically occurs in the presence of elevated partial pressure of hydrogen gas. Fuel products may be refined by catalytic cracking to generate diesel, gasoline, and/or kerosene.

The fuel products may also be refined by combining them in a unification step, for example by using catalysts, such as platinum or a platinum-rhenium mix. The unification process typically produces hydrogen gas, a by-product which may be used in cracking.

The fuel products may also be refined by altering or rearranging or restructuring hydrocarbons into smaller molecules. There are a number of chemical reactions that occur in the catalytic reforming process of which are known to one of ordinary skill in the arts. Generally, catalytic reforming is performed in the presence of a catalyst and high partial pressure of hydrogen. One common process is alkylation. For example, propylene and butylene are mixed with a catalyst such as hydrofluoric acid or sulfuric acid.

The fuel products may also be blended or combined into mixtures to obtain an end product. For example, the fuel products may be blended to form gasoline of various grades, gasoline with or without additives, lubricating oils of various weights and grades, kerosene of various grades, jet fuel, diesel fuel, heating oil, and chemicals for making plastics and other polymers. Compositions of the fuel products described herein may be combined or blended with fuel products produced by other means.

Some fuel products produced from the host cells of the invention, especially after refining, will be identical to existing petrochemicals, i.e. same structure. Some of the fuel products may not be the same as existing petrochemicals. However, although a molecule may not exist in conventional petrochemicals or refining, it may still be useful in these industries. For example, a hydrocarbon could be produced that is in the boiling point range of gasoline, and that could be used as gasoline or an additive, even though it does not normally occur in gasoline.

### Methods.

Thus, a product (e.g. isoprenoid, fuel product, fragrance product, insecticide product) may be produced by a method that comprises: transforming a host organism (e.g., non-vascular, photosynthetic organism) with an expression vector; growing the organism; and collecting the product produced by the organism. In a related yet distinct aspect, the present invention provides a method for producing a product comprising: transforming a photosynthetic organism with an expression vector, growing the organism; and collecting the product produced by the oganism. The expression vector is typically the type of expression vector described herein, and is specifically used to add additional biosynthetic capacity to an organism or to modify an existing biosynthetic pathway within the organisms, either with the intension of increasing or allowing the production of a molecule by the photosynthetic organism.

The methods herein comprise selecting genes that are useful to produce products, such as isoprenoids, fuels, fragrances, and insecticides, transforming a cell of a photosynthetic organism with such gene(s), and growing such organisms under conditions suitable to allow the product to be produced. Organisms of the present invention can be cultured in conventional fermentation bioreactors, which include, but are not limited to, batch, fed-batch, cell recycle, and continuous fermentors. Further, they may be grown in photobioreactors (see e.g. US Appl. Publ. No. 20050260553; U.S. Pat. No. 5,958,761; U.S. Pat. No. 6,083,740). Culturing can also be conducted in shake flasks, test tubes, microtiter dishes, and petri plates. Culturing is carried out at a temperature, pH and oxygen content appropriate for the recombinant cell. Such culturing conditions are well within the expertise of one of ordinary skill in the art.

A host organism is an organism comprising a host cell. In preferred embodiments, the host organism is photosynthetic. A photosynthetic organism is one that naturally photosynthesizes (has a plastid) or that is genetically engineered or otherwise modified to be photosynthetic. In some instances, a photosynthetic organism may be transformed with a construct of the invention which renders all or part of the photosynthetic apparatus inoperable. In some instances a host organism is non-vascular and photosynthetic. The host cell can be prokaryotic. Examples of some prokaryotic organisms of the present invention include, but are not limited to, cyanobacteria (e.g., *Synechococcus, Synechocystis, Athrospira*)*.* The host organism can be unicellular or multicellular. In most embodiments, the host organism is eukaryotic (e.g. green algae, red algae, brown algae). In preferred embodiments, the host cell is a microalga (e.g., *Chlamydomonas reinhardtii, Dunaliella salina, Haematococcus pluvalis, Scenedesmus dimorphus, D. viridis,* or *D. tertiolecta).* Examples of organisms contemplated herein include, but are not limited to, rhodophyta, chlorophyta, heterokontophyta, tribophyta, glaucophyta, chlorarachniophytes, euglenoids, haptophyta, cryptomonads, dinoflagellata, and phytoplankton.

Some of the host organisms which may be used to practice the present invention are halophilic (e.g., *Dunaliella salina, D. viridis,* or *D. tertiolecta*)*.* For example, *D. salina* can grow in ocean water and salt lakes (salinity from 30-300 parts per thousand) and high salinity media (e.g., artificial seawater medium, seawater nutrient agar, brackish water medium, seawater medium, etc.). In some embodiments of the invention, a host cell comprising a vector of the present invention can be grown in a liquid environment which is 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 31., 3.2, 3.3, 3.4, 3.5, 3.6, 3.7, 3.8, 3.9, 4.0, 4.1, 4.2, 4.3 molar or higher concentrations of sodium chloride. One of skill in the art will recognize that other salts (sodium salts, calcium salts, potassium salts, etc.) may also be present in the liquid environments.

Where a halophilic organism is utilized for the present invention, it may be transformed with any of the vectors described herein. For example, *D. salina* may be transformed with a vector which is capable of insertion into the chloroplast genome and which contains nucleic acids which encode an isoprenoid producing enzyme (e.g., FPP synthase, zingiberene synthase, squalene synthase). Transformed halophilic organisms may then be grown in high-saline environments (e.g., salt lakes, salt ponds, high-saline media, etc.) to produce the products (e.g., isoprenoids) of interest. Isolation of the products may involve removing a transformed organism from a high-saline environment prior to extracting the product from the organism. In instances where the product is secreted into the surrounding environment, it may be necessary to desalinate the liquid environment prior to any further processing of the product.

A host organism may be grown under conditions which permit photosynthesis, however, this is not a requirement (e.g., a host organism may be grown in the absence of light). In some instances, the host organism may be genetically modified in such a way that photosynthetic capability is diminished and/or destroyed (see examples below). In growth conditions where a host organism is not capable of photosynthesis (e.g., because of the absence of light and/or genetic modification), typically, the organism will be provided with the necessary nutrients to support growth in the absence of photosynthesis. For example, a culture medium in (or on) which an organism is grown, may be supplemented with any required nutrient, including an organic carbon source, nitrogen source, phosphorous source, vitamins, metals, lipids, nucleic acids, micronutrients, or an organism-specific requirement. Organic carbon sources includ any source of carbon which the host organism is able to metabolize including, but not limited to, acetate, simple carbohydrates (e.g., glucose, sucrose, lactose), complex carbohydrates (e.g., starch, glycogen), proteins, and lipids. One of skill in the art will recognize that not all organisms will be able to sufficiently metabolize a particular nutrient and that nutrient mixtures may need to be modified from one organism to another in order to provide the appropriate nutrient mix.

A host organism may also be grown on land, e.g., landfills. In some cases, host organism(s) are grown near ethanol production plants or other facilities or regions (e.g., cities, highways, etc.) generating CO₂. As such, the methods herein contemplate business methods for selling carbon credits to ethanol plants or other facilities or regions generating CO₂ while making fuels by growing one or more of the modified organisms described herein near the ethanol production plant.

Further, the organisms may be grown in outdoor open water, such as ponds, the ocean, sea, rivers, waterbeds, marsh water, shallow pools, lakes, reservoirs, etc. When grown in water, the organisms can be contained in a halo like object comprising of lego-like particles. The halo object encircles the algae and allows it to retain nutrients from the water beneath while keeping it in open sunlight.

In some instances, organisms can be grown in containers wherein each container comprises 1 or 2 or a plurality of organisms. The containers can be configured to float on water. For example, a container can be filled by a combination of air and water to make the container and the host organism(s) in it buoyant. A host organism that is adapted to grow in fresh water can thus be grown in salt water (i.e., the ocean) and vice versa. This mechanism allows for automatic death of the organism if there is any damage to the container.

In some instances a plurality of containers can be contained within a halo-like structure as described above. For example, up to 100, 1,000, 10,000, 100,000, or 1,000,000 containers can be arranged in a meter-square of a halo-like structure.

In some embodiments, the product (e.g. fuel product, fragrance product, insecticide product) is collected by harvesting the organism. The product may then be extracted from the organism. In some instances, the product may be produced without killing the organisms. Producing and/or expressing the product may not render the organism unviable.

In some embodiments, the production of the product (e.g. fuel product, fragrance product, insecticide product) is inducible. The product may be induced to be expressed and/or produced, for example, by exposure to light. In yet other embodiments, the production of the product is autoregulatable. The product may form a feedback loop, wherein when the product (e.g. fuel product, fragrance product, insecticide product) reaches a certain level, expression or secretion of the product may be inhibited. In other embodiments, the level of a metabolite of the organism inhibits expression or secretion of the product. For example, endogenous ATP produced by the organism as a result of increased energy production to express or produce the product, may form a feedback loop to inhibit expression of the product. In yet another embodiment, production of the product may be inducible, for example, by light or an exogenous agent. For example, an expression vector for effecting production of a product in the host organism may comprise an inducible regulatory control sequence that is activated or inactivated by an exogenous agent.

The present invention also relates to methods for screening for new genes/expression vectors to create any of the fuel products described herein. Such methods comprise the steps of: (1) inserting a candidate expression vector of nucleic acids into a photosynthetic organism, (2) collecting a putative fuel product produced there from, (3) applying the putative fuel product to a mass spectrometer to determine a characteristic of the putative fuel product, and whether it may be used as a fuel product. In some embodiments, step (2) may comprise collecting a known fuel product and whether a candidate expression vector increases production or secretion of the fuel product relative to a photosynthetic organism without the candidate expression vector.

### Other Methods

The present invention also provides a business method comprising providing a carbon credit to a party growing a genetically modified non-vascular, photosynthetic organism adapted to produce a fuel product. The method of producing a fuel product provided by the present invention provides a possibly more environmentally friendly way of generating fuel products relative to current methods. As such, the methods and compositions described herein may be used in a business method in exchange for carbon credits.

Carbon credits may be an allowance, permit, credit, or the like which are or have been allowed, authorized, or recognized by some relevant sovereign entity (such as but not limited to a city (including municipalities of all sizes and types including both incorporated and unincorporated municipalities), a county, a state or province, or a nation, as well as related governmental entities such regional, multi-national, or other international bodies such as the United Nations or the European Union).

The carbon credit may be substantially received directly from a regulatory agency or administrative entity. In other instances, they may be received indirectly, for example, an entity using the methods or compositions herein may receive the carbon credits directly from a regulatory agency, and may then transfer the carbon credits to another entity. Transfer of the carbon credit may be in association with a given process, product using the genetically modified non-vascular, photosynthetic organism adapted to produce a fuel product.

For example, a first entity may be identified that provides a consumable product that is distributed for consumption in an end-user mobile platform, wherein the consumption and/or production of the consumable product includes a corresponding resultant emission. For example, combustion of diesel fuel often results in the environmental release of corresponding nitrogen oxides combustion of diesel fuel often results in the environmental release of corresponding nitrogen oxides and combustion of gasoline often results in the environmental release of corresponding sulfur oxide.

The first party may adopt a method of producing its products using the genetically modified organisms described above, or use the products generated by the genetically modified organisms described above in their compositions, resulting in less harmful effects on the environment than conventional methods of generating, for example, diesel fuel. Thus off-setting the environmental effects of the end product. The first party may then receive a carbon, or emission, credit as a result of a reduction of the total emission. The carbon credit may be received from a regulatory or administrative agency, or may be transferred to the first party from a second party, wherein the second party may have sold the genetically modified organism or the products of the genetically modified organism to the first party.

The carbon credit may be exchanged for a substantially liquid monetary instrument. For example, the carbon credit may be exchanged for a cash equivalent, such as cash, check, and the like. The carbon credit may also be exchanged for a legal grant regarding an intellectual property right, for example, but not limited to, an assignment or a license. The carbon credit may also be exchanged for a government tax subsidy or access to purchasers of a given market. The carbon credit may also be exchanged for use of another carbon emission process, such as one not comprising growing the organism. For example, a party may have a limited number of emissions it may release in a time period, for example, a month or a year, and going over the limit may incur fines and penalties. However, with carbon credits, the party going over the limit may exchange of carbon credits to offset the fines or penalties or may be taken into account when determining the amount of emissions generated by the party.

The business methods of the invention can also involve the production of products other than fuel products, such as fragrances and insecticides. Business methods associated with fuel products, including those involving the use of carbon credits, are also relevant to the production of other types of useful products and materials.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby. The following examples merely illustrate the invention disclosed herein, but do not limit it.

### EXAMPLES

### Example 1. Production of FPP synthases and sesquiterpene synthases in C. reinhardtii

In this example a nucleic acids encoding FPP synthase from *G. gallus* and bisabolene synthase from *P. abies* were introduced into *C. reinhardtii.* Transforming DNA is shown graphically in **FIG. 3****.** For these examples, the transforming DNA was contained in a vector with *E. coli* elements (e.g., origin of replication, antibiotic resistance marker). In this instance the gene encoding FPP synthase (SEQ ID NO. 82, Table 5; SEQ ID NO. 135, Table 6) is the segment labeled "transgene" in **FIG. 3** and is regulated by the 5' UTR and promoter sequence for the *psbA* gene from *C. reinhardtii* and the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The bisabolene synthase gene (SEQ ID NO. 115, Table 5; SEQ ID NO. 168, Table 6) is the segment labeled "transgene" in **FIG. 3** and is regulated by the 5' UTR and promoter sequence for the *psbA* gene from *C. reinhardtii* and the 3' UTR for the *psbA* gene from *C. reinhardtii,* and the segment labeled "Selection Marker" is the streptomycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The FPP synthase transgene cassette is targeted to the *psbA* loci of C. *reinhardtii* via the segments labeled "Homology A" and "Homology B," which are identical to sequences of DNA flanking the *psbA* loci on the 5' and 3' sides, respectively. The bisabolene synthase transgene cassette is targeted to the 3HB locus of *C. reinhardtii* via the segments labeled "Homology C" and "Homology D," which are identical to sequences of DNA flanking the 3HB locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (100 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration was varied to compensate for amount and concentration of algae lysate in EDTA that was added. Annealing temperature gradients were employed to determine optimal annealing temperature for specific primer pairs.

To identify strains that contain the FPP synthase gene, a primer pair was used in which one primer anneals to a site within the *psbA* 5'UTR (SEQ ID NO. 55) and the other primer (SEQ ID NO. 66) anneals within the FPP synthase coding segment. Desired clones are those that yield a PCR product of expected size. To identify strains that contain the bisabolene synthase gene, a primer pair was used in which one primer anneals to a site within the *psbA* 5'UTR (SEQ ID NO. 55) and the other primer anneals within the bisabolene synthase coding segment (SEQ ID NO. 73). Desired clones are those that yield a PCR product of expected size in both reactions.

To determine the degree to which the endogenous *psbA* gene locus is displaced (heteroplasmic vs. homoplasmic), a PCR reaction consisting of two sets of primer pairs were employed (in the same reaction). The first pair of primers amplifies the endogenous locus targeted by the expression vector and consists of a primer that anneals within the psbA 5'UTR (SEQ ID NO. 57) and one that anneals within the psbA coding region (SEQ ID NO. 58). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction. Results from this PCR are shown in **FIG. 4****,** panels A, B, and C.

Results from this PCR on 96 clones were determined and the results are shown in **FIG. 4. Figures 4A and 4B** show PCR results using the pairs specific for the FPP synthase and squalene synthase genes, respectively. As can be seen, multiple transformed clones are positive for insertion of both the FPP synthase and squalene synthase genes (e.g. numbers 1-3). **Figure 4C** shows the PCR results using the primer pairs to differentiate homoplasmic from heteroplasmic clones. As can be seen, multiple transformed clones are either homoplasmic or heteroplasmic to a degree in favor of incorporation of the transgene (e.g. numbers 1-3). Unnumbered clones demonstrate the presence of wild-type locus and, thus, were not selected for further analysis.

To determine if the FPP synthase gene led to expression of the FPP synthase and if the bisabolene synthase gene led to expression of the bisabolene synthase in transformed algae cells, both soluble proteins were immunoprecipitated and visualized by Western blot. Briefly, 500 ml of algae cell culture was harvested by centrifugation at 4000xg at 4°C for 15 min. The supernatant was decanted and the cells resuspended in 10 ml of lysis buffer (100 mM Tris-HCl, pH=8.0, 300 mM NaCl, 2% Tween-20). Cells were lysed by sonication (10x30sec at 35% power). Lysate was clarified by centrifugation at 14,000xg at 4°C for 1 hour. The supernatant was removed and incubated with anti-FLAG antibody-conjugated agarose resin at 4°C for 10 hours. Resin was separated from the lysate by gravity filtration and washed 3x with wash buffer ((100 mM Tris-HCl, pH=8.0, 300 mM NaCl, 2% Tween-20). Resin was mixed 4:1 with loading buffer (XT Sample buffer; Bio-Rad), samples were heated to 95°C for 1 min, cooled to 23 °C, and insoluble proteins were removed by centrifugation. Soluble proteins were separated by SDS-PAGE, followed by transfer to PVDF membrane. The membrane was blocked with TBST + 0.5% dried, nonfat milk at 23°C for 30 min, incubated with anti-FLAG, alkaline phosphatase-conjugate antibody (diluted 1:2,500 in TBST + 0.5% dried, nonfat milk) at 4°C for 10 hours, washed three times with TBST. Proteins were visualized with chemifluorenscent detection. Results from multiple clones **(****FIG. 4D****)** show that expression of the FPP synthase gene led to expression of the FPP synthase and expression of the bisabolene synthase gene led to expression of the bisabolene synthase.

Cultivation of *C. reinhardtii* transformants for expression of FPP synthase and bisabolene synthase was carried out in liquid TAP medium at 23°C under constant illumination of 5,000 Lux on a rotary shaker set at 100 rpm, unless stated otherwise. Cultures were maintained at a density of 1x10⁷ cells per ml for at least 48 hr prior to harvest.

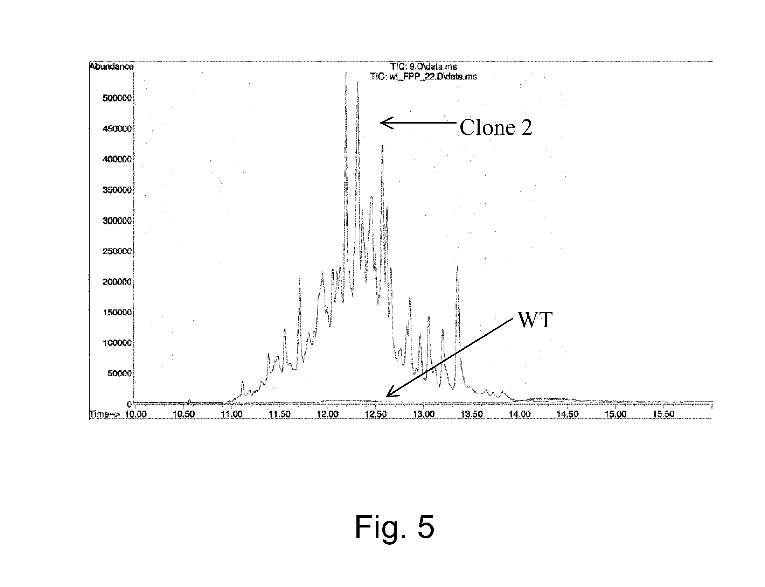

To determine whether bisabolene synthase produced in the algae chloroplast is a functional enzyme, sesquiterpene production from FPP was examined. Briefly, 50 mL of algae cell culture was harvested by centrifugation at 4000xg at 4°C for 15 min. The supernatant was decanted and the cells resuspended in 0.5 mL of reaction buffer (25 mM HEPES, pH=7.2, 100 mM KCI, 10 mM MnCl2, 10% glycerol, and 5 mM DTT). Cells were lysed by sonication (10x30sec at 35% power). 0.33 mg/mL of FPP were added to the lysate and the mixture was transferred to a glass vial. The reaction was overlaid with heptane and incubated at 23°C for 12 hours. The reaction was quenched and extracted by vortexing the mixture. 0.1 mL of heptane was removed and the sample was analyzed by gas chromatography - mass spectrometry (GC-MS). Results are shown in **FIG. 5****.** The results show a large increase (indicated by the peaks) in sesquiterpene over a wild-type strain.

### Example 2. Production of triterpene molecules in C. reinhardtii

In this example a nucleic acids encoding FPP synthase from *G. gallus* and squalene synthase *S. aureus* were introduced into C. *reinhardtii.* Transforming DNA is shown graphically in **FIG. 3****.** In this instance the segment labeled "Transgene 1" is the gene encoding FPP synthase (SEQ ID NO. 82, Table 5; SEQ ID NO. 135, Table 6), the segment labeled "Transgene 2" is the gene encoding squalene synthase (SEQ ID NO. 85, Table 5; SEQ ID NO. 138, Table 6), the segments labeled "5' UTR" are the 5' UTR and promoter sequence for the *rbcL* gene from *C. reinhardtii,* the segments labeled "3' UTR" contain the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The transgene cassette is targeted to the 3HB locus of *C*. *reinhardtii* via the segments labeled "5' Homology" and "3' Homology," which are identical to sequences of DNA flanking the 3HB locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (150 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration is varied to compensate for amount and concentration of algae lysate in EDTA added. Annealing temperature gradients were employed to determine optimal annealing temperature for specific primer pairs.

To identify strains that contain the FPP synthase gene, a primer pair was used in which one primer anneals to a site within the *psbC* 5'UTR (SEQ ID NO. 64) and the other primer anneals within the FPP synthase coding segment (SEQ ID NO. 66). To identify strains that contain the squalene synthase gene, a primer pair was used in which one primer anneals to a site within the *psbC* 5'UTR (SEQ ID NO. 64) and the other primer anneals within the squalene synthase coding segment (SEQ ID NO. 72). Desired clones are those that yield a PCR product of expected size in both reactions. To determine the degree to which the endogenous gene locus is displaced (heteroplasmic vs. homoplasmic), a PCR reaction consisting of two sets of primer pairs were employed (in the same reaction). The first pair of primers amplifies the endogenous locus targeted by the expression vector (SEQ ID NOs. 68 and 69). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction.

Results from this PCR on 96 clones were determined and the results are shown in **FIG. 6. Figures 6A and 6B** show PCR results using the pairs specific for the FPP synthase and squalene synthase genes, respectively. As can be seen, multiple transformed clones are positive for insertion of both the FPP synthase and squalene synthase genes (e.g. numbers 1-10). **Figure 6C** shows the PCR results using the primer pairs to differentiate homoplasmic from heteroplasmic clones. As can be seen, multiple transformed clones are either homoplasmic or heteroplasmic to a degree in favor of incorporation of the transgene (e.g. numbers 1-10). Unnumbered clones demonstrate the presence of wild-type locus and, thus, were not selected for further analysis.

To ensure that the presence of the FPP synthase and squalene synthase genes led to expression of the FPP synthase and squalene synthase enzymes, a Western blot was performed. Approximately 1x10⁸ algae cells were collected from TAP agar medium and suspended in 0.5 ml of lysis buffer (750 mM Tris, pH=8.0, 15% sucrose, 100 mM beta-mercaptoethanol). Cells were lysed by sonication (5x30sec at 15% power). Lysate was mixed 1:1 with loading buffer (5% SDS, 5% beta-mercaptoethanol, 30% sucrose, bromophenol blue) and proteins were separated by SDS-PAGE, followed by transfer to PVDF membrane. The membrane was blocked with TBST + 5% dried, nonfat milk at 23°C for 30 min, incubated with anti-FLAG antibody (diluted 1:1,000 in TBST + 5% dried, nonfat milk) at 4°C for 10 hours, washed three times with TBST, incubated with horseradish-linked anti-mouse antibody (diluted 1:10,000 in TBST + 5% dried, nonfat milk) at 23°C for 1 hour, and washed three times with TBST. Proteins were visualized with chemiluminescent detection. Results from multiple clones **(****FIG. 6D****)** show that expression of the FPP synthase gene in *C. reinhardtii* cells resulted in production of the protein. Visualization of the product of the squalene synthase gene was occluded by the signal from an unidentified protein present in all samples.

Cultivation of *C. reinhardtii* transformants for expression of endo-β-glucanase was carried out in liquid TAP medium at 23°C under constant illumination of 5,000 Lux on a rotary shaker set at 100 rpm, unless stated otherwise. Cultures were maintained at a density of 1x10⁷ cells per ml for at least 48 hr prior to harvest.

To determine if the FPP synthase and squalene synthase enzymes were produced in transformed algae cells, both enzymes were immunopreciptated and visualized by Western blot. Briefly, 500 ml of algae cell culture was harvested by centrifugation at 4000xg at 4°C for 15 min. The supernatant was decanted and the cells resuspended in 10 ml of lysis buffer (100 mM Tris-HCl, pH=8.0, 300 mM NaCl, 2% Tween-20). Cells were lysed by sonication (10x30sec at 35% power). Lysate was clarified by centrifugation at 14,000xg at 4°C for 1 hour. The supernatant was removed and incubated with anti-FLAG antibody-conjugated agarose resin at 4°C for 10 hours. Resin was separated from the lysate by gravity filtration and washed 3x with wash buffer ((100 mM Tris-HCl, pH=8.0, 300 mM NaCl, 2% Tween-20). Results from Western blot analysis of multiple samples **(****FIGS. 6D and 6E****)** show that the both enzymes are indeed produced.

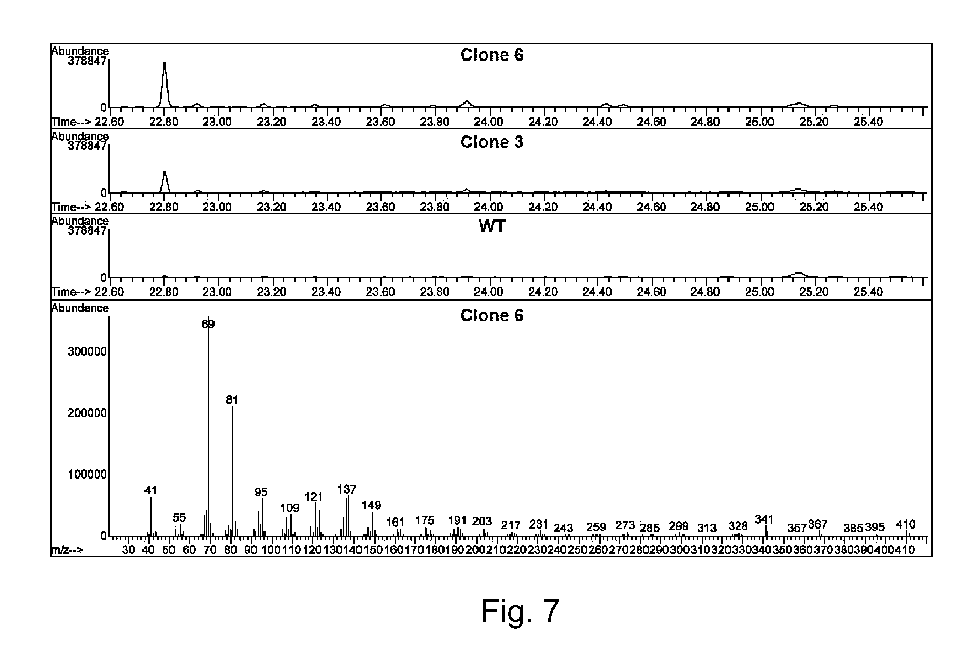

To determine whether FPP synthase and squalene synthase comprise a functional squalene biosynthesis pathway *in vivo,* the accumulation of squalene was determined. Briefly, 1.2 L of algae cell culture was harvested by centrifugation at 4000xg at 4°C for 5 min. The supernatant was poured off and the remaining cell pellet resuspended in 10 ml of TAP medium and transferred to 50mL centrifuge tube. Cells were pelleted again by centrifugation at 3,000 RPM for 5 minutes. All of the supernatant was removed and the cell mass determined. Samples were kept on ice and cell pellets resuspended in ice-cold methanol (MeOH) at a ratio of 0.75 mg biomass:5 mL MeOH. A solvent blank consisting of 30mL MeOH was stored on ice in a 50mL conical vial to control for leaching. Cell pellets were solublized by repeated pipetting and lysates stored on ice to precipitate protein. Lysates were then clarified by centrifugation at 1,000 RPM for 5 min. 4 mL of soluble fraction was transferred to amber glass vials and overlaid with 8 mL of heptane. Lysates were extracted overnight at 23°C on a rotating wheel. 1.5 mL of heptane from each sample was lyophilized to complete dryness, and then resuspended in 100 uL heptane. Analysis was performed on GC-MS. Results are shown in **FIG. 7****.** These analyses were conducted with 5 replicates per strain.

### Example 3. Production of monoterpene synthases in C. reinhardtii

In this example a nucleic acids encoding limonene synthase from *M. spicata* was introduced into *C. reinhardtii.* Transforming DNA is shown graphically in **FIG. 3****.** In this instance the segment labeled "Transgene" is the gene encoding limonene synthase (SEQ ID NO. 74, Table 5; SEQ ID NO. 127, Table 6) that is regulated by the 5' UTR and promoter sequence for the *psbA* gene from *C. reinhardtii* and the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The transgene cassette is targeted to the *psbA* loci of *C. reinhardtii* via the segments labeled "Homology A" and "Homology B," which are identical to sequences of DNA flanking the *psbA* locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (100 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration is varied to compensate for amount and concentration of algae lysate in EDTA added. Annealing temperature gradients were employed to determine optimal annealing temperature for specific primer pairs.

To identify strains that contain the limonene synthase gene, a primer pair was used in which one primer anneals to a site within the psbA 5'UTR (SEQ ID NO. 55) and the other primer anneals within the limonene synthase coding segment (SEQ ID NO. 56). Desired clones are those that yield a PCR product of expected size. To determine the degree to which the endogenous gene locus is displaced (heteroplasmic vs. homoplasmic), a PCR reaction consisting of two sets of primer pairs were employed (in the same reaction). The first pair of primers amplifies the endogenous locus targeted by the expression vector and consists of a primer that anneals within the psbA 5'UTR (SEQ ID NO. 57) and one that anneals within the psbA coding region (SEQ ID NO. 58). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction.

Cultivation of *C. reinhardtii* transformants for expression of limonene synthase was carried out in liquid TAP medium at 23°C in the dark on a rotary shaker set at 100 rpm, unless stated otherwise. Cultures were maintained at a density of 1x10⁷ cells per ml for at least 48 hr prior to harvest.

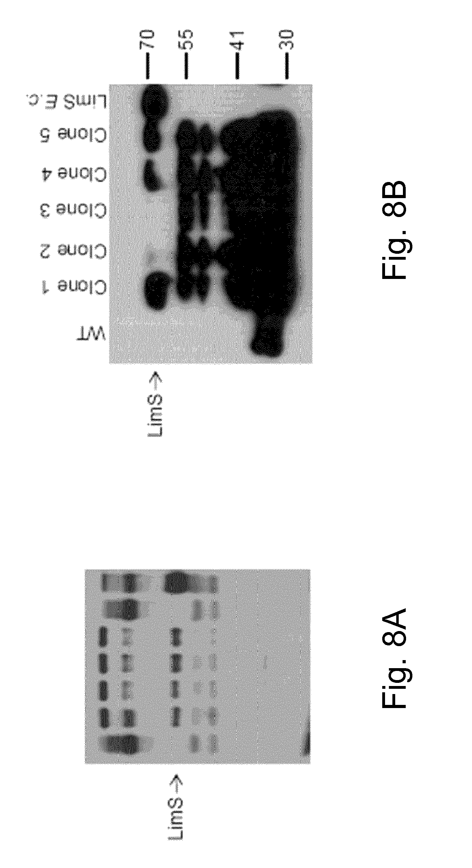

To determine if the limonene synthase gene led to expression of the limonene synthase in transformed algae cells, both soluble proteins were immunopreciptated and visualized by Western blot. Briefly, 500 ml of algae cell culture was harvested by centrifugation at 4000xg at 4°C for 15 min. The supernatant was decanted and the cells resuspended in 10 ml of lysis buffer (100 mM Tris-HCl, pH=8.0, 300 mM NaCl, 2% Tween-20). Cells were lysed by sonication (10x30sec at 35% power). Lysate was clarified by centrifugation at 14,000xg at 4°C for 1 hour. The supernatant was removed and incubated with anti-FLAG antibody-conjugated agarose resin at 4°C for 10 hours. Resin was separated from the lysate by gravity filtration and washed 3x with wash buffer ((100 mM Tris-HCl, pH=8.0, 300 mM NaCl, 2% Tween-20). Results from Western blot analysis of multiple samples **(****FIG. 8****)** show that limonene synthase is indeed produced.

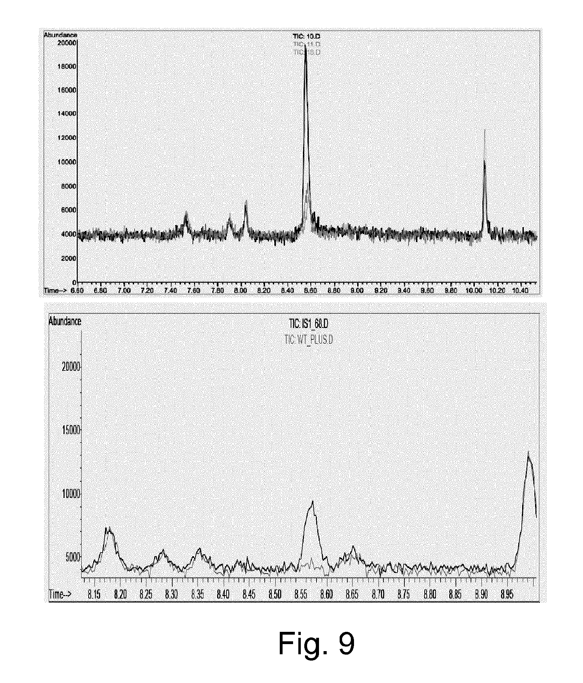

To determine whether limonene synthase produced in the algae chloroplast is a functional enzyme, limonene production from GPP was examined. Briefly, 50 uL of the limonene synthase-bound agarose (same samples prepared above) was suspended in 300 uL of reaction buffer (25 mM HEPES, pH=7.2, 100 mM KCI, 10 mM MnCl2, 10% glycerol, and 5 mM DTT) with 0.33 mg/mL GPP and transferred to a glass vial. The reaction was overlaid with heptane and incubated at 23°C for 12 hours. The reaction was quenched and extracted by vortexing the mixture. 0.1 mL of heptane was removed and the sample was analyzed by GC-MS. Results are shown in **FIG. 9****.** The results show that the isolated enzyme was capable of converting GPP to limonene *in vitro.*

Limonene synthase activity from crude cell lysates was also examined. Briefly, 50 mL of algae cell culture was harvested by centrifugation at 4000xg at 4°C for 15 min. The supernatant was decanted and the cells resuspended in 0.5 mL of reaction buffer (25 mM HEPES, pH=7.2, 100 mM KCI, 10 mM MnCl2, 10% glycerol, and 5 mM DTT). Cells were lysed by sonication (10x30sec at 35% power). 0.33 mg/mL of GPP was added to the lysate and the mixture was transferred to a glass vial. The reaction was overlaid with heptane and incubated at 23°C for 12 hours. The reaction was quenched and extracted by vortexing the mixture. 0.1 mL of heptane was removed and the sample was analyzed by GC-MS. Results are shown in **FIG. 9****.** The results show that the strain producing limonene synthase is capable of producing limonene *in vivo.*

### Example 4. Production of GPP synthases in C. reinhardtii

In this example a nucleic acids encoding GPP synthase from *A. thaliana* was introduced into *C. reinhardtii.* Transforming DNA is shown graphically in **FIG. 3****.** In this instance the segment labeled "Transgene" is the gene encoding GPP synthase (SEQ ID NO. 89, Table 5; SEQ ID NO. 142, Table 6) that is regulated by the 5' UTR and promoter sequence for the *psbA* gene from *C. reinhardtii* and the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The transgene cassette is targeted to the *psbA* loci of *C. reinhardtii* via the segments labeled "Homology A" and "Homology B," which are identical to sequences of DNA flanking the *psbA* locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (100 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration is varied to compensate for amount and concentration of algae lysate in EDTA added. Annealing temperature gradients were employed to determine optimal annealing temperature for specific primer pairs.

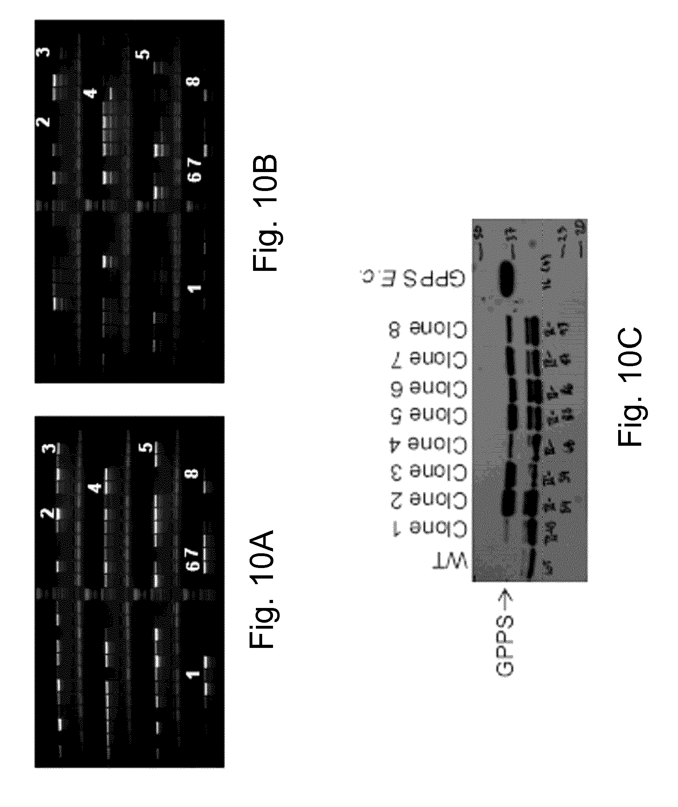

To identify strains that contain the GPP synthase gene, a primer pair was used in which one primer anneals to a site within the psbA 5'UTR (SEQ ID NO. 55) and the other primer anneals within the GPP synthase coding segment (SEQ ID NO. 61). Desired clones are those that yield a PCR product of expected size. To determine the degree to which the endogenous gene locus is displaced (heteroplasmic vs. homoplasmic), a PCR reaction consisting of two sets of primer pairs were employed (in the same reaction). The first pair of primers amplifies the endogenous locus targeted by the expression vector and consists of a primer that anneals within the psbA 5'UTR (SEQ ID NO. 57) and one that anneals within the psbA coding region (SEQ ID NO.58). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction. Results from this PCR are shown in **FIG. 10** **(A and B).**

To ensure that the presence of the GPP synthase gene led to expression of the GPP synthase, a Western blot was performed. Approximately 1x10⁸ algae cells were collected from TAP agar medium and suspended in 0.05 ml of lysis buffer (Bugbuster; Novagen). Solutions were heated to 95°C for 5 min and then cooled to 23°C. Lysate was mixed 3:1 with loading buffer (XT Sample buffer; Bio-Rad), samples were heated to 95°C for 1 min, cooled to 23°C, and insoluble proteins were removed by centrifugation. Soluble proteins were separated by SDS-PAGE, followed by transfer to PVDF membrane. The membrane was blocked with TBST + 5% dried, nonfat milk at 23°C for 30 min, incubated with anti-FLAG antibody (diluted 1:2,500 in TBST + 5% dried, nonfat milk) at 4°C for 10 hours, washed three times with TBST, incubated with horseradish-linked anti-mouse antibody (diluted 1:5,000 in TBST + 5% dried, nonfat milk) at 23°C for 1 hour, and washed three times with TBST. Proteins were visualized with chemiluminescent detection. Results from multiple clones **(****FIG. 10C****)** show that expression of the GPP synthase gene in *C. reinhardtii* cells resulted in production of the protein. **Fig. 10C****.**

Cultivation of *C. reinhardtii* transformants for expression of GPP synthase was carried out in liquid TAP medium at 23°C under constant illumination of 5,000 Lux on a rotary shaker set at 100 rpm, unless stated otherwise. Cultures were maintained at a density of 1x10⁷ cells per ml for at least 48 hr prior to harvest.

To determine whether GPP synthase produced in the algae chloroplast is a functional enzyme, limonene production from IPP and DMAPP is examined. Briefly, 50 mL of algae cell culture is harvested by centrifugation at 4000xg at 4°C for 15 min. The supernatant is decanted and the cells resuspended in reaction buffer (25 mM HEPES, pH=7.2, 100 mM KCI, 10 mM MnCl2, 10% glycerol, and 5 mM DTT). Cells are lysed by sonication (10x30sec at 35% power). One ug of limonene synthase (prepared from *E. coli)* is added to the lysate along with 0.33 mg/mL IPP and 0.33 mg/mL DMAPP and the mixture is transferred to a glass vial. The reaction is overlaid with heptane and incubated at 23°C for 12 hours. The reaction is quenched and extracted by vortexing the mixture. 0.1 mL of heptane is removed and the sample analyzed by GC-MS.

### Example 5. Production of FPP synthases and sesquiterpene synthases in C. reinhardtii

In this example a nucleic acids encoding FPP synthase from *G. gallus* and zingiberene synthase from *O*. *basilicum* were introduced into *C. reinhardtii.* Transforming DNA is shown graphically in **FIG. 3****.** In this instance the segment labeled "Transgene 1" is the gene encoding FPP synthase (SEQ ID NO. 82, Table 5; SEQ ID NO. 135, Table 6) that is regulated by the 5' UTR and promoter sequence for the *psbD* gene from C. *reinhardtii* and the 3' UTR for the *psbA* gene from C. *reinhardtii,* the segment labeled "Transgene 2" is the gene encoding zingiberene synthase (SEQ ID NO. 101, Table 5; SEQ ID NO. 154, Table 6) that is regulated by the 5' UTR and promoter sequence for the *psbD* gene from *C. reinhardtii* and the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The transgene cassette is targeted to the 3HB locus of *C. reinhardtii* via the segments labeled "Homology C" and "Homology D," which are identical to sequences of DNA flanking the 3HB locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (100 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration is varied to compensate for amount and concentration of algae lysate in EDTA added. Annealing temperature gradients were employed to determine optimal annealing temperature for specific primer pairs. To identify strains that contain the FPP synthase gene, a primer pair was used in which one primer anneals to a site within the *psbD* 5'UTR (SEQ ID NO. 62) and the other primer anneals within the FPP synthase coding segment (SEQ ID NO. 66). To identify strains that contain the zingiberene synthase gene, a primer pair was used in which one primer anneals to a site within the *psbD* 5'UTR (SEQ ID NO. 62) and the other primer anneals within the zingiberene synthase coding segment (SEQ ID NO. 67). Desired clones are those that yield a PCR product of expected size in both reactions. To determine the degree to which the endogenous gene locus is displaced (heteroplasmic vs. homoplasmic), a PCR reaction consisting of two sets of primer pairs were employed (in the same reaction). The first pair of primers amplifies the endogenous locus targeted by the expression vector (SEQ ID NOs. 68 and 69). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction.

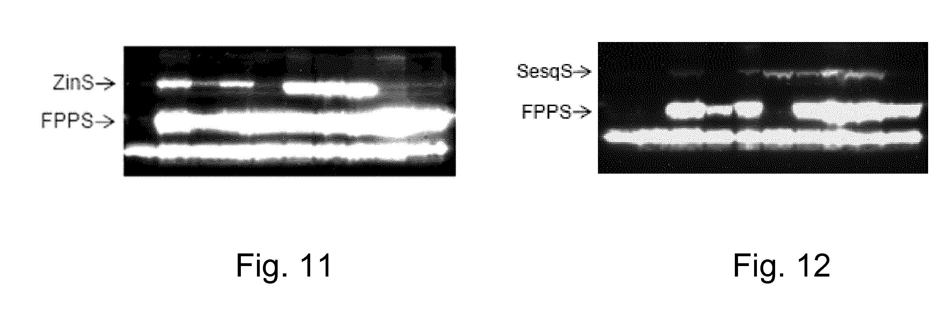

To ensure that the presence of the FPP synthase and zingiberene synthase genes led to expression of the FPP synthase and zingiberene synthase enzymes, a Western blot was performed. Approximately 1x10⁸ algae cells were collected from TAP agar medium and suspended in 0.05 ml of lysis buffer (Bugbuster; Novagen). Solutions were heated to 95°C for 5 min and then cooled to 23°C. Lysate was mixed 3:1 with loading buffer (XT Sample buffer; Bio-Rad), samples were heated to 95°C for 1 min, cooled to 23 °C, and insoluble proteins were removed by centrifugation. Soluble proteins were separated by SDS-PAGE, followed by transfer to PVDF membrane. The membrane was blocked with TBST + 5% dried, nonfat milk at 23°C for 30 min, incubated with anti-FLAG antibody (diluted 1:2,500 in TBST + 5% dried, nonfat milk) at 4°C for 10 hours, washed three times with TBST, incubated with horseradish-linked anti-mouse antibody (diluted 1:5,000 in TBST + 5% dried, nonfat milk) at 23°C for 1 hour, and washed three times with TBST. Proteins were visualized with chemiluminescent detection. Results from multiple clones **(****FIG. 11****)** show expression of the GPP synthase gene in *C. reinhardtii* cells resulted in production of the protein.

Cultivation of *C. reinhardtii* transformants for expression of FPP synthase and zingiberene synthase was carried out in liquid TAP medium at 23°C under constant illumination of 5,000 Lux on a rotary shaker set at 100 rpm, unless stated otherwise. Cultures were maintained at a density of 1x10⁷ cells per ml for at least 48 hr prior to harvest.

To determine whether FPP synthase and zingiberene synthase produced in the algae chloroplast are functional, sesquiterpene production from DMAPP and IPP is examined. Briefly, 50 mL of algae cell culture is harvested by centrifugation at 4000xg at 4°C for 15 min. The supernatant is decanted and the cells resuspended in 0.5 mL of reaction buffer (25 mM HEPES, pH=7.2, 100 mM KCI, 10 mM MnCl2, 10% glycerol, and 5 mM DTT). Cells are lysed by sonication (10x30sec at 35% power). 0.33 mg/mL of FPP are added to the lysate and the mixture transferred to a glass vial. The reaction is overlaid with heptane and incubated at 23°C for 12 hours. The reaction is quenched and extracted by vortexing the mixture. 0.1 mL of heptane is removed and the sample analyzed by gas chromatography - mass spectrometry (GC-MS).

### Example 6. Production of FPP synthases and sesquiterpene synthases in C. reinhardtii

In this example a nucleic acids encoding FPP synthase from *G. gallus* and sesquiterpene synthase from Z. *mays* were introduced into *C. reinhardtii.* Transforming DNA is shown graphically in **FIG. 3****.** In this instance the segment labeled "Transgene 1" is the gene encoding FPP synthase (SEQ ID NO. 82, Table 5; SEQ ID NO. 135, Table 6) that is regulated by the 5' UTR and promoter sequence for the *psbD* gene from *C. reinhardtii* and the 3' UTR for the *psbA* gene from *C. reinhardtii,* the segment labeled "Transgene 2" is the gene encoding sesquiterpene synthase (SEQ ID NO. 106, Table 5; SEQ ID NO. 159, Table 6) that is regulated by the 5' UTR and promoter sequence for the *psbD* gene from *C. reinhardtii* and the 3' UTR for the *psbA* gene from *C. reinhardtii,* and the segment labeled "Selection Marker" is the kanamycin resistance encoding gene from bacteria, which is regulated by the 5' UTR and promoter sequence for the *atpA* gene from *C. reinhardtii* and the 3' UTR sequence for the *rbcL* gene from *C. reinhardtii.* The transgene cassette is targeted to the 3HB locus of *C. reinhardtii* via the segments labeled "Homology C" and "Homology D," which are identical to sequences of DNA flanking the 3HB locus on the 5' and 3' sides, respectively. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on *C. reinhardtii* strain 137c (mt+). Cells were grown to late log phase (approximately 7 days) in the presence of 0.5 mM 5-fluorodeoxyuridine in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells were harvested by centrifugation at 4,000xg at 23°C for 5 min. The supernatant was decanted and cells resuspended in 4 ml TAP medium for subsequent chloroplast transformation by particle bombardment (Cohen et al., supra, 1998). All transformations were carried out under kanamycin selection (100 µg/ml) in which resistance was conferred by the gene encoded by the segment in **FIG. 3** labeled "Selection Marker." (Chlamydomonas Stock Center, Duke University).

PCR was used to identify transformed strains. For PCR analysis, 10⁶ algae cells (from agar plate or liquid culture) were suspended in 10 mM EDTA and heated to 95 °C for 10 minutes, then cooled to near 23°C. A PCR cocktail consisting of reaction buffer, MgCl2, dNTPs, PCR primer pair(s) **(Table 4),** DNA polymerase, and water was prepared. Algae lysate in EDTA was added to provide template for reaction. Magnesium concentration is varied to compensate for amount and concentration of algae lysate in EDTA added. Annealing temperature gradients were employed to determine optimal annealing temperature for specific primer pairs.

To identify strains that contain the FPP synthase gene, a primer pair was used in which one primer anneals to a site within the *psbD* 5'UTR (SEQ ID NO. 62) and the other primer anneals within the FPP synthase coding segment (SEQ ID NO. 66). To identify strains that contain the sesquiterpene synthase gene, a primer pair was used in which one primer anneals to a site within the *psbD* 5'UTR (SEQ ID NO. 62) and the other primer anneals within the sesquiterpene synthase coding segment (SEQ ID NO. 70). Desired clones are those that yield a PCR product of expected size in both reactions. To determine the degree to which the endogenous gene locus is displaced (heteroplasmic vs. homoplasmic), a PCR reaction consisting of two sets of primer pairs were employed (in the same reaction). The first pair of primers amplifies the endogenous locus targeted by the expression vector (SEQ ID NOs. 68 and 69). The second pair of primers (SEQ ID NOs. 59 and 60) amplifies a constant, or control region that is not targeted by the expression vector, so should produce a product of expected size in all cases. This reaction confirms that the absence of a PCR product from the endogenous locus did not result from cellular and/or other contaminants that inhibited the PCR reaction. Concentrations of the primer pairs are varied so that both reactions work in the same tube; however, the pair for the endogenous locus is 5X the concentration of the constant pair. The number of cycles used was >30 to increase sensitivity. The most desired clones are those that yield a product for the constant region but not for the endogenous gene locus. Desired clones are also those that give weak-intensity endogenous locus products relative to the control reaction.

To ensure that the presence of the FPP synthase and sesquiterpene synthase genes led to expression of the FPP synthase and sesquiterpene synthase enzymes, a Western blot was performed. Approximately 1x10⁸ algae cells were collected from TAP agar medium and suspended in 0.05 ml of lysis buffer (Bugbuster; Novagen). Solutions were heated to 95°C for 5 min and then cooled to 23°C. Lysate was mixed 3:1 with loading buffer (XT Sample buffer; Bio-Rad), samples were heated to 95°C for 1 min, cooled to 23 °C, and insoluble proteins were removed by centrifugation. Soluble proteins were separated by SDS-PAGE, followed by transfer to PVDF membrane. The membrane was blocked with TBST + 5% dried, nonfat milk at 23°C for 30 min, incubated with anti-FLAG antibody (diluted 1:2,500 in TBST + 5% dried, nonfat milk) at 4°C for 10 hours, washed three times with TBST, incubated with horseradish-linked anti-mouse antibody (diluted 1:5,000 in TBST + 5% dried, nonfat milk) at 23°C for 1 hour, and washed three times with TBST. Proteins were visualized with chemiluminescent detection. Results from multiple clones (FIG. 12) show expression of the FPP synthase gene in *C. reinhardtii* cells resulted in production of the protein.

Cultivation of *C*. *reinhardtii* transformants for expression of FPP synthase and sesquiterpene synthase was carried out in liquid TAP medium at 23°C under constant illumination of 5,000 Lux on a rotary shaker set at 100 rpm, unless stated otherwise. Cultures were maintained at a density of 1x10⁷ cells per ml for at least 48 hr prior to harvest.

To determine whether FPP synthase and sesquiterpene synthase produced in the algae chloroplast are functional, sesquiterpene production from DMAPP and IPP is examined. Briefly, 50 mL of algae cell culture is harvested by centrifugation at 4000xg at 4°C for 15 min. The supernatant is decanted and the cells resuspended in 0.5 mL of reaction buffer (25 mM HEPES, pH=7.2, 100 mM KCI, 10 mM MnCl2, 10% glycerol, and 5 mM DTT). Cells are lysed by sonication (10x30sec at 35% power). 0.33 mg/mL of FPP are added to the lysate and the mixture transferred to a glass vial. The reaction is overlaid with heptane and incubated at 23°C for 12 hours. The reaction is quenched and extracted by vortexing the mixture. 0.1 mL of heptane is removed and the sample analyzed by gas chromatography - mass spectrometry (GC-MS).

### Example 7. Production of diterpene molecules in C. reinhardtii

In this example, strains of *C. reinhardtii* were engineered to express FPP synthase from *G*. *gallus* and squalene synthase from *S. aureus* (as described above). Cultivation of *C. reinhardtii* transformants for expression of FPP synthase and squalene synthase was carried out in liquid HSM medium at 23°C under constant illumination of 5,000 Lux on a rotary shaker set at 100 rpm, unless stated otherwise.

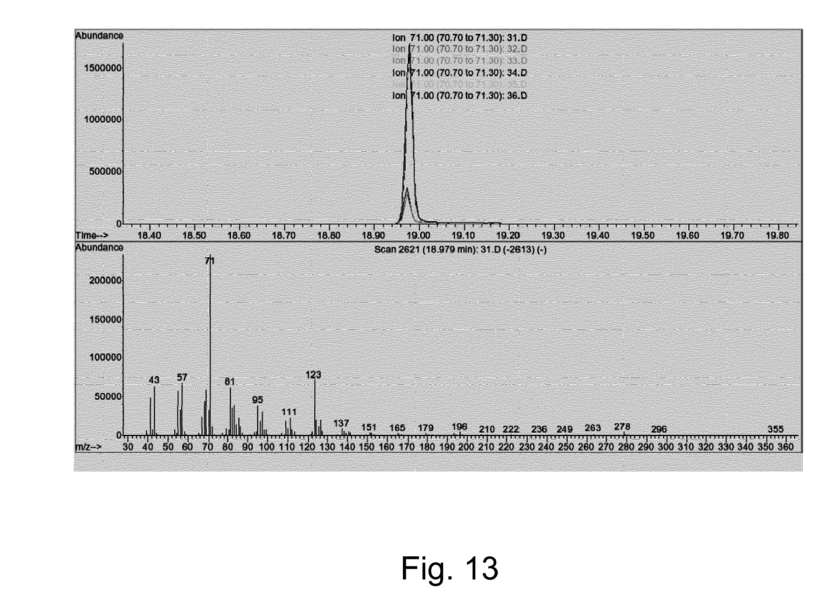

To determine if expression of either enzyme impacts the metabolic pathways that produce diterpenes, phytol production was examined. Briefly, 800 mL of algae cell culture was harvested by centrifugation at 4000xg at 4°C for 5 min. The supernatant was poured off and the remaining cell pellet resuspended in 10 ml of HSM medium and transferred to 50mL centrifuge tube. Cells were pelleted again by centrifugation at 3,000 RPM for 5 minutes. All of the supernatant was removed and the cell mass determined. Samples were maintained at 23°C and cell pellets resuspended in MeOH:KOH (1:10) at a ratio of 0.75 mg biomass:5mL MeOH. A solvent blank consisting of 30mL MeOH:KOH (1:10) was stored in a 50mL conical vial to control for leaching. Cell pellets were solublized by repeated pipetting. Lysates were heated to 55°C for 30 minutes (shaken at 10 minute intervals to ensure complete mixing). Lysates were cooled to approximately 23°C and 4 mL of each samples was transferred to amber glass vials and overlaid with 8 mL of heptane and mixed for 10-12 hours at 23°C on a rotating wheel. 100 uL of heptane was collected. Analysis was performed on GC-MS. Results are shown in **FIG. 13****.** The results show that expression of these enzymes increases the production of phytol in *C. reinhardtii.*

### Example 8. Production of enzymes comprising a monoterpene biosynthesis pathway in Escherichia coli.

In this example a nucleic acids encoding GPP synthase from *A. thaliana* and limonene synthase from *M. spicata* were introduced into *E. coli* BL-21 cells. In this instance the gene encoding GPP synthase (SEQ ID NO. 89, Table 5; SEQ ID NO. 142, Table 6) and the gene encoding limonene synthase (SEQ ID NO. 74, Table 5; SEQ ID NO. 127, Table 6) were each ligated into the plasmid pET-21a using the *Nde*I and *Xho*I sites. The resulting plasmid was transformed into *E. coli* BL-21 cells. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Expression of the synthases was induced when cell density reached OD=0.6. Cells were grown at 30°C for 5 hours and then harvested. To ensure that the presence of the GPP synthase and limonene synthase genes led to expression of the enzymes, a Western blot was performed essentially as described above. Results **(****FIG. 14****;** lane 1: MW ladder; lane 2: GPP synthase; and lane 3: limonene synthase) show expression of the GPP synthase and limonene synthase proteins in *E. coli* cells.

To determine whether the enzymes were functional, limonene production from IPP and DMAPP was examined. Briefly, 500 ml of *E. coli* cell culture was harvested by centrifugation at 4000xg at 4°C for 15 min. The supernatant was decanted and the cells resuspended in 10 ml of lysis buffer (100 mM Tris-HCl, pH=8.0, 300 mM NaCl, 2% Tween-20). Cells were lysed by sonication (3x30sec at 35% power). Lysate was clarified by centrifugation at 14,000xg at 4°C for 1 hour. The supernatant was removed and incubated with anti-FLAG antibody-conjugated agarose resin at 4°C for 10 hours. Resin was separated from the lysate by gravity filtration and washed 3x with wash buffer (100 mM Tris-HCl, pH=8.0, 300 mM NaCl, 2% Tween-20). Enzymes were eluted from the resin with elution buffer (100 mM Tris-HCl, pH=8.0, 300 mM NaCl, 250 µg/mL FLAG peptide).

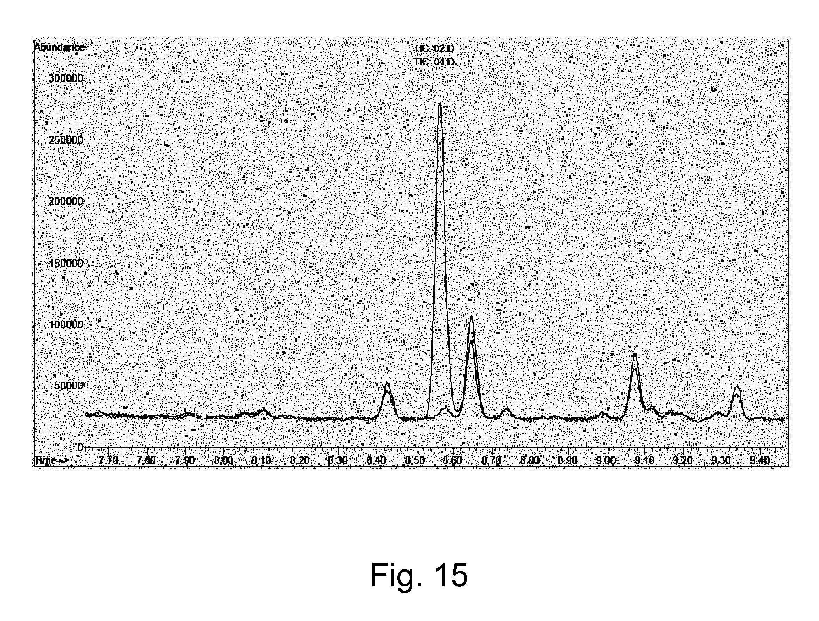

Reactions were carried out in reaction buffer (25 mM HEPES, pH=7.2, 100 mM KCI, 10 mM MnCl2, 10% glycerol, and 5 mM DTT), with or without the addition of limonene synthase, and IPP and DMAPP. The reaction was overlaid with heptane and incubated at 23°C for 12 hours. The reaction was quenched and extracted by vortexing the mixture. 0.1 mL of heptane is removed and the sample analyzed by gas chromatography - mass spectrometry (GC-MS). Results are shown in **FIG. 15****.** A large peak resulted in the reaction containing the limonene synthase isolated from the transformed *E. coli* strain.

### Example 9. Nuclear transformation of C. reinhardtii with a nucleic acid encoding a fused resistance marker and gene of interest.

In this example, a nucleic acid encoding xylanase 2 from *T. reesei* is introduced into *C. reinhardtii.* Transforming DNA is shown graphically in **FIG. 16A****.** The segment labeled "Transgene" is xylanase 2 encoding gene, the segment labeled "Promoter / 5' UTR" is the *C. reinhardtii* HSP70 / rbcS2 5' UTR with introns, the segment labeled "Selectable Marker" is a bleomycin resistance gene, the segment labeled CM (cleavage moiety) is the A2 viral protease of foot and mouth disease virus (FMDV), and the segment labeled 3' UTR is the 3'UTR from *C. reinhardtii* rbcS2. The bleomycin resistance gene, A2 and xylanase 2 coding regions are physically linked in-frame, resulting in a chimeric single ORF. A Metal Affinity Tag (MAT) and FLAG epitope tag were added to the 3' end of the ORF, using standard techniques. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations are carried out on cell-wall-deficient C. *reinhardtii* strain CC3395 (an arginine auxotrophic mutant mt-). Cells were grown and transformed via electroporation. Cells are grown to mid-log phase (approximately 2-6 x 10⁶ cells/ml). Tween-20 was added into cell cultures to a concentration of 0.05% before harvest to prevent cells from sticking to centrifugation tubes. Spin cells down gently (between 2000 and 5000g) for 5 min. The supernatant was removed and cells resuspended in TAP+40 mM sucrose media. 1 to 2 ug of transforming DNA was mixed with ∼ 1 x 10⁸ cells on ice and transferred to electroporation cuvettes. Electroporation was performed with the capacitance set at 25 uF, the voltage at 800 V to deliver V/cm of 2000 and a time constant for 10-14 ms. Following electroporation, the cuvette is returned to room temperature for 5-20 min. Cells were transferred to 10 ml of TAP+40 mM sucrose + 50 ug/ml arginine and allowed to recover at room temperature for 12-16 hours with continuous shaking. Cells were then harvested by centrifugation at between 2000g and 5000g and resuspended in 0.5 ml TAP+40 mM sucrose medium. 0.25 ml of cells were plated on TAP + 100 ug/ml bleomycin + 50 ug/ml arginine. All transformations were carried out under bleomycin selection (100 µg/ml) in which resistance was conferred by the gene encoded by the segment in **FIG. 16A** labeled "Selection Marker." Transformed strains are maintained in the presence of bleomycin to prevent loss of the exogenous DNA.

Colonies growing in the presence of bleomycin were screened by dot blot. Briefly, colonies were lysed by BugBuster Protein Extraction Reagent (Novagen) and MAT-tagged proteins were separated using Co2+ magnetic beads (Invitrogen), according to manufacturer's instructions. After exposure to the proteins, the beads were washed three times by 150 ul of 1X Tris Buffered Saline with 0.05% Tween-20 (TBST) at room temperature. Proteins were released from beads by 150 ul 10 uM EDTA, 25 mM Tris-HCl pH 7.0, 400 mM NaCl, and the 150 ul eluates were dot blotted onto nitrocellulose membranes. Membranes were blocked by Starting Block (TBS) blocking buffer (Thermo Scientific) and probed for one hour with mouse anti-FLAG antibody-horseradish peroxidase conjugate (Sigma) diluted 1:3000 in Starting Block buffer. After probing, membranes were washed four times with TBST, then developed with Supersignal West Dura chemiluminescent subrate (Thermo Scientific) and imaged using a CCD camera (Alpha Innotech). Colonies showing positive results in the dot blot analysis are then screened by western blotting.

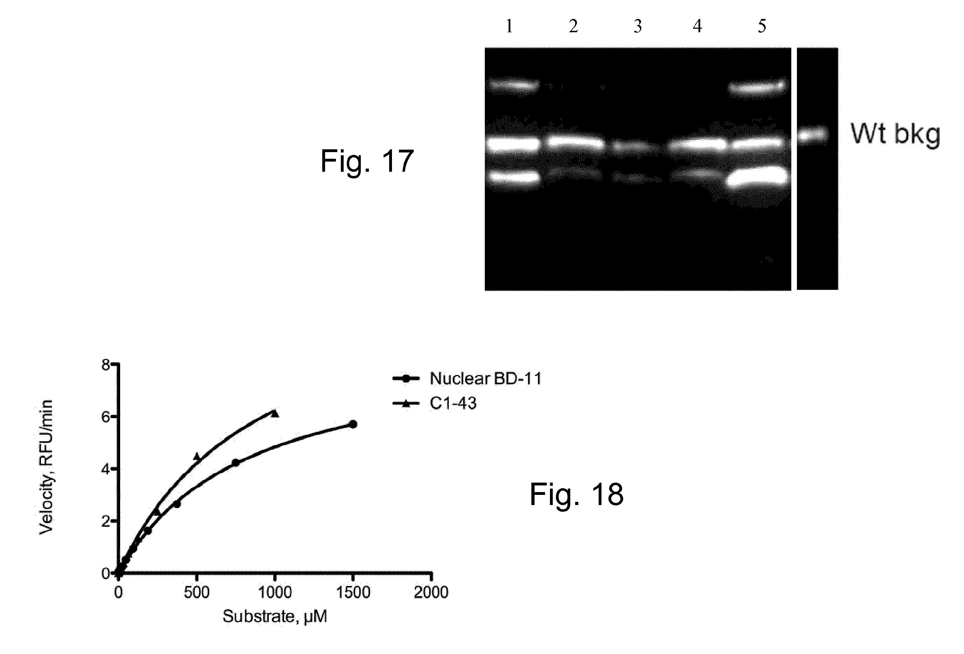

Patches of algae cells growing on TAP agar plates were lysed by resuspending cells in 50 ul of 1X SDS sample buffer with reducing agent (BioRad). Samples were then boiled and run on a 10% Bis-tris polyacrylamide gel (BioRad) and transferred to PVDF membranes using a Trans-blot semi-dry blotter (BioRad) according to manufacturers instructions. Membranes were blocked by Starting Block (TBS) blocking buffer (Thermo Scientific) and probed for one hour with mouse anti-FLAG antibody-horseradish peroxidase conjugate (Sigma) diluted 1:3000 in Starting Block buffer. After probing, membranes were washed four times with TBST, then developed with Supersignal West Dura chemiluminescent subrate (Thermo Scientific) and imaged using a CCD camera (Alpha Innotech). Results from 5 colonies (and wild-type control) are shown in **FIG 17****.** Positive colonies show a band with a lower molecular weight than the WT background. A small amount of intact fusion (Bleomycin resistance marker fused to xylanase) is translated by the cells; as the resistance marker forms a dimer, these products migrate at a higher molecular weight. The results indicate that xylanase 2 is being produced from the strains.

To determine whether xylanase produced is functional, enzyme activity is examined. Patches of cells were homogenized by 50 ul by BugBuster Protein Extraction Reagent (Novagen) and EnzCheck Ultra Xylanase Assay Kit (Molecular Probe) was used to examine xylanase activity according to manufacturer's instructions.

Results are shown in **FIG. 18** and are compared with xylanase isolated from a C. *reinhardtii* strain producing exogenous xylanase from a transformed chloroplast.

Similar protocols for plasmid construction, transformation, colony selection Western blot, and enzyme analysis were performed with each of the enzymes listed in Table 3. For each of these enzymes, experiments were repeated where the fusion protein was prepared with the *C. reinhardtii* carbonic anhydrase secretion signal.

**Table 3. Select enzymes expressed from the nucleus in C. reinhardtii**

| Enzyme | Source |
|---|---|
| CBH1 | T. viride |
| CBHII | T. Reesei |
| CBH1 | A. aculeatus |
| Endoglucanase I | T. Reesei |
| Endoglucanase III | T. Reesei |
| Endoglucanase V | T. reesei |
| Endoglucanase A | A. niger |
| beta-D-glucoside glucohydrolase | T. reesei |
| beta-glucosidase | T. reesei |
| Beta glucosidase | A. niger |
| Xylanase 2 | T. reesei |
| Xylanase 1 | T. reesei |
| FPP synthase | Chicken |
| Squalene desaturase | S. aureus |

### Example 10. Nuclear transformation of C. reinhardtii with a nucleic acids encoding a resistance marker and a gene of interest.

In this example, a nucleic acid encoding endoglucanase from *T. reesei* is introduced into *C. reinhardtii.* Transforming DNA is shown graphically in **FIG. 16****B.** The segment labeled "Transgene" is the endoglucanase encoding gene, the segment labeled "Promoter / 5' UTR" is the *C. reinhardtii* HSP70 / rbcS2 5' UTR, the segment labeled "Selectable Marker" is a hygromycin resistance gene (should we include statement regarding non-functional paromomycin resistance gene?maybe not I think), and the segment labeled 3' UTR is the 3'UTR from *C. reinhardtii* rbcS2. The hygromycin resistance gene and endoglucanase coding regions are expressed separately with hygromycin marker driven by a beta 2-tubulin promoter Endoglucanase coding sequence was fused to a DNA fragment that encodes a *C. reinhardtii* carbonic anhydrase secretion signal. A Metal Affinity Tag (MAT) and FLAG epitope tag were added to the 3' end of the ORF, using standard techniques. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations are carried out on cell-wall strain *C. reinhardtii* strain CC1960 (mt+), essentially as described above. All transformations were carried out under hygromycin selection (20 µg/ml) in which resistance was conferred by the gene encoded by the segment in **FIG. 16B** labeled "Selection Marker." Transformed strains are maintained in the presence of hygromycin.

Colonies growing in the presence of hygromycin were screened by dot blot. Briefly, colonies were lysed and MAT-tagged proteins were purified for dot blots as previously described. After exposure to the proteins, the beads were washed two times with 150 ul of 1X TBST and one time with 150 ul of 100 mM Tris-HCl pH 7.5, 400 mM NaCl, and 20 mM Imidazole at room temperature. Proteins were released from beads by 150 ul 10 uM EDTA, 25 mM Tris-HCl pH 7.0, 400 mM NaCl and the 150 ul eluates were dot blotted onto nitrocellulose membranes. Membranes were blocked and probed with anti-FLAG antibody as previously described.

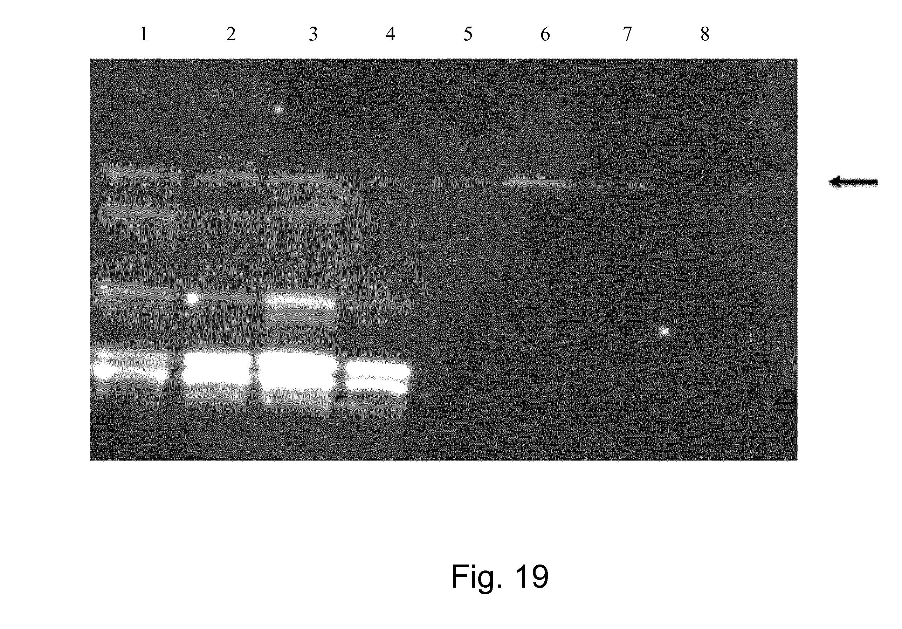

Colonies showing positive results in the dot blot analysis are then screened by western blotting. 1 x 10⁸ cells from log phase liquid cultures were harvested at 3000xg at for 5 min. The supernatant was decanted and the cells resuspended in 1 ml of protein binding buffer (100 mM Tris-HCl, pH=7.5, 400 mM NaCl, 20 mM Imidazole). Cells were lysed by vortexing in the presence of 500 ul zirconium beads at the highest speed (1 mm, BioSpec Products, Inc.). Lysate was clarified by centrifugation at 2400 g at 4°C for 1 min. The supernatant was removed and incubated with Ni2+ agarose resin (Invitrogen) at 4°C for 1 hour. Resin was separated from the lysate by centrifugation at 2400 g and washed 3x with protein binding buffer (100 mM Tris-HCl, pH=7.5, 400 mM NaCl, 20 mM Imidazole). Proteins were eluted by incubation of the resin with 100 ul elution buffer (25 mM Tris-HCl, pH=7.5, 400 mM NaCl, 20 mM EDTA). Results from 3 colonies (and wild-type control) from samples bound to the resin (lanes 1-4) and samples after elution (lanes 5-8) are shown in **FIG 19****.** Positive colonies show a band (indicated by arrow) that is missing in wild type control. The results indicate that endo-glucanase is being produced from the strains.

### Example 11. Nuclear transformation of C. reinhardtii with a nucleic acids encoding a resistance marker and a gene of interest.

In this example, a nucleic acid encoding CBH1 from *A. aculeatus* is introduced into *C. reinhardtii.* Transforming DNA is shown graphically in **FIG. 16****B.** The segment labeled "Transgene" is the exoglucanase encoding gene, the segment labeled "Promoter / 5' UTR" is the *C. reinhardtii* HSP70 / rbcS2 5' UTR, the segment labeled "Selectable Marker" is a hygromycin resistance gene, and the segment labeled 3' UTR is the 3'UTR from *C. reinhardtii* rbcS2. The hygromycin resistance gene and CBH1 coding regions were expressed separately with hygromycin marker driven by a beta-2 tubulin promoter. CBH1 coding sequence was fused to a DNA fragment that encodes a *C. reinhardtii* carbonic anhydrase secretion signal. A Metal Affinity Tag (MAT) and FLAG epitope tag were added to the 3' end of the ORF, using standard techniques. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations are carried out on cell-wall strain *C. reinhardtii* strain CC1960 (mt+), essentially as described above. All transformations were carried out under hygromycin selection (20 µg/ml) in which resistance was conferred by the gene encoded by the segment in **FIG. 16B** labeled "Selection Marker." Transformed strains are maintained in the presence of hygromycin.

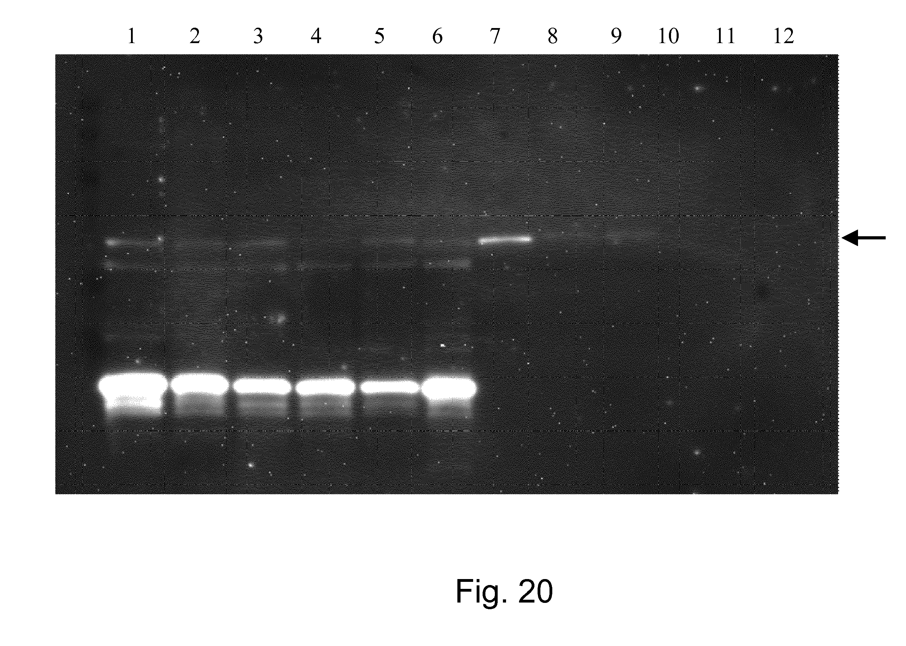

Colonies growing in the presence of hygromycin were screened by pull-down assay followed by Western blot analysis. Four ml of liquid cultures was harvested at 3000xg at for 5 min. The supernatant was decanted and the cells resuspended in 1 ml of protein binding buffer (100 mM Tris-HCl, pH=7.5, 400 mM NaCl, 20 mM Imidazole, 0.005% NP40). The protein purification and Western blot analysis were carried out as described above. Results from 5 colonies (and wild-type control) from samples bound to the resin (lanes 1-5) and samples after elution (lanes 7-11) are shown in **FIG 20****.** Positive colonies (7, 8, 9) show a band (indicated by arrow) that is missing in wild type control. The results indicate that CBH1 is being produced from the strains.

### Example 12. Nuclear transformation of C. reinhardtii with a nucleic acids encoding a resistance marker and a secreted gene of interest.

In this example, a nucleic acid encoding Xylanase 2 from *T. reesei* is introduced into *C. reinhardtii.* Transforming DNA is shown graphically in **FIG. 16A****.** The segment labeled "Transgene" is xylanase 2 encoding gene, the segment labeled "Promoter / 5' UTR" is the *C. reinhardtii* HSP70 / rbcS2 5' UTR with introns (intron position not indicated), the segment labeled "Selectable Marker" is a bleomycin resistance gene, the segment labeled CM (cleavage moiety) is the A2 viral protease of foot and mouth disease virus (FMDV), and the segment labeled 3' UTR is the 3'UTR from *C. reinhardtii* rbcS2. The bleomycin resistance gene, A2 and xylanase 2 coding regions are physically linked in-frame, resulting in a chimeric single ORF. Nucleic acids encoding the secretion signal sequence of the *Chlamydomonas* carbonic anhydrase gene were placed between the A2 sequence and the 5'end of the xylanase encoding sequence. A Metal Affinity Tag (MAT) and FLAG epitope tag were added to the 3' end of the ORF, using standard techniques. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

For these experiments, all transformations were carried out on cell-wall-deficient C. *reinhardtii* strain CC3395 (an arginine auxotrophic mutant mt-). Cells were grown and transformed via electroporation. Cells are grown to mid-log phase (approximately 2-6 x 10⁶ cells/ml). Tween-20 was added into cell cultures to a concentration of 0.05% before harvest to prevent cells from sticking to centrifugation tubes. Spin cells down gently (between 2000 and 5000g) for 5 min. The supernatant was removed and cells resuspended in TAP+40 mM sucrose media. 1 to 2 ug of transforming DNA was mixed with ∼ 1 x 10⁸ cells on ice and transferred to a electroporation cuvettes. Electroporation was performed with the capacitance set at 25 uF, the voltage at 800 V to deliver V/cm of 2000 and a time constant for 10-14 ms. Following electroporation, the cuvette is returned to to room temperature for 5-20 min. Cells were transferred to 10 ml of TAP+40 mM sucrose + 50 ug/ml arginine and allowed to recover at room temperature for 12-16 hours with continuous shaking. Cells were then harvested by centrifugation at between 2000g and 5000g and resuspendeded in 0.5 ml TAP+40 mM sucrose medium. 0.25 ml of cells were plated on TAP + 100 ug/ml bleomycin + 50 ug/ml arginine. All transformations were carried out under bleomycin selection (100 µg/ml) in which resistance was conferred by the gene encoded by the segment in FIG. 16A labeled "Selection Marker." Transformed strains are maintained in the presence of bleomycin to prevent loss of the exogenous DNA.

Colonies growing in the presence of bleomycin were screened by dot blot. Briefly, colonies were lysed by BugBuster Protein Extraction Reagent (Novagen) and MAT-tagged proteins were separated using Co2+ magnetic beads (Invitrogen), according to manufacturers instructions. After exposure to the proteins, the beads were washed three times by 150 ul of 1X Tris Buffered Saline with 0.05% Tween-20 (TBST) at room temperature. Proteins were released from beads by 150 ul 10 mM EDTA, 25 mM Tris-HCl pH 7.0, 400 mM NaCl, and the 150 ul eluates were dot blotted onto nitrocellulose membranes. Membranes were blocked by Starting Block (TBS) blocking buffer (Thermo Scientific) and probed for one hour with mouse anti-FLAG antibody-horseradish peroxidase conjugate (Sigma) diluted 1:3000 in Starting Block buffer. After probing, membranes were washed four times with TBST, then developed with Supersignal West Dura chemiluminescent subrate (Thermo Scientific) and imaged using a CCD camera (Alpha Innotech). Colonies showing positive results in the dot blot analysis are then screened by western blotting. Algae cells were cultured at a volume of 50ml, and carefully centrifuged to avoid cell lysis. Cobalt-derivatized magnetic beads (Invitrogen) were added to the conditioned media, and incubated, and washed according to manufacturer instructions. Proteins were released from beads by 150 ul 10 mM EDTA, 25 mM Tris-HCl pH 7.0, 400 mM NaCl, and 50ul of 4X SDS sample buffer with reducing agent (BioRad) was added to the eluates. Samples were then boiled and run on a 10% Bis-tris polyacrylamide gel (BioRad) and transferred to PVDF membranes using a Trans-blot semi-dry blotter (BioRad) according to manufacturers instructions. Membranes were blocked by Starting Block (TBS) blocking buffer (Thermo Scientific) and probed for one hour with mouse anti-FLAG antibody-horseradish peroxidase conjugate (Sigma) diluted 1:3000 in Starting Block buffer. After probing, membranes were washed four times with TBST, then developed with Supersignal West Dura chemiluminescent subrate (Thermo Scientific) and imaged using a CCD camera (Alpha Innotech).

**Table 4. PCR Primers**

| SEQ ID NO. | Use | Sequence |
|---|---|---|
| 55 | psbA 5' UTR forward primer | GTGCTAGGTAACTAACGTTTGATTTTT |
| 56 | Limonene synthase reverse primer (155) | TGGGTTCATATCTGGACGTT |
| 57 | *psbA* 5' UTR forward primer (wild-type) | GGAAGGGGACGTAGGTACATAAA |
| 58 | *psbA* 3' reverse primer (wild-type) | TTAGAACGTGTTTTGTTCCCAAT |
| 59 | Control forward primer | CCGAACTGAGGTTGGGTTTA |
| 60 | Control reverse primer | GGGGGAGCGAATAGGATTAG |
| 61 | GPP synthase reverse primer (281) | GCAACTTCAGCTGTTTGACCT |
| 62 | *psbD* 5' UTR forward primer | AAATTTAACGTAACGATGAGTTG |
| 63 | *psbA* 3' reverse primer (wild-type) | TTAGAACGTGTTTTGTTCCCAAT |
| 64 | *psbC* 5' UTR forward primer | TGGTACAAGAGGATTTTTGTTGTT |
| 65 | *psbD* 5' UTR forward primer | AAATTTAACGTAACGATGAGTTG |
| 66 | FPP synthase reverse primer (163) | CGTTCTTCTGAGAAATGGCTTA |
| 67 | Zingiberene synthase reverse primer (293) | ATTAGCATCAGAGCCGCATT |
| 68 | 3HB forward primer (wild-type) | CTCGCCTATCGGCTAACAAG |
| 69 | 3HB forward primer (wild-type) | CACAAGAAGCAACCCCTTGA |
| 70 | Sesquiterpene synthase reverse primer (298) | CGACGGAATAAAGGTGTACGA |
| 71 | *psbC* 5' UTR forward primer | TGGTACAAGAGGATTTTTGTTGTT |
| 72 | Squalene synthase reverse primer (166) | TCAGCAATTGCAGCGTAATA |
| 73 | Bisabolene synthase reverse primer (307) | GACGTTCTTGACGTTTTGTTTG |

**Table 5. Nucleic acids encoding exemplary isoprenoid producing enzymes.**

| **SEQ ID NO** | **Codon-biased, Synthesized Gene Sequence** | **Enzyme (synthase) encoded** |
|---|---|---|
| 74 | ATGGTACCAAGACGTTCAGGTAACTATAATCCTAGCCGTTGGGACGT AAATTTCATTCAATCTTTATTATCTGATTATAAAGAAGATAAACACG TTATTAGAGCTTCTGAATTAGTAACACTTGTTAAGATGGAATTAGAA AAAGAAACAGATCAAATCCGTCAATTAGAATTAATTGACGATTTAC AACGTATGGGTTTATCTGATCATTTCCAAAACGAATTTAAAGAAATC TTATCAAGTATTTACTTAGATCATCATTATTACAAAAATCCATTTCCA AAAGAAGAGCGTGATTTATACTCAACTAGCTTAGCTTTTCGTTTATT ACGTGAACACGGTTTTCAAGTAGCACAAGAAGTTTTTGATTCATTCA AAAATGAAGAGGGTGAATTTAAGGAGAGCTTATCTGACGATACTCG TGGCTTATTACAATTATATGAAGCATCATTCTTATTAACAGAGGGTG AAACAACCTTAGAAAGTGCACGCGAATTTGCTACAAAATTTTTAGA AGAAAAAGTTAACGAAGGTGGCGTTGATGGTGACTTATTAACAAGA ATTGCTTACTCATTAGATATTCCCTTACATTGGCGCATTAAACGTCCT AATGCCCCAGTTTGGATTGAATGGTATCGTAAACGTCCAGATATGAA CCCAGTGGTTTTAGAATTAGCAATTTTAGACTTAAACATTGTACAAG CTCAATTTCAAGAGGAATTAAAAGAGTCTTTTCGCTGGTGGCGTAAT ACTGGTTTTGTTGAGAAATTACCATTTGCACGTGATCGTTTAGTTGA ATGTTACTTTTGGAACACTGGTATTATTGAACCACGTCAACACGCAT CAGCTCGTATTATGATGGGTAAAGTAAATGCATTAATTACAGTAATT GATGACATCTATGATGTTTATGGAACACTTGAAGAATTAGAACAATT CACTGATTTAATTCGCAGATGGGACATAAACTCAATAGATCAATTAC CAGATTATATGCAATTATGTTTTCTTGCATTAAACAATTTCGTTGATG ACACTTCATACGATGTTATGAAAGAAAAGGGTGTTAATGTTATTCCT TACTTACGTCAATCTTGGGTAGACCTTGCAGACAAATATATGGTAGA AGCACGTTGGTTCTACGGTGGCCATAAACCATCATTAGAAGAATACT TAGAAAATTCTTGGCAATCTATCTCAGGTCCATGTATGTTAACTCAT ATATTCTTTCGTGTAACAGATAGCTTTACTAAAGAAACTGTTGATTC TCTTTACAAATATCATGATTTAGTTAGATGGTCATCATTCGTGCTTCG TCTTGCTGACGACTTAGGTACAAGCGTTGAAGAAGTATCTCGTGGTG ATGTGCCAAAATCTTTACAATGCTACATGAGTGATTATAACGCTAGT GAGGCTGAAGCACGTAAACACGTAAAATGGTTAATTGCAGAAGTAT GGAAAAAGATGAATGCAGAACGTGTTTCTAAAGATAGTCCTTTTGGT AAAGATTTTATAGGTTGTGCTGTTGATTTAGGTCGTATGGCTCAATT AATGTATCACAATGGAGATGGTCATGGTACTCAACACCCTATTATTC ATCAACAAATGACACGTACTTTATTTGAACCATTCGCTGGTACCGGT GAAAACTTATACTTTCAAGGCTCAGGTGGCGGTGGAAGTGATTACA | Limonene (M. spicata) |
| | AAGATGATGATGATAAAGGAACCGGTTAA | |
| 75 | ATGGTACCAAGACGTACTGGTGGCTATCAACCTACACTTTGGGATTT TTCAACAATTCAATTATTTGATAGTGAATATAAAGAAGAAAAACATC TTATGCGTGCTGCTGGTATGATTGCTCAAGTGAACATGTTACTTCAA GAAGAAGTAGACAGCATCCAACGTCTTGAATTAATTGATGACTTAC GTCGTTTAGGTATATCTTGCCACTTTGATCGTGAAATTGTAGAGATTT TAAACAGTAAATACTACACCAACAATGAAATTGATGAATCAGATTT ATACAGTACAGCACTTAGATTCAAACTTTTACGTCAATATGATTTTA GCGTTAGCCAAGAAGTTTTTGATTGTTTTAAAAATGACAAAGGTACA GATTTCAAACCATCATTAGTTGACGATACACGTGGCTTATTACAATT ATATGAAGCATCATTTTTATCAGCTCAGGGTGAAGAAACTTTACATT TAGCACGTGATTTTGCTACTAAATTCTTACATAAAAGAGTTTTAGTA GATAAAGATATCAATTTATTATCTAGTATCGAGCGTGCTTTAGAATT ACCAACACACTGGCGTGTACAAATGCCTAACGCTAGATCATTCATCG ACGCATATAAAAGAAGACCAGACATGAACCCTACAGTATTAGAGTT AGCAAAACTTGACTTTAACATGGTTCAAGCACAGTTCCAACAAGAA TTAAAAGAAGCCAGTCGCTGGTGGAACTCTACAGGATTAGTACATG AATTACCATTTGTACGTGATCGTATTGTGGAATGTTATTATTGGACT ACTGGTGTAGTAGAACGTCGTGAACACGGTTACGAACGTATTATGTT AACAAAAATTAACGCTTTAGTTACAACAATCGATGATGTTTTTGACA TTTATGGTACTTTAGAAGAATTACAACTTTTTACAACTGCTATTCAA AGATGGGACATTGAGTCTATGAAACAACTTCCACCCTATATGCAAAT CTGCTACTTAGCTTTATTCAACTTCGTAAATGAGATGGCTTACGATA CATTACGTGATAAAGGTTTTAATAGTACTCCATATTTACGCAAAGCC TGGGTAGACTTAGTAGAAAGCTACTTAATTGAAGCTAAATGGTATTA TATGGGTCACAAACCAAGTTTAGAAGAGTACATGAAAAACTCATGG ATTTCTATCGGAGGTATTCCAATTTTATCACATTTATTCTTTCGTTTA ACAGACAGTATCGAAGAAGAAGACGCTGAATCAATGCATAAATATC ACGATATAGTACGTGCCTCTTGTACTATTTTACGTTTAGCTGATGATA TGGGTACATCATTAGATGAAGTTGAACGTGGCGATGTTCCTAAATCT GTACAATGCTATATGAATGAGAAAAACGCCTCTGAAGAAGAAGCAC GTGAACATGTTCGTAGTTTAATTGATCAGACATGGAAGATGATGAAT AAAGAAATGATGACTTCATCATTTTCAAAATACTTCGTACAAGTGTC TGCAAATCTTGCTCGTATGGCACAATGGATATATCAACATGAAAGTG ATGGTTTCGGTATGCAACACTCTTTAGTTAACAAAATGCTTCGTGGT TTACTTTTTGACCGTTATGAAGGTACCGGTGAAAACTTATACTTTCA AGGCTCAGGTGGCGGTGGAAGTGATTACAAAGATGATGATGATAAA GGAACCGGTTAA | Cineole (S. officinalis) |
| 76 | ATGGTACCACGTCGCATGGGTGATTTTCATTCAAACTTATGGATGA TGATGTAATTCAATCTTTACCCACAGCTTACGAAGAAAAATCTTATC | Pinene (A. grandis) |
| | TTGAACGTGCTGAGAAGTTAATTGGAGAAGTTGAAAATATGTTCAA CAGTATGAGTTTAGAAGATGGTGAACTTATGAGTCCATTAAATGATT TAATTCAACGCCTTTGGATTGTTGATTCTTTAGGTAGATTAGGTATCC ATCGTCACTTTAAAGATGAGATTAAAAGTGCTTTAGATTATGTTTAC AGTTACTGGGGTGAAAACGGAATAGGTTGTGGTCGTGAAAGTGCTG TAACTGATTTAAACAGTACAGCTTTAGGCTTTCGTACACTTCGTTTAC ACGGTTATCCAGTTTCATCTGATGTATTTAAAGCATTTAAAGGTCAA AATGGTCAATTCAGTTGTTCAGAAAATATCCAAACAGATGAAGAAA TTCGTGGTGTTCTTAACTTATTTAGAGCCAGTTTAATAGCCTTCCCTG GTGAGAAAATAATGGACGAAGCTGAAATCTTCTCTACAAAATACTT AAAGGAAGCATTACAAAAGATCCCAGTTAGTTCATTATCACGTGAA ATCGGTGATGTACTTGAATATGGATGGCATACATACTTACCACGTTT AGAAGCACGTAACTATATTCATGTTTTCGGACAAGATACAGAGAAT ACAAAAAGTTATGTAAAATCAAAGAAACTTTTAGAATTAGCTAAAT TAGAATTTAACATTTTTCAGAGCTTACAAAAACGTGAATTAGAAAGC CTTGTTCGTTGGTGGAAAGAATCTGGATTTCCTGAAATGACATTCTG TAGACACAGACACGTGGAATATTACACACTTGCATCATGTATTGCAT TCGAACCTCAGCATAGTGGTTTTCGTTTAGGTTTTGCTAAAACATGT CACCTTATAACAGTTTTAGATGACATGTATGACACTTTCGGCACCGT AGACGAATTAGAGTTATTTACAGCAACTATGAAACGTTGGGACCCA AGTTCAATTGACTGCCTTCCAGAATACATGAAAGGAGTTTACATTGC TGTGTATGATACAGTTAATGAAATGGCTCGTGAAGCTGAGGAAGCT CAAGGTCGCGATACACTTACATACGCTCGTGAGGCCTGGGAGGCTT ATATAGATTCTTATATGCAAGAAGCTCGCTGGATTGCTACTGGATAC TTACCTTCTTTCGATGAATATTATGAAAATGGTAAGGTTTCATGTGG TCACCGTATATCTGCTTTACAACCAATTCTTACTATGGATATTCCATT TCCAGATCACATTTTAAAGGAAGTTGACTTTCCTTCTAAACTTAATG ACTTAGCTTGTGCTATCTTACGCCTTCGCGGTGATACTCGTTGTTACA AAGCAGACCGTGCACGTGGTGAAGAGGCTAGTTCTATTTCTTGTTAT ATGAAAGATAATCCAGGTGTTTCTGAAGAAGATGCCTTAGATCATAT TAACGCAATGATCAGTGATGTTATTAAGGGCTTAAACTGGGAATTAC TTAAACCCGACATTAACGTACCTATTTCTGCTAAGAAACATGCTTTC GACATTGCTCGTGCTTTTCACTACGGTTATAAATATCGTGATGGCTA TTCAGTTGCTAATGTTGAAACAAAATCTTTAGTTACACGTACTTTACT TGAATCAGTTCCATTAGGTACCGGTGAAAACTTATACTTTCAAGGCT CAGGTGGCGGTGGAAGTGATTACAAAGATGATGATGATAAAGGAAC CGGTTAA | |
| 77 | ATGGTACCACGTAGAGTTGGTAATTATCATTCTAATCTTTGGATGA TGATTTTATACAAAGTTTAATTTCTACACCTTACGGTGCTCCTGACTA CCGTGAACGCGCTGATCGTCTTATTGGTGAAGTAAAAGATATTATGT | Camphene (A. grandis) |
| | TTAATTTCAAATCTTTAGAGGATGGTGGTAATGACTTATTACAACGT TTACTTTTAGTTGATGACGTAGAACGTTTAGGCATTGATCGTCATTTC AAAAAGGAAATTAAGACTGCATTAGATTATGTAAATAGTTATTGGA ATGAAAAAGGAATTGGTTGTGGTCGTGAGTCTGTAGTTACAGACTTA AATTCAACTGCTTTAGGCCTTCGTACCTTAAGATTACATGGTTATACT GTTAGCTCTGACGTTTTAAATGTTTTTAAAGATAAAAATGGTCAATT TTCATCTACAGCTAATATTCAAATTGAAGGTGAAATTCGTGGTGTTT TAAATCTTTTTCGTGCCTCTCTTGTAGCTTTTCCAGGTGAGAAAGTGA TGGATGAGGCTGAAACTTTTTCAACAAAATATCTTCGTGAAGCATTA CAGAAAATTCCTGCCAGTTCAATTTTATCATTAGAAATACGTGATGT ATTAGAATATGGATGGCATACTAATTTACCACGTTTAGAAGCACGTA ATTACATGGATGTTTTCGGTCAGCACACCAAGAACAAAAATGCAGC CGAAAAATTACTTGAATTAGCAAAATTAGAGTTCAATATCTTTCACA GCTTACAAGAACGTGAATTAAAGCACGTTTCAAGATGGTGGAAAGA CTCTGGTAGTCCAGAGATGACTTTCTGTCGCCACCGCCATGTGGAAT ATTATGCTTTAGCTTCTTGTATTGCTTTCGAACCCCAGCACAGTGGTT TCCGTTTAGGTTTTACTAAAATGAGTCATTTAATCACAGTGTTAGAT GATATGTATGATGTATTCGGTACAGTTGATGAATTAGAGTTATTTAC CGCCACTATTAAACGTTGGGACCCTTCTGCTATGGAATGTTTACCAG AGTACATGAAAGGTGTTTACATGATGGTTTATCATACAGTTAACGAA ATGGCTCGTGTGGCAGAAAAGGCTCAAGGTAGAGACACATTAAACT ATGCTCGTCAAGCCTGGGAAGCATGTTTTGACTCTTATATGCAAGAA GCAAAATGGATTGCAACAGGTTACTTACCTACATTCGAGGAATATTT AGAAAATGGTAAAGTGAGTTCAGCACATCGTCCTTGTGCATTACAAC CTATTTTAACTCTTGATATTCCATTTCCCGATCATATTCTTAAAGAAG TGGATTTCCCAAGCAAACTTAATGACTTAATTTGTATTATCTTACGTC TTAGAGGAGACACACGTTGCTATAAAGCAGACCGTGCCCGTGGTGA AGAAGCATCATCAATATCTTGTTATATGAAAGATAACCCAGGTTTAA CTGAAGAAGATGCTTTAAACCACATTAACTTTATGATTCGTGACGCA ATCCGCGAATTAAACTGGGAGTTACTTAAACCAGATAATAGTGTTCC AATTACTTCAAAGAAACATGCTTTTGATATTTCACGTGTGTGGCACC ACGGATACCGTTATCGTGATGGTTACAGCTTTGCAAACGTGGAAACT AAAAGTCTTGTAATGCGTACTGTAATAGAACCAGTACCATTAGGTAC CGGTGAAAACTTATACTTTCAAGGCTCAGGTGGCGGTGGAAGTGATT ACAAAGATGATGATGATAAAGGAACCGGTTAA | |
| 78 | ATGGTACCACGTCGTTCAGGAGATTATCAACCAAGTTTATGGACTT TAATTACATTCAATCTTTAAACACACCTTACAAAGAACAACGTCATT TTAATCGTCAAGCTGAGTTAATTATGCAAGTTCGTATGTTATTAAAG GTAAAAATGGAAGCAATTCAACAATTAGAGTTAATAGATGATTTAC AGTACTTAGGATTATCATATTTCTTTCAAGACGAAATTAAACAAATC | Sabinene (S. officinalis) |
| | TTAAGCTCTATTCACAATGAACCTCGTTATTTCATAATAATGACCTT TATTTCACTGCTTTAGGTTTAGAATTTACGTCAACATGGTTTAAT GTTTCAGAAGACGTATTTGACTGCTTTAAAATCGAAAAATGTTCTGA CTTTAATGCTAACTTAGCTCAGGACACAAAGGGTATGTTACAATTAT ATGAAGCTAGTTTCTTATTAAGAGAAGGAGAAGATACACTTGAATT AGCTCGTCGTTTTAGTACACGTTCTTTACGTGAAAAATTTGATGAAG GTGGTGACGAGATAGATGAAGATTTAAGTAGTTGGATTCGTCATTCT TTAGATTTACCATTACACTGGCGTGTTCAAGGTTTAGAAGCTCGTTG GTTTTAGATGCCTATGCTCGTCGTCCAGATATGAACCCTCTTATTTT CAAATTAGCTAAATTAAATTTTAACATTGTTCAAGCTACATACCAAG AAGAATTAAAAGACATCTCTCGTTGGTGGAACAGTAGTTGTTTAGCA GAGAAATTACCCTTCGTTCGCGATCGTATTGTAGAATGTTTCTTCTG GGCTATTGCTGCTTTCGAACCACACCAATACTCATATCAACGTAAAA TGGCCGCTGTAATTATTACATTTATTACTATTATTGATGATGTTTACG ATGTATATGGTACTATTGAAGAATTAGAGTTATTAACAGATATGATT CGTAGATGGGATAATAAGAGTATTAGTCAACTTCCTTACTATATGCA AGTTTGTTATTTAGCTCTTTATAACTTCGTAAGTGAACGCGCATACG ACATCTTAAAAGATCAACACTTTAACAGTATTCCATACCTTCAAAGA AGTTGGGTTTCATTAGTTGAGGGATACTTAAAAGAAGCATATTGGTA CTATAACGGTTACAAACCAAGTCTTGAAGAATATCTTAATAATGCAA AAATTAGTATTAGTGCACCCACCATTATTTCACAATTATACTTTACTT TAGCAAATAGTATCGACGAAACTGCCATTGAAAGTTTATACCAATAT CACAACATTTTATACTTATCAGGTACTATCTTACGTTTAGCTGATGAT TTAGGAACTTCACAACATGAATTAGAACGTGGTGATGTTCCCAAAGC TATTCAATGTTATATGAATGATACAAATGCATCAGAAAGAGAAGCT GTAGAACATGTTAAATTTCTTATTCGTGAAGCCTGGAAAGAAATGAA TACAGTTACTACCGCATCAGATTGTCCTTTTACAGACGATCTTGTTGC CGCCGCAGCTAATTTAGCTCGTGCTGCTCAATTCATTTACTTAGATG GTGATGGTCATGGTGTACAACATAGCGAAATTCATCAGCAAATGGG CGGTCTTCTTTTTCAACCATACGTTGGTACCGGTGAAAACTTATACTT TCAAGGCTCAGGTGGCGGTGGAAGTGATTACAAAGATGATGATGAT AAAGGAACCGGTTAA | |
| 79 | ATGGTACCACGCAGAATTGGTGATTACCATAGTAACATTTGGGATGA TGATTTTATCCAGTCACTTTCTACCCCTTATGGTGAACCATCTTACCA AGAAAGAGCTGAACGTCTTATTGTAGAAGTGAAAAAGATTTTCAAC AGTATGTACTTAGATGACGGTCGTTTAATGAGTTCTTTTAATGACTT AATGCAACGTTTATGGATTGTAGACTCAGTAGAACGTTTAGGTATTG CCCGTCACTTCAAAAATGAAATTACATCTGCCCTTGACTATGTTTTTC GTTATTGGGAAGAAAACGGTATAGGTTGTGGTCGTGATTCTATTGTA ACTGACTTAAATAGCACAGCTTTAGGTTTTCGTACACTTCGTTTACA | Myrcene (A. grandis) |
| | CGGTTATACAGTTTCTCCAGAGGTTTTAAAAGCATTTCAAGATCAAA ATGGTCAATTCGTTTGTTCACCAGGACAAACAGAAGGTGAAATTCGT TCAGTTTTAAATTTATATCGTGCAAGTTTAATTGCCTTTCCAGGCGAA AAAGTTATGGAAGAAGCAGAAATCTTCTCTACTCGCTATTTAAAAGA AGCTCTTCAAAAGATTCCAGTTAGCGCATTATCACAAGAAATCAAAT TTGTTATGGAATATGGATGGCATACAAATTTACCTAGATTAGAAGCA CGTAACTATATTGATACTTTAGAAAAGGATACATCAGCTTGGTTAAA CAAAAATGCAGGTAAAAAGTTATTAGAATTAGCTAAATTAGAATTT AACATCTTTAACTCATTACAACAAAAAGAATTACAATACTTACTTCG CTGGTGGAAAGAATCTGACTTACCTAAATTAACCTTTGCACGTCATA GACACGTTGAATTTTACACATTAGCTTCTTGTATTGCTATTGATCCCA AACATTCAGCATTCCGTTTAGGATTCGCTAAAATGTGTCACTTAGTT ACAGTTCTTGACGATATTTATGATACTTTCGGTACTATTGATGAACTT GAGTTATTTACTTCTGCAATTAAACGTTGGAATAGTTCTGAAATTGA ACACTTACCAGAATATATGAAATGCGTGTATATGGTTGTTTTTGAAA CTGTTAATGAATTAACTCGTGAAGCTGAGAAAACACAAGGACGTAA CACTTTAAACTATGTTCGTAAAGCATGGAAGCATATTTTGATTCTT ATATGGAGGAAGCAAAGTGGATCTCAAACGGATATTTACCAATGTT TGAAGAATACCACGAAAATGGTAAAGTGTCATCTGCATACCGTGTA GCAACATTACAACCAATTTTAACTTTAAACGCTTGGTTACCCGACTA CATTCTTAAAGGAATTGATTTCCCAAGTCGTTTTAACGATTTAGCTA GTTCATTCTTACGTTTACGTGGCGATACTCGCTGTTACAAAGCTGAC CGTGATCGTGGTGAAGAAGCTAGCTGCATTTCTTGTTACATGAAAGA TAATCCAGGTTCTACCGAAGAAGATGCACTTAATCACATTAACGCTA TGGTAAATGACATCATTAAAGAATTAAACTGGGAATTATTACGCAGT AATGATAATATTCCTATGTTAGCTAAAAAGCACGCTTTTGATATTAC TCGTGCACTTCACCACTTATACATTTATCGCGATGGTTTCAGTGTTGC TAATAAAGAAACTAAAAAGTTAGTTATGGAGACATTACTTGAATCA ATGTTATTTGGTACCGGTGAAAACTTATACTTTCAAGGCTCAGGTGG CGGTGGAAGTGATTACAAAGATGATGATGATAAAGGAACCGGTTAA | |
| 80 | ATGGTACCACAATCTGCTGAAAAGAACGACTCTTATCAAGTTCTAC ATTAGTTAAGAGAGAATTTCCACCCGGTTTCTGGAAAGACGACTTAA TCGACAGTTTAACTTCAAGTCACAAAGTAGCTGCTAGCGATGAAAA ACGTATCGAAACCTTAATTTCAGAAATTAAGAATATGTTTCGTTGTA TGGGTTATGGTGAGACAAATCCATCAGCTTATGATACTGCTTGGGTA GCTCGCATCCCAGCAGTTGATGGATCAGATAATCCTCACTTTCCAGA GACTGTGGAATGGATCTTACAAAATCAATTAAAAGATGGTTCTTGGG GTGAAGGTTTTTACTTCCTTGCTTATGATCGCATTTTAGCCACTTTAG CTTGTATTATCACACTTACACTTTGGCGTACTGGAGAAACACAAGTA CAGAAAGGTATCGAATTTTTCCGCACTCAAGCAGGTAAAATGGAAG | Abietadiene (A. grandis) |
| | ATGAAGCAGATTCACACCGTCCAAGTGGTTTTGAGATTGTATTTCCT GCTATGTTAAAAGAGGCTAAGATTTAGGCTTAGATTTACCTTATGA TCTTCCTTTTCTTAAACAAATTATTGAAAAGAGAGAAGCTAAGTTAA AACGTATTCCTACAGATGTTTATATGCTTTACCAACTACTTTACTTT ATTCATTAGAAGGTTTACAAGAAATAGTAGACTGGCAAAAAATCAT GAAATTACAAAGTAAAGATGGTAGTTTCTTATCTTCTCCTGCCTCAA CAGCAGCAGTATTTATGAGAACAGGTAACAAAAAGTGTTTAGATTT CTTAAATTTCGTGCTTAAAAAGTTCGGTAATCATGTTCCATGCCACT ATCCTTTAGACCTTTTTGAGCGTCTTTGGGCAGTTGATACTGTTGAAA GATTAGGTATTGACCGTCATTTTAAAGAAGAAATAAAAGAGGCTTT AGACTATGTGTATTCACACTGGGACGAACGTGGTATTGGTTGGGCTC GTGAAAACCCCGTTCCAGATATTGACGATACAGCAATGGGTCTTCGT ATTTTACGTCTTCATGGTTACAATGTTAGCAGCGATGTTCTTAAAAC ATTTCGTGATGAAAATGGTGAGTTCTTTTGCTTTTTAGGACAAACAC AAAGAGGTGTGACTGATATGTTAAATGTTAATCGTTGTAGCCATGTA TCTTTCCCTGGTGAAACTATAATGGAAGAGGCAAAATTATGTACTGA ACGTTACTTACGCAACGCATTAGAAAATGTAGACGCTTTTGATAAGT GGGCATTTAAGAAAAACATTCGTGGTGAGGTAGAATATGCTCTTAA ATATCCTTGGCATAAATCAATGCCACGTTTAGAAGCACGTTCATATA TTGAAAATTACGGTCCAGATGATGTTTGGTTAGGTAAAACTGTTTAT ATGATGCCTTACATTTCAAATGAAAAGTACTTAGAGTTAGCTAAACT TGATTTTAACAAAGTTCAGTCAATCCACCAGACAGAACTTCAAGACT TACGCCGTTGGTGGAAAAGTTCTGGTTTTACAGATTTAAACTTTACA AGAGAACGTGTTACTGAAATTTACTTTTCACCTGCATCTTTTATCTTC GAACCAGAATTTAGTAAATGTCGTGAGGTTTATACAAAAACTTCTAA TTTTACTGTAATTTTAGACGATTTATATGACGCTCATGGCTCTTTAGA TGACTTAAAACTTTTTACAGAGAGTGTTAAACGTTGGGATTTATCTT TAGTTGACCAAATGCCCCAGCAGATGAAAATCTGTTTTGTAGGTTTC TATAATACATTCAACGATATTGCTAAAGAAGGTAGAGAACGTCAAG GTCGTGATGTTTTAGGTTATATTCAAAACGTATGGAAAGTACAACTT GAAGCATATACTAAAGAAGCAGAATGGTCAGAAGCAAAATATGTTC CTAGTTTTAACGAATACATTGAAAATGCTTCAGTTTCAATTGCCTTA GGTACAGTAGTACTTATCAGTGCTTTATTTACCGGAGAAGTTTTAAC AGATGAAGTTTTATCTAAAATTGACCGTGAAAGTAGATTCTTACAGT TAATGGGCTTAACTGGACGTTTAGTAAATGATACTAAAACATATCAA GCTGAGCGTGGTCAAGGTGAAGTTGCTAGTGCAATTCAATGTTATAT GAAAGACCACCCTAAAATTAGTGAAGAAGAAGCATTACAACATGTA TATTCTGTAATGGAAAATGCATTAGAAGAATTAAATCGTGAGTTCGT TAACAACAAAATTCCAGACATCTATAAACGTCTTGTTTTCGAAACTG CACGTATAATGCAATTATTTTACATGCAAGGTGATGGTTTAACATTA AGTCACGATATGGAAATTAAAGAGCACGTAAAGAATTGTTTATTCC | |
| | AGCCAGTAGCTGGTACCGGTGAAAACTTATACTTTCAAGGCTCAGGT GGCGGTGGAAGTGATTACAAAGATGATGATGATAAAGGAACCGGTT AA | |
| 81 | ATGGTACCATCTTCATCAACAGGCACTTCAAAAGTAGTAAGCGAAA CATCTTCAACTATTGTAGACGATATTCCACGTCTTTCAGCAAATTATC ATGGTGATTTATGGCATCACAACGTAATTCAGACTTTAGAAACACCA TTTAGAGAAAGTTCAACTTATCAAGAGCGTGCAGATGAATTAGTAGT GAAAATCAAAGATATGTTCAATGCATTAGGTGACGGTGACATCTCA CCTTCAGCTTATGATACTGCATGGGTAGCTCGTGTTGCTACCATTTCT TCTGATGGTAGCGAAAAACCACGTTTTCCTCAAGCTCTTAATTGGGT TTTTAACAATCAATTACAAGATGGATCATGGGGTATTGAATCACATT TTAGTTTATGCGATCGTTTACTTAATACTACAAATTCAGTTATTGCTT TATCAGTATGGAAAACTGGTCACTCACAGGTTCAACAAGGTGCCGA ATTTATTGCTGAAAATTTACGTCTTTTAAATGAAGAAGACGAATTAA GTCCTGATTTTCAAATTATCTTCCCAGCTTTATTACAGAAAGCCAAG GCTTTAGGAATCAATTTACCCTATGATTTACCATTCATCAAATATCTT AGTACAACACGCGAAGCTCGTTTAACAGATGTGTCAGCTGCTGCTGA CAACATACCAGCCAATATGCTTAATGCACTTGAAGGTTTAGAAGAA GTGATTGATTGGAATAAAATCATGCGTTTTCAATCTAAAGATGGTTC ATTTTTATCTTCTCCAGCTAGTACAGCCTGTGTTTAATGAATACAGG TGATGAAAAATGTTTCACATTCTTAAATAACTTATTAGATAAATTCG GCGGTTGTGTTCCATGTATGTATAGCATTGATTTATTAGAACGTTTAT CTTTAGTGGACAACATTGAACACTTAGGTATTGGTCGTCACTTTAAA CAAGAAATCAAAGGTGCATTAGATTATGTATATCGTCATTGGTCTGA ACGCGGTATCGGTTGGGGTAGAGACTCTTTAGTTCCAGATTTAAACA CCACAGCTTTAGGTTTACGCACATTAAGAATGCACGGTTATAACGTG TCTAGTGATGTACTTAACAATTTCAAAGACGAAAATGGTCGTTTCTT TAGTAGTGCTGGTCAAACACACGTAGAGTTACGTTCTGTTGTAAATC TTTTTCGCGCCTCAGATTTAGCCTTTCCAGACGAACGTGCAATGGAT GATGCTCGTAAATTCGCAGAACCATATTTACGTGAAGCATTAGCTAC AAAAATATCAACAAATACAAAGTTATTCAAAGAAATTGAATATGTT GTTGAATACCCTTGGCACATGTCAATTCCACGTTTAGAAGCTCGTAG TTATATTGACAGTTATGATGATAATTATGTATGGCAACGTAAGACTT TATATCGTATGCCATCATTAAGTAATTCAAAATGTTTAGAACTTGCT AAATTAGATTTCAATATTGTTCAATCTTTACACCAAGAAGAACTTAA ACTTTTAACTCGTTGGTGGAAAGAATCTGGTATGGCAGACATAAATT TCACCCGCCATCGTGTAGCTGAAGTTTACTTTTCTAGTGCTACATTTG AGCCAGAATATAGTGCTACTCGTATTGCATTCACAAAAATTGGTTGC TTACAAGTACTTTTCGATGATATGGCTGACATTTTCGCCACTTTAGAT GAGTTAAAAAGTTTACTGAAGGTGTTAAACGCTGGGACACATCATT | Taxadiene (T. brevifolia) |
| | ATTACATGAAATTCCCGAATGTATGCAAACTTGTTTTAAAGTATGGT TTAAACTTATGGAAGAAGTAAACAACGACGTAGTAAAAGTTCAAGG AAGAGATATGTTAGCACATATTCGTAAACCCTGGGAATTATACTTTA ATTGTTATGTTCAAGAACGTGAATGGTTAGAAGCTGGTTATATTCCT ACATTCGAAGAATATCTTAAAACTTATGCTATTAGTGTAGGCCTTGG TCCTTGTACCTTACAACCTATTCTTTTAATGGGTGAGTTAGTTAAAGA TGATGTAGTAGAAAAAGTTCATTACCCTTCTAACATGTTCGAATTAG TTTCTTTAAGCTGGCGTTTAACTAATGATACCAAAACATATCAAGCA GAAAAAGTACGCGGTCAACAAGCTAGTGGCATTGCCTGTTATATGA AAGACAATCCAGGTGCTACTGAAGAAGATGCTATTAAACACATTTG TCGTGTTGTTGATCGTGCATTAAAAGAAGCAAGTTTCGAATATTTCA AGCCTTCAAATGACATTCCTATGGGTTGTAAATCTTTTATCTTTAACT TACGTTTATGTGTACAAATTTTCTATAAATTCATTGATGGTTATGGTA TCGCAAACGAAGAAATTAAGGACTACATTCGTAAGGTTTATATTGAT CCAATTCAAGTTGGTACCGGTGAAAACTTATACTTTCAAGGCTCAGG TGGCGGTGGAAGTGATTACAAAGATGATGATGATAAAGGAACCGGT TAA | |
| 82 | ATGGTACCACACAAGTTCACAGGTGTTAACGCTAAATTCCAGCAACC AGCATTAAGAAATTTATCTCCAGTGGTAGTTGAGCGCGAACGTGAG GAATTTGTAGGATTCTTTCCACAAATTGTTCGTGACTTAACTGAAGA TGGTATTGGTCATCCAGAAGTAGGTGACGCTGTAGCTCGTCTTAAAG AAGTATTACAATACAACGCACCTGGTGGTAAATGCAATAGAGGTTT AACAGTTGTTGCAGCTTACCGTGAACTTTCTGGACCAGGTCAAAAAG ACGCTGAAAGTCTTCGTTGTGCTTTAGCAGTAGGATGGTGTATTGAA TTATTCCAAGCCTTTTTCTTAGTTGCTGACGATATAATGGACCAGTCA TTAACTAGACGTGGTCAATTATGTTGGTACAAGAAAGAAGGTGTTG GTTTAGATGCAATAAATGATTCTTTTCTTTTAGAAAGCTCTGTGTATC GCGTTCTTAAAAAGTATTGCCGTCAACGTCCATATTATGTACATTTA TTAGAGCTTTTTCTTCAAACAGCTTACCAAACAGAATTAGGACAAAT GTTAGATTTAATCACTGCTCCTGTATCTAAGGTAGATTTAAGCCATTT CTCAGAAGAACGTTACAAAGCTATTGTTAAGTATAAAACTGCTTTCT ATTCATTCTATTTACCAGTTGCAGCAGCTATGTATATGGTTGGTATA GATTCTAAAGAAGAACATGAAAACGCAAAAGCTATTTTACTTGAGA TGGGTGAATACTTCCAAATTCAAGATGATTATTTAGATTGTTTTGGC GATCCTGCTTTAACAGGTAAAGTAGGTACTGATATTCAAGATAACAA ATGTTCATGGTTAGTTGTGCAATGCTTACAAAGAGTAACACCAGAAC AACGTCAACTTTTAGAAGATAATTACGGTCGTAAAGAACCAGAAAA AGTTGCTAAAGTTAAAGAATTATATGAGGCTGTAGGTATGAGAGCC GCCTTTCAACAATACGAAGAAAGTAGTTACCGTCGTCTTCAAGAGTT AATTGAGAAACATTCTAATCGTTTACCAAAAGAAATTTTCTTAGGTT | FPP (G. gallus) |
| | TAGCTCAGAAAATATACAAACGTCAAAAAGGTACCGGTGAAAACTT ATACTTTCAAGGCTCAGGTGGCGGTGGAAGTGATTACAAAGATGAT GATGATAAAGGAACCGGTTAA | |
| 83 | ATGGTACCATCATTAACTGAAGAAAAACCAATTCGCCCAATCGCAA ACTTTCCTCCAAGCATTTGGGGAGATCAATTCTTAATTTACGAAAAA CAAGTAGAACAAGGTGTTGAACAGATTGTTAACGACCTTAAGAAAG AAGTGCGCCAACTTTTAAAAGAGGCTTTAGATATTCCAATGAAACAC GCAAACCTTTTAAAACTTATTGACGAAATTCAACGTCTTGGTATTCC ATATCACTTTGAACGTGAAATTGATCATGCATTACAATGTATCTATG AAACTTATGGTGATAATTGGAATGGTGATCGTTCTTCATTATGGTTC CGTTTAATGCGTAAACAAGGTTATTATGTTACATGTGACGTGTTTAA CAATTACAAAGATAAAAATGGTGCATTTAAACAATCTTTAGCTAATG ATGTTGAAGGTTTATTAGAATTATATGAAGCTACTTCAATGCGTGTT CCAGGTGAAATTATTCTTGAAGATGCATTAGGTTTTACACGTTCTCG TTTATCTATTATGACAAAAGACGCATTTAGTACAAATCCTGCTTTATT TACTGAAATTCAGCGTGCCCTTAAACAGCCTTTATGGAAACGTTTAC CAAGAATTGAAGCTGCTCAATATATTCCATTTTATCAACAACAAGAT TCTCACAATAAGACATTACTTAAATTAGCCAAATTAGAATTTAATCT TTTACAATCATTACATAAAGAAGAATTAAGTCATGTGTGTAAATGGT GGAAAGCATTTGATATTAAGAAGAATGCTCCATGTTTACGTGATAGA ATTGTAGAGTGTTACTTTTGGGGCCTTGGTAGTGGTTACGAGCCACA ATATTCACGTGCTCGTGTATTCTTTACAAAAGCTGTTGCAGTTATTAC TTTAATTGACGATACCTATGATGCATACGGAACCTATGAGGAGCTTA AAATTTTCACTGAAGCTGTAGAACGTTGGTCTATAACTTGTTTAGAT ACTTTACCAGAATATATGAAACCCATCTACAAATTATTCATGGACAC ATACACTGAAATGGAAGAATTTTTAGCAAAAGAAGGTCGCACAGAC CTTTTTAACTGTGGTAAAGAATTTGTTAAAGAGTTTGTTCGTAACTTA ATGGTAGAAGCTAAGTGGGCTAATGAAGGTCACATTCCTACTACAG AAGAGCACGATCCAGTAGTAATAATTACAGGTGGAGCAAACTTACT TACCACAACTTGTTACTTAGGTATGTCTGACATTTTTACAAAAGAAT CAGTAGAGTGGGCAGTATCTGCACCACCATTATTCCGTTATTCTGGC ATACTTGGTCGTCGTCTTAATGATTTAATGACTCATAAAGCTGAACA AGAGCGTAAACACTCATCAAGTAGTTTAGAAAGCTATATGAAGGAA TATAACGTTAACGAAGAGTATGCTCAAACACTTATTTACAAAGAGGT TGAAGACGTTTGGAAGGACATTAACCGTGAATACTTAACAACTAAA AACATTCCACGTCCTCTTTTAATGGCTGTAATATACTTATGTCAATTC TTAGAAGTACAATACGCTGGAAAAGATAACTTTACACGTATGGGTG ATGAATATAAACACTTAATAAAGAGTTTATTAGTTTATCCTATGTCA ATAGGTACCGGTGAAAACTTATACTTTCAAGGCTCAGGTGGCGGTG GAAGTGATTACAAAGATGATGATGATAAAGGAACCGGTTAA | Amor-phadiene (A. annua) |
| 84 | ATGGTACCAGCAGGTGTATCAGCTGTGTCAAAAGTTTCTTCATTAGT ATGTGACTTAAGTAGTACTAGCGGCTTAATTCGTAGAACTGCAAATC CTCACCCTAATGTATGGGGTTATGACTTAGTTCATTCTTTAAAATCTC CATATATTGATAGTAGCTATCGTGAACGTGCTGAAGTGCTTGTAAGT GAAATAAAAGCTATGTTAAATCCAGCAATTACTGGAGATGGTGAAT CAATGATTACACCTTCAGCTTATGACACTGCTTGGGTTGCACGTGTA CCAGCAATTGATGGTAGCGCACGTCCACAATTTCCACAAACAGTAG ATTGGATTTTAAAGAATCAATTAAAAGATGGTTCTTGGGGTATTCAA TCACACTTTTTACTTTCAGACCGTTTATTAGCTACTCTTAGCTGTGTT TTAGTTTTACTTAAATGGAATGTTGGTGATTTACAGGTTGAGCAAGG TATTGAGTTTATTAAGTCAAACCTTGAATTAGTAAAAGATGAAACTG ATCAAGATTCTTTAGTGACTGATTTTGAGATTATTTTCCCTAGCTTAC TTCGTGAGGCCCAAAGTTTACGTTTAGGTCTTCCATACGATTTACCTT ACATCCACTTATTACAAACAAAACGTCAGGAACGTTTAGCAAAATT AAGCCGTGAAGAAATATATGCAGTTCCAAGTCCACTTTTATATTCTT TAGAGGGTATTCAAGATATTGTTGAGTGGGAACGTATTATGGAAGT ACAATCTCAGGATGGATCATTTTTAAGTTCTCCAGCATCAACCGCAT GTGTTTTTATGCATACAGGTGACGCTAAGTGTTTAGAATTTCTTAAC AGTGTAATGATTAAGTTTGGTAATTTTGTACCATGCCTTTATCCTGTA GATTTATTAGAACGTTTACTTATAGTAGATAATATAGTTCGTCTTGGT ATTTACCGTCACTTCGAAAAAGAAATTAAAGAAGCATTAGATTATGT ATATCGCCATTGGAATGAACGTGGTATTGGTTGGGGTCGTTTAAATC CAATTGCTGACTTAGAAACAACTGCTTTAGGTTTTCGTTTATTACGTT TACACCGTTATAATGTATCTCCAGCAATCTTTGATAATTTCAAAGAT GCCAATGGCAAATTCATTTGTAGCACTGGTCAGTTTAATAAGGATGT GGCTTCAATGTTAAACTTATACCGTGCATCACAATTAGCATTCCCAG GCGAAAACATTTTAGATGAAGCTAAATCTTTTGCCACCAAATACTTA CGTGAAGCCCTTGAAAAATCTGAAACTTCATCAGCTTGGAACAATA AACAGAATTTAAGTCAAGAAATCAAGTATGCATTAAAAACTTCATG GCACGCTTCTGTACCACGTGTTGAAGCAAAACGTTATTGTCAAGTTT ATCGTCCTGATTACGCTCGTATTGCTAAGTGTGTATACAAATTACCA TACGTTAACAACGAAAAATTCTTAGAATTAGGTAAATTAGATTTTAA CATCATTCAATCAATTCATCAAGAAGAAATGAAAAATGTGACAAGT TGGTTTCGTGATTCTGGCTTACCATTATTTACTTTCGCTCGCGAACGT CCTTTAGAATTTTACTTCTTAGTTGCTGCTGGTACTTATGAACCTCAA TATGCTAAATGTCGTTTCTTATTCACAAAAGTAGCTTGTCTTCAAAC AGTATTAGACGATATGTACGATACTTACGGTACTTTAGACGAATTAA AACTTTTTACCGAGGCTGTGCGTCGTTGGGATTTATCTTTTACAGAA AATTTACCTGACTATATGAAATTATGTTATCAAATCTATTATGACAT CGTTCATGAAGTGGCTTGGGAAGCTGAAAAAGAACAAGGTAGAGAA TTAGTGTCATTCTTCCGTAAAGGCTGGGAAGACTACTTATTAGGTTA | Bisabolene (A. grandis) |
| | CTATGAAGAAGCAGAATGGTTAGCAGCAGAATACGTTCCAACATTA GATGAATACATTAAAAACGGTATTACATCAATCGGCCAACGTATCTT ATTACTTTCAGGTGTGTTAATTATGGATGGCCAACTTTATCACAAG AAGCATTAGAAAAAGTTGATTACCCTGGTCGTCGTGTTTTAACTGAG TTAAACTCACTTATTAGCCGTTTAGCTGACGACACTAAAACTTATAA AGCAGAAAAAGCTCGTGGAGAATTAGCCTCATCAATTGAATGCTAC ATGAAAGATCATCCTGAATGTACAGAAGAAGCCTTAGACCACA TTTATTCTATTCTTGAACCAGCCGTAAAAGAATTAACTCGTGAATTT CTTAAACCAGACGACGTTCCATTTGCTTGTAAAAAGATGTTATTCGA AGAAACTCGTGTTACAATGGTGATCTTTAAAGATGGTGATGGTTTTG GTGTATCTAAGTTAGAAGTTAAAGATCACATCAAAGAATGCTTAATT GAACCATTACCATTAGGTACCGGTGAAAACTTATACTTTCAAGGCTC AGGTGGCGGTGGAAGTGATTACAAAGATGATGATGATAAAGGAACC GGTTAA | |
| 85 | ATGGTACCAACTATGATGAATATGAATTTTAAGTACTGTCACAAGAT TATGAAGAAACATTCAAAATCATTCAGTTATGCTTTTGACTTATTAC CAGAAGACCAACGTAAAGCTGTTTGGGCAATTTACGCCGTGTGCCG CAAAATTGATGATTCTATTGATGTATATGGTGATATTCAATTCTTAA ATCAGATTAAAGAAGACATACAAAGTATTGAAAAATATCCATACGA ACATCATCATTTTCAATCTGACAGACGTATTATGATGGCCTTACAGC ATGTTGCTCAGCATAAAAACATTGCATTTCAATCATTCTACAATTTA ATTGACACAGTATATAAAGATCAACACTTTACAATGTTTGAAACAGA TGCTGAACTTTTTGGTTATTGTTACGGTGTAGCTGGTACTGTGGGTG AAGTTTTAACTCCTATATTATCTGATCACGAAACACATCAAACTTAT GACGTTGCCCGTCGTTTAGGAGAGTCATTACAGTTAATCAATATTCT TAGAGATGTAGGTGAAGACTTTGACAACGAACGTATTTACTTCTCTA AACAACGTTTAAAACAATACGAAGTAGATATTGCAGAAGTGTACCA AAATGGTGTAAACAATCACTATATTGATTTATGGGAATATTACGCTG CAATTGCTGAAAAGGATTTTCAAGATGTTATGGACCAAATTAAAGTT TTCTCTATTGAAGCTCAGCCAATTATTGAGTTAGCTGCACGTATTTAT ATCGAAATTTTAGATGAAGTACGTCAAGCTAACTACACATTACATGA ACGTGTTTTTGTAGATAAACGTAAAAAGGCTAAACTTTTTCACGAAA ATAAAGGTACCGGTGAAAACTTATACTTTCAAGGCTCAGGTGGCGG TGGAAGTGATTACAAAGATGATGATGATAAAGGAACCGGTTAA | Diapo-phytoene (S. aureus) |
| 86 | ATGGTACCAAAAATTGCTGTTATTGGTGCTGGTGTTACCGGATTAGC TGCTGCTGCTCGTATTGCTAGCCAAGGTCATGAAGTTACAATCTTCG AAAAAAACAATAATGTAGGTGGTCGTATGAATCAATTAAAAAAAGA TGGTTTTACATTCGATATGGGACCTACAATTGTTATGATGCCAGATG TATATAAAGATGTATTTACTGCTTGCGGTAAAAACTATGAAGATTAT ATAGAGTTACGTCAACTTCGTTACATTTATGACGTATATTTCGATCA | Diapo-phytoene desaturase (S. aureus) |
| | CGATGATCGTATTACTGTTCCAACTGATTTAGCTGAATTACAACAAA TGTTAGAATCAATTGAACCTGGTAGTACACACGGATTTATGTCATTT TTAACAGATGTGTACAAGAAATATGAAATCGCTCGCAGATATTTCTT AGAACGTACTTACCGTAAACCATCAGACTTCTACAATATGACCTCTT TAGTACAAGGTGCTAAACTTAAAACTTTAAATCACGCTGATCAACTT ATCGAACACTACATTGATAACGAAAAGATTCAAAAACTTTTAGCATT CCAAACTCTTTATATCGGCATTGATCCAAAGCGTGGTCCTAGTTTAT ATAGTATTATTCCTATGATTGAAATGATGTTCGGTGTACATTTTATCA AAGGTGGTATGTATGGTATGGCTCAAGGATTAGCTCAACTTAACAA AGATTTAGGTGTTAATATTGAATTAAATGCTGAAATTGAACAAATCA TTATCGATCCTAAATTCAAACGCGCAGATGCAATTAAAGTTAATGGT GACATTCGCAAATTTGATAAGATTTTATGTACTGCTGACTTTCCTTCA GTTGCCGAATCACTTATGCCAGATTTCGCACCTATCAAAAAGTACCC TCCACATAAAATTGCAGATTTAGATTATTCTTGTTCAGCTTTTCTTAT GTATATTGGTATTGACATCGACGTAACTGACCAAGTTCGTTTACATA ACGTAATTTTTAGCGACGATTTTCGTGGAAATATTGAAGAAATTTTC GAAGGTCGCTTAAGTTACGACCCATCAATCTATGTTTATGTACCAGC TGTAGCCGATAAATCTTTAGCTCCTGAAGGTAAAACAGGCATTTATG TGTTAATGCCTACTCCTGAACTTAAAACAGGATCAGGTATTGACTGG TCAGATGAGGCTTTAACTCAACAAATTAAAGAAATCATTTATCGTAA ATTAGCAACAATTGAAGTATTTGAAGACATTAAATCACACATTGTAT CAGAAACAATTTTTACTCCTAATGACTTTGAACAAACCTATCACGCT AAATTTGGTTCTGCTTTCGGTTTAATGCCCACCTTAGCACAATCTAAT TATTACAGACCTCAAAATGTGTCACGTGATTATAAAGACTTATATTT CGCAGGTGCATCAACACATCCAGGTGCTGGAGTTCCAATTGTATTAA CAAGTGCCAAGATAACAGTAGACGAAATGATTAAAGATATTGAGCG TGGTGTGGGTACCGGTGAAAACTTATACTTTCAAGGCTCAGGTGGCG GTGGAAGTGATTACAAAGATGATGATGATAAAGGAACCGGTTAA | |
| 87 | ATGGTACCAGCATTTGACTTCGATGGTTACATGCTTCGTAAAGCTAA ATCTGTAAATAAAGCTCTTGAAGCTGCAGTACAAATGAAAGAACCA TTAAAAATTCATGAAAGTATGCGTTATTCTTATTAGCTGGTGGTAA ACGTGTACGTCCAATGTTATGTATTGCAGCTTGTGAATTAGTTGGTG GTGACGAAAGTACTGCTATGCCTGCTGCTTGCGCTGTAGAAATGATT CATACTATGAGTTTAATGCATGATGATTTACCATGTATGGATAATGA CGATTTACGTCGTGGTAAACCAACAAACCACATGGCATTTGGTGAA AGTGTAGCAGTATTAGCAGGTGATGCATTATTATCTTTTGCTTTTGA ACATGTAGCAGCAGCAACAAAAGGTGCTCCTCCAGAACGTATTGTT AGAGTTTTAGGTGAACTTGCAGTTTCTATTGGTTCAGAAGGTTTAGT TGCTGGACAAGTAGTTGACGTTTGTTCTGAAGGTATGGCTGAGGTTG GTTTAGATCATTTAGAATTTATTCATCACCACAAAACTGCTGCTTTAT | GPPS-LSU (M. spicata) |
| | TACAAGGTTCTGTAGTATTAGGTGCAATATTAGGTGGTGGAAAAGA AGAAGAGGTAGCAAAACTTCGTAAATTCGCTAACTGCATTGGTTTAC TTTTCCAAGTAGTAGATGATATTCTTGATGTAACAAAATCATCTAAA GAATTAGGTAAAACAGCAGGTAAAGATTTAGTTGCTGATAAAACTA CTTATCCAAAATTAATCGGTGTTGAGAAAAGTAAAGAGTTCGCAGA CCGTTTAAATCGTGAAGCTCAAGAACAACTTCTTCATTTTCATCCAC ATAGAGCAGCACCTTTAATCGCTTTAGCAAACTATATTGCTTATCGT GATAATGGTACCGGTGAAAATTTATATTTTCAAGGTTCAGGTGGCGG AGGTTCTGATTATAAAGATGATGATGATAAAGGAACCGGTTAA | |
| 88 | ATGGTACCAAGTCAACCTTACTGGGCAGCAATTGAGGCAGATATTG AACGTTACTTAAAAAAATCAATTACAATTCGTCCACCAGAAACTGTA TTTGGTCCAATGCACCACTTAACTTTTGCTGCACCAGCTACAGCTGC TAGTACTTTATGTTTAGCAGCATGTGAACTTGTAGGTGGTGATCGTA GTCAAGCTATGGCTGCAGCAGCAGCAATCCATCTTGTTCATGCAGCT GCTTATGTACATGAACATTTACCATTAACTGATGGTAGTCGTCCAGT AAGTAAACCAGCTATCCAACATAAATATGGTCCAAATGTAGAATTA CTTACAGGTGACGGTATTGTACCATTTGGTTTTGAATTATTAGCAGG TTCTGTTGATCCAGCACGTACAGATGATCCAGACCGTATTTTACGTG TAATAATTGAAATAAGTCGTGCTGGTGGTCCAGAAGGTATGATTAGT GGTTTACATCGTGAAGAAGAGATTGTAGATGGTAATACTTCTCTTGA TTTTATTGAATACGTTTGCAAAAAAAAATATGGTGAAATGCACGCAT GTGGTGCTGCATGCGGTGCAATTTTAGGTGGTGCAGCTGAAGAAGA AATTCAAAAACTTCGTAACTTCGGATTATATCAAGGAACTTTACGTG GTATGATGGAGATGAAAAACTCACACCAACTTATTGACGAAAATAT CATTGGCAAACTTAAAGAATTAGCTTTAGAAGAATTAGGTGGATTTC ATGGTAAAAATGCTGAATTAATGTCTAGTTTAGTAGCAGAACCATCA TTATATGCTGCTGGTACCGGTGAAAATTTATACTTTCAAGGTTCTGG TGGTGGTGGCAGTGATTATAAAGACGATGATGACAAAGGAACCGGT TAA | GPPS-SSU (M. spicata) |
| 89 | ATGGTACCACTTTTATCTAACAAATTAAGAGAGATGGTTTTAGCAGA AGTTCCTAAATTAGCATCTGCTGCTGAATATTTCTTTAAACGTGGTGT TCAGGGTAAACAATTCCGTTCAACAATTTTATTATTAATGGCAACAG CTCTTGACGTTCGTGTTCCAGAAGCATTAATTGGTGAATCTACTGAT ATTGTAACATCTGAATTACGTGTACGTCAACGTGGCATTGCTGAAAT TACAGAAATGATTCATGTAGCATCACTTCTTCACGATGACGTTCTTG ACGATGCTGATACTCGTCGTGGTGTTGGTAGTCTTAATGTTGTAATG GGAAACAAAATGTCAGTTTTAGCAGGTGACTTCTTACTTTCTCGTGC TTGTGGTGCTCTTGCAGCTCTTAAAAACACAGAAGTTGTAGCATTAT TAGCTACAGCAGTAGAACACTTAGTTACTGGTGAGACAATGGAAAT AACTTCATCAACTGAACAACGTTATTCTATGGATTACTACATGCAGA | GPPS (A. thalania) |
| | AAACTTATTACAAAACTGCTTCATTAATTTCAAATTCATGTAAAGCA GTTGCTGTATTAACAGGTCAAACAGCTGAAGTTGCAGTATTAGCTTT TGAATATGGTCGTAATTTAGGTTTAGCTTTCCAGTTAATTGACGACA TTTTAGATTTCACAGGCACATCTGCTAGTTTAGGAAAAGGTTCTTTA TCAGATATACGTCATGGTGTTATTACTGCTCCTATCTTATTTGCAATG GAAGAATTTCCTCAATTAAGAGAAGTAGTAGATCAAGTAGAAAAAG ATCCAAGAAATGTAGACATAGCTTTAGAATATTTAGGTAAAAGTAA AGGTATTCAACGTGCTCGTGAATTAGCAATGGAACACGCAAATTTA GCTGCTGCAGCTATTGGTTCTTTACCTGAAACAGATAACGAAGATGT TAAACGTTCACGTCGTGCTTTAATTGATTTAACACACAGAGTAATTA CACGTAACAAAGGTACCGGTGAGAATTTATACTTTCAAGGTAGTGGT GGAGGAGGTAGTGACTATAAAGATGATGACGATAAAGGAACCGGTT AA | |
| 90 | ATGGTACCAGTAGTTTCTGAACGTTTAAGACATTCTGTAACAACTGG TATTCCAGCATTAAAAACAGCAGCTGAATATTTCTTTCGTCGTGGTA TCGAAGGAAAACGTTTAAGACCTACATTAGCATTATTAATGAGTAGT GCTTTATCACCAGCTGCTCCATCACCAGAGTATTTACAAGTTGATAC AAGACCTGCTGCAGAACACCCTCATGAAATGCGTCGTCGTCAACAA CGTTTAGCTGAAATTGCAGAATTAATCCATGTAGCTTCATTACTTCA CGATGATGTTATTGATGACGCACAAACACGTCGTGGTGTTTTAAGTT TAAATACATCTGTTGGTAATAAAACAGCTATCTTAGCAGGTGATTTC TTATTAGCTCGTGCATCTGTAACATTAGCTAGTTTAAGAAACTCTGA AATTGTAGAATTAATGTCACAGGTTTTAGAACACTTAGTATCTGGTG AAATTATGCAAATGACTGCTACTTCAGAACAACTTTTAGATTTAGAA CATTATTTAGCAAAAACATATTGTAAAACTGCTTCATTAATGGCTAA TAGTTCTCGTTCTGTTGCAGTTCTTGCAGGTGCAGCTCCTGAAGTTTG TGATATGGCATGGTCATACGGTCGTCATTTAGGTATTGCTTTCCAAG TAGTTGACGATTTATTAGATTTAACAGGTTCATCTTCTGTTTTAGGTA AACCTGCTTTAAACGATATGCGTTCTGGTTTAGCAACAGCACCAGTA TTATTCGCTGCACAAGAAGAACCTGCATTACAGGCTCTTATATTACG TCGTTTTAAACACGACGGTGACGTAACAAAAGCAATGTCATTAATTG AACGTACACAAGGCTTACGTCGTGCTGAAGAACTTGCAGCACAACA CGCAAAAGCTGCTGCTGATATGATTCGTTGCTTACCTACAGCTCAAT CAGACCATGCAGAAATTGCTCGTGAAGCATTAATTCAAATTACACAT CGTGTTTTAACACGTAAAAAAGGTACCGGTGAAAACTTATACTTTCA AGGTTCTGGTGGTGGTGGATCAGATTATAAAGATGATGATGACAAA GGAACCGGTTAA | GPPS(C. reinhardtii) |
| 91 | ATGGTACCAGATTTTCCACAACAATTAGAAGCATGTGTTAAACAAGC AAATCAAGCATTATCACGTTTCATCGCACCACTTCCATTCCAAAATA CTCCTGTTGTTGAAACAATGCAATATGGTGCATTATTAGGAGGTAAA | FPP (E. coli) |
| | AGATTAAGACCATTTCTTGTATATGCAACAGGTCACATGTTTGGAGT ATCTACTAACACATTAGATGCTCCAGCTGCTGCAGTTGAATGTATTC ATGCATATAGTTTAATTCATGATGATTTACCTGCAATGGATGATGAT GACTTAAGAAGAGGTTTACCTACATGTCATGTTAAATTTGGTGAAGC TAATGCTATTTTAGCTGGCGATGCACTTCAAACTCTTGCATTCAGTAT TTTATCAGATGCTGATATGCCAGAAGTTTCAGATCGTGATCGTATTT CTATGATATCTGAATTAGCTTCTGCTAGTGGTATTGCTGGTATGTGC GGTGGCCAAGCTCTTGATTTAGACGCAGAAGGAAAACACGTTCCTTT AGATGCTTTAGAGCGTATACATCGTCACAAAACAGGAGCTTTAATTA GAGCTGCTGTTCGTCTTGGTGCTTTATCAGCTGGAGACAAAGGTCGT CGTGCTTTACCAGTTTTAGACAAATACGCTGAAAGTATTGGTTTAGC TTTTCAAGTTCAGGATGATATCTTAGATGTTGTAGGTGATACTGCTA CTTTAGGTAAACGTCAAGGTGCTGATCAACAGTTAGGCAAATCTACA TACCCAGCACTTTTAGGTTTAGAACAAGCTCGTAAAAAAGCAAGAG ACTTAATTGACGATGCTCGTCAAAGTCTTAAACAATTAGCAGAACAA TCACTTGATACAAGTGCTTTAGAAGCATTAGCAGATTACATTATTCA ACGTAATAAAGGTACCGGTGAAAATTTATATTTTCAAGGTTCTGGTG GTGGAGGTTCAGACTATAAAGATGACGATGATAAAGGAACCGGTTA A | |
| 92 | ATGGTACCAAGTGTTAGTTGTTGTTGTAGAAATTTAGGAAAAACTAT CAAAAAAGCTATTCCAAGTCACCACTTACATTTACGTTCTTTAGGTG GTAGTTTATATAGAAGACGTATTCAATCATCTTCAATGGAAACAGAC TTAAAATCTACATTCTTAAATGTTTATTCAGTTCTTAAATCAGATTTA TTACACGACCCATCATTTGAATTTACAAATGAAAGTCGTTTATGGGT AGATAGAATGCTTGATTATAATGTTCGTGGCGGTAAACTTAATCGTG GTCTTTCTGTAGTAGACTCTTTCAAATTACTTAAACAAGGTAATGAT TTAACTGAACAAGAAGTTTTCTTATCTTGTGCATTAGGTTGGTGTATT GAGTGGTTACAGGCTTACTTTTTAGTTCTTGATGATATTATGGATAAT TCAGTTACACGTCGTGGTCAACCTTGTTGGTTTCGTGTACCACAAGT TGGTATGGTAGCTATTAATGATGGCATTCTTCTTCGTAACCATATTCA TCGTATTCTTAAAAAACACTTCCGTGATAAACCATATTATGTAGATT TAGTTGACCTTTTCAATGAAGTAGAGTTACAAACTGCATGTGGACAA ATGATTGATTTAATCACAACATTTGAAGGTGAAAAAGACTTAGCTAA ATATAGTTTATCAATTCACCGTCGTATTGTTCAATACAAAACTGCAT ATTACTCATTCTATTTACCAGTTGCATGTGCTCTTTTAATGGCTGGCG AAAATTTAGAAAACCACATTGATGTTAAAAATGTATTAGTAGATATG GGTATTTACTTTCAAGTTCAGGATGATTATTTAGACTGTTTTGCTGAT CCTGAAACATTAGGTAAAATTGGCACTGATATTGAGGACTTTAAATG TTCTTGGTTAGTTGTAAAAGCATTAGAACGTTGTAGTGAAGAACAAA CAAAAATTCTTTACGAAAACTATGGCAAACCTGATCCATCTAATGTT | FPP (A. thalania) |
| | GCTAAAGTAAAAGATTTATACAAAGAATTAGATTTAGAAGGCGTTTT CATGGAATATGAATCTAAATCATACGAGAAATTAACTGGTGCTATCG AAGGTCACCAATCTAAAGCAATTCAAGCTGTTCTTAAATCTTTCTTA GCAAAAATCTATAAACGTCAAAAAGGTACCGGTGAAAACTTATACT TTCAAGGTAGTGGTGGCGGTGGTAGTGATTATAAAGATGATGATGA TAAAGGAACCGGTTAA | |
| 93 | ATGGTACCAGCTGATCTTAAATCAACATTCTTAGATGTTATTCAGT ATTAAAAAGTGATTTATTACAAGATCCATCTTTTGAATTTACACACG AAAGTCGTCAATGGTTAGAACGTATGTTAGATTATAATGTTCGTGGA GGCAAATTAAACAGAGGTTTAAGTGTAGTAGACAGTTACAAACTTTT AAAACAAGGTCAAGACTTAACAGAAAAAGAAACATTTTTATCTTGT GCTTTAGGTTGGTGTATTGAATGGTTACAAGCATACTTCTTAGTTTTA GACGATATTATGGATAATTCTGTAACTAGACGTGGTCAACCATGTTG GTTTCGTAAACCAAAAGTAGGTATGATTGCTATTAATGATGGAATAC TTCTTCGTAACCACATTCATCGTATTCTTAAAAAACACTTTCGTGAA ATGCCTTATTATGTAGACCTTGTAGACTTATTTAACGAAGTAGAATT TCAAACAGCTTGTGGTCAAATGATTGACTTAATTACAACATTTGATG GTGAAAAAGACCTTTCAAAATATTCACTTCAGATTCACCGTCGTATT GTTGAGTACAAAACAGCATACTACTCTTTCTATTTACCTGTAGCATG TGCTTTACTTATGGCAGGTGAAAATTTAGAAAATCACACAGATGTTA AAACTGTATTAGTTGATATGGGTATCTATTTCCAAGTTCAAGATGAT TATTTAGATTGCTTCGCTGATCCAGAAACATTAGGTAAAATTGGTAC AGATATTGAAGACTTTAAATGTAGTTGGTTAGTAGTAAAAGCATTAG AACGTTGTAGTGAAGAACAAACAAAAATTCTTTACGAAAATTATGG AAAAGCTGAACCTTCAAATGTAGCTAAAGTTAAAGCATTATACAAA GAATTAGATTTAGAGGGTGCATTTATGGAATATGAAAAAGAATCAT ACGAGAAACTTACAAAACTTATTGAAGCACATCAATCAAAAGCTAT TCAAGCAGTTCTTAAATCTTTCTTAGCTAAAATTTATAAACGTCAAA AAGGTACCGGTGAAAACTTATACTTTCAAGGCTCTGGAGGTGGTGGT TCAGACTATAAAGATGATGATGATAAAGGAACCGGTTAA | FPP (A. thalania) |
| 94 | ATGGTACCAAGTGGCGAACCTACTCCAAAAAAAATGAAAGCAACAT ACGTTCACGACCGTGAAAACTTTACAAAAGTATACGAAACTCTTCGT GACGAATTACTTAACGATGATTGTCTTAGTCCAGCTGGTTCACCTCA GGCTCAAGCTGCTCAAGAGTGGTTTAAAGAAGTTAATGATTATAATG TTCCTGGTGGAAAACTTAACCGTGGTATGGCTGTATATGACGTTTTA GCTTCAGTTAAAGGTCCAGATGGTTTAAGTGAAGACGAAGTATTTAA AGCTAACGCTCTTGGTTGGTGTATTGAGTGGTTACAAGCATTTTTCTT AGTTGCTGATGATATAATGGATGGTTCAATTACACGTCGTGGCCAAC CTTGTTGGTACAAACAACCTAAAGTTGGTATGATTGCTTGTAATGAT TACATCTTATTAGAATGCTGTATTTACTCAATTCTTAAAAGACATTTT | FPP (C. reinhardtii) |
| | AGAGGTCACGCTGCATACGCTCAACTTATGGACCTTTTCCATGAAAC TACATTCCAGACTTCACACGGTCAATTATTAGATTTAACAACAGCAC CTATCGGTTCTGTAGACTTATCAAAATATACAGAAGATAATTACCTT CGTATTGTAACATATAAAACTGCATACTATTCTTTTTATTTACCTGTA GCATGTGGTATGGTATTAGCTGGCATTACAGATCCAGCTGCTTTTGA TCTTGCAAAAAATATTTGTGTTGAAATGGGTCAATATTTCCAGATTC AAGACGATTATTTAGATTGCTATGGTGACCCTGAGGTTATTGGTAAA ATCGGTACAGACATAGAAGACAACAAATGTAGTTGGTTAGTTTGCA CAGCTCTTAAAATCGCAACAGAAGAACAAAAAGAGGTTATAAAAGC TAATTATGGTCACAAAGAGGCTGAATCAGTAGCAGCAATTAAAGCA TTATACGTTGAATTAGGTATTGAACAACGTTTTAAAGACTATGAAGC TGCATCATACGCAAAATTAGAAGGTACAATTAGTGAACAAACTTTAT TACCTAAAGCAGTATTTACTTCTTTATTAGCTAAAATCTATAAAAGA AAAAAAGGTACCGGTGAGAACTTATACTTTCAAGGTAGTGGAGGTG GTGGTTCAGACTATAAAGATGATGATGATAAAGGAACCGGTTAA | |
| 95 | ATGGTACCACAAACTGAACATGTTATCTTATTAAACGCTCAAGGTGT TCCTACAGGTACATTAGAAAAATATGCTGCACACACTGCTGATACTC GTTTACACTTAGCTTTCTCATCTTGGTTATTCAATGCTAAAGGTCAAC TTTTAGTTACAAGACGTGCATTAAGTAAAAAAGCATGGCCTGGTGTT TGGACTAACTCAGTTTGTGGTCATCCACAATTAGGTGAAAGTAATGA AGATGCAGTTATACGTCGTTGCAGATATGAATTAGGTGTTGAAATAA CTCCACCAGAATCAATTTATCCAGATTTCCGTTATCGTGCAACTGAT CCTAGTGGTATCGTTGAAAACGAAGTATGTCCTGTTTTTGCTGCACG TACAACAAGTGCATTACAAATTAATGATGATGAAGTAATGGATTATC AATGGTGTGACTTAGCTGATGTTTTACATGGTATTGATGCAACACCA TGGGCATTTTCACCATGGATGGTAATGCAAGCAACAAATCGTGAAG CACGTAAAAGATTAAGTGCTTTTACACAGTTAAAAGGTACCGGTGA AAACTTATACTTTCAAGGTAGTGGAGGTGGTGGTTCTGACTATAAAG ATGACGATGATAAAGGAACCGGTTAA | IPP isomerase (E. coli) |
| 96 | ATGGTACCACTTCGTAGTTTATTAAGAGGTTTAACACACATTCCTCG TGTTAATAGTGCTCAGCAACCTTCTTGCGCTCACGCTCGTCTTCAATT TAAACTTCGTTCTATGCAATTATTAGCAGAAAACCGTACAGATCACA TGCGTGGTGCTTCTACATGGGCAGGTGGTCAGTCTCAAGATGAATTA ATGCTTAAAGATGAATGTATCTTAGTAGATGCTGATGATAACATTAC TGGTCACGCTTCTAAATTAGAATGTCACAAATTTCTTCCACATCAAC CAGCTGGATTATTACACCGTGCTTTTTCTGTATTTCTTTTCGACGATC AAGGTCGTTTACTTTTACAACAACGTGCTCGTAGTAAAATTACATTT CCATCTGTATGGGCTAATACATGTTGTAGTCATCCATTACATGGTCA AACACCAGATGAAGTAGATCAACAATCACAAGTAGCAGACGGAACT GTACCAGGTGCAAAAGCTGCTGCAATCAGAAAATTAGAACATGAAT | IPP isomerase (H. pluvalis) |
| | TAGGTATTCCAGCTCACCAATTACCAGCATCAGCTTTTCGTTTCTTAA CACGTCTTCACTATTGTGCAGCTGACGTTCAACCTGCAGCAACACAA TCTGCATTATGGGGTGAACACGAAATGGATTACATTTATTCATTAG AGCTAATGTTACACTTGCTCCTAATCCTGACGAAGTAGATGAGGTAC GTTATGTAACTCAAGAAGAATTAAGACAAATGATGCAACCAGATAA TGGTTTACAATGGTCACCATGGTTCCGTATTATTGCAGCAAGATTTTT AGAACGTTGGTGGGCTGATTTAGATGCTGCATTAAATACAGATAAA CATGAAGACTGGGGAACAGTTCATCACATTAACGAAGCTGGTACCG GTGAAAACTTATACTTTCAAGGATCAGGAGGCGGTGGAAGTGATTA TAAAGATGATGATGATAAAGGAACCGGTTAA | |
| 97 | ATGGTACCAAGAAGATCAGGCAATTATAACCCAACAGCATGGGACT TCAATTATATCCAATCATTAGACAATCAATACAAAAAGAACGTTAC TCTACTCGTCACGCTGAATTAACAGTTCAAGTTAAAAATTATTAGA AGAAGAAATGGAAGCTGTTCAAAAACTTGAACTTATAGAGGATCTT AAAAACTTAGGCATTTCTTACCCATTTAAAGATAATATTCAACAAAT CTTAAATCAAATTTACAATGAACACAAATGTTGTCACAACTCAGAAG TTGAAGAAAAAGACCTTTATTTCACTGCTTTACGTTTTAGATTATTAC GTCAACAAGGTTTTGAAGTAAGTCAAGAAGTATTTGATCACTTTAAA AACGAAAAAGGTACAGATTTTAAACCTAATTTAGCAGATGATACTA AAGGATTATTACAATTATATGAAGCATCATTCTTATTACGTGAAGCA GAAGACACATTAGAACTTGCTCGTCAATTCTCTACTAAACTTTTACA AAAAAAAGTTGATGAAAACGGTGACGATAAAATTGAAGATAACTTA TTACTTTGGATTAGACGTAGTTTAGAATTACCATTACATTGGCGTGT ACAAAGATTAGAAGCTCGTGGCTTTTTAGATGCTTACGTTCGTAGAC CTGATATGAATCCTATTGTATTTGAATTAGCAAAATTAGACTTTAAC ATTACTCAAGCAACACAACAAGAAGAACTTAAAGATTTATCAAGAT GGTGGAATAGTACTGGCTTAGCTGAAAAACTTCCTTTTGCTCGTGAT CGTGTAGTTGAATCATATTTCTGGGCTATGGGTACTTTTGAACCACA TCAATACGGATACCAACGTGAATTAGTTGCTAAAATCATTGCACTTG CTACAGTTGTAGACGATGTTTACGATGTATATGGTACTTTAGAGGAA TTAGAACTTTTTACTGATGCTATTCGTCGTTGGGACCGTGAATCTATT GACCAATTACCATATTACATGCAATTATGTTTTCTTACTGTAAACAA CTTTGTTTTTGAGTTAGCTCACGACGTATTAAAAGATAAATCATTCA ATTGTTTACCTCATTTACAAAGATCATGGTTAGATTTAGCTGAAGCA TACCTTGTAGAAGCAAAATGGTATCATAGTCGTTATACACCTTCTTT AGAAGAATATCTTAATATTGCTCGTGTTTCAGTAACATGTCCAACTA TTGTTTCTCAAATGTATTTTGCATTACCAATTCCAATCGAAAAACCTG TAATTGAGATCATGTACAAATATCACGATATCTTATACTTATCAGGT ATGTTATTACGTTTACCAGATGACTTAGGAACAGCATCATTCGAACT TAAACGTGGTGATGTACAAAAAGCAGTTCAATGTTATATGAAAGAA | Limonene (L. angustifolia) |
| | CGTAATGTTCCTGAAAATGAAGCTCGTGAACATGTTAAATTCTTAAT TCGTGAGGCTTCTAAACAAATTAATACAGCAATGGCAACAGACTGT CCATTTACAGAAGATTTTGCAGTTGCAGCAGCAAACTTAGGTCGTGT AGCAAATTTTGTATATGTTGATGGTGATGGTTTTGGAGTACAACACA GTAAAATCTATGAGCAAATTGGTACACTTATGTTTGAACCATATCCA GGTACCGGTGAAAACTTATACTTTCAAGGTAGTGGTGGTGGAGGTTC TGATTACAAAGACGATGATGATAAAGGAACCGGTTAA | |
| 98 | ATGGTACCAAGAAGAAGTGGAAACTATAAACCTACAATGTGGGATT TTCAATTTATTCAAAGTGTAAATAATCTTTACGCTGGTGATAAATAC ATGGAACGTTTCGATGAAGTAAAAAAAGAAATGAAAAAAACTTAA TGATGATGGTTGAGGGTTTAATAGAGGAATTAGATGTTAAATTAGA ATTAATAGATAATTTAGAAAGATTAGGTGTTAGTTATCATTTCAAAA ATGAAATAATGCAAATCCTTAAATCTGTACACCAGCAAATCACTTGT CGTGATAATTCATTATACTCTACTGCATTAAAATTTCGTTTATTACGT CAACACGGATTCCACATTAGTCAAGACATCTTTAACGATTTTAAAGA TATGAATGGCAATGTTAAACAAAGTATCTGTAACGATACTAAAGGTT TATTAGAACTTTATGAAGCATCTTTCTTATCTACTGAATGTGAAACA ACACTTAAAAACTTCACTGAAGCACACTTAAAAAATTATGTTTATAT TAACCACTCATGTGGAGATCAATACAATAACATAATGATGGAATTA GTTGTTCACGCTTTAGAATTACCACGTCACTGGATGATGCCTCGTTT AGAGACACGTTGGTATATATCAATTTATGAACGTATGCCTAATGCTA ATCCACTTTTACTTGAACTTGCTAAATTAGACTTCAATATTGTTCAAG CTACACACCAACAAGACTTAAAATCATTATCACGTTGGTGGAAAAA CATGTGTTTAGCTGAAAAATTATCATTTCTCGTAACCGTTTAGTAG AAAATCTTTTCTGGGCAGTTGGAACTAATTTTGAACCACAACACAGT TATTTCCGTCGTTTAATCACTAAAATCATTGTTTTTGTTGGTATTATT GATGATATTTATGATGTTTACGGCAAACTTGATGAGTTAGAATTATT CACTTTAGCTGTACAACGTTGGGATACAAAAGCAATGGAAGACTTA CCATATTACATGCAAGTTTGTTATTTAGCTTTAATTAATACAACAAA TGATGTTGCTTATGAAGTTCTTCGTAAACATAACATTAATGTATTAC CATACTTAACTAAATCTTGGACAGACTTATGTAAATCATATTTACAA GAAGCTCGTTGGTACTACAATGGTTACAAACCTTCATTAGAGGAATA TATGGATAATGGTTGGATTAGTATAGCAGTTCCTATGGTATTAGCAC ATGCACTTTTCTTAGTTACAGATCCAATTACAAAAGAAGCATTAGAA TCATTAACAAACTATCCTGATATTATTCGTTGCTCAGCTACAATATTC CGTTTAAATGATGATCTTGGTACAAGTTCAGATGAATTAAAACGTGG AGATGTACCAAAATCAATTCAATGCTATATGAACGAAAAGGCGTT TCAGAGGAAGAAGCTCGTGAACATATTCGTTTCTTAATCAAAGAAA CATGGAAATTCATGAACACTGCACACCATAAAGAGAAAAGTTTATT TTGTGAGACATTTGTAGAAATTGCAAAAAATATTGCAACAACAGCTC | Mono-terpene (S. lyco-persicum) |
| | ATTGTATGTACTTAAAAGGTGATTCTCACGGTATTCAAAACACTGAT GTTAAAAACTCAATAAGTAATATACTTTTCCATCCAATTATTATCGG TACCGGTGAAAACCTTTACTTTCAAGGTTCAGGTGGTGGCGGTTCAG ACTATAAAGATGACGATGATAAAGGAACCGGTTAA | |
| 99 | ATGGTACCAAGACGTAGTGGAAATTATGAGCCATCTGCATGGGACT TCAATTACTTACAATCTCTTAATAATTATCACCATAAAGAAGAACGT TACTTACGTCGTCAAGCTGATTTAATTGAAAAAGTAAAAATGATTCT TAAAGAAGAGAAAATGGAAGCATTACAGCAATTAGAACTTATAGAC GATCTTCGTAATTTAGGTCTTTCATATTGTTTTGATGATCAAATTAAT CATATTCTTACAACAATTTACAACCAACATTCTTGTTTCCATTATCAC GAAGCTGCAACAAGTGAAGAAGCTAACTTATATTTCACAGCTTTAG GTTTCCGTTTACTTCGTGAACACGGATTCAAAGTATCACAAGAAGTA TTTGACCGTTTCAAAAATGAAAAAGGTACAGATTTTCGTCCAGATTT AGTAGATGATACTCAAGGTTTATTACAACTTTATGAAGCATCTTTCC TTCTTCGTGAAGGTGAAGACACTTTAGAATTTGCACGTCAATTTGCT ACTAAATTTCTTCAAAAAAAAGTTGAGGAGAAAATGATAGAAGAGG AAAATCTTTTATCTTGGACTTTACATTCACTTGAATTACCATTACATT GGCGTATACAACGTTTAGAAGCTAAATGGTTTTTAGATGCTTATGCT AGTCGTCCTGATATGAATCCAATAATCTTTGAATTAGCAAAATTAGA ATTTAACATTGCTCAGGCACTTCAACAAGAAGAACTTAAAGATTTAT CAAGATGGTGGAACGATACTGGTATTGCTGAAAAATTACCTTTCGCT CGTGATAGAATCGTTGAATCTCATTATTGGGCAATTGGTACTTTAGA ACCTTATCAATACCGTTATCAGCGTTCATTAATTGCAAAAATCATTG CTTTAACTACAGTTGTTGATGATGTATATGATGTTTACGGTACATTA GACGAATTACAGTTATTTACTGATGCAATTCGTCGTTGGGACATTGA AAGTATAAATCAATTACCTTCTTATATGCAATTATGTTATTTAGCTAT TTATAATTTCGTATCAGAATTAGCTTATGATATTTTCAGAGATAAAG GTTTTAATTCTTTACCATATTTACACAAAAGTTGGCTTGACTTAGTTG AGGCTTACTTTCAAGAAGCAAAATGGTATCATTCTGGCTACACACCA TCATTAGAACAATACTTAAATATCGCTCAAATTTCTGTAGCAAGTCC AGCTATATTAAGTCAAATTTACTTTACTATGGCTGGTTCAATTGATA AACCAGTAATCGAATCAATGTACAAATATAGACACATTTTAAACTTA TCTGGTATATTACTTAGATTACCAGATGACTTAGGTACTGCTAGTGA TGAATTAGGTCGTGGTGATTTAGCAAAAGCAATGCAATGTTACATGA AAGAGCGTAACGTTTCTGAAGAAGAAGCTCGTGATCATGTACGTTTC TTAAATCGTGAGGTTTCAAAACAAATGAATCCTGCTCGTGCTGCTGA TGATTGTCCATTCACTGATGATTTTGTAGTAGCTGCTGCTAATTTAGG AAGAGTTGCAGATTTCATGTATGTTGAAGGCGATGGTTTAGGTTTAC AATACCCAGCTATCCACCAACACATGGCAGAACTTTTATTTCACCCT TACGCAGGTACCGGTGAAAACTTATACTTTCAAGGTTCAGGTGGTGG | Terpinolene (O. basilicum) |
| | AGGTTCTGACTATAAAGATGATGATGATAAAGGAACCGGTTAA | |
| 100 | ATGGTACCAAGAAGATCAGGAAATTATCAACCTAGTGCATGGGATT TTAACTATATCCAATCTCTTAATAACAACCATTCTAAAGAAGAACGT CACTTAGAGCGTAAAGCAAAACTTATTGAAGAAGTAAAAATGTTAT TAGAGCAAGAAATGGCTGCTGTACAACAATTAGAGCTTATTGAAGA CCTTAAAAACTTAGGTTTATCTTACTTATTCCAAGATGAAATCAAAA TAATCCTTAATTCTATTTACAATCATCATAAATGTTTCATAATAATC ACGAACAATGTATTCACGTTAATAGTGACTTATACTTTGTTGCATTA GGCTTCCGTTTATTTCGTCAACATGGTTTCAAAGTTTCTCAAGAGGTT TTTGACTGTTTTAAAAACGAAGAAGGATCAGACTTTAGTGCTAACTT AGCAGATGATACTAAAGGTTTACTTCAATTATACGAGGCTTCATATT TAGTTACAGAAGATGAAGACACATTAGAAATGGCACGTCAATTTTC AACTAAAATCTTACAAAAAAAAGTAGAAGAGAAAATGATTGAGAA AGAGAACTTATTAAGTTGGACTTTACATAGTTTAGAATTACCACTTC ACTGGCGTATTCAACGTTTAGAAGCAAAATGGTTCCTTGATGCTTAT GCTAGTCGTCCAGATATGAATCCAATTATTTTTGAATTAGCTAAATT AGAGTTTAACATTGCTCAAGCATTACAACAAGAAGAATTAAAAGAT TTAAGTAGATGGTGGAATGATACAGGCATTGCTGAAAAATTACCTTT TGCTCGTGATAGAATAGTAGAGAGTCATTACTGGGCAATTGGTACTT TAGAACCTTATCAATATAGATATCAACGTTCATTAATTGCTAAAATT ATTGCTTTAACAACAGTTGTTGATGACGTTTACGACGTATATGGAAC TTTAGATGAATTACAGTTATTTACAGACGCTATTCGTCGTTGGGATA TTGAATCTATTAATCAATTACCAAGTTATATGCAATTATGCTATTTAG CTATTTATAACTTTGTTTCTGAATTAGCATACGATATTTTTCGTGACA AAGGATTCAATTCTTTACCTTACCTTCATAAATCATGGTTAGATTTAG TAGAAGCATACTTTGTTGAAGCTAAATGGTTTCATGATGGTTATACT CCAACTCTTGAAGAATATTTAAATAACTCAAAAATTACTATTATATG TCCTGCTATTGTTAGTGAAATCTACTTCGCATTCGCTAATTCAATTGA TAAAACAGAAGTTGAATCAATCTACAAATATCACGATATTTTATATT TATCAGGAATGCTTGCACGTTTACCAGACGACTTAGGTACTTCATCA TTTGAAATGAAAAGAGGTGATGTTGCTAAAGCTATTCAATGTTACAT GAAAGAACATAATGCTTCAGAGGAAGAAGCTCGTGAACACATTCGT TTCTTAATGCGTGAAGCATGGAAACACATGAATACTGCTGCAGCTGC TGATGACTGTCCATTTGAATCTGATTTAGTAGTAGGTGCTGCATCAT TAGGTAGAGTTGCAAACTTTGTATATGTTGAAGGTGACGGTTTTGGT GTACAACATTCAAAAATACATCAACAAATGGCTGAATTACTTTTTTA TCCATATCAAGGTACCGGTGAAAACTTATACTTTCAAGGTAGTGGAG GTGGTGGTAGTGACTATAAAGACGATGACGATAAAGGAACCGGTTA A | Myrcene (O. basilicum) |
| 101 | ATGGTACCAAGAAGAAGTGCTAATTATCAAGCAAGTATTTGGGATG | Zingiberene |
| | ATAATTTCATTCAAAGTCTTGCATCTCCTTATGCAGGAGAAAAATAT GCAGAAAAAGCAGAAAAACTTAAAACAGAAGTTAAAACTATGATTG ATCAAACAAGAGATGAACTTAAACAATTAGAACTTATTGATAACTT ACAACGTTTAGGTATATGTCATCACTTTCAAGACCTTACAAAAAAA TTTTACAAAAAATTTATGGAGAAGAACGTAACGGAGATCACCAACA TTACAAAGAAAAAGGCTTACATTTACAGCATTACGTTTCCGTATTT TACGTCAGGACGGTTATCATGTTCCACAAGATGTATTTTCATCATTT ATGAATAAAGCTGGTGACTTTGAAGAATCTTTAAGTAAAGACACAA AAGGTTTAGTTAGTTTATATGAGGCTTCTTACTTATCAATGGAAGGT GAAACTATTTTAGATATGGCAAAAGACTTTTCATCTCACCATTTACA TAAAATGGTTGAAGATGCTACTGACAAACGTGTAGCTAATCAAATT ATCCATTCTCTTGAAATGCCACTTCACAGACGTGTTCAAAAACTTGA AGCAATTTGGTTTATTCAATTCTACGAATGCGGCTCTGATGCTAATC CAACTTTAGTAGAATTAGCAAAATTAGATTTCAACATGGTTCAGGCA ACATACCAAGAAGAATTAAAACGTTTATCACGTTGGTATGAAGAAA CAGGCTTACAAGAGAAACTTTCATTCGCTCGTCACCGTCTTGCTGAA GCATTCTTATGGTCTATGGGTATTATTCCAGAAGGACACTTTGGTTA TGGTCGTATGCACTTAATGAAAATTGGTGCTTACATTACATTACTTG ATGATATTTATGATGTTTATGGTACTTTAGAAGAACTTCAAGTATTA ACAGAAATTATTGAACGTTGGGATATTAACTTATTAGATCAATTACC TGAATACATGCAAATCTTCTTTTTATACATGTTTAATTCTACAAATGA ACTTGCTTATGAAATTTTACGTGATCAAGGTATCAATGTAATATCAA ACTTAAAAGGATTATGGGTAGAGTTATCTCAGTGTTACTTTAAAGAA GCTACTTGGTTCCATAACGGTTACACACCAACAACTGAAGAATATCT TAATGTTGCTTGTATTTCTGCTAGTGGTCCTGTTATTTTATTTTCAGG TTACTTTACTACTACTAATCCTATTAATAAACACGAATTACAATCTTT AGAACGTCACGCACATTCATTATCTATGATATTACGTTTAGCTGATG ATTTAGGTACATCAAGTGATGAAATGAAACGTGGAGATGTACCAAA AGCTATTCAATGTTTTATGAATGACACTGGTTGTTGTGAAGAAGAAG CACGTCAACACGTAAAAAGATTAATAGATGCTGAATGGAAAAAAAT GAACAAAGACATCTTAATGGAGAAACCATTTAAAAATTTTGTCCAA CTGCTATGAATTTAGGTCGTATTTCTATGAGTTTTTATGAACACGGA GATGGTTATGGAGGTCCTCACTCTGATACAAAAAAAAAAATGGTAT CTTTATTTGTACAACCAATGAATATTACTATTGGTACCGGTGAAAAC CTTTATTTTCAAGGTTCTGGTGGTGGCGGTTCAGATTATAAAGATGA TGACGACAAAGGAACCGGTTAA | (O. basilicum) |
| 102 | ATGGTACCAAGACGTTCAGCTAACTATCAACCTAGTATTTGGAACCA CGATTACATTGAATCACTTCGTATCGAATATGTTGGTGAAACATGTA CACGTCAAATTAACGTTTTAAAAGAACAAGTTCGTATGATGTTACAC AAAGTTGTTAATCCATTAGAACAATTAGAATTAATTGAAATTTTACA | Myrcene (Q. ilex) |
| | ACGTTTAGGTTTAAGTTACCATTTCGAAGAAGAAATAAAACGTATTT TAGATGGTGTTTACAATAACGATCATGGTGGTGATACATGGAAAGC AGAAAACCTTTATGCAACAGCTCTTAAATTCCGTCTTTTACGTCAGC ACGGTTATTCTGTTTCTCAAGAAGTTTTCAACTCTTTTAAAGATGAGC GTGGCAGTTTCAAAGCATGTTTATGTGAAGATACTAAAGGTATGTTA TCACTTTATGAAGCATCTTTCTTTCTTATTGAAGGTGAAAACATTTTA GAGGAAGCTAGAGACTTTAGTACAAAACATCTTGAAGAATATGTAA AACAAAATAAAGAGAAAAACTTAGCTACTTTAGTTAATCACTCATTA GAATTTCCATTACATTGGCGTATGCCTCGTTTAGAAGCTCGTTGGTTC ATCAATATCTATCGTCATAATCAAGATGTAAATCCAATCCTTTTAGA ATTTGCTGAACTTGACTTCAATATTGTACAAGCTGCTCACCAAGCAG ATTTAAAACAAGTATCAACATGGTGGAAATCAACTGGTTTAGTAGA AAATCTTTCATTCGCTCGTGATCGTCCTGTAGAAAACTTCTTTTGGAC AGTTGGTCTTATTTTCCAACCACAATTCGGTTATTGTCGTAGAATGTT TACTAAAGTATTCGCATTAATTACTACAATTGATGACGTATATGATG TATATGGTACTTTAGATGAATTAGAACTTTTCACAGACGTTGTTGAA AGATGGGATATTAATGCAATGGATCAATTACCTGATTATATGAAAAT TTGCTTTTTAACATTACACAATAGTGTTAACGAAATGGCATTAGACA CTATGAAAGAACAACGTTTTCACATCATTAAATACCTTAAAAAAGCA TGGGTTGATCTTTGTCGTTATTACTTAGTTGAAGCTAAATGGTATAGT AATAAATATAGACCTTCTTTACAAGAATACATTGAAAATGCATGGAT TTCAATTGGTGCTCCAACTATTTTAGTTCATGCATATTTCTTCGTTAC AAATCCAATTACAAAAGAAGCATTAGACTGTTTAGAAGAATATCCA AACATTATTCGTTGGAGTAGTATTATTGCACGTTTAGCTGATGATTT AGGTACTTCAACAGACGAATTAAAACGTGGTGACGTACCAAAAGCA ATTCAATGTTATATGAATGAAACAGGTGCTTCAGAAGAAGGTGCTC GTGAGTACATTAAATACTTAATTTCTGCTACTTGGAAAAAAATGAAC AAAGATAGAGCAGCATCAAGTCCATTTTCACATATCTTCATTGAAAT TGCTCTTAATTTAGCACGTATGGCACAATGTTTATATCAACACGGTG ACGGCCACGGTTTAGGTAACCGTGAAACAAAAGATCGTATACTTTC ATTACTTATTCAACCAATTCCATTAAACAAAGATGGTACCGGTGAGA ACTTATACTTTCAAGGCTCAGGTGGTGGTGGTTCTGATTACAAAGAT GATGATGATAAAGGAACCGGTTAA | |
| 103 | ATGGTACCAAGAAGAATTGGAGACTATCACTCAAACTTATGGAATG ATGACTTCATTCAATCATTAACAACACCATACGGTGCTCCATCATAT ATTGAACGTGCTGATAGATTAATATCTGAAGTAAAAGAAATGTTTAA TAGAATGTGTATGGAAGATGGTGAGTTAATGTCTCCATTAAATGATC TTATTCAAAGATTATGGACTGTTGATAGTGTTGAACGTTTAGGTATA GATCGTCACTTCAAAAATGAAATAAAAGCTAGTTTAGATTATGTATA CTCATACTGGAACGAAAAAGGTATCGGTTGTGGTCGTCAATCAGTA | Myrcene (P. abies) |
| | GTTACAGATTTAAACTCTACTGCTCTTGGATTAAGAATTTTACGTCA ACATGGTTACACAGTTTCAAGTGAAGTTTTAAAAGTTTTGAAGAAG AAAACGGTCAATTTGCTTGTTCACCTTCACAGACTGAGGGCGAAATT CGTTCATTCTTAAACTTATATCGTGCTTCATTAATTGCTTTTCCTGGT GAAAAAGTAATGGAAGAAGCTCAAATCTTTTCTAGTCGTTACTTAAA AGAAGCAGTTCAGAAAATTCCAGTTTCAGGTTTATCTCGTGAAATAG GCGATGTTTTAGAATATGGTTGGCACACAAACTTACCTCGTTGGGAA GCTCGTAACTATATGGACGTATTCGGTCAAGACACAAATACATCATT CAACAAAAACAAAATGCAATATATGAATACAGAGAAAATTCTTCAA TTAGTAAAATTAGAGTTTAATATCTTTCATTCATTACAACAACGTGA ATTACAATGTTTATTACGTTGGTGGAAAGAAAGTGGTCTTCCACAAT TAACATTTGCACGTCACCGTCACGTTGAATTTTACACTTTAGCTTCTT GTATTGCATGTGAACCAAAACACAGTGCATTTCGTTTAGGTTTTGCA AAAATGTGTCACTTAGTAACAGTTTTAGATGATGTATATGACACATT TGGCAAAATGGATGAATTAGAACTTTTTACTGCAGCTGTTAAACGTT GGGACTTATCAGAAACTGAGCGTTTACCTGAGTATATGAAAGGTTTA TATGTTGTAGTTTTCGAGACTGTTAATGAATTAGCACAAGAAGCAGA GAAAACTCAAGGACGTAATACATTAAATTACGTTCGTAAAGCATGG GAAGCATACTTCGATAGTTATATGAAAGAAGCAGAATGGATCTCAA CAGGCTATTTACCAACATTCGAAGAGTATTGTGAAAACGGTAAAGT ATCAAGTGCATATAGAGTTGCTGCACTTCAACCTATTTTAACATTAG ATGTACAACTTCCAGATGACATCTTAAAAGGTATTGATTTTCCATCT CGTTTCAATGATTTAGCATCTTCATTTCTTCGTTTACGTGGAGATACT AGATGTTACGAGGCTGATCGTGCTCGTGGTGAAGAAGCAAGTTGTA TTTCTTGTTACATGAAAGACAATCCAGGTTCAACTGAAGAAGATGCA TTAAATCACATTAATGCTATGATAAATGATATTATTCGTGAATTAAA CTGGGAATTTCTTAAACCAGACTCAAATATCCCAATGCCAGCTCGTA AACATGCTTTCGATATTACAAGAGCTTTACATCACTTATATATTTATC GTGACGGTTTTTCTGTTGCTAACAAAGAGACTAAAAATCTTGTTGAG AAAACTTTATTAGAATCAATGTTATTCGGTACCGGTGAGAACCTTTA TTTTCAAGGTTCAGGTGGTGGTGGTTCAGATTATAAAGACGATGATG ATAAAGGAACCGGTTAA | |
| 104 | ATGGTACCAAGAAGATCAGCTAATTATCAACCTAGTCGTTGGATCA TCATCACCTTTTAAGTGTAGAAAACAAATTCGCTAAAGATAAACGTG TAAGAGAACGTGACTTACTTAAAGAAAAAGTTCGTAAAATGTTAAA TGACGAACAGAAAACTTACTTAGATCAATTAGAATTTATTGACGATC TTCAAAAATTAGGTGTTAGTTATCACTTCGAAGCAGAAATAGATAAT ATACTTACAAGTTCATACAAAAAAGATCGTACAAATATACAAGAAA GTGATTTACACGCAACTGCATTAGAGTTTCGTCTTTTTCGTCAACAC GGTTTTAACGTTTCAGAAGATGTATTTGATGTATTTATGGAAAATTG | Myrcene, ocimene (A. thalania) |
| | TGGTAAATTCGACCGTGATGACATTTATGGTTTAATTTCATTATATG AAGCTAGTTATCTTTCTACTAAACTTGACAAAAATCTTCAAATCTTT ATCCGTCCATTTGCTACTCAACAATTACGTGATTTTGTAGATACTCAC AGTAATGAAGATTTCGGTTCATGTGATATGGTAGAAATAGTTGTTCA AGCATTAGACATGCCATACTATTGGCAAATGCGTCGTTTATCTACAC GTTGGTATATTGATGTTTATGGTAAAAGACAAAATTACAAAAACTTA GTAGTTGTTGAATTTGCAAAAATTGATTTCAATATTGTTCAAGCTATT CACCAGGAAGAACTTAAAAATGTATCATCTTGGTGGATGGAAACTG GTTTAGGTAAACAACTTTATTTTGCTCGTGATCGTATTGTAGAGAAC TATTTTTGGACAATTGGTCAAATTCAAGAACCTCAATATGGATATGT TAGACAAACAATGACTAAAATCAATGCTTTATTAACAACAATTGATG ATATTTATGATATATACGGTACATTAGAAGAATTACAGTTATTCACA GTTGCATTTGAGAATTGGGACATAAATCGTTTAGACGAATTACCAGA ATATATGCGTTTATGTTTCTTAGTTATCTATAACGAAGTAAATAGTAT AGCATGTGAAATTCTTAGAACAAAAAATATTAACGTTATTCCTTTCT TAAAAAAATCTTGGACTGATGTAAGTAAAGCATACTTAGTTGAAGCT AAATGGTATAAATCAGGCCATAAACCAAATTTAGAAGAGTATATGC AAAATGCACGTATTTCTATTTCTTCACCAACAATCTTTGTTCACTTTT ATTGTGTATTTTCAGACCAATTATCTATTCAAGTTTTAGAAACTTTAT CACAACACCAACAAAATGTTGTAAGATGTAGTTCTTCTGTTTTCCGT TTAGCTAATGACTTAGTAACTTCTCCAGATGAATTAGCTAGAGGTGA TGTTTGTAAATCAATTCAATGTTATATGTCAGAAACTGGTGCAAGTG AAGATAAAGCTAGATCACACGTTCGTCAAATGATTAATGATTTATGG GACGAAATGAATTACGAGAAAATGGCACATTCAAGTAGTATCTTAC ATCATGATTTTATGGAGACAGTAATCAATTTAGCTAGAATGTCTCAA TGTATGTACCAATATGGTGACGGACACGGTTCTCCAGAAAAAGCTA AAATTGTAGATCGTGTAATGAGTTTACTTTTCAACCCTATTCCTTTAG ATGGTACCGGTGAGAATTTATATTTTCAAGGCTCTGGAGGTGGTGGT TCAGATTATAAAGATGATGACGACAAAGGAACCGGTTAA | |
| 105 | ATGGTACCAAGAAGAAGTGCAAACTATCAACCTTCATTATGGCAAC ATGAATACTTATTATCATTAGGCAACACTTATGTTAAAGAAGATAAT GTTGAAAGAGTAACTCTTTTAAAACAAGAAGTTTCTAAAATGTTAAA CGAAACAGAAGGTTTACTTGAACAACTTGAATTAATTGACACTTTAC AAAGATTAGGTGTTTCTTATCATTTTGAACAGGAGATTAAAAAAACA TTAACTAATGTTCATGTTAAAAACGTACGTGCTCATAAAAATCGTAT TGATCGTAACCGTTGGGGCGATTTATATGCAACTGCATTAGAATTTC GTTTATTACGTCAACATGGTTTTTCTATTGCTCAAGACGTTTTTGATG GTAATATTGGTGTTGACTTAGACGACAAAGACATTAAAGGTATTTTA AGTTTATACGAAGCTAGTTACTTATCAACACGTATTGATACAAAACT TAAAGAATCAATCTATTACACAACAAAACGTTTAAGAAAATTCGTA | Myrcene, ocimene (A. thalania) |
| | GAGGTAAACAAAAACGAAACTAAAAGTTACACTCTTCGTCGTATGG TTATTCACGCACTTGAGATGCCTTATCACCGTCGTGTTGGTCGTCTTG AAGCTCGTTGGTATATCGAGGTATATGGAGAAAGACACGACATGAA TCCTATTTTATTAGAATTAGCTAAATTAGATTTTAACTTTGTTCAGGC TATCCACCAAGACGAATTAAAATCATTATCTAGTTGGTGGTCTAAAA CAGGATTAACAAAACATTTAGACTTTGTTCGTGATCGTATTACAGAG GGTTACTTCAGTAGTGTAGGTGTTATGTATGAACCAGAATTTGCATA TCATCGTCAAATGCTTACAAAAGTATTTATGCTTATTACAACTATTG ATGACATCTATGACATTTACGGTACACTTGAAGAATTACAATTATTC ACAACTATCGTTGAAAAATGGGATGTTAATCGTTTAGAAGAACTTCC TAACTATATGAAATTATGCTTCTTATGTTTAGTTAACGAAATAAATC AAATTGGATATTTTGTATTAAGAGATAAAGGTTTTAATGTAATTCCT TATCTTAAAGAGTCTTGGGCTGACATGTGTACTACATTTCTTAAAGA AGCTAAATGGTACAAATCAGGTTATAAACCAAATTTTGAAGAGTAT ATGCAAAATGGCTGGATTTCATCATCAGTTCCAACTATTCTTTTACA CTTATTTTGTTTATTAAGTGACCAAACTTTAGACATTCTTGGTTCTTA TAATCACAGTGTTGTTCGTAGTTCAGCAACAATTTTACGTCTTGCAA ATGATTTAGCTACTTCTTCAGAAGAATTAGCAAGAGGAGATACAAT GAAATCAGTTCAATGTCACATGCATGAAACTGGTGCTTCAGAAGCTG AATCAAGAGCTTACATTCAAGGTATTATTGGCGTAGCTTGGGATGAC CTTAATATGGAGAAAAAATCATGTCGTTTACACCAGGGATTCTTAGA AGCAGCAGCAAATTTAGGACGTGTAGCACAATGCGTATATCAATAT GGAGACGGTCACGGTTGTCCAGATAAAGCAAAAACAGTAAATCATG TTCGTAGTTTATTAGTTCACCCATTACCATTAAACGGTACCGGTGAA AACCTTTATTTTCAAGGTAGTGGTGGAGGTGGTTCTGATTATAAAGA CGACGATGACAAAGGAACCGGTTAA | |
| 106 | ATGGTACCAGCTTCTCCACCTGCTCATCGTTCATCTAAAGCAGCAGA CGAAGAGTTACCAAAAGCATCTTCTACATTCCATCCATCTCTTTGGG GTTCATTTTTCTTAACATATCAGCCACCTACAGCTCCACAACGTGCA AATATGAAAGAACGTGCTGAAGTTCTTCGTGAACGTGTTCGTAAAGT ATTAAAAGGTTCAACAACAGATCAATTACCTGAAACAGTTAACTTA ATTCTTACATTACAAAGACTTGGTTTAGGTTATTACTATGAAAATGA AATTGACAAATTACTTCATCAAATTTACTCTAATTCAGATTATAACG TAAAAGACTTAAACTTAGTTTCTCAACGTTTTTACTTACTTCGTAAAA ACGGTTATGACGTACCTTCTGATGTTTTCTTATCTTTTAAAACTGAAG AAGGTGGTTTCGCTTGTGCTGCAGCTGACACACGTTCACTTTTAAGT TTATACAATGCTGCTTACCTTCGTAAACATGGTGAAGAAGTATTAGA TGAAGCAATTTCATCAACACGTTTAAGATTACAAGACTTATTAGGTC GTTTATTACCTGAATCACCATTCGCTAAAGAAGTATCAAGTTCACTT CGTACACCTTTATTCCGTCGTGTAGGTATTTTAGAAGCTCGTAACTAT | Sesquiterpene (Z. mays; B73) |
| | ATTCCAATCTATGAAACTGAAGCTACAAGAAATGAAGCTGTATTAG AGCTTGCTAAACTTAACTTCAATTTACAACAGCTTGATTTCTGTGAA GAATTAAAACATTGTAGTGCATGGTGGAATGAGATGATTGCTAAAA GTAAATTAACTTTTGTACGTGACCGTATAGTTGAAGAATACTTTTGG ATGAATGGTGCATGTTATGATCCACCATATTCATTAAGTCGTATTAT TCTTACAAAAATCACTGGTTTAATTACTATTATTGATGATATGTTCGA TACTCATGGTACAACAGAGGATTGCATGAAATTCGCAGAAGCATTT GGTCGTTGGGATGAATCAGCAATTCATCTTCTTCCAGAATACATGAA AGATTTTTACATTTTAATGTTAGAAACTTTCCAGTCATTTGAAGATGC ACTTGGTCCAGAAAAATCATACCGTGTATTATACTTAAAACAAGCAA TGGAACGTTTAGTAGAGTTATATTCTAAAGAAATCAAATGGCGTGAT GACGATTATGTTCCAACAATGTCAGAACATTTACAAGTTAGTGCTGA AACAATTGCTACAATTGCTTTAACTTGCTCTGCTTATGCTGGTATGG GTGATATGTCTATTCGTAAAGAAACATTTGAATGGGCATTATCTTTC CCTCAATTCATTAGAACTTTTGGTTCATTTGTACGTTTATCAAATGAT GTTGTATCAACAAAACGTGAACAAACTAAAGATCATTCACCTTCAAC AGTTCACTGTTATATGAAAGAACACGGTACAACTATGGACGATGCTT GTGAAAAAATCAAAGAATTAATTGAGGACTCATGGAAAGACATGTT AGAACAATCTTTAGCTCTTAAAGGCTTACCTAAAGTAGTACCTCAAT TAGTTTTTGATTTCTCTCGTACTACAGATAACATGTATCGTGACCGTG ATGCTTTAACATCATCAGAAGCATTAAAAGAAATGATACAGTTATTA TTCGTAGAACCTATACCTGAAGGTACCGGTGAGAATCTTTATTTTCA AGGATCAGGTGGTGGAGGCTCAGATTACAAAGATGACGACGATAAA GGAACCGGTTAA | |
| 107 | ATGGTACCAGAGGCTTTAGGAAATTTTGATTATGAGAGTTATACTAA TTTTACAAAATTACCATCATCACAATGGGGTGATCAATTCCTTAAAT TTTCTATAGCAGATTCTGACTTCGATGTATTAGAAAGAGAAATAGAA GTATTAAAACCAAAAGTAAGAGAGAACATTTTTGTTTCATCAAGTAC TGATAAAGATGCAATGAAAAAAACAATTTTAAGTATTCATTTCTTAG ATAGTTTAGGTTTATCTTATCACTTCGAAAAAGAAATAGAGGAGAGT TTAAAACATGCTTTCGAGAAAATTGAAGACCTTATTGCTGATGAAAA TAAACTTCATACAATAAGTACAATTTTCCGTGTATTCCGTACATACG GCTATTATATGTCTTCTGATGTATTCAAAATTTCAAAGGAGACGAT GGTAAATTCAAAGAAAGTTTAATTGAAGACGTTAAAGGTATGCTTTC TTTTTATGAAGCTGTTCATTTTGGAACAACTACTGATCACATTTTAGA CGAAGCTCTTAGTTTTACATTAAACCACTTAGAGTCACTTGCAACAG GCCGTCGTGCATCACCACCACATATTAGTAAATTAATCCAAAATGCT TTACATATTCCTCAACATCGTAACATCCAGGCATTAGTAGCTCGTGA ATACATTAGTTTTTACGAACACGAAGAAGATCACGATGAAACATTAT TAAAATTAGCTAAATTAAACTTTAAATTCTTACAACTTCACTATTTTC | Sesquiterpene (A. thalania) |
| | AAGAATTAAAAACAATTACAATGTGGTGGACTAAATTAGATCATAC ATCTAATTTACCACCAAATTTTCGTGAACGTACAGTTGAAACATGGT TTGCAGCTTTAATGATGTATTTCGAACCACAATTTAGTTTAGGTCGT ATTATGAGTGCAAAATTATATTTAGTAATTACTTTCTTAGATGACGC ATGTGATACATACGGATCAATATCTGAAGTAGAGTCATTAGCTGATT GTTTAGAACGTTGGGACCCAGATTATATGGAAAATTTACAAGGTCAC ATGAAAACAGCATTCAAATTCGTTATGTATTTATTCAAAGAATACGA AGAAATTTTACGTTCACAAGGCCGTTCATTCGTATTAGAGAAAATGA TTGAGGAGTTTAAAATTATCGCACGTAAAAACTTAGAACTTGTAAAA TGGGCTCGTGGTGGTCACGTTCCTTCTTTTGACGAATATATAGAGAG TGGTGGTGCTGAGATTGGTACTTATGCTACAATCGCTTGTTCAATTA TGGGTCTTGGTGAAATTGGTAAAAAAGAAGCATTTGAGTGGTTAATC TCTCGTCCTAAACTTGTTCGTATTTTAGGTGCTAAAACACGTTTAATG GATGATATCGCAGACTTTGAAGAAGACATGGAAAAAGGCTATACAG CTAATGCACTTAACTATTATATGAATGAACACGGAGTAACTAAAGA AGAAGCTAGTCGTGAACTTGAGAAAATGAATGGTGATATGAACAAA ATTGTAAACGAAGAATGTCTTAAAATTACAACTATGCCACGTCGTAT CTTAATGCAAAGTGTTAACTACGCTCGTAGTTTAGATGTATTATACA CAGCTGATGATGTATATAACCACCGTGAAGGCAAACTTAAAGAATA TATGAGATTACTTTTAGTAGATCCAATTTTACTTGGTACCGGTGAAA ATCTTTATTTTCAAGGTTCAGGTGGTGGTGGTTCTGATTATAAAGAT GATGACGATAAAGGAACCGGTTAA | |
| 108 | ATGGTACCAGAGAGTCAAACAACATTCAAATACGAATCATTAGCAT TTACAAAACTTAGTCACTGTCAATGGACAGACTATTTCTTAGTGTT CCAATTGATGAAAGTGAATTAGATGTTATTACTCGTGAAATTGATAT TCTTAAACCAGAAGTTATGGAGTTATTAAGTAGTCAAGGAGATGAT GAAACAAGTAAAAGAAAAGTTCTTCTTATTCAGTTATTACTTTCTTT AGGTTTAGCATTCCACTTTGAAAATGAGATTAAAAACATACTTGAAC ACGCATTTCGTAAAATAGATGATATAACTGGTGACGAAAAAGACTT ATCAACAATTAGTATTATGTTCCGTGTTTTCCGTACTTATGGACACA ATCTTCCAAGTAGTGTTTTTAAACGTTTCACAGGTGATGATGGTAAA TTTCAGCAAAGTTTAACAGAAGACGCAAAAGGTATTTTAAGTTTATA TGAAGCTGCACATTTAGGTACTACTACAGATTACATTTTAGATGAAG CTCTTAAATTCACATCTAGTCACTTAAAAAGTTTACTTGCTGGTGGT ACATGTCGTCCTCACATCTTACGTTTAATCCGTAATACATTATACTTA CCACAACGTTGGAACATGGAAGCTGTTATCGCTCGTGAATACATATC ATTTTACGAGCAGGAAGAAGATCACGATAAAATGCTTTTACGTCTTG CAAAACTTAACTTTAAACTTCTTCAATTACACTACATTAAAGAGCTT AAAAGTTTCATTAAATGGTGGATGGAACTTGGTTTAACTTCTAAATG GCCTTCTCAATTTCGTGAACGTATTGTTGAAGCATGGTTAGCTGGAT | Sesquiterpene (A. thalania) |
| | TAATGATGTATTTTGAACCACAGTTCTCAGGTGGTCGTGTTATTGCT GCAAAATTCAACTATTTACTTACAATATTAGACGACGCATGTGACCA CTATTTTTCTATTCACGAATTAACACGTTTAGTTGCATGTGTAGAACG TTGGTCACCAGATGGTATTGACACATTAGAAGATATTTCACGTTCTG TATTCAAATTAATGTTAGATGTTTTCGACGATATTGGTAAAGGTGTA CGTTCAGAAGGTTCTAGTTACCACTTAAAAGAAATGTTAGAGGAATT AAACACTTTAGTTCGTGCTAATTTAGATTTAGTTAAATGGGCTCGTG GAATACAAACAGCTGGTAAAGAGGCTTATGAATGGGTTCGTTCACG TCCACGTTTAATCAAATCTTTAGCAGCTAAAGGTAGACTTATGGATG ATATTACAGACTTTGACTCAGATATGAGTAATGGATTCGCAGCTAAT GCTATTAACTACTATATGAAACAATTTGTTGTTACAAAAGAAGAAGC TATTCTTGAATGTCAACGTATGATTGTAGACATTAACAAAACTATTA ATGAAGAGTTATTAAAAACTACTTCAGTTCCAGGTCGTGTATTAAAA CAAGCTCTTAACTTTGGCCGTTTATTAGAATTATTATATACAAAATCT GACGATATTTACAATTGTTCTGAAGGCAAACTTAAAGAATACATTGT AACTCTTTTAATTGATCCTATAAGACTTGGTACCGGTGAAAACTTAT ACTTTCAAGGTTCAGGCGGTGGTGGTAGTGATTACAAAGATGATGAT GACAAAGGAACCGGTTAA | |
| 109 | ATGGTACCAGAGAGTCAAACAAAATTCGACTACGAATCATTAGCTTT TACAAAATTATCACATTCACAATGGACTGATTACTTTTATCAGTAC CTATAGACGACTCTGAACTTGACGCAATTACTCGTGAAATCGACATT ATCAAACCTGAAGTTCGTAAATTACTTTCAAGTAAAGGTGATGATGA AACTTCTAAACGTAAAGTATTACTTATCCAAAGTTTATTATCATTAG GTTTAGCATTTCATTTTGAAAACGAAATTAAAGATATTTTAGAAGAT GCATTTAGACGTATTGATGACATTACAGGTGATGAAAACGACTTAA GTACTATTAGTATTATGTTCCGTGTATTCCGTACATACGGTCACAATT TACCAAGTAGTGTTTTTAAACGTTTCACTGGTGATGACGGTAAATTT GAACGTTCTTTAACTGAAGATGCTAAAGGAATTTTATCATTATATGA AGCTGCACATTTAGGAACAACTACTGATTATATTCTTGATGAAGCAT TAGAATTTACTTCATCACACTTAAAATCTTTACTTGTTGGTGGTATGT GTCGTCCACATATTTTACGTCTTATTAGAAATACTTTATATCTTCCAC AACGTTGGAATATGGAAGCAGTAATTGCAAGAGAATACATTAGTTT TTATGAACAAGAAGAAGATCACGATAAAATGTTACTTCGTTTAGCTA AATTAAATTTCAAATTACTTCAATTACACTACATTAAAGAGTTAAAA ACATTCATTAAATGGTGGATGGAATTAGGACTTACATCAAAATGGCC TTCTCAATTTCGTGAACGTATTGTTGAAGCATGGTTAGCTGGTCTTAT GATGTATTTTGAACCACAGTTTTCTGGAGGTCGTGTAATAGCTGCTA AATTCAATTACTTATTAACAATTTTAGATGATGCATGTGATCACTATT TCTCAATTCCAGAATTAACTCGTTTAGTTGATTGCGTAGAAAGATGG AATCATGATGGTATACATACTTTAGAAGACATCTCACGTATCATCTT | Sesquiterpene (A. thalania) |
| | TAAACTTGCATTAGATGTATTTGATGATATTGGTCGTGGTGTTCGTTC TAAAGGTTGTTCTTATTACTTAAAAGAAATGTTAGAAGAGTTAAAAA TCTTAGTTCGTGCAAACTTAGATTTAGTTAAATGGGCTCGTGGTAAT CAATTACCTAGTTTTGAAGAACACGTTGAGGTAGGTGGTATTGCTCT TACAACATACGCAACTTTAATGTACTCTTTTGTTGGCATGGGTGAAG CAGTAGGTAAAGAAGCATACGAATGGGTACGTTCTCGTCCACGTTTA ATCAAAAGTTTAGCAGCAAAAGGTCGTCTTATGGACGATATTACTGA TTTCGAAGTAAAAATTATCAACTTATTTTTCGACCTTCTTTTATTTGT ATTCGGTACCGGTGAAAACTTATATTTCCAGGGTAGTGGTGGAGGA GGTTCAGACTACAAAGATGACGATGACAAAGGAACCGGTTAA | |
| 110 | ATGGTACCAGCAGCTTTCACAGCAAATGCAGTTGACATGCGTCCACC AGTTATTACAATTCACCCACGTTCAAAAGATATTTCTCTCAATTTTC TTTAGATGATAAATTACAAAAACAATACGCTCAAGGAATCGAAGCT CTTAAAGAAGAAGCTCGTTCTATGCTTATGGCTGCAAAATCTGCTAA AGTAATGATCTTAATTGATACACTTGAACGTTTAGGATTAGGTTATC ACTTTGAAAAAGAAATTGAAGAGAAATTAGAAGCTATTTACAAAAA AGAGGATGGTGACGATTATGATCTTTTTACAACTGCTTTAAGATTCC GTTTACTTAGACAACACCAACGTCGTGTACCATGTTCTGTTTTTGAC AAATTTATGAATAAAGAGGGTAAATTCGAAGAAGAACCATTAATTT CAGATGTTGAAGGTCTTCTTTCATTATATGACGCTGCTTATTTACAGA TTCACGGTGAACACATTTTACAAGAGGCTTTAATTTTCACTACACAT CATTTAACTCGTATTGAACCACAATTAGATGATCACTCTCCTTTAAA ATTAAAATTAAACCGTGCTTTAGAATTTCCTTTTTACAGAGAAATCC CTATAATCTATGCACATTTTTACATTTCAGTATATGAACGTGACGATT CTCGTGATGAAGTATTATTAAAAATGGCTAAATTATCTTATAATTTC TTACAAAACTTATACAAAAAAGAATTAAGTCAACTTTCTCGTTGGTG GAACAAATTAGAACTTATTCCTAATTTACCTTATATTCGTGATTCTGT AGCTGGAGCTTATTTATGGGCTGTTGCTTTATATTTCGAACCTCAATA TTCAGACGTTCGTATGGCAATTGCTAAACTTATCCAAATTGCAGCAG CTGTAGATGATACTTACGATAATTATGCTACTATACGTGAAGCTCAA TTATTAACAGAAGCATTAGAACGTTTAAATGTACACGAAATTGACAC ATTACCAGATTATATGAAAATTGTTTATCGTTTTGTAATGTCATGGA GTGAAGATTTCGAACGTGATGCTACAATTAAAGAACAGATGTTAGC TACACCTTATTTCAAAGCTGAAATGAAAAAACTTGGTCGTGCTTATA ATCAAGAACTTAAATGGGTTATGGAACGTCAATTACCTAGTTTCGAA GAATACATGAAAAACTCTGAAATCACTTCTGGTGTTTACATTATGTT TACTGTAATTAGTCCTTACTTAAATAGTGCAACACAAAAAAACATTG ACTGGTTATTATCACAACCTCGTTTAGCATCTTCAACTGCAATTGTTA TGCGTTGTTGTAATGATTTAGGCTCTAATCAACGTGAATCTAAAGGA GGAGAAGTTATGACATCTTTAGATTGCTATATGAAACAACACGGTGC | Curcumene (P. cablin) |
| | TAGTAAACAAGAAACAATTTCTAAATTCAAACTTATTATCGAAGATG AATGGAAAAACTTAAATGAAGAATGGGCTGCAACAACATGTCTTCC AAAAGTTATGGTAGAAATTTTTCGTAACTATGCACGTATTGCAGGCT TTTGCTACAAAAATAACGGTGATGCTTATACATCTCCAAAAATTGTA CAACAATGTTTTGACGCTTTATTTGTAAATCCATTAAGAATTGGTAC CGGTGAGAATTTATACTTTCAAGGCTCAGGTGGAGGTGGTAGTGATT ATAAAGATGATGATGATAAAGGAACCGGTTAA | |
| 111 | ATGGTACCAGAATTTAGAGTTCATTTACAGGCTGATAATGAACAGA AAATATTCCAGAACCAAATGAAACCTGAACCTGAAGCATCATATCTT ATTAATCAACGTAGATCAGCTAATTACAAACCTAATATTTGGAAAAA TGACTTTTTAGATCAAAGTTTAATTAGTAAATACGACGGTGATGAAT ATCGTAAATTAAGTGAGAAATTAATCGAGGAAGTAAAAATTTATAT ATCTGCTGAGACAATGGACTTAGTAGCTAAATTAGAACTTATTGATT CTGTTCGTAAATTAGGTTTAGCTAATCTTTTTGAAAAAGAAATTAAA GAAGCATTAGATTCTATCGCAGCTATTGAGTCAGATAATTTAGGTAC TCGTGATGACTTATATGGTACTGCTTTACACTTTAAAATTTTACGTCA ACATGGTTATAAAGTTTCTCAAGATATTTTTGGTCGTTTCATGGATG AAAAAGGTACATTAGAAAATCATCACTTCGCTCACTTAAAAGGTAT GTTAGAATTATTTGAAGCATCTAATTTAGGTTTTGAAGGTGAAGATA TTTTAGATGAAGCAAAAGCATCACTTACATTAGCTCTTCGTGATAGT GGTCATATTTGTTATCCAGATTCTAACTTAAGTCGTGATGTAGTACA CTCATTAGAATTACCTAGTCACCGTCGTGTTCAATGGTTTGATGTTA AATGGCAAATTAATGCTTATGAAAAAGATATTTGTAGAGTTAATGCA ACTCTTTTAGAATTAGCAAAATTAAATTTTAACGTAGTACAAGCACA ACTTCAAAAAAACTTACGTGAAGCATCTCGTTGGTGGGCTAACTTAG GTTTCGCTGATAACTTAAAATTCGCTCGTGATCGTTTAGTTGAATGTT TTTCTTGCGCAGTAGGCGTAGCATTTGAACCTGAACACTCTTCTTTC GTATCTGTTTAACAAAAGTTATTAATTTAGTTTTAATAATTGATGAC GTATACGACATATATGGAAGTGAAGAAGAATTAAAACACTTTACAA ATGCTGTTGATCGTTGGGATTCTCGTGAAACAGAACAATTACCAGAA TGTATGAAAATGTGCTTTCAAGTTTTATACAATACTACATGTGAAAT TGCTCGTGAAATTGAAGAAGAAAATGGATGGAATCAAGTTTTACCT CAATTAACTAAAGTATGGGCTGATTTTTGTAAAGCATTATTAGTAGA AGCTGAATGGTACAATAAAAGTCACATCCCAACTTTAGAAGAATAT CTTCGTAATGGCTGTATTTCATCAAGTGTTTCTGTATTATTAGTACAT TCTTTCTTTAGTATTACACATGAAGGTACAAAAGAAATGGCAGATTT CTTACACAAAAACGAAGACTTATTATACAACATCTCATTAATTGTAC GTTTAAACAACGACTTAGGTACAAGTGCAGCTGAACAAGAACGTGG TGATTCACCATCATCTATTGTATGTTACATGCGTGAAGTTAATGCTA GTGAAGAAACAGCTCGTAAAAATATAAAAGGAATGATCGACAATGC | Farnesene (M. domestica) |
| | TTGGAAAAAAGTTAATGGTAAATGTTTTACAACTAATCAAGTTCCTT TTCTTTCTTCTTTTATGAATAACGCTACTAATATGGCTCGTGTAGCTC ATTCATTATATAAAGACGGAGACGGTTTTGGCGATCAGGAAAAAGG TCCACGTACTCACATCTTATCTTTATTATTCCAACCATTAGTTAACGG TACCGGTGAAAACTTATACTTTCAAGGTTCTGGTGGTGGTGGTTCTG ACTACAAAGATGACGATGACAAAGGAACCGGTTAA | |
| 112 | ATGGTACCAAGTAGTAATGTATCAGCTATTCCTAATTCTTTTGAATT AATTCGTCGTTCAGCTCAATTTCAGGCTTCTGTATGGGGTGATTACTT TTTATCTTATCACTCTTTACCACCTGAGAAAGGTAATAAAGTAATGG AAAAACAAACTGAAGAACTTAAAGAGGAAATCAAAATGGAATTAGT TTCTACTACTAAAGATGAACCAGAGAAATTACGTTTAATTGACCTTA TTCAACGTTTAGGTGTATGTTATCACTTTGAAAATGAAATTAACAAC ATTTTACAACAATTACACCACATTACTATTACTTCTGAGAAAAACGG TGACGATAATCCTTATAACATGACTTTATGTTTCCGTTTATTACGTCA ACAAGGTTACAATGTATCTAGTGAACCTTTTGATCGTTTTCGTGGCA AATGGGAATCTTCTTATGATAACAATGTAGAAGAACTTTTATCATTA TATGAAGCATCTCAATTAAGAATGCAAGGTGAAGAAGCATTAGATG AAGCATTCTGTTTTGCAACTGCACAATTAGAAGCTATTGTTCAAGAT CCTACTACAGATCCAATGGTTGCAGCAGAAATCAGACAAGCATTAA AATGGCCAATGTACAAAAACTTACCTCGTTTAAAAGCTCGTCATCAT ATTGGTTTATATTCTGAGAAACCATGGCGTAATGAGTCATTACTTAA TTTCGCAAAAATGGACTTCAATAAACTTCAAAATTTACATCAAACTG AAATTGCATATATTTCTAAATGGTGGGACGATTACGGCTTTGCAGAA AAACTTTCTTTCGCACGTAATCGTATTGTTGAAGGCTATTTCTTCGCA TTAGGTATCTTTTTCGAACCTCAACTTTTAACAGCACGTCTTATAATG ACAAAAGTAATCGCTATTGGTTCTATGTTAGATGACATTTATGATGT TTATGGTACTTTTGAAGAGTTAAAACTTTTAACATTAGCTTTAGAAC GTTGGGATAAATCAGAAACAAAACAATTACCTAATTACATGAAAAT GTACTACGAAGCATTATTAGATGTTTTTGAAGAAATTGAGCAAGAA ATGTCACAAAAAGAAACTGAAACAACACCATACTGTATTCATCACA TGAAAGAAGCTACTAAAGAACTTGGACGTGTATTTTTAGTTGAAGCA ACTTGGTGTAAAGAAGGTTATACTCCTAAAGTAGAGGAATACTTAG ACATTGCTTTAATTTCTTTTGGTCATAAATTACTTATGGTAACTGCTT TATTAGGTATGGGTTCTCACATGGCTACACAACAAATTGTACAATGG ATTACATCTATGCCAAATATCTTAAAAGCATCTGCAGTAATATGTCG TTTAATGAATGACATTGTATCTCATAAATTTGAACAAGAACGTGGTC ATGTTGCTTCTGCTATCGAATGCTACATGGAACAAAACCACCTTAGT GAATATGAAGCATTAATTGCTCTTCGTAAACAAATTGATGATTTATG GAAAGACATGGTAGAAAATTACTGTGCAGTAATCACAGAAGACGAA GTACCTCGTGGTGTTTTAATGCGTGTTTTAAATCTTACACGTTTATTC | Farnesene (C. sativus) |
| | AATGTTATTTACAAAGACGGTGATGGATACACACAAAGTCATGGTA GTACAAAAGCTCACATTAAAAGTCTTTTAGTTGATAGTGTACCTCTT GGTACCGGTGAAAATCTTTACTTTCAAGGTTCAGGTGGAGGTGGTTC TGATTATAAAGATGATGATGACAAAGGAACCGGTTAA | |
| 113 | ATGGTACCAAAAGACATGAGTATTCCATTATTAGCAGCTGTATCTTC TAGTACAGAAGAAACAGTACGTCCTATCGCAGATTTTCATCCAACAC TTTGGGGTAATCATTTTCTTAAATCTGCTGCTGACGTAGAAACTATT GATGCAGCAACACAAGAGCAACACGCTGCATTAAAACAAGAAGTAC GTCGTATGATTACTACAACAGCAAATAAACTTGCACAAAAACTTCAC ATGATTGATGCTGTACAACGTTTAGGTGTTGCTTATCATTTTGAAAA AGAAATTGAAGACGAATTAGGTAAAGTAAGTCACGATTTAGATTCA GATGATTTATACGTTGTATCTTTACGTTTTCGTTTATTCCGTCAACAA GGTGTAAAAATTAGTTGCGATGTTTTCGACAAATTCAAAGATGACGA AGGAAAATTCAAAGAGTCTCTTATTAACGATATTAGAGGAATGTTAT CATTATACGAAGCAGCTTACTTAGCTATTAGAGGTGAAGATATTTTA GACGAAGCAATTGTTTTCACAACTACTCACTTAAAAAGTGTTATCTC TATTAGTGATCATTCACATGCTAATAGTAATTTAGCTGAACAAATAC GTCATAGTTTACAAATTCCACTTCGTAAAGCTGCTGCAAGATTAGAA GCACGTTATTTCTTAGATATTTACTCTCGTGATGATTTACATGATGAA ACATTACTTAAATTCGCTAAACTTGACTTTAACATTCTTCAAGCTGC ACACCAAAAAGAAGCTAGTATTATGACTCGTTGGTGGAACGATTTA GGTTTTCCTAAAAAAGTTCCTTATGCTCGTGACCGTATTATAGAAAC TTATATTTGGATGTTATTAGGAGTTTCATACGAACCTAATTTAGCATT TGGAAGAATTTTTGCAAGTAAAGTAGTATGTATGATTACAACAATTG ATGATACATTTGATGCTTATGGTACATTTGAAGAGTTAACATTATTC ACTGAAGCTGTTACACGTTGGGATATTGGTTTAATTGACACATTACC TGAATATATGAAATTCATTGTAAAAGCTCTTTTAGACATTTACCGTG AAGCTGAAGAAGAATTAGCTAAAGAAGGTAGATCATACGGTATTCC ATACGCTAAACAAATGATGCAAGAGTTAATCATTTTATACTTTACTG AGGCTAAATGGTTATACAAAGGTTACGTTCCTACATTTGACGAATAC AAAAGTGTAGCTTTACGTTCTATTGGTCTTAGAACATTAGCAGTAGC TTCATTTGTAGATTTAGGTGACTTTATTGCTACAAAAGACAATTTTG AATGTATTCTTAAAAATGCAAAAAGTTTAAAAGCTACTGAAACAATT GGCCGTTTAATGGATGATATAGCTGGTTACAAATTTGAACAGAAAC GTGGTCATAACCCATCTGCTGTTGAGTGTTACAAAAATCAACACGGA GTATCAGAAGAAGAAGCAGTTAAAGAGCTTTTATTAGAAGTTGCAA ACAGTTGGAAAGATATTAACGAGGAACTTTTAAATCCAACTACAGTT CCATTACCTATGTTACAGCGTTTATTATATTTTGCTCGTTCAGGTCAC TTCATCTATGATGATGGACATGATCGTTATACACATTCTTTAATGAT GAAAAGACAAGTTGCACTTTTATTAACTGAACCTTTAGCTATTGGTA | Farnesene (C. junos) |
| | CCGGTGAAAACTTATACTTTCAAGGTTCAGGTGGTGGTGGATCTGAT TATAAAGATGATGATGACAAAGGAACCGGTTAA | |
| 114 | ATGGTACCAGATTTAGCTGTTGAGATTGCAATGGACTTAGCTGTTGA TGACGTTGAGCGTCGTGTAGGTGACTATCATAGTAACCTTTGGGATG ATGATTTATTCAGAGTTTATCAACACCATACGGCGCATCATCATAT CGTGAACGTGCTGAAAGATTAGTAGGAGAAGTTAAAGAAATGTTTA CTTCTATTTCTATCGAAGATGGTGAACTTACATCTGATTTATTACAAC GTTTATGGATGGTAGATAATGTAGAGCGTTTAGGCATTTCACGTCAT TTCGAGAACGAAATAAAAGCAGCTATTGATTATGTTTATTCATATTG GAGTGACAAAGGTATTGTACGTGGTCGTGATTCAGCTGTTCCTGACT TAAATAGTATTGCTTTAGGTTTTCGTACATTACGTTTACACGGTTACA CAGTTAGTAGTGATGTATTTAAAGTTTTCCAAGATCGTAAAGGTGAA TTTGCTTGCAGTGCAATTCCAACTGAAGGAGATATTAAAGGAGTTTT AAACTTACTTCGTGCAAGTTATATTGCATTCCCTGGTGAAAAAGTAA TGGAAAAAGCTCAAACTTTTGCAGCAACATACCTTAAAGAAGCATT ACAGAAAATTCAAGTAAGTAGTTTAAGTCGTGAAATCGAATATGTTC TTGAATACGGTTGGTTAACTAACTTTCCTCGTTTAGAAGCACGTAAC TATATTGACGTATTCGGTGAAGAAATTTGTCCATACTTCAAAAAACC ATGTATTATGGTTGACAAACTTTTAGAATTAGCAAAATTAGAATTTA ACTTATTTCACAGTCTTCAACAAACAGAGTTAAAACATGTTAGTCGT TGGTGGAAAGATAGTGGTTTCTCTCAATTAACATTTACAAGACACCG TCATGTTGAGTTTTATACATTAGCTAGTTGTATAGCAATTGAACCAA AACACAGTGCTTTTCGTCTTGGTTTTGCTAAAGTTTGTTATTTAGGTA TAGTTTTAGATGATATTTATGACACATTTGGTAAAATGAAAGAATTA GAACTTTTTACTGCAGCAATCAAACGTTGGGACCCTTCTACTACAGA ATGCTTACCTGAATACATGAAAGGTGTTTATATGGCTTTTTACAATT GTGTTAATGAATTAGCACTTCAAGCAGAGAAAACACAAGGTCGTGA TATGTTAAACTATGCACGTAAAGCATGGGAAGCTCTTTTTGATGCAT TTTTAGAAGAAGCAAAATGGATCTCTTCTGGCTATTTACCAACATTC GAAGAATACTTAGAAAATGGTAAAGTATCTTTTGGTTATCGTGCTGC TACATTACAACCAATTTTAACATTAGATATTCCTTTACCTTTACATAT TTTACAACAGATTGATTTTCCAAGTCGTTTTAATGATTTAGCTTCATC TATTTTACGTTTAAGAGGTGATATCTGTGGTTACCAAGCTGAACGTA GTCGTGGTGAAGAAGCATCATCAATTTCATGTTATATGAAAGATAAT CCAGGTTCTACTGAAGAAGATGCATTATCTCACATTAATGCAATGAT CTCAGACAATATTAACGAATTAAACTGGGAACTTTTAAAACCAAATT CAAATGTACCAATTTCATCAAAAAAACATGCATTTGACATTCTTCGT GCTTTCTATCACTTATACAAATATCGTGATGGCTTCTCTATCGCAAA AATTGAAACTAAAAATCTTGTAATGCGTACAGTTTTAGAACCTGTAC CAATGGGTACCGGTGAAAACTTATACTTTCAGGGTTCTGGTGGAGGT | Farnesene (P. abies) |
| | GGTTCAGACTATAAAGATGATGATGATAAAGGAACCGGTTAA | |
| 115 | ATGGTACCAACAAGTGTATCAGTAGAATCAGGAACAGTATCTTGTTT ATCATCAAACAACTTAATTAGACGTACAGCTAATCCACATCCTAACA TTTGGGGATATGATTTTGTTCACTCACTTAAATCACCATATACACAC GACTCATCATATCGTGAACGTGCTGAGACTTTAATTTCAGAAATAAA AGTTATGCTTGGAGGTGGTGAATTAATGATGACTCCATCAGCTTATG ATACAGCATGGGTAGCTCGTGTTCCATCAATTGACGGTAGTGCTTGT CCACAATTTCCACAAACTGTTGAATGGATTCTTAAAAACCAATTAAA AGATGGTAGTTGGGGAACTGAATCTCACTTCTTACTTAGTGACAGAT TATTAGCTACATTAAGTTGTGTATTAGCATTATTAAAATGGAAAGTA GCTGATGTTCAAGTAGAGCAAGGTATTGAGTTTATCAAACGTAATTT ACAAGCTATTAAAGACGAACGTGATCAAGACAGTTTAGTAACTGAT TTCGAGATTATTTTCCCATCACTTTTAAAAGAGGCTCAATCTTTAAAC TTAGGCTTACCTTATGATTTACCATATATTAGATTATTACAAACAAA ACGTCAAGAACGTCTTGCTAACTTAAGTATGGATAAAATTCACGGTG GTACTTTATTATCATCTTTAGAGGGCATTCAAGATATAGTTGAATGG GAAACAATTATGGATGTACAATCTCAAGATGGTTCTTTCTTATCATC ACCAGCTTCTACAGCATGTGTATTCATGCATACAGGAGATATGAAAT GTTTAGATTTCTTAAACAACGTATTAACTAAATTTGGTAGTAGTGTT CCTTGTTTATACCCTGTAGATTTATTAGAACGTCTTTTAATTGTAGAT AATGTAGAGCGTCTTGGTATTGACCGTCATTTTGAAAAAGAAATCAA AGAGGCTTTAGATTATGTTTATCGTCATTGGAACGATCGTGGTATTG GTTGGGGTCGTTTATCACCTATCGCAGACTTAGAAACAACAGCTTTA GGTTTTCGTTTACTTCGTCTTCATCGTTACAATGTTTCTCCTGTAGTA TTAGACAATTTCAAAGACGCAGATGGCGAGTTCTTCTGCAGTACAGG TCAATTTAACAAAGATGTTGCAAGTATGTTATCTTTATACCGTGCTTC TCAATTAGCTTTCCCTGAAGAATCAATTTTAGATGAAGCTAAATCAT TCTCAACACAATATCTTCGTGAAGCATTAGAAAAATCAGAAACATTT TCTTCTTGGAATCATCGTCAGAGTTTATCAGAAGAAATTAAATATGC TTTAAAAACATCATGGCACGCTTCAGTTCCTCGTGTTGAAGCAAAAC GTTATTGTCAGGTTTACCGTCAAGACTATGCTCATTTAGCAAAATCA GTTTATAAACTTCCTAAAGTAAATAATGAGAAAATTCTTGAATTAGC AAAATTAGATTTTAACATTATTCAATCTATCCATCAAAAAGAAATGA AAAATGTTACATCATGGTTTCGTGATTCAGGCTTACCACTTTTCACAT TTGCTCGTGAAAGACCTTTAGAGTTTTACTTTTTAATCGCTGGTGGA ACATACGAACCTCAATACGCAAAATGTAGATTCTTATTTACAAAAGT AGCTTGTTTACAAACTGTTTTAGACGATATGTACGATACTTACGGTA CACCATCAGAGTTAAAATTATTTACTGAGGCAGTTCGTCGTTGGGAT TTATCATTCACAGAAAACTTACCTGATTATATGAAATTATGCTACAA AATTTACTATGATATTGTTCATGAAGTTGCTTGGGAAGTAGAAAAAG | Bisabolene (P. abies) |
| | AACAGGGACGTGAGCTTGTTTCATTTTTCCGTAAAGGTTGGGAAGAC TATCTTTTAGGTTATTATGAAGAAGCTGAATGGTTAGCTGCTGAATA CGTTCCTACTTTAGATGAATACATTAAAAACGGTATTACATCTATTG GTCAACGTATTTTACTTTTATCAGGTGTACTTATTATGGAAGGTCAA CTTTTATCACAAGAAGCTCTTGAAAAAGTAGATTATCCAGGTCGTCG TGTTTTAACAGAATTAAACAGTTTAATTAGTCGTTTAGCAGACGATA CTAAAACATACAAAGCAGAAAAAGCTCGTGGTGAACTTGCTAGTAG TATTGAATGTTATATGAAAGACCACCCTGGTTGTCAAGAAGAAGAA GCATTAAACCATATTTATGGCATTTTAGAACCAGCTGTTAAAGAATT AACTCGTGAGTTTCTTAAAGCAGATCACGTACCATTCCCTTGCAAAA AAATGTTATTTGATGAAACAAGAGTTACAATGGTAATTTTCAAAGAT GGTGATGGTTTCGGTATTTCTAAATTAGAAGTAAAAGACCACATAAA AGAATGTTTAATTGAGCCATTACCACTTGGTACCGGTGAAAATCTTT ATTTTCAAGGTAGTGGTGGTGGCGGTTCTGACTACAAAGATGACGAC GATAAAGGAACCGGTTAA | |
| 116 | ATGGTACCAGGTTCTGAAGTAAATAGACCTTTAGCAGACTTTCCAGC AAACATTTGGGAAGACCCATTAACTTCTTTCTCAAAATCTGATCTTG GTACAGAAACATTTAAAGAGAAACATAGTACTTTAAAAGAAGCTGT TAAAGAGGCATTTATGAGTTCTAAAGCTAATCCAATCGAAAATATCA AATTCATAGATGCATTATGCCGTTTAGGAGTATCTTATCACTTTGAA AAAGATATTGTAGAACAATTAGATAAATCATTTGATTGCTTAGATTT TCCACAAATGGTACGTCAAGAAGGTTGCGATTTATATACAGTTGGTA TTATCTTTCAAGTTTTTAGACAATTTGGTTTCAAATTAAGTGCTGATG TTTTTGAAAAATTCAAAGATGAAAATGGTAAATTCAAAGGTCACTTA GTAACTGATGCTTATGGTATGTTATCATTATACGAAGCTGCACAATG GGGTACTCACGGTGAAGACATCATTGACGAAGCTCTTGCTTTTTCTC GTAGTCACTTAGAAGAAATATCTAGTCGTAGTTCACCACACTTAGCA ATTCGTATTAAAAACGCTTTAAAACATCCATATCATAAAGGTATTTC ACGTATTGAAACACGTCAATACATTAGTTACTATGAAGAAGAA TCTTGTGATCCAACATTATTAGAGTTCGCTAAAATTGACTTTAACTTA TTACAAATTTTACACCGTGAAGAGTTAGCTTGTGTAACTCGTTGGCA TCATGAAATGGAATTTAAAAGTAAAGTAACTTACACACGTCATCGTA TTACAGAAGCATATTTATGGAGTCTTGGAACATATTTTGAACCACAA TACAGTCAAGCTCGTGTAATAACTACAATGGCATTAATCTTATTTAC TGCTTTAGACGACATGTACGATGCTTACGGTACTATGGAGGAGTTAG AGTTATTCACAGATGCTATGGACGAATGGTTACCAGTTGTTCCAGAT GAAATTCCTATTCCAGATTCAATGAAATTCATTTACAATGTTACAGT TGAATTTTACGATAAATTAGACGAAGAATTAGAAAAAGAAGGTCGT TCTGGTTGTGGTTTCCATCTTAAAAAAAGTTTACAAAAACAGCTAA TGGATATATGCAAGAAGCAAAATGGCTTAAAAAAGATTACATTGCT | Sesquiterpe ne (A. thalania) |
| | ACATTTGATGAGTATAAAGAAAATGCTATTTATCTTCAGGTTATTA TGCATTAATTGCAATGACATTTGTTCGTATGACTGATGTTGCTAAATT AGATGCTTTTGAATGGTTAAGTAGTCACCCAAAAATTCGTGTAGCAA GTGAAATCATTTCACGTTTTACAGACGATATTTCAAGTTATGAATTT GAACACAAACGTGAACACGTTGCTACAGGTATTGATTGTTATATGCA ACAATTCGGAGTTAGTAAAGAACGTGCTGTTGAAGTTATGGGCAAT ATAGTTTCTGATGCATGGAAAGACTTAAATCAAGAACTTATGCGTCC TCATGTTTTCCCATTTCCACTTCTTATGCGTGTTTTAAATCTTTCAAG AGTAATTGATGTATTTTATCGTTACCAAGATGCATATACTAATCCAA AATTACTTAAAGAGCACATTGTTTCTTTACTTATTGAAACTATTCCAA TTGGTACCGGTGAAAACTTATACTTTCAAGGTAGTGGTGGAGGTGGT TCTGATTATAAAGACGACGATGACAAAGGAACCGGTTAA | |
| 117 | ATGGTACCAGAGGCAATTAGAGTATTTGGCTTAAAACTTGGTTCAAA ATTATCTATTCACTCACAAACAAATGCTTTTCCTGCATTCAAATTATC TCGTTTTCCATTAACATCTTTCCCTGGTAAACATGCTCACTTAGATCC ATTAAAAGCAACAACTCATCCATTAGCTTTTGATGGTGAAGAAAATA ACCGTGAGTTTAAAAACTTAGGTCCAAGTGAGTGGGGCCATCAATTT CTTTCTGCTCATGTAGATTTATCTGAAATGGATGCATTAGAACGTGA AATTGAAGCTCTTAAACCAAAAGTACGTGATATGTTAATATCAAGTG AAAGTTCAAAAAAAAAAATCTTATTTCTTTATCTTTTAGTATCATTA GGATTAGCTTATCACTTTGAAGATGAAATTAAAGAAAGTTTAGAGG ATGGATTACAGAAAATTGAGGAAATGATGGCTTCAGAAGATGATCT TCGTTTTAAAGGCGATAATGGTAAATTCAAAGAATGTTTAGCAAAA GATGCTAAAGGTATTTTATCTCTTTATGAGGCTGCTCACATGGGTAC AACAACTGATTATATTCTTGATGAGGCTTTATCATTTACTTTAACATA TATGGAATCATTAGCAGCTTCAGGAACATGTAAAATCAACTTATCAC GTCGTATTAGAAAAGCATTAGATCAACCTCAACACAAAAATATGGA AATAATTGTAGCAATGAAATACATTCAATTTTATGAAGAAGAGGAA GATTGCGATAAAACTTTACTTAAATTTGCTAAACTTAACTTTAAATT CTTACAATTACACTATTTACAAGAACTTAAAATCTTATCTAAATGGT ATAAAGACCAAGACTTTAAATCAAAATTACCTCCATATTTCCGTGAC CGTCTTGTAGAATGTCATTTTGCATCATTAACATGTTTTGAGCCTAAA TATGCTCGTGCACGTATTTTCTTATCTAAAATCTTCACTGTTCAAATT TTCATTGACGATACTTGTGACCGTTACGCATCATTAGGTGAAGTTGA GTCATTAGCTGACACTATCGAACGTTGGGACCCTGATGATCATGCTA TGGACGGATTACCTGATTATCTTAAATCAGTAGTTAAATTTGTATTC AATACATTTCAAGAATTTGAACGTAAATGTAAACGTTCACTTCGTAT TAACTTACAAGTAGCAAAATGGGTTAAAGCTGGTCACTTACCATCTT TTGATGAGTATCTTGATGTAGCTGGTTTAGAATTAGCTATTTCATTCA CTTTCGCTGGTATCTTAATGGGCATGGAAAATGTTTGTAAACCTGAA | Sesquiterpe ne (A. thalania) |
| | GCATACGAATGGTTAAAATCTCGTGACAAACTTGTTCGTGGTGTAAT CACAAAAGTTCGTTTACTTAATGATATTTTTGGCTATGAAGATGATA TGCGTCGTGGTTATGTAACAAATTCAATAAACTGCTACAAAAAACA ATATGGAGTAACAGAGGAAGAAGCTATTCGTAAATTACATCAAATC GTTGCTGATGGAGAGAAAATGATGAATGAAGAGTTCTTAAAACCTA TTAATGTACCATATCAGGTTCCTAAAGTAGTTATTTTAGACACTTTAC GTGCAGCTAATGTTTCATACGAAAAAGATGACGAATTTACACGTCCA GGCGAACACCTTAAAAACTGCATTACATCTATTTACTTCGATTTAGG TACCGGTGAAAACTTATACTTTCAAGGTAGTGGTGGCGGTGGTAGTG ATTACAAAGATGATGATGATAAAGGAACCGGTTAA | |
| 118 | ATGGTACCAACTACAACATTATCATCTAACCTTAACTCACAATTCAT GCAGGTTTACGAGACTCTTAAATCAGAACTTATTCATGACCCATTAT TTGAGTTCGATGACGATTCAAGACAATGGGTAGAACGTATGATTGAT TATACTGTACCAGGTGGTAAAATGGTTCGTGGTTATAGTGTAGTAGA TAGTTATCAATTACTTAAAGGTGAAGAACTTACAGAAGAAGAGGCA TTTTTAGCTTGTGCACTTGGTTGGTGTACAGAATGGTTTCAAGCATTC ATTCTTTTACATGATGATATGATGGATGGTAGTCACACAAGACGTGG TCAACCATGTTGGTTTCGTTTACCTGAGGTTGGTGCTGTTGCTATTAA TGATGGTGTTTTACTTCGTAATCACGTTCACCGTATTCTTAAAAAAC ATTTTCAAGGTAAAGCATATTATGTTCATTTAGTTGATTTATTCAATG AAACTGAATTTCAAACAATTAGTGGACAAATGATCGACTTAATTACA ACATTAGTTGGTGAAAAAGACTTATCTAAATATTCATTAAGTATTCA TCGTCGTATCGTTCAATACAAAACAGCATACTACTCATTTTACTTAC CAGTTGCTTGTGCTTTACTTATGTTTGGTGAGGATCTTGATAAACATG TAGAAGTTAAAAATGTTCTTGTTGAAATGGGTACATATTTTCAAGTT CAAGATGATTATTTAGATTGTTTTGGTGCTCCAGAAGTTATTGGCAA AATTGGTACTGATATTGAAGACTTTAAATGTTCATGGTTAGTAGTTA AAGCATTAGAATTAGCAAATGAAGAACAGAAAAAACTTTACACGA AAATTATGGAAAAAAGATCCAGCATCAGTTGCTAAAGTTAAAGAA GTATACCACACACTTAATTTACAAGCTGTTTTCGAAGATTATGAAGC AACATCATACAAAAAACTTATTACTTCTATTGAAAATCACCCATCTA AAGCTGTTCAAGCTGTTTTAAAATCTTTCTTAGGCAAAATATACAAA CGTCAAAAAGGTACCGGTGAAAACTTATACTTTCAAGGTTCTGGTGG CGGTGGAAGTGATTACAAAGATGATGACGATAAAGGAACCGGTTAA | GPP Chimera |
| 119 | ATGGTACCAAGTCAACCTTACTGGGCTGCAATTGAAGCAGACATTG AAAGATATTTAAAAAAATCAATTACAATTCGTCCACCAGAAACTGT ATTTGGTCCTATGCACCATTTAACATTTGCTGCTCCTGCTACTGCAGC TAGTACATTATGCCTTGCTGCTTGTGAATTAGTTGGCGGTGATCGTA GTCAAGCTATGGCAGCTGCTGCTGCTATCCATTTAGTTCATGCAGCT GCTTACGTTCACGAACATCTTCCTTTAACAGATGGATCACGTCCTGT | GPPS-LSU+SSU fusion |
| | AAGTAAACCTGCTATTCAACATAAATATGGTCCAAACGTTGAACTTT TAACAGGTGATGGTATCGTTCCTTTCGGTTTTGAGTTATTAGCAGGTT CAGTAGATCCAGCACGTACTGATGACCCTGATCGTATTTACGTGTA ATTATTGAAATTTCTCGTGCTGGTGGACCAGAAGGCATGATTTCTGG TTTACACCGTGAGGAAGAAATCGTAGATGGTAACACATCATTAGAC TTTATAGAATATGTATGCAAAAAAAAATACGGTGAAATGCACGCAT GTGGTGCAGCTTGCGGAGCTATTTAGGTGGAGCTGCTGAAGAAGA AATTCAAAAACTTCGTAACTTTGGTCTTTATCAAGGCACATTACGTG GTATGATGGAAATGAAAAATAGTCATCAGTTAATTGACGAAAATAT CATTGGAAAACTTAAAGAACTTGCTCTTGAAGAATTAGGTGGATTCC ACGGTAAAAACGCTGAATTAATGAGTTCTTTAGTTGCTGAACCTAGT TTATATGCAGCTTCATCAAATAACTTAGGTATCGAAGGTCGTTTTGA CTTTGACGGTTACATGCTTCGTAAAGCAAAATCTGTAAATAAAGCAT TAGAAGCTGCTGTTCAAATGAAAGAACCACTTAAAATTCACGAATC AATGCGTTATTCATTATTAGCTGGTGGTAAACGTGTTCGTCCAATGT TATGTATTGCAGCTTGTGAACTTGTTGGTGGTGACGAATCTACAGCA ATGCCTGCAGCATGTGCTGTTGAAATGATTCACACAATGTCTTTAAT GCATGATGACCTTCCATGTATGGATAACGATGACTTACGTCGTGGTA AACCTACAAACCACATGGCTTTTGGTGAGTCTGTAGCTGTTCTTGCT GGTGATGCATTACTTAGTTTTGCTTTTGAACATGTTGCTGCTGCAACA AAAGGCGCACCACCTGAACGTATCGTACGTGTATTAGGTGAATTAG CTGTTAGTATTGGTTCAGAAGGACTTGTAGCAGGTCAAGTTGTAGAC GTTTGTTCTGAAGGCATGGCTGAAGTAGGATTAGATCATCTTGAATT TATTCACCATCATAAAACTGCTGCATTATTACAAGGTTCAGTTGTTTT AGGTGCAATATTAGGAGGCGGTAAAGAAGAAGTAGCTAAACTT CGTAAATTTGCTAACTGTATTGGTTTACTTTTCCAAGTTGTTGATGAT ATTTTAGATGTTACTAAAAGTAGTAAAGAGTTAGGTAAAACTGCAG GTAAAGACTTAGTAGCTGATAAAACTACATATCCTAAACTTATAGGC GTTGAAAAATCAAAAGAATTTGCTGACCGTTTAAATCGTGAAGCAC AAGAACAATTATTACATTTTCATCCTCACCGTGCTGCTCCATTAATC GCTTTAGCTAACTACATCGCTTACCGTGATAATGGTACCGGTGAAAA CTTATACTTCCAGGGTAGTGGTGGTGGCGGATCAGATTATAAAGATG ACGATGATAAAGGAACCGGTTAA | |
| 120 | ATGGTACCAGTAACAGCAGCACGTGCAACACCAAAATTAAGTAATA GAAAATTACGTGTTGCTGTAATTGGAGGCGGTCCAGCAGGAGGTGC AGCTGCTGAAACATTAGCACAAGGAGGTATTGAAACAATTCTTATC GAACGTAAAATGGATAATTGTAAACCATGTGGTGGTGCTATTCCATT ATGTATGGTAGGAGAGTTCAATTTACCTTTAGACATTATTGACCGTC GTGTAACAAAAATGAAAATGATCTCTCCTTCAAACATTGCAGTTGAT ATCGGTCGTACACTTAAAGAACACGAATATATTGGTATGGTTCGTCG | Geranyl-geranyl reductase (A. thalania) |
| | TGAGGTACTTGATGCTTATCTTCGTGAACGTGCAGAAAAATCAGGTG CTACTGTTATTAACGGTTTATTCTTAAAAATGGATCACCCAGAAAAT TGGGATTCACCATATACACTTCACTACACAGAGTATGATGGAAAAA CAGGTGCTACAGGAACTAAAAAAACTATGGAAGTAGATGCTGTTAT TGGTGCTGATGGTGCTAATTCTCGTGTTGCAAAAAGTATTGACGCAG GTGATTATGATTATGCTATTGCATTTCAAGAACGTATTCGTATACCT GATGAGAAAATGACTTATTATGAGGACTTAGCTGAGATGTATGTAG GTGATGATGTATCACCAGACTTCTACGGTTGGGTATTCCCAAAATGT GATCATGTAGCTGTTGGTACAGGTACTGTAACACATAAAGGTGATAT CAAAAAATTCCAGTTAGCTACACGTAATCGTGCTAAAGATAAAATTC TTGGTGGCAAAATAATCCGTGTAGAGGCTCATCCTATTCCAGAGCAT CCTAGACCACGTCGTTTATCAAAACGTGTTGCATTAGTAGGCGACGC AGCAGGTTACGTTACTAAATGTTCAGGAGAAGGAATTTACTTCGCAG CTAAATCTGGTCGTATGTGTGCTGAAGCTATCGTTGAAGGTTCACAA AATGGCAAAAAAATGATAGATGAAGGCGATTTAAGAAAATACTTAG AAAAATGGGATAAAACTTACTTACCAACTTATCGTGTTTTAGATGTA CTTCAAAAAGTTTTCTATCGTTCTAACCCAGCTCGTGAGGCTTTTGTT GAAATGTGTAACGATGAGTATGTACAGAAAATGACATTTGATTCTTA CCTTTATAAACGTGTAGCTCCTGGTAGTCCATTAGAAGATATCAAAT TAGCTGTAAATACTATTGGTTCACTTGTTCGTGCTAACGCATTACGTC GTGAAATTGAGAAATTATCAGTAGGTACCGGTGAGAATCTTTACTTT CAAGGATCAGGTGGTGGTGGTTCTGATTATAAAGATGACGATGATA AAGGAACCGGTTAA | |
| 121 | ATGGTACCAGTAGCTGTTATTGGTGGTGGTCCAAGTGGCGCTTGTGC AGCAGAAACTTTAGCAAAAGGTGGTGTAGAAACTTTCTTACTTGAGC GTAAATTAGATAATTGTAAACCTTGTGGAGGTGCAATTCCATTATGT ATGGTTGAAGAATTTGATTTACCAATGGAAATAATTGACCGTCGTGT TACTAAAATGAAAATGATATCACCTTCAAACCGTGAAGTTGATGTTG GAAAAACTTTATCAGAAACTGAATGGATCGGTATGTGTCGTCGTGA AGTATTTGACGATTACTTAAGAAACCGTGCACAGAAATTAGGTGCTA ATATTGTTAACGGTTTATTCATGCGTTCAGAACAACAATCTGCAGAG GGTCCATTCACAATTCACTATAATTCTTATGAAGACGGTAGTAAAAT GGGAAAACCTGCTACTTTAGAAGTTGATATGATAATTGGTGCAGATG GAGCAAATTCTCGTATTGCAAAAGAGATAGATGCAGGTGAATACGA CTACGCTATAGCTTTTCAAGAACGTATTCGTATTCCTGATGATAAAA TGAAATATTACGAAAACCTTGCTGAAATGTATGTAGGTGATGACGTA TCTCCTGATTTCTATGGTTGGGTTTTTCCTAAATATGATCACGTTGCT GTTGGTACAGGTACTGTTGTAAACAAAACAGCTATTAAACAATATCA ACAGGCAACACGTGACAGATCAAAAGTTAAAACAGAAGGTGGCAA AATTATACGTGTTGAAGCACACCCAATTCCAGAACATCCACGTCCAC | Geranyl-geranyl reductase (C. reinhardtii) |
| | GTCGTTGTAAAGGTCGTGTTGCATTAGTAGGCGACGCAGCTGGTTAT GTTACAAAATGTTCTGGCGAGGGCATTTACTTTGCTGCTAAATCTGG TAGAATGGCTGCTGAAGCTATTGTAGAAGGTTCTGCTAACGGTACAA AAATGTGTGGTGAGGATGCAATTCGTGTTTATTTAGATAAATGGGAT CGTAAATATTGGACAACATACAAAGTATTAGACATTTTACAAAAAG TATTTTATCGTAGTAATCCAGCACGTGAAGCATTTGTTGAATTATGT GAAGATAGTTATGTACAGAAAATGACATTTGATTCATACTTATATAA AACTGTTGTTCCAGGAAACCCATTAGACGACGTAAAATTACTTGTTC GTACAGTATCTTCTATTTTACGTTCAAATGCTTTACGTTCTGTTAATT CTAAATCTGTAAATGTTTCTTTCGGCTCTAAAGCAAATGAGGAACGT GTTATGGCTGCAGGTACCGGTGAAAATCTTTATTTTCAAGGTTCAGG AGGTGGTGGTTCAGATTATAAAGATGATGATGACAAAGGAACCGGT TAA | |
| 122 | ATGGTACCAGCAATGGCAGTACCATTAGATGTAGTAATTACATATCC TTCTTCAGGTGCTGCTGCTTATCCAGTACTTGTTATGTATAACGGTTT CCAAGCTAAAGCTCCATGGTATCGTGGTATTGTAGATCATGTTTCTA GTTGGGGTTACACAGTTGTTCAATATACAAATGGTGGCTTATTTCCT ATTGTTGTAGATCGTGTTGAGTTAACTTATTTAGAGCCATTATTAACT TGGTTAGAAACACAAAGTGCTGATGCTAAATCTCCTTTATACGGTCG TGCAGATGTTTCTCGTTTAGGTACAATGGGTCATTCACGTGGTGGTA AATTAGCAGCTTTACAATTTGCTGGACGTACAGATGTAAGTGGTTGT GTATTATTTGACCCTGTAGATGGAAGTCCAATGACACCAGAATCTGC TGATTATCCTTCAGCTACAAAAGCATTAGCAGCAGCTGGTCGTTCTG CTGGCTTAGTAGGTGCAGCTATTACAGGTTCATGTAATCCAGTAGGT CAAAATTACCCAAAATTCTGGGGTGCTTTAGCTCCTGGTTCTTGGCA AATGGTATTATCACAAGCTGGTCACATGCAATTTGCTCGTACTGGTA ATCCATTCTTAGATTGGTCATTAGACCGTTTATGTGGTCGTGGTACA ATGATGAGTTCAGATGTTATTACATATAGTGCAGCATTTACTGTTGC TTGGTTTGAAGGTATTTTTCGTCCTGCTCAAAGTCAAATGGGTATTTC TAATTTCAAAACTTGGGCTAATACTCAAGTTGCAGCTCGTAGTATCA CTTTTGATATTAAACCTATGCAATCTCCTCAGGGTACCGGTGAAAAC CTTTACTTTCAAGGTAGTGGTGGTGGAGGAAGTGATTATAAAGATGA TGATGACAAAGGAACCGGTTAA | Chloro-phyllido hydrolase (C. reinhardtii) |
| 123 | ATGGTACCAGCACCACCAAAACCAGTTCGTATAACTTGTCCAACAGT AGCTGGCACTTATCCTGTTGTTTATTCTTTCACGGTTTTATCTTCGT AACTATTTCTATTCAGATGTTTTAAATCATATTGCTAGTCATGGTTAC ATCTTAGTTGCACCACAATTATGTAAACTTTTACCTCCAGGTGGCCA AGTAGAAGTTGATGACGCTGGTTCAGTTATTAACTGGGCTTCAGAGA ATCTTAAAGCACACCTTCCAACTTCTGTTAATGCTAATGGTAAATAT ACATCTTTAGTTGGACATTCACGTGGTGGCAAAACAGCTTTCGCAGT | Chloro-phyllido hydrolase (A. thalania) |
| | TGCATTAGGTCACGCAGCTACATTAGATCCATCAATTACATTTTCAG CATTAATTGGTATTGATCCAGTAGCAGGAACTAACAAATACATTCGT ACAGATCCACACATCTTAACTTATAAACCTGAATCATTTGAATTAGA TATTCCTGTAGCTGTTGTAGGCACTGGTCTTGGTCCAAAATGGAATA ACGTAATGCCTCCATGCGCACCTACAGATTTAAACCACGAAGAATTT TACAAAGAATGTAAAGCTACTAAAGCTCACTTTGTTGCTGCTGATTA TGGTCACATGGACATGTTAGACGACGATCTTCCAGGTTTTGTAGGCT TCATGGCTGGTTGTATGTGTAAAAATGGTCAACGTAAAAAATCAGA AATGCGTTCTTTTGTAGGTGGTATAGTTGTAGCATTCTTAAAATATTC TTTATGGGGTGAAAAAGCTGAAATAAGATTAATTGTTAAAGATCCTA GTGTATCTCCTGCTAAATTAGACCCATCACCAGAATTAGAAGAAGCA TCAGGTATTTTTGTTGGTACCGGTGAAAATCTTTATTTTCAAGGTTCA GGTGGAGGTGGTTCTGATTATAAAGATGATGATGACAAAGGAACCG GTTAA | |
| 124 | ATGGTACCAGCTACACCAGTTGAAGAAGGTGATTATCCAGTTGTAAT GTTATTACATGGCTACCTTTTATATAATTCATTTATTCACAATTAAT GTTACATGTATCATCTCACGGTTTCATCTTAATTGCTCCACAATTATA CTCAATTGCTGGTCCTGATACTATGGATGAAATTAAAAGTACTGCTG AGATTATGGACTGGTTATCAGTTGGTTTAAATCACTTTTTACCAGCTC AAGTTACACCTAATTTATCTAAATTTGCATTATCTGGTCATAGTCGTG GTGGTAAAACTGCTTTTGCTGTAGCATTAAAAAAATTTGGTTATTCT TCAAACTTAAAAATTAGTACTTTAATTGGTATTGATCCAGTAGACGG AACAGGTAAAGGTAAACAAACTCCACCTCCTGTTTTAGCATATTTAC CTAATAGTTTTGACTTAGACAAAACACCAATTTTAGTAATTGGTTCA GGTTTAGGTGAAACTGCACGTAATCCTTTATTTCCTCCATGTGCTCCT CCAGGTGTTAACCACCGTGAGTTTTTCCGTGAATGTCAAGGTCCAGC ATGGCACTTTGTTGCTAAAGATTATGGTCATTTAGACATGCTTGATG ATGATACAAAAGGTATTCGTGGCAAATCTAGTTACTGTTTATGCAAA AATGGTGAAGAACGTCGTCCAATGCGTCGTTTCGTTGGTGGTTTAGT TGTTAGTTTTCTTAAAGCATATCTTGAAGGTGATGATCGTGAATTAG TAAAAATCAAAGATGGTTGTCATGAAGATGTACCTGTTGAAATTCAA GAATTTGAAGTAATTATGGGTACCGGTGAAAATCTTTACTTTCAAGG TTCAGGCGGTGGAGGTTCAGATTATAAAGATGATGATGACAAAGGA ACCGGTTAA | Chloro-phyllido hydrolase (A. thalania) |
| 125 | ATGGTACCAAGTCACAAAAAAAAAAACGTAATCTTCTTCGTAACTG ATGGTATGGGTCCTGCTTCTCTTTCAATGGCTCGTTCATTTAATCAAC ACGTTAATGATTTACCAATTGATGATATTTTAACATTAGATGAACAT TTTATTGGAAGTTCAAGAACACGTTCATCAGATTCACTTGTAACTGA CTCAGCTGCTGGAGCTACAGCTTTTGCTTGTGCACTTAAATCATACA ATGGTGCTATAGGTGTAGATCCACACCATCGTCCATGTGGAACTGTT | Phosphatase (S. cerevisiae) |
| | TTAGAAGCTGCTAAATTAGCAGGTTATTTAACAGGATTAGTAGTTAC TACACGTATTACTGATGCTACACCAGCTAGTTTCTCAAGTCACGTAG ATTATCGTTGGCAAGAAGATTTAATTGCAACACACCAATTAGGTGAA TATCCTTTAGGACGTGTTGTTGATCTTCTTATGGGTGGTGGTCGTTCT CACTTTTATCCTCAAGGTGAAAAAGCTAGTCCATACGGTCACCACGG TGCACGTAAAGATGGTCGTGATTTAATCGATGAAGCTCAAAGTAAT GGCTGGCAGTATGTAGGAGATCGTAAAAATTTTGATTCTTTACTTAA ATCACATGGTGAAAATGTTACTTTACCATTTTTAGGTTTATTTGCTGA CAACGATATCCCATTTGAAATTGATCGTGATGAAAAAGAATATCCTA GTTTAAAAGAACAAGTAAAAGTAGCATTAGGTGCTTTAGAAAAAGC AAGTAACGAAGATAAAGATAGTAATGGTTTCTTTTTAATGGTAGAA GGTTCTCGTATTGATCATGCTGGCCATCAAAACGATCCTGCATCTCA AGTACGTGAAGTATTAGCATTTGATGAGGCTTTTCAATATGTATTAG AATTTGCAGAAAACAGTGATACAGAAACAGTATTAGTAAGTACATC AGATCATGAAACAGGTGGTTTAGTTACTTCAAGACAAGTAACAGCA TCATACCCACAATATGTATGGTATCCTCAAGTATTAGCTAACGCTAC ACATAGTGGAGAGTTTCTTAAACGTAAATTAGTTGATTTCGTTCATG AACACAAAGGCGCATCATCAAAAATAGAAAACTTCATAAAACACGA AATTCTTGAAAAAGATTTAGGTATTTATGATTATACAGATTCTGACT TAGAAACACTTATTCATTTAGATGATAACGCTAATGCAATTCAAGAT AAACTTAATGATATGGTAAGTTTAGAGCTCAAATTGGTTGGACAAC ACATGGTCATTCAGCAGTTGATGTAAACATATATGCTTACGCAAACA AAAAAGCTACATGGTCTTATGTTCTTAATAACTTACAAGGTAATCAC GAAAACACAGAAGTTGGTCAATTCTTAGAGAATTTCTTAGAATTAAA CTTAAATGAAGTTACTGATTTAATCCGTGATACAAAACATACTTCTG ATTTTGACGCAACAGAAATAGCAAGTGAGGTTCAACACTATGATGA ATATTACCACGAATTAACAAATGGTACCGGTGAAAATCTTTATTTTC AAGGTTCTGGTGGAGGTGGCAGTGATTATAAAGATGATGATGACAA AGGAACCGGTTAA | |
| 126 | ATGGTACCACACAAGTTCACAGGTGTTAACGCTAAATTCCAGCAACC AGCATTAAGAAATTTATCTCCAGTGGTAGTTGAGCGCGAACGTGAG GAATTTGTAGGATTCTTTCCACAAATTGTTCGTGACTTAACTGAAGA TGGTATTGGTCATCCAGAAGTAGGTGACGCTGTAGCTCGTCTTAAAG AAGTATTACAATACAACGCACCTGGTGGTAAATGCAATAGAGGTTT AACAGTTGTTGCAGCTTACCGTGAACTTTCTGGACCAGGTCAAAAAG ACGCTGAAAGTCTTCGTTGTGCTTTAGCAGTAGGATGGTGTATTGAA TTATTCCAAGCCTTTTTCTTAGTTTGGGACGATATAATGGACCAGTC ATTAACTAGACGTGGTCAATTATGTTGGTACAAGAAAGAAGGTGTT GGTTTAGATGCAATAAATGATTCTTTTCTTTTAGAAAGCTCTGTGTAT CGCGTTCTTAAAAAGTATTGCCGTCAACGTCCATATTATGTACATTT | FPP A118W (G. gallus) |
| | ATTAGAGCTTTTTCTTCAAACAGCTTACCAAACAGAATTAGGACAAA TGTTAGATTTAATCACTGCTCCTGTATCTAAGGTAGATTTAAGCCATT TCTCAGAAGAACGTTACAAAGCTATTGTTAAGTATAAAACTGCTTTC TATTCATTCTATTTACCAGTTGCAGCAGCTATGTATATGGTTGGTATA GATTCTAAAGAAGAACATGAAAACGCAAAAGCTATTTTACTTGAGA TGGGTGAATACTTCCAAATTCAAGATGATTATTTAGATTGTTTTGGC GATCCTGCTTTAACAGGTAAAGTAGGTACTGATATTCAAGATAACAA ATGTTCATGGTTAGTTGTGCAATGCTTACAAAGAGTAACACCAGAAC AACGTCAACTTTTAGAAGATAATTACGGTCGTAAAGAACCAGAAAA AGTTGCTAAAGTTAAAGAATTATATGAGGCTGTAGGTATGAGAGCC GCCTTTCAACAATACGAAGAAAGTAGTTACCGTCGTCTTCAAGAGTT AATTGAGAAACATTCTAATCGTTTACCAAAAGAAATTTTCTTAGGTT TAGCTCAGAAAATATACAAACGTCAAAAAGGTACCGGTGAAAACTT ATACTTTCAAGGCTCAGGTGGCGGTGGAAGTGATTACAAAGATGAT GATGATAAAGGAACCGGTTAA | |

**Table 6. Nuleic acids encoding exemplary isoprenoid producing enzymes (with restriction enzyme sites**

| **SEQ ID NO** | **Codon-biased, Synthesized Gene Sequence w/ Cloning Sites** | **Enzyme encoded (synthetic)** |
|---|---|---|
| 127 | CATATGGTACCAAGACGTTCAGGTAACTATAATCCTAGCCGTTGGGA CGTAAATTTCATTCAATCTTTATTATCTGATTATAAAGAAGATAAAC ACGTTATTAGAGCTTCTGAATTAGTAACACTTGTTAAGATGGAATTA GAAAAAGAAACAGATCAAATCCGTCAATTAGAATTAATTGACGATT TACAACGTATGGGTTTATCTGATCATTTCCAAAACGAATTTAAAGAA ATCTTATCAAGTATTTACTTAGATCATCATTATTACAAAAATCCATTT CCAAAAGAAGAGCGTGATTTATACTCAACTAGCTTAGCTTTTCGTTT ATTACGTGAACACGGTTTTCAAGTAGCACAAGAAGTTTTTGATTCAT TCAAAAATGAAGAGGGTGAATTTAAGGAGAGCTTATCTGACGATAC TCGTGGCTTATTACAATTATATGAAGCATCATTCTTATTAACAGAGG GTGAAACAACCTTAGAAAGTGCACGCGAATTTGCTACAAAATTTTTA GAAGAAAAAGTTAACGAAGGTGGCGTTGATGGTGACTTATTAACAA GAATTGCTTACTCATTAGATATTCCCTTACATTGGCGCATTAAACGT CCTAATGCCCCAGTTTGGATTGAATGGTATCGTAAACGTCCAGATAT GAACCCAGTGGTTTTAGAATTAGCAATTTTAGACTTAAACATTGTAC AAGCTCAATTTCAAGAGGAATTAAAAGAGTCTTTTCGCTGGTGGCGT AATACTGGTTTTGTTGAGAAATTACCATTTGCACGTGATCGTTTAGTT GAATGTTACTTTTGGAACACTGGTATTATTGAACCACGTCAACACGC ATCAGCTCGTATTATGATGGGTAAAGTAAATGCATTAATTACAGTAA TTGATGACATCTATGATGTTTATGGAACACTTGAAGAATTAGAACAA TTCACTGATTTAATTCGCAGATGGGACATAAACTCAATAGATCAATT ACCAGATTATATGCAATTATGTTTTCTTGCATTAAACAATTTCGTTGA TGACACTTCATACGATGTTATGAAAGAAAAGGGTGTTAATGTTATTC CTTACTTACGTCAATCTTGGGTAGACCTTGCAGACAAATATATGGTA GAAGCACGTTGGTTCTACGGTGGCCATAAACCATCATTAGAAGAAT ACTTAGAAAATTCTTGGCAATCTATCTCAGGTCCATGTATGTTAACT CATATATTCTTTCGTGTAACAGATAGCTTTACTAAAGAAACTGTTGA TTCTCTTTACAAATATCATGATTTAGTTAGATGGTCATCATTCGTGCT TCGTCTTGCTGACGACTTAGGTACAAGCGTTGAAGAAGTATCTCGTG GTGATGTGCCAAAATCTTTACAATGCTACATGAGTGATTATAACGCT AGTGAGGCTGAAGCACGTAAACACGTAAAATGGTTAATTGCAGAAG TATGGAAAAAGATGAATGCAGAACGTGTTTCTAAAGATAGTCCTTTT GGTAAAGATTTTATAGGTTGTGCTGTTGATTTAGGTCGTATGGCTCA ATTAATGTATCACAATGGAGATGGTCATGGTACTCAACACCCTATTA TTCATCAACAAATGACACGTACTTTATTTGAACCATTCGCTGGTACC GGTGAAAACTTATACTTTCAAGGCTCAGGTGGCGGTGGAAGTGATT | Limonene (M. spicata) |
| | ACAAAGATGATGATGATAAAGGAACCGGTTAATCTAGACTCGAG | |
| 128 | CATATGGTACCAAGACGTACTGGTGGCTATCAACCTACACTTTGGGA TTTTTCAACAATTCAATTATTTGATAGTGAATATAAAGAAGAAAAAC ATCTTATGCGTGCTGCTGGTATGATTGCTCAAGTGAACATGTTACTT CAAGAAGAAGTAGACAGCATCCAACGTCTTGAATTAATTGATGACT TACGTCGTTTAGGTATATCTTGCCACTTTGATCGTGAAATTGTAGAG ATTTTAAACAGTAAATACTACACCAACAATGAAATTGATGAATCAG ATTTATACAGTACAGCACTTAGATTCAAACTTTTACGTCAATATGAT TTTAGCGTTAGCCAAGAAGTTTTTGATTGTTTTAAAAATGACAAAGG TACAGATTTCAAACCATCATTAGTTGACGATACACGTGGCTTATTAC AATTATATGAAGCATCATTTTATCAGCTCAGGGTGAAGAAACTTTA CATTTAGCACGTGATTTTGCTACTAAATTCTTACATAAAAGAGTTTT AGTAGATAAAGATATCAATTTATTATCTAGTATCGAGCGTGCTTTAG AATTACCAACACACTGGCGTGTACAAATGCCTAACGCTAGATCATTC ATCGACGCATATAAAAGAAGACCAGACATGAACCCTACAGTATTAG AGTTAGCAAAACTTGACTTTAACATGGTTCAAGCACAGTTCCAACAA GAATTAAAAGAAGCCAGTCGCTGGTGGAACTCTACAGGATTAGTAC ATGAATTACCATTTGTACGTGATCGTATTGTGGAATGTTATTATTGG ACTACTGGTGTAGTAGAACGTCGTGAACACGGTTACGAACGTATTAT GTTAACAAAAATTAACGCTTTAGTTACAACAATCGATGATGTTTTTG ACATTTATGGTACTTTAGAAGAATTACAACTTTTTACAACTGCTATTC AAAGATGGGACATTGAGTCTATGAAACAACTTCCACCCTATATGCA AATCTGCTACTTAGCTTTATTCAACTTCGTAAATGAGATGGCTTACG ATACATTACGTGATAAAGGTTTTAATAGTACTCCATATTTACGCAAA GCCTGGGTAGACTTAGTAGAAAGCTACTTAATTGAAGCTAAATGGT ATTATATGGGTCACAAACCAAGTTTAGAAGAGTACATGAAAAACTC ATGGATTTCTATCGGAGGTATTCCAATTTTATCACATTTATTCTTTCG TTTAACAGACAGTATCGAAGAAGAAGACGCTGAATCAATGCATAAA TATCACGATATAGTACGTGCCTCTTGTACTATTTTACGTTTAGCTGAT GATATGGGTACATCATTAGATGAAGTTGAACGTGGCGATGTTCCTAA ATCTGTACAATGCTATATGAATGAGAAAAACGCCTCTGAAGAAGAA GCACGTGAACATGTTCGTAGTTTAATTGATCAGACATGGAAGATGAT GAATAAAGAAATGATGACTTCATCATTTTCAAAATACTTCGTACAAG TGTCTGCAAATCTTGCTCGTATGGCACAATGGATATATCAACATGAA AGTGATGGTTTCGGTATGCAACACTCTTTAGTTAACAAAATGCTTCG TGGTTTACTTTTTGACCGTTATGAAGGTACCGGTGAAAACTTATACT TTCAAGGCTCAGGTGGCGGTGGAAGTGATTACAAAGATGATGATGA TAAAGGAACCGGTTAATCTAGACTCGAG | Cineole (S. officinalis) |
| 129 | CATATGGTACCACGTCGCATGGGTGATTTTCATTCAAACTTATGGGA TGATGATGTAATTCAATCTTTACCCACAGCTTACGAAGAAAAATCTT | Pinene (A. grandis) |
| | ATCTTGAACGTGCTGAGAAGTTAATTGGAGAAGTTGAAAATATGTTC AACAGTATGAGTTTAGAAGATGGTGAACTTATGAGTCCATTAAATG ATTTAATTCAACGCCTTTGGATTGTTGATTCTTAGGTAGATTAGGTA TCCATCGTCACTTTAAAGATGAGATTAAAAGTGCTTTAGATTATGTT TACAGTTACTGGGGTGAAAACGGAATAGGTTGTGGTCGTGAAAGTG CTGTAACTGATTTAAACAGTACAGCTTTAGGCTTTCGTACACTTCGTT TACACGGTTATCCAGTTTCATCTGATGTATTTAAAGCATTTAAAGGT CAAAATGGTCAATTCAGTTGTTCAGAAAATATCCAAACAGATGAAG AAATTCGTGGTGTTCTTAACTTATTTAGAGCCAGTTTAATAGCCTTCC CTGGTGAGAAAATAATGGACGAAGCTGAAATCTTCTCTACAAAATA CTTAAAGGAAGCATTACAAAAGATCCCAGTTAGTTCATTATCACGTG AAATCGGTGATGTACTTGAATATGGATGGCATACATACTTACCACGT TTAGAAGCACGTAACTATATTCATGTTTTCGGACAAGATACAGAGAA TACAAAAAGTTATGTAAAATCAAAGAAACTTTTAGAATTAGCTAAA TTAGAATTTAACATTTTTCAGAGCTTACAAAAACGTGAATTAGAAAG CCTTGTTCGTTGGTGGAAAGAATCTGGATTTCCTGAAATGACATTCT GTAGACACAGACACGTGGAATATTACACACTTGCATCATGTATTGCA TTCGAACCTCAGCATAGTGGTTTTCGTTTAGGTTTTGCTAAAACATGT CACCTTATAACAGTTTTAGATGACATGTATGACACTTTCGGCACCGT AGACGAATTAGAGTTATTTACAGCAACTATGAAACGTTGGGACCCA AGTTCAATTGACTGCCTTCCAGAATACATGAAAGGAGTTTACATTGC TGTGTATGATACAGTTAATGAAATGGCTCGTGAAGCTGAGGAAGCT CAAGGTCGCGATACACTTACATACGCTCGTGAGGCCTGGGAGGCTT ATATAGATTCTTATATGCAAGAAGCTCGCTGGATTGCTACTGGATAC TTACCTTCTTTCGATGAATATTATGAAAATGGTAAGGTTTCATGTGG TCACCGTATATCTGCTTTACAACCAATTCTTACTATGGATATTCCATT TCCAGATCACATTTTAAAGGAAGTTGACTTTCCTTCTAAACTTAATG ACTTAGCTTGTGCTATCTTACGCCTTCGCGGTGATACTCGTTGTTACA AAGCAGACCGTGCACGTGGTGAAGAGGCTAGTTCTATTTCTTGTTAT ATGAAAGATAATCCAGGTGTTTCTGAAGAAGATGCCTTAGATCATAT TAACGCAATGATCAGTGATGTTATTAAGGGCTTAAACTGGGAATTAC TTAAACCCGACATTAACGTACCTATTTCTGCTAAGAAACATGCTTTC GACATTGCTCGTGCTTTTCACTACGGTTATAAATATCGTGATGGCTA TTCAGTTGCTAATGTTGAAACAAAATCTTTAGTTACACGTACTTTACT TGAATCAGTTCCATTAGGTACCGGTGAAAACTTATACTTTCAAGGCT CAGGTGGCGGTGGAAGTGATTACAAAGATGATGATGATAAAGGAAC CGGTTAATCTAGACTCGAG | |
| 130 | CATATGGTACCACGTAGAGTTGGTAATTATCATTCTAATCTTTGGGA TGATGATTTTATACAAAGTTTAATTTCTACACCTTACGGTGCTCCTGA CTACCGTGAACGCGCTGATCGTCTTATTGGTGAAGTAAAAGATATTA | Camphene (A. grandis) |
| | TGTTTAATTTCAAATCTTTAGAGGATGGTGGTAATGACTTATTACAA CGTTTACTTTTAGTTGATGACGTAGAACGTTTAGGCATTGATCGTCA TTTCAAAAAGGAAATTAAGACTGCATTAGATTATGTAAATAGTTATT GGAATGAAAAAGGAATTGGTTGTGGTCGTGAGTCTGTAGTTACAGA CTTAAATTCAACTGCTTTAGGCCTTCGTACCTTAAGATTACATGGTTA TACTGTTAGCTCTGACGTTTTAAATGTTTTTAAAGATAAAAATGGTC AATTTTCATCTACAGCTAATATTCAAATTGAAGGTGAAATTCGTGGT GTTTTAAATCTTTTTCGTGCCTCTCTTGTAGCTTTTCCAGGTGAGAAA GTGATGGATGAGGCTGAAACTTTTTCAACAAAATATCTTCGTGAAGC ATTACAGAAAATTCCTGCCAGTTCAATTTTATCATTAGAAATACGTG ATGTATTAGAATATGGATGGCATACTAATTTACCACGTTTAGAAGCA CGTAATTACATGGATGTTTTCGGTCAGCACACCAAGAACAAAAATG CAGCCGAAAAATTACTTGAATTAGCAAAATTAGAGTTCAATATCTTT CACAGCTTACAAGAACGTGAATTAAAGCACGTTTCAAGATGGTGGA AAGACTCTGGTAGTCCAGAGATGACTTTCTGTCGCCACCGCCATGTG GAATATTATGCTTTAGCTTCTTGTATTGCTTTCGAACCCCAGCACAGT GGTTTCCGTTTAGGTTTTACTAAAATGAGTCATTTAATCACAGTGTTA GATGATATGTATGATGTATTCGGTACAGTTGATGAATTAGAGTTATT TACCGCCACTATTAAACGTTGGGACCCTTCTGCTATGGAATGTTTAC CAGAGTACATGAAAGGTGTTTACATGATGGTTTATCATACAGTTAAC GAAATGGCTCGTGTGGCAGAAAAGGCTCAAGGTAGAGACACATTAA ACTATGCTCGTCAAGCCTGGGAAGCATGTTTTGACTCTTATATGCAA GAAGCAAAATGGATTGCAACAGGTTACTTACCTACATTCGAGGAAT ATTTAGAAAATGGTAAAGTGAGTTCAGCACATCGTCCTTGTGCATTA CAACCTATTTTAACTCTTGATATTCCATTTCCCGATCATATTCTTAAA GAAGTGGATTTCCCAAGCAAACTTAATGACTTAATTTGTATTATCTT ACGTCTTAGAGGAGACACACGTTGCTATAAAGCAGACCGTGCCCGT GGTGAAGAAGCATCATCAATATCTTGTTATATGAAAGATAACCCAG GTTTAACTGAAGAAGATGCTTTAAACCACATTAACTTTATGATTCGT GACGCAATCCGCGAATTAAACTGGGAGTTACTTAAACCAGATAATA GTGTTCCAATTACTTCAAAGAAACATGCTTTTGATATTTCACGTGTGT GGCACCACGGATACCGTTATCGTGATGGTTACAGCTTTGCAAACGTG GAAACTAAAAGTCTTGTAATGCGTACTGTAATAGAACCAGTACCATT AGGTACCGGTGAAAACTTATACTTTCAAGGCTCAGGTGGCGGTGGA AGTGATTACAAAGATGATGATGATAAAGGAACCGGTTAATCTAGAC TCGAG | |
| 131 | CATATGGTACCACGTCGTTCAGGAGATTATCAACCAAGTTTATGGGA CTTTAATTACATTCAATCTTTAAACACACCTTACAAAGAACAACGTC ATTTTAATCGTCAAGCTGAGTTAATTATGCAAGTTCGTATGTTATTA AAGGTAAAAATGGAAGCAATTCAACAATTAGAGTTAATAGATGATT | Sabinene (S. officinalis) |
| | TACAGTACTTAGGATTATCATATTTCTTTCAAGACGAAATTAAACAA ATCTTAAGCTCTATTCACAATGAACCTCGTTATTTCATAATAATGAC CTTTATTTCACTGCTTTAGGTTTAGAATTTACGTCAACATGGTTTT AATGTTTCAGAAGACGTATTTGACTGCTTTAAAATCGAAAAATGTTC TGACTTTAATGCTAACTTAGCTCAGGACACAAAGGGTATGTTACAAT TATATGAAGCTAGTTTCTTATTAAGAGAAGGAGAAGATACACTTGA ATTAGCTCGTCGTTTTAGTACACGTTCTTTACGTGAAAAATTTGATG AAGGTGGTGACGAGATAGATGAAGATTTAAGTAGTTGGATTCGTCA TTCTTTAGATTTACCATTACACTGGCGTGTTCAAGGTTTAGAAGCTC GTTGGTTTTTAGATGCCTATGCTCGTCGTCCAGATATGAACCCTCTTA TTTTCAAATTAGCTAAATTAAATTTTAACATTGTTCAAGCTACATACC AAGAAGAATTAAAAGACATCTCTCGTTGGTGGAACAGTAGTTGTTTA GCAGAGAAATTACCCTTCGTTCGCGATCGTATTGTAGAATGTTTCTT CTGGGCTATTGCTGCTTTCGAACCACACCAATACTCATATCAACGTA AAATGGCCGCTGTAATTATTACATTTATTACTATTATTGATGATGTTT ACGATGTATATGGTACTATTGAAGAATTAGAGTTATTAACAGATATG ATTCGTAGATGGGATAATAAGAGTATTAGTCAACTTCCTTACTATAT GCAAGTTTGTTATTTAGCTCTTTATAACTTCGTAAGTGAACGCGCAT ACGACATCTTAAAAGATCAACACTTTAACAGTATTCCATACCTTCAA AGAAGTTGGGTTTCATTAGTTGAGGGATACTTAAAAGAAGCATATTG GTACTATAACGGTTACAAACCAAGTCTTGAAGAATATCTTAATAATG CAAAAATTAGTATTAGTGCACCCACCATTATTTCACAATTATACTTT ACTTTAGCAAATAGTATCGACGAAACTGCCATTGAAAGTTTATACCA ATATCACAACATTTTATACTTATCAGGTACTATCTTACGTTTAGCTGA TGATTTAGGAACTTCACAACATGAATTAGAACGTGGTGATGTTCCCA AAGCTATTCAATGTTATATGAATGATACAAATGCATCAGAAAGAGA AGCTGTAGAACATGTTAAATTTCTTATTCGTGAAGCCTGGAAAGAAA TGAATACAGTTACTACCGCATCAGATTGTCCTTTTACAGACGATCTT GTTGCCGCCGCAGCTAATTTAGCTCGTGCTGCTCAATTCATTTACTTA GATGGTGATGGTCATGGTGTACAACATAGCGAAATTCATCAGCAAA TGGGCGGTCTTCTTTTTCAACCATACGTTGGTACCGGTGAAAACTTA TACTTTCAAGGCTCAGGTGGCGGTGGAAGTGATTACAAAGATGATG ATGATAAAGGAACCGGTTAATCTAGACTCGAG | |
| 132 | CATATGGTACCACGCAGAATTGGTGATTACCATAGTAACATTTGGGA TGATGATTTTATCCAGTCACTTTCTACCCCTTATGGTGAACCATCTTA CCAAGAAAGAGCTGAACGTCTTATTGTAGAAGTGAAAAAGATTTTC AACAGTATGTACTTAGATGACGGTCGTTTAATGAGTTCTTTTAATGA CTTAATGCAACGTTTATGGATTGTAGACTCAGTAGAACGTTTAGGTA TTGCCCGTCACTTCAAAAATGAAATTACATCTGCCCTTGACTATGTTT TTCGTTATTGGGAAGAAAACGGTATAGGTTGTGGTCGTGATTCTATT | Myrcene (A. grandis) |
| | GTAACTGACTTAAATAGCACAGCTTTAGGTTTTCGTACACTTCGTTT ACACGGTTATACAGTTTCTCCAGAGGTTTTAAAAGCATTTCAAGATC AAAATGGTCAATTCGTTTGTTCACCAGGACAAACAGAAGGTGAAAT TCGTTCAGTTTTAAATTTATATCGTGCAAGTTTAATTGCCTTTCCAGG CGAAAAAGTTATGGAAGAAGCAGAAATCTTCTCTACTCGCTATTTAA AAGAAGCTCTTCAAAAGATTCCAGTTAGCGCATTATCACAAGAAAT CAAATTTGTTATGGAATATGGATGGCATACAAATTTACCTAGATTAG AAGCACGTAACTATATTGATACTTTAGAAAAGGATACATCAGCTTGG TTAAACAAAAATGCAGGTAAAAAGTTATTAGAATTAGCTAAATTAG AATTTAACATCTTTAACTCATTACAACAAAAAGAATTACAATACTTA CTTCGCTGGTGGAAAGAATCTGACTTACCTAAATTAACCTTTGCACG TCATAGACACGTTGAATTTTACACATTAGCTTCTTGTATTGCTATTGA TCCCAAACATTCAGCATTCCGTTTAGGATTCGCTAAAATGTGTCACT TAGTTACAGTTCTTGACGATATTTATGATACTTTCGGTACTATTGATG AACTTGAGTTATTTACTTCTGCAATTAAACGTTGGAATAGTTCTGAA ATTGAACACTTACCAGAATATATGAAATGCGTGTATATGGTTGTTTT TGAAACTGTTAATGAATTAACTCGTGAAGCTGAGAAAACACAAGGA CGTAACACTTTAAACTATGTTCGTAAAGCATGGGAAGCATATTTTGA TTCTTATATGGAGGAAGCAAAGTGGATCTCAAACGGATATTTACCAA TGTTTGAAGAATACCACGAAAATGGTAAAGTGTCATCTGCATACCGT GTAGCAACATTACAACCAATTTTAACTTTAAACGCTTGGTTACCCGA CTACATTCTTAAAGGAATTGATTTCCCAAGTCGTTTTAACGATTTAG CTAGTTCATTCTTACGTTTACGTGGCGATACTCGCTGTTACAAAGCT GACCGTGATCGTGGTGAAGAAGCTAGCTGCATTTCTTGTTACATGAA AGATAATCCAGGTTCTACCGAAGAAGATGCACTTAATCACATTAAC GCTATGGTAAATGACATCATTAAAGAATTAAACTGGGAATTATTACG CAGTAATGATAATATTCCTATGTTAGCTAAAAAGCACGCTTTTGATA TTACTCGTGCACTTCACCACTTATACATTTATCGCGATGGTTTCAGTG TTGCTAATAAAGAAACTAAAAAGTTAGTTATGGAGACATTACTTGA ATCAATGTTATTTGGTACCGGTGAAAACTTATACTTTCAAGGCTCAG GTGGCGGTGGAAGTGATTACAAAGATGATGATGATAAAGGAACCGG TTAATCTAGACTCGAG | |
| 133 | CATATGGTACCACAATCTGCTGAAAAGAACGACTCTTTATCAAGTTC TACATTAGTTAAGAGAGAATTTCCACCCGGTTTCTGGAAAGACGACT TAATCGACAGTTTAACTTCAAGTCACAAAGTAGCTGCTAGCGATGAA AAACGTATCGAAACCTTAATTTCAGAAATTAAGAATATGTTTCGTTG TATGGGTTATGGTGAGACAAATCCATCAGCTTATGATACTGCTTGGG TAGCTCGCATCCCAGCAGTTGATGGATCAGATAATCCTCACTTTCCA GAGACTGTGGAATGGATCTTACAAAATCAATTAAAAGATGGTTCTTG GGGTGAAGGTTTTTACTTCCTTGCTTATGATCGCATTTTAGCCACTTT | Abietadiene (A. grandis) |
| | AGCTTGTATTATCACACTTACACTTTGGCGTACTGGAGAAACACAAG TACAGAAAGGTATCGAATTTTTCCGCACTCAAGCAGGTAAAATGGA AGATGAAGCAGATTCACACCGTCCAAGTGGTTTTGAGATTGTATTTC CTGCTATGTTAAAAGAGGCTAAGATTTAGGCTTAGATTTACCTTAT GATCTTCCTTTTCTTAAACAAATTATTGAAAAGAGAGAAGCTAAGTT AAAACGTATTCCTACAGATGTTTATATGCTTTACCAACTACTTTACT TTATTCATTAGAAGGTTTACAAGAAATAGTAGACTGGCAAAAAATC ATGAAATTACAAAGTAAAGATGGTAGTTTCTTATCTTCTCCTGCCTC AACAGCAGCAGTATTTATGAGAACAGGTAACAAAAAGTGTTTAGAT TTCTTAAATTTCGTGCTTAAAAAGTTCGGTAATCATGTTCCATGCCAC TATCCTTTAGACCTTTTTGAGCGTCTTTGGGCAGTTGATACTGTTGAA AGATTAGGTATTGACCGTCATTTTAAAGAAGAAATAAAAGAGGCTT TAGACTATGTGTATTCACACTGGGACGAACGTGGTATTGGTTGGGCT CGTGAAAACCCCGTTCCAGATATTGACGATACAGCAATGGGTCTTCG TATTTTACGTCTTCATGGTTACAATGTTAGCAGCGATGTTCTTAAAAC ATTTCGTGATGAAAATGGTGAGTTCTTTTGCTTTTTAGGACAAACAC AAAGAGGTGTGACTGATATGTTAAATGTTAATCGTTGTAGCCATGTA TCTTTCCCTGGTGAAACTATAATGGAAGAGGCAAAATTATGTACTGA ACGTTACTTACGCAACGCATTAGAAAATGTAGACGCTTTTGATAAGT GGGCATTTAAGAAAAACATTCGTGGTGAGGTAGAATATGCTCTTAA ATATCCTTGGCATAAATCAATGCCACGTTTAGAAGCACGTTCATATA TTGAAAATTACGGTCCAGATGATGTTTGGTTAGGTAAAACTGTTTAT ATGATGCCTTACATTTCAAATGAAAAGTACTTAGAGTTAGCTAAACT TGATTTTAACAAAGTTCAGTCAATCCACCAGACAGAACTTCAAGACT TACGCCGTTGGTGGAAAAGTTCTGGTTTTACAGATTTAAACTTTACA AGAGAACGTGTTACTGAAATTTACTTTTCACCTGCATCTTTTATCTTC GAACCAGAATTTAGTAAATGTCGTGAGGTTTATACAAAAACTTCTAA TTTTACTGTAATTTTAGACGATTTATATGACGCTCATGGCTCTTTAGA TGACTTAAAACTTTTTACAGAGAGTGTTAAACGTTGGGATTTATCTT TAGTTGACCAAATGCCCCAGCAGATGAAAATCTGTTTTGTAGGTTTC TATAATACATTCAACGATATTGCTAAAGAAGGTAGAGAACGTCAAG GTCGTGATGTTTTAGGTTATATTCAAAACGTATGGAAAGTACAACTT GAAGCATATACTAAAGAAGCAGAATGGTCAGAAGCAAAATATGTTC CTAGTTTTAACGAATACATTGAAAATGCTTCAGTTTCAATTGCCTTA GGTACAGTAGTACTTATCAGTGCTTTATTTACCGGAGAAGTTTTAAC AGATGAAGTTTTATCTAAAATTGACCGTGAAAGTAGATTCTTACAGT TAATGGGCTTAACTGGACGTTTAGTAAATGATACTAAAACATATCAA GCTGAGCGTGGTCAAGGTGAAGTTGCTAGTGCAATTCAATGTTATAT GAAAGACCACCCTAAAATTAGTGAAGAAGAAGCATTACAACATGTA TATTCTGTAATGGAAAATGCATTAGAAGAATTAAATCGTGAGTTCGT TAACAACAAAATTCCAGACATCTATAAACGTCTTGTTTTCGAAACTG | |
| | CACGTATAATGCAATTATTTTACATGCAAGGTGATGGTTTAACATTA AGTCACGATATGGAAATTAAAGAGCACGTAAAGAATTGTTTATTCC AGCCAGTAGCTGGTACCGGTGAAAACTTATACTTTCAAGGCTCAGGT GGCGGTGGAAGTGATTACAAAGATGATGATGATAAAGGAACCGGTT AATCTAGACTCGAG | |
| 134 | CATATGGTACCATCTTCATCAACAGGCACTTCAAAAGTAGTAAGCGA AACATCTTCAACTATTGTAGACGATATTCCACGTCTTTCAGCAAATT ATCATGGTGATTTATGGCATCACAACGTAATTCAGACTTTAGAAACA CCATTTAGAGAAAGTTCAACTTATCAAGAGCGTGCAGATGAATTAGT AGTGAAAATCAAAGATATGTTCAATGCATTAGGTGACGGTGACATC TCACCTTCAGCTTATGATACTGCATGGGTAGCTCGTGTTGCTACCATT TCTTCTGATGGTAGCGAAAAACCACGTTTCCTCAAGCTCTTAATTG GGTTTTTAACAATCAATTACAAGATGGATCATGGGGTATTGAATCAC ATTTTAGTTTATGCGATCGTTTACTTAATACTACAAATTCAGTTATTG CTTTATCAGTATGGAAAACTGGTCACTCACAGGTTCAACAAGGTGCC GAATTTATTGCTGAAAATTTACGTCTTTTAAATGAAGAAGACGAATT AAGTCCTGATTTTCAAATTATCTTCCCAGCTTTATTACAGAAAGCCA AGGCTTTAGGAATCAATTTACCCTATGATTTACCATTCATCAAATAT CTTAGTACAACACGCGAAGCTCGTTTAACAGATGTGTCAGCTGCTGC TGACAACATACCAGCCAATATGCTTAATGCACTTGAAGGTTTAGAAG AAGTGATTGATTGGAATAAAATCATGCGTTTTCAATCTAAAGATGGT TCATTTTTATCTTCTCCAGCTAGTACAGCCTGTGTTTAATGAATACA GGTGATGAAAAATGTTTCACATTCTTAAATAACTTATTAGATAAATT CGGCGGTTGTGTTCCATGTATGTATAGCATTGATTTATTAGAACGTTT ATCTTTAGTGGACAACATTGAACACTTAGGTATTGGTCGTCACTTTA AACAAGAAATCAAAGGTGCATTAGATTATGTATATCGTCATTGGTCT GAACGCGGTATCGGTTGGGGTAGAGACTCTTTAGTTCCAGATTTAAA CACCACAGCTTTAGGTTTACGCACATTAAGAATGCACGGTTATAACG TGTCTAGTGATGTACTTAACAATTTCAAAGACGAAAATGGTCGTTTC TTTAGTAGTGCTGGTCAAACACACGTAGAGTTACGTTCTGTTGTAAA TCTTTTTCGCGCCTCAGATTTAGCCTTTCCAGACGAACGTGCAATGG ATGATGCTCGTAAATTCGCAGAACCATATTTACGTGAAGCATTAGCT ACAAAAATATCAACAAATACAAAGTTATTCAAAGAAATTGAATATG TTGTTGAATACCCTTGGCACATGTCAATTCCACGTTTAGAAGCTCGT AGTTATATTGACAGTTATGATGATAATTATGTATGGCAACGTAAGAC TTTATATCGTATGCCATCATTAAGTAATTCAAAATGTTTAGAACTTG CTAAATTAGATTTCAATATTGTTCAATCTTTACACCAAGAAGAACTT AAACTTTTAACTCGTTGGTGGAAAGAATCTGGTATGGCAGACATAA ATTTCACCCGCCATCGTGTAGCTGAAGTTTACTTTTCTAGTGCTACAT TTGAGCCAGAATATAGTGCTACTCGTATTGCATTCACAAAAATTGGT | Taxadiene (T. brevifolia) |
| | TGCTTACAAGTACTTTTCGATGATATGGCTGACATTTTCGCCACTTTA GATGAGTTAAAAAGTTTACTGAAGGTGTTAAACGCTGGACACAT CATTATTACATGAAATTCCCGAATGTATGCAAACTTGTTTTAAAGTA TGGTTTAAACTTATGGAAGAAGTAAACAACGACGTAGTAAAAGTTC AAGGAAGAGATATGTTAGCACATATTCGTAAACCCTGGGAATTATA CTTTAATTGTTATGTTCAAGAACGTGAATGGTTAGAAGCTGGTTATA TTCCTACATTCGAAGAATATCTTAAAACTTATGCTATTAGTGTAGGC CTTGGTCCTTGTACCTTACAACCTATTCTTTTAATGGGTGAGTTAGTT AAAGATGATGTAGTAGAAAAAGTTCATTACCCTTCTAACATGTTCGA ATTAGTTTCTTTAAGCTGGCGTTTAACTAATGATACCAAAACATATC AAGCAGAAAAAGTACGCGGTCAACAAGCTAGTGGCATTGCCTGTTA TATGAAAGACAATCCAGGTGCTACTGAAGAAGATGCTATTAAACAC ATTTGTCGTGTTGTTGATCGTGCATTAAAAGAAGCAAGTTTCGAATA TTTCAAGCCTTCAAATGACATTCCTATGGGTTGTAAATCTTTTATCTT TAACTTACGTTTATGTGTACAAATTTTCTATAAATTCATTGATGGTTA TGGTATCGCAAACGAAGAAATTAAGGACTACATTCGTAAGGTTTAT ATTGATCCAATTCAAGTTGGTACCGGTGAAAACTTATACTTTCAAGG CTCAGGTGGCGGTGGAAGTGATTACAAAGATGATGATGATAAAGGA ACCGGTTAATCTAGACTCGAG | |
| 135 | CATATGGTACCACACAAGTTCACAGGTGTTAACGCTAAATTCCAGCA ACCAGCATTAAGAAATTTATCTCCAGTGGTAGTTGAGCGCGAACGTG AGGAATTTGTAGGATTCTTTCCACAAATTGTTCGTGACTTAACTGAA GATGGTATTGGTCATCCAGAAGTAGGTGACGCTGTAGCTCGTCTTAA AGAAGTATTACAATACAACGCACCTGGTGGTAAATGCAATAGAGGT TTAACAGTTGTTGCAGCTTACCGTGAACTTTCTGGACCAGGTCAAAA AGACGCTGAAAGTCTTCGTTGTGCTTTAGCAGTAGGATGGTGTATTG AATTATTCCAAGCCTTTTTCTTAGTTGCTGACGATATAATGGACCAG TCATTAACTAGACGTGGTCAATTATGTTGGTACAAGAAAGAAGGTGT TGGTTTAGATGCAATAAATGATTCTTTTCTTTTAGAAAGCTCTGTGTA TCGCGTTCTTAAAAAGTATTGCCGTCAACGTCCATATTATGTACATTT ATTAGAGCTTTTTCTTCAAACAGCTTACCAAACAGAATTAGGACAAA TGTTAGATTTAATCACTGCTCCTGTATCTAAGGTAGATTTAAGCCATT TCTCAGAAGAACGTTACAAAGCTATTGTTAAGTATAAAACTGCTTTC TATTCATTCTATTTACCAGTTGCAGCAGCTATGTATATGGTTGGTATA GATTCTAAAGAAGAACATGAAAACGCAAAAGCTATTTTACTTGAGA TGGGTGAATACTTCCAAATTCAAGATGATTATTTAGATTGTTTTGGC GATCCTGCTTTAACAGGTAAAGTAGGTACTGATATTCAAGATAACAA ATGTTCATGGTTAGTTGTGCAATGCTTACAAAGAGTAACACCAGAAC AACGTCAACTTTTAGAAGATAATTACGGTCGTAAAGAACCAGAAAA AGTTGCTAAAGTTAAAGAATTATATGAGGCTGTAGGTATGAGAGCC | FPP (G. gallus) |
| | GCCTTTCAACAATACGAAGAAAGTAGTTACCGTCGTCTTCAAGAGTT AATTGAGAAACATTCTAATCGTTTACCAAAAGAAATTTTCTTAGGTT TAGCTCAGAAAATATACAAACGTCAAAAAGGTACCGGTGAAAACTT ATACTTTCAAGGCTCAGGTGGCGGTGGAAGTGATTACAAAGATGAT GATGATAAAGGAACCGGTTAATCTAGACTCGAG | |
| 136 | CATATGGTACCATCATTAACTGAAGAAAAACCAATTCGCCCAATCGC AAACTTTCCTCCAAGCATTTGGGGAGATCAATTCTTAATTTACGAAA AACAAGTAGAACAAGGTGTTGAACAGATTGTTAACGACCTTAAGAA AGAAGTGCGCCAACTTTTAAAAGAGGCTTTAGATATTCCAATGAAA CACGCAAACCTTTTAAAACTTATTGACGAAATTCAACGTCTTGGTAT TCCATATCACTTTGAACGTGAAATTGATCATGCATTACAATGTATCT ATGAAACTTATGGTGATAATTGGAATGGTGATCGTTCTTCATTATGG TTCCGTTTAATGCGTAAACAAGGTTATTATGTTACATGTGACGTGTTT AACAATTACAAAGATAAAAATGGTGCATTTAAACAATCTTTAGCTA ATGATGTTGAAGGTTTATTAGAATTATATGAAGCTACTTCAATGCGT GTTCCAGGTGAAATTATTCTTGAAGATGCATTAGGTTTTACACGTTC TCGTTTATCTATTATGACAAAAGACGCATTTAGTACAAATCCTGCTT TATTTACTGAAATTCAGCGTGCCCTTAAACAGCCTTTATGGAAACGT TTACCAAGAATTGAAGCTGCTCAATATATTCCATTTTATCAACAACA AGATTCTCACAATAAGACATTACTTAAATTAGCCAAATTAGAATTTA ATCTTTTACAATCATTACATAAAGAAGAATTAAGTCATGTGTGTAAA TGGTGGAAAGCATTTGATATTAAGAAGAATGCTCCATGTTTACGTGA TAGAATTGTAGAGTGTTACTTTTGGGGCCTTGGTAGTGGTTACGAGC CACAATATTCACGTGCTCGTGTATTCTTTACAAAAGCTGTTGCAGTT ATTACTTTAATTGACGATACCTATGATGCATACGGAACCTATGAGGA GCTTAAAATTTTCACTGAAGCTGTAGAACGTTGGTCTATAACTTGTT TAGATACTTTACCAGAATATATGAAACCCATCTACAAATTATTCATG GACACATACACTGAAATGGAAGAATTTTTAGCAAAAGAAGGTCGCA CAGACCTTTTTAACTGTGGTAAAGAATTTGTTAAAGAGTTTGTTCGT AACTTAATGGTAGAAGCTAAGTGGGCTAATGAAGGTCACATTCCTA CTACAGAAGAGCACGATCCAGTAGTAATAATTACAGGTGGAGCAAA CTTACTTACCACAACTTGTTACTTAGGTATGTCTGACATTTTTACAAA AGAATCAGTAGAGTGGGCAGTATCTGCACCACCATTATTCCGTTATT CTGGCATACTTGGTCGTCGTCTTAATGATTTAATGACTCATAAAGCT GAACAAGAGCGTAAACACTCATCAAGTAGTTTAGAAAGCTATATGA AGGAATATAACGTTAACGAAGAGTATGCTCAAACACTTATTTACAA AGAGGTTGAAGACGTTTGGAAGGACATTAACCGTGAATACTTAACA ACTAAAAACATTCCACGTCCTCTTTTAATGGCTGTAATATACTTATGT CAATTCTTAGAAGTACAATACGCTGGAAAAGATAACTTTACACGTAT GGGTGATGAATATAAACACTTAATAAAGAGTTTATTAGTTTATCCTA | Amor-phadiene (A. annua) |
| | TGTCAATAGGTACCGGTGAAAACTTATACTTTCAAGGCTCAGGTGGC GGTGGAAGTGATTACAAAGATGATGATGATAAAGGAACCGGTTAAT CTAGACTCGAG | |
| 137 | CATATGGTACCAGCAGGTGTATCAGCTGTGTCAAAAGTTTCTTCATT AGTATGTGACTTAAGTAGTACTAGCGGCTTAATTCGTAGAACTGCAA ATCCTCACCCTAATGTATGGGGTTATGACTTAGTTCATTCTTAAAAT CTCCATATATTGATAGTAGCTATCGTGAACGTGCTGAAGTGCTTGTA AGTGAAATAAAAGCTATGTTAAATCCAGCAATTACTGGAGATGGTG AATCAATGATTACACCTTCAGCTTATGACACTGCTTGGGTTGCACGT GTACCAGCAATTGATGGTAGCGCACGTCCACAATTTCCACAAACAGT AGATTGGATTTTAAAGAATCAATTAAAAGATGGTTCTTGGGGTATTC AATCACACTTTTTACTTTCAGACCGTTTATTAGCTACTCTTAGCTGTG TTTTAGTTTTACTTAAATGGAATGTTGGTGATTTACAGGTTGAGCAA GGTATTGAGTTTATTAAGTCAAACCTTGAATTAGTAAAAGATGAAAC TGATCAAGATTCTTTAGTGACTGATTTTGAGATTATTTCCCTAGCTT ACTTCGTGAGGCCCAAAGTTTACGTTTAGGTCTTCCATACGATTTAC CTTACATCCACTTATTACAAACAAAACGTCAGGAACGTTTAGCAAAA TTAAGCCGTGAAGAAATATATGCAGTTCCAAGTCCACTTTTATATTC TTTAGAGGGTATTCAAGATATTGTTGAGTGGAACGTATTATGGAAG TACAATCTCAGGATGGATCATTTTTAAGTTCTCCAGCATCAACCGCA TGTGTTTTTATGCATACAGGTGACGCTAAGTGTTTAGAATTTCTTAAC AGTGTAATGATTAAGTTTGGTAATTTTGTACCATGCCTTTATCCTGTA GATTTATTAGAACGTTTACTTATAGTAGATAATATAGTTCGTCTTGGT ATTTACCGTCACTTCGAAAAAGAAATTAAAGAAGCATTAGATTATGT ATATCGCCATTGGAATGAACGTGGTATTGGTTGGGGTCGTTTAAATC CAATTGCTGACTTAGAAACAACTGCTTTAGGTTTTCGTTTATTACGTT TACACCGTTATAATGTATCTCCAGCAATCTTTGATAATTTCAAAGAT GCCAATGGCAAATTCATTTGTAGCACTGGTCAGTTTAATAAGGATGT GGCTTCAATGTTAAACTTATACCGTGCATCACAATTAGCATTCCCAG GCGAAAACATTTTAGATGAAGCTAAATCTTTTGCCACCAAATACTTA CGTGAAGCCCTTGAAAAATCTGAAACTTCATCAGCTTGGAACAATA AACAGAATTTAAGTCAAGAAATCAAGTATGCATTAAAAACTTCATG GCACGCTTCTGTACCACGTGTTGAAGCAAAACGTTATTGTCAAGTTT ATCGTCCTGATTACGCTCGTATTGCTAAGTGTGTATACAAATTACCA TACGTTAACAACGAAAAATTCTTAGAATTAGGTAAATTAGATTTTAA CATCATTCAATCAATTCATCAAGAAGAAATGAAAAATGTGACAAGT TGGTTTCGTGATTCTGGCTTACCATTATTTACTTTCGCTCGCGAACGT CCTTTAGAATTTTACTTCTTAGTTGCTGCTGGTACTTATGAACCTCAA TATGCTAAATGTCGTTTCTTATTCACAAAAGTAGCTTGTCTTCAAAC AGTATTAGACGATATGTACGATACTTACGGTACTTTAGACGAATTAA | Bisabolene (A. grandis) |
| | AACTTTTTACCGAGGCTGTGCGTCGTTGGGATTTATCTTTTACAGAA AATTTACCTGACTATATGAAATTATGTTATCAAATCTATTATGACAT CGTTCATGAAGTGGCTTGGGAAGCTGAAAAAGAACAAGGTAGAGAA TTAGTGTCATTCTTCCGTAAAGGCTGGGAAGACTACTTATTAGGTTA CTATGAAGAAGCAGAATGGTTAGCAGCAGAATACGTTCCAACATTA GATGAATACATTAAAAACGGTATTACATCAATCGGCCAACGTATCTT ATTACTTTCAGGTGTGTTAATTATGGATGGCCAACTTTTATCACAAG AAGCATTAGAAAAAGTTGATTACCCTGGTCGTCGTGTTTTAACTGAG TTAAACTCACTTATTAGCCGTTTAGCTGACGACACTAAAACTTATAA AGCAGAAAAAGCTCGTGGAGAATTAGCCTCATCAATTGAATGCTAC ATGAAAGATCATCCTGAATGTACAGAAGAAGAAGCCTTAGACCACA TTTATTCTATTCTTGAACCAGCCGTAAAAGAATTAACTCGTGAATTT CTTAAACCAGACGACGTTCCATTTGCTTGTAAAAAGATGTTATTCGA AGAAACTCGTGTTACAATGGTGATCTTTAAAGATGGTGATGGTTTTG GTGTATCTAAGTTAGAAGTTAAAGATCACATCAAAGAATGCTTAATT GAACCATTACCATTAGGTACCGGTGAAAACTTATACTTTCAAGGCTC AGGTGGCGGTGGAAGTGATTACAAAGATGATGATGATAAAGGAACC GGTTAATCTAGACTCGAG | |
| 138 | CATATGGTACCAACTATGATGAATATGAATTTTAAGTACTGTCACAA GATTATGAAGAAACATTCAAAATCATTCAGTTATGCTTTTGACTTAT TACCAGAAGACCAACGTAAAGCTGTTTGGGCAATTTACGCCGTGTGC CGCAAAATTGATGATTCTATTGATGTATATGGTGATATTCAATTCTT AAATCAGATTAAAGAAGACATACAAAGTATTGAAAAATATCCATAC GAACATCATCATTTTCAATCTGACAGACGTATTATGATGGCCTTACA GCATGTTGCTCAGCATAAAAACATTGCATTTCAATCATTCTACAATT TAATTGACACAGTATATAAAGATCAACACTTTACAATGTTTGAAACA GATGCTGAACTTTTTGGTTATTGTTACGGTGTAGCTGGTACTGTGGG TGAAGTTTTAACTCCTATATTATCTGATCACGAAACACATCAAACTT ATGACGTTGCCCGTCGTTTAGGAGAGTCATTACAGTTAATCAATATT CTTAGAGATGTAGGTGAAGACTTTGACAACGAACGTATTTACTTCTC TAAACAACGTTTAAAACAATACGAAGTAGATATTGCAGAAGTGTAC CAAAATGGTGTAAACAATCACTATATTGATTTATGGGAATATTACGC TGCAATTGCTGAAAAGGATTTCAAGATGTTATGGACCAAATTAAAG TTTTCTCTATTGAAGCTCAGCCAATTATTGAGTTAGCTGCACGTATTT ATATCGAAATTTTAGATGAAGTACGTCAAGCTAACTACACATTACAT GAACGTGTTTTTGTAGATAAACGTAAAAAGGCTAAACTTTTTCACGA AAATAAAGGTACCGGTGAAAACTTATACTTTCAAGGCTCAGGTGGC GGTGGAAGTGATTACAAAGATGATGATGATAAAGGAACCGGTTAAT CTAGACTCGAG | Diapo-phytoene (S. aureus) |
| 139 | CATATGGTACCAAAAATTGCTGTTATTGGTGCTGGTGTTACCGGATT | Diapo-phytoene desaturase (S. aureus) |
| | AGCTGCTGCTGCTCGTATTGCTAGCCAAGGTCATGAAGTTACAATCT TCGAAAAAACAATAATGTAGGTGGTCGTATGAATCAATTAAAAAA AGATGGTTTACATTCGATATGGACCTACAATTGTTATGATGCCAG ATGTATATAAAGATGTATTTACTGCTTGCGGTAAAAACTATGAAGAT TATATAGAGTTACGTCAACTTCGTTACATTTATGACGTATATTTCGAT CACGATGATCGTATTACTGTTCCAACTGATTTAGCTGAATTACAACA AATGTTAGAATCAATTGAACCTGGTAGTACACACGGATTTATGTCAT TTTTAACAGATGTGTACAAGAAATATGAAATCGCTCGCAGATATTTC TTAGAACGTACTTACCGTAAACCATCAGACTTCTACAATATGACCTC TTTAGTACAAGGTGCTAAACTTAAAACTTTAAATCACGCTGATCAAC TTATCGAACACTACATTGATAACGAAAAGATTCAAAAACTTTAGCA TTCCAAACTCTTTATATCGGCATTGATCCAAAGCGTGGTCCTAGTTT ATATAGTATTATTCCTATGATTGAAATGATGTTCGGTGTACATTTAT CAAAGGTGGTATGTATGGTATGGCTCAAGGATTAGCTCAACTTAACA AAGATTTAGGTGTTAATATTGAATTAAATGCTGAAATTGAACAAATC ATTATCGATCCTAAATTCAAACGCGCAGATGCAATTAAAGTTAATGG TGACATTCGCAAATTTGATAAGATTTATGTACTGCTGACTTTCCTTC AGTTGCCGAATCACTTATGCCAGATTTCGCACCTATCAAAAAGTACC CTCCACATAAAATTGCAGATTTAGATTATTCTTGTTCAGCTTTTCTTA TGTATATTGGTATTGACATCGACGTAACTGACCAAGTTCGTTTACAT AACGTAATTTTTAGCGACGATTTTCGTGGAAATATTGAAGAAATTTT CGAAGGTCGCTTAAGTTACGACCCATCAATCTATGTTTATGTACCAG CTGTAGCCGATAAATCTTTAGCTCCTGAAGGTAAAACAGGCATTTAT GTGTTAATGCCTACTCCTGAACTTAAAACAGGATCAGGTATTGACTG GTCAGATGAGGCTTTAACTCAACAAATTAAAGAAATCATTTATCGTA AATTAGCAACAATTGAAGTATTTGAAGACATTAAATCACACATTGTA TCAGAAACAATTTTTACTCCTAATGACTTTGAACAAACCTATCACGC TAAATTTGGTTCTGCTTTCGGTTTAATGCCCACCTTAGCACAATCTAA TTATTACAGACCTCAAAATGTGTCACGTGATTATAAAGACTTATATT TCGCAGGTGCATCAACACATCCAGGTGCTGGAGTTCCAATTGTATTA ACAAGTGCCAAGATAACAGTAGACGAAATGATTAAAGATATTGAGC GTGGTGTGGGTACCGGTGAAAACTTATACTTTCAAGGCTCAGGTGGC GGTGGAAGTGATTACAAAGATGATGATGATAAAGGAACCGGTTAAT CTAGACTCGAG | |
| 140 | CATATGGTACCAGCATTTGACTTCGATGGTTACATGCTTCGTAAAGC TAAATCTGTAAATAAAGCTCTTGAAGCTGCAGTACAAATGAAAGAA CCATTAAAAATTCATGAAAGTATGCGTTATTCTTTATTAGCTGGTGG TAAACGTGTACGTCCAATGTTATGTATTGCAGCTTGTGAATTAGTTG GTGGTGACGAAAGTACTGCTATGCCTGCTGCTTGCGCTGTAGAAATG ATTCATACTATGAGTTTAATGCATGATGATTTACCATGTATGGATAA | GPPS-LSU (M. spicata) |
| | TGACGATTTACGTCGTGGTAAACCAACAAACCACATGGCATTTGGTG AAAGTGTAGCAGTATTAGCAGGTGATGCATTATTATCTTTTGCTTTT GAACATGTAGCAGCAGCAACAAAAGGTGCTCCTCCAGAACGTATTG TTAGAGTTTTAGGTGAACTTGCAGTTTCTATTGGTTCAGAAGGTTTA GTTGCTGGACAAGTAGTTGACGTTTGTTCTGAAGGTATGGCTGAGGT TGGTTTAGATCATTTAGAATTTATTCATCACCACAAAACTGCTGCTTT ATTACAAGGTTCTGTAGTATTAGGTGCAATATTAGGTGGTGGAAAAG AAGAAGAGGTAGCAAAACTTCGTAAATTCGCTAACTGCATTGGTTTA CTTTTCCAAGTAGTAGATGATATTCTTGATGTAACAAAATCATCTAA AGAATTAGGTAAAACAGCAGGTAAAGATTTAGTTGCTGATAAAACT ACTTATCCAAAATTAATCGGTGTTGAGAAAAGTAAAGAGTTCGCAG ACCGTTTAAATCGTGAAGCTCAAGAACAACTTCTTCATTTTCATCCA CATAGAGCAGCACCTTTAATCGCTTTAGCAAACTATATTGCTTATCG TGATAATGGTACCGGTGAAAATTTATATTTTCAAGGTTCAGGTGGCG GAGGTTCTGATTATAAAGATGATGATGATAAAGGAACCGGTTAATC TAGACTCGAG | |
| 141 | CATATGGTACCAAGTCAACCTTACTGGGCAGCAATTGAGGCAGATA TTGAACGTTACTTAAAAAATCAATTACAATTCGTCCACCAGAAACT GTATTTGGTCCAATGCACCACTTAACTTTTGCTGCACCAGCTACAGC TGCTAGTACTTTATGTTTAGCAGCATGTGAACTTGTAGGTGGTGATC GTAGTCAAGCTATGGCTGCAGCAGCAATCCATCTTGTTCATGCA GCTGCTTATGTACATGAACATTTACCATTAACTGATGGTAGTCGTCC AGTAAGTAAACCAGCTATCCAACATAAATATGGTCCAAATGTAGAA TTACTTACAGGTGACGGTATTGTACCATTTGGTTTTGAATTATTAGCA GGTTCTGTTGATCCAGCACGTACAGATGATCCAGACCGTATTTTACG TGTAATAATTGAAATAAGTCGTGCTGGTGGTCCAGAAGGTATGATTA GTGGTTTACATCGTGAAGAAGAGATTGTAGATGGTAATACTTCTCTT GATTTTATTGAATACGTTTGCAAAAAAAAATATGGTGAAATGCACGC ATGTGGTGCTGCATGCGGTGCAATTTTAGGTGGTGCAGCTGAAGAA GAAATTCAAAAACTTCGTAACTTCGGATTATATCAAGGAACTTTACG TGGTATGATGGAGATGAAAAACTCACACCAACTTATTGACGAAAAT ATCATTGGCAAACTTAAAGAATTAGCTTTAGAAGAATTAGGTGGATT TCATGGTAAAAATGCTGAATTAATGTCTAGTTTAGTAGCAGAACCAT CATTATATGCTGCTGGTACCGGTGAAAATTTATACTTTCAAGGTTCT GGTGGTGGTGGCAGTGATTATAAAGACGATGATGACAAAGGAACCG GTTAATCTAGACTCGAG | GPPS-SSU (M. spicata) |
| 142 | CATATGGTACCACTTTTATCTAACAAATTAAGAGAGATGGTTTTAGC AGAAGTTCCTAAATTAGCATCTGCTGCTGAATATTTCTTTAAACGTG GTGTTCAGGGTAAACAATTCCGTTCAACAATTTTATTATTAATGGCA ACAGCTCTTGACGTTCGTGTTCCAGAAGCATTAATTGGTGAATCTAC | GPPS (A. thalania) |
| | TGATATTGTAACATCTGAATTACGTGTACGTCAACGTGGCATTGCTG AAATTACAGAAATGATTCATGTAGCATCACTTCTTCACGATGACGTT CTTGACGATGCTGATACTCGTCGTGGTGTTGGTAGTCTTAATGTTGT AATGGGAAACAAAATGTCAGTTTTAGCAGGTGACTTCTTACTTTCTC GTGCTTGTGGTGCTCTTGCAGCTCTTAAAAACACAGAAGTTGTAGCA TTATTAGCTACAGCAGTAGAACACTTAGTTACTGGTGAGACAATGGA AATAACTTCATCAACTGAACAACGTTATTCTATGGATTACTACATGC AGAAAACTTATTACAAAACTGCTTCATTAATTTCAAATTCATGTAAA GCAGTTGCTGTATTAACAGGTCAAACAGCTGAAGTTGCAGTATTAGC TTTTGAATATGGTCGTAATTTAGGTTTAGCTTTCCAGTTAATTGACGA CATTTTAGATTTCACAGGCACATCTGCTAGTTTAGGAAAAGGTTCTT TATCAGATATACGTCATGGTGTTATTACTGCTCCTATCTTATTTGCAA TGGAAGAATTTCCTCAATTAAGAGAAGTAGTAGATCAAGTAGAAAA AGATCCAAGAAATGTAGACATAGCTTTAGAATATTTAGGTAAAAGT AAAGGTATTCAACGTGCTCGTGAATTAGCAATGGAACACGCAAATT TAGCTGCTGCAGCTATTGGTTCTTTACCTGAAACAGATAACGAAGAT GTTAAACGTTCACGTCGTGCTTTAATTGATTTAACACACAGAGTAAT TACACGTAACAAAGGTACCGGTGAGAATTTATACTTTCAAGGTAGTG GTGGAGGAGGTAGTGACTATAAAGATGATGACGATAAAGGAACCGG TTAATCTAGACTCGAG | |
| 143 | CATATGGTACCAGTAGTTTCTGAACGTTTAAGACATTCTGTAACAAC TGGTATTCCAGCATTAAAAACAGCAGCTGAATATTTCTTTCGTCGTG GTATCGAAGGAAAACGTTTAAGACCTACATTAGCATTATTAATGAGT AGTGCTTTATCACCAGCTGCTCCATCACCAGAGTATTTACAAGTTGA TACAAGACCTGCTGCAGAACACCCTCATGAAATGCGTCGTCGTCAAC AACGTTTAGCTGAAATTGCAGAATTAATCCATGTAGCTTCATTACTT CACGATGATGTTATTGATGACGCACAAACACGTCGTGGTGTTTTAAG TTTAAATACATCTGTTGGTAATAAAACAGCTATCTTAGCAGGTGATT TCTTATTAGCTCGTGCATCTGTAACATTAGCTAGTTTAAGAAACTCT GAAATTGTAGAATTAATGTCACAGGTTTTAGAACACTTAGTATCTGG TGAAATTATGCAAATGACTGCTACTTCAGAACAACTTTTAGATTTAG AACATTATTTAGCAAAAACATATTGTAAAACTGCTTCATTAATGGCT AATAGTTCTCGTTCTGTTGCAGTTCTTGCAGGTGCAGCTCCTGAAGTT TGTGATATGGCATGGTCATACGGTCGTCATTTAGGTATTGCTTTCCA AGTAGTTGACGATTTATTAGATTTAACAGGTTCATCTTCTGTTTTAGG TAAACCTGCTTTAAACGATATGCGTTCTGGTTTAGCAACAGCACCAG TATTATTCGCTGCACAAGAAGAACCTGCATTACAGGCTCTTATATTA CGTCGTTTTAAACACGACGGTGACGTAACAAAAGCAATGTCATTAAT TGAACGTACACAAGGCTTACGTCGTGCTGAAGAACTTGCAGCACAA CACGCAAAAGCTGCTGCTGATATGATTCGTTGCTTACCTACAGCTCA | GPPS (C. reinhardtii) |
| | ATCAGACCATGCAGAAATTGCTCGTGAAGCATTAATTCAAATTACAC ATCGTGTTTTAACACGTAAAAAAGGTACCGGTGAAAACTTATACTTT CAAGGTTCTGGTGGTGGTGGATCAGATTATAAAGATGATGATGACA AAGGAACCGGTTAATCTAGACTCGAG | |
| 144 | CATATGGTACCAGATTTTCCACAACAATTAGAAGCATGTGTTAAACA AGCAAATCAAGCATTATCACGTTTCATCGCACCACTTCCATTCCAAA ATACTCCTGTTGTTGAAACAATGCAATATGGTGCATTATTAGGAGGT AAAAGATTAAGACCATTTCTTGTATATGCAACAGGTCACATGTTTGG AGTATCTACTAACACATTAGATGCTCCAGCTGCTGCAGTTGAATGTA TTCATGCATATAGTTTAATTCATGATGATTTACCTGCAATGGATGAT GATGACTTAAGAAGAGGTTTACCTACATGTCATGTTAAATTTGGTGA AGCTAATGCTATTTTAGCTGGCGATGCACTTCAAACTCTTGCATTCA GTATTTTATCAGATGCTGATATGCCAGAAGTTTCAGATCGTGATCGT ATTTCTATGATATCTGAATTAGCTTCTGCTAGTGGTATTGCTGGTATG TGCGGTGGCCAAGCTCTTGATTTAGACGCAGAAGGAAAACACGTTC CTTTAGATGCTTTAGAGCGTATACATCGTCACAAAACAGGAGCTTTA ATTAGAGCTGCTGTTCGTCTTGGTGCTTTATCAGCTGGAGACAAAGG TCGTCGTGCTTTACCAGTTTTAGACAAATACGCTGAAAGTATTGGTT TAGCTTTTCAAGTTCAGGATGATATCTTAGATGTTGTAGGTGATACT GCTACTTTAGGTAAACGTCAAGGTGCTGATCAACAGTTAGGCAAATC TACATACCCAGCACTTTTAGGTTTAGAACAAGCTCGTAAAAAGCA AGAGACTTAATTGACGATGCTCGTCAAAGTCTTAAACAATTAGCAG AACAATCACTTGATACAAGTGCTTTAGAAGCATTAGCAGATTACATT ATTCAACGTAATAAAGGTACCGGTGAAAATTTATATTTTCAAGGTTC TGGTGGTGGAGGTTCAGACTATAAAGATGACGATGATAAAGGAACC GGTTAATCTAGACTCGAG | FPP (E. coli) |
| 145 | CATATGGTACCAAGTGTTAGTTGTTGTTGTAGAAATTTAGGAAAAAC TATCAAAAAAGCTATTCCAAGTCACCACTTACATTTACGTTCTTTAG GTGGTAGTTTATATAGAAGACGTATTCAATCATCTTCAATGGAAACA GACTTAAAATCTACATTCTTAAATGTTTATTCAGTTCTTAAATCAGAT TTATTACACGACCCATCATTTGAATTTACAAATGAAAGTCGTTTATG GGTAGATAGAATGCTTGATTATAATGTTCGTGGCGGTAAACTTAATC GTGGTCTTTCTGTAGTAGACTCTTTCAAATTACTTAAACAAGGTAAT GATTTAACTGAACAAGAAGTTTTCTTATCTTGTGCATTAGGTTGGTG TATTGAGTGGTTACAGGCTTACTTTTTAGTTCTTGATGATATTATGGA TAATTCAGTTACACGTCGTGGTCAACCTTGTTGGTTTCGTGTACCAC AAGTTGGTATGGTAGCTATTAATGATGGCATTCTTCTTCGTAACCAT ATTCATCGTATTCTTAAAAAACACTTCCGTGATAAACCATATTATGT AGATTTAGTTGACCTTTTCAATGAAGTAGAGTTACAAACTGCATGTG GACAAATGATTGATTTAATCACAACATTTGAAGGTGAAAAAGACTT | FPP (A. thalania) |
| | AGCTAAATATAGTTTATCAATTCACCGTCGTATTGTTCAATACAAAA CTGCATATTACTCATTCTATTTACCAGTTGCATGTGCTCTTTTAATGG CTGGCGAAAATTTAGAAAACCACATTGATGTTAAAAATGTATTAGTA GATATGGGTATTTACTTTCAAGTTCAGGATGATTATTTAGACTGTTTT GCTGATCCTGAAACATTAGGTAAAATTGGCACTGATATTGAGGACTT TAAATGTTCTTGGTTAGTTGTAAAAGCATTAGAACGTTGTAGTGAAG AACAAACAAAAATTCTTTACGAAAACTATGGCAAACCTGATCCATCT AATGTTGCTAAAGTAAAAGATTTATACAAAGAATTAGATTTAGAAG GCGTTTTCATGGAATATGAATCTAAATCATACGAGAAATTAACTGGT GCTATCGAAGGTCACCAATCTAAAGCAATTCAAGCTGTTCTTAAATC TTTCTTAGCAAAAATCTATAAACGTCAAAAAGGTACCGGTGAAAAC TTATACTTTCAAGGTAGTGGTGGCGGTGGTAGTGATTATAAAGATGA TGATGATAAAGGAACCGGTTAATCTAGACTCGAG | |
| 146 | CATATGGTACCAGCTGATCTTAAATCAACATTCTTAGATGTTTATTC AGTATTAAAAGTGATTTATTACAAGATCCATCTTTTGAATTTACAC ACGAAAGTCGTCAATGGTTAGAACGTATGTTAGATTATAATGTTCGT GGAGGCAAATTAAACAGAGGTTTAAGTGTAGTAGACAGTTACAAAC TTTTAAAACAAGGTCAAGACTTAACAGAAAAAGAAACATTTTTATCT TGTGCTTTAGGTTGGTGTATTGAATGGTTACAAGCATACTTCTTAGTT TTAGACGATATTATGGATAATTCTGTAACTAGACGTGGTCAACCATG TTGGTTTCGTAAACCAAAAGTAGGTATGATTGCTATTAATGATGGAA TACTTCTTCGTAACCACATTCATCGTATTCTTAAAAAACACTTTCGTG AAATGCCTTATTATGTAGACCTTGTAGACTTATTTAACGAAGTAGAA TTTCAAACAGCTTGTGGTCAAATGATTGACTTAATTACAACATTTGA TGGTGAAAAAGACCTTTCAAAATATTCACTTCAGATTCACCGTCGTA TTGTTGAGTACAAAACAGCATACTACTCTTTCTATTTACCTGTAGCAT GTGCTTTACTTATGGCAGGTGAAAATTTAGAAAATCACACAGATGTT AAAACTGTATTAGTTGATATGGGTATCTATTTCCAAGTTCAAGATGA TTATTTAGATTGCTTCGCTGATCCAGAAACATTAGGTAAAATTGGTA CAGATATTGAAGACTTTAAATGTAGTTGGTTAGTAGTAAAAGCATTA GAACGTTGTAGTGAAGAACAAACAAAAATTCTTTACGAAAATTATG GAAAAGCTGAACCTTCAAATGTAGCTAAAGTTAAAGCATTATACAA AGAATTAGATTTAGAGGGTGCATTTATGGAATATGAAAAAGAATCA TACGAGAAACTTACAAAACTTATTGAAGCACATCAATCAAAAGCTA TTCAAGCAGTTCTTAAATCTTTCTTAGCTAAAATTTATAAACGTCAA AAAGGTACCGGTGAAAACTTATACTTTCAAGGCTCTGGAGGTGGTG GTTCAGACTATAAAGATGATGATGATAAAGGAACCGGTTAATCTAG ACTCGAG | FPP (A. thalania) |
| 147 | CATATGGTACCAAGTGGCGAACCTACTCCAAAAAAAATGAAAGCAA CATACGTTCACGACCGTGAAAACTTTACAAAAGTATACGAAACTCTT | FPP (C. reinhardtii) |
| | CGTGACGAATTACTTAACGATGATTGTCTTAGTCCAGCTGGTTCACC TCAGGCTCAAGCTGCTCAAGAGTGGTTTAAAGAAGTTAATGATTATA ATGTTCCTGGTGGAAAACTTAACCGTGGTATGGCTGTATATGACGTT TTAGCTTCAGTTAAAGGTCCAGATGGTTTAAGTGAAGACGAAGTATT TAAAGCTAACGCTCTTGGTTGGTGTATTGAGTGGTTACAAGCATTTT TCTTAGTTGCTGATGATATAATGGATGGTTCAATTACACGTCGTGGC CAACCTTGTTGGTACAAACAACCTAAAGTTGGTATGATTGCTTGTAA TGATTACATCTTATTAGAATGCTGTATTTACTCAATTCTTAAAAGAC ATTTTAGAGGTCACGCTGCATACGCTCAACTTATGGACCTTTTCCAT GAAACTACATTCCAGACTTCACACGGTCAATTATTAGATTTAACAAC AGCACCTATCGGTTCTGTAGACTTATCAAAATATACAGAAGATAATT ACCTTCGTATTGTAACATATAAAACTGCATACTATTCTTTTTATTTAC CTGTAGCATGTGGTATGGTATTAGCTGGCATTACAGATCCAGCTGCT TTTGATCTTGCAAAAAATATTTGTGTTGAAATGGGTCAATATTTCCA GATTCAAGACGATTATTTAGATTGCTATGGTGACCCTGAGGTTATTG GTAAAATCGGTACAGACATAGAAGACAACAAATGTAGTTGGTTAGT TTGCACAGCTCTTAAAATCGCAACAGAAGAACAAAAAGAGGTTATA AAAGCTAATTATGGTCACAAAGAGGCTGAATCAGTAGCAGCAATTA AAGCATTATACGTTGAATTAGGTATTGAACAACGTTTTAAAGACTAT GAAGCTGCATCATACGCAAAATTAGAAGGTACAATTAGTGAACAAA CTTTATTACCTAAAGCAGTATTTACTTCTTTATTAGCTAAAATCTATA AAAGAAAAAAAGGTACCGGTGAGAACTTATACTTTCAAGGTAGTGG AGGTGGTGGTTCAGACTATAAAGATGATGATGATAAAGGAACCGGT TAATCTAGACTCGAG | |
| 148 | CATATGGTACCACAAACTGAACATGTTATCTTATTAAACGCTCAAGG TGTTCCTACAGGTACATTAGAAAAATATGCTGCACACACTGCTGATA CTCGTTTACACTTAGCTTTCTCATCTTGGTTATTCAATGCTAAAGGTC AACTTTTAGTTACAAGACGTGCATTAAGTAAAAAAGCATGGCCTGGT GTTTGGACTAACTCAGTTTGTGGTCATCCACAATTAGGTGAAAGTAA TGAAGATGCAGTTATACGTCGTTGCAGATATGAATTAGGTGTTGAAA TAACTCCACCAGAATCAATTTATCCAGATTTCCGTTATCGTGCAACT GATCCTAGTGGTATCGTTGAAAACGAAGTATGTCCTGTTTTTGCTGC ACGTACAACAAGTGCATTACAAATTAATGATGATGAAGTAATGGAT TATCAATGGTGTGACTTAGCTGATGTTTTACATGGTATTGATGCAAC ACCATGGGCATTTTCACCATGGATGGTAATGCAAGCAACAAATCGT GAAGCACGTAAAAGATTAAGTGCTTTTACACAGTTAAAAGGTACCG GTGAAAACTTATACTTTCAAGGTAGTGGAGGTGGTGGTTCTGACTAT AAAGATGACGATGATAAAGGAACCGGTTAATCTAGACTCGAG | IPP isomerase (E. coli) |
| 149 | CATATGGTACCACTTCGTAGTTTATTAAGAGGTTTAACACACATTCC TCGTGTTAATAGTGCTCAGCAACCTTCTTGCGCTCACGCTCGTCTTCA | IPP isomerase |
| | ATTTAAACTTCGTTCTATGCAATTATTAGCAGAAAACCGTACAGATC ACATGCGTGGTGCTTCTACATGGGCAGGTGGTCAGTCTCAAGATGAA TTAATGCTTAAAGATGAATGTATCTTAGTAGATGCTGATGATAACAT TACTGGTCACGCTTCTAAATTAGAATGTCACAAATTTCTTCCACATC AACCAGCTGGATTATTACACCGTGCTTTTTCTGTATTTCTTTTCGACG ATCAAGGTCGTTTACTTTTACAACAACGTGCTCGTAGTAAAATTACA TTTCCATCTGTATGGGCTAATACATGTTGTAGTCATCCATTACATGGT CAAACACCAGATGAAGTAGATCAACAATCACAAGTAGCAGACGGAA CTGTACCAGGTGCAAAAGCTGCTGCAATCAGAAAATTAGAACATGA ATTAGGTATTCCAGCTCACCAATTACCAGCATCAGCTTTTCGTTTCTT AACACGTCTTCACTATTGTGCAGCTGACGTTCAACCTGCAGCAACAC AATCTGCATTATGGGGTGAACACGAAATGGATTACATTTTATTCATT AGAGCTAATGTTACACTTGCTCCTAATCCTGACGAAGTAGATGAGGT ACGTTATGTAACTCAAGAAGAATTAAGACAAATGATGCAACCAGAT AATGGTTTACAATGGTCACCATGGTTCCGTATTATTGCAGCAAGATT TTTAGAACGTTGGTGGGCTGATTTAGATGCTGCATTAAATACAGATA AACATGAAGACTGGGGAACAGTTCATCACATTAACGAAGCTGGTAC CGGTGAAAACTTATACTTTCAAGGATCAGGAGGCGGTGGAAGTGAT TATAAAGATGATGATGATAAAGGAACCGGTTAATCTAGACTCGAG | (H. pluvalis) |
| 150 | CATATGGTACCAAGAAGATCAGGCAATTATAACCCAACAGCATGGG ACTTCAATTATATCCAATCATTAGACAATCAATACAAAAAAGAACGT TACTCTACTCGTCACGCTGAATTAACAGTTCAAGTTAAAAAATTATT AGAAGAAGAAATGGAAGCTGTTCAAAAACTTGAACTTATAGAGGAT CTTAAAAACTTAGGCATTTCTTACCCATTTAAAGATAATATTCAACA AATCTTAAATCAAATTTACAATGAACACAAATGTTGTCACAACTCAG AAGTTGAAGAAAAAGACCTTTATTTCACTGCTTTACGTTTTAGATTA TTACGTCAACAAGGTTTTGAAGTAAGTCAAGAAGTATTTGATCACTT TAAAAACGAAAAAGGTACAGATTTTAAACCTAATTTAGCAGATGAT ACTAAAGGATTATTACAATTATATGAAGCATCATTCTTATTACGTGA AGCAGAAGACACATTAGAACTTGCTCGTCAATTCTCTACTAAACTTT TACAAAAAAAAGTTGATGAAAACGGTGACGATAAAATTGAAGATAA CTTATTACTTTGGATTAGACGTAGTTTAGAATTACCATTACATTGGC GTGTACAAAGATTAGAAGCTCGTGGCTTTTTAGATGCTTACGTTCGT AGACCTGATATGAATCCTATTGTATTTGAATTAGCAAAATTAGACTT TAACATTACTCAAGCAACACAACAAGAAGAACTTAAAGATTTATCA AGATGGTGGAATAGTACTGGCTTAGCTGAAAAACTTCCTTTTGCTCG TGATCGTGTAGTTGAATCATATTTCTGGGCTATGGGTACTTTTGAAC CACATCAATACGGATACCAACGTGAATTAGTTGCTAAAATCATTGCA CTTGCTACAGTTGTAGACGATGTTTACGATGTATATGGTACTTTAGA GGAATTAGAACTTTTTACTGATGCTATTCGTCGTTGGGACCGTGAAT | Limonene (L. angustifolia) |
| | CTATTGACCAATTACCATATTACATGCAATTATGTTTTCTTACTGTAA ACAACTTTGTTTTTGAGTTAGCTCACGACGTATTAAAAGATAAATCA TTCAATTGTTTACCTCATTTACAAAGATCATGGTTAGATTTAGCTGA AGCATACCTTGTAGAAGCAAAATGGTATCATAGTCGTTATACACCTT CTTTAGAAGAATATCTTAATATTGCTCGTGTTTCAGTAACATGTCCA ACTATTGTTTCTCAAATGTATTTTGCATTACCAATTCCAATCGAAAA ACCTGTAATTGAGATCATGTACAAATATCACGATATCTTATACTTAT CAGGTATGTTATTACGTTTACCAGATGACTTAGGAACAGCATCATTC GAACTTAAACGTGGTGATGTACAAAAAGCAGTTCAATGTTATATGA AAGAACGTAATGTTCCTGAAAATGAAGCTCGTGAACATGTTAAATTC TTAATTCGTGAGGCTTCTAAACAAATTAATACAGCAATGGCAACAG ACTGTCCATTTACAGAAGATTTTGCAGTTGCAGCAGCAAACTTAGGT CGTGTAGCAAATTTTGTATATGTTGATGGTGATGGTTTTGGAGTACA ACACAGTAAAATCTATGAGCAAATTGGTACACTTATGTTTGAACCAT ATCCAGGTACCGGTGAAAACTTATACTTTCAAGGTAGTGGTGGTGGA GGTTCTGATTACAAAGACGATGATGATAAAGGAACCGGTTAATCTA GACTCGAG | |
| 151 | CATATGGTACCAAGAAGAAGTGGAAACTATAAACCTACAATGTGGG ATTTTCAATTTATTCAAAGTGTAAATAATCTTTACGCTGGTGATAAA TACATGGAACGTTTCGATGAAGTAAAAAAAGAAATGAAAAAAAACT TAATGATGATGGTTGAGGGTTTAATAGAGGAATTAGATGTTAAATTA GAATTAATAGATAATTTAGAAAGATTAGGTGTTAGTTATCATTTCAA AAATGAAATAATGCAAATCCTTAAATCTGTACACCAGCAAATCACTT GTCGTGATAATTCATTATACTCTACTGCATTAAAATTTCGTTTATTAC GTCAACACGGATTCCACATTAGTCAAGACATCTTTAACGATTTTAAA GATATGAATGGCAATGTTAAACAAAGTATCTGTAACGATACTAAAG GTTTATTAGAACTTTATGAAGCATCTTTCTTATCTACTGAATGTGAAA CAACACTTAAAAACTTCACTGAAGCACACTTAAAAAATTATGTTTAT ATTAACCACTCATGTGGAGATCAATACAATAACATAATGATGGAATT AGTTGTTCACGCTTTAGAATTACCACGTCACTGGATGATGCCTCGTT TAGAGACACGTTGGTATATATCAATTTATGAACGTATGCCTAATGCT AATCCACTTTTACTTGAACTTGCTAAATTAGACTTCAATATTGTTCAA GCTACACACCAACAAGACTTAAAATCATTATCACGTTGGTGGAAAA ACATGTGTTTAGCTGAAAAATTATCATTTCTCGTAACCGTTTAGTA GAAAATCTTTTCTGGGCAGTTGGAACTAATTTTGAACCACAACACAG TTATTTCCGTCGTTTAATCACTAAAATCATTGTTTTGTTGGTATTAT TGATGATATTTATGATGTTTACGGCAAACTTGATGAGTTAGAATTAT TCACTTTAGCTGTACAACGTTGGGATACAAAAGCAATGGAAGACTT ACCATATTACATGCAAGTTTGTTATTTAGCTTTAATTAATACAACAA ATGATGTTGCTTATGAAGTTCTTCGTAAACATAACATTAATGTATTA | Mono-terpene (S. lycopersicu m) |
| | CCATACTTAACTAAATCTTGGACAGACTTATGTAAATCATATTTACA AGAAGCTCGTTGGTACTACAATGGTTACAAACCTTCATTAGAGGAAT ATATGGATAATGGTTGGATTAGTATAGCAGTTCCTATGGTATTAGCA CATGCACTTTTCTTAGTTACAGATCCAATTACAAAAGAAGCATTAGA ATCATTAACAAACTATCCTGATATTATTCGTTGCTCAGCTACAATATT CCGTTTAAATGATGATCTTGGTACAAGTTCAGATGAATTAAAACGTG GAGATGTACCAAAATCAATTCAATGCTATATGAACGAAAAAGGCGT TTCAGAGGAAGAAGCTCGTGAACATATTCGTTTCTTAATCAAAGAAA CATGGAAATTCATGAACACTGCACACCATAAAGAGAAAAGTTTATT TTGTGAGACATTTGTAGAAATTGCAAAAAATATTGCAACAACAGCTC ATTGTATGTACTTAAAAGGTGATTCTCACGGTATTCAAAACACTGAT GTTAAAAACTCAATAAGTAATATACTTTTCCATCCAATTATTATCGG TACCGGTGAAAACCTTTACTTTCAAGGTTCAGGTGGTGGCGGTTCAG ACTATAAAGATGACGATGATAAAGGAACCGGTTAATCTAGACTCGA G | |
| 152 | CATATGGTACCAAGACGTAGTGGAAATTATGAGCCATCTGCATGGG ACTTCAATTACTTACAATCTCTTAATAATTATCACCATAAAGAAGAA CGTTACTTACGTCGTCAAGCTGATTTAATTGAAAAAGTAAAAATGAT TCTTAAAGAAGAGAAAATGGAAGCATTACAGCAATTAGAACTTATA GACGATCTTCGTAATTTAGGTCTTTCATATTGTTTTGATGATCAAATT AATCATATTCTTACAACAATTTACAACCAACATTCTTGTTTCCATTAT CACGAAGCTGCAACAAGTGAAGAAGCTAACTTATATTTCACAGCTTT AGGTTTCCGTTTACTTCGTGAACACGGATTCAAAGTATCACAAGAAG TATTTGACCGTTTCAAAAATGAAAAAGGTACAGATTTTCGTCCAGAT TTAGTAGATGATACTCAAGGTTTATTACAACTTTATGAAGCATCTTT CCTTCTTCGTGAAGGTGAAGACACTTTAGAATTTGCACGTCAATTTG CTACTAAATTTCTTCAAAAAAAAGTTGAGGAGAAAATGATAGAAGA GGAAAATCTTTTATCTTGGACTTTACATTCACTTGAATTACCATTACA TTGGCGTATACAACGTTTAGAAGCTAAATGGTTTTTAGATGCTTATG CTAGTCGTCCTGATATGAATCCAATAATCTTTGAATTAGCAAAATTA GAATTTAACATTGCTCAGGCACTTCAACAAGAAGAACTTAAAGATTT ATCAAGATGGTGGAACGATACTGGTATTGCTGAAAAATTACCTTTCG CTCGTGATAGAATCGTTGAATCTCATTATTGGGCAATTGGTACTTTA GAACCTTATCAATACCGTTATCAGCGTTCATTAATTGCAAAAATCAT TGCTTTAACTACAGTTGTTGATGATGTATATGATGTTTACGGTACATT AGACGAATTACAGTTATTTACTGATGCAATTCGTCGTTGGGACATTG AAAGTATAAATCAATTACCTTCTTATATGCAATTATGTTATTTAGCTA TTTATAATTTCGTATCAGAATTAGCTTATGATATTTTCAGAGATAAA GGTTTTAATTCTTTACCATATTTACACAAAAGTTGGCTTGACTTAGTT GAGGCTTACTTTCAAGAAGCAAAATGGTATCATTCTGGCTACACACC | Ter-pinolene (O. basilicum) |
| | ATCATTAGAACAATACTTAAATATCGCTCAAATTTCTGTAGCAAGTC CAGCTATATTAAGTCAAATTTACTTTACTATGGCTGGTTCAATTGAT AAACCAGTAATCGAATCAATGTACAAATATAGACACATTTTAAACTT ATCTGGTATATTACTTAGATTACCAGATGACTTAGGTACTGCTAGTG ATGAATTAGGTCGTGGTGATTTAGCAAAAGCAATGCAATGTTACATG AAAGAGCGTAACGTTTCTGAAGAAGAAGCTCGTGATCATGTACGTTT CTTAAATCGTGAGGTTTCAAAACAAATGAATCCTGCTCGTGCTGCTG ATGATTGTCCATTCACTGATGATTTTGTAGTAGCTGCTGCTAATTTAG GAAGAGTTGCAGATTTCATGTATGTTGAAGGCGATGGTTTAGGTTTA CAATACCCAGCTATCCACCAACACATGGCAGAACTTTTATTTCACCC TTACGCAGGTACCGGTGAAAACTTATACTTTCAAGGTTCAGGTGGTG GAGGTTCTGACTATAAAGATGATGATGATAAAGGAACCGGTTAATC TAGACTCGAG | |
| 153 | CATATGGTACCAAGAAGATCAGGAAATTATCAACCTAGTGCATGGG ATTTTAACTATATCCAATCTCTTAATAACAACCATTCTAAAGAAGAA CGTCACTTAGAGCGTAAAGCAAAACTTATTGAAGAAGTAAAAATGT TATTAGAGCAAGAAATGGCTGCTGTACAACAATTAGAGCTTATTGA AGACCTTAAAAACTTAGGTTTATCTTACTTATTCCAAGATGAAATCA AAATAATCCTTAATTCTATTTACAATCATCATAAATGTTTTCATAATA ATCACGAACAATGTATTCACGTTAATAGTGACTTATACTTTGTTGCA TTAGGCTTCCGTTTATTTCGTCAACATGGTTTCAAAGTTTCTCAAGAG GTTTTTGACTGTTTTAAAAACGAAGAAGGATCAGACTTTAGTGCTAA CTTAGCAGATGATACTAAAGGTTTACTTCAATTATACGAGGCTTCAT ATTTAGTTACAGAAGATGAAGACACATTAGAAATGGCACGTCAATT TTCAACTAAAATCTTACAAAAAAAAGTAGAAGAGAAAATGATTGAG AAAGAGAACTTATTAAGTTGGACTTTACATAGTTTAGAATTACCACT TCACTGGCGTATTCAACGTTTAGAAGCAAAATGGTTCCTTGATGCTT ATGCTAGTCGTCCAGATATGAATCCAATTATTTTTGAATTAGCTAAA TTAGAGTTTAACATTGCTCAAGCATTACAACAAGAAGAATTAAAAG ATTTAAGTAGATGGTGGAATGATACAGGCATTGCTGAAAAATTACCT TTTGCTCGTGATAGAATAGTAGAGAGTCATTACTGGGCAATTGGTAC TTTAGAACCTTATCAATATAGATATCAACGTTCATTAATTGCTAAAA TTATTGCTTTAACAACAGTTGTTGATGACGTTTACGACGTATATGGA ACTTTAGATGAATTACAGTTATTTACAGACGCTATTCGTCGTTGGGA TATTGAATCTATTAATCAATTACCAAGTTATATGCAATTATGCTATTT AGCTATTTATAACTTTGTTTCTGAATTAGCATACGATATTTTTCGTGA CAAAGGATTCAATTCTTTACCTTACCTTCATAAATCATGGTTAGATTT AGTAGAAGCATACTTTGTTGAAGCTAAATGGTTTCATGATGGTTATA CTCCAACTCTTGAAGAATATTTAAATAACTCAAAAATTACTATTATA TGTCCTGCTATTGTTAGTGAAATCTACTTCGCATTCGCTAATTCAATT | Myrcene (O. basilicum) |
| | GATAAAACAGAAGTTGAATCAATCTACAAATATCACGATATTTATA TTTATCAGGAATGCTTGCACGTTTACCAGACGACTTAGGTACTTCAT CATTTGAAATGAAAAGAGGTGATGTTGCTAAAGCTATTCAATGTTAC ATGAAAGAACATAATGCTTCAGAGGAAGAAGCTCGTGAACACATTC GTTTCTTAATGCGTGAAGCATGGAAACACATGAATACTGCTGCAGCT GCTGATGACTGTCCATTTGAATCTGATTTAGTAGTAGGTGCTGCATC ATTAGGTAGAGTTGCAAACTTTGTATATGTTGAAGGTGACGGTTTTG GTGTACAACATTCAAAAATACATCAACAAATGGCTGAATTACTTTTT TATCCATATCAAGGTACCGGTGAAAACTTATACTTTCAAGGTAGTGG AGGTGGTGGTAGTGACTATAAAGACGATGACGATAAAGGAACCGGT TAATCTAGACTCGAG | |
| 154 | CATATGGTACCAAGAAGAAGTGCTAATTATCAAGCAAGTATTTGGG ATGATAATTTCATTCAAAGTCTTGCATCTCCTTATGCAGGAGAAAAA TATGCAGAAAAAGCAGAAAAACTTAAAACAGAAGTTAAAACTATGA TTGATCAAACAAGAGATGAACTTAAACAATTAGAACTTATTGATAA CTTACAACGTTTAGGTATATGTCATCACTTTCAAGACCTTACAAAAA AAATTTTACAAAAAATTTATGGAGAAGAACGTAACGGAGATCACCA ACATTACAAAGAAAAAGGCTTACATTTACAGCATTACGTTTCCGTA TTTTACGTCAGGACGGTTATCATGTTCCACAAGATGTATTTTCATCAT TTATGAATAAAGCTGGTGACTTTGAAGAATCTTTAAGTAAAGACACA AAAGGTTTAGTTAGTTTATATGAGGCTTCTTACTTATCAATGGAAGG TGAAACTATTTTAGATATGGCAAAAGACTTTTCATCTCACCATTTAC ATAAAATGGTTGAAGATGCTACTGACAAACGTGTAGCTAATCAAAT TATCCATTCTCTTGAAATGCCACTTCACAGACGTGTTCAAAAACTTG AAGCAATTTGGTTTATTCAATTCTACGAATGCGGCTCTGATGCTAAT CCAACTTTAGTAGAATTAGCAAAATTAGATTTCAACATGGTTCAGGC AACATACCAAGAAGAATTAAAACGTTTATCACGTTGGTATGAAGAA ACAGGCTTACAAGAGAAACTTTCATTCGCTCGTCACCGTCTTGCTGA AGCATTCTTATGGTCTATGGGTATTATTCCAGAAGGACACTTTGGTT ATGGTCGTATGCACTTAATGAAAATTGGTGCTTACATTACATTACTT GATGATATTTATGATGTTTATGGTACTTTAGAAGAACTTCAAGTATT AACAGAAATTATTGAACGTTGGGATATTAACTTATTAGATCAATTAC CTGAATACATGCAAATCTTCTTTTTATACATGTTTAATTCTACAAATG AACTTGCTTATGAAATTTTACGTGATCAAGGTATCAATGTAATATCA AACTTAAAAGGATTATGGGTAGAGTTATCTCAGTGTTACTTTAAAGA AGCTACTTGGTTCCATAACGGTTACACACCAACAACTGAAGAATATC TTAATGTTGCTTGTATTTCTGCTAGTGGTCCTGTTATTTTATTTTCAG GTTACTTTACTACTACTAATCCTATTAATAAACACGAATTACAATCTT TAGAACGTCACGCACATTCATTATCTATGATATTACGTTTAGCTGAT GATTTAGGTACATCAAGTGATGAAATGAAACGTGGAGATGTACCAA | Zingiberene (O. basilicum) |
| | AAGCTATTCAATGTTTTATGAATGACACTGGTTGTTGTGAAGAAGAA GCACGTCAACACGTAAAAAGATTAATAGATGCTGAATGGAAAAAAA TGAACAAAGACATCTTAATGGAGAAACCATTTAAAAATTTTGTCCA ACTGCTATGAATTTAGGTCGTATTTCTATGAGTTTTTATGAACACGG AGATGGTTATGGAGGTCCTCACTCTGATACAAAAAAAAAAATGGTA TCTTTATTTGTACAACCAATGAATATTACTATTGGTACCGGTGAAAA CCTTTATTTTCAAGGTTCTGGTGGTGGCGGTTCAGATTATAAAGATG ATGACGACAAAGGAACCGGTTAATCTAGACTCGAG | |
| 155 | CATATGGTACCAAGACGTTCAGCTAACTATCAACCTAGTATTTGGAA CCACGATTACATTGAATCACTTCGTATCGAATATGTTGGTGAAACAT GTACACGTCAAATTAACGTTTTAAAAGAACAAGTTCGTATGATGTTA CACAAAGTTGTTAATCCATTAGAACAATTAGAATTAATTGAAATTTT ACAACGTTTAGGTTTAAGTTACCATTTCGAAGAAGAAATAAAACGT ATTTTAGATGGTGTTTACAATAACGATCATGGTGGTGATACATGGAA AGCAGAAAACCTTTATGCAACAGCTCTTAAATTCCGTCTTTTACGTC AGCACGGTTATTCTGTTTCTCAAGAAGTTTTCAACTCTTTTAAAGATG AGCGTGGCAGTTTCAAAGCATGTTTATGTGAAGATACTAAAGGTATG TTATCACTTTATGAAGCATCTTTCTTTCTTATTGAAGGTGAAAACATT TTAGAGGAAGCTAGAGACTTTAGTACAAAACATCTTGAAGAATATG TAAAACAAAATAAAGAGAAAAACTTAGCTACTTTAGTTAATCACTC ATTAGAATTTCCATTACATTGGCGTATGCCTCGTTTAGAAGCTCGTT GGTTCATCAATATCTATCGTCATAATCAAGATGTAAATCCAATCCTT TTAGAATTTGCTGAACTTGACTTCAATATTGTACAAGCTGCTCACCA AGCAGATTTAAAACAAGTATCAACATGGTGGAAATCAACTGGTTTA GTAGAAAATCTTTCATTCGCTCGTGATCGTCCTGTAGAAAACTTCTTT TGGACAGTTGGTCTTATTTCCAACCACAATTCGGTTATTGTCGTAG AATGTTTACTAAAGTATTCGCATTAATTACTACAATTGATGACGTAT ATGATGTATATGGTACTTTAGATGAATTAGAACTTTTCACAGACGTT GTTGAAAGATGGGATATTAATGCAATGGATCAATTACCTGATTATAT GAAAATTTGCTTTTTAACATTACACAATAGTGTTAACGAAATGGCAT TAGACACTATGAAAGAACAACGTTTTCACATCATTAAATACCTTAAA AAAGCATGGGTTGATCTTTGTCGTTATTACTTAGTTGAAGCTAAATG GTATAGTAATAAATATAGACCTTCTTTACAAGAATACATTGAAAATG CATGGATTTCAATTGGTGCTCCAACTATTTTAGTTCATGCATATTTCT TCGTTACAAATCCAATTACAAAAGAAGCATTAGACTGTTTAGAAGA ATATCCAAACATTATTCGTTGGAGTAGTATTATTGCACGTTTAGCTG ATGATTTAGGTACTTCAACAGACGAATTAAAACGTGGTGACGTACC AAAAGCAATTCAATGTTATATGAATGAAACAGGTGCTTCAGAAGAA GGTGCTCGTGAGTACATTAAATACTTAATTTCTGCTACTTGGAAAAA AATGAACAAAGATAGAGCAGCATCAAGTCCATTTTCACATATCTTCA | Myrcene (Q. ilex) |
| | TTGAAATTGCTCTTAATTTAGCACGTATGGCACAATGTTTATATCAA CACGGTGACGGCCACGGTTTAGGTAACCGTGAAACAAAAGATCGTA TACTTTCATTACTTATTCAACCAATTCCATTAAACAAAGATGGTACC GGTGAGAACTTATACTTTCAAGGCTCAGGTGGTGGTGGTTCTGATTA CAAAGATGATGATGATAAAGGAACCGGTTAATCTAGACTCGAG | |
| 156 | CATATGGTACCAAGAAGAATTGGAGACTATCACTCAAACTTATGGA ATGATGACTTCATTCAATCATTAACAACACCATACGGTGCTCCATCA TATATTGAACGTGCTGATAGATTAATATCTGAAGTAAAAGAAATGTT TAATAGAATGTGTATGGAAGATGGTGAGTTAATGTCTCCATTAAATG ATCTTATTCAAAGATTATGGACTGTTGATAGTGTTGAACGTTTAGGT ATAGATCGTCACTTCAAAAATGAAATAAAAGCTAGTTTAGATTATGT ATACTCATACTGGAACGAAAAAGGTATCGGTTGTGGTCGTCAATCA GTAGTTACAGATTTAAACTCTACTGCTCTTGGATTAAGAATTTTACG TCAACATGGTTACACAGTTTCAAGTGAAGTTTTAAAAGTTTTTGAAG AAGAAAACGGTCAATTTGCTTGTTCACCTTCACAGACTGAGGGCGA AATTCGTTCATTCTTAAACTTATATCGTGCTTCATTAATTGCTTTTCC TGGTGAAAAAGTAATGGAAGAAGCTCAAATCTTTTCTAGTCGTTACT TAAAAGAAGCAGTTCAGAAAATTCCAGTTTCAGGTTTATCTCGTGAA ATAGGCGATGTTTTAGAATATGGTTGGCACACAAACTTACCTCGTTG GGAAGCTCGTAACTATATGGACGTATTCGGTCAAGACACAAATACA TCATTCAACAAAAACAAAATGCAATATATGAATACAGAGAAAATTC TTCAATTAGTAAAATTAGAGTTTAATATCTTTCATTCATTACAACAA CGTGAATTACAATGTTTATTACGTTGGTGGAAAGAAAGTGGTCTTCC ACAATTAACATTTGCACGTCACCGTCACGTTGAATTTTACACTTTAG CTTCTTGTATTGCATGTGAACCAAAACACAGTGCATTTCGTTTAGGT TTTGCAAAAATGTGTCACTTAGTAACAGTTTTAGATGATGTATATGA CACATTTGGCAAAATGGATGAATTAGAACTTTTTACTGCAGCTGTTA AACGTTGGGACTTATCAGAAACTGAGCGTTTACCTGAGTATATGAAA GGTTTATATGTTGTAGTTTTCGAGACTGTTAATGAATTAGCACAAGA AGCAGAGAAAACTCAAGGACGTAATACATTAAATTACGTTCGTAAA GCATGGGAAGCATACTTCGATAGTTATATGAAAGAAGCAGAATGGA TCTCAACAGGCTATTTACCAACATTCGAAGAGTATTGTGAAAACGGT AAAGTATCAAGTGCATATAGAGTTGCTGCACTTCAACCTATTTTAAC ATTAGATGTACAACTTCCAGATGACATCTTAAAAGGTATTGATTTTC CATCTCGTTTCAATGATTTAGCATCTTCATTTCTTCGTTTACGTGGAG ATACTAGATGTTACGAGGCTGATCGTGCTCGTGGTGAAGAAGCAAG TTGTATTTCTTGTTACATGAAAGACAATCCAGGTTCAACTGAAGAAG ATGCATTAAATCACATTAATGCTATGATAAATGATATTATTCGTGAA TTAAACTGGGAATTTCTTAAACCAGACTCAAATATCCCAATGCCAGC TCGTAAACATGCTTTCGATATTACAAGAGCTTTACATCACTTATATA | Myrcene (P. abies) |
| | TTTATCGTGACGGTTTTCTGTTGCTAACAAAGAGACTAAAAATCTT GTTGAGAAAACTTTATTAGAATCAATGTTATTCGGTACCGGTGAGAA CCTTTATTTTCAAGGTTCAGGTGGTGGTGGTTCAGATTATAAAGACG ATGATGATAAAGGAACCGGTTAATCTAGACTCGAG | |
| 157 | CATATGGTACCAAGAAGATCAGCTAATTATCAACCTAGTCGTTGGGA TCATCATCACCTTTTAAGTGTAGAAAACAAATTCGCTAAAGATAAAC GTGTAAGAGAACGTGACTTACTTAAAGAAAAAGTTCGTAAAATGTT AAATGACGAACAGAAAACTTACTTAGATCAATTAGAATTTATTGAC GATCTTCAAAAATTAGGTGTTAGTTATCACTTCGAAGCAGAAATAGA TAATATACTTACAAGTTCATACAAAAAAGATCGTACAAATATACAA GAAAGTGATTTACACGCAACTGCATTAGAGTTTCGTCTTTTCGTCA ACACGGTTTTAACGTTTCAGAAGATGTATTTGATGTATTTATGGAAA ATTGTGGTAAATTCGACCGTGATGACATTTATGGTTTAATTTCATTAT ATGAAGCTAGTTATCTTTCTACTAAACTTGACAAAAATCTTCAAATC TTTATCCGTCCATTTGCTACTCAACAATTACGTGATTTTGTAGATACT CACAGTAATGAAGATTTCGGTTCATGTGATATGGTAGAAATAGTTGT TCAAGCATTAGACATGCCATACTATTGGCAAATGCGTCGTTTATCTA CACGTTGGTATATTGATGTTTATGGTAAAAGACAAAATTACAAAAAC TTAGTAGTTGTTGAATTTGCAAAAATTGATTTCAATATTGTTCAAGCT ATTCACCAGGAAGAACTTAAAAATGTATCATCTTGGTGGATGGAAA CTGGTTTAGGTAAACAACTTTATTTGCTCGTGATCGTATTGTAGAG AACTATTTTTGGACAATTGGTCAAATTCAAGAACCTCAATATGGATA TGTTAGACAAACAATGACTAAAATCAATGCTTTATTAACAACAATTG ATGATATTTATGATATATACGGTACATTAGAAGAATTACAGTTATTC ACAGTTGCATTTGAGAATTGGGACATAAATCGTTTAGACGAATTACC AGAATATATGCGTTTATGTTTCTTAGTTATCTATAACGAAGTAAATA GTATAGCATGTGAAATTCTTAGAACAAAAAATATTAACGTTATTCCT TTCTTAAAAAAATCTTGGACTGATGTAAGTAAAGCATACTTAGTTGA AGCTAAATGGTATAAATCAGGCCATAAACCAAATTTAGAAGAGTAT ATGCAAAATGCACGTATTTCTATTTCTTCACCAACAATCTTTGTTCAC TTTTATTGTGTATTTTCAGACCAATTATCTATTCAAGTTTTAGAAACT TTATCACAACACCAACAAAATGTTGTAAGATGTAGTTCTTCTGTTTT CCGTTTAGCTAATGACTTAGTAACTTCTCCAGATGAATTAGCTAGAG GTGATGTTTGTAAATCAATTCAATGTTATATGTCAGAAACTGGTGCA AGTGAAGATAAAGCTAGATCACACGTTCGTCAAATGATTAATGATTT ATGGGACGAAATGAATTACGAGAAAATGGCACATTCAAGTAGTATC TTACATCATGATTTTATGGAGACAGTAATCAATTTAGCTAGAATGTC TCAATGTATGTACCAATATGGTGACGGACACGGTTCTCCAGAAAAA GCTAAAATTGTAGATCGTGTAATGAGTTTACTTTTCAACCCTATTCCT TTAGATGGTACCGGTGAGAATTTATATTTTCAAGGCTCTGGAGGTGG | Myrcene, ocimene (A. thalania) |
| | TGGTTCAGATTATAAAGATGATGACGACAAAGGAACCGGTTAATCT AGACTCGAG | |
| 158 | CATATGGTACCAAGAAGAAGTGCAAACTATCAACCTTCATTATGGC AACATGAATACTTATTATCATTAGGCAACACTTATGTTAAAGAAGAT AATGTTGAAAGAGTAACTCTTTTAAAACAAGAAGTTTCTAAAATGTT AAACGAAACAGAAGGTTTACTTGAACAACTTGAATTAATTGACACTT TACAAAGATTAGGTGTTTCTTATCATTTTGAACAGGAGATTAAAAA ACATTAACTAATGTTCATGTTAAAAACGTACGTGCTCATAAAAATCG TATTGATCGTAACCGTTGGGGCGATTTATATGCAACTGCATTAGAAT TTCGTTTATTACGTCAACATGGTTTTCTATTGCTCAAGACGTTTTG ATGGTAATATTGGTGTTGACTTAGACGACAAAGACATTAAAGGTATT TTAAGTTTATACGAAGCTAGTTACTTATCAACACGTATTGATACAAA ACTTAAAGAATCAATCTATTACACAACAAAACGTTTAAGAAAATTC GTAGAGGTAAACAAAAACGAAACTAAAAGTTACACTCTTCGTCGTA TGGTTATTCACGCACTTGAGATGCCTTATCACCGTCGTGTTGGTCGTC TTGAAGCTCGTTGGTATATCGAGGTATATGGAGAAAGACACGACAT GAATCCTATTTTATTAGAATTAGCTAAATTAGATTTTAACTTTGTTCA GGCTATCCACCAAGACGAATTAAAATCATTATCTAGTTGGTGGTCTA AAACAGGATTAACAAAACATTTAGACTTTGTTCGTGATCGTATTACA GAGGGTTACTTCAGTAGTGTAGGTGTTATGTATGAACCAGAATTTGC ATATCATCGTCAAATGCTTACAAAAGTATTTATGCTTATTACAACTA TTGATGACATCTATGACATTTACGGTACACTTGAAGAATTACAATTA TTCACAACTATCGTTGAAAAATGGGATGTTAATCGTTTAGAAGAACT TCCTAACTATATGAAATTATGCTTCTTATGTTTAGTTAACGAAATAA ATCAAATTGGATATTTTGTATTAAGAGATAAAGGTTTTAATGTAATT CCTTATCTTAAAGAGTCTTGGGCTGACATGTGTACTACATTTCTTAA AGAAGCTAAATGGTACAAATCAGGTTATAAACCAAATTTTGAAGAG TATATGCAAAATGGCTGGATTTCATCATCAGTTCCAACTATTCTTTTA CACTTATTTTGTTTATTAAGTGACCAAACTTTAGACATTCTTGGTTCT TATAATCACAGTGTTGTTCGTAGTTCAGCAACAATTTTACGTCTTGC AAATGATTTAGCTACTTCTTCAGAAGAATTAGCAAGAGGAGATACA ATGAAATCAGTTCAATGTCACATGCATGAAACTGGTGCTTCAGAAGC TGAATCAAGAGCTTACATTCAAGGTATTATTGGCGTAGCTTGGGATG ACCTTAATATGGAGAAAAAATCATGTCGTTTACACCAGGGATTCTTA GAAGCAGCAGCAAATTTAGGACGTGTAGCACAATGCGTATATCAAT ATGGAGACGGTCACGGTTGTCCAGATAAAGCAAAAACAGTAAATCA TGTTCGTAGTTTATTAGTTCACCCATTACCATTAAACGGTACCGGTG AAAACCTTTATTTTCAAGGTAGTGGTGGAGGTGGTTCTGATTATAAA GACGACGATGACAAAGGAACCGGTTAATCTAGACTCGAG | Myrcene, ocimene (A. thalania) |
| 159 | CATATGGTACCAGCTTCTCCACCTGCTCATCGTTCATCTAAAGCAGC | Sesqui-terpene (Z. mays; B73) |
| | AGACGAAGAGTTACCAAAAGCATCTTCTACATTCCATCCATCTCTTT GGGGTTCATTTTTCTTAACATATCAGCCACCTACAGCTCCACAACGT GCAAATATGAAAGAACGTGCTGAAGTTCTTCGTGAACGTGTTCGTAA AGTATTAAAAGGTTCAACAACAGATCAATTACCTGAAACAGTTAAC TTAATTCTTACATTACAAAGACTTGGTTTAGGTTATTACTATGAAAA TGAAATTGACAAATTACTTCATCAAATTTACTCTAATTCAGATTATA ACGTAAAAGACTTAAACTTAGTTTCTCAACGTTTTACTTACTTCGTA AAAACGGTTATGACGTACCTTCTGATGTTTCTTATCTTTTAAAACTG AAGAAGGTGGTTTCGCTTGTGCTGCAGCTGACACACGTTCACTTTTA AGTTTATACAATGCTGCTTACCTTCGTAAACATGGTGAAGAAGTATT AGATGAAGCAATTTCATCAACACGTTTAAGATTACAAGACTTATTAG GTCGTTTATTACCTGAATCACCATTCGCTAAAGAAGTATCAAGTTCA CTTCGTACACCTTTATTCCGTCGTGTAGGTATTTTAGAAGCTCGTAAC TATATTCCAATCTATGAAACTGAAGCTACAAGAAATGAAGCTGTATT AGAGCTTGCTAAACTTAACTTCAATTTACAACAGCTTGATTTCTGTG AAGAATTAAAACATTGTAGTGCATGGTGGAATGAGATGATTGCTAA AAGTAAATTAACTTTTGTACGTGACCGTATAGTTGAAGAATACTTTT GGATGAATGGTGCATGTTATGATCCACCATATTCATTAAGTCGTATT ATTCTTACAAAAATCACTGGTTTAATTACTATTATTGATGATATGTTC GATACTCATGGTACAACAGAGGATTGCATGAAATTCGCAGAAGCAT TTGGTCGTTGGGATGAATCAGCAATTCATCTTCTTCCAGAATACATG AAAGATTTTTACATTTTAATGTTAGAAACTTTCCAGTCATTTGAAGA TGCACTTGGTCCAGAAAAATCATACCGTGTATTATACTTAAAACAAG CAATGGAACGTTTAGTAGAGTTATATTCTAAAGAAATCAAATGGCGT GATGACGATTATGTTCCAACAATGTCAGAACATTTACAAGTTAGTGC TGAAACAATTGCTACAATTGCTTTAACTTGCTCTGCTTATGCTGGTAT GGGTGATATGTCTATTCGTAAAGAAACATTTGAATGGGCATTATCTT TCCCTCAATTCATTAGAACTTTTGGTTCATTTGTACGTTTATCAAATG ATGTTGTATCAACAAAACGTGAACAAACTAAAGATCATTCACCTTCA ACAGTTCACTGTTATATGAAAGAACACGGTACAACTATGGACGATG CTTGTGAAAAAATCAAAGAATTAATTGAGGACTCATGGAAAGACAT GTTAGAACAATCTTTAGCTCTTAAAGGCTTACCTAAAGTAGTACCTC AATTAGTTTTTGATTTCTCTCGTACTACAGATAACATGTATCGTGACC GTGATGCTTTAACATCATCAGAAGCATTAAAAGAAATGATACAGTT ATTATTCGTAGAACCTATACCTGAAGGTACCGGTGAGAATCTTTATT TTCAAGGATCAGGTGGTGGAGGCTCAGATTACAAAGATGACGACGA TAAAGGAACCGGTTAATCTAGACTCGAG | |
| 160 | CATATGGTACCAGAGGCTTTAGGAAATTTTGATTATGAGAGTTATAC TAATTTTACAAAATTACCATCATCACAATGGGGTGATCAATTCCTTA AATTTTCTATAGCAGATTCTGACTTCGATGTATTAGAAAGAGAAATA | Sesqui-terpene (A. thalania) |
| | GAAGTATTAAAACCAAAAGTAAGAGAGAACATTTTTGTTTCATCAA GTACTGATAAAGATGCAATGAAAAAAACAATTTTAAGTATTCATTTC TTAGATAGTTTAGGTTTATCTTATCACTTCGAAAAAGAAATAGAGGA GAGTTTAAAACATGCTTTCGAGAAAATTGAAGACCTTATTGCTGATG AAAATAAACTTCATACAATAAGTACAATTTTCCGTGTATTCCGTACA TACGGCTATTATATGTCTTCTGATGTATTCAAAATTTTCAAAGGAGA CGATGGTAAATTCAAAGAAAGTTTAATTGAAGACGTTAAAGGTATG CTTTCTTTTTATGAAGCTGTTCATTTTGGAACAACTACTGATCACATT TTAGACGAAGCTCTTAGTTTTACATTAAACCACTTAGAGTCACTTGC AACAGGCCGTCGTGCATCACCACCACATATTAGTAAATTAATCCAAA ATGCTTTACATATTCCTCAACATCGTAACATCCAGGCATTAGTAGCT CGTGAATACATTAGTTTTTACGAACACGAAGAAGATCACGATGAAA CATTATTAAAATTAGCTAAATTAAACTTTAAATTCTTACAACTTCACT ATTTTCAAGAATTAAAAACAATTACAATGTGGTGGACTAAATTAGAT CATACATCTAATTTACCACCAAATTTTCGTGAACGTACAGTTGAAAC ATGGTTTGCAGCTTTAATGATGTATTTCGAACCACAATTTAGTTTAG GTCGTATTATGAGTGCAAAATTATATTTAGTAATTACTTTCTTAGATG ACGCATGTGATACATACGGATCAATATCTGAAGTAGAGTCATTAGCT GATTGTTTAGAACGTTGGGACCCAGATTATATGGAAAATTTACAAGG TCACATGAAAACAGCATTCAAATTCGTTATGTATTTATTCAAAGAAT ACGAAGAAATTTTACGTTCACAAGGCCGTTCATTCGTATTAGAGAAA ATGATTGAGGAGTTTAAAATTATCGCACGTAAAAACTTAGAACTTGT AAAATGGGCTCGTGGTGGTCACGTTCCTTCTTTTGACGAATATATAG AGAGTGGTGGTGCTGAGATTGGTACTTATGCTACAATCGCTTGTTCA ATTATGGGTCTTGGTGAAATTGGTAAAAAAGAAGCATTTGAGTGGTT AATCTCTCGTCCTAAACTTGTTCGTATTTTAGGTGCTAAAACACGTTT AATGGATGATATCGCAGACTTTGAAGAAGACATGGAAAAAGGCTAT ACAGCTAATGCACTTAACTATTATATGAATGAACACGGAGTAACTA AAGAAGAAGCTAGTCGTGAACTTGAGAAAATGAATGGTGATATGAA CAAAATTGTAAACGAAGAATGTCTTAAAATTACAACTATGCCACGTC GTATCTTAATGCAAAGTGTTAACTACGCTCGTAGTTTAGATGTATTA TACACAGCTGATGATGTATATAACCACCGTGAAGGCAAACTTAAAG AATATATGAGATTACTTTTAGTAGATCCAATTTTACTTGGTACCGGT GAAAATCTTTATTTTCAAGGTTCAGGTGGTGGTGGTTCTGATTATAA AGATGATGACGATAAAGGAACCGGTTAATCTAGACTCGAG | |
| 161 | CATATGGTACCAGAGAGTCAAACAACATTCAAATACGAATCATTAG CATTTACAAAACTTAGTCACTGTCAATGGACAGACTATTTCTTAGT GTTCCAATTGATGAAAGTGAATTAGATGTTATTACTCGTGAAATTGA TATTCTTAAACCAGAAGTTATGGAGTTATTAAGTAGTCAAGGAGATG ATGAAACAAGTAAAAGAAAAGTTCTTCTTATTCAGTTATTACTTTCT | Sesqui-terpene (A. thalania) |
| | TTAGGTTTAGCATTCCACTTTGAAAATGAGATTAAAAACATACTTGA ACACGCATTTCGTAAAATAGATGATATAACTGGTGACGAAAAGAC TTATCAACAATTAGTATTATGTTCCGTGTTTTCCGTACTTATGGACAC AATCTTCCAAGTAGTGTTTTTAAACGTTTCACAGGTGATGATGGTAA ATTTCAGCAAAGTTAACAGAAGACGCAAAAGGTATTTAAGTTTAT ATGAAGCTGCACATTTAGGTACTACTACAGATTACATTTAGATGAA GCTCTTAAATTCACATCTAGTCACTTAAAAAGTTTACTTGCTGGTGG TACATGTCGTCCTCACATCTTACGTTTAATCCGTAATACATTATACTT ACCACAACGTTGGAACATGGAAGCTGTTATCGCTCGTGAATACATAT CATTTTACGAGCAGGAAGAAGATCACGATAAAATGCTTTTACGTCTT GCAAAACTTAACTTTAAACTTCTTCAATTACACTACATTAAAGAGCT TAAAAGTTTCATTAAATGGTGGATGGAACTTGGTTTAACTTCTAAAT GGCCTTCTCAATTTCGTGAACGTATTGTTGAAGCATGGTTAGCTGGA TTAATGATGTATTTTGAACCACAGTTCTCAGGTGGTCGTGTTATTGCT GCAAAATTCAACTATTTACTTACAATATTAGACGACGCATGTGACCA CTATTTTTCTATTCACGAATTAACACGTTTAGTTGCATGTGTAGAACG TTGGTCACCAGATGGTATTGACACATTAGAAGATATTTCACGTTCTG TATTCAAATTAATGTTAGATGTTTTCGACGATATTGGTAAAGGTGTA CGTTCAGAAGGTTCTAGTTACCACTTAAAAGAAATGTTAGAGGAATT AAACACTTTAGTTCGTGCTAATTTAGATTTAGTTAAATGGGCTCGTG GAATACAAACAGCTGGTAAAGAGGCTTATGAATGGGTTCGTTCACG TCCACGTTTAATCAAATCTTTAGCAGCTAAAGGTAGACTTATGGATG ATATTACAGACTTTGACTCAGATATGAGTAATGGATTCGCAGCTAAT GCTATTAACTACTATATGAAACAATTTGTTGTTACAAAAGAAGAAGC TATTCTTGAATGTCAACGTATGATTGTAGACATTAACAAAACTATTA ATGAAGAGTTATTAAAAACTACTTCAGTTCCAGGTCGTGTATTAAAA CAAGCTCTTAACTTTGGCCGTTTATTAGAATTATTATATACAAAATCT GACGATATTTACAATTGTTCTGAAGGCAAACTTAAAGAATACATTGT AACTCTTTTAATTGATCCTATAAGACTTGGTACCGGTGAAAACTTAT ACTTTCAAGGTTCAGGCGGTGGTGGTAGTGATTACAAAGATGATGAT GACAAAGGAACCGGTTAATCTAGACTCGAG | |
| 162 | CATATGGTACCAGAGAGTCAAACAAAATTCGACTACGAATCATTAG CTTTTACAAAATTATCACATTCACAATGGACTGATTACTTTTTATCAG TACCTATAGACGACTCTGAACTTGACGCAATTACTCGTGAAATCGAC ATTATCAAACCTGAAGTTCGTAAATTACTTTCAAGTAAAGGTGATGA TGAAACTTCTAAACGTAAAGTATTACTTATCCAAAGTTTATTATCAT TAGGTTTAGCATTTCATTTTGAAAACGAAATTAAAGATATTTTAGAA GATGCATTTAGACGTATTGATGACATTACAGGTGATGAAAACGACTT AAGTACTATTAGTATTATGTTCCGTGTATTCCGTACATACGGTCACA ATTTACCAAGTAGTGTTTTTAAACGTTTCACTGGTGATGACGGTAAA | Sesqui-terpene (A. thalania) |
| | TTTGAACGTTCTTTAACTGAAGATGCTAAAGGAATTTTATCATTATA TGAAGCTGCACATTTAGGAACAACTACTGATTATATTCTTGATGAAG CATTAGAATTTACTTCATCACACTTAAAATCTTTACTTGTTGGTGGTA TGTGTCGTCCACATATTTTACGTCTTATTAGAAATACTTTATATCTTC CACAACGTTGGAATATGGAAGCAGTAATTGCAAGAGAATACATTAG TTTTTATGAACAAGAAGAAGATCACGATAAAATGTTACTTCGTTTAG CTAAATTAAATTTCAAATTACTTCAATTACACTACATTAAAGAGTTA AAAACATTCATTAAATGGTGGATGGAATTAGGACTTACATCAAAAT GGCCTTCTCAATTTCGTGAACGTATTGTTGAAGCATGGTTAGCTGGT CTTATGATGTATTTTGAACCACAGTTTTCTGGAGGTCGTGTAATAGC TGCTAAATTCAATTACTTATTAACAATTTTAGATGATGCATGTGATC ACTATTTCTCAATTCCAGAATTAACTCGTTTAGTTGATTGCGTAGAA AGATGGAATCATGATGGTATACATACTTTAGAAGACATCTCACGTAT CATCTTTAAACTTGCATTAGATGTATTTGATGATATTGGTCGTGGTGT TCGTTCTAAAGGTTGTTCTTATTACTTAAAAGAAATGTTAGAAGAGT TAAAAATCTTAGTTCGTGCAAACTTAGATTTAGTTAAATGGGCTCGT GGTAATCAATTACCTAGTTTTGAAGAACACGTTGAGGTAGGTGGTAT TGCTCTTACAACATACGCAACTTTAATGTACTCTTTTGTTGGCATGGG TGAAGCAGTAGGTAAAGAAGCATACGAATGGGTACGTTCTCGTCCA CGTTTAATCAAAAGTTTAGCAGCAAAAGGTCGTCTTATGGACGATAT TACTGATTTCGAAGTAAAAATTATCAACTTATTTTTCGACCTTCTTTT ATTTGTATTCGGTACCGGTGAAAACTTATATTTCCAGGGTAGTGGTG GAGGAGGTTCAGACTACAAAGATGACGATGACAAAGGAACCGGTTA ATCTAGACTCGAG | |
| 163 | CATATGGTACCAGCAGCTTTCACAGCAAATGCAGTTGACATGCGTCC ACCAGTTATTACAATTCACCCACGTTCAAAAGATATTTCTCTCAATT TTCTTTAGATGATAAATTACAAAAACAATACGCTCAAGGAATCGAA GCTCTTAAAGAAGAAGCTCGTTCTATGCTTATGGCTGCAAAATCTGC TAAAGTAATGATCTTAATTGATACACTTGAACGTTTAGGATTAGGTT ATCACTTTGAAAAAGAAATTGAAGAGAAATTAGAAGCTATTTACAA AAAAGAGGATGGTGACGATTATGATCTTTTTACAACTGCTTTAAGAT TCCGTTTACTTAGACAACACCAACGTCGTGTACCATGTTCTGTTTTTG ACAAATTTATGAATAAAGAGGGTAAATTCGAAGAAGAACCATTAAT TTCAGATGTTGAAGGTCTTCTTTCATTATATGACGCTGCTTATTTACA GATTCACGGTGAACACATTTTACAAGAGGCTTTAATTTTCACTACAC ATCATTTAACTCGTATTGAACCACAATTAGATGATCACTCTCCTTTA AAATTAAAATTAAACCGTGCTTTAGAATTTCCTTTTTACAGAGAAAT CCCTATAATCTATGCACATTTTTACATTTCAGTATATGAACGTGACG ATTCTCGTGATGAAGTATTATTAAAAATGGCTAAATTATCTTATAAT TTCTTACAAAACTTATACAAAAAAGAATTAAGTCAACTTTCTCGTTG | Curcumene (P. cablin) |
| | GTGGAACAAATTAGAACTTATTCCTAATTTACCTTATATTCGTGATTC TGTAGCTGGAGCTTATTTATGGGCTGTTGCTTTATATTTCGAACCTCA ATATTCAGACGTTCGTATGGCAATTGCTAAACTTATCCAAATTGCAG CAGCTGTAGATGATACTTACGATAATTATGCTACTATACGTGAAGCT CAATTATTAACAGAAGCATTAGAACGTTTAAATGTACACGAAATTG ACACATTACCAGATTATATGAAAATTGTTTATCGTTTTGTAATGTCAT GGAGTGAAGATTTCGAACGTGATGCTACAATTAAAGAACAGATGTT AGCTACACCTTATTTCAAAGCTGAAATGAAAAAACTTGGTCGTGCTT ATAATCAAGAACTTAAATGGGTTATGGAACGTCAATTACCTAGTTTC GAAGAATACATGAAAAACTCTGAAATCACTTCTGGTGTTTACATTAT GTTTACTGTAATTAGTCCTTACTTAAATAGTGCAACACAAAAAAACA TTGACTGGTTATTATCACAACCTCGTTTAGCATCTTCAACTGCAATTG TTATGCGTTGTTGTAATGATTTAGGCTCTAATCAACGTGAATCTAAA GGAGGAGAAGTTATGACATCTTTAGATTGCTATATGAAACAACACG GTGCTAGTAAACAAGAAACAATTTCTAAATTCAAACTTATTATCGAA GATGAATGGAAAAACTTAAATGAAGAATGGGCTGCAACAACATGTC TTCCAAAAGTTATGGTAGAAATTTTCGTAACTATGCACGTATTGCA GGCTTTTGCTACAAAAATAACGGTGATGCTTATACATCTCCAAAAAT TGTACAACAATGTTTTGACGCTTTATTTGTAAATCCATTAAGAATTG GTACCGGTGAGAATTTATACTTTCAAGGCTCAGGTGGAGGTGGTAGT GATTATAAAGATGATGATGATAAAGGAACCGGTTAATCTAGACTCG AG | |
| 164 | CATATGGTACCAGAATTTAGAGTTCATTTACAGGCTGATAATGAACA GAAAATATTCCAGAACCAAATGAAACCTGAACCTGAAGCATCATAT CTTATTAATCAACGTAGATCAGCTAATTACAAACCTAATATTTGGAA AAATGACTTTTTAGATCAAAGTTTAATTAGTAAATACGACGGTGATG AATATCGTAAATTAAGTGAGAAATTAATCGAGGAAGTAAAAATTTA TATATCTGCTGAGACAATGGACTTAGTAGCTAAATTAGAACTTATTG ATTCTGTTCGTAAATTAGGTTTAGCTAATCTTTTTGAAAAAGAAATT AAAGAAGCATTAGATTCTATCGCAGCTATTGAGTCAGATAATTTAGG TACTCGTGATGACTTATATGGTACTGCTTTACACTTTAAAATTTTACG TCAACATGGTTATAAAGTTTCTCAAGATATTTTTGGTCGTTTCATGGA TGAAAAAGGTACATTAGAAAATCATCACTTCGCTCACTTAAAAGGT ATGTTAGAATTATTTGAAGCATCTAATTTAGGTTTTGAAGGTGAAGA TATTTTAGATGAAGCAAAAGCATCACTTACATTAGCTCTTCGTGATA GTGGTCATATTTGTTATCCAGATTCTAACTTAAGTCGTGATGTAGTA CACTCATTAGAATTACCTAGTCACCGTCGTGTTCAATGGTTTGATGTT AAATGGCAAATTAATGCTTATGAAAAAGATATTTGTAGAGTTAATGC AACTCTTTTAGAATTAGCAAAATTAAATTTTAACGTAGTACAAGCAC AACTTCAAAAAAACTTACGTGAAGCATCTCGTTGGTGGGCTAACTTA | Farnesene (M. domestica) |
| | GGTTTCGCTGATAACTTAAAATTCGCTCGTGATCGTTTAGTTGAATG TTTTTCTTGCGCAGTAGGCGTAGCATTTGAACCTGAACACTCTTCTTT TCGTATCTGTTTAACAAAAGTTATTAATTTAGTTTAATAATTGATGA CGTATACGACATATATGGAAGTGAAGAATTAAAACACTTTACA AATGCTGTTGATCGTTGGGATTCTCGTGAAACAGAACAATTACCAGA ATGTATGAAAATGTGCTTTCAAGTTTATACAATACTACATGTGAAA TTGCTCGTGAAATTGAAGAAAATGGATGGAATCAAGTTTTACCT CAATTAACTAAAGTATGGGCTGATTTTGTAAAGCATTATTAGTAGA AGCTGAATGGTACAATAAAAGTCACATCCCAACTTTAGAAGAATAT CTTCGTAATGGCTGTATTTCATCAAGTGTTTCTGTATTATTAGTACAT TCTTTCTTTAGTATTACACATGAAGGTACAAAAGAAATGGCAGATTT CTTACACAAAAACGAAGACTTATTATACAACATCTCATTAATTGTAC GTTTAAACAACGACTTAGGTACAAGTGCAGCTGAACAAGAACGTGG TGATTCACCATCATCTATTGTATGTTACATGCGTGAAGTTAATGCTA GTGAAGAAACAGCTCGTAAAAATATAAAGGAATGATCGACAATGC TTGGAAAAAAGTTAATGGTAAATGTTTTACAACTAATCAAGTTCCTT TTCTTTCTTCTTTTATGAATAACGCTACTAATATGGCTCGTGTAGCTC ATTCATTATATAAAGACGGAGACGGTTTTGGCGATCAGGAAAAAGG TCCACGTACTCACATCTTATCTTTATTATTCCAACCATTAGTTAACGG TACCGGTGAAAACTTATACTTTCAAGGTTCTGGTGGTGGTGGTTCTG ACTACAAAGATGACGATGACAAAGGAACCGGTTAATCTAGACTCGA G | |
| 165 | CATATGGTACCAAGTAGTAATGTATCAGCTATTCCTAATTCTTTTGA ATTAATTCGTCGTTCAGCTCAATTTCAGGCTTCTGTATGGGGTGATTA CTTTTTATCTTATCACTCTTTACCACCTGAGAAAGGTAATAAAGTAA TGGAAAAACAAACTGAAGAACTTAAAGAGGAAATCAAAATGGAATT AGTTTCTACTACTAAAGATGAACCAGAGAAATTACGTTTAATTGACC TTATTCAACGTTTAGGTGTATGTTATCACTTTGAAAATGAAATTAAC AACATTTACAACAATTACACCACATTACTATTACTTCTGAGAAAAA CGGTGACGATAATCCTTATAACATGACTTTATGTTTCCGTTTATTACG TCAACAAGGTTACAATGTATCTAGTGAACCTTTTGATCGTTTTCGTG GCAAATGGGAATCTTCTTATGATAACAATGTAGAAGAACTTTTATCA TTATATGAAGCATCTCAATTAAGAATGCAAGGTGAAGAAGCATTAG ATGAAGCATTCTGTTTTGCAACTGCACAATTAGAAGCTATTGTTCAA GATCCTACTACAGATCCAATGGTTGCAGCAGAAATCAGACAAGCAT TAAAATGGCCAATGTACAAAAACTTACCTCGTTTAAAAGCTCGTCAT CATATTGGTTTATATTCTGAGAAACCATGGCGTAATGAGTCATTACT TAATTTCGCAAAAATGGACTTCAATAAACTTCAAAATTTACATCAAA CTGAAATTGCATATATTTCTAAATGGTGGGACGATTACGGCTTTGCA GAAAAACTTTCTTTCGCACGTAATCGTATTGTTGAAGGCTATTTCTTC | Farnesene (C. sativus) |
| | GCATTAGGTATCTTTTTCGAACCTCAACTTTTAACAGCACGTCTTATA ATGACAAAAGTAATCGCTATTGGTTCTATGTTAGATGACATTTATGA TGTTTATGGTACTTTTGAAGAGTTAAAACTTTTAACATTAGCTTTAGA ACGTTGGGATAAATCAGAAACAAAACAATTACCTAATTACATGAAA ATGTACTACGAAGCATTATTAGATGTTTTTGAAGAAATTGAGCAAGA AATGTCACAAAAAGAAACTGAAACAACACCATACTGTATTCATCAC ATGAAAGAAGCTACTAAAGAACTTGGACGTGTATTTTTAGTTGAAGC AACTTGGTGTAAAGAAGGTTATACTCCTAAAGTAGAGGAATACTTA GACATTGCTTTAATTTCTTTTGGTCATAAATTACTTATGGTAACTGCT TTATTAGGTATGGGTTCTCACATGGCTACACAACAAATTGTACAATG GATTACATCTATGCCAAATATCTTAAAAGCATCTGCAGTAATATGTC GTTTAATGAATGACATTGTATCTCATAAATTTGAACAAGAACGTGGT CATGTTGCTTCTGCTATCGAATGCTACATGGAACAAAACCACCTTAG TGAATATGAAGCATTAATTGCTCTTCGTAAACAAATTGATGATTTAT GGAAAGACATGGTAGAAAATTACTGTGCAGTAATCACAGAAGACGA AGTACCTCGTGGTGTTTTAATGCGTGTTTTAAATCTTACACGTTTATT CAATGTTATTTACAAAGACGGTGATGGATACACACAAAGTCATGGT AGTACAAAAGCTCACATTAAAAGTCTTTTAGTTGATAGTGTACCTCT TGGTACCGGTGAAAATCTTTACTTTCAAGGTTCAGGTGGAGGTGGTT CTGATTATAAAGATGATGATGACAAAGGAACCGGTTAATCTAGACT CGAG | |
| 166 | CATATGGTACCAAAAGACATGAGTATTCCATTATTAGCAGCTGTATC TTCTAGTACAGAAGAAACAGTACGTCCTATCGCAGATTTTCATCCAA CACTTTGGGGTAATCATTTTCTTAAATCTGCTGCTGACGTAGAAACT ATTGATGCAGCAACACAAGAGCAACACGCTGCATTAAAACAAGAAG TACGTCGTATGATTACTACAACAGCAAATAAACTTGCACAAAAACTT CACATGATTGATGCTGTACAACGTTTAGGTGTTGCTTATCATTTTGA AAAAGAAATTGAAGACGAATTAGGTAAAGTAAGTCACGATTTAGAT TCAGATGATTTATACGTTGTATCTTTACGTTTTCGTTTATTCCGTCAA CAAGGTGTAAAAATTAGTTGCGATGTTTTCGACAAATTCAAAGATGA CGAAGGAAAATTCAAAGAGTCTCTTATTAACGATATTAGAGGAATG TTATCATTATACGAAGCAGCTTACTTAGCTATTAGAGGTGAAGATAT TTTAGACGAAGCAATTGTTTTCACAACTACTCACTTAAAAAGTGTTA TCTCTATTAGTGATCATTCACATGCTAATAGTAATTTAGCTGAACAA ATACGTCATAGTTTACAAATTCCACTTCGTAAAGCTGCTGCAAGATT AGAAGCACGTTATTTCTTAGATATTTACTCTCGTGATGATTTACATG ATGAAACATTACTTAAATTCGCTAAACTTGACTTTAACATTCTTCAA GCTGCACACCAAAAAGAAGCTAGTATTATGACTCGTTGGTGGAACG ATTTAGGTTTTCCTAAAAAAGTTCCTTATGCTCGTGACCGTATTATAG AAACTTATATTTGGATGTTATTAGGAGTTTCATACGAACCTAATTTA | Farnesene (C. junos) |
| | GCATTTGGAAGAATTTTTGCAAGTAAAGTAGTATGTATGATTACAAC AATTGATGATACATTTGATGCTTATGGTACATTTGAAGAGTTAACAT TATTCACTGAAGCTGTTACACGTTGGGATATTGGTTTAATTGACACA TTACCTGAATATATGAAATTCATTGTAAAAGCTCTTTTAGACATTTA CCGTGAAGCTGAAGAAGAATTAGCTAAAGAAGGTAGATCATACGGT ATTCCATACGCTAAACAAATGATGCAAGAGTTAATCATTTTATACTT TACTGAGGCTAAATGGTTATACAAAGGTTACGTTCCTACATTTGACG AATACAAAAGTGTAGCTTTACGTTCTATTGGTCTTAGAACATTAGCA GTAGCTTCATTTGTAGATTTAGGTGACTTTATTGCTACAAAAGACAA TTTTGAATGTATTCTTAAAAATGCAAAAAGTTTAAAAGCTACTGAAA CAATTGGCCGTTTAATGGATGATATAGCTGGTTACAAATTTGAACAG AAACGTGGTCATAACCCATCTGCTGTTGAGTGTTACAAAAATCAACA CGGAGTATCAGAAGAAGAAGCAGTTAAAGAGCTTTTATTAGAAGTT GCAAACAGTTGGAAAGATATTAACGAGGAACTTTTAAATCCAACTA CAGTTCCATTACCTATGTTACAGCGTTTATTATATTTGCTCGTTCAG GTCACTTCATCTATGATGATGGACATGATCGTTATACACATTCTTTA ATGATGAAAAGACAAGTTGCACTTTTATTAACTGAACCTTTAGCTAT TGGTACCGGTGAAAACTTATACTTTCAAGGTTCAGGTGGTGGTGGAT CTGATTATAAAGATGATGATGACAAAGGAACCGGTTAATCTAGACT CGAG | |
| 167 | CATATGGTACCAGATTTAGCTGTTGAGATTGCAATGGACTTAGCTGT TGATGACGTTGAGCGTCGTGTAGGTGACTATCATAGTAACCTTTGGG ATGATGATTTATTCAGAGTTTATCAACACCATACGGCGCATCATCA TATCGTGAACGTGCTGAAAGATTAGTAGGAGAAGTTAAAGAAATGT TTACTTCTATTTCTATCGAAGATGGTGAACTTACATCTGATTTATTAC AACGTTTATGGATGGTAGATAATGTAGAGCGTTTAGGCATTTCACGT CATTTCGAGAACGAAATAAAAGCAGCTATTGATTATGTTTATTCATA TTGGAGTGACAAAGGTATTGTACGTGGTCGTGATTCAGCTGTTCCTG ACTTAAATAGTATTGCTTTAGGTTTTCGTACATTACGTTTACACGGTT ACACAGTTAGTAGTGATGTATTTAAAGTTTTCCAAGATCGTAAAGGT GAATTTGCTTGCAGTGCAATTCCAACTGAAGGAGATATTAAAGGAG TTTTAAACTTACTTCGTGCAAGTTATATTGCATTCCCTGGTGAAAAA GTAATGGAAAAAGCTCAAACTTTTGCAGCAACATACCTTAAAGAAG CATTACAGAAAATTCAAGTAAGTAGTTTAAGTCGTGAAATCGAATAT GTTCTTGAATACGGTTGGTTAACTAACTTTCCTCGTTTAGAAGCACG TAACTATATTGACGTATTCGGTGAAGAAATTTGTCCATACTTCAAAA AACCATGTATTATGGTTGACAAACTTTTAGAATTAGCAAAATTAGAA TTTAACTTATTTCACAGTCTTCAACAAACAGAGTTAAAACATGTTAG TCGTTGGTGGAAAGATAGTGGTTTCTCTCAATTAACATTTACAAGAC ACCGTCATGTTGAGTTTTATACATTAGCTAGTTGTATAGCAATTGAA | Farnesene (P. abies) |
| | CCAAAACACAGTGCTTTTCGTCTTGGTTTTGCTAAAGTTTGTTATTTA GGTATAGTTTAGATGATATTTATGACACATTTGGTAAAATGAAAGA ATTAGAACTTTTTACTGCAGCAATCAAACGTTGGGACCCTTCTACTA CAGAATGCTTACCTGAATACATGAAAGGTGTTTATATGGCTTTTTAC AATTGTGTTAATGAATTAGCACTTCAAGCAGAGAAAACACAAGGTC GTGATATGTTAAACTATGCACGTAAAGCATGGGAAGCTCTTTTTGAT GCATTTTTAGAAGAAGCAAAATGGATCTCTTCTGGCTATTTACCAAC ATTCGAAGAATACTTAGAAAATGGTAAAGTATCTTTTGGTTATCGTG CTGCTACATTACAACCAATTTTAACATTAGATATTCCTTTACCTTTAC ATATTTTACAACAGATTGATTTTCCAAGTCGTTTTAATGATTTAGCTT CATCTATTTTACGTTTAAGAGGTGATATCTGTGGTTACCAAGCTGAA CGTAGTCGTGGTGAAGAAGCATCATCAATTTCATGTTATATGAAAGA TAATCCAGGTTCTACTGAAGAAGATGCATTATCTCACATTAATGCAA TGATCTCAGACAATATTAACGAATTAAACTGGGAACTTTTAAAACCA AATTCAAATGTACCAATTTCATCAAAAAAACATGCATTTGACATTCT TCGTGCTTTCTATCACTTATACAAATATCGTGATGGCTTCTCTATCGC AAAAATTGAAACTAAAAATCTTGTAATGCGTACAGTTTTAGAACCTG TACCAATGGGTACCGGTGAAAACTTATACTTTCAGGGTTCTGGTGGA GGTGGTTCAGACTATAAAGATGATGATGATAAAGGAACCGGTTAAT CTAGACTCGAG | |
| 168 | CATATGGTACCAACAAGTGTATCAGTAGAATCAGGAACAGTATCTT GTTTATCATCAAACAACTTAATTAGACGTACAGCTAATCCACATCCT AACATTTGGGGATATGATTTTGTTCACTCACTTAAATCACCATATAC ACACGACTCATCATATCGTGAACGTGCTGAGACTTTAATTTCAGAAA TAAAAGTTATGCTTGGAGGTGGTGAATTAATGATGACTCCATCAGCT TATGATACAGCATGGGTAGCTCGTGTTCCATCAATTGACGGTAGTGC TTGTCCACAATTTCCACAAACTGTTGAATGGATTCTTAAAAACCAAT TAAAAGATGGTAGTTGGGGAACTGAATCTCACTTCTTACTTAGTGAC AGATTATTAGCTACATTAAGTTGTGTATTAGCATTATTAAAATGGAA AGTAGCTGATGTTCAAGTAGAGCAAGGTATTGAGTTTATCAAACGTA ATTTACAAGCTATTAAAGACGAACGTGATCAAGACAGTTTAGTAACT GATTTCGAGATTATTTTCCCATCACTTTTAAAAGAGGCTCAATCTTTA AACTTAGGCTTACCTTATGATTTACCATATATTAGATTATTACAAAC AAAACGTCAAGAACGTCTTGCTAACTTAAGTATGGATAAAATTCAC GGTGGTACTTTATTATCATCTTTAGAGGGCATTCAAGATATAGTTGA ATGGGAAACAATTATGGATGTACAATCTCAAGATGGTTCTTTCTTAT CATCACCAGCTTCTACAGCATGTGTATTCATGCATACAGGAGATATG AAATGTTTAGATTTCTTAAACAACGTATTAACTAAATTTGGTAGTAG TGTTCCTTGTTTATACCCTGTAGATTTATTAGAACGTCTTTTAATTGT AGATAATGTAGAGCGTCTTGGTATTGACCGTCATTTTGAAAAAGAAA | Bisabolene (P. abies) |
| | TCAAAGAGGCTTTAGATTATGTTTATCGTCATTGGAACGATCGTGGT ATTGGTTGGGGTCGTTTATCACCTATCGCAGACTTAGAAACAACAGC TTTAGGTTTCGTTTACTTCGTCTTCATCGTTACAATGTTTCTCCTGTA GTATTAGACAATTTCAAAGACGCAGATGGCGAGTTCTTCTGCAGTAC AGGTCAATTTAACAAAGATGTTGCAAGTATGTTATCTTTATACCGTG CTTCTCAATTAGCTTTCCCTGAAGAATCAATTTTAGATGAAGCTAAA TCATTCTCAACACAATATCTTCGTGAAGCATTAGAAAAATCAGAAAC ATTTTCTTCTTGGAATCATCGTCAGAGTTATCAGAAGAAATTAAAT ATGCTTTAAAAACATCATGGCACGCTTCAGTTCCTCGTGTTGAAGCA AAACGTTATTGTCAGGTTTACCGTCAAGACTATGCTCATTTAGCAAA ATCAGTTTATAAACTTCCTAAAGTAAATAATGAGAAAATTCTTGAAT TAGCAAAATTAGATTTTAACATTATTCAATCTATCCATCAAAAAGAA ATGAAAAATGTTACATCATGGTTTCGTGATTCAGGCTTACCACTTTT CACATTTGCTCGTGAAAGACCTTTAGAGTTTACTTTTTAATCGCTGG TGGAACATACGAACCTCAATACGCAAAATGTAGATTCTTATTTACAA AAGTAGCTTGTTTACAAACTGTTTTAGACGATATGTACGATACTTAC GGTACACCATCAGAGTTAAAATTATTTACTGAGGCAGTTCGTCGTTG GGATTTATCATTCACAGAAAACTTACCTGATTATATGAAATTATGCT ACAAAATTTACTATGATATTGTTCATGAAGTTGCTTGGGAAGTAGAA AAAGAACAGGGACGTGAGCTTGTTTCATTTTTCCGTAAAGGTTGGGA AGACTATCTTTTAGGTTATTATGAAGAAGCTGAATGGTTAGCTGCTG AATACGTTCCTACTTTAGATGAATACATTAAAAACGGTATTACATCT ATTGGTCAACGTATTTTACTTTTATCAGGTGTACTTATTATGGAAGGT CAACTTTTATCACAAGAAGCTCTTGAAAAAGTAGATTATCCAGGTCG TCGTGTTTTAACAGAATTAAACAGTTTAATTAGTCGTTTAGCAGACG ATACTAAAACATACAAAGCAGAAAAAGCTCGTGGTGAACTTGCTAG TAGTATTGAATGTTATATGAAAGACCACCCTGGTTGTCAAGAAGAA GAAGCATTAAACCATATTTATGGCATTTTAGAACCAGCTGTTAAAGA ATTAACTCGTGAGTTTCTTAAAGCAGATCACGTACCATTCCCTTGCA AAAAAATGTTATTTGATGAAACAAGAGTTACAATGGTAATTTTCAAA GATGGTGATGGTTTCGGTATTTCTAAATTAGAAGTAAAAGACCACAT AAAAGAATGTTTAATTGAGCCATTACCACTTGGTACCGGTGAAAATC TTTATTTTCAAGGTAGTGGTGGTGGCGGTTCTGACTACAAAGATGAC GACGATAAAGGAACCGGTTAATCTAGACTCGAG | |
| 169 | CATATGGTACCAGGTTCTGAAGTAAATAGACCTTTAGCAGACTTTCC AGCAAACATTTGGGAAGACCCATTAACTTCTTTCTCAAAATCTGATC TTGGTACAGAAACATTTAAAGAGAAACATAGTACTTTAAAAGAAGC TGTTAAAGAGGCATTTATGAGTTCTAAAGCTAATCCAATCGAAAATA TCAAATTCATAGATGCATTATGCCGTTTAGGAGTATCTTATCACTTTG AAAAAGATATTGTAGAACAATTAGATAAATCATTTGATTGCTTAGAT | Sesqui-terpene (A. thalania) |
| | TTTCCACAAATGGTACGTCAAGAAGGTTGCGATTTATATACAGTTGG TATTATCTTTCAAGTTTTAGACAATTTGGTTTCAAATTAAGTGCTGA TGTTTTGAAAAATTCAAAGATGAAAATGGTAAATTCAAAGGTCACT TAGTAACTGATGCTTATGGTATGTTATCATTATACGAAGCTGCACAA TGGGGTACTCACGGTGAAGACATCATTGACGAAGCTCTTGCTTTTTC TCGTAGTCACTTAGAAGAAATATCTAGTCGTAGTTCACCACACTTAG CAATTCGTATTAAAAACGCTTTAAAACATCCATATCATAAAGGTATT TCACGTATTGAAACACGTCAATACATTAGTTACTATGAAGAAGAAG AATCTTGTGATCCAACATTATTAGAGTTCGCTAAAATTGACTTTAAC TTATTACAAATTTTACACCGTGAAGAGTTAGCTTGTGTAACTCGTTG GCATCATGAAATGGAATTTAAAAGTAAAGTAACTTACACACGTCAT CGTATTACAGAAGCATATTTATGGAGTCTTGGAACATATTTTGAACC ACAATACAGTCAAGCTCGTGTAATAACTACAATGGCATTAATCTTAT TTACTGCTTTAGACGACATGTACGATGCTTACGGTACTATGGAGGAG TTAGAGTTATTCACAGATGCTATGGACGAATGGTTACCAGTTGTTCC AGATGAAATTCCTATTCCAGATTCAATGAAATTCATTTACAATGTTA CAGTTGAATTTTACGATAAATTAGACGAAGAATTAGAAAAAGAAGG TCGTTCTGGTTGTGGTTTCCATCTTAAAAAAAGTTTACAAAAAACAG CTAATGGATATATGCAAGAAGCAAAATGGCTTAAAAAAGATTACAT TGCTACATTTGATGAGTATAAAGAAAATGCTATTTTATCTTCAGGTT ATTATGCATTAATTGCAATGACATTTGTTCGTATGACTGATGTTGCTA AATTAGATGCTTTTGAATGGTTAAGTAGTCACCCAAAAATTCGTGTA GCAAGTGAAATCATTTCACGTTTTACAGACGATATTTCAAGTTATGA ATTTGAACACAAACGTGAACACGTTGCTACAGGTATTGATTGTTATA TGCAACAATTCGGAGTTAGTAAAGAACGTGCTGTTGAAGTTATGGG CAATATAGTTTCTGATGCATGGAAAGACTTAAATCAAGAACTTATGC GTCCTCATGTTTTCCCATTTCCACTTCTTATGCGTGTTTTAAATCTTTC AAGAGTAATTGATGTATTTTATCGTTACCAAGATGCATATACTAATC CAAAATTACTTAAAGAGCACATTGTTTCTTTACTTATTGAAACTATTC CAATTGGTACCGGTGAAAACTTATACTTTCAAGGTAGTGGTGGAGGT GGTTCTGATTATAAAGACGACGATGACAAAGGAACCGGTTAATCTA GACTCGAG | |
| 170 | CATATGGTACCAGAGGCAATTAGAGTATTTGGCTTAAAACTTGGTTC AAAATTATCTATTCACTCACAAACAAATGCTTTTCCTGCATTCAAAT TATCTCGTTTTCCATTAACATCTTTCCCTGGTAAACATGCTCACTTAG ATCCATTAAAAGCAACAACTCATCCATTAGCTTTTGATGGTGAAGAA AATAACCGTGAGTTTAAAAACTTAGGTCCAAGTGAGTGGGGCCATC AATTTCTTTCTGCTCATGTAGATTTATCTGAAATGGATGCATTAGAA CGTGAAATTGAAGCTCTTAAACCAAAAGTACGTGATATGTTAATATC AAGTGAAAGTTCAAAAAAAAAAATCTTATTTCTTTATCTTTTAGTAT | Sesqui-terpene (A. thalania) |
| | CATTAGGATTAGCTTATCACTTTGAAGATGAAATTAAAGAAAGTTTA GAGGATGGATTACAGAAAATTGAGGAAATGATGGCTTCAGAAGATG ATCTTCGTTTTAAAGGCGATAATGGTAAATTCAAAGAATGTTTAGCA AAAGATGCTAAAGGTATTTATCTCTTTATGAGGCTGCTCACATGGG TACAACAACTGATTATATTCTTGATGAGGCTTTATCATTTACTTTAAC ATATATGGAATCATTAGCAGCTTCAGGAACATGTAAAATCAACTTAT CACGTCGTATTAGAAAAGCATTAGATCAACCTCAACACAAAAATAT GGAAATAATTGTAGCAATGAAATACATTCAATTTTATGAAGAAGAG GAAGATTGCGATAAAACTTTACTTAAATTTGCTAAACTTAACTTTAA ATTCTTACAATTACACTATTTACAAGAACTTAAAATCTTATCTAAAT GGTATAAAGACCAAGACTTTAAATCAAAATTACCTCCATATTTCCGT GACCGTCTTGTAGAATGTCATTTTGCATCATTAACATGTTTTGAGCCT AAATATGCTCGTGCACGTATTTTCTTATCTAAAATCTTCACTGTTCAA ATTTTCATTGACGATACTTGTGACCGTTACGCATCATTAGGTGAAGT TGAGTCATTAGCTGACACTATCGAACGTTGGGACCCTGATGATCATG CTATGGACGGATTACCTGATTATCTTAAATCAGTAGTTAAATTTGTA TTCAATACATTTCAAGAATTTGAACGTAAATGTAAACGTTCACTTCG TATTAACTTACAAGTAGCAAAATGGGTTAAAGCTGGTCACTTACCAT CTTTTGATGAGTATCTTGATGTAGCTGGTTTAGAATTAGCTATTTCAT TCACTTTCGCTGGTATCTTAATGGGCATGGAAAATGTTTGTAAACCT GAAGCATACGAATGGTTAAAATCTCGTGACAAACTTGTTCGTGGTGT AATCACAAAAGTTCGTTTACTTAATGATATTTTTGGCTATGAAGATG ATATGCGTCGTGGTTATGTAACAAATTCAATAAACTGCTACAAAAAA CAATATGGAGTAACAGAGGAAGAAGCTATTCGTAAATTACATCAAA TCGTTGCTGATGGAGAGAAAATGATGAATGAAGAGTTCTTAAAACC TATTAATGTACCATATCAGGTTCCTAAAGTAGTTATTTTAGACACTTT ACGTGCAGCTAATGTTTCATACGAAAAAGATGACGAATTTACACGTC CAGGCGAACACCTTAAAAACTGCATTACATCTATTTACTTCGATTTA GGTACCGGTGAAAACTTATACTTTCAAGGTAGTGGTGGCGGTGGTA GTGATTACAAAGATGATGATGATAAAGGAACCGGTTAATCTAGACT CGAG | |
| 171 | CATATGGTACCAACTACAACATTATCATCTAACCTTAACTCACAATT CATGCAGGTTTACGAGACTCTTAAATCAGAACTTATTCATGACCCAT TATTTGAGTTCGATGACGATTCAAGACAATGGGTAGAACGTATGATT GATTATACTGTACCAGGTGGTAAAATGGTTCGTGGTTATAGTGTAGT AGATAGTTATCAATTACTTAAAGGTGAAGAACTTACAGAAGAAGAG GCATTTTTAGCTTGTGCACTTGGTTGGTGTACAGAATGGTTTCAAGC ATTCATTCTTTTACATGATGATATGATGGATGGTAGTCACACAAGAC GTGGTCAACCATGTTGGTTTCGTTTACCTGAGGTTGGTGCTGTTGCTA TTAATGATGGTGTTTTACTTCGTAATCACGTTCACCGTATTCTTAAAA | GPP Chimera |
| | AACATTTTCAAGGTAAAGCATATTATGTTCATTTAGTTGATTTATTCA ATGAAACTGAATTTCAAACAATTAGTGGACAAATGATCGACTTAATT ACAACATTAGTTGGTGAAAAAGACTTATCTAAATATTCATTAAGTAT TCATCGTCGTATCGTTCAATACAAAACAGCATACTACTCATTTACTT ACCAGTTGCTTGTGCTTTACTTATGTTTGGTGAGGATCTTGATAAAC ATGTAGAAGTTAAAAATGTTCTTGTTGAAATGGGTACATATTTTCAA GTTCAAGATGATTATTTAGATTGTTTTGGTGCTCCAGAAGTTATTGG CAAAATTGGTACTGATATTGAAGACTTTAAATGTTCATGGTTAGTAG TTAAAGCATTAGAATTAGCAAATGAAGAACAGAAAAAAACTTTACA CGAAAATTATGGAAAAAAAGATCCAGCATCAGTTGCTAAAGTTAAA GAAGTATACCACACACTTAATTTACAAGCTGTTTTCGAAGATTATGA AGCAACATCATACAAAAAACTTATTACTTCTATTGAAAATCACCCAT CTAAAGCTGTTCAAGCTGTTTTAAAATCTTTCTTAGGCAAAATATAC AAACGTCAAAAAGGTACCGGTGAAAACTTATACTTTCAAGGTTCTG GTGGCGGTGGAAGTGATTACAAAGATGATGACGATAAAGGAACCGG TTAATCTAGACTCGAG | |
| 172 | CATATGGTACCAAGTCAACCTTACTGGGCTGCAATTGAAGCAGACAT TGAAAGATATTAAAAAAATCAATTACAATTCGTCCACCAGAAACT GTATTTGGTCCTATGCACCATTTAACATTTGCTGCTCCTGCTACTGCA GCTAGTACATTATGCCTTGCTGCTTGTGAATTAGTTGGCGGTGATCG TAGTCAAGCTATGGCAGCTGCTGCTATCCATTTAGTTCATGCAG CTGCTTACGTTCACGAACATCTTCCTTTAACAGATGGATCACGTCCT GTAAGTAAACCTGCTATTCAACATAAATATGGTCCAAACGTTGAACT TTTAACAGGTGATGGTATCGTTCCTTTCGGTTTTGAGTTATTAGCAGG TTCAGTAGATCCAGCACGTACTGATGACCCTGATCGTATTTTACGTG TAATTATTGAAATTTCTCGTGCTGGTGGACCAGAAGGCATGATTTCT GGTTTACACCGTGAGGAAGAAATCGTAGATGGTAACACATCATTAG ACTTTATAGAATATGTATGCAAAAAAAAATACGGTGAAATGCACGC ATGTGGTGCAGCTTGCGGAGCTATTTTAGGTGGAGCTGCTGAAGAA GAAATTCAAAAACTTCGTAACTTTGGTCTTTATCAAGGCACATTACG TGGTATGATGGAAATGAAAAATAGTCATCAGTTAATTGACGAAAAT ATCATTGGAAAACTTAAAGAACTTGCTCTTGAAGAATTAGGTGGATT CCACGGTAAAAACGCTGAATTAATGAGTTCTTTAGTTGCTGAACCTA GTTTATATGCAGCTTCATCAAATAACTTAGGTATCGAAGGTCGTTTT GACTTTGACGGTTACATGCTTCGTAAAGCAAAATCTGTAAATAAAGC ATTAGAAGCTGCTGTTCAAATGAAAGAACCACTTAAAATTCACGAA TCAATGCGTTATTCATTATTAGCTGGTGGTAAACGTGTTCGTCCAAT GTTATGTATTGCAGCTTGTGAACTTGTTGGTGGTGACGAATCTACAG CAATGCCTGCAGCATGTGCTGTTGAAATGATTCACACAATGTCTTTA ATGCATGATGACCTTCCATGTATGGATAACGATGACTTACGTCGTGG | GPPS-LSU+SSU fusion |
| | TAAACCTACAAACCACATGGCTTTTGGTGAGTCTGTAGCTGTTCTTG CTGGTGATGCATTACTTAGTTTTGCTTTTGAACATGTTGCTGCTGCAA CAAAAGGCGCACCACCTGAACGTATCGTACGTGTATTAGGTGAATT AGCTGTTAGTATTGGTTCAGAAGGACTTGTAGCAGGTCAAGTTGTAG ACGTTTGTTCTGAAGGCATGGCTGAAGTAGGATTAGATCATCTTGAA TTTATTCACCATCATAAAACTGCTGCATTATTACAAGGTTCAGTTGTT TTAGGTGCAATATTAGGAGGCGGTAAAGAAGAAGTAGCTAAAC TTCGTAAATTTGCTAACTGTATTGGTTTACTTTTCCAAGTTGTTGATG ATATTTTAGATGTTACTAAAAGTAGTAAAGAGTTAGGTAAAACTGCA GGTAAAGACTTAGTAGCTGATAAAACTACATATCCTAAACTTATAGG CGTTGAAAAATCAAAAGAATTTGCTGACCGTTTAAATCGTGAAGCA CAAGAACAATTATTACATTTTCATCCTCACCGTGCTGCTCCATTAATC GCTTTAGCTAACTACATCGCTTACCGTGATAATGGTACCGGTGAAAA CTTATACTTCCAGGGTAGTGGTGGTGGCGGATCAGATTATAAAGATG ACGATGATAAAGGAACCGGTTAATCTAGACTCGAG | |
| 173 | CATATGGTACCAGTAACAGCAGCACGTGCAACACCAAAATTAAGTA ATAGAAAATTACGTGTTGCTGTAATTGGAGGCGGTCCAGCAGGAGG TGCAGCTGCTGAAACATTAGCACAAGGAGGTATTGAAACAATTCTT ATCGAACGTAAAATGGATAATTGTAAACCATGTGGTGGTGCTATTCC ATTATGTATGGTAGGAGAGTTCAATTTACCTTTAGACATTATTGACC GTCGTGTAACAAAAATGAAAATGATCTCTCCTTCAAACATTGCAGTT GATATCGGTCGTACACTTAAAGAACACGAATATATTGGTATGGTTCG TCGTGAGGTACTTGATGCTTATCTTCGTGAACGTGCAGAAAAATCAG GTGCTACTGTTATTAACGGTTTATTCTTAAAAATGGATCACCCAGAA AATTGGGATTCACCATATACACTTCACTACACAGAGTATGATGGAAA AACAGGTGCTACAGGAACTAAAAAAACTATGGAAGTAGATGCTGTT ATTGGTGCTGATGGTGCTAATTCTCGTGTTGCAAAAAGTATTGACGC AGGTGATTATGATTATGCTATTGCATTTCAAGAACGTATTCGTATAC CTGATGAGAAAATGACTTATTATGAGGACTTAGCTGAGATGTATGTA GGTGATGATGTATCACCAGACTTCTACGGTTGGGTATTCCCAAAATG TGATCATGTAGCTGTTGGTACAGGTACTGTAACACATAAAGGTGATA TCAAAAAATTCCAGTTAGCTACACGTAATCGTGCTAAAGATAAAATT CTTGGTGGCAAAATAATCCGTGTAGAGGCTCATCCTATTCCAGAGCA TCCTAGACCACGTCGTTTATCAAAACGTGTTGCATTAGTAGGCGACG CAGCAGGTTACGTTACTAAATGTTCAGGAGAAGGAATTTACTTCGCA GCTAAATCTGGTCGTATGTGTGCTGAAGCTATCGTTGAAGGTTCACA AAATGGCAAAAAAATGATAGATGAAGGCGATTTAAGAAAATACTTA GAAAAATGGGATAAAACTTACTTACCAACTTATCGTGTTTTAGATGT ACTTCAAAAAGTTTTCTATCGTTCTAACCCAGCTCGTGAGGCTTTTGT TGAAATGTGTAACGATGAGTATGTACAGAAAATGACATTTGATTCTT | Geranyl-geranyl reductase (A. thalania) |
| | ACCTTTATAAACGTGTAGCTCCTGGTAGTCCATTAGAAGATATCAAA TTAGCTGTAAATACTATTGGTTCACTTGTTCGTGCTAACGCATTACGT CGTGAAATTGAGAAATTATCAGTAGGTACCGGTGAGAATCTTTACTT TCAAGGATCAGGTGGTGGTGGTTCTGATTATAAAGATGACGATGAT AAAGGAACCGGTTAATCTAGACTCGAG | |
| 174 | CATATGGTACCAGTAGCTGTTATTGGTGGTGGTCCAAGTGGCGCTTG TGCAGCAGAAACTTTAGCAAAAGGTGGTGTAGAAACTTTCTTACTTG AGCGTAAATTAGATAATTGTAAACCTTGTGGAGGTGCAATTCCATTA TGTATGGTTGAAGAATTTGATTTACCAATGGAAATAATTGACCGTCG TGTTACTAAAATGAAAATGATATCACCTTCAAACCGTGAAGTTGATG TTGGAAAAACTTTATCAGAAACTGAATGGATCGGTATGTGTCGTCGT GAAGTATTTGACGATTACTTAAGAAACCGTGCACAGAAATTAGGTG CTAATATTGTTAACGGTTTATTCATGCGTTCAGAACAACAATCTGCA GAGGGTCCATTCACAATTCACTATAATTCTTATGAAGACGGTAGTAA AATGGGAAAACCTGCTACTTTAGAAGTTGATATGATAATTGGTGCAG ATGGAGCAAATTCTCGTATTGCAAAAGAGATAGATGCAGGTGAATA CGACTACGCTATAGCTTTTCAAGAACGTATTCGTATTCCTGATGATA AAATGAAATATTACGAAAACCTTGCTGAAATGTATGTAGGTGATGA CGTATCTCCTGATTTCTATGGTTGGGTTTTCCTAAATATGATCACGT TGCTGTTGGTACAGGTACTGTTGTAAACAAAACAGCTATTAAACAAT ATCAACAGGCAACACGTGACAGATCAAAAGTTAAAACAGAAGGTGG CAAAATTATACGTGTTGAAGCACACCCAATTCCAGAACATCCACGTC CACGTCGTTGTAAAGGTCGTGTTGCATTAGTAGGCGACGCAGCTGGT TATGTTACAAAATGTTCTGGCGAGGGCATTTACTTTGCTGCTAAATC TGGTAGAATGGCTGCTGAAGCTATTGTAGAAGGTTCTGCTAACGGTA CAAAAATGTGTGGTGAGGATGCAATTCGTGTTTATTTAGATAAATGG GATCGTAAATATTGGACAACATACAAAGTATTAGACATTTTACAAA AAGTATTTTATCGTAGTAATCCAGCACGTGAAGCATTTGTTGAATTA TGTGAAGATAGTTATGTACAGAAAATGACATTTGATTCATACTTATA TAAAACTGTTGTTCCAGGAAACCCATTAGACGACGTAAAATTACTTG TTCGTACAGTATCTTCTATTTTACGTTCAAATGCTTTACGTTCTGTTA ATTCTAAATCTGTAAATGTTTCTTTCGGCTCTAAAGCAAATGAGGAA CGTGTTATGGCTGCAGGTACCGGTGAAAATCTTTATTTTCAAGGTTC AGGAGGTGGTGGTTCAGATTATAAAGATGATGATGACAAAGGAACC GGTTAATCTAGACTCGAG | Geranylgera nyl reductase (C. reinhardtii) |
| 175 | CATATGGTACCAGCAATGGCAGTACCATTAGATGTAGTAATTACATA TCCTTCTTCAGGTGCTGCTGCTTATCCAGTACTTGTTATGTATAACGG TTTCCAAGCTAAAGCTCCATGGTATCGTGGTATTGTAGATCATGTTT CTAGTTGGGGTTACACAGTTGTTCAATATACAAATGGTGGCTTATTT CCTATTGTTGTAGATCGTGTTGAGTTAACTTATTTAGAGCCATTATTA | Chloro-phyllidohyd rolase (C. reinhardtii) |
| | ACTTGGTTAGAAACACAAAGTGCTGATGCTAAATCTCCTTTATACGG TCGTGCAGATGTTTCTCGTTTAGGTACAATGGGTCATTCACGTGGTG GTAAATTAGCAGCTTTACAATTTGCTGGACGTACAGATGTAAGTGGT TGTGTATTATTTGACCCTGTAGATGGAAGTCCAATGACACCAGAATC TGCTGATTATCCTTCAGCTACAAAAGCATTAGCAGCAGCTGGTCGTT CTGCTGGCTTAGTAGGTGCAGCTATTACAGGTTCATGTAATCCAGTA GGTCAAAATTACCCAAAATTCTGGGGTGCTTTAGCTCCTGGTTCTTG GCAAATGGTATTATCACAAGCTGGTCACATGCAATTTGCTCGTACTG GTAATCCATTCTTAGATTGGTCATTAGACCGTTTATGTGGTCGTGGT ACAATGATGAGTTCAGATGTTATTACATATAGTGCAGCATTTACTGT TGCTTGGTTTGAAGGTATTTTTCGTCCTGCTCAAAGTCAAATGGGTA TTTCTAATTTCAAAACTTGGGCTAATACTCAAGTTGCAGCTCGTAGT ATCACTTTTGATATTAAACCTATGCAATCTCCTCAGGGTACCGGTGA AAACCTTTACTTTCAAGGTAGTGGTGGTGGAGGAAGTGATTATAAA GATGATGATGACAAAGGAACCGGTTAATCTAGACTCGAG | |
| 176 | CATATGGTACCAGCACCACCAAAACCAGTTCGTATAACTTGTCCAAC AGTAGCTGGCACTTATCCTGTTGTTTATTCTTTCACGGTTTTATCTT CGTAACTATTTCTATTCAGATGTTTAAATCATATTGCTAGTCATGGT TACATCTTAGTTGCACCACAATTATGTAAACTTTTACCTCCAGGTGG CCAAGTAGAAGTTGATGACGCTGGTTCAGTTATTAACTGGGCTTCAG AGAATCTTAAAGCACACCTTCCAACTTCTGTTAATGCTAATGGTAAA TATACATCTTTAGTTGGACATTCACGTGGTGGCAAAACAGCTTTCGC AGTTGCATTAGGTCACGCAGCTACATTAGATCCATCAATTACATTTT CAGCATTAATTGGTATTGATCCAGTAGCAGGAACTAACAAATACATT CGTACAGATCCACACATCTTAACTTATAAACCTGAATCATTTGAATT AGATATTCCTGTAGCTGTTGTAGGCACTGGTCTTGGTCCAAAATGGA ATAACGTAATGCCTCCATGCGCACCTACAGATTTAAACCACGAAGA ATTTTACAAAGAATGTAAAGCTACTAAAGCTCACTTTGTTGCTGCTG ATTATGGTCACATGGACATGTTAGACGACGATCTTCCAGGTTTTGTA GGCTTCATGGCTGGTTGTATGTGTAAAAATGGTCAACGTAAAAAATC AGAAATGCGTTCTTTTGTAGGTGGTATAGTTGTAGCATTCTTAAAAT ATTCTTTATGGGGTGAAAAAGCTGAAATAAGATTAATTGTTAAAGAT CCTAGTGTATCTCCTGCTAAATTAGACCCATCACCAGAATTAGAAGA AGCATCAGGTATTTTTGTTGGTACCGGTGAAAATCTTTATTTTCAAG GTTCAGGTGGAGGTGGTTCTGATTATAAAGATGATGATGACAAAGG AACCGGTTAATCTAGACTCGAG | Chloro-phyllido hydrolase (A. thalania) |
| 177 | CATATGGTACCAGCTACACCAGTTGAAGAAGGTGATTATCCAGTTGT AATGTTATTACATGGCTACCTTTTATATAATTCATTTTATTCACAATT AATGTTACATGTATCATCTCACGGTTTCATCTTAATTGCTCCACAATT ATACTCAATTGCTGGTCCTGATACTATGGATGAAATTAAAAGTACTG | Chloro-phyllidohyd rolase (A. thalania) |
| | CTGAGATTATGGACTGGTTATCAGTTGGTTTAAATCACTTTTTACCA GCTCAAGTTACACCTAATTTATCTAAATTTGCATTATCTGGTCATAGT CGTGGTGGTAAAACTGCTTTTGCTGTAGCATTAAAAAAATTTGGTTA TTCTTCAAACTTAAAAATTAGTACTTTAATTGGTATTGATCCAGTAG ACGGAACAGGTAAAGGTAAACAAACTCCACCTCCTGTTTAGCATAT TTACCTAATAGTTTTGACTTAGACAAAACACCAATTTTAGTAATTGG TTCAGGTTTAGGTGAAACTGCACGTAATCCTTTATTTCCTCCATGTGC TCCTCCAGGTGTTAACCACCGTGAGTTTTTCCGTGAATGTCAAGGTC CAGCATGGCACTTTGTTGCTAAAGATTATGGTCATTTAGACATGCTT GATGATGATACAAAAGGTATTCGTGGCAAATCTAGTTACTGTTTATG CAAAAATGGTGAAGAACGTCGTCCAATGCGTCGTTTCGTTGGTGGTT TAGTTGTTAGTTTTCTTAAAGCATATCTTGAAGGTGATGATCGTGAA TTAGTAAAAATCAAAGATGGTTGTCATGAAGATGTACCTGTTGAAAT TCAAGAATTTGAAGTAATTATGGGTACCGGTGAAAATCTTTACTTTC AAGGTTCAGGCGGTGGAGGTTCAGATTATAAAGATGATGATGACAA AGGAACCGGTTAATCTAGACTCGAG | |
| 178 | CATATGGTACCAAGTCACAAAAAAAAAAACGTAATCTTCTTCGTAA CTGATGGTATGGGTCCTGCTTCTCTTTCAATGGCTCGTTCATTTAATC AACACGTTAATGATTTACCAATTGATGATATTTTAACATTAGATGAA CATTTTATTGGAAGTTCAAGAACACGTTCATCAGATTCACTTGTAAC TGACTCAGCTGCTGGAGCTACAGCTTTTGCTTGTGCACTTAAATCAT ACAATGGTGCTATAGGTGTAGATCCACACCATCGTCCATGTGGAACT GTTTTAGAAGCTGCTAAATTAGCAGGTTATTTAACAGGATTAGTAGT TACTACACGTATTACTGATGCTACACCAGCTAGTTTCTCAAGTCACG TAGATTATCGTTGGCAAGAAGATTTAATTGCAACACACCAATTAGGT GAATATCCTTTAGGACGTGTTGTTGATCTTCTTATGGGTGGTGGTCGT TCTCACTTTTATCCTCAAGGTGAAAAAGCTAGTCCATACGGTCACCA CGGTGCACGTAAAGATGGTCGTGATTTAATCGATGAAGCTCAAAGT AATGGCTGGCAGTATGTAGGAGATCGTAAAAATTTTGATTCTTTACT TAAATCACATGGTGAAAATGTTACTTTACCATTTTTAGGTTTATTTGC TGACAACGATATCCCATTTGAAATTGATCGTGATGAAAAAGAATATC CTAGTTTAAAAGAACAAGTAAAAGTAGCATTAGGTGCTTTAGAAAA AGCAAGTAACGAAGATAAAGATAGTAATGGTTTCTTTTTAATGGTAG AAGGTTCTCGTATTGATCATGCTGGCCATCAAAACGATCCTGCATCT CAAGTACGTGAAGTATTAGCATTTGATGAGGCTTTTCAATATGTATT AGAATTTGCAGAAAACAGTGATACAGAAACAGTATTAGTAAGTACA TCAGATCATGAAACAGGTGGTTTAGTTACTTCAAGACAAGTAACAG CATCATACCCACAATATGTATGGTATCCTCAAGTATTAGCTAACGCT ACACATAGTGGAGAGTTTCTTAAACGTAAATTAGTTGATTTCGTTCA TGAACACAAAGGCGCATCATCAAAAATAGAAAACTTCATAAAACAC | Phosphatase (S. cerevisiae) |
| | GAAATTCTTGAAAAAGATTTAGGTATTTATGATTATACAGATTCTGA CTTAGAAACACTTATTCATTTAGATGATAACGCTAATGCAATTCAAG ATAAACTTAATGATATGGTAAGTTTAGAGCTCAAATTGGTTGGACA ACACATGGTCATTCAGCAGTTGATGTAAACATATATGCTTACGCAAA CAAAAAAGCTACATGGTCTTATGTTCTTAATAACTTACAAGGTAATC ACGAAAACACAGAAGTTGGTCAATTCTTAGAGAATTTCTTAGAATTA AACTTAAATGAAGTTACTGATTTAATCCGTGATACAAAACATACTTC TGATTTTGACGCAACAGAAATAGCAAGTGAGGTTCAACACTATGAT GAATATTACCACGAATTAACAAATGGTACCGGTGAAAATCTTTATTT TCAAGGTTCTGGTGGAGGTGGCAGTGATTATAAAGATGATGATGAC AAAGGAACCGGTTAATCTAGACTCGAG | |
| 179 | CATATGGTACCACACAAGTTCACAGGTGTTAACGCTAAATTCCAGCA ACCAGCATTAAGAAATTTATCTCCAGTGGTAGTTGAGCGCGAACGTG AGGAATTTGTAGGATTCTTTCCACAAATTGTTCGTGACTTAACTGAA GATGGTATTGGTCATCCAGAAGTAGGTGACGCTGTAGCTCGTCTTAA AGAAGTATTACAATACAACGCACCTGGTGGTAAATGCAATAGAGGT TTAACAGTTGTTGCAGCTTACCGTGAACTTTCTGGACCAGGTCAAAA AGACGCTGAAAGTCTTCGTTGTGCTTTAGCAGTAGGATGGTGTATTG AATTATTCCAAGCCTTTTTCTTAGTTTGGGACGATATAATGGACCAG TCATTAACTAGACGTGGTCAATTATGTTGGTACAAGAAAGAAGGTGT TGGTTTAGATGCAATAAATGATTCTTTTCTTTTAGAAAGCTCTGTGTA TCGCGTTCTTAAAAAGTATTGCCGTCAACGTCCATATTATGTACATTT ATTAGAGCTTTTTCTTCAAACAGCTTACCAAACAGAATTAGGACAAA TGTTAGATTTAATCACTGCTCCTGTATCTAAGGTAGATTTAAGCCATT TCTCAGAAGAACGTTACAAAGCTATTGTTAAGTATAAAACTGCTTTC TATTCATTCTATTTACCAGTTGCAGCAGCTATGTATATGGTTGGTATA GATTCTAAAGAAGAACATGAAAACGCAAAAGCTATTTTACTTGAGA TGGGTGAATACTTCCAAATTCAAGATGATTATTTAGATTGTTTTGGC GATCCTGCTTTAACAGGTAAAGTAGGTACTGATATTCAAGATAACAA ATGTTCATGGTTAGTTGTGCAATGCTTACAAAGAGTAACACCAGAAC AACGTCAACTTTTAGAAGATAATTACGGTCGTAAAGAACCAGAAAA AGTTGCTAAAGTTAAAGAATTATATGAGGCTGTAGGTATGAGAGCC GCCTTTCAACAATACGAAGAAAGTAGTTACCGTCGTCTTCAAGAGTT AATTGAGAAACATTCTAATCGTTTACCAAAAGAAATTTTCTTAGGTT TAGCTCAGAAAATATACAAACGTCAAAAAGGTACCGGTGAAAACTT ATACTTTCAAGGCTCAGGTGGCGGTGGAAGTGATTACAAAGATGAT GATGATAAAGGAACCGGTTAATCTAGACTCGAG | FPP A118W (G. gallus) |

**Table 7. Nuleic acids encoding exemplary isoprenoid producing enzymes used to increase phytol production**

| **SEQ ID NO.** | **Codon-biased, Synthesized Gene Sequence** | **Enzyme (synthase) encoded** |
|---|---|---|
| 180 | ATGGTACCAGCATTTGACTTCGATGGTTACATGCTTCGTAAAGCT AAATCTGTAAATAAAGCTCTTGAAGCTGCAGTACAAATGAAAGA ACCATTAAAAATTCATGAAAGTATGCGTTATTCTTTATTAGCTGG TGGTAAACGTGTACGTCCAATGTTATGTATTGCAGCTTGTGAATT AGTTGGTGGTGACGAAAGTACTGCTATGCCTGCTGCTTGCGCTG TAGAAATGATTCATACTATGAGTTTAATGCATGATGATTTACCAT GTATGGATAATGACGATTTACGTCGTGGTAAACCAACAAACCAC ATGGCATTTGGTGAAAGTGTAGCAGTATTAGCAGGTGATGCATT ATTATCTTTTGCTTTTGAACATGTAGCAGCAGCAACAAAAGGTG CTCCTCCAGAACGTATTGTTAGAGTTTTAGGTGAACTTGCAGTTT CTATTGGTTCAGAAGGTTTAGTTGCTGGACAAGTAGTTGACGTTT GTTCTGAAGGTATGGCTGAGGTTGGTTTAGATCATTTAGAATTTA TTCATCACCACAAAACTGCTGCTTTATTACAAGGTTCTGTAGTAT TAGGTGCAATATTAGGTGGTGGAAAAGAAGAGGTAGCAAA ACTTCGTAAATTCGCTAACTGCATTGGTTTACTTTTCCAAGTAGT AGATGATATTCTTGATGTAACAAAATCATCTAAAGAATTAGGTA AAACAGCAGGTAAAGATTTAGTTGCTGATAAAACTACTTATCCA AAATTAATCGGTGTTGAGAAAAGTAAAGAGTTCGCAGACCGTTT AAATCGTGAAGCTCAAGAACAACTTCTTCATTTTCATCCACATA GAGCAGCACCTTTAATCGCTTTAGCAAACTATATTGCTTATCGTG ATAATGGTACCGGTGAAAATTTATATTTTCAAGGTTCAGGTGGC GGAGGTTCTGATTATAAAGATGATGATGATAAAGGAACCGGTTA A | GPPS-LSU (M. spicata) |
| 181 | ATGGTACCAAGTCAACCTTACTGGGCAGCAATTGAGGCAGATAT TGAACGTTACTTAAAAAAATCAATTACAATTCGTCCACCAGAAA CTGTATTTGGTCCAATGCACCACTTAACTTTTGCTGCACCAGCTA CAGCTGCTAGTACTTTATGTTTAGCAGCATGTGAACTTGTAGGTG GTGATCGTAGTCAAGCTATGGCTGCAGCAGCAGCAATCCATCTT GTTCATGCAGCTGCTTATGTACATGAACATTTACCATTAACTGAT GGTAGTCGTCCAGTAAGTAAACCAGCTATCCAACATAAATATGG TCCAAATGTAGAATTACTTACAGGTGACGGTATTGTACCATTTG GTTTTGAATTATTAGCAGGTTCTGTTGATCCAGCACGTACAGATG ATCCAGACCGTATTTTACGTGTAATAATTGAAATAAGTCGTGCT GGTGGTCCAGAAGGTATGATTAGTGGTTTACATCGTGAAGAAGA GATTGTAGATGGTAATACTTCTCTTGATTTTATTGAATACGTTTG CAAAAAAAAATATGGTGAAATGCACGCATGTGGTGCTGCATGC | GPPS-SSU (M. spicata) |
| | GGTGCAATTTTAGGTGGTGCAGCTGAAGAAGAAATTCAAAAACT TCGTAACTTCGGATTATATCAAGGAACTTTACGTGGTATGATGG AGATGAAAAACTCACACCAACTTATTGACGAAAATATCATTGGC AAACTTAAAGAATTAGCTTTAGAAGAATTAGGTGGATTTCATGG TAAAAATGCTGAATTAATGTCTAGTTTAGTAGCAGAACCATCAT TATATGCTGCTGGTACCGGTGAAAATTTATACTTTCAAGGTTCTG GTGGTGGTGGCAGTGATTATAAAGACGATGATGACAAAGGAAC CGGTTAA | |
| 182 | ATGGTACCACTTTTATCTAACAAATTAAGAGAGATGGTTTTAGC AGAAGTTCCTAAATTAGCATCTGCTGCTGAATATTTCTTTAAACG TGGTGTTCAGGGTAAACAATTCCGTTCAACAATTTATTATTAAT GGCAACAGCTCTTGACGTTCGTGTTCCAGAAGCATTAATTGGTG AATCTACTGATATTGTAACATCTGAATTACGTGTACGTCAACGT GGCATTGCTGAAATTACAGAAATGATTCATGTAGCATCACTTCT TCACGATGACGTTCTTGACGATGCTGATACTCGTCGTGGTGTTGG TAGTCTTAATGTTGTAATGGGAAACAAAATGTCAGTTTTAGCAG GTGACTTCTTACTTTCTCGTGCTTGTGGTGCTCTTGCAGCTCTTA AAAACACAGAAGTTGTAGCATTATTAGCTACAGCAGTAGAACAC TTAGTTACTGGTGAGACAATGGAAATAACTTCATCAACTGAACA ACGTTATTCTATGGATTACTACATGCAGAAAACTTATTACAAAA CTGCTTCATTAATTTCAAATTCATGTAAAGCAGTTGCTGTATTAA CAGGTCAAACAGCTGAAGTTGCAGTATTAGCTTTTGAATATGGT CGTAATTTAGGTTTAGCTTTCCAGTTAATTGACGACATTTTAGAT TTCACAGGCACATCTGCTAGTTTAGGAAAAGGTTCTTTATCAGA TATACGTCATGGTGTTATTACTGCTCCTATCTTATTTGCAATGGA AGAATTTCCTCAATTAAGAGAAGTAGTAGATCAAGTAGAAAAA GATCCAAGAAATGTAGACATAGCTTTAGAATATTTAGGTAAAAG TAAAGGTATTCAACGTGCTCGTGAATTAGCAATGGAACACGCAA ATTTAGCTGCTGCAGCTATTGGTTCTTTACCTGAAACAGATAACG AAGATGTTAAACGTTCACGTCGTGCTTTAATTGATTTAACACAC AGAGTAATTACACGTAACAAAGGTACCGGTGAGAATTTATACTT TCAAGGTAGTGGTGGAGGAGGTAGTGACTATAAAGATGATGAC GATAAAGGAACCGGTTAA | GPPS (A. thaliana) |
| 183 | ATGGTACCAGTAGTTTCTGAACGTTTAAGACATTCTGTAACAAC TGGTATTCCAGCATTAAAAACAGCAGCTGAATATTTCTTTCGTCG TGGTATCGAAGGAAAACGTTTAAGACCTACATTAGCATTATTAA TGAGTAGTGCTTTATCACCAGCTGCTCCATCACCAGAGTATTTAC | GPPS (C. reinhardtii) |
| | AAGTTGATACAAGACCTGCTGCAGAACACCCTCATGAAATGCGT CGTCGTCAACAACGTTTAGCTGAAATTGCAGAATTAATCCATGT AGCTTCATTACTTCACGATGATGTTATTGATGACGCACAAACAC GTCGTGGTGTTTTAAGTTTAAATACATCTGTTGGTAATAAAACA GCTATCTTAGCAGGTGATTTCTTATTAGCTCGTGCATCTGTAACA TTAGCTAGTTTAAGAAACTCTGAAATTGTAGAATTAATGTCACA GGTTTTAGAACACTTAGTATCTGGTGAAATTATGCAAATGACTG CTACTTCAGAACAACTTTTAGATTTAGAACATTATTTAGCAAAA ACATATTGTAAAACTGCTTCATTAATGGCTAATAGTTCTCGTTCT GTTGCAGTTCTTGCAGGTGCAGCTCCTGAAGTTTGTGATATGGC ATGGTCATACGGTCGTCATTTAGGTATTGCTTTCCAAGTAGTTGA CGATTTATTAGATTTAACAGGTTCATCTTCTGTTTTAGGTAAACC TGCTTTAAACGATATGCGTTCTGGTTTAGCAACAGCACCAGTATT ATTCGCTGCACAAGAAGAACCTGCATTACAGGCTCTTATATTAC GTCGTTTTAAACACGACGGTGACGTAACAAAAGCAATGTCATTA ATTGAACGTACACAAGGCTTACGTCGTGCTGAAGAACTTGCAGC ACAACACGCAAAAGCTGCTGCTGATATGATTCGTTGCTTACCTA CAGCTCAATCAGACCATGCAGAAATTGCTCGTGAAGCATTAATT CAAATTACACATCGTGTTTTAACACGTAAAAAAGGTACCGGTGA AAACTTATACTTTCAAGGTTCTGGTGGTGGTGGATCAGATTATA AAGATGATGATGACAAAGGAACCGGTTAA | |
| 184 | ATGGTACCAACTACAACATTATCATCTAACCTTAACTCACAATTC ATGCAGGTTTACGAGACTCTTAAATCAGAACTTATTCATGACCC ATTATTTGAGTTCGATGACGATTCAAGACAATGGGTAGAACGTA TGATTGATTATACTGTACCAGGTGGTAAAATGGTTCGTGGTTAT AGTGTAGTAGATAGTTATCAATTACTTAAAGGTGAAGAACTTAC AGAAGAAGAGGCATTTTTAGCTTGTGCACTTGGTTGGTGTACAG AATGGTTTCAAGCATTCATTCTTTTACATGATGATATGATGGATG GTAGTCACACAAGACGTGGTCAACCATGTTGGTTTCGTTTACCT GAGGTTGGTGCTGTTGCTATTAATGATGGTGTTTACTTCGTAAT CACGTTCACCGTATTCTTAAAAAACATTTTCAAGGTAAAGCATA TTATGTTCATTTAGTTGATTTATTCAATGAAACTGAATTTCAAAC AATTAGTGGACAAATGATCGACTTAATTACAACATTAGTTGGTG AAAAAGACTTATCTAAATATTCATTAAGTATTCATCGTCGTATCG TTCAATACAAAACAGCATACTACTCATTTTACTTACCAGTTGCTT GTGCTTTACTTATGTTTGGTGAGGATCTTGATAAACATGTAGAA GTTAAAAATGTTCTTGTTGAAATGGGTACATATTTTCAAGTTCAA | GPP Chimera |
| | GATGATTATTTAGATTGTTTTGGTGCTCCAGAAGTTATTGGCAAA ATTGGTACTGATATTGAAGACTTTAAATGTTCATGGTTAGTAGTT AAAGCATTAGAATTAGCAAATGAAGAACAGAAAAAAACTTTAC ACGAAAATTATGGAAAAAAGATCCAGCATCAGTTGCTAAAGTT AAAGAAGTATACCACACACTTAATTTACAAGCTGTTTTCGAAGA TTATGAAGCAACATCATACAAAAAACTTATTACTTCTATTGAAA ATCACCCATCTAAAGCTGTTCAAGCTGTTTTAAAATCTTTCTTAG GCAAAATATACAAACGTCAAAAAGGTACCGGTGAAAACTTATA CTTTCAAGGTTCTGGTGGCGGTGGAAGTGATTACAAAGATGATG ACGATAAAGGAACCGGTTAA | |
| 185 | ATGGTACCAAGTCAACCTTACTGGGCTGCAATTGAAGCAGACAT TGAAAGATATTTAAAAAAATCAATTACAATTCGTCCACCAGAAA CTGTATTTGGTCCTATGCACCATTTAACATTTGCTGCTCCTGCTA CTGCAGCTAGTACATTATGCCTTGCTGCTTGTGAATTAGTTGGCG GTGATCGTAGTCAAGCTATGGCAGCTGCTGCTGCTATCCATTTA GTTCATGCAGCTGCTTACGTTCACGAACATCTTCCTTTAACAGAT GGATCACGTCCTGTAAGTAAACCTGCTATTCAACATAAATATGG TCCAAACGTTGAACTTTTAACAGGTGATGGTATCGTTCCTTTCGG TTTTGAGTTATTAGCAGGTTCAGTAGATCCAGCACGTACTGATG ACCCTGATCGTATTTTACGTGTAATTATTGAAATTTCTCGTGCTG GTGGACCAGAAGGCATGATTTCTGGTTTACACCGTGAGGAAGAA ATCGTAGATGGTAACACATCATTAGACTTTATAGAATATGTATG CAAAAAAAAATACGGTGAAATGCACGCATGTGGTGCAGCTTGC GGAGCTATTTTAGGTGGAGCTGCTGAAGAAATTCAAAAACT TCGTAACTTTGGTCTTTATCAAGGCACATTACGTGGTATGATGGA AATGAAAAATAGTCATCAGTTAATTGACGAAAATATCATTGGAA AACTTAAAGAACTTGCTCTTGAAGAATTAGGTGGATTCCACGGT AAAAACGCTGAATTAATGAGTTCTTTAGTTGCTGAACCTAGTTT ATATGCAGCTTCATCAAATAACTTAGGTATCGAAGGTCGTTTTG ACTTTGACGGTTACATGCTTCGTAAAGCAAAATCTGTAAATAAA GCATTAGAAGCTGCTGTTCAAATGAAAGAACCACTTAAAATTCA CGAATCAATGCGTTATTCATTATTAGCTGGTGGTAAACGTGTTCG TCCAATGTTATGTATTGCAGCTTGTGAACTTGTTGGTGGTGACGA ATCTACAGCAATGCCTGCAGCATGTGCTGTTGAAATGATTCACA CAATGTCTTTAATGCATGATGACCTTCCATGTATGGATAACGAT GACTTACGTCGTGGTAAACCTACAAACCACATGGCTTTTGGTGA GTCTGTAGCTGTTCTTGCTGGTGATGCATTACTTAGTTTTGCTTTT | IS-14-15 fusion |
| | GAACATGTTGCTGCTGCAACAAAAGGCGCACCACCTGAACGTAT CGTACGTGTATTAGGTGAATTAGCTGTTAGTATTGGTTCAGAAG GACTTGTAGCAGGTCAAGTTGTAGACGTTTGTTCTGAAGGCATG GCTGAAGTAGGATTAGATCATCTTGAATTTATTCACCATCATAA AACTGCTGCATTATTACAAGGTTCAGTTGTTTTAGGTGCAATATT AGGAGGCGGTAAAGAAGAAGTAGCTAAACTTCGTAAATTT GCTAACTGTATTGGTTTACTTTTCCAAGTTGTTGATGATATTTTA GATGTTACTAAAAGTAGTAAAGAGTTAGGTAAAACTGCAGGTA AAGACTTAGTAGCTGATAAAACTACATATCCTAAACTTATAGGC GTTGAAAAATCAAAAGAATTTGCTGACCGTTTAAATCGTGAAGC ACAAGAACAATTATTACATTTTCATCCTCACCGTGCTGCTCCATT AATCGCTTTAGCTAACTACATCGCTTACCGTGATAATGGTACCG GTGAAAACTTATACTTCCAGGGTAGTGGTGGTGGCGGATCAGAT TATAAAGATGACGATGATAAAGGAACCGGTTAA | |
| 186 | ATGGTACCACACAAGTTCACAGGTGTTAACGCTAAATTCCAGCA ACCAGCATTAAGAAATTTATCTCCAGTGGTAGTTGAGCGCGAAC GTGAGGAATTTGTAGGATTCTTTCCACAAATTGTTCGTGACTTAA CTGAAGATGGTATTGGTCATCCAGAAGTAGGTGACGCTGTAGCT CGTCTTAAAGAAGTATTACAATACAACGCACCTGGTGGTAAATG CAATAGAGGTTTAACAGTTGTTGCAGCTTACCGTGAACTTTCTG GACCAGGTCAAAAAGACGCTGAAAGTCTTCGTTGTGCTTTAGCA GTAGGATGGTGTATTGAATTATTCCAAGCCTTTTTCTTAGTTTGG GACGATATAATGGACCAGTCATTAACTAGACGTGGTCAATTATG TTGGTACAAGAAAGAAGGTGTTGGTTTAGATGCAATAAATGATT CTTTTCTTTTAGAAAGCTCTGTGTATCGCGTTCTTAAAAAGTATT GCCGTCAACGTCCATATTATGTACATTTATTAGAGCTTTTTCTTC AAACAGCTTACCAAACAGAATTAGGACAAATGTTAGATTTAATC ACTGCTCCTGTATCTAAGGTAGATTTAAGCCATTTCTCAGAAGA ACGTTACAAAGCTATTGTTAAGTATAAAACTGCTTTCTATTCATT CTATTTACCAGTTGCAGCAGCTATGTATATGGTTGGTATAGATTC TAAAGAAGAACATGAAAACGCAAAAGCTATTTTACTTGAGATG GGTGAATACTTCCAAATTCAAGATGATTATTTAGATTGTTTTGGC GATCCTGCTTTAACAGGTAAAGTAGGTACTGATATTCAAGATAA CAAATGTTCATGGTTAGTTGTGCAATGCTTACAAAGAGTAACAC CAGAACAACGTCAACTTTTAGAAGATAATTACGGTCGTAAAGAA CCAGAAAAAGTTGCTAAAGTTAAAGAATTATATGAGGCTGTAGG TATGAGAGCCGCCTTTCAACAATACGAAGAAAGTAGTTACCGTC | IS-09 A118W (G. gallus) |
| | GTCTTCAAGAGTTAATTGAGAAACATTCTAATCGTTTACCAAAA GAAATTTCTTAGGTTTAGCTCAGAAAATATACAAACGTCAAAA AGGTACCGGTGAAAACTTATACTTTCAAGGCTCAGGTGGCGGTG GAAGTGATTACAAAGATGATGATGATAAAGGAACCGGTTAA | |
| 187 | ATGGTACCACACAAGTTCACAGGTGTTAACGCTAAATTCCAGCA ACCAGCATTAAGAAATTTATCTCCAGTGGTAGTTGAGCGCGAAC GTGAGGAATTTGTAGGATTCTTTCCACAAATTGTTCGTGACTTAA CTGAAGATGGTATTGGTCATCCAGAAGTAGGTGACGCTGTAGCT CGTCTTAAAGAAGTATTACAATACAACGCACCTGGTGGTAAATG CAATAGAGGTTTAACAGTTGTTGCAGCTTACCGTGAACTTTCTG GACCAGGTCAAAAAGACGCTGAAAGTCTTCGTTGTGCTTTAGCA GTAGGATGGTGTATTGAATTATTCCAAGCCTTTTTCTTAGTTGCT GACGATATAATGGACCAGTCATTAACTAGACGTGGTCAATTATG TTGGTACAAGAAAGAAGGTGTTGGTTTAGATGCAATAAATGATT CTTTTCTTTTAGAAAGCTCTGTGTATCGCGTTCTTAAAAAGTATT GCCGTCAACGTCCATATTATGTACATTTATTAGAGCTTTTTCTTC AAACAGCTTACCAAACAGAATTAGGACAAATGTTAGATTTAATC ACTGCTCCTGTATCTAAGGTAGATTTAAGCCATTTCTCAGAAGA ACGTTACAAAGCTATTGTTAAGTATAAAACTGCTTTCTATTCATT CTATTTACCAGTTGCAGCAGCTATGTATATGGTTGGTATAGATTC TAAAGAAGAACATGAAAACGCAAAAGCTATTTTACTTGAGATG GGTGAATACTTCCAAATTCAAGATGATTATTTAGATTGTTTTGGC GATCCTGCTTTAACAGGTAAAGTAGGTACTGATATTCAAGATAA CAAATGTTCATGGTTAGTTGTGCAATGCTTACAAAGAGTAACAC CAGAACAACGTCAACTTTTAGAAGATAATTACGGTCGTAAAGAA CCAGAAAAAGTTGCTAAAGTTAAAGAATTATATGAGGCTGTAGG TATGAGAGCCGCCTTTCAACAATACGAAGAAAGTAGTTACCGTC GTCTTCAAGAGTTAATTGAGAAACATTCTAATCGTTTACCAAAA GAAATTTTCTTAGGTTTAGCTCAGAAAATATACAAACGTCAAAA AGGTACCGGTGAAAACTTATACTTTCAAGGCTCAGGTGGCGGTG GAAGTGATTACAAAGATGATGATGATAAAGGAACCGGTTAA | FPP (G. gallus) |
| 188 | ATGGTACCAGATTTTCCACAACAATTAGAAGCATGTGTTAAACA AGCAAATCAAGCATTATCACGTTTCATCGCACCACTTCCATTCCA AAATACTCCTGTTGTTGAAACAATGCAATATGGTGCATTATTAG GAGGTAAAAGATTAAGACCATTTCTTGTATATGCAACAGGTCAC ATGTTTGGAGTATCTACTAACACATTAGATGCTCCAGCTGCTGC AGTTGAATGTATTCATGCATATAGTTTAATTCATGATGATTTACC | FPP (E. coli) |
| | TGCAATGGATGATGATGACTTAAGAAGAGGTTTACCTACATGTC ATGTTAAATTTGGTGAAGCTAATGCTATTTTAGCTGGCGATGCA CTTCAAACTCTTGCATTCAGTATTTATCAGATGCTGATATGCCA GAAGTTTCAGATCGTGATCGTATTTCTATGATATCTGAATTAGCT TCTGCTAGTGGTATTGCTGGTATGTGCGGTGGCCAAGCTCTTGAT TTAGACGCAGAAGGAAAACACGTTCCTTTAGATGCTTTAGAGCG TATACATCGTCACAAAACAGGAGCTTTAATTAGAGCTGCTGTTC GTCTTGGTGCTTTATCAGCTGGAGACAAAGGTCGTCGTGCTTTAC CAGTTTTAGACAAATACGCTGAAAGTATTGGTTTAGCTTTTCAA GTTCAGGATGATATCTTAGATGTTGTAGGTGATACTGCTACTTTA GGTAAACGTCAAGGTGCTGATCAACAGTTAGGCAAATCTACATA CCCAGCACTTTTAGGTTTAGAACAAGCTCGTAAAAAAGCAAGAG ACTTAATTGACGATGCTCGTCAAAGTCTTAAACAATTAGCAGAA CAATCACTTGATACAAGTGCTTTAGAAGCATTAGCAGATTACAT TATTCAACGTAATAAAGGTACCGGTGAAAATTTATATTTTCAAG GTTCTGGTGGTGGAGGTTCAGACTATAAAGATGACGATGATAAA GGAACCGGTTAA | |
| 189 | ATGGTACCAAGTGTTAGTTGTTGTAGAAATTAGGAAAAAC TATCAAAAAAGCTATTCCAAGTCACCACTTACATTTACGTTCTTT AGGTGGTAGTTTATATAGAAGACGTATTCAATCATCTTCAATGG AAACAGACTTAAAATCTACATTCTTAAATGTTTATTCAGTTCTTA AATCAGATTTATTACACGACCCATCATTTGAATTTACAAATGAA AGTCGTTTATGGGTAGATAGAATGCTTGATTATAATGTTCGTGG CGGTAAACTTAATCGTGGTCTTTCTGTAGTAGACTCTTTCAAATT ACTTAAACAAGGTAATGATTTAACTGAACAAGAAGTTTTCTTAT CTTGTGCATTAGGTTGGTGTATTGAGTGGTTACAGGCTTACTTTT TAGTTCTTGATGATATTATGGATAATTCAGTTACACGTCGTGGTC AACCTTGTTGGTTTCGTGTACCACAAGTTGGTATGGTAGCTATTA ATGATGGCATTCTTCTTCGTAACCATATTCATCGTATTCTTAAAA AACACTTCCGTGATAAACCATATTATGTAGATTTAGTTGACCTTT TCAATGAAGTAGAGTTACAAACTGCATGTGGACAAATGATTGAT TTAATCACAACATTTGAAGGTGAAAAAGACTTAGCTAAATATAG TTTATCAATTCACCGTCGTATTGTTCAATACAAAACTGCATATTA CTCATTCTATTTACCAGTTGCATGTGCTCTTTTAATGGCTGGCGA AAATTTAGAAAACCACATTGATGTTAAAAATGTATTAGTAGATA TGGGTATTTACTTTCAAGTTCAGGATGATTATTTAGACTGTTTTG CTGATCCTGAAACATTAGGTAAAATTGGCACTGATATTGAGGAC | FPP (A. thaliana) |
| | TTTAAATGTTCTTGGTTAGTTGTAAAAGCATTAGAACGTTGTAGT GAAGAACAAACAAAAATTCTTTACGAAAACTATGGCAAACCTG ATCCATCTAATGTTGCTAAAGTAAAAGATTTATACAAAGAATTA GATTTAGAAGGCGTTTTCATGGAATATGAATCTAAATCATACGA GAAATTAACTGGTGCTATCGAAGGTCACCAATCTAAAGCAATTC AAGCTGTTCTTAAATCTTTCTTAGCAAAAATCTATAAACGTCAA AAAGGTACCGGTGAAAACTTATACTTTCAAGGTAGTGGTGGCGG TGGTAGTGATTATAAAGATGATGATGATAAAGGAACCGGTTAA | |
| 190 | ATGGTACCAGCTGATCTTAAATCAACATTCTTAGATGTTTATTCA GTATTAAAAAGTGATTTATTACAAGATCCATCTTTTGAATTTACA CACGAAAGTCGTCAATGGTTAGAACGTATGTTAGATTATAATGT TCGTGGAGGCAAATTAAACAGAGGTTTAAGTGTAGTAGACAGTT ACAAACTTTTAAAACAAGGTCAAGACTTAACAGAAAAAGAAAC ATTTTTATCTTGTGCTTTAGGTTGGTGTATTGAATGGTTACAAGC ATACTTCTTAGTTTTAGACGATATTATGGATAATTCTGTAACTAG ACGTGGTCAACCATGTTGGTTTCGTAAACCAAAAGTAGGTATGA TTGCTATTAATGATGGAATACTTCTTCGTAACCACATTCATCGTA TTCTTAAAAAACACTTTCGTGAAATGCCTTATTATGTAGACCTTG TAGACTTATTTAACGAAGTAGAATTTCAAACAGCTTGTGGTCAA ATGATTGACTTAATTACAACATTTGATGGTGAAAAAGACCTTTC AAAATATTCACTTCAGATTCACCGTCGTATTGTTGAGTACAAAA CAGCATACTACTCTTTCTATTTACCTGTAGCATGTGCTTTACTTA TGGCAGGTGAAAATTTAGAAAATCACACAGATGTTAAAACTGTA TTAGTTGATATGGGTATCTATTTCCAAGTTCAAGATGATTATTTA GATTGCTTCGCTGATCCAGAAACATTAGGTAAAATTGGTACAGA TATTGAAGACTTTAAATGTAGTTGGTTAGTAGTAAAAGCATTAG AACGTTGTAGTGAAGAACAAACAAAAATTCTTTACGAAAATTAT GGAAAAGCTGAACCTTCAAATGTAGCTAAAGTTAAAGCATTATA CAAAGAATTAGATTTAGAGGGTGCATTTATGGAATATGAAAAAG AATCATACGAGAAACTTACAAAACTTATTGAAGCACATCAATCA AAAGCTATTCAAGCAGTTCTTAAATCTTTCTTAGCTAAAATTTAT AAACGTCAAAAAGGTACCGGTGAAAACTTATACTTTCAAGGCTC TGGAGGTGGTGGTTCAGACTATAAAGATGATGATGATAAAGGA ACCGGTTAA | FPP (A. thaliana) |
| 191 | ATGGTACCAAGTGGCGAACCTACTCCAAAAAAAATGAAAGCAA CATACGTTCACGACCGTGAAAACTTTACAAAAGTATACGAAACT CTTCGTGACGAATTACTTAACGATGATTGTCTTAGTCCAGCTGGT | FPP (C. reinhardtii) |
| | TCACCTCAGGCTCAAGCTGCTCAAGAGTGGTTTAAAGAAGTTAA TGATTATAATGTTCCTGGTGGAAAACTTAACCGTGGTATGGCTG TATATGACGTTTTAGCTTCAGTTAAAGGTCCAGATGGTTTAAGTG AAGACGAAGTATTTAAAGCTAACGCTCTTGGTTGGTGTATTGAG TGGTTACAAGCATTTTTCTTAGTTGCTGATGATATAATGGATGGT TCAATTACACGTCGTGGCCAACCTTGTTGGTACAAACAACCTAA AGTTGGTATGATTGCTTGTAATGATTACATCTTATTAGAATGCTG TATTTACTCAATTCTTAAAAGACATTTTAGAGGTCACGCTGCATA CGCTCAACTTATGGACCTTTTCCATGAAACTACATTCCAGACTTC ACACGGTCAATTATTAGATTTAACAACAGCACCTATCGGTTCTG TAGACTTATCAAAATATACAGAAGATAATTACCTTCGTATTGTA ACATATAAAACTGCATACTATTCTTTTTATTTACCTGTAGCATGT GGTATGGTATTAGCTGGCATTACAGATCCAGCTGCTTTTGATCTT GCAAAAAATATTTGTGTTGAAATGGGTCAATATTTCCAGATTCA AGACGATTATTTAGATTGCTATGGTGACCCTGAGGTTATTGGTA AAATCGGTACAGACATAGAAGACAACAAATGTAGTTGGTTAGTT TGCACAGCTCTTAAAATCGCAACAGAAGAACAAAAAGAGGTTA TAAAAGCTAATTATGGTCACAAAGAGGCTGAATCAGTAGCAGC AATTAAAGCATTATACGTTGAATTAGGTATTGAACAACGTTTTA AAGACTATGAAGCTGCATCATACGCAAAATTAGAAGGTACAATT AGTGAACAAACTTTATTACCTAAAGCAGTATTTACTTCTTTATTA GCTAAAATCTATAAAAGAAAAAAAGGTACCGGTGAGAACTTAT ACTTTCAAGGTAGTGGAGGTGGTGGTTCAGACTATAAAGATGAT GATGATAAAGGAACCGGTTAA | |
| 192 | ATGGTACCAGTAACAGCAGCACGTGCAACACCAAAATTAAGTA ATAGAAAATTACGTGTTGCTGTAATTGGAGGCGGTCCAGCAGGA GGTGCAGCTGCTGAAACATTAGCACAAGGAGGTATTGAAACAA TTCTTATCGAACGTAAAATGGATAATTGTAAACCATGTGGTGGT GCTATTCCATTATGTATGGTAGGAGAGTTCAATTTACCTTTAGAC ATTATTGACCGTCGTGTAACAAAAATGAAAATGATCTCTCCTTC AAACATTGCAGTTGATATCGGTCGTACACTTAAAGAACACGAAT ATATTGGTATGGTTCGTCGTGAGGTACTTGATGCTTATCTTCGTG AACGTGCAGAAAAATCAGGTGCTACTGTTATTAACGGTTTATTC TTAAAAATGGATCACCCAGAAAATTGGGATTCACCATATACACT TCACTACACAGAGTATGATGGAAAAACAGGTGCTACAGGAACT AAAAAAACTATGGAAGTAGATGCTGTTATTGGTGCTGATGGTGC TAATTCTCGTGTTGCAAAAAGTATTGACGCAGGTGATTATGATT | Geranylgeranyl reductase (A. thaliana) |
| | ATGCTATTGCATTTCAAGAACGTATTCGTATACCTGATGAGAAA ATGACTTATTATGAGGACTTAGCTGAGATGTATGTAGGTGATGA TGTATCACCAGACTTCTACGGTTGGGTATTCCCAAAATGTGATC ATGTAGCTGTTGGTACAGGTACTGTAACACATAAAGGTGATATC AAAAAATTCCAGTTAGCTACACGTAATCGTGCTAAAGATAAAAT TCTTGGTGGCAAAATAATCCGTGTAGAGGCTCATCCTATTCCAG AGCATCCTAGACCACGTCGTTTATCAAAACGTGTTGCATTAGTA GGCGACGCAGCAGGTTACGTTACTAAATGTTCAGGAGAAGGAA TTTACTTCGCAGCTAAATCTGGTCGTATGTGTGCTGAAGCTATCG TTGAAGGTTCACAAAATGGCAAAAAAATGATAGATGAAGGCGA TTTAAGAAAATACTTAGAAAAATGGGATAAAACTTACTTACCAA CTTATCGTGTTTTAGATGTACTTCAAAAAGTTTTCTATCGTTCTA ACCCAGCTCGTGAGGCTTTTGTTGAAATGTGTAACGATGAGTAT GTACAGAAAATGACATTTGATTCTTACCTTTATAAACGTGTAGCT CCTGGTAGTCCATTAGAAGATATCAAATTAGCTGTAAATACTAT TGGTTCACTTGTTCGTGCTAACGCATTACGTCGTGAAATTGAGA AATTATCAGTAGGTACCGGTGAGAATCTTTACTTTCAAGGATCA GGTGGTGGTGGTTCTGATTATAAAGATGACGATGATAAAGGAAC CGGTTAA | |
| 193 | ATGGTACCAGTAGCTGTTATTGGTGGTGGTCCAAGTGGCGCTTG TGCAGCAGAAACTTTAGCAAAAGGTGGTGTAGAAACTTTCTTAC TTGAGCGTAAATTAGATAATTGTAAACCTTGTGGAGGTGCAATT CCATTATGTATGGTTGAAGAATTTGATTTACCAATGGAAATAAT TGACCGTCGTGTTACTAAAATGAAAATGATATCACCTTCAAACC GTGAAGTTGATGTTGGAAAAACTTTATCAGAAACTGAATGGATC GGTATGTGTCGTCGTGAAGTATTTGACGATTACTTAAGAAACCG TGCACAGAAATTAGGTGCTAATATTGTTAACGGTTTATTCATGC GTTCAGAACAACAATCTGCAGAGGGTCCATTCACAATTCACTAT AATTCTTATGAAGACGGTAGTAAAATGGGAAAACCTGCTACTTT AGAAGTTGATATGATAATTGGTGCAGATGGAGCAAATTCTCGTA TTGCAAAAGAGATAGATGCAGGTGAATACGACTACGCTATAGCT TTTCAAGAACGTATTCGTATTCCTGATGATAAAATGAAATATTA CGAAAACCTTGCTGAAATGTATGTAGGTGATGACGTATCTCCTG ATTTCTATGGTTGGGTTTTTCCTAAATATGATCACGTTGCTGTTG GTACAGGTACTGTTGTAAACAAAACAGCTATTAAACAATATCAA CAGGCAACACGTGACAGATCAAAAGTTAAAACAGAAGGTGGCA AAATTATACGTGTTGAAGCACACCCAATTCCAGAACATCCACGT | Geranyl-geranyl reductase (C. reinhardtii) |
| | CCACGTCGTTGTAAAGGTCGTGTTGCATTAGTAGGCGACGCAGC TGGTTATGTTACAAAATGTTCTGGCGAGGGCATTTACTTTGCTGC TAAATCTGGTAGAATGGCTGCTGAAGCTATTGTAGAAGGTTCTG CTAACGGTACAAAAATGTGTGGTGAGGATGCAATTCGTGTTTAT TTAGATAAATGGGATCGTAAATATTGGACAACATACAAAGTATT AGACATTTTACAAAAAGTATTTTATCGTAGTAATCCAGCACGTG AAGCATTTGTTGAATTATGTGAAGATAGTTATGTACAGAAAATG ACATTTGATTCATACTTATATAAAACTGTTGTTCCAGGAAACCCA TTAGACGACGTAAAATTACTTGTTCGTACAGTATCTTCTATTTTA CGTTCAAATGCTTTACGTTCTGTTAATTCTAAATCTGTAAATGTT TCTTTCGGCTCTAAAGCAAATGAGGAACGTGTTATGGCTGCAGG TACCGGTGAAAATCTTTATTTTCAAGGTTCAGGAGGTGGTGGTT CAGATTATAAAGATGATGATGACAAAGGAACCGGTTAA | |
| 194 | ATGGTACCAGCAATGGCAGTACCATTAGATGTAGTAATTACATA TCCTTCTTCAGGTGCTGCTGCTTATCCAGTACTTGTTATGTATAA CGGTTTCCAAGCTAAAGCTCCATGGTATCGTGGTATTGTAGATC ATGTTTCTAGTTGGGGTTACACAGTTGTTCAATATACAAATGGTG GCTTATTTCCTATTGTTGTAGATCGTGTTGAGTTAACTTATTTAG AGCCATTATTAACTTGGTTAGAAACACAAAGTGCTGATGCTAAA TCTCCTTTATACGGTCGTGCAGATGTTTCTCGTTTAGGTACAATG GGTCATTCACGTGGTGGTAAATTAGCAGCTTTACAATTTGCTGG ACGTACAGATGTAAGTGGTTGTGTATTATTTGACCCTGTAGATG GAAGTCCAATGACACCAGAATCTGCTGATTATCCTTCAGCTACA AAAGCATTAGCAGCAGCTGGTCGTTCTGCTGGCTTAGTAGGTGC AGCTATTACAGGTTCATGTAATCCAGTAGGTCAAAATTACCCAA AATTCTGGGGTGCTTTAGCTCCTGGTTCTTGGCAAATGGTATTAT CACAAGCTGGTCACATGCAATTTGCTCGTACTGGTAATCCATTCT TAGATTGGTCATTAGACCGTTTATGTGGTCGTGGTACAATGATG AGTTCAGATGTTATTACATATAGTGCAGCATTTACTGTTGCTTGG TTTGAAGGTATTTTTCGTCCTGCTCAAAGTCAAATGGGTATTTCT AATTTCAAAACTTGGGCTAATACTCAAGTTGCAGCTCGTAGTAT CACTTTTGATATTAAACCTATGCAATCTCCTCAGGGTACCGGTGA AAACCTTTACTTTCAAGGTAGTGGTGGTGGAGGAAGTGATTATA AAGATGATGATGACAAAGGAACCGGTTAA | Chlorophyllidohydrolase (C. reinhardtii) |
| 195 | ATGGTACCAGCACCACCAAAACCAGTTCGTATAACTTGTCCAAC AGTAGCTGGCACTTATCCTGTTGTTTATTCTTTCACGGTTTTAT CTTCGTAACTATTTCTATTCAGATGTTTTAAATCATATTGCTAGT | Chlorophyllidohydrolase (A. thaliana) |
| | CATGGTTACATCTTAGTTGCACCACAATTATGTAAACTTTTACCT CCAGGTGGCCAAGTAGAAGTTGATGACGCTGGTTCAGTTATTAA CTGGGCTTCAGAGAATCTTAAAGCACACCTTCCAACTTCTGTTA ATGCTAATGGTAAATATACATCTTTAGTTGGACATTCACGTGGT GGCAAAACAGCTTTCGCAGTTGCATTAGGTCACGCAGCTACATT AGATCCATCAATTACATTTTCAGCATTAATTGGTATTGATCCAGT AGCAGGAACTAACAAATACATTCGTACAGATCCACACATCTTAA CTTATAAACCTGAATCATTTGAATTAGATATTCCTGTAGCTGTTG TAGGCACTGGTCTTGGTCCAAAATGGAATAACGTAATGCCTCCA TGCGCACCTACAGATTTAAACCACGAAGAATTTTACAAAGAATG TAAAGCTACTAAAGCTCACTTTGTTGCTGCTGATTATGGTCACAT GGACATGTTAGACGACGATCTTCCAGGTTTTGTAGGCTTCATGG CTGGTTGTATGTGTAAAAATGGTCAACGTAAAAAATCAGAAATG CGTTCTTTTGTAGGTGGTATAGTTGTAGCATTCTTAAAATATTCT TTATGGGGTGAAAAAGCTGAAATAAGATTAATTGTTAAAGATCC TAGTGTATCTCCTGCTAAATTAGACCCATCACCAGAATTAGAAG AAGCATCAGGTATTTTTGTTGGTACCGGTGAAAATCTTTATTTTC AAGGTTCAGGTGGAGGTGGTTCTGATTATAAAGATGATGATGAC AAAGGAACCGGTTAA | |
| 196 | ATGGTACCAGCTACACCAGTTGAAGAAGGTGATTATCCAGTTGT AATGTTATTACATGGCTACCTTTTATATAATTCATTTATTCACA ATTAATGTTACATGTATCATCTCACGGTTTCATCTTAATTGCTCC ACAATTATACTCAATTGCTGGTCCTGATACTATGGATGAAATTA AAAGTACTGCTGAGATTATGGACTGGTTATCAGTTGGTTTAAAT CACTTTTTACCAGCTCAAGTTACACCTAATTTATCTAAATTTGCA TTATCTGGTCATAGTCGTGGTGGTAAAACTGCTTTTGCTGTAGCA TTAAAAAAATTTGGTTATTCTTCAAACTTAAAAATTAGTACTTTA ATTGGTATTGATCCAGTAGACGGAACAGGTAAAGGTAAACAAA CTCCACCTCCTGTTTTAGCATATTTACCTAATAGTTTTGACTTAG ACAAAACACCAATTTTAGTAATTGGTTCAGGTTTAGGTGAAACT GCACGTAATCCTTTATTTCCTCCATGTGCTCCTCCAGGTGTTAAC CACCGTGAGTTTTTCCGTGAATGTCAAGGTCCAGCATGGCACTTT GTTGCTAAAGATTATGGTCATTTAGACATGCTTGATGATGATAC AAAAGGTATTCGTGGCAAATCTAGTTACTGTTTATGCAAAAATG GTGAAGAACGTCGTCCAATGCGTCGTTTCGTTGGTGGTTTAGTTG TTAGTTTTCTTAAAGCATATCTTGAAGGTGATGATCGTGAATTAG TAAAAATCAAAGATGGTTGTCATGAAGATGTACCTGTTGAAATT | Chlorophyllidohydrolase (A. thaliana) |
| | CAAGAATTTGAAGTAATTATGGGTACCGGTGAAAATCTTTACTT TCAAGGTTCAGGCGGTGGAGGTTCAGATTATAAAGATGATGATG ACAAAGGAACCGGTTAA | |
| 197 | ATGGTACCAGCTGCTGCACCTGCTGAGACAATGAATAAATC TGCAGCTGGCGCTGAAGTACCAGAGGCTTTCACATCAGTTTTTC AACCAGGTAAATTAGCAGTTGAAGCAATTCAAGTAGATGAAAA TGCAGCTCCTACTCCACCTATTCCTGTTTAATAGTTGCTCCAAA AGATGCTGGTACATATCCAGTTGCTATGTTATTACACGGATTTTT CTTACATAATCACTTTTATGAACACTTATTACGTCACGTTGCATC TCATGGCTTTATCATTGTTGCTCCACAATTTTCTATTAGTATTATT CCATCAGGAGATGCTGAAGACATCGCTGCTGCAAAAGTAGC AGATTGGTTACCTGACGGATTACCAAGTGTTTACCAAAAGGTG TTGAACCAGAGTTATCAAAACTTGCTTTAGCTGGACACAGTCGT GGTGGTCACACAGCTTTTTCTTTAGCTTTAGGTCACGCTAAAACA CAATTAACTTTCAGTGCATTAATTGGTTTAGATCCTGTTGCTGGA ACAGGTAAATCATCTCAATTACAACCAAAAATTCTTACTTATGA GCCAAGTTCATTTGGTATGGCTATGCCAGTTTTAGTTATTGGTAC AGGTTTAGGAGAAGAAAAAAAAACATTTCTTTCCTCCATGTG CTCCTAAAGACGTAAACCATGCAGAATTTTATCGTGAATGTAGA CCACCATGTTACTATTTTGTAACTAAAGATTATGGCCATCTTGAT ATGTTAGATGATGACGCTCCAAAATTTATCACATGTGTTTGTAA AGACGGTAATGGATGTAAAGGAAAAATGCGTCGTTGTGTAGCTG GCATCATGGTTGCTTTCTTAAACGCTGCTTTAGGTGAAAAAGAC GCAGATTTAGAAGCTATTTTACGTGATCCAGCAGTTGCTCCTAC AACATTAGACCCAGTTGAACACCGTGTTGCTGGTACCGGTGAGA ATTTATACTTCCAGGGATCTGGTGGTGGTGGCAGTGATTATAAA GATGATGATGATAAAGGAACCGGTTAA | Chloro-phyllido-hydrolase (T. Aestivum) |
| 198 | ATGGTACCAAGTCACAAAAAAAAAAACGTAATCTTCTTCGTAAC TGATGGTATGGGTCCTGCTTCTCTTTCAATGGCTCGTTCATTTAA TCAACACGTTAATGATTTACCAATTGATGATATTTTAACATTAGA TGAACATTTTATTGGAAGTTCAAGAACACGTTCATCAGATTCAC TTGTAACTGACTCAGCTGCTGGAGCTACAGCTTTTGCTTGTGCAC TTAAATCATACAATGGTGCTATAGGTGTAGATCCACACCATCGT CCATGTGGAACTGTTTTAGAAGCTGCTAAATTAGCAGGTTATTT AACAGGATTAGTAGTTACTACACGTATTACTGATGCTACACCAG CTAGTTTCTCAAGTCACGTAGATTATCGTTGGCAAGAAGATTTA ATTGCAACACACCAATTAGGTGAATATCCTTTAGGACGTGTTGT | Phosphatase (S. cerevisiae) |
| | TGATCTTCTTATGGGTGGTGGTCGTTCTCACTTTTATCCTCAAGG TGAAAAAGCTAGTCCATACGGTCACCACGGTGCACGTAAAGATG GTCGTGATTTAATCGATGAAGCTCAAAGTAATGGCTGGCAGTAT GTAGGAGATCGTAAAAATTTTGATTCTTTACTTAAATCACATGGT GAAAATGTTACTTTACCATTTTTAGGTTTATTTGCTGACAACGAT ATCCCATTTGAAATTGATCGTGATGAAAAAGAATATCCTAGTTT AAAAGAACAAGTAAAAGTAGCATTAGGTGCTTTAGAAAAAGCA AGTAACGAAGATAAAGATAGTAATGGTTTCTTTTTAATGGTAGA AGGTTCTCGTATTGATCATGCTGGCCATCAAAACGATCCTGCAT CTCAAGTACGTGAAGTATTAGCATTTGATGAGGCTTTTCAATAT GTATTAGAATTTGCAGAAAACAGTGATACAGAAACAGTATTAGT AAGTACATCAGATCATGAAACAGGTGGTTTAGTTACTTCAAGAC AAGTAACAGCATCATACCCACAATATGTATGGTATCCTCAAGTA TTAGCTAACGCTACACATAGTGGAGAGTTTCTTAAACGTAAATT AGTTGATTTCGTTCATGAACACAAAGGCGCATCATCAAAAATAG AAAACTTCATAAAACACGAAATTCTTGAAAAAGATTTAGGTATT TATGATTATACAGATTCTGACTTAGAAACACTTATTCATTTAGAT GATAACGCTAATGCAATTCAAGATAAACTTAATGATATGGTAAG TTTTAGAGCTCAAATTGGTTGGACAACACATGGTCATTCAGCAG TTGATGTAAACATATATGCTTACGCAAACAAAAAAGCTACATGG TCTTATGTTCTTAATAACTTACAAGGTAATCACGAAAACACAGA AGTTGGTCAATTCTTAGAGAATTTCTTAGAATTAAACTTAAATG AAGTTACTGATTTAATCCGTGATACAAAACATACTTCTGATTTG ACGCAACAGAAATAGCAAGTGAGGTTCAACACTATGATGAATA TTACCACGAATTAACAAATGGTACCGGTGAAAATCTTTATTTTC AAGGTTCTGGTGGAGGTGGCAGTGATTATAAAGATGATGATGAC AAAGGAACCGGTTAA | |
| 199 | ATGGTACCAGCTTTATACGACATTATTAACTATTTCTACGGTTCA AACTCTAAATTCAACCGTATTACATGGGGTTTTAAATCACCAAC TTTCATCAAATGGAGAATTACTGATTTCATTTTAATCATCGTTTT AATTGTTCTTTTCTTCGTAACTTCTCAAGCAGAGCCATTCCATCG TCAATTTTATCTTAACGACATGACTATCCAACATCCTTTTGCAGA ACATGAACGTGTAACTAATATTCAACTTGGTTTATATTCAACAGT AATTCCTTTATCAGTTATTATCATTGTTGCTTTAATTAGTACATGT CCACCTAAATACAAATTATACAACACTTGGGTTTCAAGTATTGG TTTACTTTTATCAGTTTTAATCACATCTTTTGTTACAAACATCGTT AAAAACTGGTTTGGACGTTTACGTCCTGACTTCTTAGATCGTTGC | Phosphatase (C. albicans) |
| | CAACCAGCTAACGATACACCTAAAGATAAATTAGTTTCTATTGA GGTTTGTACTACAGACAATTTAGACCGTTTAGCTGACGGTTTCG TACAACACCTTCTGGTCATTCTTCAATCTCATTTGCTGGTTTATTC TATTTAACATTATTTCTTTTAGGTCAATCTCAGGCAAATAATGGT AAAACATCTTCATGGCGTACAATGATCAGTTTTATACCTTGGTTA ATGGCTTGTTATATCGCTTTAAGTCGTACACAAGACTACCGTCAT CATTTCATTGACGTATTTGTTGGTAGTTGCTTAGGCTTAATTATC GCAATTTGGCAATACTTCCGTTTATTCCCTTGGTTCGGTGGTAAC CAAGCAAATGATTCATTTAACAACCGTATTATGATTGAAGAGAT TAAACGTAAAGAGGAAATTAAACAAGATGAAAATAACTACCGT CGTATTTCTGATATTTCTACTAATGTAGGTACCGGTGAAAACCTT TACTTTCAAGGTTCAGGTGGCGGCGGTTCAGATTATAAAGATGA TGACGACAAAGGAACCGGTTAA | |

**Table 8. Nuleic acids encoding exemplary isoprenoid producing enzymes used to increase phytol production (with restriction enzyme sites)**

| **SEQ ID NO.** | **Codon-biased, Synthesized Gene Sequence w/Restriction Sites** | **Enzyme (synthase) encoded** |
|---|---|---|
| 200 | CATATGGTACCAGCTTTATACGACATTATTAACTATTTCTACGGT TCAAACTCTAAATTCAACCGTATTACATGGGGTTTAAATCACC AACTTTCATCAAATGGAGAATTACTGATTTCATTTTAATCATCGT TTTAATTGTTCTTTTCTTCGTAACTTCTCAAGCAGAGCCATTCCA TCGTCAATTTTATCTTAACGACATGACTATCCAACATCCTTTTGC AGAACATGAACGTGTAACTAATATTCAACTTGGTTTATATTCAA CAGTAATTCCTTTATCAGTTATTATCATTGTTGCTTTAATTAGTA CATGTCCACCTAAATACAAATTATACAACACTTGGGTTTCAAGT ATTGGTTTACTTTTATCAGTTTTAATCACATCTTTTGTTACAAAC ATCGTTAAAAACTGGTTTGGACGTTTACGTCCTGACTTCTTAGAT CGTTGCCAACCAGCTAACGATACACCTAAAGATAAATTAGTTTC TATTGAGGTTTGTACTACAGACAATTTAGACCGTTTAGCTGACG GTTTTCGTACAACACCTTCTGGTCATTCTTCAATCTCATTTGCTG GTTTATTCTATTTAACATTATTTCTTTTAGGTCAATCTCAGGCAA ATAATGGTAAAACATCTTCATGGCGTACAATGATCAGTTTTATA CCTTGGTTAATGGCTTGTTATATCGCTTTAAGTCGTACACAAGAC TACCGTCATCATTTCATTGACGTATTTGTTGGTAGTTGCTTAGGC TTAATTATCGCAATTTGGCAATACTTCCGTTTATTCCCTTGGTTC GGTGGTAACCAAGCAAATGATTCATTTAACAACCGTATTATGAT | GPPS-LSU (M. spicata) |
| | TGAAGAGATTAAACGTAAAGAGGAAATTAAACAAGATGAAAAT AACTACCGTCGTATTTCTGATATTTCTACTAATGTAGGTACCGGT GAAAACCTTTACTTTCAAGGTTCAGGTGGCGGCGGTTCAGATTA TAAAGATGATGACGACAAAGGAACCGGTTAATCTAGACTCGAG | |
| 201 | CATATGGTACCAAGTCAACCTTACTGGGCAGCAATTGAGGCAGA TATTGAACGTTACTTAAAAAATCAATTACAATTCGTCCACCAG AAACTGTATTTGGTCCAATGCACCACTTAACTTTTGCTGCACCAG CTACAGCTGCTAGTACTTTATGTTTAGCAGCATGTGAACTTGTAG GTGGTGATCGTAGTCAAGCTATGGCTGCAGCAGCAGCAATCCAT CTTGTTCATGCAGCTGCTTATGTACATGAACATTTACCATTAACT GATGGTAGTCGTCCAGTAAGTAAACCAGCTATCCAACATAAATA TGGTCCAAATGTAGAATTACTTACAGGTGACGGTATTGTACCAT TTGGTTTTGAATTATTAGCAGGTTCTGTTGATCCAGCACGTACAG ATGATCCAGACCGTATTTTACGTGTAATAATTGAAATAAGTCGT GCTGGTGGTCCAGAAGGTATGATTAGTGGTTTACATCGTGAAGA AGAGATTGTAGATGGTAATACTTCTCTTGATTTTATTGAATACGT TTGCAAAAAAAAATATGGTGAAATGCACGCATGTGGTGCTGCAT GCGGTGCAATTTTAGGTGGTGCAGCTGAAGAAGAAATTCAAAA ACTTCGTAACTTCGGATTATATCAAGGAACTTTACGTGGTATGAT GGAGATGAAAAACTCACACCAACTTATTGACGAAAATATCATTG GCAAACTTAAAGAATTAGCTTTAGAAGAATTAGGTGGATTTCAT GGTAAAAATGCTGAATTAATGTCTAGTTTAGTAGCAGAACCATC ATTATATGCTGCTGGTACCGGTGAAAATTTATACTTTCAAGGTTC TGGTGGTGGTGGCAGTGATTATAAAGACGATGATGACAAAGGA ACCGGTTAATCTAGACTCGAG | GPPS-SSU (M. spicata) |
| 202 | CATATGGTACCACTTTTATCTAACAAATTAAGAGAGATGGTTTT AGCAGAAGTTCCTAAATTAGCATCTGCTGCTGAATATTTCTTTAA ACGTGGTGTTCAGGGTAAACAATTCCGTTCAACAATTTTATTATT AATGGCAACAGCTCTTGACGTTCGTGTTCCAGAAGCATTAATTG GTGAATCTACTGATATTGTAACATCTGAATTACGTGTACGTCAA CGTGGCATTGCTGAAATTACAGAAATGATTCATGTAGCATCACT TCTTCACGATGACGTTCTTGACGATGCTGATACTCGTCGTGGTGT TGGTAGTCTTAATGTTGTAATGGGAAACAAAATGTCAGTTTTAG CAGGTGACTTCTTACTTTCTCGTGCTTGTGGTGCTCTTGCAGCTC TTAAAAACACAGAAGTTGTAGCATTATTAGCTACAGCAGTAGAA CACTTAGTTACTGGTGAGACAATGGAAATAACTTCATCAACTGA ACAACGTTATTCTATGGATTACTACATGCAGAAAACTTATTACA | GPPS (A. thaliana) |
| | AAACTGCTTCATTAATTTCAAATTCATGTAAAGCAGTTGCTGTAT TAACAGGTCAAACAGCTGAAGTTGCAGTATTAGCTTTTGAATAT GGTCGTAATTTAGGTTTAGCTTTCCAGTTAATTGACGACATTTTA GATTTCACAGGCACATCTGCTAGTTTAGGAAAAGGTTCTTTATC AGATATACGTCATGGTGTTATTACTGCTCCTATCTTATTTGCAAT GGAAGAATTTCCTCAATTAAGAGAAGTAGTAGATCAAGTAGAA AAAGATCCAAGAAATGTAGACATAGCTTTAGAATATTTAGGTAA AAGTAAAGGTATTCAACGTGCTCGTGAATTAGCAATGGAACACG CAAATTTAGCTGCTGCAGCTATTGGTTCTTTACCTGAAACAGATA ACGAAGATGTTAAACGTTCACGTCGTGCTTTAATTGATTTAACA CACAGAGTAATTACACGTAACAAAGGTACCGGTGAGAATTTATA CTTTCAAGGTAGTGGTGGAGGAGGTAGTGACTATAAAGATGATG ACGATAAAGGAACCGGTTAATCTAGACTCGAG | |
| 203 | CATATGGTACCAGTAGTTTCTGAACGTTTAAGACATTCTGTAAC AACTGGTATTCCAGCATTAAAAACAGCAGCTGAATATTTCTTTC GTCGTGGTATCGAAGGAAAACGTTTAAGACCTACATTAGCATTA TTAATGAGTAGTGCTTTATCACCAGCTGCTCCATCACCAGAGTAT TTACAAGTTGATACAAGACCTGCTGCAGAACACCCTCATGAAAT GCGTCGTCGTCAACAACGTTTAGCTGAAATTGCAGAATTAATCC ATGTAGCTTCATTACTTCACGATGATGTTATTGATGACGCACAA ACACGTCGTGGTGTTTTAAGTTTAAATACATCTGTTGGTAATAAA ACAGCTATCTTAGCAGGTGATTTCTTATTAGCTCGTGCATCTGTA ACATTAGCTAGTTTAAGAAACTCTGAAATTGTAGAATTAATGTC ACAGGTTTTAGAACACTTAGTATCTGGTGAAATTATGCAAATGA CTGCTACTTCAGAACAACTTTTAGATTTAGAACATTATTTAGCAA AAACATATTGTAAAACTGCTTCATTAATGGCTAATAGTTCTCGTT CTGTTGCAGTTCTTGCAGGTGCAGCTCCTGAAGTTTGTGATATGG CATGGTCATACGGTCGTCATTTAGGTATTGCTTTCCAAGTAGTTG ACGATTTATTAGATTTAACAGGTTCATCTTCTGTTTTAGGTAAAC CTGCTTTAAACGATATGCGTTCTGGTTTAGCAACAGCACCAGTA TTATTCGCTGCACAAGAAGAACCTGCATTACAGGCTCTTATATT ACGTCGTTTTAAACACGACGGTGACGTAACAAAAGCAATGTCAT TAATTGAACGTACACAAGGCTTACGTCGTGCTGAAGAACTTGCA GCACAACACGCAAAAGCTGCTGCTGATATGATTCGTTGCTTACC TACAGCTCAATCAGACCATGCAGAAATTGCTCGTGAAGCATTAA TTCAAATTACACATCGTGTTTTAACACGTAAAAAGGTACCGGT GAAAACTTATACTTTCAAGGTTCTGGTGGTGGATCAGATTA | GPPS (C. reinhardtii) |
| | TAAAGATGATGATGACAAAGGAACCGGTTAATCTAGACTCGAG | |
| 204 | CATATGGTACCAACTACAACATTATCATCTAACCTTAACTCACA ATTCATGCAGGTTTACGAGACTCTTAAATCAGAACTTATTCATG ACCCATTATTTGAGTTCGATGACGATTCAAGACAATGGGTAGAA CGTATGATTGATTATACTGTACCAGGTGGTAAAATGGTTCGTGG TTATAGTGTAGTAGATAGTTATCAATTACTTAAAGGTGAAGAAC TTACAGAAGAAGAGGCATTTTTAGCTTGTGCACTTGGTTGGTGT ACAGAATGGTTTCAAGCATTCATTCTTTTACATGATGATATGATG GATGGTAGTCACACAAGACGTGGTCAACCATGTTGGTTTCGTTT ACCTGAGGTTGGTGCTGTTGCTATTAATGATGGTGTTTACTTCG TAATCACGTTCACCGTATTCTTAAAAAACATTTTCAAGGTAAAG CATATTATGTTCATTTAGTTGATTTATTCAATGAAACTGAATTTC AAACAATTAGTGGACAAATGATCGACTTAATTACAACATTAGTT GGTGAAAAAGACTTATCTAAATATTCATTAAGTATTCATCGTCG TATCGTTCAATACAAAACAGCATACTACTCATTTTACTTACCAGT TGCTTGTGCTTTACTTATGTTTGGTGAGGATCTTGATAAACATGT AGAAGTTAAAAATGTTCTTGTTGAAATGGGTACATATTTTCAAG TTCAAGATGATTATTTAGATTGTTTTGGTGCTCCAGAAGTTATTG GCAAAATTGGTACTGATATTGAAGACTTTAAATGTTCATGGTTA GTAGTTAAAGCATTAGAATTAGCAAATGAAGAACAGAAAAAA CTTTACACGAAAATTATGGAAAAAAGATCCAGCATCAGTTGCT AAAGTTAAAGAAGTATACCACACACTTAATTTACAAGCTGTTTT CGAAGATTATGAAGCAACATCATACAAAAAACTTATTACTTCTA TTGAAAATCACCCATCTAAAGCTGTTCAAGCTGTTTTAAAATCTT TCTTAGGCAAAATATACAAACGTCAAAAAGGTACCGGTGAAAA CTTATACTTTCAAGGTTCTGGTGGCGGTGGAAGTGATTACAAAG ATGATGACGATAAAGGAACCGGTTAATCTAGACTCGAG | GPP Chimera |
| 205 | CATATGGTACCAAGTCAACCTTACTGGGCTGCAATTGAAGCAGA CATTGAAAGATATTTAAAAAAATCAATTACAATTCGTCCACCAG AAACTGTATTTGGTCCTATGCACCATTTAACATTTGCTGCTCCTG CTACTGCAGCTAGTACATTATGCCTTGCTGCTTGTGAATTAGTTG GCGGTGATCGTAGTCAAGCTATGGCAGCTGCTGCTGCTATCCAT TTAGTTCATGCAGCTGCTTACGTTCACGAACATCTTCCTTTAACA GATGGATCACGTCCTGTAAGTAAACCTGCTATTCAACATAAATA TGGTCCAAACGTTGAACTTTTAACAGGTGATGGTATCGTTCCTTT CGGTTTTGAGTTATTAGCAGGTTCAGTAGATCCAGCACGTACTG ATGACCCTGATCGTATTTTACGTGTAATTATTGAAATTTCTCGTG | IS-14-15 fusion |
| | CTGGTGGACCAGAAGGCATGATTTCTGGTTTACACCGTGAGGAA GAAATCGTAGATGGTAACACATCATTAGACTTTATAGAATATGT ATGCAAAAAAAATACGGTGAAATGCACGCATGTGGTGCAGCT TGCGGAGCTATTTTAGGTGGAGCTGCTGAAGAAATTCAAAA ACTTCGTAACTTTGGTCTTTATCAAGGCACATTACGTGGTATGAT GGAAATGAAAAATAGTCATCAGTTAATTGACGAAAATATCATTG GAAAACTTAAAGAACTTGCTCTTGAAGAATTAGGTGGATTCCAC GGTAAAAACGCTGAATTAATGAGTTCTTTAGTTGCTGAACCTAG TTTATATGCAGCTTCATCAAATAACTTAGGTATCGAAGGTCGTTT TGACTTTGACGGTTACATGCTTCGTAAAGCAAAATCTGTAAATA AAGCATTAGAAGCTGCTGTTCAAATGAAAGAACCACTTAAAATT CACGAATCAATGCGTTATTCATTATTAGCTGGTGGTAAACGTGTT CGTCCAATGTTATGTATTGCAGCTTGTGAACTTGTTGGTGGTGAC GAATCTACAGCAATGCCTGCAGCATGTGCTGTTGAAATGATTCA CACAATGTCTTTAATGCATGATGACCTTCCATGTATGGATAACG ATGACTTACGTCGTGGTAAACCTACAAACCACATGGCTTTTGGT GAGTCTGTAGCTGTTCTTGCTGGTGATGCATTACTTAGTTTTGCT TTTGAACATGTTGCTGCTGCAACAAAAGGCGCACCACCTGAACG TATCGTACGTGTATTAGGTGAATTAGCTGTTAGTATTGGTTCAGA AGGACTTGTAGCAGGTCAAGTTGTAGACGTTTGTTCTGAAGGCA TGGCTGAAGTAGGATTAGATCATCTTGAATTTATTCACCATCATA AAACTGCTGCATTATTACAAGGTTCAGTTGTTTTAGGTGCAATAT TAGGAGGCGGTAAAGAAGAAGTAGCTAAACTTCGTAAATT TGCTAACTGTATTGGTTTACTTTTCCAAGTTGTTGATGATATTTT AGATGTTACTAAAAGTAGTAAAGAGTTAGGTAAAACTGCAGGT AAAGACTTAGTAGCTGATAAAACTACATATCCTAAACTTATAGG CGTTGAAAAATCAAAAGAATTTGCTGACCGTTTAAATCGTGAAG CACAAGAACAATTATTACATTTTCATCCTCACCGTGCTGCTCCAT TAATCGCTTTAGCTAACTACATCGCTTACCGTGATAATGGTACCG GTGAAAACTTATACTTCCAGGGTAGTGGTGGTGGCGGATCAGAT TATAAAGATGACGATGATAAAGGAACCGGTTAATCTAGACTCGA G | |
| 206 | CATATGGTACCACACAAGTTCACAGGTGTTAACGCTAAATTCCA GCAACCAGCATTAAGAAATTTATCTCCAGTGGTAGTTGAGCGCG AACGTGAGGAATTTGTAGGATTCTTTCCACAAATTGTTCGTGACT TAACTGAAGATGGTATTGGTCATCCAGAAGTAGGTGACGCTGTA GCTCGTCTTAAAGAAGTATTACAATACAACGCACCTGGTGGTAA | IS-09 A118W (G. gallus) |
| | ATGCAATAGAGGTTTAACAGTTGTTGCAGCTTACCGTGAACTTT CTGGACCAGGTCAAAAGACGCTGAAAGTCTTCGTTGTGCTTTA GCAGTAGGATGGTGTATTGAATTATTCCAAGCCTTTTTCTTAGTT TGGGACGATATAATGGACCAGTCATTAACTAGACGTGGTCAATT ATGTTGGTACAAGAAAGAAGGTGTTGGTTTAGATGCAATAAATG ATTCTTTTCTTTTAGAAAGCTCTGTGTATCGCGTTCTTAAAAAGT ATTGCCGTCAACGTCCATATTATGTACATTTATTAGAGCTTTTTC TTCAAACAGCTTACCAAACAGAATTAGGACAAATGTTAGATTTA ATCACTGCTCCTGTATCTAAGGTAGATTTAAGCCATTTCTCAGAA GAACGTTACAAAGCTATTGTTAAGTATAAAACTGCTTTCTATTCA TTCTATTTACCAGTTGCAGCAGCTATGTATATGGTTGGTATAGAT TCTAAAGAAGAACATGAAAACGCAAAAGCTATTTTACTTGAGAT GGGTGAATACTTCCAAATTCAAGATGATTATTTAGATTGTTTTGG CGATCCTGCTTTAACAGGTAAAGTAGGTACTGATATTCAAGATA ACAAATGTTCATGGTTAGTTGTGCAATGCTTACAAAGAGTAACA CCAGAACAACGTCAACTTTTAGAAGATAATTACGGTCGTAAAGA ACCAGAAAAAGTTGCTAAAGTTAAAGAATTATATGAGGCTGTAG GTATGAGAGCCGCCTTTCAACAATACGAAGAAAGTAGTTACCGT CGTCTTCAAGAGTTAATTGAGAAACATTCTAATCGTTTACCAAA AGAAATTTTCTTAGGTTTAGCTCAGAAAATATACAAACGTCAAA AAGGTACCGGTGAAAACTTATACTTTCAAGGCTCAGGTGGCGGT GGAAGTGATTACAAAGATGATGATGATAAAGGAACCGGTTAAT CTAGACTCGAG | |
| 207 | CATATGGTACCACACAAGTTCACAGGTGTTAACGCTAAATTCCA GCAACCAGCATTAAGAAATTTATCTCCAGTGGTAGTTGAGCGCG AACGTGAGGAATTTGTAGGATTCTTTCCACAAATTGTTCGTGACT TAACTGAAGATGGTATTGGTCATCCAGAAGTAGGTGACGCTGTA GCTCGTCTTAAAGAAGTATTACAATACAACGCACCTGGTGGTAA ATGCAATAGAGGTTTAACAGTTGTTGCAGCTTACCGTGAACTTT CTGGACCAGGTCAAAAAGACGCTGAAAGTCTTCGTTGTGCTTTA GCAGTAGGATGGTGTATTGAATTATTCCAAGCCTTTTTCTTAGTT GCTGACGATATAATGGACCAGTCATTAACTAGACGTGGTCAATT ATGTTGGTACAAGAAAGAAGGTGTTGGTTTAGATGCAATAAATG ATTCTTTTCTTTTAGAAAGCTCTGTGTATCGCGTTCTTAAAAAGT ATTGCCGTCAACGTCCATATTATGTACATTTATTAGAGCTTTTTC TTCAAACAGCTTACCAAACAGAATTAGGACAAATGTTAGATTTA ATCACTGCTCCTGTATCTAAGGTAGATTTAAGCCATTTCTCAGAA | FPP (G. gallus) |
| | GAACGTTACAAAGCTATTGTTAAGTATAAAACTGCTTTCTATTCA TTCTATTTACCAGTTGCAGCAGCTATGTATATGGTTGGTATAGAT TCTAAAGAAGAACATGAAAACGCAAAAGCTATTTACTTGAGAT GGGTGAATACTTCCAAATTCAAGATGATTATTTAGATTGTTTTGG CGATCCTGCTTTAACAGGTAAAGTAGGTACTGATATTCAAGATA ACAAATGTTCATGGTTAGTTGTGCAATGCTTACAAAGAGTAACA CCAGAACAACGTCAACTTTTAGAAGATAATTACGGTCGTAAAGA ACCAGAAAAAGTTGCTAAAGTTAAAGAATTATATGAGGCTGTAG GTATGAGAGCCGCCTTTCAACAATACGAAGAAAGTAGTTACCGT CGTCTTCAAGAGTTAATTGAGAAACATTCTAATCGTTTACCAAA AGAAATTTTCTTAGGTTTAGCTCAGAAAATATACAAACGTCAAA AAGGTACCGGTGAAAACTTATACTTTCAAGGCTCAGGTGGCGGT GGAAGTGATTACAAAGATGATGATGATAAAGGAACCGGTTAAT CTAGACTCGAG | |
| | CATATGGTACCAGATTTTCCACAACAATTAGAAGCATGTGTTAA ACAAGCAAATCAAGCATTATCACGTTTCATCGCACCACTTCCAT TCCAAAATACTCCTGTTGTTGAAACAATGCAATATGGTGCATTA TTAGGAGGTAAAAGATTAAGACCATTTCTTGTATATGCAACAGG TCACATGTTTGGAGTATCTACTAACACATTAGATGCTCCAGCTGC TGCAGTTGAATGTATTCATGCATATAGTTTAATTCATGATGATTT ACCTGCAATGGATGATGATGACTTAAGAAGAGGTTTACCTACAT GTCATGTTAAATTTGGTGAAGCTAATGCTATTTTAGCTGGCGATG CACTTCAAACTCTTGCATTCAGTATTTTATCAGATGCTGATATGC CAGAAGTTTCAGATCGTGATCGTATTTCTATGATATCTGAATTAG CTTCTGCTAGTGGTATTGCTGGTATGTGCGGTGGCCAAGCTCTTG ATTTAGACGCAGAAGGAAAACACGTTCCTTTAGATGCTTTAGAG CGTATACATCGTCACAAAACAGGAGCTTTAATTAGAGCTGCTGT TCGTCTTGGTGCTTTATCAGCTGGAGACAAAGGTCGTCGTGCTTT ACCAGTTTTAGACAAATACGCTGAAAGTATTGGTTTAGCTTTTCA AGTTCAGGATGATATCTTAGATGTTGTAGGTGATACTGCTACTTT AGGTAAACGTCAAGGTGCTGATCAACAGTTAGGCAAATCTACAT ACCCAGCACTTTTAGGTTTAGAACAAGCTCGTAAAAAAGCAAGA GACTTAATTGACGATGCTCGTCAAAGTCTTAAACAATTAGCAGA ACAATCACTTGATACAAGTGCTTTAGAAGCATTAGCAGATTACA TTATTCAACGTAATAAAGGTACCGGTGAAAATTTATATTTTCAA GGTTCTGGTGGTGGAGGTTCAGACTATAAAGATGACGATGATAA AGGAACCGGTTAATCTAGACTCGAG | FPP (E. coli) |
| | CATATGGTACCAAGTGTTAGTTGTTGTAGAAATTAGGAAA AACTATCAAAAAGCTATTCCAAGTCACCACTTACATTTACGTT CTTTAGGTGGTAGTTTATATAGAAGACGTATTCAATCATCTTCAA TGGAAACAGACTTAAAATCTACATTCTTAAATGTTTATTCAGTTC TTAAATCAGATTTATTACACGACCCATCATTTGAATTTACAAATG AAAGTCGTTTATGGGTAGATAGAATGCTTGATTATAATGTTCGT GGCGGTAAACTTAATCGTGGTCTTTCTGTAGTAGACTCTTTCAAA TTACTTAAACAAGGTAATGATTTAACTGAACAAGAAGTTTTCTT ATCTTGTGCATTAGGTTGGTGTATTGAGTGGTTACAGGCTTACTT TTTAGTTCTTGATGATATTATGGATAATTCAGTTACACGTCGTGG TCAACCTTGTTGGTTTCGTGTACCACAAGTTGGTATGGTAGCTAT TAATGATGGCATTCTTCTTCGTAACCATATTCATCGTATTCTTAA AAAACACTTCCGTGATAAACCATATTATGTAGATTTAGTTGACC TTTTCAATGAAGTAGAGTTACAAACTGCATGTGGACAAATGATT GATTTAATCACAACATTTGAAGGTGAAAAAGACTTAGCTAAATA TAGTTTATCAATTCACCGTCGTATTGTTCAATACAAAACTGCATA TTACTCATTCTATTTACCAGTTGCATGTGCTCTTTTAATGGCTGG CGAAAATTTAGAAAACCACATTGATGTTAAAAATGTATTAGTAG ATATGGGTATTTACTTTCAAGTTCAGGATGATTATTTAGACTGTT TTGCTGATCCTGAAACATTAGGTAAAATTGGCACTGATATTGAG GACTTTAAATGTTCTTGGTTAGTTGTAAAAGCATTAGAACGTTGT AGTGAAGAACAAACAAAAATTCTTTACGAAAACTATGGCAAAC CTGATCCATCTAATGTTGCTAAAGTAAAAGATTTATACAAAGAA TTAGATTTAGAAGGCGTTTTCATGGAATATGAATCTAAATCATA CGAGAAATTAACTGGTGCTATCGAAGGTCACCAATCTAAAGCAA TTCAAGCTGTTCTTAAATCTTTCTTAGCAAAAATCTATAAACGTC AAAAAGGTACCGGTGAAAACTTATACTTTCAAGGTAGTGGTGGC GGTGGTAGTGATTATAAAGATGATGATGATAAAGGAACCGGTTA ATCTAGACTCGAG | FPP (A. thaliana) |
| | CATATGGTACCAGCTGATCTTAAATCAACATTCTTAGATGTTTAT TCAGTATTAAAAAGTGATTTATTACAAGATCCATCTTTTGAATTT ACACACGAAAGTCGTCAATGGTTAGAACGTATGTTAGATTATAA TGTTCGTGGAGGCAAATTAAACAGAGGTTTAAGTGTAGTAGACA GTTACAAACTTTTAAAACAAGGTCAAGACTTAACAGAAAAAGA AACATTTTTATCTTGTGCTTTAGGTTGGTGTATTGAATGGTTACA AGCATACTTCTTAGTTTTAGACGATATTATGGATAATTCTGTAAC TAGACGTGGTCAACCATGTTGGTTTCGTAAACCAAAAGTAGGTA | FPP (A. thaliana) |
| | TGATTGCTATTAATGATGGAATACTTCTTCGTAACCACATTCATC GTATTCTTAAAAAACACTTTCGTGAAATGCCTTATTATGTAGACC TTGTAGACTTATTTAACGAAGTAGAATTTCAAACAGCTTGTGGT CAAATGATTGACTTAATTACAACATTTGATGGTGAAAAAGACCT TTCAAAATATTCACTTCAGATTCACCGTCGTATTGTTGAGTACAA AACAGCATACTACTCTTTCTATTTACCTGTAGCATGTGCTTTACT TATGGCAGGTGAAAATTTAGAAAATCACACAGATGTTAAAACTG TATTAGTTGATATGGGTATCTATTTCCAAGTTCAAGATGATTATT TAGATTGCTTCGCTGATCCAGAAACATTAGGTAAAATTGGTACA GATATTGAAGACTTTAAATGTAGTTGGTTAGTAGTAAAAGCATT AGAACGTTGTAGTGAAGAACAAACAAAAATTCTTTACGAAAATT ATGGAAAAGCTGAACCTTCAAATGTAGCTAAAGTTAAAGCATTA TACAAAGAATTAGATTTAGAGGGTGCATTTATGGAATATGAAAA AGAATCATACGAGAAACTTACAAAACTTATTGAAGCACATCAAT CAAAAGCTATTCAAGCAGTTCTTAAATCTTTCTTAGCTAAAATTT ATAAACGTCAAAAAGGTACCGGTGAAAACTTATACTTTCAAGGC TCTGGAGGTGGTGGTTCAGACTATAAAGATGATGATGATAAAGG AACCGGTTAATCTAGACTCGAG | |
| | CATATGGTACCAAGTGGCGAACCTACTCCAAAAAAAATGAAAG CAACATACGTTCACGACCGTGAAAACTTTACAAAAGTATACGAA ACTCTTCGTGACGAATTACTTAACGATGATTGTCTTAGTCCAGCT GGTTCACCTCAGGCTCAAGCTGCTCAAGAGTGGTTTAAAGAAGT TAATGATTATAATGTTCCTGGTGGAAAACTTAACCGTGGTATGG CTGTATATGACGTTTTAGCTTCAGTTAAAGGTCCAGATGGTTTAA GTGAAGACGAAGTATTTAAAGCTAACGCTCTTGGTTGGTGTATT GAGTGGTTACAAGCATTTTTCTTAGTTGCTGATGATATAATGGAT GGTTCAATTACACGTCGTGGCCAACCTTGTTGGTACAAACAACC TAAAGTTGGTATGATTGCTTGTAATGATTACATCTTATTAGAATG CTGTATTTACTCAATTCTTAAAAGACATTTAGAGGTCACGCTGC ATACGCTCAACTTATGGACCTTTTCCATGAAACTACATTCCAGAC TTCACACGGTCAATTATTAGATTTAACAACAGCACCTATCGGTTC TGTAGACTTATCAAAATATACAGAAGATAATTACCTTCGTATTG TAACATATAAAACTGCATACTATTCTTTTTATTTACCTGTAGCAT GTGGTATGGTATTAGCTGGCATTACAGATCCAGCTGCTTTTGATC TTGCAAAAAATATTTGTGTTGAAATGGGTCAATATTTCCAGATTC AAGACGATTATTTAGATTGCTATGGTGACCCTGAGGTTATTGGT AAAATCGGTACAGACATAGAAGACAACAAATGTAGTTGGTTAG | FPP (C. reinhardtii) |
| | TTTGCACAGCTCTTAAAATCGCAACAGAAGAACAAAAAGAGGTT ATAAAAGCTAATTATGGTCACAAAGAGGCTGAATCAGTAGCAG CAATTAAAGCATTATACGTTGAATTAGGTATTGAACAACGTTTT AAAGACTATGAAGCTGCATCATACGCAAAATTAGAAGGTACAA TTAGTGAACAAACTTTATTACCTAAAGCAGTATTTACTTCTTTAT TAGCTAAAATCTATAAAAGAAAAAAAGGTACCGGTGAGAACTT ATACTTTCAAGGTAGTGGAGGTGGTGGTTCAGACTATAAAGATG ATGATGATAAAGGAACCGGTTAATCTAGACTCGAG | |
| | CATATGGTACCAGTAACAGCAGCACGTGCAACACCAAAATTAA GTAATAGAAAATTACGTGTTGCTGTAATTGGAGGCGGTCCAGCA GGAGGTGCAGCTGCTGAAACATTAGCACAAGGAGGTATTGAAA CAATTCTTATCGAACGTAAAATGGATAATTGTAAACCATGTGGT GGTGCTATTCCATTATGTATGGTAGGAGAGTTCAATTTACCTTTA GACATTATTGACCGTCGTGTAACAAAAATGAAAATGATCTCTCC TTCAAACATTGCAGTTGATATCGGTCGTACACTTAAAGAACACG AATATATTGGTATGGTTCGTCGTGAGGTACTTGATGCTTATCTTC GTGAACGTGCAGAAAAATCAGGTGCTACTGTTATTAACGGTTTA TTCTTAAAAATGGATCACCCAGAAAATTGGGATTCACCATATAC ACTTCACTACACAGAGTATGATGGAAAAACAGGTGCTACAGGA ACTAAAAAAACTATGGAAGTAGATGCTGTTATTGGTGCTGATGG TGCTAATTCTCGTGTTGCAAAAAGTATTGACGCAGGTGATTATG ATTATGCTATTGCATTTCAAGAACGTATTCGTATACCTGATGAGA AAATGACTTATTATGAGGACTTAGCTGAGATGTATGTAGGTGAT GATGTATCACCAGACTTCTACGGTTGGGTATTCCCAAAATGTGA TCATGTAGCTGTTGGTACAGGTACTGTAACACATAAAGGTGATA TCAAAAAATTCCAGTTAGCTACACGTAATCGTGCTAAAGATAAA ATTCTTGGTGGCAAAATAATCCGTGTAGAGGCTCATCCTATTCC AGAGCATCCTAGACCACGTCGTTTATCAAAACGTGTTGCATTAG TAGGCGACGCAGCAGGTTACGTTACTAAATGTTCAGGAGAAGG AATTTACTTCGCAGCTAAATCTGGTCGTATGTGTGCTGAAGCTAT CGTTGAAGGTTCACAAAATGGCAAAAAAATGATAGATGAAGGC GATTTAAGAAAATACTTAGAAAAATGGGATAAAACTTACTTACC AACTTATCGTGTTTTAGATGTACTTCAAAAAGTTTTCTATCGTTC TAACCCAGCTCGTGAGGCTTTTGTTGAAATGTGTAACGATGAGT ATGTACAGAAAATGACATTTGATTCTTACCTTTATAAACGTGTA GCTCCTGGTAGTCCATTAGAAGATATCAAATTAGCTGTAAATAC TATTGGTTCACTTGTTCGTGCTAACGCATTACGTCGTGAAATTGA | Geranylgeranyl reductase (A. thaliana) |
| | GAAATTATCAGTAGGTACCGGTGAGAATCTTTACTTTCAAGGAT CAGGTGGTGGTGGTTCTGATTATAAAGATGACGATGATAAAGGA ACCGGTTAATCTAGACTCGAG | |
| | CATATGGTACCAGTAGCTGTTATTGGTGGTGGTCCAAGTGGCGC TTGTGCAGCAGAAACTTTAGCAAAAGGTGGTGTAGAAACTTTCT TACTTGAGCGTAAATTAGATAATTGTAAACCTTGTGGAGGTGCA ATTCCATTATGTATGGTTGAAGAATTTGATTTACCAATGGAAAT AATTGACCGTCGTGTTACTAAAATGAAAATGATATCACCTTCAA ACCGTGAAGTTGATGTTGGAAAAACTTATCAGAAACTGAATGG ATCGGTATGTGTCGTCGTGAAGTATTTGACGATTACTTAAGAAA CCGTGCACAGAAATTAGGTGCTAATATTGTTAACGGTTTATTCAT GCGTTCAGAACAACAATCTGCAGAGGGTCCATTCACAATTCACT ATAATTCTTATGAAGACGGTAGTAAAATGGGAAAACCTGCTACT TTAGAAGTTGATATGATAATTGGTGCAGATGGAGCAAATTCTCG TATTGCAAAAGAGATAGATGCAGGTGAATACGACTACGCTATAG CTTTTCAAGAACGTATTCGTATTCCTGATGATAAAATGAAATATT ACGAAAACCTTGCTGAAATGTATGTAGGTGATGACGTATCTCCT GATTTCTATGGTTGGGTTTTCCTAAATATGATCACGTTGCTGTT GGTACAGGTACTGTTGTAAACAAAACAGCTATTAAACAATATCA ACAGGCAACACGTGACAGATCAAAAGTTAAAACAGAAGGTGGC AAAATTATACGTGTTGAAGCACACCCAATTCCAGAACATCCACG TCCACGTCGTTGTAAAGGTCGTGTTGCATTAGTAGGCGACGCAG CTGGTTATGTTACAAAATGTTCTGGCGAGGGCATTTACTTTGCTG CTAAATCTGGTAGAATGGCTGCTGAAGCTATTGTAGAAGGTTCT GCTAACGGTACAAAAATGTGTGGTGAGGATGCAATTCGTGTTTA TTTAGATAAATGGGATCGTAAATATTGGACAACATACAAAGTAT TAGACATTTTACAAAAAGTATTTTATCGTAGTAATCCAGCACGT GAAGCATTTGTTGAATTATGTGAAGATAGTTATGTACAGAAAAT GACATTTGATTCATACTTATATAAAACTGTTGTTCCAGGAAACCC ATTAGACGACGTAAAATTACTTGTTCGTACAGTATCTTCTATTTT ACGTTCAAATGCTTTACGTTCTGTTAATTCTAAATCTGTAAATGT TTCTTTCGGCTCTAAAGCAAATGAGGAACGTGTTATGGCTGCAG GTACCGGTGAAAATCTTTATTTTCAAGGTTCAGGAGGTGGTGGT TCAGATTATAAAGATGATGATGACAAAGGAACCGGTTAATCTAG ACTCGAG | Geranylgeranyl reductase (C. reinhardtii) |
| | CATATGGTACCAGCAATGGCAGTACCATTAGATGTAGTAATTAC ATATCCTTCTTCAGGTGCTGCTGCTTATCCAGTACTTGTTATGTA | Chlorophyllidohydrolase (C.reinhardtii) |
| | TAACGGTTTCCAAGCTAAAGCTCCATGGTATCGTGGTATTGTAG ATCATGTTTCTAGTTGGGGTTACACAGTTGTTCAATATACAAATG GTGGCTTATTTCCTATTGTTGTAGATCGTGTTGAGTTAACTTATT TAGAGCCATTATTAACTTGGTTAGAAACACAAAGTGCTGATGCT AAATCTCCTTTATACGGTCGTGCAGATGTTTCTCGTTTAGGTACA ATGGGTCATTCACGTGGTGGTAAATTAGCAGCTTTACAATTTGCT GGACGTACAGATGTAAGTGGTTGTGTATTATTTGACCCTGTAGA TGGAAGTCCAATGACACCAGAATCTGCTGATTATCCTTCAGCTA CAAAAGCATTAGCAGCAGCTGGTCGTTCTGCTGGCTTAGTAGGT GCAGCTATTACAGGTTCATGTAATCCAGTAGGTCAAAATTACCC AAAATTCTGGGGTGCTTTAGCTCCTGGTTCTTGGCAAATGGTATT ATCACAAGCTGGTCACATGCAATTTGCTCGTACTGGTAATCCATT CTTAGATTGGTCATTAGACCGTTTATGTGGTCGTGGTACAATGAT GAGTTCAGATGTTATTACATATAGTGCAGCATTTACTGTTGCTTG GTTTGAAGGTATTTTTCGTCCTGCTCAAAGTCAAATGGGTATTTC TAATTTCAAAACTTGGGCTAATACTCAAGTTGCAGCTCGTAGTA TCACTTTTGATATTAAACCTATGCAATCTCCTCAGGGTACCGGTG AAAACCTTTACTTTCAAGGTAGTGGTGGTGGAGGAAGTGATTAT AAAGATGATGATGACAAAGGAACCGGTTAATCTAGACTCGAG | |
| | CATATGGTACCAGCACCACCAAAACCAGTTCGTATAACTTGTCC AACAGTAGCTGGCACTTATCCTGTTGTTTATTCTTTCACGGTTT TTATCTTCGTAACTATTTCTATTCAGATGTTTAAATCATATTGCT AGTCATGGTTACATCTTAGTTGCACCACAATTATGTAAACTTTTA CCTCCAGGTGGCCAAGTAGAAGTTGATGACGCTGGTTCAGTTAT TAACTGGGCTTCAGAGAATCTTAAAGCACACCTTCCAACTTCTG TTAATGCTAATGGTAAATATACATCTTTAGTTGGACATTCACGTG GTGGCAAAACAGCTTTCGCAGTTGCATTAGGTCACGCAGCTACA TTAGATCCATCAATTACATTTTCAGCATTAATTGGTATTGATCCA GTAGCAGGAACTAACAAATACATTCGTACAGATCCACACATCTT AACTTATAAACCTGAATCATTTGAATTAGATATTCCTGTAGCTGT TGTAGGCACTGGTCTTGGTCCAAAATGGAATAACGTAATGCCTC CATGCGCACCTACAGATTTAAACCACGAAGAATTTTACAAAGAA TGTAAAGCTACTAAAGCTCACTTTGTTGCTGCTGATTATGGTCAC ATGGACATGTTAGACGACGATCTTCCAGGTTTTGTAGGCTTCAT GGCTGGTTGTATGTGTAAAAATGGTCAACGTAAAAAATCAGAAA TGCGTTCTTTTGTAGGTGGTATAGTTGTAGCATTCTTAAAATATT CTTTATGGGGTGAAAAAGCTGAAATAAGATTAATTGTTAAAGAT | Chlorophyllidohydrolase (A. thaliana) |
| | CCTAGTGTATCTCCTGCTAAATTAGACCCATCACCAGAATTAGA AGAAGCATCAGGTATTTTTGTTGGTACCGGTGAAAATCTTTATTT TCAAGGTTCAGGTGGAGGTGGTTCTGATTATAAAGATGATGATG ACAAAGGAACCGGTTAATCTAGACTCGAG | |
| | CATATGGTACCAGCTACACCAGTTGAAGAAGGTGATTATCCAGT TGTAATGTTATTACATGGCTACCTTTTATATAATTCATTTATTCA CAATTAATGTTACATGTATCATCTCACGGTTTCATCTTAATTGCT CCACAATTATACTCAATTGCTGGTCCTGATACTATGGATGAAATT AAAAGTACTGCTGAGATTATGGACTGGTTATCAGTTGGTTTAAA TCACTTTTTACCAGCTCAAGTTACACCTAATTTATCTAAATTTGC ATTATCTGGTCATAGTCGTGGTGGTAAAACTGCTTTTGCTGTAGC ATTAAAAAAATTTGGTTATTCTTCAAACTTAAAAATTAGTACTTT AATTGGTATTGATCCAGTAGACGGAACAGGTAAAGGTAAACAA ACTCCACCTCCTGTTTTAGCATATTTACCTAATAGTTTTGACTTA GACAAAACACCAATTTTAGTAATTGGTTCAGGTTTAGGTGAAAC TGCACGTAATCCTTTATTTCCTCCATGTGCTCCTCCAGGTGTTAA CCACCGTGAGTTTTTCCGTGAATGTCAAGGTCCAGCATGGCACT TTGTTGCTAAAGATTATGGTCATTTAGACATGCTTGATGATGATA CAAAAGGTATTCGTGGCAAATCTAGTTACTGTTTATGCAAAAAT GGTGAAGAACGTCGTCCAATGCGTCGTTTCGTTGGTGGTTTAGTT GTTAGTTTTCTTAAAGCATATCTTGAAGGTGATGATCGTGAATTA GTAAAAATCAAAGATGGTTGTCATGAAGATGTACCTGTTGAAAT TCAAGAATTTGAAGTAATTATGGGTACCGGTGAAAATCTTTACT TTCAAGGTTCAGGCGGTGGAGGTTCAGATTATAAAGATGATGAT GACAAAGGAACCGGTTAATCTAGACTCGAG | Chlorophyllidohydrolase (A. thaliana) |
| | CATATGGTACCAGCTGCTGCTGCACCTGCTGAGACAATGAATAA ATCTGCAGCTGGCGCTGAAGTACCAGAGGCTTTCACATCAGTTT TTCAACCAGGTAAATTAGCAGTTGAAGCAATTCAAGTAGATGAA AATGCAGCTCCTACTCCACCTATTCCTGTTTTAATAGTTGCTCCA AAAGATGCTGGTACATATCCAGTTGCTATGTTATTACACGGATTT TTCTTACATAATCACTTTTATGAACACTTATTACGTCACGTTGCA TCTCATGGCTTTATCATTGTTGCTCCACAATTTTCTATTAGTATTA TTCCATCAGGAGATGCTGAAGACATCGCTGCTGCTGCAAAAGTA GCAGATTGGTTACCTGACGGATTACCAAGTGTTTTACCAAAAGG TGTTGAACCAGAGTTATCAAAACTTGCTTTAGCTGGACACAGTC GTGGTGGTCACACAGCTTTTTCTTTAGCTTTAGGTCACGCTAAAA CACAATTAACTTTCAGTGCATTAATTGGTTTAGATCCTGTTGCTG | Chlorophyllidohydrolase (T. Aestivum) |
| | GAACAGGTAAATCATCTCAATTACAACCAAAAATTCTTACTTAT GAGCCAAGTTCATTTGGTATGGCTATGCCAGTTTTAGTTATTGGT ACAGGTTTAGGAGAAGAAAAAAAAACATTTCTTTCCTCCATG TGCTCCTAAAGACGTAAACCATGCAGAATTTTATCGTGAATGTA GACCACCATGTTACTATTTTGTAACTAAAGATTATGGCCATCTTG ATATGTTAGATGATGACGCTCCAAAATTTATCACATGTGTTTGTA AAGACGGTAATGGATGTAAAGGAAAAATGCGTCGTTGTGTAGCT GGCATCATGGTTGCTTTCTTAAACGCTGCTTTAGGTGAAAAAGA CGCAGATTTAGAAGCTATTTTACGTGATCCAGCAGTTGCTCCTAC AACATTAGACCCAGTTGAACACCGTGTTGCTGGTACCGGTGAGA ATTTATACTTCCAGGGATCTGGTGGTGGTGGCAGTGATTATAAA GATGATGATGATAAAGGAACCGGTTAATCTAGACTCGAG | |
| | CATATGGTACCAAGTCACAAAAAAAAAAACGTAATCTTCTTCGT AACTGATGGTATGGGTCCTGCTTCTCTTTCAATGGCTCGTTCATT TAATCAACACGTTAATGATTTACCAATTGATGATATTTTAACATT AGATGAACATTTTATTGGAAGTTCAAGAACACGTTCATCAGATT CACTTGTAACTGACTCAGCTGCTGGAGCTACAGCTTTTGCTTGTG CACTTAAATCATACAATGGTGCTATAGGTGTAGATCCACACCAT CGTCCATGTGGAACTGTTTTAGAAGCTGCTAAATTAGCAGGTTA TTTAACAGGATTAGTAGTTACTACACGTATTACTGATGCTACACC AGCTAGTTTCTCAAGTCACGTAGATTATCGTTGGCAAGAAGATT TAATTGCAACACACCAATTAGGTGAATATCCTTTAGGACGTGTT GTTGATCTTCTTATGGGTGGTGGTCGTTCTCACTTTTATCCTCAA GGTGAAAAAGCTAGTCCATACGGTCACCACGGTGCACGTAAAG ATGGTCGTGATTTAATCGATGAAGCTCAAAGTAATGGCTGGCAG TATGTAGGAGATCGTAAAAATTTTGATTCTTTACTTAAATCACAT GGTGAAAATGTTACTTTACCATTTTTAGGTTTATTTGCTGACAAC GATATCCCATTTGAAATTGATCGTGATGAAAAAGAATATCCTAG TTTAAAAGAACAAGTAAAAGTAGCATTAGGTGCTTTAGAAAAA GCAAGTAACGAAGATAAAGATAGTAATGGTTTCTTTTTAATGGT AGAAGGTTCTCGTATTGATCATGCTGGCCATCAAAACGATCCTG CATCTCAAGTACGTGAAGTATTAGCATTTGATGAGGCTTTTCAAT ATGTATTAGAATTTGCAGAAAACAGTGATACAGAAACAGTATTA GTAAGTACATCAGATCATGAAACAGGTGGTTTAGTTACTTCAAG ACAAGTAACAGCATCATACCCACAATATGTATGGTATCCTCAAG TATTAGCTAACGCTACACATAGTGGAGAGTTTCTTAAACGTAAA TTAGTTGATTTCGTTCATGAACACAAAGGCGCATCATCAAAAAT | Phosphatase (S. cerevisiae) |
| | AGAAAACTTCATAAAACACGAAATTCTTGAAAAAGATTTAGGTA TTTATGATTATACAGATTCTGACTTAGAAACACTTATTCATTTAG ATGATAACGCTAATGCAATTCAAGATAAACTTAATGATATGGTA AGTTTTAGAGCTCAAATTGGTTGGACAACACATGGTCATTCAGC AGTTGATGTAAACATATATGCTTACGCAAACAAAAAAGCTACAT GGTCTTATGTTCTTAATAACTTACAAGGTAATCACGAAAACACA GAAGTTGGTCAATTCTTAGAGAATTTCTTAGAATTAAACTTAAA TGAAGTTACTGATTTAATCCGTGATACAAAACATACTTCTGATTT TGACGCAACAGAAATAGCAAGTGAGGTTCAACACTATGATGAA TATTACCACGAATTAACAAATGGTACCGGTGAAAATCTTTATTTT CAAGGTTCTGGTGGAGGTGGCAGTGATTATAAAGATGATGATGA CAAAGGAACCGGTTAATCTAGACTCGAG | |
| | CATATGGTACCAGCTTTATACGACATTATTAACTATTTCTACGGT TCAAACTCTAAATTCAACCGTATTACATGGGGTTTAAATCACC AACTTTCATCAAATGGAGAATTACTGATTTCATTTTAATCATCGT TTTAATTGTTCTTTCTTCGTAACTTCTCAAGCAGAGCCATTCCA TCGTCAATTTTATCTTAACGACATGACTATCCAACATCCTTTTGC AGAACATGAACGTGTAACTAATATTCAACTTGGTTTATATTCAA CAGTAATTCCTTTATCAGTTATTATCATTGTTGCTTTAATTAGTA CATGTCCACCTAAATACAAATTATACAACACTTGGGTTTCAAGT ATTGGTTTACTTTTATCAGTTTTAATCACATCTTTTGTTACAAAC ATCGTTAAAAACTGGTTTGGACGTTTACGTCCTGACTTCTTAGAT CGTTGCCAACCAGCTAACGATACACCTAAAGATAAATTAGTTTC TATTGAGGTTTGTACTACAGACAATTTAGACCGTTTAGCTGACG GTTTTCGTACAACACCTTCTGGTCATTCTTCAATCTCATTTGCTG GTTTATTCTATTTAACATTATTTCTTTTAGGTCAATCTCAGGCAA ATAATGGTAAAACATCTTCATGGCGTACAATGATCAGTTTTATA CCTTGGTTAATGGCTTGTTATATCGCTTTAAGTCGTACACAAGAC TACCGTCATCATTTCATTGACGTATTTGTTGGTAGTTGCTTAGGC TTAATTATCGCAATTTGGCAATACTTCCGTTTATTCCCTTGGTTC GGTGGTAACCAAGCAAATGATTCATTTAACAACCGTATTATGAT TGAAGAGATTAAACGTAAAGAGGAAATTAAACAAGATGAAAAT AACTACCGTCGTATTTCTGATATTTCTACTAATGTAGGTACCGGT GAAAACCTTTACTTTCAAGGTTCAGGTGGCGGCGGTTCAGATTA TAAAGATGATGACGACAAAGGAACCGGTTAATCTAGACTCGAG | Phosphatase (C. albicans) |

Technical and scientific terms used herein have the meanings commonly understood by one of ordinary skill in the art to which the instant invention pertains, unless otherwise defined. Reference is made herein to various materials and methodologies known to those of skill in the art. Standard reference works setting forth the general principles of recombinant DNA technology include Sambrook et al., "Molecular Cloning: A Laboratory Manual", 2d ed., Cold Spring Harbor Laboratory Press, Plainview, N.Y., 1989; Kaufman et al., eds., "Handbook of Molecular and Cellular Methods in Biology and Medicine", CRC Press, Boca Raton, 1995; and McPherson, ed., "Directed Mutagenesis: A Practical Approach", IRL Press, Oxford, 1991. Standard reference literature teaching general methodologies and principles of yeast genetics useful for selected aspects of the invention include: Sherman et al. "Laboratory Course Manual Methods in Yeast Genetics", Cold Spring Harbor Laboratory, Cold Spring Harbor, N.Y., 1986 and Guthrie et al., "Guide to Yeast Genetics and Molecular Biology", Academic, New York, 1991.

Where a range of values is provided, it is understood that each intervening value, between the upper and lower limits of that range is also specifically disclosed. Each smaller range between any stated value or intervening value in a stated range and any other stated or intervening value in that stated range is encompassed. The upper and lower limits of these smaller ranges can independently be included or excluded in the range, and each range where either, neither or both limits are included in the smaller ranges is also encompassed, subject to any specifically excluded limit in the stated range. Where the stated range includes one or both of the limits, ranges excluding either or both of those included limits are also included.

The following numbered paragraphs (paras) contain further statements of various aspects of the present invention
1. An isolated vector comprising: (a) a nucleic acid encoding an enzyme that produces an isoprenoid with two phosphates; and (b) a promoter configured for expression of said nucleic acid in a chloroplast of a non-vascular, photosynthetic organism, wherein the vector does not comprise the entire genome of a chloroplast.
2. The vector of para 1, wherein said isoprenoid with two phosphates is GPP, IPP, FPP, GGPP or DMAPP.
3. The vector of para 1, wherein insertion of said vector into a chloroplast genome does not disrupt photosynthetic capability of said chloroplast.
4. The vector of para 1, further comprising a nucleic acid sequence which facilitates homologous recombination with a chloroplast genome.
5. The vector of para 1, further comprising a nucleic acid encoding a second enzyme which modifies the isoprenoid with two phosphates.
6. The vector of para 5, wherein said second enzyme is botyrococcene synthase, limonene synthase, cineole synthase, pinene synthase, camphene synthase, sabinene synthase, myrcene synthase, abietadiene synthase, taxadiene synthase, bisabolene synthase, diapophytoene desaturase, diapophytoene synthase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.
7. The vector of para 1, wherein said vector further comprises a selectable marker.
8. The vector of para 1, wherein the nucleic acid encoding said enzyme is codon biased for said chloroplast.
9. The vector of para 8, wherein said chloroplast is from a microalga.
10. The vector of para 9, wherein said microalga is *C. reinhardtii, D. salina, H. pluvalis, S. dimorphus, D. viridis,* or *D. tertiolecta.*
11. The vector of para 1, comprising any of the sequences in Table 5 or a sequence having at least 70% identity thereto.
12. An isolated vector comprising: (a) a nucleic acid encoding an enzyme that produces an isoprenoid with two phosphates; and (b) a nucleic acid encoding a chloroplast targeting molecule for targeting said enzyme to a chloroplast.
13. The vector of para 12, further comprising a selectable marker, a nucleic acid sequence which facilitates homologous recombination with a chloroplast genome, or a combination thereof.
14. The vector of para 12, wherein said isoprenoid with two phosphates is GPP, IPP, FPP, GGPP or DMAPP.
15. The vector of para 12, further comprising a nucleic acid sequence which facilitates homologous recombination with a nuclear genome.
16. The vector of para 12, further comprising a nucleic acid encoding a second enzyme which modifies the isoprenoid with two phosphates and a nucleic acid encoding a chloroplast targeting molecule for targeting said second enzyme to a chloroplast.
17. The vector of para 16, wherein said second enzyme is botyrococcene synthase, limonene synthase, cineole synthase, pinene synthase, camphene synthase, sabinene synthase, myrcene synthase, abietadiene synthase, taxadiene synthase, bisabolene synthase, diapophytoene desaturase, diapophytoene synthase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.
18. The vector of para 12, wherein the nucleic acid encoding said enzyme is codon biased for said organism.
19. The vector of para 12, wherein said organism is a microalga.
20. The vector of para 19, wherein said microalga is *C. reinhardtii, D. salina, H. pluvalis, S. dimorphus, D. viridis,* or *D. tertiolecta.*
21. The vector of para 12, comprising any of the sequences in Table 5 or a sequence having at least 70% identity thereto.
22. A host cell comprising:
   (a) a vector comprising: (a) a nucleic acid encoding an enzyme that produces an isoprenoid with two phosphates; and (b) a promoter configured for expression of said nucleic acid in a chloroplast of a non-vascular, photosynthetic organism, wherein the vector does not comprise the entire genome of a chloroplast; or
   (b) a vector comprising: (a) a nucleic acid encoding an enzyme that produces an isoprenoid with two phosphates; and (b) a nucleic acid encoding a chloroplast targeting molecule for targeting said enzyme to a chloroplast.
23. The host cell of para 22, wherein said host cell is a homoplasmic for said nucleic acid.
24. The host cell of para 22, wherein said cell is selected from the group consisting of: cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
25. The host cell of para 22, wherein said host cell is *C. reinhardtii, D. salina, H. pluvalis, S. dimorphus, D. viridis,* or *D. tertiolecta.*
26. The host cell of para 22, wherein chlorophyll levels are sufficient for said host cell following transformation to be photoautotrophic.
27. The host cell of para 22, wherein said host cell produces at least one naturally occurring isoprenoid at levels greater than a wild-type strain of the same organism.
28. A host cell comprising at least two copies of a nucleotide sequence of Table 5, or a nucleotide sequence having at least 70% identity thereof.
29. The host cell of para 28, wherein said host cell is a non-vascular photosynthetic organism.
30. The host cell of para 28, wherein said host cell is homoplasmic for said nucleotide sequence.
31. The host cell of para 28, wherein said host cell is *C. reinhardtii, D. salina, H. pluvalis, S. dimorphus, D. viridis,* or *D. tertiolecta.*
32. A genetically modified chloroplast comprising the vector of para 1.
33. A non-vascular, photosynthetic organism comprising the chloroplast of para 32.
34. A method of producing an isoprenoid comprising: (a) transforming a chloroplast of a non-vascular, photosynthetic organism with a nucleic acid encoding an enzyme that produces an isoprenoid with two phosphates, and; (b) collecting at least one isoprenoid produced by said transformed organism.
35. The method of para 34, further comprising growing said organism in an aqueous environment, wherein CO₂ is supplied to said organism.
36. The method of para 35, wherein said CO₂ is at least partially derived from a burned fossil fuel.
37. The method of para 35, wherein said CO₂ is at least partially derived from flue gas.
38. The method of para 34, wherein said isoprenoid with two phosphates is GPP, IPP, FPP, GGPP or DMAPP.
39. The method of para 34, wherein said collecting step comprises one or more of the following steps: (a) harvesting said transformed organism; (b) harvesting said isoprenoid from a cell medium; (c) mechanically disrupting said organism; or (d) chemically disrupting said organism.
40. The method of para 34, wherein said organism is a microalga.
41. The method of para 37, wherein said microalga is *C. reinhardtii, D. salina, H. pluvalis, S. dimorphus, D. viridis,* or *D. tertiolecta.*
42. A method for producing an isoprenoid comprising: (a) transforming the chloroplast of a non-vascular, photosynthetic organism to produce said isoprenoid, wherein said organism is not transformed with isoprene synthase or a methyl-butenol synthase; and (b) collecting said isoprenoid.
43. The method of para 42, further comprising growing said organism in an aqueous environment, wherein CO₂ is supplied to said organism.
44. The method of para 43, wherein said CO₂ is at least partially derived from a burned fossil fuel.
45. The method of para 43, wherein said CO₂ is at least partially derived from flue gas.
46. The method of para 42, wherein isoprenoid is GPP, IPP, FPP, GGPP or DMAPP.
47. The method of para 42, wherein said chloroplast is transformed with a nucleic acid encoding botyrococcene synthase, limonene synthase, cineole synthase, pinene synthase, camphene synthase, sabinene synthase, myrcene synthase, abietadiene synthase, taxadiene synthase, bisabolene synthase, diapophytoene desaturase, diapophytoene synthase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, 8-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.
48. The method of para 42, wherein said collecting step comprises one or more of the following steps: (a) harvesting said transformed organism; (b) harvesting said isoprenoid from a cell medium; (d) mechanically disrupting said organism; or (e) chemically disrupting said organism.
49. The method of para 42, wherein said organism is a microalga.
50. The method of para 49, wherein said microalga is *C. reinhardtii, D. salina, H. pluvalis, S. dimorphus, D. viridis,* or *D. tertiolecta.*
51. An organism comprising a genetically modified chloroplast, wherein said chloroplast comprises a nucleic acid encoding an isoprenoid producing enzyme and wherein said organism can grow in a high saline environment.
52. The organism of para 51, wherein said organism is a non-vascular, photosynthetic organism.
53. The organism of para 51, wherein said organism is *D. salina.*
54. The organism of para 51, wherein said high saline environment comprises 0.5-4.0 molar sodium chloride.
55. A method for preparing an isoprenoid comprising: (a) transforming an organism with a nucleic acid to increase or initiate production of said isoprenoid, wherein said organism is grown in a high-saline environment; and (b) collecting said isoprenoid.
56. The method of para 55, wherein said organism is a non-vascular photosynthetic organism.
57. The method of para 55, wherein said organism is *D. salina, D. viridis,* or *D. tertiolecta.*
58. The method of para 55, wherein said transforming step is a chloroplast transformation.
59. The method of para 55, wherein said collecting step comprises one or more of the following steps: (a) harvesting said transformed organism; (b) harvesting said isoprenoid from a cell medium; (c) mechanically disrupting said organism; or (d) chemically disrupting said organism.
60. The method of para 55, wherein said high-saline environment comprises 0.5-4.0 molar sodium chloride.
61. A vector comprising: a heterologous nucleic acid encoding one or more isoprenoid producing enzymes; and a promoter configured for expression of said nucleic acid in a photosynthetic bacteria.
62. The vector of para 61, wherein said photosynthetic bacteria is a cyanobacterial species.
63. The vector of para 61, wherein said cyanobacterial species is a member of a genus selected from the group consisting of *Synechocystis, Synechococcus,* or *Athrospira.*
64. A host cell comprising the vector of para 61.
65. The host cell of para 64, wherein said host cell is a cyanobacterial species.
66. The host cell of para 66, wherein said host cell is a member of a genus selected from the group consisting of: *Synechocystis, Synechococcus,* and *Athrospira.*
67. A host cell comprising: a first nucleic acid encoding a botryococcene synthase, and a second nucleic acid encoding an FPP synthase, wherein said host cell is a non-vascular photosynthetic organism.
68. The host cell of para 67, wherein said first and second nucleic acids are integrated into a chloroplast genome.
69. The host cell of para 67, wherein said host cell is a microalga.
70. The host cell of para 69, wherein said microalga is *C. reinhardtii, D. salina, H. pluvalis, S. dimorphus, D. viridis,* or *D. tertiolecta.*
71. A vector comprising: (a) a first nucleic acid encoding a protein; (b) a second nucleic acid encoding a selectable marker, wherein said first and second nucleic acids comprise one open reading frame; and (c) a promoter configured for expression of said first and second nucleic acids in a non-vascular, photosynthetic organism.
72. The vector of para 71, wherein said protein is an isoprenoid producing enzyme or a biomass degrading enzyme.
73. The vector of para 72, wherein said isoprenoid producing enzyme is botyrococcene synthase, limonene synthase, cineole synthase, pinene synthase, camphene synthase, sabinene synthase, myrcene synthase, abietadiene synthase, taxadiene synthase, FPP synthase, bisabolene synthase, diapophytoene desaturase, diapophytoene synthase, GPP synthase, IPP isomerase, monoterpene synthase, terpinolene synthase, zingiberene synthase, ocimene synthase, sesquiterpene synthase, curcumene synthase, farnesene synthase, geranylgeranyl reductase, chlorophyllidohydrolase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β- phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, gamma-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.
74. The vector of para 72, wherein said biomass degrading enzyme is exo-β-glucanase, endo-β-glucanase, β-glucosidase, endoxylanase, or ligninase.
75. The vector of para 71, wherein said first or second nucleic acid comprises at least one codon optimized for expression in the nucleus of a non-vascular, photosynthetic organism.
76. The vector of para 71, further comprising a third nucleic acid encoding a cleavage moiety in-frame with said first and second nucleic acids.
77. The vector of para 76, wherein said cleavage moiety is a self-cleaving protease.
78. The vector of para 77, wherein said protease is a functional portion of the A2 region from foot and mouth disease virus.
79. The vector of para 76, wherein said cleavage moiety is capable of being cleaved by a protease naturally produced by said organism.
80. The vector of para 71, wherein said promoter comprises an HSP70, a functional portion of HSP70, rbcS2 5' upstream translated region (UTR), a functional portion of rbcS2 5' UTR, or a combination thereof.
81. The vector of para 80, wherein said promoter is derived from said organism.
82. The vector of paras 71 or 76, further comprising a fourth nucleic acid encoding a secretion signal.
83. The vector of para 82, wherein said first, second, and fourth nucleic acids are in-frame.
84. The vector of para 82, wherein said first, second, third and fourth nucleic acids are in-frame.
85. The vector of para 82, wherein said secretion signal is a *C. reinhardtii* carbonic anhydrase secretion signal.
86. The vector of para 71, wherein said organism is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
87. The vector of para 71, wherein said vector is capable of stable transformation in *C. reinhardtii, D. salina, H. pluvalis, S. dimorphus, D. viridis,* or *D. tertiolecta.*
88. The vector of para 71, wherein said vector is capable of stable transformation in a bacterium of the genus *Synechocystis,* the genus *Synechococcus,* or the genus *Athrospira.*
89. The vector of para 71, comprising any of the sequences in Table 7 or a sequence having at least 70% identity thereto.
90. The vector of para 71, 76, or 82 further comprising a fifth nucleic acid in-frame with said first, second, third and/encoding a tag.
91. The vector of para 90, wherein said tag is an epitope tag or a metal affinity tag.
92. A host cell comprising a vector, wherein said vector comprises
   a first nucleic acid encoding a protein,
   a second nucleic acid encoding a selectable marker, wherein said first and second nucleic acids comprise one open reading frame,
   a promoter configured for expression of said first and second nucleic acids in a non-vascular, photosynthetic organism and
   optionally one or more additional nucleic acids encoding a cleavage moiety, a secretion signal, a tag or a combination thereof, wherein said one or more additional nucleic acids are in-frame with said first and second nucleic acids.
93. The host cell of para 92, wherein said cell is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
94. The host cell of para 92, wherein said host cell is *C. reinhardtii, D. salina, H. pluvalis, S. dimorphus, D. viridis,* or *D. tertiolecta.*
95. The host cell of para 92, wherein said host cell is a bacterium of the genus *Synechocystis,* the genus *Synechococcus,* or the genus *A throspira.*
96. The host cell of para 92, wherein said vector is stably integrated into the nuclear genome of said host cell.
97. A method of producing a protein in a non-vascular photosynthetic organism, comprising: growing a non-vascular photosynthetic organism, wherein said organism comprises an exogenous nucleic acid comprising a single open reading frame, wherein said open reading frame comprises a first nucleic acid encoding a protein and a second nucleic acid encoding a selectable marker, and wherein said organism further comprises a promoter configured for expression of said open reading frame in said organism, thereby producing said protein.
98. The method of para 97, wherein said protein is an isoprenoid producing enzyme or a biomass degrading enzyme.
99. The method of para 97, wherein said open reading frame comprises at least one codon optimized for expression in the nucleus of a non-vascular, photosynthetic organism.
100. The method of para 97, wherein said open reading frame further comprises a third nucleic acid encoding a cleavage moiety in-frame with said first and second nucleic acids.
101. The method of para 100, wherein said cleavage moiety is a self-cleaving protease.
102. The method of para 101, wherein said protease is a functional portion of the A2 region from foot and mouth disease virus.
103. The method of para 100, wherein said cleavage moiety is capable of being cleaved by a protease naturally produced by said organism.
104. The method of paras 97 or 100, further comprising a fourth nucleic acid encoding a secretion signal.
105. The method of para 104, wherein said fourth nucleic acid is in-frame with all other nucleic acids comprising said open reading frame.
106. The method of para 104, wherein said secretion signal is a *C. reinhardtii* carbonic anhydrase secretion signal.
107. The method of para 97, wherein said organism is *C. reinhardtii, D. salina, H. pluvalis, S. dimorphus, D. viridis, D. tertiolecta,* a bacterium of the genus *Synechocystis,* the genus *Synechococcus,* or the genus *Athrospira.*
108. The method of para 97, 100, or 104, wherein said open reading frame further comprises a fifth nucleic acid encoding a tag, wherein said fifth nucleic acid is in-frame with all other nucleic acids comprising said open reading frame.
109. The method of para 108, wherein said tag is an epitope tag or a metal affinity tag.
110. A host cell comprising a fusion protein, wherein said fusion protein comprises a first nucleic acid encoding a protein and a second nucleic acid encoding a selectable marker and wherein said host cell is a non-vascular photosynthetic organism.
111. The host cell of para 110, wherein said host cell is *C. reinhardtii, D. salina, H. pluvalis, S. dimorphus, D. viridis,* or *D. tertiolecta.*
112. The host cell of para 110, wherein said host cell is a bacterium of the genus *Synechocystis,* the genus *Synechococcus,* or the genus *A throspira.*
113. The host cell of para 110, wherein said vector is stably integrated into a nuclear genome of said host cell.
114. The host cell of para 110, wherein said fusion protein further comprises a cleavage moiety, a secretion signal, a tag, or a combination thereof.
115. The host cell of para 110, wherein said cleavage moiety is a self-cleaving protease.
116. The host cell of para 115, wherein said protease is a functional portion of the A2 region from foot and mouth disease virus.
117. The host cell of para 110, wherein said cleavage moiety is capable of being cleaved by a protease naturally produced by said organism.
118. The host cell of para 110, wherein said secretion signal is a *C. reinhardtii* carbonic anhydrase secretion signal.
119. The host cell of para 110, wherein said tag is an epitope tag or a metal affinity tag.
120. A method of producing a transgenic non-vascular photosynthetic organism expressing a protein of interest under selective conditions, comprising:
   transforming a non-vascular photosynthetic organism with a nucleic acid comprising a single open reading frame, wherein said open reading frame encodes a fusion protein comprising said protein of interest and a selectable marker;
   wherein said organism is capable of expressing said selectable marker under environmental conditions which require expression of the selectable marker for continued viability of said organism, thereby resulting in expression of said protein of interest.
121. The method of para 120, wherein said protein is an isoprenoid producing enzyme or a biomass degrading enzyme.
122. The method of para 120, wherein said open reading frame further comprises one or more nucleic acids encoding a cleavage moiety, a secretion signal, a tag, or a combination thereof.
123. The method of para 122, wherein said cleavage moiety is a self-cleaving protease.
124. The method of para 123, wherein said protease is a functional portion of the A2 region from foot and mouth disease virus.
125. The method of para 122, wherein said cleavage moiety is capable of being cleaved by a protease naturally produced by said organism.
126. The method of para 122, wherein said secretion signal is a *C. reinhardtii* carbonic anhydrase secretion signal.
127. The method of para 122, wherein said tag is an epitope tag or a metal affinity tag.
128. A method of increasing phytol production in a non-vascular photosynthetic organism, comprising:
   transforming said organism with a nucleic acid which results in an increase in production of phytol by said organism above a level produced by the host cell not containing said nucleic acid.
129. The method of para 128, wherein said nucleic acid encodes an enzyme selected from the group consisting of a GPP synthase, a FPP synthase, a geranylgeranyl reductase, a chlorophyllidohydrolase, and a pyrophosphatase.
130. The method of para 128, wherein said transformation is a chloroplast transformation.
131. The method of para 129, wherein said enzyme is endogenous to said organism or is homologous to an endogenous enzyme of said organism.
132. The method of para 131 wherein said enzyme is overexpressed.
133. The method of para 129, wherein said enzyme is exogenous to said organism.
134. The method of para 129, wherein expression of said enzyme is regulated by an inducible promoter.
135. The method of para 128, further comprising transformation of said organism with a nucleic acid which results in production of dimethylallyl alcohol, isopentyl alcohol, geraniol, farnesol or geranylgeraniol.
136. The method of para 128, wherein said organism is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
137. The method of para 128, wherein said organism is *C. reinhardtii, D. salina, H. pluvalis, S. dimorphus, D. viridis,* or *D. tertiolecta.*
138. The method of para 128, wherein said nucleic acid comprises a sequence from Table 7 or a sequence with 70% homology thereto.
139. A host cell comprising an introduced nucleic acid, wherein said nucleic acid results in an increase in production of phytol by said host cell above a level produced by a host cell not containing said nucleic acid, wherein said host cell is a non-vascular photosynthetic organism.
140. The host cell of para 139, wherein said host cell can grow in a high-saline environment.
141. The host cell of para 140, wherein said host cell is *D. salina, D. viridis,* or *D. tertiolecta.*
142. The host cell of para 140, wherein said high-saline environment comprises 0.5-4.0 molar sodium chloride.
143. The host cell of para 139, wherein said nucleic acid is present in a chloroplast.
144. The host cell of para 139, wherein said nucleic acid encodes an enzyme selected from the group consisting of a GPP synthase, a FPP synthase, a geranylgeranyl reductase, a chlorophyllidohydrolase, and a pyrophosphatase.
145. The host cell of para 139, further comprising a nucleic acid which results in production of dimethylallyl alcohol, isopentyl alcohol, geraniol, farnesol or geranylgeraniol.
146. The host cell of para 139, wherein said cell is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
147. The host cell of para 139, wherein said cell is *C. reinhardtii, D. salina, H. pluvalis, S. dimorphus, D. viridis,* or *D. tertiolecta.*
148. A method of producing phytol in a non-vascular photosynthetic organism, comprising:
   transforming said organism with a nucleic acid which results in an increase in production of phytol by said organism above a level produced by said organism not containing said nucleic acid; and
   collecting said phytol from said organism.
149. The method of para 148, wherein said nucleic acid encodes an enzyme selected from the group consisting of a GPP synthase, a FPP synthase, a geranylgeranyl reductase, a chlorophyllidohydrolase, and a pyrophosphatase.
150. The method of para 148, wherein said transformation is a chloroplast transformation.
151. The method of para 148, wherein said nucleic acid encodes an enzyme endogenous to said organism or an enzyme homologous to an endogenous enzyme of said organism.
152. The method of para 151, wherein said enzyme is overexpressed.
153. The method of claim 148, wherein said nucleic acid encodes an enzyme exogenous to said organism.
154. The method of para 148, wherein expression of said nucleic acid is regulated by an inducible promoter.
155. The method of para 148, further comprising transformation of said organism with a nucleic acid which results in production of dimethylallyl alcohol, isopentyl alcohol, geraniol, farnesol or geranylgeraniol.
156. The method of para 148, wherein said organism is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
157. The method of para 148, wherein said organism is *C. reinhardtii, D. salina* or *H. pluvalis.*
158. A composition comprising at least 3% phytol and at least a trace amount of a cellular portion of a genetically modified non-vascular photosynthetic organism.
159. The composition of para 158, wherein said genetically modified organism is modified by an endogenous, heterologous, or exogenous GPP synthase, FPP synthase, geranylgeranyl reductase, chlorophyllidohydrolase, or pyrophosphatase.
160. The composition of para 158, wherein a chloroplast of said organism is genetically modified.
161. The composition of para 158, further comprising dimethylallyl alcohol, isopentyl alcohol, geraniol, farnesol or geranylgeraniol.
162. The composition of para 158, wherein said organism is selected from the group consisting of: photosynthetic bacteria, cyanobacteria, cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton.
163. The composition of para 158, wherein said organism is *C. reinhardtii, D. salina, H. pluvalis, S. dimorphus, D. viridis,* or *D. tertiolecta.*

## Claims

1. A genetically modified chloroplast of a non-vascular, photosynthetic organism comprising an exogenous nucleic acid molecule, said exogenous nucleic acid molecule comprising:
(a) a first nucleic acid encoding one or more polypeptides of the mevalonate (MVA) pathway or non-mevalonate (MEP) pathway selected from the group consisting of thiolase, HMG-CoA synthase, HMG-CoA reductase, mevalonate kinase, phosphemevalonate kinase, mevalonate-5-pyrophosphate decarboxylase, DOXP synthase, DOXP reductase, 4-diphosphocytidyl-2-C-methyl-D-erythritol synthase, 4-diphophocytidyl-2-C-methyl-D-erythritol kinase, 2-C-methyl-D-erythritol 2,4,-cyclodiphosphate synthase, HMB-PP synthase, HMB-PP reductase, and DOXP reductoisomerase;
(b) a second nucleic acid encoding one or more enzymes selected from the group consisting of limonene synthase, cineole synthase, pinene synthase, camphene synthase, sabinene synthase, myrcene synthase, abietadiene synthase, taxadiene synthase, bisabolene synthase, monoterpene synthase, terpinolene synthase, zingiberene synthase, ocimene synthase, sesquiterpene synthase, curcumene synthase, farnesene synthase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolochene synthase, α-humulene synthase, (E,E)-α-farnesene synthase, (-)-β-pinene synthase, γ-terpinene synthase, limonene cyclase, linalool synthase, levopimaradiene synthase, isopimaradiene synthase, (E)-γ-bisabolene synthase, kaurene synthase, longifolene synthase, γ-humulene synthase, δ-selinene synthase, β- phellandrene synthase, (+)-3-carene synthase, α-terpineol synthase, syn-pimara-7,15-diene synthase, ent-sandaaracopimaradiene synthase, sterner- 13-ene synthase, S-linalool synthase, geraniol synthase, gamma-terpinene synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, cedrol synthase, copalol synthase, and manool synthase; and
(c) a promoter configured for expression of said first and second nucleic acid in a chloroplast of said non-vascular, photosynthetic organism, wherein said exogenous nucleic acid molecule does not comprise the entire genome of a chloroplast; and
wherein said first nucleic acid sequence and said second nucleic acid sequence are expressed.

2. A non-vascular, photosynthetic organism comprising the genetically modified chloroplast of claim 1.

3. The genetically modified chloroplast of claim 1, wherein said first or second nucleic acid, or combination thereof, is integrated into the genome of said genetically modified chloroplast.

4. The genetically modified chloroplast of claim 1, wherein said second nucleic acid encoding one or more enzymes is at least one of SEQ ID NO: 74, 75, 76, 77, 78, 79, 80, 81, 83, 84, 85, 86, 97, 98, 99, 100, 101, 102, 103, 104, 105, 106, 107, 108, 109, 110, 111, 112, 113, 114, 115, 116, 117, 120, 121, 122, 123, 124 or a nucleic acid having at least 70% sequence identity to SEQ ID NO: 74, 75, 76, 77, 78, 79, 80, 81, 83, 84, 85, 86, 97, 98, 99, 100, 101, 102, 103, 104, 105, 106, 107, 108, 109, 110, 111, 112, 113, 114, 115, 116, 117, 120, 121, 122, 123 or 124.

5. The genetically modified chloroplast of claim 1, wherein said non-vascular, photosynthetic organism is a microalga.

6. The genetically modified chloroplast of claim 5, wherein said microalga is *Chlamydomonas reinhardtii, Dunaliella salina, Haematococcus pluvalis, Scenedesmus dimorphus, Dunaliella viridis,* or *Dunaliella tertiolecta.*

7. The genetically modified chloroplast of claim 1, wherein said non-vascular, photosynthetic organism is a cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, or phytoplankton.

8. The genetically modified chloroplast of claim 1, wherein said non-vascular, photosynthetic organism is a bacterium of the genus *Synechocystis,* the genus *Synechococcus,* or the genus *Athrospira.*

9. The genetically modified chloroplast of claim 1, wherein said non-vascular, photosynthetic organism is *Dunaliella salina.*

10. The genetically modified chloroplast of claim 1, wherein said one or more enzymes are selected from the group consisting of farnesene synthase, (E)-β-farnesene synthase, (E,E)-α-farnesene synthase, and kaurene synthase.

11. The genetically modified chloroplast of claim 1, wherein said second nucleic acid comprises a nucleotide sequence having at least 90% sequence identity to a sequence selected from the group consisting of SEQ ID NOs: 111, 112, 113, and 114.

12. The genetically modified chloroplast of claim 1, wherein said second nucleic acid has an amino acid sequence having at least 90% sequence identity to a sequence selected from the group consisting of SEQ ID NOs: 39 to 42.

13. A method of producing a genetically modified chloroplast of a non-vascular, photosynthetic organism comprising transforming said non-vascular, photosynthetic organism with a vector comprising:
(a) a first nucleic acid encoding one or more polypeptides of the mevalonate (MVA) pathway or non-mevalonate (MEP) pathway selected from the group consisting of thiolase, HMG-CoA synthase, HMG-CoA reductase, mevalonate kinase, phosphemevalonate kinase, mevalonate-5-pyrophosphate decarboxylase, DOXP synthase, DOXP reductase, 4-diphosphocytidyl-2-C-methyl-D-erythritol synthase, 4-diphophocytidyl-2-C-methyl-D-erythritol kinase, 2-C-methyl-D-erythritol 2,4,-cyclodiphosphate synthase, HMB-PP synthase, HMB-PP reductase, and DOXP reductoisomerase;
(b) a second nucleic acid encoding one or more enzymes selected from the group consisting of limonene synthase, cineole synthase, pinene synthase, camphene synthase, sabinene synthase, myrcene synthase, abietadiene synthase, taxadiene synthase, bisabolene synthase, monoterpene synthase, terpinolene synthase, zingiberene synthase, ocimene synthase, sesquiterpene synthase, curcumene synthase, farnesene synthase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolochene synthase, α-humulene synthase, (E,E)-α-farnesene synthase, (-)-β-pinene synthase, γ-terpinene synthase, limonene cyclase, linalool synthase, levopimaradiene synthase, isopimaradiene synthase, (E)-γ-bisabolene synthase, kaurene synthase, longifolene synthase, γ-humulene synthase, δ-selinene synthase, β- phellandrene synthase, (+)-3-carene synthase, α-terpineol synthase, syn-pimara-7,15-diene synthase, ent-sandaaracopimaradiene synthase, sterner- 13-ene synthase, S-linalool synthase, geraniol synthase, gamma-terpinene synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, cedrol synthase, copalol synthase, and manool synthase; and
(c) a promoter configured for expression of said first and second nucleic acid in a chloroplast of a non-vascular, photosynthetic organism, wherein said vector does not comprise the entire genome of a chloroplast.

14. The method of claim 13, wherein said first nucleic acid sequence and said second nucleic acid sequence are expressed.


SEQUENCE LISTING

<110> SAPPHIRE ENERGY, Inc. AND THE SCRIPPS RESEARCH INSTITUTE

<120> MOLECULE PRODUCTION BY PHOTOSYNTHETIC ORGANISMS

<130> SJK/FP6948970

<140> EP Divisional of 08799404.2
<141> 2008-09-10

<150> EP 08799404.2
<151> 2008-09-10

<150> 61/130,892
<151> 2008-06-02

<150> 60/973,924
<151> 2007-09-20

<150> 60/971,418
<151> 2007-09-11

<150> 60/971,412
<151> 2007-09-11

<160> 209

<170> PatentIn version 3.5

<210> 1
<211> 572
<212> PRT
<213> Artificial Sequence

<220>
<223> Description of Artificial Sequence: Synthetic
      polypeptide

<400> 1
Met Val Pro Arg Arg Ser Gly Asn Tyr Asn Pro Ser Arg Trp Asp Val
1               5                   10                  15

Asn Phe Ile Gln Ser Leu Leu Ser Asp Tyr Lys Glu Asp Lys His Val
            20                  25                  30

Ile Arg Ala Ser Glu Leu Val Thr Leu Val Lys Met Glu Leu Glu Lys
        35                  40                  45

Glu Thr Asp Gln Ile Arg Gln Leu Glu Leu Ile Asp Asp Leu Gln Arg
    50                  55                  60

Met Gly Leu Ser Asp His Phe Gln Asn Glu Phe Lys Glu Ile Leu Ser
65                  70                  75                  80

Ser Ile Tyr Leu Asp His His Tyr Tyr Lys Asn Pro Phe Pro Lys Glu
                85                  90                  95

Glu Arg Asp Leu Tyr Ser Thr Ser Leu Ala Phe Arg Leu Leu Arg Glu
        100                 105                 110

His Gly Phe Gln Val Ala Gln Glu Val Phe Asp Ser Phe Lys Asn Glu
    115                 120                 125

Glu Gly Glu Phe Lys Glu Ser Leu Ser Asp Asp Thr Arg Gly Leu Leu
    130                 135                 140

Gln Leu Tyr Glu Ala Ser Phe Leu Leu Thr Glu Gly Glu Thr Thr Leu
145                 150                 155                 160

Glu Ser Ala Arg Glu Phe Ala Thr Lys Phe Leu Glu Glu Lys Val Asn
                165                 170                 175

Glu Gly Gly Val Asp Gly Asp Leu Leu Thr Arg Ile Ala Tyr Ser Leu
        180                 185                 190

Asp Ile Pro Leu His Trp Arg Ile Lys Arg Pro Asn Ala Pro Val Trp
    195                 200                 205

Ile Glu Trp Tyr Arg Lys Arg Pro Asp Met Asn Pro Val Val Leu Glu
    210                 215                 220

Leu Ala Ile Leu Asp Leu Asn Ile Val Gln Ala Gln Phe Gln Glu Glu
225                 230                 235                 240

Leu Lys Glu Ser Phe Arg Trp Trp Arg Asn Thr Gly Phe Val Glu Lys
                245                 250                 255

Leu Pro Phe Ala Arg Asp Arg Leu Val Glu Cys Tyr Phe Trp Asn Thr
        260                 265                 270

Gly Ile Ile Glu Pro Arg Gln His Ala Ser Ala Arg Ile Met Met Gly
    275                 280                 285

Lys Val Asn Ala Leu Ile Thr Val Ile Asp Asp Ile Tyr Asp Val Tyr
    290                 295                 300

Gly Thr Leu Glu Glu Leu Glu Gln Phe Thr Asp Leu Ile Arg Arg Trp
305                 310                 315                 320

Asp Ile Asn Ser Ile Asp Gln Leu Pro Asp Tyr Met Gln Leu Cys Phe
                325                 330                 335

Leu Ala Leu Asn Asn Phe Val Asp Asp Thr Ser Tyr Asp Val Met Lys
        340                 345                 350

Glu Lys Gly Val Asn Val Ile Pro Tyr Leu Arg Gln Ser Trp Val Asp
        355                 360                 365

Leu Ala Asp Lys Tyr Met Val Glu Ala Arg Trp Phe Tyr Gly Gly His
    370                 375                 380

Lys Pro Ser Leu Glu Glu Tyr Leu Glu Asn Ser Trp Gln Ser Ile Ser
385                 390                 395                 400

Gly Pro Cys Met Leu Thr His Ile Phe Phe Arg Val Thr Asp Ser Phe
                405                 410                 415

Thr Lys Glu Thr Val Asp Ser Leu Tyr Lys Tyr His Asp Leu Val Arg
            420                 425                 430

Trp Ser Ser Phe Val Leu Arg Leu Ala Asp Asp Leu Gly Thr Ser Val
        435                 440                 445

Glu Glu Val Ser Arg Gly Asp Val Pro Lys Ser Leu Gln Cys Tyr Met
    450                 455                 460

Ser Asp Tyr Asn Ala Ser Glu Ala Glu Ala Arg Lys His Val Lys Trp
465                 470                 475                 480

Leu Ile Ala Glu Val Trp Lys Lys Met Asn Ala Glu Arg Val Ser Lys
                485                 490                 495

Asp Ser Pro Phe Gly Lys Asp Phe Ile Gly Cys Ala Val Asp Leu Gly
            500                 505                 510

Arg Met Ala Gln Leu Met Tyr His Asn Gly Asp Gly His Gly Thr Gln
        515                 520                 525

His Pro Ile Ile His Gln Gln Met Thr Arg Thr Leu Phe Glu Pro Phe
    530                 535                 540

Ala Gly Thr Gly Glu Asn Leu Tyr Phe Gln Gly Ser Gly Gly Gly Gly
545                 550                 555                 560

Ser Asp Tyr Lys Asp Asp Asp Asp Lys Gly Thr Gly
                565                 570

<210> 2
<211> 565
<212> PRT
<213> Artificial Sequence

<220>
<223> Description of Artificial Sequence: Synthetic
      polypeptide

<400> 2
Met Val Pro Arg Arg Thr Gly Gly Tyr Gln Pro Thr Leu Trp Asp Phe
1               5                   10                  15

Ser Thr Ile Gln Leu Phe Asp Ser Glu Tyr Lys Glu Glu Lys His Leu
            20                  25                  30

Met Arg Ala Ala Gly Met Ile Ala Gln Val Asn Met Leu Leu Gln Glu
        35                  40                  45

Glu Val Asp Ser Ile Gln Arg Leu Glu Leu Ile Asp Asp Leu Arg Arg
    50                  55                  60

Leu Gly Ile Ser Cys His Phe Asp Arg Glu Ile Val Glu Ile Leu Asn
65                  70                  75                  80

Ser Lys Tyr Tyr Thr Asn Asn Glu Ile Asp Glu Ser Asp Leu Tyr Ser
                85                  90                  95

Thr Ala Leu Arg Phe Lys Leu Leu Arg Gln Tyr Asp Phe Ser Val Ser
            100                 105                 110

Gln Glu Val Phe Asp Cys Phe Lys Asn Asp Lys Gly Thr Asp Phe Lys
        115                 120                 125

Pro Ser Leu Val Asp Asp Thr Arg Gly Leu Leu Gln Leu Tyr Glu Ala
    130                 135                 140

Ser Phe Leu Ser Ala Gln Gly Glu Glu Thr Leu His Leu Ala Arg Asp
145                 150                 155                 160

Phe Ala Thr Lys Phe Leu His Lys Arg Val Leu Val Asp Lys Asp Ile
                165                 170                 175

Asn Leu Leu Ser Ser Ile Glu Arg Ala Leu Glu Leu Pro Thr His Trp
            180                 185                 190

Arg Val Gln Met Pro Asn Ala Arg Ser Phe Ile Asp Ala Tyr Lys Arg
        195                 200                 205

Arg Pro Asp Met Asn Pro Thr Val Leu Glu Leu Ala Lys Leu Asp Phe
    210                 215                 220

Asn Met Val Gln Ala Gln Phe Gln Gln Glu Leu Lys Glu Ala Ser Arg
225                 230                 235                 240

Trp Trp Asn Ser Thr Gly Leu Val His Glu Leu Pro Phe Val Arg Asp
                245                 250                 255

Arg Ile Val Glu Cys Tyr Tyr Trp Thr Thr Gly Val Val Glu Arg Arg
            260                 265                 270

Glu His Gly Tyr Glu Arg Ile Met Leu Thr Lys Ile Asn Ala Leu Val
        275                 280                 285

Thr Thr Ile Asp Asp Val Phe Asp Ile Tyr Gly Thr Leu Glu Glu Leu
    290                 295                 300

Gln Leu Phe Thr Thr Ala Ile Gln Arg Trp Asp Ile Glu Ser Met Lys
305                 310                 315                 320

Gln Leu Pro Pro Tyr Met Gln Ile Cys Tyr Leu Ala Leu Phe Asn Phe
                325                 330                 335

Val Asn Glu Met Ala Tyr Asp Thr Leu Arg Asp Lys Gly Phe Asn Ser
            340                 345                 350

Thr Pro Tyr Leu Arg Lys Ala Trp Val Asp Leu Val Glu Ser Tyr Leu
        355                 360                 365

Ile Glu Ala Lys Trp Tyr Tyr Met Gly His Lys Pro Ser Leu Glu Glu
    370                 375                 380

Tyr Met Lys Asn Ser Trp Ile Ser Ile Gly Gly Ile Pro Ile Leu Ser
385                 390                 395                 400

His Leu Phe Phe Arg Leu Thr Asp Ser Ile Glu Glu Glu Asp Ala Glu
                405                 410                 415

Ser Met His Lys Tyr His Asp Ile Val Arg Ala Ser Cys Thr Ile Leu
            420                 425                 430

Arg Leu Ala Asp Asp Met Gly Thr Ser Leu Asp Glu Val Glu Arg Gly
        435                 440                 445

Asp Val Pro Lys Ser Val Gln Cys Tyr Met Asn Glu Lys Asn Ala Ser
    450                 455                 460

Glu Glu Glu Ala Arg Glu His Val Arg Ser Leu Ile Asp Gln Thr Trp
465                 470                 475                 480

Lys Met Met Asn Lys Glu Met Met Thr Ser Ser Phe Ser Lys Tyr Phe

485                 490                 495

Val Gln Val Ser Ala Asn Leu Ala Arg Met Ala Gln Trp Ile Tyr Gln
            500                 505                 510

His Glu Ser Asp Gly Phe Gly Met Gln His Ser Leu Val Asn Lys Met
        515                 520                 525

Leu Arg Gly Leu Leu Phe Asp Arg Tyr Glu Gly Thr Gly Glu Asn Leu
    530                 535                 540

Tyr Phe Gln Gly Ser Gly Gly Gly Gly Ser Asp Tyr Lys Asp Asp Asp
545                 550                 555                 560

Asp Lys Gly Thr Gly
                565

<210> 3
<211> 595
<212> PRT
<213> Artificial Sequence

<220>
<223> Description of Artificial Sequence: Synthetic
      polypeptide

<400> 3
Met Val Pro Arg Arg Met Gly Asp Phe His Ser Asn Leu Trp Asp Asp
1               5                   10                  15

Asp Val Ile Gln Ser Leu Pro Thr Ala Tyr Glu Glu Lys Ser Tyr Leu
            20                  25                  30

Glu Arg Ala Glu Lys Leu Ile Gly Glu Val Glu Asn Met Phe Asn Ser
        35                  40                  45

Met Ser Leu Glu Asp Gly Glu Leu Met Ser Pro Leu Asn Asp Leu Ile
    50                  55                  60

Gln Arg Leu Trp Ile Val Asp Ser Leu Gly Arg Leu Gly Ile His Arg
65                  70                  75                  80

His Phe Lys Asp Glu Ile Lys Ser Ala Leu Asp Tyr Val Tyr Ser Tyr
                85                  90                  95

Trp Gly Glu Asn Gly Ile Gly Cys Gly Arg Glu Ser Ala Val Thr Asp
            100                 105                 110

Leu Asn Ser Thr Ala Leu Gly Phe Arg Thr Leu Arg Leu His Gly Tyr
        115                 120                 125

Pro Val Ser Ser Asp Val Phe Lys Ala Phe Lys Gly Gln Asn Gly Gln
    130                 135                 140

Phe Ser Cys Ser Glu Asn Ile Gln Thr Asp Glu Glu Ile Arg Gly Val
145                 150                 155                 160

Leu Asn Leu Phe Arg Ala Ser Leu Ile Ala Phe Pro Gly Glu Lys Ile
                165                 170                 175

Met Asp Glu Ala Glu Ile Phe Ser Thr Lys Tyr Leu Lys Glu Ala Leu
            180                 185                 190

Gln Lys Ile Pro Val Ser Ser Leu Ser Arg Glu Ile Gly Asp Val Leu
        195                 200                 205

Glu Tyr Gly Trp His Thr Tyr Leu Pro Arg Leu Glu Ala Arg Asn Tyr
    210                 215                 220

Ile His Val Phe Gly Gln Asp Thr Glu Asn Thr Lys Ser Tyr Val Lys
225                 230                 235                 240

Ser Lys Lys Leu Leu Glu Leu Ala Lys Leu Glu Phe Asn Ile Phe Gln
                245                 250                 255

Ser Leu Gln Lys Arg Glu Leu Glu Ser Leu Val Arg Trp Trp Lys Glu
            260                 265                 270

Ser Gly Phe Pro Glu Met Thr Phe Cys Arg His Arg His Val Glu Tyr
        275                 280                 285

Tyr Thr Leu Ala Ser Cys Ile Ala Phe Glu Pro Gln His Ser Gly Phe
    290                 295                 300

Arg Leu Gly Phe Ala Lys Thr Cys His Leu Ile Thr Val Leu Asp Asp
305                 310                 315                 320

Met Tyr Asp Thr Phe Gly Thr Val Asp Glu Leu Glu Leu Phe Thr Ala
                325                 330                 335

Thr Met Lys Arg Trp Asp Pro Ser Ser Ile Asp Cys Leu Pro Glu Tyr
            340                 345                 350

Met Lys Gly Val Tyr Ile Ala Val Tyr Asp Thr Val Asn Glu Met Ala
        355                 360                 365

Arg Glu Ala Glu Glu Ala Gln Gly Arg Asp Thr Leu Thr Tyr Ala Arg

370                 375                 380

Glu Ala Trp Glu Ala Tyr Ile Asp Ser Tyr Met Gln Glu Ala Arg Trp
385                 390                 395                 400

Ile Ala Thr Gly Tyr Leu Pro Ser Phe Asp Glu Tyr Tyr Glu Asn Gly
                405                 410                 415

Lys Val Ser Cys Gly His Arg Ile Ser Ala Leu Gln Pro Ile Leu Thr
            420                 425                 430

Met Asp Ile Pro Phe Pro Asp His Ile Leu Lys Glu Val Asp Phe Pro
        435                 440                 445

Ser Lys Leu Asn Asp Leu Ala Cys Ala Ile Leu Arg Leu Arg Gly Asp
    450                 455                 460

Thr Arg Cys Tyr Lys Ala Asp Arg Ala Arg Gly Glu Glu Ala Ser Ser
465                 470                 475                 480

Ile Ser Cys Tyr Met Lys Asp Asn Pro Gly Val Ser Glu Glu Asp Ala
                485                 490                 495

Leu Asp His Ile Asn Ala Met Ile Ser Asp Val Ile Lys Gly Leu Asn
            500                 505                 510

Trp Glu Leu Leu Lys Pro Asp Ile Asn Val Pro Ile Ser Ala Lys Lys
        515                 520                 525

His Ala Phe Asp Ile Ala Arg Ala Phe His Tyr Gly Tyr Lys Tyr Arg
    530                 535                 540

Asp Gly Tyr Ser Val Ala Asn Val Glu Thr Lys Ser Leu Val Thr Arg
545                 550                 555                 560

Thr Leu Leu Glu Ser Val Pro Leu Gly Thr Gly Glu Asn Leu Tyr Phe
                565                 570                 575

Gln Gly Ser Gly Gly Gly Gly Ser Asp Tyr Lys Asp Asp Asp Asp Lys
            580                 585                 590

Gly Thr Gly
        595

<210> 4
<211> 590
<212> PRT
<213> Artificial Sequence

<220>
<223> Description of Artificial Sequence: Synthetic
      polypeptide

<400> 4
Met Val Pro Arg Arg Val Gly Asn Tyr His Ser Asn Leu Trp Asp Asp
1               5                   10                  15

Asp Phe Ile Gln Ser Leu Ile Ser Thr Pro Tyr Gly Ala Pro Asp Tyr
            20                  25                  30

Arg Glu Arg Ala Asp Arg Leu Ile Gly Glu Val Lys Asp Ile Met Phe
        35                  40                  45

Asn Phe Lys Ser Leu Glu Asp Gly Gly Asn Asp Leu Leu Gln Arg Leu
    50                  55                  60

Leu Leu Val Asp Asp Val Glu Arg Leu Gly Ile Asp Arg His Phe Lys
65                  70                  75                  80

Lys Glu Ile Lys Thr Ala Leu Asp Tyr Val Asn Ser Tyr Trp Asn Glu
                85                  90                  95

Lys Gly Ile Gly Cys Gly Arg Glu Ser Val Val Thr Asp Leu Asn Ser
            100                 105                 110

Thr Ala Leu Gly Leu Arg Thr Leu Arg Leu His Gly Tyr Thr Val Ser
        115                 120                 125

Ser Asp Val Leu Asn Val Phe Lys Asp Lys Asn Gly Gln Phe Ser Ser
    130                 135                 140

Thr Ala Asn Ile Gln Ile Glu Gly Glu Ile Arg Gly Val Leu Asn Leu
145                 150                 155                 160

Phe Arg Ala Ser Leu Val Ala Phe Pro Gly Glu Lys Val Met Asp Glu
                165                 170                 175

Ala Glu Thr Phe Ser Thr Lys Tyr Leu Arg Glu Ala Leu Gln Lys Ile
            180                 185                 190

Pro Ala Ser Ser Ile Leu Ser Leu Glu Ile Arg Asp Val Leu Glu Tyr
        195                 200                 205

Gly Trp His Thr Asn Leu Pro Arg Leu Glu Ala Arg Asn Tyr Met Asp
    210                 215                 220

Val Phe Gly Gln His Thr Lys Asn Lys Asn Ala Ala Glu Lys Leu Leu

225                 230                 235                 240

Glu Leu Ala Lys Leu Glu Phe Asn Ile Phe His Ser Leu Gln Glu Arg
                245                 250                 255

Glu Leu Lys His Val Ser Arg Trp Trp Lys Asp Ser Gly Ser Pro Glu
            260                 265                 270

Met Thr Phe Cys Arg His Arg His Val Glu Tyr Tyr Ala Leu Ala Ser
        275                 280                 285

Cys Ile Ala Phe Glu Pro Gln His Ser Gly Phe Arg Leu Gly Phe Thr
    290                 295                 300

Lys Met Ser His Leu Ile Thr Val Leu Asp Asp Met Tyr Asp Val Phe
305                 310                 315                 320

Gly Thr Val Asp Glu Leu Glu Leu Phe Thr Ala Thr Ile Lys Arg Trp
                325                 330                 335

Asp Pro Ser Ala Met Glu Cys Leu Pro Glu Tyr Met Lys Gly Val Tyr
            340                 345                 350

Met Met Val Tyr His Thr Val Asn Glu Met Ala Arg Val Ala Glu Lys
        355                 360                 365

Ala Gln Gly Arg Asp Thr Leu Asn Tyr Ala Arg Gln Ala Trp Glu Ala
    370                 375                 380

Cys Phe Asp Ser Tyr Met Gln Glu Ala Lys Trp Ile Ala Thr Gly Tyr
385                 390                 395                 400

Leu Pro Thr Phe Glu Glu Tyr Leu Glu Asn Gly Lys Val Ser Ser Ala
                405                 410                 415

His Arg Pro Cys Ala Leu Gln Pro Ile Leu Thr Leu Asp Ile Pro Phe
            420                 425                 430

Pro Asp His Ile Leu Lys Glu Val Asp Phe Pro Ser Lys Leu Asn Asp
        435                 440                 445

Leu Ile Cys Ile Ile Leu Arg Leu Arg Gly Asp Thr Arg Cys Tyr Lys
    450                 455                 460

Ala Asp Arg Ala Arg Gly Glu Glu Ala Ser Ser Ile Ser Cys Tyr Met
465                 470                 475                 480

Lys Asp Asn Pro Gly Leu Thr Glu Glu Asp Ala Leu Asn His Ile Asn
                485                 490                 495

Phe Met Ile Arg Asp Ala Ile Arg Glu Leu Asn Trp Glu Leu Leu Lys
            500                 505                 510

Pro Asp Asn Ser Val Pro Ile Thr Ser Lys Lys His Ala Phe Asp Ile
        515                 520                 525

Ser Arg Val Trp His His Gly Tyr Arg Tyr Arg Asp Gly Tyr Ser Phe
    530                 535                 540

Ala Asn Val Glu Thr Lys Ser Leu Val Met Arg Thr Val Ile Glu Pro
545                 550                 555                 560

Val Pro Leu Gly Thr Gly Glu Asn Leu Tyr Phe Gln Gly Ser Gly Gly
                565                 570                 575

Gly Gly Ser Asp Tyr Lys Asp Asp Asp Asp Lys Gly Thr Gly
            580                 585                 590

<210> 5
<211> 569
<212> PRT
<213> Artificial Sequence

<220>
<223> Description of Artificial Sequence: Synthetic
      polypeptide

<400> 5
Met Val Pro Arg Arg Ser Gly Asp Tyr Gln Pro Ser Leu Trp Asp Phe
1               5                   10                  15

Asn Tyr Ile Gln Ser Leu Asn Thr Pro Tyr Lys Glu Gln Arg His Phe
            20                  25                  30

Asn Arg Gln Ala Glu Leu Ile Met Gln Val Arg Met Leu Leu Lys Val
        35                  40                  45

Lys Met Glu Ala Ile Gln Gln Leu Glu Leu Ile Asp Asp Leu Gln Tyr
    50                  55                  60

Leu Gly Leu Ser Tyr Phe Phe Gln Asp Glu Ile Lys Gln Ile Leu Ser
65                  70                  75                  80

Ser Ile His Asn Glu Pro Arg Tyr Phe His Asn Asn Asp Leu Tyr Phe
                85                  90                  95

Thr Ala Leu Gly Phe Arg Ile Leu Arg Gln His Gly Phe Asn Val Ser

            100                 105                 110

Glu Asp Val Phe Asp Cys Phe Lys Ile Glu Lys Cys Ser Asp Phe Asn
        115                 120                 125

Ala Asn Leu Ala Gln Asp Thr Lys Gly Met Leu Gln Leu Tyr Glu Ala
    130                 135                 140

Ser Phe Leu Leu Arg Glu Gly Glu Asp Thr Leu Glu Leu Ala Arg Arg
145                 150                 155                 160

Phe Ser Thr Arg Ser Leu Arg Glu Lys Phe Asp Glu Gly Gly Asp Glu
                165                 170                 175

Ile Asp Glu Asp Leu Ser Ser Trp Ile Arg His Ser Leu Asp Leu Pro
            180                 185                 190

Leu His Trp Arg Val Gln Gly Leu Glu Ala Arg Trp Phe Leu Asp Ala
        195                 200                 205

Tyr Ala Arg Arg Pro Asp Met Asn Pro Leu Ile Phe Lys Leu Ala Lys
    210                 215                 220

Leu Asn Phe Asn Ile Val Gln Ala Thr Tyr Gln Glu Glu Leu Lys Asp
225                 230                 235                 240

Ile Ser Arg Trp Trp Asn Ser Ser Cys Leu Ala Glu Lys Leu Pro Phe
                245                 250                 255

Val Arg Asp Arg Ile Val Glu Cys Phe Phe Trp Ala Ile Ala Ala Phe
            260                 265                 270

Glu Pro His Gln Tyr Ser Tyr Gln Arg Lys Met Ala Ala Val Ile Ile
        275                 280                 285

Thr Phe Ile Thr Ile Ile Asp Asp Val Tyr Asp Val Tyr Gly Thr Ile
    290                 295                 300

Glu Glu Leu Glu Leu Leu Thr Asp Met Ile Arg Arg Trp Asp Asn Lys
305                 310                 315                 320

Ser Ile Ser Gln Leu Pro Tyr Tyr Met Gln Val Cys Tyr Leu Ala Leu
                325                 330                 335

Tyr Asn Phe Val Ser Glu Arg Ala Tyr Asp Ile Leu Lys Asp Gln His
            340                 345                 350

Phe Asn Ser Ile Pro Tyr Leu Gln Arg Ser Trp Val Ser Leu Val Glu
        355                 360                 365

Gly Tyr Leu Lys Glu Ala Tyr Trp Tyr Tyr Asn Gly Tyr Lys Pro Ser
    370                 375                 380

Leu Glu Glu Tyr Leu Asn Asn Ala Lys Ile Ser Ile Ser Ala Pro Thr
385                 390                 395                 400

Ile Ile Ser Gln Leu Tyr Phe Thr Leu Ala Asn Ser Ile Asp Glu Thr
                405                 410                 415

Ala Ile Glu Ser Leu Tyr Gln Tyr His Asn Ile Leu Tyr Leu Ser Gly
            420                 425                 430

Thr Ile Leu Arg Leu Ala Asp Asp Leu Gly Thr Ser Gln His Glu Leu
        435                 440                 445

Glu Arg Gly Asp Val Pro Lys Ala Ile Gln Cys Tyr Met Asn Asp Thr
    450                 455                 460

Asn Ala Ser Glu Arg Glu Ala Val Glu His Val Lys Phe Leu Ile Arg
465                 470                 475                 480

Glu Ala Trp Lys Glu Met Asn Thr Val Thr Thr Ala Ser Asp Cys Pro
                485                 490                 495

Phe Thr Asp Asp Leu Val Ala Ala Ala Ala Asn Leu Ala Arg Ala Ala
            500                 505                 510

Gln Phe Ile Tyr Leu Asp Gly Asp Gly His Gly Val Gln His Ser Glu
        515                 520                 525

Ile His Gln Gln Met Gly Gly Leu Leu Phe Gln Pro Tyr Val Gly Thr
    530                 535                 540

Gly Glu Asn Leu Tyr Phe Gln Gly Ser Gly Gly Gly Gly Ser Asp Tyr
545                 550                 555                 560

Lys Asp Asp Asp Asp Lys Gly Thr Gly
                565

<210> 6
<211> 594
<212> PRT
<213> Artificial Sequence

<220>
<223> Description of Artificial Sequence: Synthetic

polypeptide

<400> 6
Met Val Pro Arg Arg Ile Gly Asp Tyr His Ser Asn Ile Trp Asp Asp
1               5                   10                  15

Asp Phe Ile Gln Ser Leu Ser Thr Pro Tyr Gly Glu Pro Ser Tyr Gln
            20                  25                  30

Glu Arg Ala Glu Arg Leu Ile Val Glu Val Lys Lys Ile Phe Asn Ser
        35                  40                  45

Met Tyr Leu Asp Asp Gly Arg Leu Met Ser Ser Phe Asn Asp Leu Met
    50                  55                  60

Gln Arg Leu Trp Ile Val Asp Ser Val Glu Arg Leu Gly Ile Ala Arg
65                  70                  75                  80

His Phe Lys Asn Glu Ile Thr Ser Ala Leu Asp Tyr Val Phe Arg Tyr
                85                  90                  95

Trp Glu Glu Asn Gly Ile Gly Cys Gly Arg Asp Ser Ile Val Thr Asp
            100                 105                 110

Leu Asn Ser Thr Ala Leu Gly Phe Arg Thr Leu Arg Leu His Gly Tyr
        115                 120                 125

Thr Val Ser Pro Glu Val Leu Lys Ala Phe Gln Asp Gln Asn Gly Gln
    130                 135                 140

Phe Val Cys Ser Pro Gly Gln Thr Glu Gly Glu Ile Arg Ser Val Leu
145                 150                 155                 160

Asn Leu Tyr Arg Ala Ser Leu Ile Ala Phe Pro Gly Glu Lys Val Met
                165                 170                 175

Glu Glu Ala Glu Ile Phe Ser Thr Arg Tyr Leu Lys Glu Ala Leu Gln
            180                 185                 190

Lys Ile Pro Val Ser Ala Leu Ser Gln Glu Ile Lys Phe Val Met Glu
        195                 200                 205

Tyr Gly Trp His Thr Asn Leu Pro Arg Leu Glu Ala Arg Asn Tyr Ile
    210                 215                 220

Asp Thr Leu Glu Lys Asp Thr Ser Ala Trp Leu Asn Lys Asn Ala Gly
225                 230                 235                 240

Lys Lys Leu Leu Glu Leu Ala Lys Leu Glu Phe Asn Ile Phe Asn Ser
                245                 250                 255

Leu Gln Gln Lys Glu Leu Gln Tyr Leu Leu Arg Trp Trp Lys Glu Ser
            260                 265                 270

Asp Leu Pro Lys Leu Thr Phe Ala Arg His Arg His Val Glu Phe Tyr
        275                 280                 285

Thr Leu Ala Ser Cys Ile Ala Ile Asp Pro Lys His Ser Ala Phe Arg
    290                 295                 300

Leu Gly Phe Ala Lys Met Cys His Leu Val Thr Val Leu Asp Asp Ile
305                 310                 315                 320

Tyr Asp Thr Phe Gly Thr Ile Asp Glu Leu Glu Leu Phe Thr Ser Ala
                325                 330                 335

Ile Lys Arg Trp Asn Ser Ser Glu Ile Glu His Leu Pro Glu Tyr Met
            340                 345                 350

Lys Cys Val Tyr Met Val Val Phe Glu Thr Val Asn Glu Leu Thr Arg
        355                 360                 365

Glu Ala Glu Lys Thr Gln Gly Arg Asn Thr Leu Asn Tyr Val Arg Lys
    370                 375                 380

Ala Trp Glu Ala Tyr Phe Asp Ser Tyr Met Glu Glu Ala Lys Trp Ile
385                 390                 395                 400

Ser Asn Gly Tyr Leu Pro Met Phe Glu Glu Tyr His Glu Asn Gly Lys
                405                 410                 415

Val Ser Ser Ala Tyr Arg Val Ala Thr Leu Gln Pro Ile Leu Thr Leu
            420                 425                 430

Asn Ala Trp Leu Pro Asp Tyr Ile Leu Lys Gly Ile Asp Phe Pro Ser
        435                 440                 445

Arg Phe Asn Asp Leu Ala Ser Ser Phe Leu Arg Leu Arg Gly Asp Thr
    450                 455                 460

Arg Cys Tyr Lys Ala Asp Arg Asp Arg Gly Glu Glu Ala Ser Cys Ile
465                 470                 475                 480

Ser Cys Tyr Met Lys Asp Asn Pro Gly Ser Thr Glu Glu Asp Ala Leu
                485                 490                 495

Asn His Ile Asn Ala Met Val Asn Asp Ile Ile Lys Glu Leu Asn Trp
                500                 505                 510

Glu Leu Leu Arg Ser Asn Asp Asn Ile Pro Met Leu Ala Lys Lys His
        515                 520                 525

Ala Phe Asp Ile Thr Arg Ala Leu His His Leu Tyr Ile Tyr Arg Asp
    530                 535                 540

Gly Phe Ser Val Ala Asn Lys Glu Thr Lys Lys Leu Val Met Glu Thr
545                 550                 555                 560

Leu Leu Glu Ser Met Leu Phe Gly Thr Gly Glu Asn Leu Tyr Phe Gln
                565                 570                 575

Gly Ser Gly Gly Gly Gly Ser Asp Tyr Lys Asp Asp Asp Asp Lys Gly
                580                 585                 590

Thr Gly

<210> 7
<211> 828
<212> PRT
<213> Artificial Sequence

<220>
<223> Description of Artificial Sequence: Synthetic
      polypeptide

<400> 7
Met Val Pro Gln Ser Ala Glu Lys Asn Asp Ser Leu Ser Ser Ser Thr
1               5                   10                  15

Leu Val Lys Arg Glu Phe Pro Pro Gly Phe Trp Lys Asp Asp Leu Ile
            20                  25                  30

Asp Ser Leu Thr Ser Ser His Lys Val Ala Ala Ser Asp Glu Lys Arg
        35                  40                  45

Ile Glu Thr Leu Ile Ser Glu Ile Lys Asn Met Phe Arg Cys Met Gly
    50                  55                  60

Tyr Gly Glu Thr Asn Pro Ser Ala Tyr Asp Thr Ala Trp Val Ala Arg
65                  70                  75                  80

Ile Pro Ala Val Asp Gly Ser Asp Asn Pro His Phe Pro Glu Thr Val
                85                  90                  95

Glu Trp Ile Leu Gln Asn Gln Leu Lys Asp Gly Ser Trp Gly Glu Gly
        100                 105                 110

Phe Tyr Phe Leu Ala Tyr Asp Arg Ile Leu Ala Thr Leu Ala Cys Ile
        115                 120                 125

Ile Thr Leu Thr Leu Trp Arg Thr Gly Glu Thr Gln Val Gln Lys Gly
    130                 135                 140

Ile Glu Phe Phe Arg Thr Gln Ala Gly Lys Met Glu Asp Glu Ala Asp
145                 150                 155                 160

Ser His Arg Pro Ser Gly Phe Glu Ile Val Phe Pro Ala Met Leu Lys
                165                 170                 175

Glu Ala Lys Ile Leu Gly Leu Asp Leu Pro Tyr Asp Leu Pro Phe Leu
            180                 185                 190

Lys Gln Ile Ile Glu Lys Arg Glu Ala Lys Leu Lys Arg Ile Pro Thr
        195                 200                 205

Asp Val Leu Tyr Ala Leu Pro Thr Thr Leu Leu Tyr Ser Leu Glu Gly
    210                 215                 220

Leu Gln Glu Ile Val Asp Trp Gln Lys Ile Met Lys Leu Gln Ser Lys
225                 230                 235                 240

Asp Gly Ser Phe Leu Ser Ser Pro Ala Ser Thr Ala Ala Val Phe Met
                245                 250                 255

Arg Thr Gly Asn Lys Lys Cys Leu Asp Phe Leu Asn Phe Val Leu Lys
            260                 265                 270

Lys Phe Gly Asn His Val Pro Cys His Tyr Pro Leu Asp Leu Phe Glu
        275                 280                 285

Arg Leu Trp Ala Val Asp Thr Val Glu Arg Leu Gly Ile Asp Arg His
    290                 295                 300

Phe Lys Glu Glu Ile Lys Glu Ala Leu Asp Tyr Val Tyr Ser His Trp
305                 310                 315                 320

Asp Glu Arg Gly Ile Gly Trp Ala Arg Glu Asn Pro Val Pro Asp Ile
                325                 330                 335

Asp Asp Thr Ala Met Gly Leu Arg Ile Leu Arg Leu His Gly Tyr Asn
            340                 345                 350

Val Ser Ser Asp Val Leu Lys Thr Phe Arg Asp Glu Asn Gly Glu Phe
        355                 360                 365

Phe Cys Phe Leu Gly Gln Thr Gln Arg Gly Val Thr Asp Met Leu Asn
    370                 375                 380

Val Asn Arg Cys Ser His Val Ser Phe Pro Gly Glu Thr Ile Met Glu
385                 390                 395                 400

Glu Ala Lys Leu Cys Thr Glu Arg Tyr Leu Arg Asn Ala Leu Glu Asn
                405                 410                 415

Val Asp Ala Phe Asp Lys Trp Ala Phe Lys Lys Asn Ile Arg Gly Glu
            420                 425                 430

Val Glu Tyr Ala Leu Lys Tyr Pro Trp His Lys Ser Met Pro Arg Leu
        435                 440                 445

Glu Ala Arg Ser Tyr Ile Glu Asn Tyr Gly Pro Asp Asp Val Trp Leu
    450                 455                 460

Gly Lys Thr Val Tyr Met Met Pro Tyr Ile Ser Asn Glu Lys Tyr Leu
465                 470                 475                 480

Glu Leu Ala Lys Leu Asp Phe Asn Lys Val Gln Ser Ile His Gln Thr
                485                 490                 495

Glu Leu Gln Asp Leu Arg Arg Trp Trp Lys Ser Ser Gly Phe Thr Asp
            500                 505                 510

Leu Asn Phe Thr Arg Glu Arg Val Thr Glu Ile Tyr Phe Ser Pro Ala
        515                 520                 525

Ser Phe Ile Phe Glu Pro Glu Phe Ser Lys Cys Arg Glu Val Tyr Thr
    530                 535                 540

Lys Thr Ser Asn Phe Thr Val Ile Leu Asp Asp Leu Tyr Asp Ala His
545                 550                 555                 560

Gly Ser Leu Asp Asp Leu Lys Leu Phe Thr Glu Ser Val Lys Arg Trp
                565                 570                 575

Asp Leu Ser Leu Val Asp Gln Met Pro Gln Gln Met Lys Ile Cys Phe
            580                 585                 590

Val Gly Phe Tyr Asn Thr Phe Asn Asp Ile Ala Lys Glu Gly Arg Glu
        595                 600                 605

Arg Gln Gly Arg Asp Val Leu Gly Tyr Ile Gln Asn Val Trp Lys Val
    610                 615                 620

Gln Leu Glu Ala Tyr Thr Lys Glu Ala Glu Trp Ser Glu Ala Lys Tyr
625                 630                 635                 640

Val Pro Ser Phe Asn Glu Tyr Ile Glu Asn Ala Ser Val Ser Ile Ala
                645                 650                 655

Leu Gly Thr Val Val Leu Ile Ser Ala Leu Phe Thr Gly Glu Val Leu
            660                 665                 670

Thr Asp Glu Val Leu Ser Lys Ile Asp Arg Glu Ser Arg Phe Leu Gln
        675                 680                 685

Leu Met Gly Leu Thr Gly Arg Leu Val Asn Asp Thr Lys Thr Tyr Gln
    690                 695                 700

Ala Glu Arg Gly Gln Gly Glu Val Ala Ser Ala Ile Gln Cys Tyr Met
705                 710                 715                 720

Lys Asp His Pro Lys Ile Ser Glu Glu Glu Ala Leu Gln His Val Tyr
                725                 730                 735

Ser Val Met Glu Asn Ala Leu Glu Glu Leu Asn Arg Glu Phe Val Asn
            740                 745                 750

Asn Lys Ile Pro Asp Ile Tyr Lys Arg Leu Val Phe Glu Thr Ala Arg
        755                 760                 765

Ile Met Gln Leu Phe Tyr Met Gln Gly Asp Gly Leu Thr Leu Ser His
    770                 775                 780

Asp Met Glu Ile Lys Glu His Val Lys Asn Cys Leu Phe Gln Pro Val
785                 790                 795                 800

Ala Gly Thr Gly Glu Asn Leu Tyr Phe Gln Gly Ser Gly Gly Gly Gly
                805                 810                 815

Ser Asp Tyr Lys Asp Asp Asp Asp Lys Gly Thr Gly
            820                 825

<210> 8
<211> 831
<212> PRT
<213> Artificial Sequence

<220>
<223> Description of Artificial Sequence: Synthetic
      polypeptide

<400> 8
Met Val Pro Ser Ser Ser Thr Gly Thr Ser Lys Val Val Ser Glu Thr
1               5                   10                  15

Ser Ser Thr Ile Val Asp Asp Ile Pro Arg Leu Ser Ala Asn Tyr His
            20                  25                  30

Gly Asp Leu Trp His His Asn Val Ile Gln Thr Leu Glu Thr Pro Phe
        35                  40                  45

Arg Glu Ser Ser Thr Tyr Gln Glu Arg Ala Asp Glu Leu Val Val Lys
    50                  55                  60

Ile Lys Asp Met Phe Asn Ala Leu Gly Asp Gly Asp Ile Ser Pro Ser
65                  70                  75                  80

Ala Tyr Asp Thr Ala Trp Val Ala Arg Val Ala Thr Ile Ser Ser Asp
                85                  90                  95

Gly Ser Glu Lys Pro Arg Phe Pro Gln Ala Leu Asn Trp Val Phe Asn
            100                 105                 110

Asn Gln Leu Gln Asp Gly Ser Trp Gly Ile Glu Ser His Phe Ser Leu
        115                 120                 125

Cys Asp Arg Leu Leu Asn Thr Thr Asn Ser Val Ile Ala Leu Ser Val
    130                 135                 140

Trp Lys Thr Gly His Ser Gln Val Gln Gln Gly Ala Glu Phe Ile Ala
145                 150                 155                 160

Glu Asn Leu Arg Leu Leu Asn Glu Glu Asp Glu Leu Ser Pro Asp Phe
                165                 170                 175

Gln Ile Ile Phe Pro Ala Leu Leu Gln Lys Ala Lys Ala Leu Gly Ile
            180                 185                 190

Asn Leu Pro Tyr Asp Leu Pro Phe Ile Lys Tyr Leu Ser Thr Thr Arg
        195                 200                 205

Glu Ala Arg Leu Thr Asp Val Ser Ala Ala Ala Asp Asn Ile Pro Ala
    210                 215                 220

Asn Met Leu Asn Ala Leu Glu Gly Leu Glu Glu Val Ile Asp Trp Asn
225                 230                 235                 240

Lys Ile Met Arg Phe Gln Ser Lys Asp Gly Ser Phe Leu Ser Ser Pro
                245                 250                 255

Ala Ser Thr Ala Cys Val Leu Met Asn Thr Gly Asp Glu Lys Cys Phe
            260                 265                 270

Thr Phe Leu Asn Asn Leu Leu Asp Lys Phe Gly Gly Cys Val Pro Cys
        275                 280                 285

Met Tyr Ser Ile Asp Leu Leu Glu Arg Leu Ser Leu Val Asp Asn Ile
    290                 295                 300

Glu His Leu Gly Ile Gly Arg His Phe Lys Gln Glu Ile Lys Gly Ala
305                 310                 315                 320

Leu Asp Tyr Val Tyr Arg His Trp Ser Glu Arg Gly Ile Gly Trp Gly
                325                 330                 335

Arg Asp Ser Leu Val Pro Asp Leu Asn Thr Thr Ala Leu Gly Leu Arg
            340                 345                 350

Thr Leu Arg Met His Gly Tyr Asn Val Ser Ser Asp Val Leu Asn Asn
        355                 360                 365

Phe Lys Asp Glu Asn Gly Arg Phe Phe Ser Ser Ala Gly Gln Thr His
    370                 375                 380

Val Glu Leu Arg Ser Val Val Asn Leu Phe Arg Ala Ser Asp Leu Ala
385                 390                 395                 400

Phe Pro Asp Glu Arg Ala Met Asp Asp Ala Arg Lys Phe Ala Glu Pro
                405                 410                 415

Tyr Leu Arg Glu Ala Leu Ala Thr Lys Ile Ser Thr Asn Thr Lys Leu
            420                 425                 430

Phe Lys Glu Ile Glu Tyr Val Val Glu Tyr Pro Trp His Met Ser Ile
        435                 440                 445

Pro Arg Leu Glu Ala Arg Ser Tyr Ile Asp Ser Tyr Asp Asp Asn Tyr
    450                 455                 460

Val Trp Gln Arg Lys Thr Leu Tyr Arg Met Pro Ser Leu Ser Asn Ser
465                 470                 475                 480

Lys Cys Leu Glu Leu Ala Lys Leu Asp Phe Asn Ile Val Gln Ser Leu

                485                 490                 495

His Gln Glu Glu Leu Lys Leu Leu Thr Arg Trp Trp Lys Glu Ser Gly
            500                 505                 510

Met Ala Asp Ile Asn Phe Thr Arg His Arg Val Ala Glu Val Tyr Phe
        515                 520                 525

Ser Ser Ala Thr Phe Glu Pro Glu Tyr Ser Ala Thr Arg Ile Ala Phe
    530                 535                 540

Thr Lys Ile Gly Cys Leu Gln Val Leu Phe Asp Asp Met Ala Asp Ile
545                 550                 555                 560

Phe Ala Thr Leu Asp Glu Leu Lys Ser Phe Thr Glu Gly Val Lys Arg
                565                 570                 575

Trp Asp Thr Ser Leu Leu His Glu Ile Pro Glu Cys Met Gln Thr Cys
            580                 585                 590

Phe Lys Val Trp Phe Lys Leu Met Glu Glu Val Asn Asn Asp Val Val
        595                 600                 605

Lys Val Gln Gly Arg Asp Met Leu Ala His Ile Arg Lys Pro Trp Glu
    610                 615                 620

Leu Tyr Phe Asn Cys Tyr Val Gln Glu Arg Glu Trp Leu Glu Ala Gly
625                 630                 635                 640

Tyr Ile Pro Thr Phe Glu Glu Tyr Leu Lys Thr Tyr Ala Ile Ser Val
                645                 650                 655

Gly Leu Gly Pro Cys Thr Leu Gln Pro Ile Leu Leu Met Gly Glu Leu
            660                 665                 670

Val Lys Asp Asp Val Val Glu Lys Val His Tyr Pro Ser Asn Met Phe
        675                 680                 685

Glu Leu Val Ser Leu Ser Trp Arg Leu Thr Asn Asp Thr Lys Thr Tyr
    690                 695                 700

Gln Ala Glu Lys Val Arg Gly Gln Gln Ala Ser Gly Ile Ala Cys Tyr
705                 710                 715                 720

Met Lys Asp Asn Pro Gly Ala Thr Glu Glu Asp Ala Ile Lys His Ile
                725                 730                 735

Cys Arg Val Val Asp Arg Ala Leu Lys Glu Ala Ser Phe Glu Tyr Phe
            740                 745                 750

Lys Pro Ser Asn Asp Ile Pro Met Gly Cys Lys Ser Phe Ile Phe Asn
        755                 760                 765

Leu Arg Leu Cys Val Gln Ile Phe Tyr Lys Phe Ile Asp Gly Tyr Gly
    770                 775                 780

Ile Ala Asn Glu Glu Ile Lys Asp Tyr Ile Arg Lys Val Tyr Ile Asp
785                 790                 795                 800

Pro Ile Gln Val Gly Thr Gly Glu Asn Leu Tyr Phe Gln Gly Ser Gly
                805                 810                 815

Gly Gly Gly Ser Asp Tyr Lys Asp Asp Asp Asp Lys Gly Thr Gly
            820                 825                 830

<210> 9
<211> 396
<212> PRT
<213> Artificial Sequence

<220>
<223> Description of Artificial Sequence: Synthetic
      polypeptide

<400> 9
Met Val Pro His Lys Phe Thr Gly Val Asn Ala Lys Phe Gln Gln Pro
1               5                   10                  15

Ala Leu Arg Asn Leu Ser Pro Val Val Val Glu Arg Glu Arg Glu Glu
            20                  25                  30

Phe Val Gly Phe Phe Pro Gln Ile Val Arg Asp Leu Thr Glu Asp Gly
        35                  40                  45

Ile Gly His Pro Glu Val Gly Asp Ala Val Ala Arg Leu Lys Glu Val
    50                  55                  60

Leu Gln Tyr Asn Ala Pro Gly Gly Lys Cys Asn Arg Gly Leu Thr Val
65                  70                  75                  80

Val Ala Ala Tyr Arg Glu Leu Ser Gly Pro Gly Gln Lys Asp Ala Glu
                85                  90                  95

Ser Leu Arg Cys Ala Leu Ala Val Gly Trp Cys Ile Glu Leu Phe Gln
            100                 105                 110

Ala Phe Phe Leu Val Ala Asp Asp Ile Met Asp Gln Ser Leu Thr Arg

115                 120                 125

Arg Gly Gln Leu Cys Trp Tyr Lys Lys Glu Gly Val Gly Leu Asp Ala
    130                 135                 140

Ile Asn Asp Ser Phe Leu Leu Glu Ser Ser Val Tyr Arg Val Leu Lys
145                 150                 155                 160

Lys Tyr Cys Arg Gln Arg Pro Tyr Tyr Val His Leu Leu Glu Leu Phe
                165                 170                 175

Leu Gln Thr Ala Tyr Gln Thr Glu Leu Gly Gln Met Leu Asp Leu Ile
            180                 185                 190

Thr Ala Pro Val Ser Lys Val Asp Leu Ser His Phe Ser Glu Glu Arg
        195                 200                 205

Tyr Lys Ala Ile Val Lys Tyr Lys Thr Ala Phe Tyr Ser Phe Tyr Leu
    210                 215                 220

Pro Val Ala Ala Ala Met Tyr Met Val Gly Ile Asp Ser Lys Glu Glu
225                 230                 235                 240

His Glu Asn Ala Lys Ala Ile Leu Leu Glu Met Gly Glu Tyr Phe Gln
                245                 250                 255

Ile Gln Asp Asp Tyr Leu Asp Cys Phe Gly Asp Pro Ala Leu Thr Gly
            260                 265                 270

Lys Val Gly Thr Asp Ile Gln Asp Asn Lys Cys Ser Trp Leu Val Val
        275                 280                 285

Gln Cys Leu Gln Arg Val Thr Pro Glu Gln Arg Gln Leu Leu Glu Asp
    290                 295                 300

Asn Tyr Gly Arg Lys Glu Pro Glu Lys Val Ala Lys Val Lys Glu Leu
305                 310                 315                 320

Tyr Glu Ala Val Gly Met Arg Ala Ala Phe Gln Gln Tyr Glu Glu Ser
                325                 330                 335

Ser Tyr Arg Arg Leu Gln Glu Leu Ile Glu Lys His Ser Asn Arg Leu
            340                 345                 350

Pro Lys Glu Ile Phe Leu Gly Leu Ala Gln Lys Ile Tyr Lys Arg Gln
        355                 360                 365

Lys Gly Thr Gly Glu Asn Leu Tyr Phe Gln Gly Ser Gly Gly Gly Gly
    370                 375                 380

Ser Asp Tyr Lys Asp Asp Asp Asp Lys Gly Thr Gly
385                 390                 395

<210> 10
<211> 575
<212> PRT
<213> Artificial Sequence

<220>
<223> Description of Artificial Sequence: Synthetic
      polypeptide

<400> 10
Met Val Pro Ser Leu Thr Glu Glu Lys Pro Ile Arg Pro Ile Ala Asn
1               5                   10                  15

Phe Pro Pro Ser Ile Trp Gly Asp Gln Phe Leu Ile Tyr Glu Lys Gln
            20                  25                  30

Val Glu Gln Gly Val Glu Gln Ile Val Asn Asp Leu Lys Lys Glu Val
        35                  40                  45

Arg Gln Leu Leu Lys Glu Ala Leu Asp Ile Pro Met Lys His Ala Asn
    50                  55                  60

Leu Leu Lys Leu Ile Asp Glu Ile Gln Arg Leu Gly Ile Pro Tyr His
65                  70                  75                  80

Phe Glu Arg Glu Ile Asp His Ala Leu Gln Cys Ile Tyr Glu Thr Tyr
                85                  90                  95

Gly Asp Asn Trp Asn Gly Asp Arg Ser Ser Leu Trp Phe Arg Leu Met
            100                 105                 110

Arg Lys Gln Gly Tyr Tyr Val Thr Cys Asp Val Phe Asn Asn Tyr Lys
        115                 120                 125

Asp Lys Asn Gly Ala Phe Lys Gln Ser Leu Ala Asn Asp Val Glu Gly
    130                 135                 140

Leu Leu Glu Leu Tyr Glu Ala Thr Ser Met Arg Val Pro Gly Glu Ile
145                 150                 155                 160

Ile Leu Glu Asp Ala Leu Gly Phe Thr Arg Ser Arg Leu Ser Ile Met
                165                 170                 175

Thr Lys Asp Ala Phe Ser Thr Asn Pro Ala Leu Phe Thr Glu Ile Gln

            180                 185                 190

Arg Ala Leu Lys Gln Pro Leu Trp Lys Arg Leu Pro Arg Ile Glu Ala
        195                 200                 205

Ala Gln Tyr Ile Pro Phe Tyr Gln Gln Gln Asp Ser His Asn Lys Thr
    210                 215                 220

Leu Leu Lys Leu Ala Lys Leu Glu Phe Asn Leu Leu Gln Ser Leu His
225                 230                 235                 240

Lys Glu Glu Leu Ser His Val Cys Lys Trp Trp Lys Ala Phe Asp Ile
                245                 250                 255

Lys Lys Asn Ala Pro Cys Leu Arg Asp Arg Ile Val Glu Cys Tyr Phe
            260                 265                 270

Trp Gly Leu Gly Ser Gly Tyr Glu Pro Gln Tyr Ser Arg Ala Arg Val
        275                 280                 285

Phe Phe Thr Lys Ala Val Ala Val Ile Thr Leu Ile Asp Asp Thr Tyr
    290                 295                 300

Asp Ala Tyr Gly Thr Tyr Glu Glu Leu Lys Ile Phe Thr Glu Ala Val
305                 310                 315                 320

Glu Arg Trp Ser Ile Thr Cys Leu Asp Thr Leu Pro Glu Tyr Met Lys
                325                 330                 335

Pro Ile Tyr Lys Leu Phe Met Asp Thr Tyr Thr Glu Met Glu Glu Phe
            340                 345                 350

Leu Ala Lys Glu Gly Arg Thr Asp Leu Phe Asn Cys Gly Lys Glu Phe
        355                 360                 365

Val Lys Glu Phe Val Arg Asn Leu Met Val Glu Ala Lys Trp Ala Asn
    370                 375                 380

Glu Gly His Ile Pro Thr Thr Glu Glu His Asp Pro Val Val Ile Ile
385                 390                 395                 400

Thr Gly Gly Ala Asn Leu Leu Thr Thr Thr Cys Tyr Leu Gly Met Ser
                405                 410                 415

Asp Ile Phe Thr Lys Glu Ser Val Glu Trp Ala Val Ser Ala Pro Pro
            420                 425                 430

Leu Phe Arg Tyr Ser Gly Ile Leu Gly Arg Arg Leu Asn Asp Leu Met
        435                 440                 445

Thr His Lys Ala Glu Gln Glu Arg Lys His Ser Ser Ser Ser Leu Glu
    450                 455                 460

Ser Tyr Met Lys Glu Tyr Asn Val Asn Glu Glu Tyr Ala Gln Thr Leu
465                 470                 475                 480

Ile Tyr Lys Glu Val Glu Asp Val Trp Lys Asp Ile Asn Arg Glu Tyr
                485                 490                 495

Leu Thr Thr Lys Asn Ile Pro Arg Pro Leu Leu Met Ala Val Ile Tyr
            500                 505                 510

Leu Cys Gln Phe Leu Glu Val Gln Tyr Ala Gly Lys Asp Asn Phe Thr
        515                 520                 525

Arg Met Gly Asp Glu Tyr Lys His Leu Ile Lys Ser Leu Leu Val Tyr
    530                 535                 540

Pro Met Ser Ile Gly Thr Gly Glu Asn Leu Tyr Phe Gln Gly Ser Gly
545                 550                 555                 560

Gly Gly Gly Ser Asp Tyr Lys Asp Asp Asp Asp Lys Gly Thr Gly
                565                 570                 575

<210> 11

<400> 11
000

<210> 12
<211> 846
<212> PRT
<213> Artificial Sequence

<220>
<223> Description of Artificial Sequence: Synthetic
      polypeptide

<400> 12
Met Val Pro Ala Gly Val Ser Ala Val Ser Lys Val Ser Ser Leu Val
1               5                   10                  15

Cys Asp Leu Ser Ser Thr Ser Gly Leu Ile Arg Arg Thr Ala Asn Pro
            20                  25                  30

His Pro Asn Val Trp Gly Tyr Asp Leu Val His Ser Leu Lys Ser Pro
        35                  40                  45

Tyr Ile Asp Ser Ser Tyr Arg Glu Arg Ala Glu Val Leu Val Ser Glu
    50                  55                  60

Ile Lys Ala Met Leu Asn Pro Ala Ile Thr Gly Asp Gly Glu Ser Met
65                  70                  75                  80

Ile Thr Pro Ser Ala Tyr Asp Thr Ala Trp Val Ala Arg Val Pro Ala
                85                  90                  95

Ile Asp Gly Ser Ala Arg Pro Gln Phe Pro Gln Thr Val Asp Trp Ile
            100                 105                 110

Leu Lys Asn Gln Leu Lys Asp Gly Ser Trp Gly Ile Gln Ser His Phe
        115                 120                 125

Leu Leu Ser Asp Arg Leu Leu Ala Thr Leu Ser Cys Val Leu Val Leu
    130                 135                 140

Leu Lys Trp Asn Val Gly Asp Leu Gln Val Glu Gln Gly Ile Glu Phe
145                 150                 155                 160

Ile Lys Ser Asn Leu Glu Leu Val Lys Asp Glu Thr Asp Gln Asp Ser
                165                 170                 175

Leu Val Thr Asp Phe Glu Ile Ile Phe Pro Ser Leu Leu Arg Glu Ala
            180                 185                 190

Gln Ser Leu Arg Leu Gly Leu Pro Tyr Asp Leu Pro Tyr Ile His Leu
        195                 200                 205

Leu Gln Thr Lys Arg Gln Glu Arg Leu Ala Lys Leu Ser Arg Glu Glu
    210                 215                 220

Ile Tyr Ala Val Pro Ser Pro Leu Leu Tyr Ser Leu Glu Gly Ile Gln
225                 230                 235                 240

Asp Ile Val Glu Trp Glu Arg Ile Met Glu Val Gln Ser Gln Asp Gly
                245                 250                 255

Ser Phe Leu Ser Ser Pro Ala Ser Thr Ala Cys Val Phe Met His Thr
            260                 265                 270

Gly Asp Ala Lys Cys Leu Glu Phe Leu Asn Ser Val Met Ile Lys Phe
        275                 280                 285

Gly Asn Phe Val Pro Cys Leu Tyr Pro Val Asp Leu Leu Glu Arg Leu
    290                 295                 300

Leu Ile Val Asp Asn Ile Val Arg Leu Gly Ile Tyr Arg His Phe Glu
305                 310                 315                 320

Lys Glu Ile Lys Glu Ala Leu Asp Tyr Val Tyr Arg His Trp Asn Glu
                325                 330                 335

Arg Gly Ile Gly Trp Gly Arg Leu Asn Pro Ile Ala Asp Leu Glu Thr
            340                 345                 350

Thr Ala Leu Gly Phe Arg Leu Leu Arg Leu His Arg Tyr Asn Val Ser
        355                 360                 365

Pro Ala Ile Phe Asp Asn Phe Lys Asp Ala Asn Gly Lys Phe Ile Cys
    370                 375                 380

Ser Thr Gly Gln Phe Asn Lys Asp Val Ala Ser Met Leu Asn Leu Tyr
385                 390                 395                 400

Arg Ala Ser Gln Leu Ala Phe Pro Gly Glu Asn Ile Leu Asp Glu Ala
                405                 410                 415

Lys Ser Phe Ala Thr Lys Tyr Leu Arg Glu Ala Leu Glu Lys Ser Glu
            420                 425                 430

Thr Ser Ser Ala Trp Asn Asn Lys Gln Asn Leu Ser Gln Glu Ile Lys
        435                 440                 445

Tyr Ala Leu Lys Thr Ser Trp His Ala Ser Val Pro Arg Val Glu Ala
    450                 455                 460

Lys Arg Tyr Cys Gln Val Tyr Arg Pro Asp Tyr Ala Arg Ile Ala Lys
465                 470                 475                 480

Cys Val Tyr Lys Leu Pro Tyr Val Asn Asn Glu Lys Phe Leu Glu Leu
                485                 490                 495

Gly Lys Leu Asp Phe Asn Ile Ile Gln Ser Ile His Gln Glu Glu Met
            500                 505                 510

Lys Asn Val Thr Ser Trp Phe Arg Asp Ser Gly Leu Pro Leu Phe Thr
        515                 520                 525

Phe Ala Arg Glu Arg Pro Leu Glu Phe Tyr Phe Leu Val Ala Ala Gly
    530                 535                 540

Thr Tyr Glu Pro Gln Tyr Ala Lys Cys Arg Phe Leu Phe Thr Lys Val
545                 550                 555                 560

Ala Cys Leu Gln Thr Val Leu Asp Asp Met Tyr Asp Thr Tyr Gly Thr
                565                 570                 575

Leu Asp Glu Leu Lys Leu Phe Thr Glu Ala Val Arg Arg Trp Asp Leu
            580                 585                 590

Ser Phe Thr Glu Asn Leu Pro Asp Tyr Met Lys Leu Cys Tyr Gln Ile
        595                 600                 605

Tyr Tyr Asp Ile Val His Glu Val Ala Trp Glu Ala Glu Lys Glu Gln
    610                 615                 620

Gly Arg Glu Leu Val Ser Phe Phe Arg Lys Gly Trp Glu Asp Tyr Leu
625                 630                 635                 640

Leu Gly Tyr Tyr Glu Glu Ala Glu Trp Leu Ala Ala Glu Tyr Val Pro
                645                 650                 655

Thr Leu Asp Glu Tyr Ile Lys Asn Gly Ile Thr Ser Ile Gly Gln Arg
            660                 665                 670

Ile Leu Leu Leu Ser Gly Val Leu Ile Met Asp Gly Gln Leu Leu Ser
        675                 680                 685

Gln Glu Ala Leu Glu Lys Val Asp Tyr Pro Gly Arg Arg Val Leu Thr
    690                 695                 700

Glu Leu Asn Ser Leu Ile Ser Arg Leu Ala Asp Asp Thr Lys Thr Tyr
705                 710                 715                 720

Lys Ala Glu Lys Ala Arg Gly Glu Leu Ala Ser Ser Ile Glu Cys Tyr
                725                 730                 735

Met Lys Asp His Pro Glu Cys Thr Glu Glu Glu Ala Leu Asp His Ile
            740                 745                 750

Tyr Ser Ile Leu Glu Pro Ala Val Lys Glu Leu Thr Arg Glu Phe Leu
        755                 760                 765

Lys Pro Asp Asp Val Pro Phe Ala Cys Lys Lys Met Leu Phe Glu Glu
    770                 775                 780

Thr Arg Val Thr Met Val Ile Phe Lys Asp Gly Asp Gly Phe Gly Val
785                 790                 795                 800

Ser Lys Leu Glu Val Lys Asp His Ile Lys Glu Cys Leu Ile Glu Pro

805                 810                 815

Leu Pro Leu Gly Thr Gly Glu Asn Leu Tyr Phe Gln Gly Ser Gly Gly
            820                 825                 830

Gly Gly Ser Asp Tyr Lys Asp Asp Asp Asp Lys Gly Thr Gly
        835                 840                 845

<210> 13
<211> 310
<212> PRT
<213> Artificial Sequence

<220>
<223> Description of Artificial Sequence: Synthetic
      polypeptide

<400> 13
Met Val Pro Thr Met Met Asn Met Asn Phe Lys Tyr Cys His Lys Ile
1               5                   10                  15

Met Lys Lys His Ser Lys Ser Phe Ser Tyr Ala Phe Asp Leu Leu Pro
            20                  25                  30

Glu Asp Gln Arg Lys Ala Val Trp Ala Ile Tyr Ala Val Cys Arg Lys
        35                  40                  45

Ile Asp Asp Ser Ile Asp Val Tyr Gly Asp Ile Gln Phe Leu Asn Gln
    50                  55                  60

Ile Lys Glu Asp Ile Gln Ser Ile Glu Lys Tyr Pro Tyr Glu His His
65                  70                  75                  80

His Phe Gln Ser Asp Arg Arg Ile Met Met Ala Leu Gln His Val Ala
                85                  90                  95

Gln His Lys Asn Ile Ala Phe Gln Ser Phe Tyr Asn Leu Ile Asp Thr
            100                 105                 110

Val Tyr Lys Asp Gln His Phe Thr Met Phe Glu Thr Asp Ala Glu Leu
        115                 120                 125

Phe Gly Tyr Cys Tyr Gly Val Ala Gly Thr Val Gly Glu Val Leu Thr
    130                 135                 140

Pro Ile Leu Ser Asp His Glu Thr His Gln Thr Tyr Asp Val Ala Arg
145                 150                 155                 160

Arg Leu Gly Glu Ser Leu Gln Leu Ile Asn Ile Leu Arg Asp Val Gly
                165                 170                 175

Glu Asp Phe Asp Asn Glu Arg Ile Tyr Phe Ser Lys Gln Arg Leu Lys
            180                 185                 190

Gln Tyr Glu Val Asp Ile Ala Glu Val Tyr Gln Asn Gly Val Asn Asn
        195                 200                 205

His Tyr Ile Asp Leu Trp Glu Tyr Tyr Ala Ala Ile Ala Glu Lys Asp
    210                 215                 220

Phe Gln Asp Val Met Asp Gln Ile Lys Val Phe Ser Ile Glu Ala Gln
225                 230                 235                 240

Pro Ile Ile Glu Leu Ala Ala Arg Ile Tyr Ile Glu Ile Leu Asp Glu
                245                 250                 255

Val Arg Gln Ala Asn Tyr Thr Leu His Glu Arg Val Phe Val Asp Lys
            260                 265                 270

Arg Lys Lys Ala Lys Leu Phe His Glu Asn Lys Gly Thr Gly Glu Asn
        275                 280                 285

Leu Tyr Phe Gln Gly Ser Gly Gly Gly Gly Ser Asp Tyr Lys Asp Asp
    290                 295                 300

Asp Asp Lys Gly Thr Gly
305                 310

<210> 14
<211> 531
<212> PRT
<213> Artificial Sequence

<220>
<223> Description of Artificial Sequence: Synthetic
      polypeptide

<400> 14
Met Val Pro Lys Ile Ala Val Ile Gly Ala Gly Val Thr Gly Leu Ala
1               5                   10                  15

Ala Ala Ala Arg Ile Ala Ser Gln Gly His Glu Val Thr Ile Phe Glu
            20                  25                  30

Lys Asn Asn Asn Val Gly Gly Arg Met Asn Gln Leu Lys Lys Asp Gly
        35                  40                  45

Phe Thr Phe Asp Met Gly Pro Thr Ile Val Met Met Pro Asp Val Tyr
    50                  55                  60

Lys Asp Val Phe Thr Ala Cys Gly Lys Asn Tyr Glu Asp Tyr Ile Glu
65                  70                  75                  80

Leu Arg Gln Leu Arg Tyr Ile Tyr Asp Val Tyr Phe Asp His Asp Asp
                85                  90                  95

Arg Ile Thr Val Pro Thr Asp Leu Ala Glu Leu Gln Gln Met Leu Glu
            100                 105                 110

Ser Ile Glu Pro Gly Ser Thr His Gly Phe Met Ser Phe Leu Thr Asp
        115                 120                 125

Val Tyr Lys Lys Tyr Glu Ile Ala Arg Arg Tyr Phe Leu Glu Arg Thr
    130                 135                 140

Tyr Arg Lys Pro Ser Asp Phe Tyr Asn Met Thr Ser Leu Val Gln Gly
145                 150                 155                 160

Ala Lys Leu Lys Thr Leu Asn His Ala Asp Gln Leu Ile Glu His Tyr
                165                 170                 175

Ile Asp Asn Glu Lys Ile Gln Lys Leu Leu Ala Phe Gln Thr Leu Tyr
            180                 185                 190

Ile Gly Ile Asp Pro Lys Arg Gly Pro Ser Leu Tyr Ser Ile Ile Pro
        195                 200                 205

Met Ile Glu Met Met Phe Gly Val His Phe Ile Lys Gly Gly Met Tyr
    210                 215                 220

Gly Met Ala Gln Gly Leu Ala Gln Leu Asn Lys Asp Leu Gly Val Asn
225                 230                 235                 240

Ile Glu Leu Asn Ala Glu Ile Glu Gln Ile Ile Ile Asp Pro Lys Phe
                245                 250                 255

Lys Arg Ala Asp Ala Ile Lys Val Asn Gly Asp Ile Arg Lys Phe Asp
            260                 265                 270

Lys Ile Leu Cys Thr Ala Asp Phe Pro Ser Val Ala Glu Ser Leu Met
        275                 280                 285

Pro Asp Phe Ala Pro Ile Lys Lys Tyr Pro Pro His Lys Ile Ala Asp
    290                 295                 300

Leu Asp Tyr Ser Cys Ser Ala Phe Leu Met Tyr Ile Gly Ile Asp Ile
305                 310                 315                 320

Asp Val Thr Asp Gln Val Arg Leu His Asn Val Ile Phe Ser Asp Asp
                325                 330                 335

Phe Arg Gly Asn Ile Glu Glu Ile Phe Glu Gly Arg Leu Ser Tyr Asp
            340                 345                 350

Pro Ser Ile Tyr Val Tyr Val Pro Ala Val Ala Asp Lys Ser Leu Ala
        355                 360                 365

Pro Glu Gly Lys Thr Gly Ile Tyr Val Leu Met Pro Thr Pro Glu Leu
    370                 375                 380

Lys Thr Gly Ser Gly Ile Asp Trp Ser Asp Glu Ala Leu Thr Gln Gln
385                 390                 395                 400

Ile Lys Glu Ile Ile Tyr Arg Lys Leu Ala Thr Ile Glu Val Phe Glu
                405                 410                 415

Asp Ile Lys Ser His Ile Val Ser Glu Thr Ile Phe Thr Pro Asn Asp
            420                 425                 430

Phe Glu Gln Thr Tyr His Ala Lys Phe Gly Ser Ala Phe Gly Leu Met
        435                 440                 445

Pro Thr Leu Ala Gln Ser Asn Tyr Tyr Arg Pro Gln Asn Val Ser Arg
    450                 455                 460

Asp Tyr Lys Asp Leu Tyr Phe Ala Gly Ala Ser Thr His Pro Gly Ala
465                 470                 475                 480

Gly Val Pro Ile Val Leu Thr Ser Ala Lys Ile Thr Val Asp Glu Met
                485                 490                 495

Ile Lys Asp Ile Glu Arg Gly Val Gly Thr Gly Glu Asn Leu Tyr Phe
            500                 505                 510

Gln Gly Ser Gly Gly Gly Gly Ser Asp Tyr Lys Asp Asp Asp Asp Lys
        515                 520                 525

Gly Thr Gly
    530

<210> 15
<211> 325
<212> PRT
<213> Artificial Sequence

<220>
<223> Description of Artificial Sequence: Synthetic
      polypeptide

<400> 15
Met Val Pro Ala Phe Asp Phe Asp Gly Tyr Met Leu Arg Lys Ala Lys
1               5                   10                  15

Ser Val Asn Lys Ala Leu Glu Ala Ala Val Gln Met Lys Glu Pro Leu
            20                  25                  30

Lys Ile His Glu Ser Met Arg Tyr Ser Leu Leu Ala Gly Gly Lys Arg
        35                  40                  45

Val Arg Pro Met Leu Cys Ile Ala Ala Cys Glu Leu Val Gly Gly Asp
    50                  55                  60

Glu Ser Thr Ala Met Pro Ala Ala Cys Ala Val Glu Met Ile His Thr
65                  70                  75                  80

Met Ser Leu Met His Asp Asp Leu Pro Cys Met Asp Asn Asp Asp Leu
                85                  90                  95

Arg Arg Gly Lys Pro Thr Asn His Met Ala Phe Gly Glu Ser Val Ala
            100                 105                 110

Val Leu Ala Gly Asp Ala Leu Leu Ser Phe Ala Phe Glu His Val Ala
        115                 120                 125

Ala Ala Thr Lys Gly Ala Pro Pro Glu Arg Ile Val Arg Val Leu Gly
    130                 135                 140

Glu Leu Ala Val Ser Ile Gly Ser Glu Gly Leu Val Ala Gly Gln Val
145                 150                 155                 160

Val Asp Val Cys Ser Glu Gly Met Ala Glu Val Gly Leu Asp His Leu
                165                 170                 175

Glu Phe Ile His His His Lys Thr Ala Ala Leu Leu Gln Gly Ser Val
            180                 185                 190

Val Leu Gly Ala Ile Leu Gly Gly Gly Lys Glu Glu Glu Val Ala Lys
        195                 200                 205

Leu Arg Lys Phe Ala Asn Cys Ile Gly Leu Leu Phe Gln Val Val Asp
    210                 215                 220

Asp Ile Leu Asp Val Thr Lys Ser Ser Lys Glu Leu Gly Lys Thr Ala
225                 230                 235                 240

Gly Lys Asp Leu Val Ala Asp Lys Thr Thr Tyr Pro Lys Leu Ile Gly
                245                 250                 255

Val Glu Lys Ser Lys Glu Phe Ala Asp Arg Leu Asn Arg Glu Ala Gln
            260                 265                 270

Glu Gln Leu Leu His Phe His Pro His Arg Ala Ala Pro Leu Ile Ala
        275                 280                 285

Leu Ala Asn Tyr Ile Ala Tyr Arg Asp Asn Gly Thr Gly Glu Asn Leu
    290                 295                 300

Tyr Phe Gln Gly Ser Gly Gly Gly Gly Ser Asp Tyr Lys Asp Asp Asp
305                 310                 315                 320

Asp Lys Gly Thr Gly
                325

<210> 16
<211> 296
<212> PRT
<213> Artificial Sequence

<220>
<223> Description of Artificial Sequence: Synthetic
      polypeptide

<400> 16
Met Val Pro Ser Gln Pro Tyr Trp Ala Ala Ile Glu Ala Asp Ile Glu
1               5                   10                  15

Arg Tyr Leu Lys Lys Ser Ile Thr Ile Arg Pro Pro Glu Thr Val Phe
            20                  25                  30

Gly Pro Met His His Leu Thr Phe Ala Ala Pro Ala Thr Ala Ala Ser
        35                  40                  45

Thr Leu Cys Leu Ala Ala Cys Glu Leu Val Gly Gly Asp Arg Ser Gln
    50                  55                  60

Ala Met Ala Ala Ala Ala Ala Ile His Leu Val His Ala Ala Ala Tyr
65                  70                  75                  80

Val His Glu His Leu Pro Leu Thr Asp Gly Ser Arg Pro Val Ser Lys
                85                  90                  95

Pro Ala Ile Gln His Lys Tyr Gly Pro Asn Val Glu Leu Leu Thr Gly
            100                 105                 110

Asp Gly Ile Val Pro Phe Gly Phe Glu Leu Leu Ala Gly Ser Val Asp
        115                 120                 125

Pro Ala Arg Thr Asp Asp Pro Asp Arg Ile Leu Arg Val Ile Ile Glu
    130                 135                 140

Ile Ser Arg Ala Gly Gly Pro Glu Gly Met Ile Ser Gly Leu His Arg
145                 150                 155                 160

Glu Glu Glu Ile Val Asp Gly Asn Thr Ser Leu Asp Phe Ile Glu Tyr
                165                 170                 175

Val Cys Lys Lys Lys Tyr Gly Glu Met His Ala Cys Gly Ala Ala Cys
            180                 185                 190

Gly Ala Ile Leu Gly Gly Ala Ala Glu Glu Glu Ile Gln Lys Leu Arg
        195                 200                 205

Asn Phe Gly Leu Tyr Gln Gly Thr Leu Arg Gly Met Met Glu Met Lys
    210                 215                 220

Asn Ser His Gln Leu Ile Asp Glu Asn Ile Ile Gly Lys Leu Lys Glu
225                 230                 235                 240

Leu Ala Leu Glu Glu Leu Gly Gly Phe His Gly Lys Asn Ala Glu Leu
                245                 250                 255

Met Ser Ser Leu Val Ala Glu Pro Ser Leu Tyr Ala Ala Gly Thr Gly
            260                 265                 270

Glu Asn Leu Tyr Phe Gln Gly Ser Gly Gly Gly Gly Ser Asp Tyr Lys
        275                 280                 285

Asp Asp Asp Asp Lys Gly Thr Gly
    290                 295

<210> 17
<211> 359
<212> PRT
<213> Artificial Sequence

<220>
<223> Description of Artificial Sequence: Synthetic
      polypeptide

<400> 17
   Met Val Pro Leu Leu Ser Asn Lys Leu Arg Glu Met Val Leu Ala Glu
   1               5                   10                  15
   Val Pro Lys Leu Ala Ser Ala Ala Glu Tyr Phe Phe Lys Arg Gly Val
   20 25 30
   Gln Gly Lys Gln Phe Arg Ser Thr Ile Leu Leu Leu Met Ala Thr Ala
   35 40 45
   Leu Asp Val Arg Val Pro Glu Ala Leu Ile Gly Glu Ser Thr Asp Ile
   50 55 60
   Val Thr Ser Glu Leu Arg Val Arg Gln Arg Gly Ile Ala Glu Ile Thr
   65 70 75 80
   Glu Met Ile His Val Ala Ser Leu Leu His Asp Asp Val Leu Asp Asp
   85 90 95
   Ala Asp Thr Arg Arg Gly Val Gly Ser Leu Asn Val Val Met Gly Asn
   100 105 110
   Lys Met Ser Val Leu Ala Gly Asp Phe Leu Leu Ser Arg Ala Cys Gly
   115 120 125
   Ala Leu Ala Ala Leu Lys Asn Thr Glu Val Val Ala Leu Leu Ala Thr
   130 135 140
   Ala Val Glu His Leu Val Thr Gly Glu Thr Met Glu Ile Thr Ser Ser
   145 150 155 160
   Thr Glu Gln Arg Tyr Ser Met Asp Tyr Tyr Met Gln Lys Thr Tyr Tyr
   165 170 175
   Lys Thr Ala Ser Leu Ile Ser Asn Ser Cys Lys Ala Val Ala Val Leu
   180 185 190
   Thr Gly Gln Thr Ala Glu Val Ala Val Leu Ala Phe Glu Tyr Gly Arg
   195 200 205
   Asn Leu Gly Leu Ala Phe Gln Leu Ile Asp Asp Ile Leu Asp Phe Thr
   210 215 220
   Gly Thr Ser Ala Ser Leu Gly Lys Gly Ser Leu Ser Asp Ile Arg His
   225 230 235 240
   Gly Val Ile Thr Ala Pro Ile Leu Phe Ala Met Glu Glu Phe Pro Gln
   245 250 255
   Leu Arg Glu Val Val Asp Gln Val Glu Lys Asp Pro Arg Asn Val Asp
   260 265 270
   Ile Ala Leu Glu Tyr Leu Gly Lys Ser Lys Gly Ile Gln Arg Ala Arg
           275                 280                 285
   Glu Leu Ala Met Glu His Ala Asn Leu Ala Ala Ala Ala Ile Gly Ser
       290                 295                 300
   Leu Pro Glu Thr Asp Asn Glu Asp Val Lys Arg Ser Arg Arg Ala Leu
   305                 310                 315                 320
   Ile Asp Leu Thr His Arg Val Ile Thr Arg Asn Lys Gly Thr Gly Glu
                   325                 330                 335
   Asn Leu Tyr Phe Gln Gly Ser Gly Gly Gly Gly Ser Asp Tyr Lys Asp
               340                 345                 350
   Asp Asp Asp Lys Gly Thr Gly
           355

<210> 18
<211> 363
<212> PRT
<213> Artificial Sequence

<220>
<223> Description of Artificial Sequence: Synthetic
      polypeptide

<400> 18
Met Val Pro Val Val Ser Glu Arg Leu Arg His Ser Val Thr Thr Gly
1               5                   10                  15

Ile Pro Ala Leu Lys Thr Ala Ala Glu Tyr Phe Phe Arg Arg Gly Ile
            20                  25                  30

Glu Gly Lys Arg Leu Arg Pro Thr Leu Ala Leu Leu Met Ser Ser Ala
        35                  40                  45

Leu Ser Pro Ala Ala Pro Ser Pro Glu Tyr Leu Gln Val Asp Thr Arg
    50                  55                  60

Pro Ala Ala Glu His Pro His Glu Met Arg Arg Arg Gln Gln Arg Leu
65                  70                  75                  80

Ala Glu Ile Ala Glu Leu Ile His Val Ala Ser Leu Leu His Asp Asp
                85                  90                  95

Val Ile Asp Asp Ala Gln Thr Arg Arg Gly Val Leu Ser Leu Asn Thr
            100                 105                 110

Ser Val Gly Asn Lys Thr Ala Ile Leu Ala Gly Asp Phe Leu Leu Ala
        115                 120                 125

Arg Ala Ser Val Thr Leu Ala Ser Leu Arg Asn Ser Glu Ile Val Glu
    130                 135                 140

Leu Met Ser Gln Val Leu Glu His Leu Val Ser Gly Glu Ile Met Gln
145                 150                 155                 160

Met Thr Ala Thr Ser Glu Gln Leu Leu Asp Leu Glu His Tyr Leu Ala
                165                 170                 175

Lys Thr Tyr Cys Lys Thr Ala Ser Leu Met Ala Asn Ser Ser Arg Ser
            180                 185                 190

Val Ala Val Leu Ala Gly Ala Ala Pro Glu Val Cys Asp Met Ala Trp
        195                 200                 205

Ser Tyr Gly Arg His Leu Gly Ile Ala Phe Gln Val Val Asp Asp Leu
    210                 215                 220

Leu Asp Leu Thr Gly Ser Ser Ser Val Leu Gly Lys Pro Ala Leu Asn
225                 230                 235                 240

Asp Met Arg Ser Gly Leu Ala Thr Ala Pro Val Leu Phe Ala Ala Gln
                245                 250                 255

Glu Glu Pro Ala Leu Gln Ala Leu Ile Leu Arg Arg Phe Lys His Asp
            260                 265                 270

Gly Asp Val Thr Lys Ala Met Ser Leu Ile Glu Arg Thr Gln Gly Leu
        275                 280                 285

Arg Arg Ala Glu Glu Leu Ala Ala Gln His Ala Lys Ala Ala Ala Asp
    290                 295                 300

Met Ile Arg Cys Leu Pro Thr Ala Gln Ser Asp His Ala Glu Ile Ala
305                 310                 315                 320

Arg Glu Ala Leu Ile Gln Ile Thr His Arg Val Leu Thr Arg Lys Lys
                325                 330                 335

Gly Thr Gly Glu Asn Leu Tyr Phe Gln Gly Ser Gly Gly Gly Gly Ser
            340                 345                 350

Asp Tyr Lys Asp Asp Asp Asp Lys Gly Thr Gly
        355                 360

<210> 19
<211> 328
<212> PRT
<213> Artificial Sequence

<220>
<223> Description of Artificial Sequence: Synthetic
      polypeptide

<400> 19
Met Val Pro Asp Phe Pro Gln Gln Leu Glu Ala Cys Val Lys Gln Ala
1               5                   10                  15

Asn Gln Ala Leu Ser Arg Phe Ile Ala Pro Leu Pro Phe Gln Asn Thr
            20                  25                  30

Pro Val Val Glu Thr Met Gln Tyr Gly Ala Leu Leu Gly Gly Lys Arg
        35                  40                  45

Leu Arg Pro Phe Leu Val Tyr Ala Thr Gly His Met Phe Gly Val Ser
    50                  55                  60

Thr Asn Thr Leu Asp Ala Pro Ala Ala Ala Val Glu Cys Ile His Ala
65                  70                  75                  80

Tyr Ser Leu Ile His Asp Asp Leu Pro Ala Met Asp Asp Asp Asp Leu
                85                  90                  95

Arg Arg Gly Leu Pro Thr Cys His Val Lys Phe Gly Glu Ala Asn Ala
            100                 105                 110

Ile Leu Ala Gly Asp Ala Leu Gln Thr Leu Ala Phe Ser Ile Leu Ser
        115                 120                 125

Asp Ala Asp Met Pro Glu Val Ser Asp Arg Asp Arg Ile Ser Met Ile
    130                 135                 140

Ser Glu Leu Ala Ser Ala Ser Gly Ile Ala Gly Met Cys Gly Gly Gln
145                 150                 155                 160

Ala Leu Asp Leu Asp Ala Glu Gly Lys His Val Pro Leu Asp Ala Leu
                165                 170                 175

Glu Arg Ile His Arg His Lys Thr Gly Ala Leu Ile Arg Ala Ala Val
            180                 185                 190

Arg Leu Gly Ala Leu Ser Ala Gly Asp Lys Gly Arg Arg Ala Leu Pro
        195                 200                 205

Val Leu Asp Lys Tyr Ala Glu Ser Ile Gly Leu Ala Phe Gln Val Gln
    210                 215                 220

Asp Asp Ile Leu Asp Val Val Gly Asp Thr Ala Thr Leu Gly Lys Arg
225                 230                 235                 240

Gln Gly Ala Asp Gln Gln Leu Gly Lys Ser Thr Tyr Pro Ala Leu Leu
                245                 250                 255

Gly Leu Glu Gln Ala Arg Lys Lys Ala Arg Asp Leu Ile Asp Asp Ala
            260                 265                 270

Arg Gln Ser Leu Lys Gln Leu Ala Glu Gln Ser Leu Asp Thr Ser Ala
        275                 280                 285

Leu Glu Ala Leu Ala Asp Tyr Ile Ile Gln Arg Asn Lys Gly Thr Gly
    290                 295                 300

Glu Asn Leu Tyr Phe Gln Gly Ser Gly Gly Gly Gly Ser Asp Tyr Lys
305                 310                 315                 320

Asp Asp Asp Asp Lys Gly Thr Gly
                325

<210> 20
<211> 413
<212> PRT
<213> Artificial Sequence

<220>
<223> Description of Artificial Sequence: Synthetic
      polypeptide

<400> 20
Met Val Pro Ser Val Ser Cys Cys Cys Arg Asn Leu Gly Lys Thr Ile
1               5                   10                  15

Lys Lys Ala Ile Pro Ser His His Leu His Leu Arg Ser Leu Gly Gly
            20                  25                  30

Ser Leu Tyr Arg Arg Arg Ile Gln Ser Ser Ser Met Glu Thr Asp Leu
        35                  40                  45

Lys Ser Thr Phe Leu Asn Val Tyr Ser Val Leu Lys Ser Asp Leu Leu
    50                  55                  60

His Asp Pro Ser Phe Glu Phe Thr Asn Glu Ser Arg Leu Trp Val Asp
65                  70                  75                  80

Arg Met Leu Asp Tyr Asn Val Arg Gly Gly Lys Leu Asn Arg Gly Leu

                85                  90                  95

Ser Val Val Asp Ser Phe Lys Leu Leu Lys Gln Gly Asn Asp Leu Thr
            100                 105                 110

Glu Gln Glu Val Phe Leu Ser Cys Ala Leu Gly Trp Cys Ile Glu Trp
        115                 120                 125

Leu Gln Ala Tyr Phe Leu Val Leu Asp Asp Ile Met Asp Asn Ser Val
    130                 135                 140

Thr Arg Arg Gly Gln Pro Cys Trp Phe Arg Val Pro Gln Val Gly Met
145                 150                 155                 160

Val Ala Ile Asn Asp Gly Ile Leu Leu Arg Asn His Ile His Arg Ile
                165                 170                 175

Leu Lys Lys His Phe Arg Asp Lys Pro Tyr Tyr Val Asp Leu Val Asp
            180                 185                 190

Leu Phe Asn Glu Val Glu Leu Gln Thr Ala Cys Gly Gln Met Ile Asp
        195                 200                 205

Leu Ile Thr Thr Phe Glu Gly Glu Lys Asp Leu Ala Lys Tyr Ser Leu
    210                 215                 220

Ser Ile His Arg Arg Ile Val Gln Tyr Lys Thr Ala Tyr Tyr Ser Phe
225                 230                 235                 240

Tyr Leu Pro Val Ala Cys Ala Leu Leu Met Ala Gly Glu Asn Leu Glu
                245                 250                 255

Asn His Ile Asp Val Lys Asn Val Leu Val Asp Met Gly Ile Tyr Phe
            260                 265                 270

Gln Val Gln Asp Asp Tyr Leu Asp Cys Phe Ala Asp Pro Glu Thr Leu
        275                 280                 285

Gly Lys Ile Gly Thr Asp Ile Glu Asp Phe Lys Cys Ser Trp Leu Val
    290                 295                 300

Val Lys Ala Leu Glu Arg Cys Ser Glu Glu Gln Thr Lys Ile Leu Tyr
305                 310                 315                 320

Glu Asn Tyr Gly Lys Pro Asp Pro Ser Asn Val Ala Lys Val Lys Asp
                325                 330                 335

Leu Tyr Lys Glu Leu Asp Leu Glu Gly Val Phe Met Glu Tyr Glu Ser
            340                 345                 350

Lys Ser Tyr Glu Lys Leu Thr Gly Ala Ile Glu Gly His Gln Ser Lys
        355                 360                 365

Ala Ile Gln Ala Val Leu Lys Ser Phe Leu Ala Lys Ile Tyr Lys Arg
    370                 375                 380

Gln Lys Gly Thr Gly Glu Asn Leu Tyr Phe Gln Gly Ser Gly Gly Gly
385                 390                 395                 400

Gly Ser Asp Tyr Lys Asp Asp Asp Asp Lys Gly Thr Gly
            405                 410

<210> 21
<211> 371
<212> PRT
<213> Artificial Sequence

<220>
<223> Description of Artificial Sequence: Synthetic
      polypeptide

<400> 21
Met Val Pro Ala Asp Leu Lys Ser Thr Phe Leu Asp Val Tyr Ser Val
1               5                   10                  15

Leu Lys Ser Asp Leu Leu Gln Asp Pro Ser Phe Glu Phe Thr His Glu
            20                  25                  30

Ser Arg Gln Trp Leu Glu Arg Met Leu Asp Tyr Asn Val Arg Gly Gly
        35                  40                  45

Lys Leu Asn Arg Gly Leu Ser Val Val Asp Ser Tyr Lys Leu Leu Lys
    50                  55                  60

Gln Gly Gln Asp Leu Thr Glu Lys Glu Thr Phe Leu Ser Cys Ala Leu
65                  70                  75                  80

Gly Trp Cys Ile Glu Trp Leu Gln Ala Tyr Phe Leu Val Leu Asp Asp
                85                  90                  95

Ile Met Asp Asn Ser Val Thr Arg Arg Gly Gln Pro Cys Trp Phe Arg
            100                 105                 110

Lys Pro Lys Val Gly Met Ile Ala Ile Asn Asp Gly Ile Leu Leu Arg
        115                 120                 125

Asn His Ile His Arg Ile Leu Lys Lys His Phe Arg Glu Met Pro Tyr

130                 135                 140

Tyr Val Asp Leu Val Asp Leu Phe Asn Glu Val Glu Phe Gln Thr Ala
145                 150                 155                 160

Cys Gly Gln Met Ile Asp Leu Ile Thr Thr Phe Asp Gly Glu Lys Asp
                165                 170                 175

Leu Ser Lys Tyr Ser Leu Gln Ile His Arg Arg Ile Val Glu Tyr Lys
            180                 185                 190

Thr Ala Tyr Tyr Ser Phe Tyr Leu Pro Val Ala Cys Ala Leu Leu Met
        195                 200                 205

Ala Gly Glu Asn Leu Glu Asn His Thr Asp Val Lys Thr Val Leu Val
    210                 215                 220

Asp Met Gly Ile Tyr Phe Gln Val Gln Asp Asp Tyr Leu Asp Cys Phe
225                 230                 235                 240

Ala Asp Pro Glu Thr Leu Gly Lys Ile Gly Thr Asp Ile Glu Asp Phe
                245                 250                 255

Lys Cys Ser Trp Leu Val Val Lys Ala Leu Glu Arg Cys Ser Glu Glu
            260                 265                 270

Gln Thr Lys Ile Leu Tyr Glu Asn Tyr Gly Lys Ala Glu Pro Ser Asn
        275                 280                 285

Val Ala Lys Val Lys Ala Leu Tyr Lys Glu Leu Asp Leu Glu Gly Ala
    290                 295                 300

Phe Met Glu Tyr Glu Lys Glu Ser Tyr Glu Lys Leu Thr Lys Leu Ile
305                 310                 315                 320

Glu Ala His Gln Ser Lys Ala Ile Gln Ala Val Leu Lys Ser Phe Leu
                325                 330                 335

Ala Lys Ile Tyr Lys Arg Gln Lys Gly Thr Gly Glu Asn Leu Tyr Phe
            340                 345                 350

Gln Gly Ser Gly Gly Gly Gly Ser Asp Tyr Lys Asp Asp Asp Asp Lys
        355                 360                 365

Gly Thr Gly
    370

<210> 22
<211> 389
<212> PRT
<213> Artificial Sequence

<220>
<223> Description of Artificial Sequence: Synthetic
      polypeptide

<400> 22
Met Val Pro Ser Gly Glu Pro Thr Pro Lys Lys Met Lys Ala Thr Tyr
1               5                   10                  15

Val His Asp Arg Glu Asn Phe Thr Lys Val Tyr Glu Thr Leu Arg Asp
            20                  25                  30

Glu Leu Leu Asn Asp Asp Cys Leu Ser Pro Ala Gly Ser Pro Gln Ala
        35                  40                  45

Gln Ala Ala Gln Glu Trp Phe Lys Glu Val Asn Asp Tyr Asn Val Pro
    50                  55                  60

Gly Gly Lys Leu Asn Arg Gly Met Ala Val Tyr Asp Val Leu Ala Ser
65                  70                  75                  80

Val Lys Gly Pro Asp Gly Leu Ser Glu Asp Glu Val Phe Lys Ala Asn
                85                  90                  95

Ala Leu Gly Trp Cys Ile Glu Trp Leu Gln Ala Phe Phe Leu Val Ala
            100                 105                 110

Asp Asp Ile Met Asp Gly Ser Ile Thr Arg Arg Gly Gln Pro Cys Trp
        115                 120                 125

Tyr Lys Gln Pro Lys Val Gly Met Ile Ala Cys Asn Asp Tyr Ile Leu
    130                 135                 140

Leu Glu Cys Cys Ile Tyr Ser Ile Leu Lys Arg His Phe Arg Gly His
145                 150                 155                 160

Ala Ala Tyr Ala Gln Leu Met Asp Leu Phe His Glu Thr Thr Phe Gln
                165                 170                 175

Thr Ser His Gly Gln Leu Leu Asp Leu Thr Thr Ala Pro Ile Gly Ser
            180                 185                 190

Val Asp Leu Ser Lys Tyr Thr Glu Asp Asn Tyr Leu Arg Ile Val Thr
        195                 200                 205

Tyr Lys Thr Ala Tyr Tyr Ser Phe Tyr Leu Pro Val Ala Cys Gly Met

210                 215                 220

Val Leu Ala Gly Ile Thr Asp Pro Ala Ala Phe Asp Leu Ala Lys Asn
225                 230                 235                 240

Ile Cys Val Glu Met Gly Gln Tyr Phe Gln Ile Gln Asp Asp Tyr Leu
                245                 250                 255

Asp Cys Tyr Gly Asp Pro Glu Val Ile Gly Lys Ile Gly Thr Asp Ile
            260                 265                 270

Glu Asp Asn Lys Cys Ser Trp Leu Val Cys Thr Ala Leu Lys Ile Ala
        275                 280                 285

Thr Glu Glu Gln Lys Glu Val Ile Lys Ala Asn Tyr Gly His Lys Glu
    290                 295                 300

Ala Glu Ser Val Ala Ala Ile Lys Ala Leu Tyr Val Glu Leu Gly Ile
305                 310                 315                 320

Glu Gln Arg Phe Lys Asp Tyr Glu Ala Ala Ser Tyr Ala Lys Leu Glu
                325                 330                 335

Gly Thr Ile Ser Glu Gln Thr Leu Leu Pro Lys Ala Val Phe Thr Ser
            340                 345                 350

Leu Leu Ala Lys Ile Tyr Lys Arg Lys Lys Gly Thr Gly Glu Asn Leu
        355                 360                 365

Tyr Phe Gln Gly Ser Gly Gly Gly Gly Ser Asp Tyr Lys Asp Asp Asp
    370                 375                 380

Asp Lys Gly Thr Gly
385

<210> 23
<211> 211
<212> PRT
<213> Artificial Sequence

<220>
<223> Description of Artificial Sequence: Synthetic
      polypeptide

<400> 23
Met Val Pro Gln Thr Glu His Val Ile Leu Leu Asn Ala Gln Gly Val
1               5                   10                  15

Pro Thr Gly Thr Leu Glu Lys Tyr Ala Ala His Thr Ala Asp Thr Arg
            20                  25                  30

Leu His Leu Ala Phe Ser Ser Trp Leu Phe Asn Ala Lys Gly Gln Leu
        35                  40                  45

Leu Val Thr Arg Arg Ala Leu Ser Lys Lys Ala Trp Pro Gly Val Trp
    50                  55                  60

Thr Asn Ser Val Cys Gly His Pro Gln Leu Gly Glu Ser Asn Glu Asp
65                  70                  75                  80

Ala Val Ile Arg Arg Cys Arg Tyr Glu Leu Gly Val Glu Ile Thr Pro
                85                  90                  95

Pro Glu Ser Ile Tyr Pro Asp Phe Arg Tyr Arg Ala Thr Asp Pro Ser
            100                 105                 110

Gly Ile Val Glu Asn Glu Val Cys Pro Val Phe Ala Ala Arg Thr Thr
        115                 120                 125

Ser Ala Leu Gln Ile Asn Asp Asp Glu Val Met Asp Tyr Gln Trp Cys
    130                 135                 140

Asp Leu Ala Asp Val Leu His Gly Ile Asp Ala Thr Pro Trp Ala Phe
145                 150                 155                 160

Ser Pro Trp Met Val Met Gln Ala Thr Asn Arg Glu Ala Arg Lys Arg
                165                 170                 175

Leu Ser Ala Phe Thr Gln Leu Lys Gly Thr Gly Glu Asn Leu Tyr Phe
            180                 185                 190

Gln Gly Ser Gly Gly Gly Gly Ser Asp Tyr Lys Asp Asp Asp Asp Lys
        195                 200                 205

Gly Thr Gly
    210

<210> 24
<211> 322
<212> PRT
<213> Artificial Sequence

<220>
<223> Description of Artificial Sequence: Synthetic
      polypeptide

<400> 24
Met Val Pro Leu Arg Ser Leu Leu Arg Gly Leu Thr His Ile Pro Arg
1               5                   10                  15

Val Asn Ser Ala Gln Gln Pro Ser Cys Ala His Ala Arg Leu Gln Phe
            20                  25                  30
Lys Leu Arg Ser Met Gln Leu Leu Ala Glu Asn Arg Thr Asp His Met
        35                  40                  45
Arg Gly Ala Ser Thr Trp Ala Gly Gly Gln Ser Gln Asp Glu Leu Met
    50                  55                  60
Leu Lys Asp Glu Cys Ile Leu Val Asp Ala Asp Asp Asn Ile Thr Gly
65                  70                  75                  80
His Ala Ser Lys Leu Glu Cys His Lys Phe Leu Pro His Gln Pro Ala
                85                  90                  95
Gly Leu Leu His Arg Ala Phe Ser Val Phe Leu Phe Asp Asp Gln Gly
            100                 105                 110
Arg Leu Leu Leu Gln Gln Arg Ala Arg Ser Lys Ile Thr Phe Pro Ser
        115                 120                 125
Val Trp Ala Asn Thr Cys Cys Ser His Pro Leu His Gly Gln Thr Pro
    130                 135                 140
Asp Glu Val Asp Gln Gln Ser Gln Val Ala Asp Gly Thr Val Pro Gly
145                 150                 155                 160
Ala Lys Ala Ala Ala Ile Arg Lys Leu Glu His Glu Leu Gly Ile Pro
                165                 170                 175
Ala His Gln Leu Pro Ala Ser Ala Phe Arg Phe Leu Thr Arg Leu His
            180                 185                 190
Tyr Cys Ala Ala Asp Val Gln Pro Ala Ala Thr Gln Ser Ala Leu Trp
        195                 200                 205
Gly Glu His Glu Met Asp Tyr Ile Leu Phe Ile Arg Ala Asn Val Thr
    210                 215                 220
Leu Ala Pro Asn Pro Asp Glu Val Asp Glu Val Arg Tyr Val Thr Gln
225                 230                 235                 240
Glu Glu Leu Arg Gln Met Met Gln Pro Asp Asn Gly Leu Gln Trp Ser
                245                 250                 255
Pro Trp Phe Arg Ile Ile Ala Ala Arg Phe Leu Glu Arg Trp Trp Ala
            260                 265                 270

Asp Leu Asp Ala Ala Leu Asn Thr Asp Lys His Glu Asp Trp Gly Thr
        275                 280                 285

Val His His Ile Asn Glu Ala Gly Thr Gly Glu Asn Leu Tyr Phe Gln
    290                 295                 300

Gly Ser Gly Gly Gly Gly Ser Asp Tyr Lys Asp Asp Asp Asp Lys Gly
305                 310                 315                 320

Thr Gly

<210> 25
<211> 574
<212> PRT
<213> Artificial Sequence

<220>
<223> Description of Artificial Sequence: Synthetic
      polypeptide

<400> 25
Met Val Pro Arg Arg Ser Gly Asn Tyr Asn Pro Thr Ala Trp Asp Phe
1               5                   10                  15

Asn Tyr Ile Gln Ser Leu Asp Asn Gln Tyr Lys Lys Glu Arg Tyr Ser
            20                  25                  30

Thr Arg His Ala Glu Leu Thr Val Gln Val Lys Lys Leu Leu Glu Glu
        35                  40                  45

Glu Met Glu Ala Val Gln Lys Leu Glu Leu Ile Glu Asp Leu Lys Asn
    50                  55                  60

Leu Gly Ile Ser Tyr Pro Phe Lys Asp Asn Ile Gln Gln Ile Leu Asn
65                  70                  75                  80

Gln Ile Tyr Asn Glu His Lys Cys Cys His Asn Ser Glu Val Glu Glu
                85                  90                  95

Lys Asp Leu Tyr Phe Thr Ala Leu Arg Phe Arg Leu Leu Arg Gln Gln
            100                 105                 110

Gly Phe Glu Val Ser Gln Glu Val Phe Asp His Phe Lys Asn Glu Lys
        115                 120                 125

Gly Thr Asp Phe Lys Pro Asn Leu Ala Asp Asp Thr Lys Gly Leu Leu
    130                 135                 140

Gln Leu Tyr Glu Ala Ser Phe Leu Leu Arg Glu Ala Glu Asp Thr Leu
145                 150                 155                 160

Glu Leu Ala Arg Gln Phe Ser Thr Lys Leu Leu Gln Lys Lys Val Asp
                165                 170                 175

Glu Asn Gly Asp Asp Lys Ile Glu Asp Asn Leu Leu Leu Trp Ile Arg
            180                 185                 190

Arg Ser Leu Glu Leu Pro Leu His Trp Arg Val Gln Arg Leu Glu Ala
        195                 200                 205

Arg Gly Phe Leu Asp Ala Tyr Val Arg Arg Pro Asp Met Asn Pro Ile
    210                 215                 220

Val Phe Glu Leu Ala Lys Leu Asp Phe Asn Ile Thr Gln Ala Thr Gln
225                 230                 235                 240

Gln Glu Glu Leu Lys Asp Leu Ser Arg Trp Trp Asn Ser Thr Gly Leu
                245                 250                 255

Ala Glu Lys Leu Pro Phe Ala Arg Asp Arg Val Val Glu Ser Tyr Phe
            260                 265                 270

Trp Ala Met Gly Thr Phe Glu Pro His Gln Tyr Gly Tyr Gln Arg Glu
        275                 280                 285

Leu Val Ala Lys Ile Ile Ala Leu Ala Thr Val Val Asp Asp Val Tyr
    290                 295                 300

Asp Val Tyr Gly Thr Leu Glu Glu Leu Glu Leu Phe Thr Asp Ala Ile
305                 310                 315                 320

Arg Arg Trp Asp Arg Glu Ser Ile Asp Gln Leu Pro Tyr Tyr Met Gln
                325                 330                 335

Leu Cys Phe Leu Thr Val Asn Asn Phe Val Phe Glu Leu Ala His Asp
            340                 345                 350

Val Leu Lys Asp Lys Ser Phe Asn Cys Leu Pro His Leu Gln Arg Ser
        355                 360                 365

Trp Leu Asp Leu Ala Glu Ala Tyr Leu Val Glu Ala Lys Trp Tyr His
    370                 375                 380

Ser Arg Tyr Thr Pro Ser Leu Glu Glu Tyr Leu Asn Ile Ala Arg Val
385                 390                 395                 400

Ser Val Thr Cys Pro Thr Ile Val Ser Gln Met Tyr Phe Ala Leu Pro
                405                 410                 415

Ile Pro Ile Glu Lys Pro Val Ile Glu Ile Met Tyr Lys Tyr His Asp
            420                 425                 430

Ile Leu Tyr Leu Ser Gly Met Leu Leu Arg Leu Pro Asp Asp Leu Gly
        435                 440                 445

Thr Ala Ser Phe Glu Leu Lys Arg Gly Asp Val Gln Lys Ala Val Gln
    450                 455                 460

Cys Tyr Met Lys Glu Arg Asn Val Pro Glu Asn Glu Ala Arg Glu His
465                 470                 475                 480

Val Lys Phe Leu Ile Arg Glu Ala Ser Lys Gln Ile Asn Thr Ala Met
                485                 490                 495

Ala Thr Asp Cys Pro Phe Thr Glu Asp Phe Ala Val Ala Ala Ala Asn
            500                 505                 510

Leu Gly Arg Val Ala Asn Phe Val Tyr Val Asp Gly Asp Gly Phe Gly
        515                 520                 525

Val Gln His Ser Lys Ile Tyr Glu Gln Ile Gly Thr Leu Met Phe Glu
    530                 535                 540

Pro Tyr Pro Gly Thr Gly Glu Asn Leu Tyr Phe Gln Gly Ser Gly Gly
545                 550                 555                 560

Gly Gly Ser Asp Tyr Lys Asp Asp Asp Asp Lys Gly Thr Gly
                565                 570

<210> 26
<211> 573
<212> PRT
<213> Artificial Sequence

<220>
<223> Description of Artificial Sequence: Synthetic
      polypeptide

<400> 26
Met Val Pro Arg Arg Ser Gly Asn Tyr Lys Pro Thr Met Trp Asp Phe
1               5                   10                  15

Gln Phe Ile Gln Ser Val Asn Asn Leu Tyr Ala Gly Asp Lys Tyr Met
            20                  25                  30

Glu Arg Phe Asp Glu Val Lys Lys Glu Met Lys Lys Asn Leu Met Met
        35                  40                  45

Met Val Glu Gly Leu Ile Glu Glu Leu Asp Val Lys Leu Glu Leu Ile
    50                  55                  60

Asp Asn Leu Glu Arg Leu Gly Val Ser Tyr His Phe Lys Asn Glu Ile
65                  70                  75                  80

Met Gln Ile Leu Lys Ser Val His Gln Gln Ile Thr Cys Arg Asp Asn
                85                  90                  95

Ser Leu Tyr Ser Thr Ala Leu Lys Phe Arg Leu Leu Arg Gln His Gly
            100                 105                 110

Phe His Ile Ser Gln Asp Ile Phe Asn Asp Phe Lys Asp Met Asn Gly
        115                 120                 125

Asn Val Lys Gln Ser Ile Cys Asn Asp Thr Lys Gly Leu Leu Glu Leu
    130                 135                 140

Tyr Glu Ala Ser Phe Leu Ser Thr Glu Cys Glu Thr Thr Leu Lys Asn
145                 150                 155                 160

Phe Thr Glu Ala His Leu Lys Asn Tyr Val Tyr Ile Asn His Ser Cys
                165                 170                 175

Gly Asp Gln Tyr Asn Asn Ile Met Met Glu Leu Val Val His Ala Leu
            180                 185                 190

Glu Leu Pro Arg His Trp Met Met Pro Arg Leu Glu Thr Arg Trp Tyr
        195                 200                 205

Ile Ser Ile Tyr Glu Arg Met Pro Asn Ala Asn Pro Leu Leu Leu Glu
    210                 215                 220

Leu Ala Lys Leu Asp Phe Asn Ile Val Gln Ala Thr His Gln Gln Asp
225                 230                 235                 240

Leu Lys Ser Leu Ser Arg Trp Trp Lys Asn Met Cys Leu Ala Glu Lys
                245                 250                 255

Leu Ser Phe Ser Arg Asn Arg Leu Val Glu Asn Leu Phe Trp Ala Val
            260                 265                 270

Gly Thr Asn Phe Glu Pro Gln His Ser Tyr Phe Arg Arg Leu Ile Thr
        275                 280                 285

Lys Ile Ile Val Phe Val Gly Ile Ile Asp Asp Ile Tyr Asp Val Tyr
    290                 295                 300

Gly Lys Leu Asp Glu Leu Glu Leu Phe Thr Leu Ala Val Gln Arg Trp
305                 310                 315                 320

Asp Thr Lys Ala Met Glu Asp Leu Pro Tyr Tyr Met Gln Val Cys Tyr
                325                 330                 335

Leu Ala Leu Ile Asn Thr Thr Asn Asp Val Ala Tyr Glu Val Leu Arg
            340                 345                 350

Lys His Asn Ile Asn Val Leu Pro Tyr Leu Thr Lys Ser Trp Thr Asp
        355                 360                 365

Leu Cys Lys Ser Tyr Leu Gln Glu Ala Arg Trp Tyr Tyr Asn Gly Tyr
    370                 375                 380

Lys Pro Ser Leu Glu Glu Tyr Met Asp Asn Gly Trp Ile Ser Ile Ala
385                 390                 395                 400

Val Pro Met Val Leu Ala His Ala Leu Phe Leu Val Thr Asp Pro Ile
                405                 410                 415

Thr Lys Glu Ala Leu Glu Ser Leu Thr Asn Tyr Pro Asp Ile Ile Arg
            420                 425                 430

Cys Ser Ala Thr Ile Phe Arg Leu Asn Asp Asp Leu Gly Thr Ser Ser
        435                 440                 445

Asp Glu Leu Lys Arg Gly Asp Val Pro Lys Ser Ile Gln Cys Tyr Met
    450                 455                 460

Asn Glu Lys Gly Val Ser Glu Glu Glu Ala Arg Glu His Ile Arg Phe
465                 470                 475                 480

Leu Ile Lys Glu Thr Trp Lys Phe Met Asn Thr Ala His His Lys Glu
                485                 490                 495

Lys Ser Leu Phe Cys Glu Thr Phe Val Glu Ile Ala Lys Asn Ile Ala
            500                 505                 510

Thr Thr Ala His Cys Met Tyr Leu Lys Gly Asp Ser His Gly Ile Gln
        515                 520                 525

Asn Thr Asp Val Lys Asn Ser Ile Ser Asn Ile Leu Phe His Pro Ile

530                 535                 540

Ile Ile Gly Thr Gly Glu Asn Leu Tyr Phe Gln Gly Ser Gly Gly Gly
545                 550                 555                 560

Gly Ser Asp Tyr Lys Asp Asp Asp Asp Lys Gly Thr Gly
                565                 570

<210> 27
<211> 578
<212> PRT
<213> Artificial Sequence

<220>
<223> Description of Artificial Sequence: Synthetic
      polypeptide

<400> 27
Met Val Pro Arg Arg Ser Gly Asn Tyr Glu Pro Ser Ala Trp Asp Phe
1               5                   10                  15

Asn Tyr Leu Gln Ser Leu Asn Asn Tyr His His Lys Glu Glu Arg Tyr
            20                  25                  30

Leu Arg Arg Gln Ala Asp Leu Ile Glu Lys Val Lys Met Ile Leu Lys
        35                  40                  45

Glu Glu Lys Met Glu Ala Leu Gln Gln Leu Glu Leu Ile Asp Asp Leu
    50                  55                  60

Arg Asn Leu Gly Leu Ser Tyr Cys Phe Asp Asp Gln Ile Asn His Ile
65                  70                  75                  80

Leu Thr Thr Ile Tyr Asn Gln His Ser Cys Phe His Tyr His Glu Ala
                85                  90                  95

Ala Thr Ser Glu Glu Ala Asn Leu Tyr Phe Thr Ala Leu Gly Phe Arg
            100                 105                 110

Leu Leu Arg Glu His Gly Phe Lys Val Ser Gln Glu Val Phe Asp Arg
        115                 120                 125

Phe Lys Asn Glu Lys Gly Thr Asp Phe Arg Pro Asp Leu Val Asp Asp
    130                 135                 140

Thr Gln Gly Leu Leu Gln Leu Tyr Glu Ala Ser Phe Leu Leu Arg Glu
145                 150                 155                 160

Gly Glu Asp Thr Leu Glu Phe Ala Arg Gln Phe Ala Thr Lys Phe Leu
                165                 170                 175

Gln Lys Lys Val Glu Glu Lys Met Ile Glu Glu Glu Asn Leu Leu Ser
        180                 185                 190

Trp Thr Leu His Ser Leu Glu Leu Pro Leu His Trp Arg Ile Gln Arg
    195                 200                 205

Leu Glu Ala Lys Trp Phe Leu Asp Ala Tyr Ala Ser Arg Pro Asp Met
    210                 215                 220

Asn Pro Ile Ile Phe Glu Leu Ala Lys Leu Glu Phe Asn Ile Ala Gln
225                 230                 235                 240

Ala Leu Gln Gln Glu Glu Leu Lys Asp Leu Ser Arg Trp Trp Asn Asp
                245                 250                 255

Thr Gly Ile Ala Glu Lys Leu Pro Phe Ala Arg Asp Arg Ile Val Glu
        260                 265                 270

Ser His Tyr Trp Ala Ile Gly Thr Leu Glu Pro Tyr Gln Tyr Arg Tyr
    275                 280                 285

Gln Arg Ser Leu Ile Ala Lys Ile Ile Ala Leu Thr Thr Val Val Asp
    290                 295                 300

Asp Val Tyr Asp Val Tyr Gly Thr Leu Asp Glu Leu Gln Leu Phe Thr
305                 310                 315                 320

Asp Ala Ile Arg Arg Trp Asp Ile Glu Ser Ile Asn Gln Leu Pro Ser
                325                 330                 335

Tyr Met Gln Leu Cys Tyr Leu Ala Ile Tyr Asn Phe Val Ser Glu Leu
        340                 345                 350

Ala Tyr Asp Ile Phe Arg Asp Lys Gly Phe Asn Ser Leu Pro Tyr Leu
    355                 360                 365

His Lys Ser Trp Leu Asp Leu Val Glu Ala Tyr Phe Gln Glu Ala Lys
    370                 375                 380

Trp Tyr His Ser Gly Tyr Thr Pro Ser Leu Glu Gln Tyr Leu Asn Ile
385                 390                 395                 400

Ala Gln Ile Ser Val Ala Ser Pro Ala Ile Leu Ser Gln Ile Tyr Phe
                405                 410                 415

Thr Met Ala Gly Ser Ile Asp Lys Pro Val Ile Glu Ser Met Tyr Lys

420                 425                 430

Tyr Arg His Ile Leu Asn Leu Ser Gly Ile Leu Leu Arg Leu Pro Asp
        435                 440                 445

Asp Leu Gly Thr Ala Ser Asp Glu Leu Gly Arg Gly Asp Leu Ala Lys
    450                 455                 460

Ala Met Gln Cys Tyr Met Lys Glu Arg Asn Val Ser Glu Glu Glu Ala
465                 470                 475                 480

Arg Asp His Val Arg Phe Leu Asn Arg Glu Val Ser Lys Gln Met Asn
                485                 490                 495

Pro Ala Arg Ala Ala Asp Asp Cys Pro Phe Thr Asp Asp Phe Val Val
            500                 505                 510

Ala Ala Ala Asn Leu Gly Arg Val Ala Asp Phe Met Tyr Val Glu Gly
        515                 520                 525

Asp Gly Leu Gly Leu Gln Tyr Pro Ala Ile His Gln His Met Ala Glu
    530                 535                 540

Leu Leu Phe His Pro Tyr Ala Gly Thr Gly Glu Asn Leu Tyr Phe Gln
545                 550                 555                 560

Gly Ser Gly Gly Gly Gly Ser Asp Tyr Lys Asp Asp Asp Asp Lys Gly
                565                 570                 575

Thr Gly

<210> 28
<211> 578
<212> PRT
<213> Artificial Sequence

<220>
<223> Description of Artificial Sequence: Synthetic
      polypeptide

<400> 28
Met Val Pro Arg Arg Ser Gly Asn Tyr Gln Pro Ser Ala Trp Asp Phe
1               5                   10                  15

Asn Tyr Ile Gln Ser Leu Asn Asn Asn His Ser Lys Glu Glu Arg His
            20                  25                  30

Leu Glu Arg Lys Ala Lys Leu Ile Glu Glu Val Lys Met Leu Leu Glu
        35                  40                  45

Gln Glu Met Ala Ala Val Gln Gln Leu Glu Leu Ile Glu Asp Leu Lys
    50                  55                  60

Asn Leu Gly Leu Ser Tyr Leu Phe Gln Asp Glu Ile Lys Ile Ile Leu
65                  70                  75                  80

Asn Ser Ile Tyr Asn His His Lys Cys Phe His Asn Asn His Glu Gln
                85                  90                  95

Cys Ile His Val Asn Ser Asp Leu Tyr Phe Val Ala Leu Gly Phe Arg
            100                 105                 110

Leu Phe Arg Gln His Gly Phe Lys Val Ser Gln Glu Val Phe Asp Cys
        115                 120                 125

Phe Lys Asn Glu Glu Gly Ser Asp Phe Ser Ala Asn Leu Ala Asp Asp
    130                 135                 140

Thr Lys Gly Leu Leu Gln Leu Tyr Glu Ala Ser Tyr Leu Val Thr Glu
145                 150                 155                 160

Asp Glu Asp Thr Leu Glu Met Ala Arg Gln Phe Ser Thr Lys Ile Leu
                165                 170                 175

Gln Lys Lys Val Glu Glu Lys Met Ile Glu Lys Glu Asn Leu Leu Ser
            180                 185                 190

Trp Thr Leu His Ser Leu Glu Leu Pro Leu His Trp Arg Ile Gln Arg
        195                 200                 205

Leu Glu Ala Lys Trp Phe Leu Asp Ala Tyr Ala Ser Arg Pro Asp Met
    210                 215                 220

Asn Pro Ile Ile Phe Glu Leu Ala Lys Leu Glu Phe Asn Ile Ala Gln
225                 230                 235                 240

Ala Leu Gln Gln Glu Glu Leu Lys Asp Leu Ser Arg Trp Trp Asn Asp
                245                 250                 255

Thr Gly Ile Ala Glu Lys Leu Pro Phe Ala Arg Asp Arg Ile Val Glu
            260                 265                 270

Ser His Tyr Trp Ala Ile Gly Thr Leu Glu Pro Tyr Gln Tyr Arg Tyr
        275                 280                 285

Gln Arg Ser Leu Ile Ala Lys Ile Ile Ala Leu Thr Thr Val Val Asp

290                 295                 300

Asp Val Tyr Asp Val Tyr Gly Thr Leu Asp Glu Leu Gln Leu Phe Thr
305                 310                 315                 320

Asp Ala Ile Arg Arg Trp Asp Ile Glu Ser Ile Asn Gln Leu Pro Ser
                325                 330                 335

Tyr Met Gln Leu Cys Tyr Leu Ala Ile Tyr Asn Phe Val Ser Glu Leu
            340                 345                 350

Ala Tyr Asp Ile Phe Arg Asp Lys Gly Phe Asn Ser Leu Pro Tyr Leu
        355                 360                 365

His Lys Ser Trp Leu Asp Leu Val Glu Ala Tyr Phe Val Glu Ala Lys
    370                 375                 380

Trp Phe His Asp Gly Tyr Thr Pro Thr Leu Glu Glu Tyr Leu Asn Asn
385                 390                 395                 400

Ser Lys Ile Thr Ile Ile Cys Pro Ala Ile Val Ser Glu Ile Tyr Phe
                405                 410                 415

Ala Phe Ala Asn Ser Ile Asp Lys Thr Glu Val Glu Ser Ile Tyr Lys
            420                 425                 430

Tyr His Asp Ile Leu Tyr Leu Ser Gly Met Leu Ala Arg Leu Pro Asp
        435                 440                 445

Asp Leu Gly Thr Ser Ser Phe Glu Met Lys Arg Gly Asp Val Ala Lys
    450                 455                 460

Ala Ile Gln Cys Tyr Met Lys Glu His Asn Ala Ser Glu Glu Glu Ala
465                 470                 475                 480

Arg Glu His Ile Arg Phe Leu Met Arg Glu Ala Trp Lys His Met Asn
                485                 490                 495

Thr Ala Ala Ala Ala Asp Asp Cys Pro Phe Glu Ser Asp Leu Val Val
            500                 505                 510

Gly Ala Ala Ser Leu Gly Arg Val Ala Asn Phe Val Tyr Val Glu Gly
        515                 520                 525

Asp Gly Phe Gly Val Gln His Ser Lys Ile His Gln Gln Met Ala Glu
    530                 535                 540

Leu Leu Phe Tyr Pro Tyr Gln Gly Thr Gly Glu Asn Leu Tyr Phe Gln
545                 550                 555                 560

Gly Ser Gly Gly Gly Gly Ser Asp Tyr Lys Asp Asp Asp Asp Lys Gly
                565                 570                 575

Thr Gly

<210> 29
<211> 568
<212> PRT
<213> Artificial Sequence

<220>
<223> Description of Artificial Sequence: Synthetic
      polypeptide

<400> 29
Met Val Pro Arg Arg Ser Ala Asn Tyr Gln Ala Ser Ile Trp Asp Asp
1               5                   10                  15

Asn Phe Ile Gln Ser Leu Ala Ser Pro Tyr Ala Gly Glu Lys Tyr Ala
            20                  25                  30

Glu Lys Ala Glu Lys Leu Lys Thr Glu Val Lys Thr Met Ile Asp Gln
        35                  40                  45

Thr Arg Asp Glu Leu Lys Gln Leu Glu Leu Ile Asp Asn Leu Gln Arg
    50                  55                  60

Leu Gly Ile Cys His His Phe Gln Asp Leu Thr Lys Lys Ile Leu Gln
65                  70                  75                  80

Lys Ile Tyr Gly Glu Glu Arg Asn Gly Asp His Gln His Tyr Lys Glu
                85                  90                  95

Lys Gly Leu His Phe Thr Ala Leu Arg Phe Arg Ile Leu Arg Gln Asp
            100                 105                 110

Gly Tyr His Val Pro Gln Asp Val Phe Ser Ser Phe Met Asn Lys Ala
        115                 120                 125

Gly Asp Phe Glu Glu Ser Leu Ser Lys Asp Thr Lys Gly Leu Val Ser
    130                 135                 140

Leu Tyr Glu Ala Ser Tyr Leu Ser Met Glu Gly Glu Thr Ile Leu Asp
145                 150                 155                 160

Met Ala Lys Asp Phe Ser Ser His His Leu His Lys Met Val Glu Asp

                165                 170                 175

Ala Thr Asp Lys Arg Val Ala Asn Gln Ile Ile His Ser Leu Glu Met
            180                 185                 190

Pro Leu His Arg Arg Val Gln Lys Leu Glu Ala Ile Trp Phe Ile Gln
        195                 200                 205

Phe Tyr Glu Cys Gly Ser Asp Ala Asn Pro Thr Leu Val Glu Leu Ala
    210                 215                 220

Lys Leu Asp Phe Asn Met Val Gln Ala Thr Tyr Gln Glu Glu Leu Lys
225                 230                 235                 240

Arg Leu Ser Arg Trp Tyr Glu Glu Thr Gly Leu Gln Glu Lys Leu Ser
                245                 250                 255

Phe Ala Arg His Arg Leu Ala Glu Ala Phe Leu Trp Ser Met Gly Ile
            260                 265                 270

Ile Pro Glu Gly His Phe Gly Tyr Gly Arg Met His Leu Met Lys Ile
        275                 280                 285

Gly Ala Tyr Ile Thr Leu Leu Asp Asp Ile Tyr Asp Val Tyr Gly Thr
    290                 295                 300

Leu Glu Glu Leu Gln Val Leu Thr Glu Ile Ile Glu Arg Trp Asp Ile
305                 310                 315                 320

Asn Leu Leu Asp Gln Leu Pro Glu Tyr Met Gln Ile Phe Phe Leu Tyr
                325                 330                 335

Met Phe Asn Ser Thr Asn Glu Leu Ala Tyr Glu Ile Leu Arg Asp Gln
            340                 345                 350

Gly Ile Asn Val Ile Ser Asn Leu Lys Gly Leu Trp Val Glu Leu Ser
        355                 360                 365

Gln Cys Tyr Phe Lys Glu Ala Thr Trp Phe His Asn Gly Tyr Thr Pro
    370                 375                 380

Thr Thr Glu Glu Tyr Leu Asn Val Ala Cys Ile Ser Ala Ser Gly Pro
385                 390                 395                 400

Val Ile Leu Phe Ser Gly Tyr Phe Thr Thr Thr Asn Pro Ile Asn Lys
                405                 410                 415

His Glu Leu Gln Ser Leu Glu Arg His Ala His Ser Leu Ser Met Ile
            420                 425                 430

Leu Arg Leu Ala Asp Asp Leu Gly Thr Ser Ser Asp Glu Met Lys Arg
        435                 440                 445

Gly Asp Val Pro Lys Ala Ile Gln Cys Phe Met Asn Asp Thr Gly Cys
    450                 455                 460

Cys Glu Glu Glu Ala Arg Gln His Val Lys Arg Leu Ile Asp Ala Glu
465                 470                 475                 480

Trp Lys Lys Met Asn Lys Asp Ile Leu Met Glu Lys Pro Phe Lys Asn
                485                 490                 495

Phe Cys Pro Thr Ala Met Asn Leu Gly Arg Ile Ser Met Ser Phe Tyr
            500                 505                 510

Glu His Gly Asp Gly Tyr Gly Gly Pro His Ser Asp Thr Lys Lys Lys
        515                 520                 525

Met Val Ser Leu Phe Val Gln Pro Met Asn Ile Thr Ile Gly Thr Gly
    530                 535                 540

Glu Asn Leu Tyr Phe Gln Gly Ser Gly Gly Gly Gly Ser Asp Tyr Lys
545                 550                 555                 560

Asp Asp Asp Asp Lys Gly Thr Gly
                565

<210> 30
<211> 571
<212> PRT
<213> Artificial Sequence

<220>
<223> Description of Artificial Sequence: Synthetic
      polypeptide

<400> 30
Met Val Pro Arg Arg Ser Ala Asn Tyr Gln Pro Ser Ile Trp Asn His
1               5                   10                  15

Asp Tyr Ile Glu Ser Leu Arg Ile Glu Tyr Val Gly Glu Thr Cys Thr
            20                  25                  30

Arg Gln Ile Asn Val Leu Lys Glu Gln Val Arg Met Met Leu His Lys
        35                  40                  45

Val Val Asn Pro Leu Glu Gln Leu Glu Leu Ile Glu Ile Leu Gln Arg

50                  55                  60

Leu Gly Leu Ser Tyr His Phe Glu Glu Glu Ile Lys Arg Ile Leu Asp
65                  70                  75                  80

Gly Val Tyr Asn Asn Asp His Gly Gly Asp Thr Trp Lys Ala Glu Asn
                85                  90                  95

Leu Tyr Ala Thr Ala Leu Lys Phe Arg Leu Leu Arg Gln His Gly Tyr
            100                 105                 110

Ser Val Ser Gln Glu Val Phe Asn Ser Phe Lys Asp Glu Arg Gly Ser
        115                 120                 125

Phe Lys Ala Cys Leu Cys Glu Asp Thr Lys Gly Met Leu Ser Leu Tyr
    130                 135                 140

Glu Ala Ser Phe Phe Leu Ile Glu Gly Glu Asn Ile Leu Glu Glu Ala
145                 150                 155                 160

Arg Asp Phe Ser Thr Lys His Leu Glu Glu Tyr Val Lys Gln Asn Lys
                165                 170                 175

Glu Lys Asn Leu Ala Thr Leu Val Asn His Ser Leu Glu Phe Pro Leu
            180                 185                 190

His Trp Arg Met Pro Arg Leu Glu Ala Arg Trp Phe Ile Asn Ile Tyr
        195                 200                 205

Arg His Asn Gln Asp Val Asn Pro Ile Leu Leu Glu Phe Ala Glu Leu
    210                 215                 220

Asp Phe Asn Ile Val Gln Ala Ala His Gln Ala Asp Leu Lys Gln Val
225                 230                 235                 240

Ser Thr Trp Trp Lys Ser Thr Gly Leu Val Glu Asn Leu Ser Phe Ala
                245                 250                 255

Arg Asp Arg Pro Val Glu Asn Phe Phe Trp Thr Val Gly Leu Ile Phe
            260                 265                 270

Gln Pro Gln Phe Gly Tyr Cys Arg Arg Met Phe Thr Lys Val Phe Ala
        275                 280                 285

Leu Ile Thr Thr Ile Asp Asp Val Tyr Asp Val Tyr Gly Thr Leu Asp
    290                 295                 300

Glu Leu Glu Leu Phe Thr Asp Val Val Glu Arg Trp Asp Ile Asn Ala
305                 310                 315                 320

Met Asp Gln Leu Pro Asp Tyr Met Lys Ile Cys Phe Leu Thr Leu His
                325                 330                 335

Asn Ser Val Asn Glu Met Ala Leu Asp Thr Met Lys Glu Gln Arg Phe
            340                 345                 350

His Ile Ile Lys Tyr Leu Lys Lys Ala Trp Val Asp Leu Cys Arg Tyr
        355                 360                 365

Tyr Leu Val Glu Ala Lys Trp Tyr Ser Asn Lys Tyr Arg Pro Ser Leu
    370                 375                 380

Gln Glu Tyr Ile Glu Asn Ala Trp Ile Ser Ile Gly Ala Pro Thr Ile
385                 390                 395                 400

Leu Val His Ala Tyr Phe Phe Val Thr Asn Pro Ile Thr Lys Glu Ala
                405                 410                 415

Leu Asp Cys Leu Glu Glu Tyr Pro Asn Ile Ile Arg Trp Ser Ser Ile
            420                 425                 430

Ile Ala Arg Leu Ala Asp Asp Leu Gly Thr Ser Thr Asp Glu Leu Lys
        435                 440                 445

Arg Gly Asp Val Pro Lys Ala Ile Gln Cys Tyr Met Asn Glu Thr Gly
    450                 455                 460

Ala Ser Glu Glu Gly Ala Arg Glu Tyr Ile Lys Tyr Leu Ile Ser Ala
465                 470                 475                 480

Thr Trp Lys Lys Met Asn Lys Asp Arg Ala Ala Ser Ser Pro Phe Ser
                485                 490                 495

His Ile Phe Ile Glu Ile Ala Leu Asn Leu Ala Arg Met Ala Gln Cys
            500                 505                 510

Leu Tyr Gln His Gly Asp Gly His Gly Leu Gly Asn Arg Glu Thr Lys
        515                 520                 525

Asp Arg Ile Leu Ser Leu Leu Ile Gln Pro Ile Pro Leu Asn Lys Asp
    530                 535                 540

Gly Thr Gly Glu Asn Leu Tyr Phe Gln Gly Ser Gly Gly Gly Gly Ser
545                 550                 555                 560

Asp Tyr Lys Asp Asp Asp Asp Lys Gly Thr Gly
                565                 570

<210> 31
<211> 599
<212> PRT
<213> Artificial Sequence

<220>
<223> Description of Artificial Sequence: Synthetic
      polypeptide

<400> 31
Met Val Pro Arg Arg Ile Gly Asp Tyr His Ser Asn Leu Trp Asn Asp
1               5                   10                  15

Asp Phe Ile Gln Ser Leu Thr Thr Pro Tyr Gly Ala Pro Ser Tyr Ile
            20                  25                  30

Glu Arg Ala Asp Arg Leu Ile Ser Glu Val Lys Glu Met Phe Asn Arg
        35                  40                  45

Met Cys Met Glu Asp Gly Glu Leu Met Ser Pro Leu Asn Asp Leu Ile
    50                  55                  60

Gln Arg Leu Trp Thr Val Asp Ser Val Glu Arg Leu Gly Ile Asp Arg
65                  70                  75                  80

His Phe Lys Asn Glu Ile Lys Ala Ser Leu Asp Tyr Val Tyr Ser Tyr
                85                  90                  95

Trp Asn Glu Lys Gly Ile Gly Cys Gly Arg Gln Ser Val Val Thr Asp
            100                 105                 110

Leu Asn Ser Thr Ala Leu Gly Leu Arg Ile Leu Arg Gln His Gly Tyr
        115                 120                 125

Thr Val Ser Ser Glu Val Leu Lys Val Phe Glu Glu Glu Asn Gly Gln
    130                 135                 140

Phe Ala Cys Ser Pro Ser Gln Thr Glu Gly Glu Ile Arg Ser Phe Leu
145                 150                 155                 160

Asn Leu Tyr Arg Ala Ser Leu Ile Ala Phe Pro Gly Glu Lys Val Met
                165                 170                 175

Glu Glu Ala Gln Ile Phe Ser Ser Arg Tyr Leu Lys Glu Ala Val Gln
            180                 185                 190

Lys Ile Pro Val Ser Gly Leu Ser Arg Glu Ile Gly Asp Val Leu Glu
        195                 200                 205

Tyr Gly Trp His Thr Asn Leu Pro Arg Trp Glu Ala Arg Asn Tyr Met
    210                 215                 220

Asp Val Phe Gly Gln Asp Thr Asn Thr Ser Phe Asn Lys Asn Lys Met
225                 230                 235                 240

Gln Tyr Met Asn Thr Glu Lys Ile Leu Gln Leu Val Lys Leu Glu Phe
                245                 250                 255

Asn Ile Phe His Ser Leu Gln Gln Arg Glu Leu Gln Cys Leu Leu Arg
            260                 265                 270

Trp Trp Lys Glu Ser Gly Leu Pro Gln Leu Thr Phe Ala Arg His Arg
        275                 280                 285

His Val Glu Phe Tyr Thr Leu Ala Ser Cys Ile Ala Cys Glu Pro Lys
    290                 295                 300

His Ser Ala Phe Arg Leu Gly Phe Ala Lys Met Cys His Leu Val Thr
305                 310                 315                 320

Val Leu Asp Asp Val Tyr Asp Thr Phe Gly Lys Met Asp Glu Leu Glu
                325                 330                 335

Leu Phe Thr Ala Ala Val Lys Arg Trp Asp Leu Ser Glu Thr Glu Arg
            340                 345                 350

Leu Pro Glu Tyr Met Lys Gly Leu Tyr Val Val Val Phe Glu Thr Val
        355                 360                 365

Asn Glu Leu Ala Gln Glu Ala Glu Lys Thr Gln Gly Arg Asn Thr Leu
    370                 375                 380

Asn Tyr Val Arg Lys Ala Trp Glu Ala Tyr Phe Asp Ser Tyr Met Lys
385                 390                 395                 400

Glu Ala Glu Trp Ile Ser Thr Gly Tyr Leu Pro Thr Phe Glu Glu Tyr
                405                 410                 415

Cys Glu Asn Gly Lys Val Ser Ser Ala Tyr Arg Val Ala Ala Leu Gln
            420                 425                 430

Pro Ile Leu Thr Leu Asp Val Gln Leu Pro Asp Asp Ile Leu Lys Gly
        435                 440                 445

Ile Asp Phe Pro Ser Arg Phe Asn Asp Leu Ala Ser Ser Phe Leu Arg
    450                 455                 460

Leu Arg Gly Asp Thr Arg Cys Tyr Glu Ala Asp Arg Ala Arg Gly Glu
465                 470                 475                 480

Glu Ala Ser Cys Ile Ser Cys Tyr Met Lys Asp Asn Pro Gly Ser Thr
                485                 490                 495

Glu Glu Asp Ala Leu Asn His Ile Asn Ala Met Ile Asn Asp Ile Ile
            500                 505                 510

Arg Glu Leu Asn Trp Glu Phe Leu Lys Pro Asp Ser Asn Ile Pro Met
        515                 520                 525

Pro Ala Arg Lys His Ala Phe Asp Ile Thr Arg Ala Leu His His Leu
    530                 535                 540

Tyr Ile Tyr Arg Asp Gly Phe Ser Val Ala Asn Lys Glu Thr Lys Asn
545                 550                 555                 560

Leu Val Glu Lys Thr Leu Leu Glu Ser Met Leu Phe Gly Thr Gly Glu
                565                 570                 575

Asn Leu Tyr Phe Gln Gly Ser Gly Gly Gly Gly Ser Asp Tyr Lys Asp
            580                 585                 590

Asp Asp Asp Lys Gly Thr Gly
        595

<210> 32
<211> 576
<212> PRT
<213> Artificial Sequence

<220>
<223> Description of Artificial Sequence: Synthetic
      polypeptide

<400> 32
   Met Val Pro Arg Arg Ser Ala Asn Tyr Gln Pro Ser Arg Trp Asp His
   1               5                   10                  15
   His His Leu Leu Ser Val Glu Asn Lys Phe Ala Lys Asp Lys Arg Val
               20                  25                  30
   Arg Glu Arg Asp Leu Leu Lys Glu Lys Val Arg Lys Met Leu Asn Asp
           35                  40                  45
   Glu Gln Lys Thr Tyr Leu Asp Gln Leu Glu Phe Ile Asp Asp Leu Gln
       50                  55                  60
   Lys Leu Gly Val Ser Tyr His Phe Glu Ala Glu Ile Asp Asn Ile Leu
   65                  70                  75                  80
   Thr Ser Ser Tyr Lys Lys Asp Arg Thr Asn Ile Gln Glu Ser Asp Leu
                   85                  90                  95
   His Ala Thr Ala Leu Glu Phe Arg Leu Phe Arg Gln His Gly Phe Asn
               100                 105                 110
   Val Ser Glu Asp Val Phe Asp Val Phe Met Glu Asn Cys Gly Lys Phe
           115                 120                 125
   Asp Arg Asp Asp Ile Tyr Gly Leu Ile Ser Leu Tyr Glu Ala Ser Tyr
       130                 135                 140
   Leu Ser Thr Lys Leu Asp Lys Asn Leu Gln Ile Phe Ile Arg Pro Phe
   145                 150                 155                 160
   Ala Thr Gln Gln Leu Arg Asp Phe Val Asp Thr His Ser Asn Glu Asp
                   165                 170                 175
   Phe Gly Ser Cys Asp Met Val Glu Ile Val Val Gln Ala Leu Asp Met
               180                 185                 190
   Pro Tyr Tyr Trp Gln Met Arg Arg Leu Ser Thr Arg Trp Tyr Ile Asp
           195                 200                 205
   Val Tyr Gly Lys Arg Gln Asn Tyr Lys Asn Leu Val Val Val Glu Phe
       210                 215                 220
   Ala Lys Ile Asp Phe Asn Ile Val Gln Ala Ile His Gln Glu Glu Leu
   225                 230                 235                 240
   Lys Asn Val Ser Ser Trp Trp Met Glu Thr Gly Leu Gly Lys Gln Leu
                   245                 250                 255
   Tyr Phe Ala Arg Asp Arg Ile Val Glu Asn Tyr Phe Trp Thr Ile Gly
               260                 265                 270
   Gln Ile Gln Glu Pro Gln Tyr Gly Tyr Val Arg Gln Thr Met Thr Lys
           275                 280                 285
   Ile Asn Ala Leu Leu Thr Thr Ile Asp Asp Ile Tyr Asp Ile Tyr Gly
       290                 295                 300
   Thr Leu Glu Glu Leu Gln Leu Phe Thr Val Ala Phe Glu Asn Trp Asp
   305                 310                 315                 320
   Ile Asn Arg Leu Asp Glu Leu Pro Glu Tyr Met Arg Leu Cys Phe Leu
                   325                 330                 335
   Val Ile Tyr Asn Glu Val Asn Ser Ile Ala Cys Glu Ile Leu Arg Thr
               340                 345                 350
   Lys Asn Ile Asn Val Ile Pro Phe Leu Lys Lys Ser Trp Thr Asp Val
           355                 360                 365
   Ser Lys Ala Tyr Leu Val Glu Ala Lys Trp Tyr Lys Ser Gly His Lys
       370                 375                 380
   Pro Asn Leu Glu Glu Tyr Met Gln Asn Ala Arg Ile Ser Ile Ser Ser
   385                 390                 395                 400
   Pro Thr Ile Phe Val His Phe Tyr Cys Val Phe Ser Asp Gln Leu Ser
                   405                 410                 415
   Ile Gln Val Leu Glu Thr Leu Ser Gln His Gln Gln Asn Val Val Arg
               420                 425                 430
   Cys Ser Ser Ser Val Phe Arg Leu Ala Asn Asp Leu Val Thr Ser Pro
           435                 440                 445
   Asp Glu Leu Ala Arg Gly Asp Val Cys Lys Ser Ile Gln Cys Tyr Met
       450                 455                 460
   Ser Glu Thr Gly Ala Ser Glu Asp Lys Ala Arg Ser His Val Arg Gln
   465                 470                 475                 480
   Met Ile Asn Asp Leu Trp Asp Glu Met Asn Tyr Glu Lys Met Ala His
                   485                 490                 495
   Ser Ser Ser Ile Leu His His Asp Phe Met Glu Thr Val Ile Asn Leu
               500                 505                 510
   Ala Arg Met Ser Gln Cys Met Tyr Gln Tyr Gly Asp Gly His Gly Ser
           515                 520                 525
   Pro Glu Lys Ala Lys Ile Val Asp Arg Val Met Ser Leu Leu Phe Asn
       530                 535                 540
   Pro Ile Pro Leu Asp Gly Thr Gly Glu Asn Leu Tyr Phe Gln Gly Ser
   545                 550                 555                 560
   Gly Gly Gly Gly Ser Asp Tyr Lys Asp Asp Asp Asp Lys Gly Thr Gly
                   565                 570                 575

<210> 33
<211> 570
<212> PRT
<213> Artificial Sequence

<220>
<223> Description of Artificial Sequence: Synthetic
      polypeptide

<400> 33
Met Val Pro Arg Arg Ser Ala Asn Tyr Gln Pro Ser Leu Trp Gln His
1               5                   10                  15

Glu Tyr Leu Leu Ser Leu Gly Asn Thr Tyr Val Lys Glu Asp Asn Val
            20                  25                  30

Glu Arg Val Thr Leu Leu Lys Gln Glu Val Ser Lys Met Leu Asn Glu
        35                  40                  45

Thr Glu Gly Leu Leu Glu Gln Leu Glu Leu Ile Asp Thr Leu Gln Arg
    50                  55                  60

Leu Gly Val Ser Tyr His Phe Glu Gln Glu Ile Lys Lys Thr Leu Thr
65                  70                  75                  80

Asn Val His Val Lys Asn Val Arg Ala His Lys Asn Arg Ile Asp Arg
                85                  90                  95

Asn Arg Trp Gly Asp Leu Tyr Ala Thr Ala Leu Glu Phe Arg Leu Leu
            100                 105                 110

Arg Gln His Gly Phe Ser Ile Ala Gln Asp Val Phe Asp Gly Asn Ile
        115                 120                 125

Gly Val Asp Leu Asp Asp Lys Asp Ile Lys Gly Ile Leu Ser Leu Tyr
    130                 135                 140

Glu Ala Ser Tyr Leu Ser Thr Arg Ile Asp Thr Lys Leu Lys Glu Ser
145                 150                 155                 160

Ile Tyr Tyr Thr Thr Lys Arg Leu Arg Lys Phe Val Glu Val Asn Lys
                165                 170                 175

Asn Glu Thr Lys Ser Tyr Thr Leu Arg Arg Met Val Ile His Ala Leu
            180                 185                 190

Glu Met Pro Tyr His Arg Arg Val Gly Arg Leu Glu Ala Arg Trp Tyr
        195                 200                 205

Ile Glu Val Tyr Gly Glu Arg His Asp Met Asn Pro Ile Leu Leu Glu
    210                 215                 220

Leu Ala Lys Leu Asp Phe Asn Phe Val Gln Ala Ile His Gln Asp Glu
225                 230                 235                 240

Leu Lys Ser Leu Ser Ser Trp Trp Ser Lys Thr Gly Leu Thr Lys His
                245                 250                 255

Leu Asp Phe Val Arg Asp Arg Ile Thr Glu Gly Tyr Phe Ser Ser Val
            260                 265                 270

Gly Val Met Tyr Glu Pro Glu Phe Ala Tyr His Arg Gln Met Leu Thr
        275                 280                 285

Lys Val Phe Met Leu Ile Thr Thr Ile Asp Asp Ile Tyr Asp Ile Tyr
    290                 295                 300

Gly Thr Leu Glu Glu Leu Gln Leu Phe Thr Thr Ile Val Glu Lys Trp
305                 310                 315                 320

Asp Val Asn Arg Leu Glu Glu Leu Pro Asn Tyr Met Lys Leu Cys Phe
                325                 330                 335

Leu Cys Leu Val Asn Glu Ile Asn Gln Ile Gly Tyr Phe Val Leu Arg
            340                 345                 350

Asp Lys Gly Phe Asn Val Ile Pro Tyr Leu Lys Glu Ser Trp Ala Asp
        355                 360                 365

Met Cys Thr Thr Phe Leu Lys Glu Ala Lys Trp Tyr Lys Ser Gly Tyr
    370                 375                 380

Lys Pro Asn Phe Glu Glu Tyr Met Gln Asn Gly Trp Ile Ser Ser Ser
385                 390                 395                 400

Val Pro Thr Ile Leu Leu His Leu Phe Cys Leu Leu Ser Asp Gln Thr
                405                 410                 415

Leu Asp Ile Leu Gly Ser Tyr Asn His Ser Val Val Arg Ser Ser Ala
            420                 425                 430

Thr Ile Leu Arg Leu Ala Asn Asp Leu Ala Thr Ser Ser Glu Glu Leu
        435                 440                 445

Ala Arg Gly Asp Thr Met Lys Ser Val Gln Cys His Met His Glu Thr
    450                 455                 460

Gly Ala Ser Glu Ala Glu Ser Arg Ala Tyr Ile Gln Gly Ile Ile Gly
465                 470                 475                 480

Val Ala Trp Asp Asp Leu Asn Met Glu Lys Lys Ser Cys Arg Leu His
                485                 490                 495

Gln Gly Phe Leu Glu Ala Ala Ala Asn Leu Gly Arg Val Ala Gln Cys
            500                 505                 510

Val Tyr Gln Tyr Gly Asp Gly His Gly Cys Pro Asp Lys Ala Lys Thr
        515                 520                 525

Val Asn His Val Arg Ser Leu Leu Val His Pro Leu Pro Leu Asn Gly
    530                 535                 540

Thr Gly Glu Asn Leu Tyr Phe Gln Gly Ser Gly Gly Gly Gly Ser Asp
545                 550                 555                 560

Tyr Lys Asp Asp Asp Asp Lys Gly Thr Gly
                565                 570

<210> 34
<211> 583
<212> PRT
<213> Artificial Sequence

<220>
<223> Description of Artificial Sequence: Synthetic
      polypeptide

<400> 34
Met Val Pro Ala Ser Pro Pro Ala His Arg Ser Ser Lys Ala Ala Asp
1               5                   10                  15

Glu Glu Leu Pro Lys Ala Ser Ser Thr Phe His Pro Ser Leu Trp Gly
            20                  25                  30

Ser Phe Phe Leu Thr Tyr Gln Pro Pro Thr Ala Pro Gln Arg Ala Asn
        35                  40                  45

Met Lys Glu Arg Ala Glu Val Leu Arg Glu Arg Val Arg Lys Val Leu
    50                  55                  60

Lys Gly Ser Thr Thr Asp Gln Leu Pro Glu Thr Val Asn Leu Ile Leu
65                  70                  75                  80

Thr Leu Gln Arg Leu Gly Leu Gly Tyr Tyr Tyr Glu Asn Glu Ile Asp
                85                  90                  95

Lys Leu Leu His Gln Ile Tyr Ser Asn Ser Asp Tyr Asn Val Lys Asp
            100                 105                 110

Leu Asn Leu Val Ser Gln Arg Phe Tyr Leu Leu Arg Lys Asn Gly Tyr
        115                 120                 125

Asp Val Pro Ser Asp Val Phe Leu Ser Phe Lys Thr Glu Glu Gly Gly
    130                 135                 140

Phe Ala Cys Ala Ala Ala Asp Thr Arg Ser Leu Leu Ser Leu Tyr Asn
145                 150                 155                 160

Ala Ala Tyr Leu Arg Lys His Gly Glu Glu Val Leu Asp Glu Ala Ile
                165                 170                 175

Ser Ser Thr Arg Leu Arg Leu Gln Asp Leu Leu Gly Arg Leu Leu Pro
            180                 185                 190

Glu Ser Pro Phe Ala Lys Glu Val Ser Ser Ser Leu Arg Thr Pro Leu
        195                 200                 205

Phe Arg Arg Val Gly Ile Leu Glu Ala Arg Asn Tyr Ile Pro Ile Tyr
    210                 215                 220

Glu Thr Glu Ala Thr Arg Asn Glu Ala Val Leu Glu Leu Ala Lys Leu
225                 230                 235                 240

Asn Phe Asn Leu Gln Gln Leu Asp Phe Cys Glu Glu Leu Lys His Cys
                245                 250                 255

Ser Ala Trp Trp Asn Glu Met Ile Ala Lys Ser Lys Leu Thr Phe Val
            260                 265                 270

Arg Asp Arg Ile Val Glu Glu Tyr Phe Trp Met Asn Gly Ala Cys Tyr
        275                 280                 285

Asp Pro Pro Tyr Ser Leu Ser Arg Ile Ile Leu Thr Lys Ile Thr Gly
    290                 295                 300

Leu Ile Thr Ile Ile Asp Asp Met Phe Asp Thr His Gly Thr Thr Glu
305                 310                 315                 320

Asp Cys Met Lys Phe Ala Glu Ala Phe Gly Arg Trp Asp Glu Ser Ala
                325                 330                 335

Ile His Leu Leu Pro Glu Tyr Met Lys Asp Phe Tyr Ile Leu Met Leu
            340                 345                 350

Glu Thr Phe Gln Ser Phe Glu Asp Ala Leu Gly Pro Glu Lys Ser Tyr
        355                 360                 365

Arg Val Leu Tyr Leu Lys Gln Ala Met Glu Arg Leu Val Glu Leu Tyr
    370                 375                 380

Ser Lys Glu Ile Lys Trp Arg Asp Asp Asp Tyr Val Pro Thr Met Ser
385                 390                 395                 400

Glu His Leu Gln Val Ser Ala Glu Thr Ile Ala Thr Ile Ala Leu Thr
                405                 410                 415

Cys Ser Ala Tyr Ala Gly Met Gly Asp Met Ser Ile Arg Lys Glu Thr
            420                 425                 430

Phe Glu Trp Ala Leu Ser Phe Pro Gln Phe Ile Arg Thr Phe Gly Ser
        435                 440                 445

Phe Val Arg Leu Ser Asn Asp Val Val Ser Thr Lys Arg Glu Gln Thr
    450                 455                 460

Lys Asp His Ser Pro Ser Thr Val His Cys Tyr Met Lys Glu His Gly
465                 470                 475                 480

Thr Thr Met Asp Asp Ala Cys Glu Lys Ile Lys Glu Leu Ile Glu Asp
                485                 490                 495

Ser Trp Lys Asp Met Leu Glu Gln Ser Leu Ala Leu Lys Gly Leu Pro
            500                 505                 510

Lys Val Val Pro Gln Leu Val Phe Asp Phe Ser Arg Thr Thr Asp Asn
        515                 520                 525

Met Tyr Arg Asp Arg Asp Ala Leu Thr Ser Ser Glu Ala Leu Lys Glu
    530                 535                 540

Met Ile Gln Leu Leu Phe Val Glu Pro Ile Pro Glu Gly Thr Gly Glu
545                 550                 555                 560

Asn Leu Tyr Phe Gln Gly Ser Gly Gly Gly Gly Ser Asp Tyr Lys Asp
                565                 570                 575

Asp Asp Asp Lys Gly Thr Gly

            580

<210> 35
<211> 586
<212> PRT
<213> Artificial Sequence

<220>
<223> Description of Artificial Sequence: Synthetic
      polypeptide

<400> 35
Met Val Pro Glu Ala Leu Gly Asn Phe Asp Tyr Glu Ser Tyr Thr Asn
1               5                   10                  15

Phe Thr Lys Leu Pro Ser Ser Gln Trp Gly Asp Gln Phe Leu Lys Phe
            20                  25                  30

Ser Ile Ala Asp Ser Asp Phe Asp Val Leu Glu Arg Glu Ile Glu Val
        35                  40                  45

Leu Lys Pro Lys Val Arg Glu Asn Ile Phe Val Ser Ser Ser Thr Asp
    50                  55                  60

Lys Asp Ala Met Lys Lys Thr Ile Leu Ser Ile His Phe Leu Asp Ser
65                  70                  75                  80

Leu Gly Leu Ser Tyr His Phe Glu Lys Glu Ile Glu Glu Ser Leu Lys
                85                  90                  95

His Ala Phe Glu Lys Ile Glu Asp Leu Ile Ala Asp Glu Asn Lys Leu
            100                 105                 110

His Thr Ile Ser Thr Ile Phe Arg Val Phe Arg Thr Tyr Gly Tyr Tyr
        115                 120                 125

Met Ser Ser Asp Val Phe Lys Ile Phe Lys Gly Asp Asp Gly Lys Phe
    130                 135                 140

Lys Glu Ser Leu Ile Glu Asp Val Lys Gly Met Leu Ser Phe Tyr Glu
145                 150                 155                 160

Ala Val His Phe Gly Thr Thr Thr Asp His Ile Leu Asp Glu Ala Leu
                165                 170                 175

Ser Phe Thr Leu Asn His Leu Glu Ser Leu Ala Thr Gly Arg Arg Ala
            180                 185                 190

Ser Pro Pro His Ile Ser Lys Leu Ile Gln Asn Ala Leu His Ile Pro
        195                 200                 205

Gln His Arg Asn Ile Gln Ala Leu Val Ala Arg Glu Tyr Ile Ser Phe
    210                 215                 220

Tyr Glu His Glu Glu Asp His Asp Glu Thr Leu Leu Lys Leu Ala Lys
225                 230                 235                 240

Leu Asn Phe Lys Phe Leu Gln Leu His Tyr Phe Gln Glu Leu Lys Thr
                245                 250                 255

Ile Thr Met Trp Trp Thr Lys Leu Asp His Thr Ser Asn Leu Pro Pro
            260                 265                 270

Asn Phe Arg Glu Arg Thr Val Glu Thr Trp Phe Ala Ala Leu Met Met
        275                 280                 285

Tyr Phe Glu Pro Gln Phe Ser Leu Gly Arg Ile Met Ser Ala Lys Leu
    290                 295                 300

Tyr Leu Val Ile Thr Phe Leu Asp Asp Ala Cys Asp Thr Tyr Gly Ser
305                 310                 315                 320

Ile Ser Glu Val Glu Ser Leu Ala Asp Cys Leu Glu Arg Trp Asp Pro
                325                 330                 335

Asp Tyr Met Glu Asn Leu Gln Gly His Met Lys Thr Ala Phe Lys Phe
            340                 345                 350

Val Met Tyr Leu Phe Lys Glu Tyr Glu Glu Ile Leu Arg Ser Gln Gly
        355                 360                 365

Arg Ser Phe Val Leu Glu Lys Met Ile Glu Glu Phe Lys Ile Ile Ala
    370                 375                 380

Arg Lys Asn Leu Glu Leu Val Lys Trp Ala Arg Gly Gly His Val Pro
385                 390                 395                 400

Ser Phe Asp Glu Tyr Ile Glu Ser Gly Gly Ala Glu Ile Gly Thr Tyr
                405                 410                 415

Ala Thr Ile Ala Cys Ser Ile Met Gly Leu Gly Glu Ile Gly Lys Lys
            420                 425                 430

Glu Ala Phe Glu Trp Leu Ile Ser Arg Pro Lys Leu Val Arg Ile Leu
        435                 440                 445

Gly Ala Lys Thr Arg Leu Met Asp Asp Ile Ala Asp Phe Glu Glu Asp

450                 455                 460

Met Glu Lys Gly Tyr Thr Ala Asn Ala Leu Asn Tyr Tyr Met Asn Glu
465                 470                 475                 480

His Gly Val Thr Lys Glu Glu Ala Ser Arg Glu Leu Glu Lys Met Asn
                485                 490                 495

Gly Asp Met Asn Lys Ile Val Asn Glu Glu Cys Leu Lys Ile Thr Thr
            500                 505                 510

Met Pro Arg Arg Ile Leu Met Gln Ser Val Asn Tyr Ala Arg Ser Leu
        515                 520                 525

Asp Val Leu Tyr Thr Ala Asp Asp Val Tyr Asn His Arg Glu Gly Lys
    530                 535                 540

Leu Lys Glu Tyr Met Arg Leu Leu Leu Val Asp Pro Ile Leu Leu Gly
545                 550                 555                 560

Thr Gly Glu Asn Leu Tyr Phe Gln Gly Ser Gly Gly Gly Gly Ser Asp
                565                 570                 575

Tyr Lys Asp Asp Asp Asp Lys Gly Thr Gly
            580                 585

<210> 36
<211> 553
<212> PRT
<213> Artificial Sequence

<220>
<223> Description of Artificial Sequence: Synthetic
      polypeptide

<400> 36
Met Val Pro Glu Ser Gln Thr Thr Phe Lys Tyr Glu Ser Leu Ala Phe
1               5                   10                  15

Thr Lys Leu Ser His Cys Gln Trp Thr Asp Tyr Phe Leu Ser Val Pro
            20                  25                  30

Ile Asp Glu Ser Glu Leu Asp Val Ile Thr Arg Glu Ile Asp Ile Leu
        35                  40                  45

Lys Pro Glu Val Met Glu Leu Leu Ser Ser Gln Gly Asp Asp Glu Thr
    50                  55                  60

Ser Lys Arg Lys Val Leu Leu Ile Gln Leu Leu Leu Ser Leu Gly Leu
65                  70                  75                  80

Ala Phe His Phe Glu Asn Glu Ile Lys Asn Ile Leu Glu His Ala Phe
                85                  90                  95

Arg Lys Ile Asp Asp Ile Thr Gly Asp Glu Lys Asp Leu Ser Thr Ile
            100                 105                 110

Ser Ile Met Phe Arg Val Phe Arg Thr Tyr Gly His Asn Leu Pro Ser
        115                 120                 125

Ser Val Phe Lys Arg Phe Thr Gly Asp Asp Gly Lys Phe Gln Gln Ser
    130                 135                 140

Leu Thr Glu Asp Ala Lys Gly Ile Leu Ser Leu Tyr Glu Ala Ala His
145                 150                 155                 160

Leu Gly Thr Thr Thr Asp Tyr Ile Leu Asp Glu Ala Leu Lys Phe Thr
                165                 170                 175

Ser Ser His Leu Lys Ser Leu Leu Ala Gly Gly Thr Cys Arg Pro His
            180                 185                 190

Ile Leu Arg Leu Ile Arg Asn Thr Leu Tyr Leu Pro Gln Arg Trp Asn
        195                 200                 205

Met Glu Ala Val Ile Ala Arg Glu Tyr Ile Ser Phe Tyr Glu Gln Glu
    210                 215                 220

Glu Asp His Asp Lys Met Leu Leu Arg Leu Ala Lys Leu Asn Phe Lys
225                 230                 235                 240

Leu Leu Gln Leu His Tyr Ile Lys Glu Leu Lys Ser Phe Ile Lys Trp
                245                 250                 255

Trp Met Glu Leu Gly Leu Thr Ser Lys Trp Pro Ser Gln Phe Arg Glu
            260                 265                 270

Arg Ile Val Glu Ala Trp Leu Ala Gly Leu Met Met Tyr Phe Glu Pro
        275                 280                 285

Gln Phe Ser Gly Gly Arg Val Ile Ala Ala Lys Phe Asn Tyr Leu Leu
    290                 295                 300

Thr Ile Leu Asp Asp Ala Cys Asp His Tyr Phe Ser Ile His Glu Leu
305                 310                 315                 320

Thr Arg Leu Val Ala Cys Val Glu Arg Trp Ser Pro Asp Gly Ile Asp

325                 330                 335

Thr Leu Glu Asp Ile Ser Arg Ser Val Phe Lys Leu Met Leu Asp Val
            340                 345                 350

Phe Asp Asp Ile Gly Lys Gly Val Arg Ser Glu Gly Ser Ser Tyr His
        355                 360                 365

Leu Lys Glu Met Leu Glu Glu Leu Asn Thr Leu Val Arg Ala Asn Leu
    370                 375                 380

Asp Leu Val Lys Trp Ala Arg Gly Ile Gln Thr Ala Gly Lys Glu Ala
385                 390                 395                 400

Tyr Glu Trp Val Arg Ser Arg Pro Arg Leu Ile Lys Ser Leu Ala Ala
                405                 410                 415

Lys Gly Arg Leu Met Asp Asp Ile Thr Asp Phe Asp Ser Asp Met Ser
            420                 425                 430

Asn Gly Phe Ala Ala Asn Ala Ile Asn Tyr Tyr Met Lys Gln Phe Val
        435                 440                 445

Val Thr Lys Glu Glu Ala Ile Leu Glu Cys Gln Arg Met Ile Val Asp
    450                 455                 460

Ile Asn Lys Thr Ile Asn Glu Glu Leu Leu Lys Thr Thr Ser Val Pro
465                 470                 475                 480

Gly Arg Val Leu Lys Gln Ala Leu Asn Phe Gly Arg Leu Leu Glu Leu
                485                 490                 495

Leu Tyr Thr Lys Ser Asp Asp Ile Tyr Asn Cys Ser Glu Gly Lys Leu
            500                 505                 510

Lys Glu Tyr Ile Val Thr Leu Leu Ile Asp Pro Ile Arg Leu Gly Thr
        515                 520                 525

Gly Glu Asn Leu Tyr Phe Gln Gly Ser Gly Gly Gly Gly Ser Asp Tyr
    530                 535                 540

Lys Asp Asp Asp Asp Lys Gly Thr Gly
545                 550

<210> 37
<211> 500
<212> PRT
<213> Artificial Sequence

<220>
<223> Description of Artificial Sequence: Synthetic
      polypeptide

<400> 37
Met Val Pro Glu Ser Gln Thr Lys Phe Asp Tyr Glu Ser Leu Ala Phe
1               5                   10                  15

Thr Lys Leu Ser His Ser Gln Trp Thr Asp Tyr Phe Leu Ser Val Pro
            20                  25                  30

Ile Asp Asp Ser Glu Leu Asp Ala Ile Thr Arg Glu Ile Asp Ile Ile
        35                  40                  45

Lys Pro Glu Val Arg Lys Leu Leu Ser Ser Lys Gly Asp Asp Glu Thr
    50                  55                  60

Ser Lys Arg Lys Val Leu Leu Ile Gln Ser Leu Leu Ser Leu Gly Leu
65                  70                  75                  80

Ala Phe His Phe Glu Asn Glu Ile Lys Asp Ile Leu Glu Asp Ala Phe
                85                  90                  95

Arg Arg Ile Asp Asp Ile Thr Gly Asp Glu Asn Asp Leu Ser Thr Ile
            100                 105                 110

Ser Ile Met Phe Arg Val Phe Arg Thr Tyr Gly His Asn Leu Pro Ser
        115                 120                 125

Ser Val Phe Lys Arg Phe Thr Gly Asp Asp Gly Lys Phe Glu Arg Ser
    130                 135                 140

Leu Thr Glu Asp Ala Lys Gly Ile Leu Ser Leu Tyr Glu Ala Ala His
145                 150                 155                 160

Leu Gly Thr Thr Thr Asp Tyr Ile Leu Asp Glu Ala Leu Glu Phe Thr
                165                 170                 175

Ser Ser His Leu Lys Ser Leu Leu Val Gly Gly Met Cys Arg Pro His
            180                 185                 190

Ile Leu Arg Leu Ile Arg Asn Thr Leu Tyr Leu Pro Gln Arg Trp Asn
        195                 200                 205

Met Glu Ala Val Ile Ala Arg Glu Tyr Ile Ser Phe Tyr Glu Gln Glu
    210                 215                 220

Glu Asp His Asp Lys Met Leu Leu Arg Leu Ala Lys Leu Asn Phe Lys

225                 230                 235                 240

Leu Leu Gln Leu His Tyr Ile Lys Glu Leu Lys Thr Phe Ile Lys Trp
                245                 250                 255

Trp Met Glu Leu Gly Leu Thr Ser Lys Trp Pro Ser Gln Phe Arg Glu
            260                 265                 270

Arg Ile Val Glu Ala Trp Leu Ala Gly Leu Met Met Tyr Phe Glu Pro
        275                 280                 285

Gln Phe Ser Gly Gly Arg Val Ile Ala Ala Lys Phe Asn Tyr Leu Leu
    290                 295                 300

Thr Ile Leu Asp Asp Ala Cys Asp His Tyr Phe Ser Ile Pro Glu Leu
305                 310                 315                 320

Thr Arg Leu Val Asp Cys Val Glu Arg Trp Asn His Asp Gly Ile His
                325                 330                 335

Thr Leu Glu Asp Ile Ser Arg Ile Ile Phe Lys Leu Ala Leu Asp Val
            340                 345                 350

Phe Asp Asp Ile Gly Arg Gly Val Arg Ser Lys Gly Cys Ser Tyr Tyr
        355                 360                 365

Leu Lys Glu Met Leu Glu Glu Leu Lys Ile Leu Val Arg Ala Asn Leu
    370                 375                 380

Asp Leu Val Lys Trp Ala Arg Gly Asn Gln Leu Pro Ser Phe Glu Glu
385                 390                 395                 400

His Val Glu Val Gly Gly Ile Ala Leu Thr Thr Tyr Ala Thr Leu Met
                405                 410                 415

Tyr Ser Phe Val Gly Met Gly Glu Ala Val Gly Lys Glu Ala Tyr Glu
            420                 425                 430

Trp Val Arg Ser Arg Pro Arg Leu Ile Lys Ser Leu Ala Ala Lys Gly
        435                 440                 445

Arg Leu Met Asp Asp Ile Thr Asp Phe Glu Val Lys Ile Ile Asn Leu
    450                 455                 460

Phe Phe Asp Leu Leu Leu Phe Val Phe Gly Thr Gly Glu Asn Leu Tyr
465                 470                 475                 480

Phe Gln Gly Ser Gly Gly Gly Gly Ser Asp Tyr Lys Asp Asp Asp Asp
                485                 490                 495

Lys Gly Thr Gly
            500

<210> 38
<211> 574
<212> PRT
<213> Artificial Sequence

<220>
<223> Description of Artificial Sequence: Synthetic
      polypeptide

<400> 38
Met Val Pro Ala Ala Phe Thr Ala Asn Ala Val Asp Met Arg Pro Pro
1               5                   10                  15

Val Ile Thr Ile His Pro Arg Ser Lys Asp Ile Phe Ser Gln Phe Ser
            20                  25                  30

Leu Asp Asp Lys Leu Gln Lys Gln Tyr Ala Gln Gly Ile Glu Ala Leu
        35                  40                  45

Lys Glu Glu Ala Arg Ser Met Leu Met Ala Ala Lys Ser Ala Lys Val
    50                  55                  60

Met Ile Leu Ile Asp Thr Leu Glu Arg Leu Gly Leu Gly Tyr His Phe
65                  70                  75                  80

Glu Lys Glu Ile Glu Glu Lys Leu Glu Ala Ile Tyr Lys Lys Glu Asp
                85                  90                  95

Gly Asp Asp Tyr Asp Leu Phe Thr Thr Ala Leu Arg Phe Arg Leu Leu
            100                 105                 110

Arg Gln His Gln Arg Arg Val Pro Cys Ser Val Phe Asp Lys Phe Met
        115                 120                 125

Asn Lys Glu Gly Lys Phe Glu Glu Glu Pro Leu Ile Ser Asp Val Glu
    130                 135                 140

Gly Leu Leu Ser Leu Tyr Asp Ala Ala Tyr Leu Gln Ile His Gly Glu
145                 150                 155                 160

His Ile Leu Gln Glu Ala Leu Ile Phe Thr Thr His His Leu Thr Arg
                165                 170                 175

Ile Glu Pro Gln Leu Asp Asp His Ser Pro Leu Lys Leu Lys Leu Asn

            180                 185                 190

Arg Ala Leu Glu Phe Pro Phe Tyr Arg Glu Ile Pro Ile Ile Tyr Ala
        195                 200                 205

His Phe Tyr Ile Ser Val Tyr Glu Arg Asp Asp Ser Arg Asp Glu Val
    210                 215                 220

Leu Leu Lys Met Ala Lys Leu Ser Tyr Asn Phe Leu Gln Asn Leu Tyr
225                 230                 235                 240

Lys Lys Glu Leu Ser Gln Leu Ser Arg Trp Trp Asn Lys Leu Glu Leu
                245                 250                 255

Ile Pro Asn Leu Pro Tyr Ile Arg Asp Ser Val Ala Gly Ala Tyr Leu
            260                 265                 270

Trp Ala Val Ala Leu Tyr Phe Glu Pro Gln Tyr Ser Asp Val Arg Met
        275                 280                 285

Ala Ile Ala Lys Leu Ile Gln Ile Ala Ala Ala Val Asp Asp Thr Tyr
    290                 295                 300

Asp Asn Tyr Ala Thr Ile Arg Glu Ala Gln Leu Leu Thr Glu Ala Leu
305                 310                 315                 320

Glu Arg Leu Asn Val His Glu Ile Asp Thr Leu Pro Asp Tyr Met Lys
                325                 330                 335

Ile Val Tyr Arg Phe Val Met Ser Trp Ser Glu Asp Phe Glu Arg Asp
            340                 345                 350

Ala Thr Ile Lys Glu Gln Met Leu Ala Thr Pro Tyr Phe Lys Ala Glu
        355                 360                 365

Met Lys Lys Leu Gly Arg Ala Tyr Asn Gln Glu Leu Lys Trp Val Met
    370                 375                 380

Glu Arg Gln Leu Pro Ser Phe Glu Glu Tyr Met Lys Asn Ser Glu Ile
385                 390                 395                 400

Thr Ser Gly Val Tyr Ile Met Phe Thr Val Ile Ser Pro Tyr Leu Asn
                405                 410                 415

Ser Ala Thr Gln Lys Asn Ile Asp Trp Leu Leu Ser Gln Pro Arg Leu
            420                 425                 430

Ala Ser Ser Thr Ala Ile Val Met Arg Cys Cys Asn Asp Leu Gly Ser
        435                 440                 445

Asn Gln Arg Glu Ser Lys Gly Gly Glu Val Met Thr Ser Leu Asp Cys
    450                 455                 460

Tyr Met Lys Gln His Gly Ala Ser Lys Gln Glu Thr Ile Ser Lys Phe
465                 470                 475                 480

Lys Leu Ile Ile Glu Asp Glu Trp Lys Asn Leu Asn Glu Glu Trp Ala
                485                 490                 495

Ala Thr Thr Cys Leu Pro Lys Val Met Val Glu Ile Phe Arg Asn Tyr
            500                 505                 510

Ala Arg Ile Ala Gly Phe Cys Tyr Lys Asn Asn Gly Asp Ala Tyr Thr
        515                 520                 525

Ser Pro Lys Ile Val Gln Gln Cys Phe Asp Ala Leu Phe Val Asn Pro
    530                 535                 540

Leu Arg Ile Gly Thr Gly Glu Asn Leu Tyr Phe Gln Gly Ser Gly Gly
545                 550                 555                 560

Gly Gly Ser Asp Tyr Lys Asp Asp Asp Asp Lys Gly Thr Gly
                565                 570

<210> 39
<211> 605
<212> PRT
<213> Artificial Sequence

<220>
<223> Description of Artificial Sequence: Synthetic
      polypeptide

<400> 39
Met Val Pro Glu Phe Arg Val His Leu Gln Ala Asp Asn Glu Gln Lys
1               5                   10                  15

Ile Phe Gln Asn Gln Met Lys Pro Glu Pro Glu Ala Ser Tyr Leu Ile
            20                  25                  30

Asn Gln Arg Arg Ser Ala Asn Tyr Lys Pro Asn Ile Trp Lys Asn Asp
        35                  40                  45

Phe Leu Asp Gln Ser Leu Ile Ser Lys Tyr Asp Gly Asp Glu Tyr Arg
    50                  55                  60

Lys Leu Ser Glu Lys Leu Ile Glu Glu Val Lys Ile Tyr Ile Ser Ala

65                  70                  75                  80

Glu Thr Met Asp Leu Val Ala Lys Leu Glu Leu Ile Asp Ser Val Arg
                85                  90                  95

Lys Leu Gly Leu Ala Asn Leu Phe Glu Lys Glu Ile Lys Glu Ala Leu
            100                 105                 110

Asp Ser Ile Ala Ala Ile Glu Ser Asp Asn Leu Gly Thr Arg Asp Asp
        115                 120                 125

Leu Tyr Gly Thr Ala Leu His Phe Lys Ile Leu Arg Gln His Gly Tyr
    130                 135                 140

Lys Val Ser Gln Asp Ile Phe Gly Arg Phe Met Asp Glu Lys Gly Thr
145                 150                 155                 160

Leu Glu Asn His His Phe Ala His Leu Lys Gly Met Leu Glu Leu Phe
                165                 170                 175

Glu Ala Ser Asn Leu Gly Phe Glu Gly Glu Asp Ile Leu Asp Glu Ala
            180                 185                 190

Lys Ala Ser Leu Thr Leu Ala Leu Arg Asp Ser Gly His Ile Cys Tyr
        195                 200                 205

Pro Asp Ser Asn Leu Ser Arg Asp Val Val His Ser Leu Glu Leu Pro
    210                 215                 220

Ser His Arg Arg Val Gln Trp Phe Asp Val Lys Trp Gln Ile Asn Ala
225                 230                 235                 240

Tyr Glu Lys Asp Ile Cys Arg Val Asn Ala Thr Leu Leu Glu Leu Ala
                245                 250                 255

Lys Leu Asn Phe Asn Val Val Gln Ala Gln Leu Gln Lys Asn Leu Arg
            260                 265                 270

Glu Ala Ser Arg Trp Trp Ala Asn Leu Gly Phe Ala Asp Asn Leu Lys
        275                 280                 285

Phe Ala Arg Asp Arg Leu Val Glu Cys Phe Ser Cys Ala Val Gly Val
    290                 295                 300

Ala Phe Glu Pro Glu His Ser Ser Phe Arg Ile Cys Leu Thr Lys Val
305                 310                 315                 320

Ile Asn Leu Val Leu Ile Ile Asp Asp Val Tyr Asp Ile Tyr Gly Ser
                325                 330                 335

Glu Glu Glu Leu Lys His Phe Thr Asn Ala Val Asp Arg Trp Asp Ser
            340                 345                 350

Arg Glu Thr Glu Gln Leu Pro Glu Cys Met Lys Met Cys Phe Gln Val
        355                 360                 365

Leu Tyr Asn Thr Thr Cys Glu Ile Ala Arg Glu Ile Glu Glu Glu Asn
    370                 375                 380

Gly Trp Asn Gln Val Leu Pro Gln Leu Thr Lys Val Trp Ala Asp Phe
385                 390                 395                 400

Cys Lys Ala Leu Leu Val Glu Ala Glu Trp Tyr Asn Lys Ser His Ile
                405                 410                 415

Pro Thr Leu Glu Glu Tyr Leu Arg Asn Gly Cys Ile Ser Ser Ser Val
            420                 425                 430

Ser Val Leu Leu Val His Ser Phe Phe Ser Ile Thr His Glu Gly Thr
        435                 440                 445

Lys Glu Met Ala Asp Phe Leu His Lys Asn Glu Asp Leu Leu Tyr Asn
    450                 455                 460

Ile Ser Leu Ile Val Arg Leu Asn Asn Asp Leu Gly Thr Ser Ala Ala
465                 470                 475                 480

Glu Gln Glu Arg Gly Asp Ser Pro Ser Ser Ile Val Cys Tyr Met Arg
                485                 490                 495

Glu Val Asn Ala Ser Glu Glu Thr Ala Arg Lys Asn Ile Lys Gly Met
            500                 505                 510

Ile Pro Arg Thr Trp Lys Lys Val Asn Gly Lys Cys Phe Thr Thr Asn
        515                 520                 525

Gln Val Pro Phe Leu Ser Ser Phe Met Asn Asn Ala Thr Asn Met Ala
    530                 535                 540

Arg Val Ala His Ser Leu Tyr Lys Asp Gly Asp Gly Phe Gly Asp Gln
545                 550                 555                 560

Glu Lys Gly Pro Arg Thr His Ile Leu Ser Leu Leu Phe Gln Pro Leu
                565                 570                 575

Val Asn Gly Thr Gly Glu Asn Leu Tyr Phe Gln Gly Ser Gly Gly Gly
                580                 585                 590

Gly Ser Asp Tyr Lys Asp Asp Asp Asp Lys Gly Thr Gly
        595                 600                 605

<210> 40
<211> 590
<212> PRT
<213> Artificial Sequence

<220>
<223> Description of Artificial Sequence: Synthetic
      polypeptide

<400> 40
Met Val Pro Ser Ser Asn Val Ser Ala Ile Pro Asn Ser Phe Glu Leu
1               5                   10                  15

Ile Arg Arg Ser Ala Gln Phe Gln Ala Ser Val Trp Gly Asp Tyr Phe
            20                  25                  30

Leu Ser Tyr His Ser Leu Pro Pro Glu Lys Gly Asn Lys Val Met Glu
        35                  40                  45

Lys Gln Thr Glu Glu Leu Lys Glu Glu Ile Lys Met Glu Leu Val Ser
    50                  55                  60

Thr Thr Lys Asp Glu Pro Glu Lys Leu Arg Leu Ile Asp Leu Ile Gln
65                  70                  75                  80

Arg Leu Gly Val Cys Tyr His Phe Glu Asn Glu Ile Asn Asn Ile Leu
                85                  90                  95

Gln Gln Leu His His Ile Thr Ile Thr Ser Glu Lys Asn Gly Asp Asp
            100                 105                 110

Asn Pro Tyr Asn Met Thr Leu Cys Phe Arg Leu Leu Arg Gln Gln Gly
        115                 120                 125

Tyr Asn Val Ser Ser Glu Pro Phe Asp Arg Phe Arg Gly Lys Trp Glu
    130                 135                 140

Ser Ser Tyr Asp Asn Asn Val Glu Glu Leu Leu Ser Leu Tyr Glu Ala
145                 150                 155                 160

Ser Gln Leu Arg Met Gln Gly Glu Glu Ala Leu Asp Glu Ala Phe Cys
                165                 170                 175

Phe Ala Thr Ala Gln Leu Glu Ala Ile Val Gln Asp Pro Thr Thr Asp
        180                 185                 190

Pro Met Val Ala Ala Glu Ile Arg Gln Ala Leu Lys Trp Pro Met Tyr
    195                 200                 205

Lys Asn Leu Pro Arg Leu Lys Ala Arg His His Ile Gly Leu Tyr Ser
    210                 215                 220

Glu Lys Pro Trp Arg Asn Glu Ser Leu Leu Asn Phe Ala Lys Met Asp
225                 230                 235                 240

Phe Asn Lys Leu Gln Asn Leu His Gln Thr Glu Ile Ala Tyr Ile Ser
                245                 250                 255

Lys Trp Trp Asp Asp Tyr Gly Phe Ala Glu Lys Leu Ser Phe Ala Arg
            260                 265                 270

Asn Arg Ile Val Glu Gly Tyr Phe Phe Ala Leu Gly Ile Phe Phe Glu
        275                 280                 285

Pro Gln Leu Leu Thr Ala Arg Leu Ile Met Thr Lys Val Ile Ala Ile
    290                 295                 300

Gly Ser Met Leu Asp Asp Ile Tyr Asp Val Tyr Gly Thr Phe Glu Glu
305                 310                 315                 320

Leu Lys Leu Leu Thr Leu Ala Leu Glu Arg Trp Asp Lys Ser Glu Thr
                325                 330                 335

Lys Gln Leu Pro Asn Tyr Met Lys Met Tyr Tyr Glu Ala Leu Leu Asp
            340                 345                 350

Val Phe Glu Glu Ile Glu Gln Glu Met Ser Gln Lys Glu Thr Glu Thr
        355                 360                 365

Thr Pro Tyr Cys Ile His His Met Lys Glu Ala Thr Lys Glu Leu Gly
    370                 375                 380

Arg Val Phe Leu Val Glu Ala Thr Trp Cys Lys Glu Gly Tyr Thr Pro
385                 390                 395                 400

Lys Val Glu Glu Tyr Leu Asp Ile Ala Leu Ile Ser Phe Gly His Lys
                405                 410                 415

Leu Leu Met Val Thr Ala Leu Leu Gly Met Gly Ser His Met Ala Thr
            420                 425                 430

Gln Gln Ile Val Gln Trp Ile Thr Ser Met Pro Asn Ile Leu Lys Ala
        435                 440                 445

Ser Ala Val Ile Cys Arg Leu Met Asn Asp Ile Val Ser His Lys Phe
    450                 455                 460

Glu Gln Glu Arg Gly His Val Ala Ser Ala Ile Glu Cys Tyr Met Glu
465                 470                 475                 480

Gln Asn His Leu Ser Glu Tyr Glu Ala Leu Ile Ala Leu Arg Lys Gln
                485                 490                 495

Ile Asp Asp Leu Trp Lys Asp Met Val Glu Asn Tyr Cys Ala Val Ile
            500                 505                 510

Thr Glu Asp Glu Val Pro Arg Gly Val Leu Met Arg Val Leu Asn Leu
        515                 520                 525

Thr Arg Leu Phe Asn Val Ile Tyr Lys Asp Gly Asp Gly Tyr Thr Gln
    530                 535                 540

Ser His Gly Ser Thr Lys Ala His Ile Lys Ser Leu Leu Val Asp Ser
545                 550                 555                 560

Val Pro Leu Gly Thr Gly Glu Asn Leu Tyr Phe Gln Gly Ser Gly Gly
                565                 570                 575

Gly Gly Ser Asp Tyr Lys Asp Asp Asp Asp Lys Gly Thr Gly
            580                 585                 590

<210> 41
<211> 589
<212> PRT
<213> Artificial Sequence

<220>
<223> Description of Artificial Sequence: Synthetic
      polypeptide

<400> 41
Met Val Pro Lys Asp Met Ser Ile Pro Leu Leu Ala Ala Val Ser Ser
1               5                   10                  15

Ser Thr Glu Glu Thr Val Arg Pro Ile Ala Asp Phe His Pro Thr Leu
            20                  25                  30

Trp Gly Asn His Phe Leu Lys Ser Ala Ala Asp Val Glu Thr Ile Asp
        35                  40                  45

Ala Ala Thr Gln Glu Gln His Ala Ala Leu Lys Gln Glu Val Arg Arg
    50                  55                  60

Met Ile Thr Thr Thr Ala Asn Lys Leu Ala Gln Lys Leu His Met Ile
65                  70                  75                  80

Asp Ala Val Gln Arg Leu Gly Val Ala Tyr His Phe Glu Lys Glu Ile
                85                  90                  95

Glu Asp Glu Leu Gly Lys Val Ser His Asp Leu Asp Ser Asp Asp Leu
            100                 105                 110

Tyr Val Val Ser Leu Arg Phe Arg Leu Phe Arg Gln Gln Gly Val Lys
        115                 120                 125

Ile Ser Cys Asp Val Phe Asp Lys Phe Lys Asp Asp Glu Gly Lys Phe
    130                 135                 140

Lys Glu Ser Leu Ile Asn Asp Ile Arg Gly Met Leu Ser Leu Tyr Glu
145                 150                 155                 160

Ala Ala Tyr Leu Ala Ile Arg Gly Glu Asp Ile Leu Asp Glu Ala Ile
                165                 170                 175

Val Phe Thr Thr Thr His Leu Lys Ser Val Ile Ser Ile Ser Asp His
            180                 185                 190

Ser His Ala Asn Ser Asn Leu Ala Glu Gln Ile Arg His Ser Leu Gln
        195                 200                 205

Ile Pro Leu Arg Lys Ala Ala Ala Arg Leu Glu Ala Arg Tyr Phe Leu
    210                 215                 220

Asp Ile Tyr Ser Arg Asp Asp Leu His Asp Glu Thr Leu Leu Lys Phe
225                 230                 235                 240

Ala Lys Leu Asp Phe Asn Ile Leu Gln Ala Ala His Gln Lys Glu Ala
                245                 250                 255

Ser Ile Met Thr Arg Trp Trp Asn Asp Leu Gly Phe Pro Lys Lys Val
            260                 265                 270

Pro Tyr Ala Arg Asp Arg Ile Ile Glu Thr Tyr Ile Trp Met Leu Leu
        275                 280                 285

Gly Val Ser Tyr Glu Pro Asn Leu Ala Phe Gly Arg Ile Phe Ala Ser
    290                 295                 300

Lys Val Val Cys Met Ile Thr Thr Ile Asp Asp Thr Phe Asp Ala Tyr
305                 310                 315                 320

Gly Thr Phe Glu Glu Leu Thr Leu Phe Thr Glu Ala Val Thr Arg Trp
                325                 330                 335

Asp Ile Gly Leu Ile Asp Thr Leu Pro Glu Tyr Met Lys Phe Ile Val
            340                 345                 350

Lys Ala Leu Leu Asp Ile Tyr Arg Glu Ala Glu Glu Glu Leu Ala Lys
        355                 360                 365

Glu Gly Arg Ser Tyr Gly Ile Pro Tyr Ala Lys Gln Met Met Gln Glu
    370                 375                 380

Leu Ile Ile Leu Tyr Phe Thr Glu Ala Lys Trp Leu Tyr Lys Gly Tyr
385                 390                 395                 400

Val Pro Thr Phe Asp Glu Tyr Lys Ser Val Ala Leu Arg Ser Ile Gly
                405                 410                 415

Leu Arg Thr Leu Ala Val Ala Ser Phe Val Asp Leu Gly Asp Phe Ile
            420                 425                 430

Ala Thr Lys Asp Asn Phe Glu Cys Ile Leu Lys Asn Ala Lys Ser Leu
        435                 440                 445

Lys Ala Thr Glu Thr Ile Gly Arg Leu Met Asp Asp Ile Ala Gly Tyr
    450                 455                 460

Lys Phe Glu Gln Lys Arg Gly His Asn Pro Ser Ala Val Glu Cys Tyr
465                 470                 475                 480

Lys Asn Gln His Gly Val Ser Glu Glu Glu Ala Val Lys Glu Leu Leu
                485                 490                 495

Leu Glu Val Ala Asn Ser Trp Lys Asp Ile Asn Glu Glu Leu Leu Asn
            500                 505                 510

Pro Thr Thr Val Pro Leu Pro Met Leu Gln Arg Leu Leu Tyr Phe Ala
        515                 520                 525

Arg Ser Gly His Phe Ile Tyr Asp Asp Gly His Asp Arg Tyr Thr His
    530                 535                 540

Ser Leu Met Met Lys Arg Gln Val Ala Leu Leu Leu Thr Glu Pro Leu
545                 550                 555                 560

Ala Ile Gly Thr Gly Glu Asn Leu Tyr Phe Gln Gly Ser Gly Gly Gly
                565                 570                 575

Gly Ser Asp Tyr Lys Asp Asp Asp Asp Lys Gly Thr Gly
            580                 585

<210> 42
<211> 609
<212> PRT
<213> Artificial Sequence

<220>
<223> Description of Artificial Sequence: Synthetic
polypeptide

<400> 42
Met Val Pro Asp Leu Ala Val Glu Ile Ala Met Asp Leu Ala Val Asp
1               5                   10                  15

Asp Val Glu Arg Arg Val Gly Asp Tyr His Ser Asn Leu Trp Asp Asp
            20                  25                  30

Asp Phe Ile Gln Ser Leu Ser Thr Pro Tyr Gly Ala Ser Ser Tyr Arg
        35                  40                  45

Glu Arg Ala Glu Arg Leu Val Gly Glu Val Lys Glu Met Phe Thr Ser
    50                  55                  60

Ile Ser Ile Glu Asp Gly Glu Leu Thr Ser Asp Leu Leu Gln Arg Leu
65                  70                  75                  80

Trp Met Val Asp Asn Val Glu Arg Leu Gly Ile Ser Arg His Phe Glu
                85                  90                  95

Asn Glu Ile Lys Ala Ala Ile Asp Tyr Val Tyr Ser Tyr Trp Ser Asp
            100                 105                 110

Lys Gly Ile Val Arg Gly Arg Asp Ser Ala Val Pro Asp Leu Asn Ser
        115                 120                 125

Ile Ala Leu Gly Phe Arg Thr Leu Arg Leu His Gly Tyr Thr Val Ser
    130                 135                 140

Ser Asp Val Phe Lys Val Phe Gln Asp Arg Lys Gly Glu Phe Ala Cys
145                 150                 155                 160

Ser Ala Ile Pro Thr Glu Gly Asp Ile Lys Gly Val Leu Asn Leu Leu
                165                 170                 175

Arg Ala Ser Tyr Ile Ala Phe Pro Gly Glu Lys Val Met Glu Lys Ala
        180                 185                 190

Gln Thr Phe Ala Ala Thr Tyr Leu Lys Glu Ala Leu Gln Lys Ile Gln
    195                 200                 205

Val Ser Ser Leu Ser Arg Glu Ile Glu Tyr Val Leu Glu Tyr Gly Trp
    210                 215                 220

Leu Thr Asn Phe Pro Arg Leu Glu Ala Arg Asn Tyr Ile Asp Val Phe
225                 230                 235                 240

Gly Glu Glu Ile Cys Pro Tyr Phe Lys Lys Pro Cys Ile Met Val Asp
                245                 250                 255

Lys Leu Leu Glu Leu Ala Lys Leu Glu Phe Asn Leu Phe His Ser Leu
            260                 265                 270

Gln Gln Thr Glu Leu Lys His Val Ser Arg Trp Trp Lys Asp Ser Gly
        275                 280                 285

Phe Ser Gln Leu Thr Phe Thr Arg His Arg His Val Glu Phe Tyr Thr
    290                 295                 300

Leu Ala Ser Cys Ile Ala Ile Glu Pro Lys His Ser Ala Phe Arg Leu
305                 310                 315                 320

Gly Phe Ala Lys Val Cys Tyr Leu Gly Ile Val Leu Asp Asp Ile Tyr
                325                 330                 335

Asp Thr Phe Gly Lys Met Lys Glu Leu Glu Leu Phe Thr Ala Ala Ile
            340                 345                 350

Lys Arg Trp Asp Pro Ser Thr Thr Glu Cys Leu Pro Glu Tyr Met Lys
        355                 360                 365

Gly Val Tyr Met Ala Phe Tyr Asn Cys Val Asn Glu Leu Ala Leu Gln
    370                 375                 380

Ala Glu Lys Thr Gln Gly Arg Asp Met Leu Asn Tyr Ala Arg Lys Ala
385                 390                 395                 400

Trp Glu Ala Leu Phe Asp Ala Phe Leu Glu Glu Ala Lys Trp Ile Ser
                405                 410                 415

Ser Gly Tyr Leu Pro Thr Phe Glu Glu Tyr Leu Glu Asn Gly Lys Val
            420                 425                 430

Ser Phe Gly Tyr Arg Ala Ala Thr Leu Gln Pro Ile Leu Thr Leu Asp
        435                 440                 445

Ile Pro Leu Pro Leu His Ile Leu Gln Gln Ile Asp Phe Pro Ser Arg
    450                 455                 460

Phe Asn Asp Leu Ala Ser Ser Ile Leu Arg Leu Arg Gly Asp Ile Cys
465                 470                 475                 480

Gly Tyr Gln Ala Glu Arg Ser Arg Gly Glu Glu Ala Ser Ser Ile Ser
                485                 490                 495

Cys Tyr Met Lys Asp Asn Pro Gly Ser Thr Glu Glu Asp Ala Leu Ser
            500                 505                 510

His Ile Asn Ala Met Ile Ser Asp Asn Ile Asn Glu Leu Asn Trp Glu
        515                 520                 525

Leu Leu Lys Pro Asn Ser Asn Val Pro Ile Ser Ser Lys Lys His Ala
    530                 535                 540

Phe Asp Ile Leu Arg Ala Phe Tyr His Leu Tyr Lys Tyr Arg Asp Gly
545                 550                 555                 560

Phe Ser Ile Ala Lys Ile Glu Thr Lys Asn Leu Val Met Arg Thr Val
                565                 570                 575

Leu Glu Pro Val Pro Met Gly Thr Gly Glu Asn Leu Tyr Phe Gln Gly
            580                 585                 590

Ser Gly Gly Gly Gly Ser Asp Tyr Lys Asp Asp Asp Asp Lys Gly Thr
        595                 600                 605

Gly

<210> 43
<211> 836
<212> PRT
<213> Artificial Sequence

<220>
<223> Description of Artificial Sequence: Synthetic
      polypeptide

<400> 43
Met Val Pro Thr Ser Val Ser Val Glu Ser Gly Thr Val Ser Cys Leu
1               5                   10                  15

Ser Ser Asn Asn Leu Ile Arg Arg Thr Ala Asn Pro His Pro Asn Ile
            20                  25                  30

Trp Gly Tyr Asp Phe Val His Ser Leu Lys Ser Pro Tyr Thr His Asp
        35                  40                  45

Ser Ser Tyr Arg Glu Arg Ala Glu Thr Leu Ile Ser Glu Ile Lys Val
    50                  55                  60

Met Leu Gly Gly Gly Glu Leu Met Met Thr Pro Ser Ala Tyr Asp Thr
65                  70                  75                  80

Ala Trp Val Ala Arg Val Pro Ser Ile Asp Gly Ser Ala Cys Pro Gln
                85                  90                  95

Phe Pro Gln Thr Val Glu Trp Ile Leu Lys Asn Gln Leu Lys Asp Gly
            100                 105                 110

Ser Trp Gly Thr Glu Ser His Phe Leu Leu Ser Asp Arg Leu Leu Ala
        115                 120                 125

Thr Leu Ser Cys Val Leu Ala Leu Leu Lys Trp Lys Val Ala Asp Val
    130                 135                 140

Gln Val Glu Gln Gly Ile Glu Phe Ile Lys Arg Asn Leu Gln Ala Ile
145                 150                 155                 160

Lys Asp Glu Arg Asp Gln Asp Ser Leu Val Thr Asp Phe Glu Ile Ile
                165                 170                 175

Phe Pro Ser Leu Leu Lys Glu Ala Gln Ser Leu Asn Leu Gly Leu Pro
            180                 185                 190

Tyr Asp Leu Pro Tyr Ile Arg Leu Leu Gln Thr Lys Arg Gln Glu Arg
        195                 200                 205

Leu Ala Asn Leu Ser Met Asp Lys Ile His Gly Gly Thr Leu Leu Ser
    210                 215                 220

Ser Leu Glu Gly Ile Gln Asp Ile Val Glu Trp Glu Thr Ile Met Asp
225                 230                 235                 240

Val Gln Ser Gln Asp Gly Ser Phe Leu Ser Ser Pro Ala Ser Thr Ala
                245                 250                 255

Cys Val Phe Met His Thr Gly Asp Met Lys Cys Leu Asp Phe Leu Asn
            260                 265                 270

Asn Val Leu Thr Lys Phe Gly Ser Ser Val Pro Cys Leu Tyr Pro Val
        275                 280                 285

Asp Leu Leu Glu Arg Leu Leu Ile Val Asp Asn Val Glu Arg Leu Gly
    290                 295                 300

Ile Asp Arg His Phe Glu Lys Glu Ile Lys Glu Ala Leu Asp Tyr Val
305                 310                 315                 320

Tyr Arg His Trp Asn Asp Arg Gly Ile Gly Trp Gly Arg Leu Ser Pro
                325                 330                 335

Ile Ala Asp Leu Glu Thr Thr Ala Leu Gly Phe Arg Leu Leu Arg Leu
            340                 345                 350

His Arg Tyr Asn Val Ser Pro Val Val Leu Asp Asn Phe Lys Asp Ala
        355                 360                 365

Asp Gly Glu Phe Phe Cys Ser Thr Gly Gln Phe Asn Lys Asp Val Ala
    370                 375                 380

Ser Met Leu Ser Leu Tyr Arg Ala Ser Gln Leu Ala Phe Pro Glu Glu
385                 390                 395                 400

Ser Ile Leu Asp Glu Ala Lys Ser Phe Ser Thr Gln Tyr Leu Arg Glu
                405                 410                 415

Ala Leu Glu Lys Ser Glu Thr Phe Ser Ser Trp Asn His Arg Gln Ser
            420                 425                 430

Leu Ser Glu Glu Ile Lys Tyr Ala Leu Lys Thr Ser Trp His Ala Ser
        435                 440                 445

Val Pro Arg Val Glu Ala Lys Arg Tyr Cys Gln Val Tyr Arg Gln Asp
    450                 455                 460

Tyr Ala His Leu Ala Lys Ser Val Tyr Lys Leu Pro Lys Val Asn Asn
465                 470                 475                 480

Glu Lys Ile Leu Glu Leu Ala Lys Leu Asp Phe Asn Ile Ile Gln Ser
                485                 490                 495

Ile His Gln Lys Glu Met Lys Asn Val Thr Ser Trp Phe Arg Asp Ser
            500                 505                 510

Gly Leu Pro Leu Phe Thr Phe Ala Arg Glu Arg Pro Leu Glu Phe Tyr

        515                 520                 525

Phe Leu Ile Ala Gly Gly Thr Tyr Glu Pro Gln Tyr Ala Lys Cys Arg
    530                 535                 540

Phe Leu Phe Thr Lys Val Ala Cys Leu Gln Thr Val Leu Asp Asp Met
545                 550                 555                 560

Tyr Asp Thr Tyr Gly Thr Pro Ser Glu Leu Lys Leu Phe Thr Glu Ala
                565                 570                 575

Val Arg Arg Trp Asp Leu Ser Phe Thr Glu Asn Leu Pro Asp Tyr Met
            580                 585                 590

Lys Leu Cys Tyr Lys Ile Tyr Tyr Asp Ile Val His Glu Val Ala Trp
        595                 600                 605

Glu Val Glu Lys Glu Gln Gly Arg Glu Leu Val Ser Phe Phe Arg Lys
    610                 615                 620

Gly Trp Glu Asp Tyr Leu Leu Gly Tyr Tyr Glu Glu Ala Glu Trp Leu
625                 630                 635                 640

Ala Ala Glu Tyr Val Pro Thr Leu Asp Glu Tyr Ile Lys Asn Gly Ile
                645                 650                 655

Thr Ser Ile Gly Gln Arg Ile Leu Leu Leu Ser Gly Val Leu Ile Met
            660                 665                 670

Glu Gly Gln Leu Leu Ser Gln Glu Ala Leu Glu Lys Val Asp Tyr Pro
        675                 680                 685

Gly Arg Arg Val Leu Thr Glu Leu Asn Ser Leu Ile Ser Arg Leu Ala
    690                 695                 700

Asp Asp Thr Lys Thr Tyr Lys Ala Glu Lys Ala Arg Gly Glu Leu Ala
705                 710                 715                 720

Ser Ser Ile Glu Cys Tyr Met Lys Asp His Pro Gly Cys Gln Glu Glu
                725                 730                 735

Glu Ala Leu Asn His Ile Tyr Gly Ile Leu Glu Pro Ala Val Lys Glu
            740                 745                 750

Leu Thr Arg Glu Phe Leu Lys Ala Asp His Val Pro Phe Pro Cys Lys
        755                 760                 765

Lys Met Leu Phe Asp Glu Thr Arg Val Thr Met Val Ile Phe Lys Asp
    770                 775                 780

Gly Asp Gly Phe Gly Ile Ser Lys Leu Glu Val Lys Asp His Ile Lys
785                 790                 795                 800

Glu Cys Leu Ile Glu Pro Leu Pro Leu Gly Thr Gly Glu Asn Leu Tyr
                805                 810                 815

Phe Gln Gly Ser Gly Gly Gly Gly Ser Asp Tyr Lys Asp Asp Asp Asp
            820                 825                 830

Lys Gly Thr Gly
        835

<210> 44
<211> 576
<212> PRT
<213> Artificial Sequence

<220>
<223> Description of Artificial Sequence: Synthetic
      polypeptide

<400> 44
   Met Val Pro Gly Ser Glu Val Asn Arg Pro Leu Ala Asp Phe Pro Ala
   1               5                   10                  15
   Asn Ile Trp Glu Asp Pro Leu Thr Ser Phe Ser Lys Ser Asp Leu Gly
               20                  25                  30
   Thr Glu Thr Phe Lys Glu Lys His Ser Thr Leu Lys Glu Ala Val Lys
           35                  40                  45
   Glu Ala Phe Met Ser Ser Lys Ala Asn Pro Ile Glu Asn Ile Lys Phe
       50                  55                  60
   Ile Asp Ala Leu Cys Arg Leu Gly Val Ser Tyr His Phe Glu Lys Asp
   65                  70                  75                  80
   Ile Val Glu Gln Leu Asp Lys Ser Phe Asp Cys Leu Asp Phe Pro Gln
                   85                  90                  95
   Met Val Arg Gln Glu Gly Cys Asp Leu Tyr Thr Val Gly Ile Ile Phe
               100                 105                 110
   Gln Val Phe Arg Gln Phe Gly Phe Lys Leu Ser Ala Asp Val Phe Glu
           115                 120                 125
   Lys Phe Lys Asp Glu Asn Gly Lys Phe Lys Gly His Leu Val Thr Asp
   130 135 140
   Ala Tyr Gly Met Leu Ser Leu Tyr Glu Ala Ala Gln Trp Gly Thr His
   145 150 155 160
   Gly Glu Asp Ile Ile Asp Glu Ala Leu Ala Phe Ser Arg Ser His Leu
   165 170 175
   Glu Glu Ile Ser Ser Arg Ser Ser Pro His Leu Ala Ile Arg Ile Lys
   180 185 190
   Asn Ala Leu Lys His Pro Tyr His Lys Gly Ile Ser Arg Ile Glu Thr
   195 200 205
   Arg Gln Tyr Ile Ser Tyr Tyr Glu Glu Glu Glu Ser Cys Asp Pro Thr
   210 215 220
   Leu Leu Glu Phe Ala Lys Ile Asp Phe Asn Leu Leu Gln Ile Leu His
   225 230 235 240
   Arg Glu Glu Leu Ala Cys Val Thr Arg Trp His His Glu Met Glu Phe
   245 250 255
   Lys Ser Lys Val Thr Tyr Thr Arg His Arg Ile Thr Glu Ala Tyr Leu
   260 265 270
   Trp Ser Leu Gly Thr Tyr Phe Glu Pro Gln Tyr Ser Gln Ala Arg Val
   275 280 285
   Ile Thr Thr Met Ala Leu Ile Leu Phe Thr Ala Leu Asp Asp Met Tyr
   290 295 300
   Asp Ala Tyr Gly Thr Met Glu Glu Leu Glu Leu Phe Thr Asp Ala Met
   305 310 315 320
   Asp Glu Trp Leu Pro Val Val Pro Asp Glu Ile Pro Ile Pro Asp Ser
   325 330 335
   Met Lys Phe Ile Tyr Asn Val Thr Val Glu Phe Tyr Asp Lys Leu Asp
   340 345 350
   Glu Glu Leu Glu Lys Glu Gly Arg Ser Gly Cys Gly Phe His Leu Lys
   355 360 365
   Lys Ser Leu Gln Lys Thr Ala Asn Gly Tyr Met Gln Glu Ala Lys Trp
   370 375 380
   Leu Lys Lys Asp Tyr Ile Ala Thr Phe Asp Glu Tyr Lys Glu Asn Ala
   385                 390                 395                 400
   Ile Leu Ser Ser Gly Tyr Tyr Ala Leu Ile Ala Met Thr Phe Val Arg
                   405                 410                 415
   Met Thr Asp Val Ala Lys Leu Asp Ala Phe Glu Trp Leu Ser Ser His
               420                 425                 430
   Pro Lys Ile Arg Val Ala Ser Glu Ile Ile Ser Arg Phe Thr Asp Asp
           435                 440                 445
   Ile Ser Ser Tyr Glu Phe Glu His Lys Arg Glu His Val Ala Thr Gly
       450                 455                 460
   Ile Asp Cys Tyr Met Gln Gln Phe Gly Val Ser Lys Glu Arg Ala Val
   465                 470                 475                 480
   Glu Val Met Gly Asn Ile Val Ser Asp Ala Trp Lys Asp Leu Asn Gln
                   485                 490                 495
   Glu Leu Met Arg Pro His Val Phe Pro Phe Pro Leu Leu Met Arg Val
               500                 505                 510
   Leu Asn Leu Ser Arg Val Ile Asp Val Phe Tyr Arg Tyr Gln Asp Ala
           515                 520                 525
   Tyr Thr Asn Pro Lys Leu Leu Lys Glu His Ile Val Ser Leu Leu Ile
       530                 535                 540
   Glu Thr Ile Pro Ile Gly Thr Gly Glu Asn Leu Tyr Phe Gln Gly Ser
   545                 550                 555                 560
   Gly Gly Gly Gly Ser Asp Tyr Lys Asp Asp Asp Asp Lys Gly Thr Gly
                   565                 570                 575

<210> 45
<211> 590
<212> PRT
<213> Artificial Sequence

<220>
<223> Description of Artificial Sequence: Synthetic
      polypeptide

<400> 45
Met Val Pro Glu Ala Ile Arg Val Phe Gly Leu Lys Leu Gly Ser Lys
1               5                   10                  15

Leu Ser Ile His Ser Gln Thr Asn Ala Phe Pro Ala Phe Lys Leu Ser

20                  25                  30

Arg Phe Pro Leu Thr Ser Phe Pro Gly Lys His Ala His Leu Asp Pro
        35                  40                  45

Leu Lys Ala Thr Thr His Pro Leu Ala Phe Asp Gly Glu Glu Asn Asn
    50                  55                  60

Arg Glu Phe Lys Asn Leu Gly Pro Ser Glu Trp Gly His Gln Phe Leu
65                  70                  75                  80

Ser Ala His Val Asp Leu Ser Glu Met Asp Ala Leu Glu Arg Glu Ile
                85                  90                  95

Glu Ala Leu Lys Pro Lys Val Arg Asp Met Leu Ile Ser Ser Glu Ser
            100                 105                 110

Ser Lys Lys Lys Ile Leu Phe Leu Tyr Leu Leu Val Ser Leu Gly Leu
        115                 120                 125

Ala Tyr His Phe Glu Asp Glu Ile Lys Glu Ser Leu Glu Asp Gly Leu
    130                 135                 140

Gln Lys Ile Glu Glu Met Met Ala Ser Glu Asp Asp Leu Arg Phe Lys
145                 150                 155                 160

Gly Asp Asn Gly Lys Phe Lys Glu Cys Leu Ala Lys Asp Ala Lys Gly
                165                 170                 175

Ile Leu Ser Leu Tyr Glu Ala Ala His Met Gly Thr Thr Thr Asp Tyr
            180                 185                 190

Ile Leu Asp Glu Ala Leu Ser Phe Thr Leu Thr Tyr Met Glu Ser Leu
        195                 200                 205

Ala Ala Ser Gly Thr Cys Lys Ile Asn Leu Ser Arg Arg Ile Arg Lys
    210                 215                 220

Ala Leu Asp Gln Pro Gln His Lys Asn Met Glu Ile Ile Val Ala Met
225                 230                 235                 240

Lys Tyr Ile Gln Phe Tyr Glu Glu Glu Glu Asp Cys Asp Lys Thr Leu
                245                 250                 255

Leu Lys Phe Ala Lys Leu Asn Phe Lys Phe Leu Gln Leu His Tyr Leu
            260                 265                 270

Gln Glu Leu Lys Ile Leu Ser Lys Trp Tyr Lys Asp Gln Asp Phe Lys
        275                 280                 285

Ser Lys Leu Pro Pro Tyr Phe Arg Asp Arg Leu Val Glu Cys His Phe
    290                 295                 300

Ala Ser Leu Thr Cys Phe Glu Pro Lys Tyr Ala Arg Ala Arg Ile Phe
305                 310                 315                 320

Leu Ser Lys Ile Phe Thr Val Gln Ile Phe Ile Asp Asp Thr Cys Asp
                325                 330                 335

Arg Tyr Ala Ser Leu Gly Glu Val Glu Ser Leu Ala Asp Thr Ile Glu
            340                 345                 350

Arg Trp Asp Pro Asp Asp His Ala Met Asp Gly Leu Pro Asp Tyr Leu
        355                 360                 365

Lys Ser Val Val Lys Phe Val Phe Asn Thr Phe Gln Glu Phe Glu Arg
    370                 375                 380

Lys Cys Lys Arg Ser Leu Arg Ile Asn Leu Gln Val Ala Lys Trp Val
385                 390                 395                 400

Lys Ala Gly His Leu Pro Ser Phe Asp Glu Tyr Leu Asp Val Ala Gly
                405                 410                 415

Leu Glu Leu Ala Ile Ser Phe Thr Phe Ala Gly Ile Leu Met Gly Met
            420                 425                 430

Glu Asn Val Cys Lys Pro Glu Ala Tyr Glu Trp Leu Lys Ser Arg Asp
        435                 440                 445

Lys Leu Val Arg Gly Val Ile Thr Lys Val Arg Leu Leu Asn Asp Ile
    450                 455                 460

Phe Gly Tyr Glu Asp Asp Met Arg Arg Gly Tyr Val Thr Asn Ser Ile
465                 470                 475                 480

Asn Cys Tyr Lys Lys Gln Tyr Gly Val Thr Glu Glu Glu Ala Ile Arg
                485                 490                 495

Lys Leu His Gln Ile Val Ala Asp Gly Glu Lys Met Met Asn Glu Glu
            500                 505                 510

Phe Leu Lys Pro Ile Asn Val Pro Tyr Gln Val Pro Lys Val Val Ile
        515                 520                 525

Leu Asp Thr Leu Arg Ala Ala Asn Val Ser Tyr Glu Lys Asp Asp Glu
    530                 535                 540

Phe Thr Arg Pro Gly Glu His Leu Lys Asn Cys Ile Thr Ser Ile Tyr
545                 550                 555                 560

Phe Asp Leu Gly Thr Gly Glu Asn Leu Tyr Phe Gln Gly Ser Gly Gly
                565                 570                 575

Gly Gly Ser Asp Tyr Lys Asp Asp Asp Asp Lys Gly Thr Gly
            580                 585                 590

<210> 46
<211> 375
<212> PRT
<213> Artificial Sequence

<220>
<223> Description of Artificial Sequence: Synthetic
      polypeptide

<400> 46
Met Val Pro Thr Thr Thr Leu Ser Ser Asn Leu Asn Ser Gln Phe Met
1               5                   10                  15

Gln Val Tyr Glu Thr Leu Lys Ser Glu Leu Ile His Asp Pro Leu Phe
            20                  25                  30

Glu Phe Asp Asp Asp Ser Arg Gln Trp Val Glu Arg Met Ile Asp Tyr
        35                  40                  45

Thr Val Pro Gly Gly Lys Met Val Arg Gly Tyr Ser Val Val Asp Ser
    50                  55                  60

Tyr Gln Leu Leu Lys Gly Glu Glu Leu Thr Glu Glu Glu Ala Phe Leu
65                  70                  75                  80

Ala Cys Ala Leu Gly Trp Cys Thr Glu Trp Phe Gln Ala Phe Ile Leu
                85                  90                  95

Leu His Asp Asp Met Met Asp Gly Ser His Thr Arg Arg Gly Gln Pro
            100                 105                 110

Cys Trp Phe Arg Leu Pro Glu Val Gly Ala Val Ala Ile Asn Asp Gly
        115                 120                 125

Val Leu Leu Arg Asn His Val His Arg Ile Leu Lys Lys His Phe Gln
    130                 135                 140

Gly Lys Ala Tyr Tyr Val His Leu Val Asp Leu Phe Asn Glu Thr Glu
145                 150                 155                 160

Phe Gln Thr Ile Ser Gly Gln Met Ile Asp Leu Ile Thr Thr Leu Val
                165                 170                 175

Gly Glu Lys Asp Leu Ser Lys Tyr Ser Leu Ser Ile His Arg Arg Ile
            180                 185                 190

Val Gln Tyr Lys Thr Ala Tyr Tyr Ser Phe Tyr Leu Pro Val Ala Cys
        195                 200                 205

Ala Leu Leu Met Phe Gly Glu Asp Leu Asp Lys His Val Glu Val Lys
    210                 215                 220

Asn Val Leu Val Glu Met Gly Thr Tyr Phe Gln Val Gln Asp Asp Tyr
225                 230                 235                 240

Leu Asp Cys Phe Gly Ala Pro Glu Val Ile Gly Lys Ile Gly Thr Asp
                245                 250                 255

Ile Glu Asp Phe Lys Cys Ser Trp Leu Val Val Lys Ala Leu Glu Leu
            260                 265                 270

Ala Asn Glu Glu Gln Lys Lys Thr Leu His Glu Asn Tyr Gly Lys Lys
        275                 280                 285

Asp Pro Ala Ser Val Ala Lys Val Lys Glu Val Tyr His Thr Leu Asn
    290                 295                 300

Leu Gln Ala Val Phe Glu Asp Tyr Glu Ala Thr Ser Tyr Lys Lys Leu
305                 310                 315                 320

Ile Thr Ser Ile Glu Asn His Pro Ser Lys Ala Val Gln Ala Val Leu
                325                 330                 335

Lys Ser Phe Leu Gly Lys Ile Tyr Lys Arg Gln Lys Gly Thr Gly Glu
            340                 345                 350

Asn Leu Tyr Phe Gln Gly Ser Gly Gly Gly Gly Ser Asp Tyr Lys Asp
        355                 360                 365

Asp Asp Asp Lys Gly Thr Gly
    370                 375

<210> 47
<211> 600
<212> PRT

<213> Artificial Sequence

<220>
<223> Description of Artificial Sequence: Synthetic
      polypeptide

<400> 47
Met Val Pro Ser Gln Pro Tyr Trp Ala Ala Ile Glu Ala Asp Ile Glu
1               5                   10                  15

Arg Tyr Leu Lys Lys Ser Ile Thr Ile Arg Pro Pro Glu Thr Val Phe
            20                  25                  30

Gly Pro Met His His Leu Thr Phe Ala Ala Pro Ala Thr Ala Ala Ser
        35                  40                  45

Thr Leu Cys Leu Ala Ala Cys Glu Leu Val Gly Gly Asp Arg Ser Gln
    50                  55                  60

Ala Met Ala Ala Ala Ala Ala Ile His Leu Val His Ala Ala Ala Tyr
65                  70                  75                  80

Val His Glu His Leu Pro Leu Thr Asp Gly Ser Arg Pro Val Ser Lys
                85                  90                  95

Pro Ala Ile Gln His Lys Tyr Gly Pro Asn Val Glu Leu Leu Thr Gly
            100                 105                 110

Asp Gly Ile Val Pro Phe Gly Phe Glu Leu Leu Ala Gly Ser Val Asp
        115                 120                 125

Pro Ala Arg Thr Asp Asp Pro Asp Arg Ile Leu Arg Val Ile Ile Glu
    130                 135                 140

Ile Ser Arg Ala Gly Gly Pro Glu Gly Met Ile Ser Gly Leu His Arg
145                 150                 155                 160

Glu Glu Glu Ile Val Asp Gly Asn Thr Ser Leu Asp Phe Ile Glu Tyr
                165                 170                 175

Val Cys Lys Lys Lys Tyr Gly Glu Met His Ala Cys Gly Ala Ala Cys
            180                 185                 190

Gly Ala Ile Leu Gly Gly Ala Ala Glu Glu Glu Ile Gln Lys Leu Arg
        195                 200                 205

Asn Phe Gly Leu Tyr Gln Gly Thr Leu Arg Gly Met Met Glu Met Lys
    210                 215                 220

Asn Ser His Gln Leu Ile Asp Glu Asn Ile Ile Gly Lys Leu Lys Glu
225                 230                 235                 240

Leu Ala Leu Glu Glu Leu Gly Gly Phe His Gly Lys Asn Ala Glu Leu
                245                 250                 255

Met Ser Ser Leu Val Ala Glu Pro Ser Leu Tyr Ala Ala Ser Ser Asn
            260                 265                 270

Asn Leu Gly Ile Glu Gly Arg Phe Asp Phe Asp Gly Tyr Met Leu Arg
        275                 280                 285

Lys Ala Lys Ser Val Asn Lys Ala Leu Glu Ala Ala Val Gln Met Lys
    290                 295                 300

Glu Pro Leu Lys Ile His Glu Ser Met Arg Tyr Ser Leu Leu Ala Gly
305                 310                 315                 320

Gly Lys Arg Val Arg Pro Met Leu Cys Ile Ala Ala Cys Glu Leu Val
                325                 330                 335

Gly Gly Asp Glu Ser Thr Ala Met Pro Ala Ala Cys Ala Val Glu Met
            340                 345                 350

Ile His Thr Met Ser Leu Met His Asp Asp Leu Pro Cys Met Asp Asn
        355                 360                 365

Asp Asp Leu Arg Arg Gly Lys Pro Thr Asn His Met Ala Phe Gly Glu
    370                 375                 380

Ser Val Ala Val Leu Ala Gly Asp Ala Leu Leu Ser Phe Ala Phe Glu
385                 390                 395                 400

His Val Ala Ala Ala Thr Lys Gly Ala Pro Pro Glu Arg Ile Val Arg
                405                 410                 415

Val Leu Gly Glu Leu Ala Val Ser Ile Gly Ser Glu Gly Leu Val Ala
            420                 425                 430

Gly Gln Val Val Asp Val Cys Ser Glu Gly Met Ala Glu Val Gly Leu
        435                 440                 445

Asp His Leu Glu Phe Ile His His His Lys Thr Ala Ala Leu Leu Gln
    450                 455                 460

Gly Ser Val Val Leu Gly Ala Ile Leu Gly Gly Gly Lys Glu Glu Glu
465                 470                 475                 480

Val Ala Lys Leu Arg Lys Phe Ala Asn Cys Ile Gly Leu Leu Phe Gln
                485                 490                 495

Val Val Asp Asp Ile Leu Asp Val Thr Lys Ser Ser Lys Glu Leu Gly
            500                 505                 510

Lys Thr Ala Gly Lys Asp Leu Val Ala Asp Lys Thr Thr Tyr Pro Lys
        515                 520                 525

Leu Ile Gly Val Glu Lys Ser Lys Glu Phe Ala Asp Arg Leu Asn Arg
    530                 535                 540

Glu Ala Gln Glu Gln Leu Leu His Phe His Pro His Arg Ala Ala Pro
545                 550                 555                 560

Leu Ile Ala Leu Ala Asn Tyr Ile Ala Tyr Arg Asp Asn Gly Thr Gly
                565                 570                 575

Glu Asn Leu Tyr Phe Gln Gly Ser Gly Gly Gly Gly Ser Asp Tyr Lys
            580                 585                 590

Asp Asp Asp Asp Lys Gly Thr Gly
        595                 600

<210> 48
<211> 456
<212> PRT
<213> Artificial Sequence

<220>
<223> Description of Artificial Sequence: Synthetic
      polypeptide

<400> 48
Met Val Pro Val Thr Ala Ala Arg Ala Thr Pro Lys Leu Ser Asn Arg
1               5                   10                  15

Lys Leu Arg Val Ala Val Ile Gly Gly Gly Pro Ala Gly Gly Ala Ala
            20                  25                  30

Ala Glu Thr Leu Ala Gln Gly Gly Ile Glu Thr Ile Leu Ile Glu Arg
        35                  40                  45

Lys Met Asp Asn Cys Lys Pro Cys Gly Gly Ala Ile Pro Leu Cys Met
    50                  55                  60

Val Gly Glu Phe Asn Leu Pro Leu Asp Ile Ile Asp Arg Arg Val Thr
65                  70                  75                  80

Lys Met Lys Met Ile Ser Pro Ser Asn Ile Ala Val Asp Ile Gly Arg
                85                  90                  95

Thr Leu Lys Glu His Glu Tyr Ile Gly Met Val Arg Arg Glu Val Leu
            100                 105                 110

Asp Ala Tyr Leu Arg Glu Arg Ala Glu Lys Ser Gly Ala Thr Val Ile
        115                 120                 125

Asn Gly Leu Phe Leu Lys Met Asp His Pro Glu Asn Trp Asp Ser Pro
    130                 135                 140

Tyr Thr Leu His Tyr Thr Glu Tyr Asp Gly Lys Thr Gly Ala Thr Gly
145                 150                 155                 160

Thr Lys Lys Thr Met Glu Val Asp Ala Val Ile Gly Ala Asp Gly Ala
                165                 170                 175

Asn Ser Arg Val Ala Lys Ser Ile Asp Ala Gly Asp Tyr Asp Tyr Ala
            180                 185                 190

Ile Ala Phe Gln Glu Arg Ile Arg Ile Pro Asp Glu Lys Met Thr Tyr
        195                 200                 205

Tyr Glu Asp Leu Ala Glu Met Tyr Val Gly Asp Asp Val Ser Pro Asp
    210                 215                 220

Phe Tyr Gly Trp Val Phe Pro Lys Cys Asp His Val Ala Val Gly Thr
225                 230                 235                 240

Gly Thr Val Thr His Lys Gly Asp Ile Lys Lys Phe Gln Leu Ala Thr
                245                 250                 255

Arg Asn Arg Ala Lys Asp Lys Ile Leu Gly Gly Lys Ile Ile Arg Val
            260                 265                 270

Glu Ala His Pro Ile Pro Glu His Pro Arg Pro Arg Arg Leu Ser Lys
        275                 280                 285

Arg Val Ala Leu Val Gly Asp Ala Ala Gly Tyr Val Thr Lys Cys Ser
    290                 295                 300

Gly Glu Gly Ile Tyr Phe Ala Ala Lys Ser Gly Arg Met Cys Ala Glu
305                 310                 315                 320

Ala Ile Val Glu Gly Ser Gln Asn Gly Lys Lys Met Ile Asp Glu Gly
                325                 330                 335

Asp Leu Arg Lys Tyr Leu Glu Lys Trp Asp Lys Thr Tyr Leu Pro Thr
            340                 345                 350

Tyr Arg Val Leu Asp Val Leu Gln Lys Val Phe Tyr Arg Ser Asn Pro
        355                 360                 365

Ala Arg Glu Ala Phe Val Glu Met Cys Asn Asp Glu Tyr Val Gln Lys
    370                 375                 380

Met Thr Phe Asp Ser Tyr Leu Tyr Lys Arg Val Ala Pro Gly Ser Pro
385                 390                 395                 400

Leu Glu Asp Ile Lys Leu Ala Val Asn Thr Ile Gly Ser Leu Val Arg
                405                 410                 415

Ala Asn Ala Leu Arg Arg Glu Ile Glu Lys Leu Ser Val Gly Thr Gly
            420                 425                 430

Glu Asn Leu Tyr Phe Gln Gly Ser Gly Gly Gly Gly Ser Asp Tyr Lys
        435                 440                 445

Asp Asp Asp Asp Lys Gly Thr Gly
    450                 455

<210> 49
<211> 453
<212> PRT
<213> Artificial Sequence

<220>
<223> Description of Artificial Sequence: Synthetic
      polypeptide

<400> 49
Met Val Pro Val Ala Val Ile Gly Gly Gly Pro Ser Gly Ala Cys Ala
1               5                   10                  15

Ala Glu Thr Leu Ala Lys Gly Gly Val Glu Thr Phe Leu Leu Glu Arg
            20                  25                  30

Lys Leu Asp Asn Cys Lys Pro Cys Gly Gly Ala Ile Pro Leu Cys Met
        35                  40                  45

Val Glu Glu Phe Asp Leu Pro Met Glu Ile Ile Asp Arg Arg Val Thr
    50                  55                  60

Lys Met Lys Met Ile Ser Pro Ser Asn Arg Glu Val Asp Val Gly Lys
65                  70                  75                  80

Thr Leu Ser Glu Thr Glu Trp Ile Gly Met Cys Arg Arg Glu Val Phe
                85                  90                  95

Asp Asp Tyr Leu Arg Asn Arg Ala Gln Lys Leu Gly Ala Asn Ile Val
            100                 105                 110

Asn Gly Leu Phe Met Arg Ser Glu Gln Gln Ser Ala Glu Gly Pro Phe
        115                 120                 125

Thr Ile His Tyr Asn Ser Tyr Glu Asp Gly Ser Lys Met Gly Lys Pro
    130                 135                 140

Ala Thr Leu Glu Val Asp Met Ile Ile Gly Ala Asp Gly Ala Asn Ser
145                 150                 155                 160

Arg Ile Ala Lys Glu Ile Asp Ala Gly Glu Tyr Asp Tyr Ala Ile Ala
                165                 170                 175

Phe Gln Glu Arg Ile Arg Ile Pro Asp Asp Lys Met Lys Tyr Tyr Glu
            180                 185                 190

Asn Leu Ala Glu Met Tyr Val Gly Asp Asp Val Ser Pro Asp Phe Tyr
        195                 200                 205

Gly Trp Val Phe Pro Lys Tyr Asp His Val Ala Val Gly Thr Gly Thr
    210                 215                 220

Val Val Asn Lys Thr Ala Ile Lys Gln Tyr Gln Gln Ala Thr Arg Asp
225                 230                 235                 240

Arg Ser Lys Val Lys Thr Glu Gly Gly Lys Ile Ile Arg Val Glu Ala
                245                 250                 255

His Pro Ile Pro Glu His Pro Arg Pro Arg Arg Cys Lys Gly Arg Val
            260                 265                 270

Ala Leu Val Gly Asp Ala Ala Gly Tyr Val Thr Lys Cys Ser Gly Glu
        275                 280                 285

Gly Ile Tyr Phe Ala Ala Lys Ser Gly Arg Met Ala Ala Glu Ala Ile
    290                 295                 300

Val Glu Gly Ser Ala Asn Gly Thr Lys Met Cys Gly Glu Asp Ala Ile
305                 310                 315                 320

Arg Val Tyr Leu Asp Lys Trp Asp Arg Lys Tyr Trp Thr Thr Tyr Lys
                325                 330                 335

Val Leu Asp Ile Leu Gln Lys Val Phe Tyr Arg Ser Asn Pro Ala Arg
            340                 345                 350

Glu Ala Phe Val Glu Leu Cys Glu Asp Ser Tyr Val Gln Lys Met Thr
        355                 360                 365

Phe Asp Ser Tyr Leu Tyr Lys Thr Val Val Pro Gly Asn Pro Leu Asp
    370                 375                 380

Asp Val Lys Leu Leu Val Arg Thr Val Ser Ser Ile Leu Arg Ser Asn
385                 390                 395                 400

Ala Leu Arg Ser Val Asn Ser Lys Ser Val Asn Val Ser Phe Gly Ser
                405                 410                 415

Lys Ala Asn Glu Glu Arg Val Met Ala Ala Gly Thr Gly Glu Asn Leu
            420                 425                 430

Tyr Phe Gln Gly Ser Gly Gly Gly Gly Ser Asp Tyr Lys Asp Asp Asp
        435                 440                 445

Asp Lys Gly Thr Gly
    450

<210> 50
<211> 305
<212> PRT
<213> Artificial Sequence

<220>
<223> Description of Artificial Sequence: Synthetic
polypeptide

<400> 50
Met Val Pro Ala Met Ala Val Pro Leu Asp Val Val Ile Thr Tyr Pro
1               5                   10                  15

Ser Ser Gly Ala Ala Ala Tyr Pro Val Leu Val Met Tyr Asn Gly Phe
            20                  25                  30

Gln Ala Lys Ala Pro Trp Tyr Arg Gly Ile Val Asp His Val Ser Ser
        35                  40                  45

Trp Gly Tyr Thr Val Val Gln Tyr Thr Asn Gly Gly Leu Phe Pro Ile
    50                  55                  60

Val Val Asp Arg Val Glu Leu Thr Tyr Leu Glu Pro Leu Leu Thr Trp
65                  70                  75                  80

Leu Glu Thr Gln Ser Ala Asp Ala Lys Ser Pro Leu Tyr Gly Arg Ala
                85                  90                  95

Asp Val Ser Arg Leu Gly Thr Met Gly His Ser Arg Gly Gly Lys Leu
            100                 105                 110

Ala Ala Leu Gln Phe Ala Gly Arg Thr Asp Val Ser Gly Cys Val Leu
        115                 120                 125

Phe Asp Pro Val Asp Gly Ser Pro Met Thr Pro Glu Ser Ala Asp Tyr
    130                 135                 140

Pro Ser Ala Thr Lys Ala Leu Ala Ala Ala Gly Arg Ser Ala Gly Leu
145                 150                 155                 160

Val Gly Ala Ala Ile Thr Gly Ser Cys Asn Pro Val Gly Gln Asn Tyr
                165                 170                 175

Pro Lys Phe Trp Gly Ala Leu Ala Pro Gly Ser Trp Gln Met Val Leu
            180                 185                 190

Ser Gln Ala Gly His Met Gln Phe Ala Arg Thr Gly Asn Pro Phe Leu
        195                 200                 205

Asp Trp Ser Leu Asp Arg Leu Cys Gly Arg Gly Thr Met Met Ser Ser
    210                 215                 220

Asp Val Ile Thr Tyr Ser Ala Ala Phe Thr Val Ala Trp Phe Glu Gly
225                 230                 235                 240

Ile Phe Arg Pro Ala Gln Ser Gln Met Gly Ile Ser Asn Phe Lys Thr
                245                 250                 255

Trp Ala Asn Thr Gln Val Ala Ala Arg Ser Ile Thr Phe Asp Ile Lys
            260                 265                 270

Pro Met Gln Ser Pro Gln Gly Thr Gly Glu Asn Leu Tyr Phe Gln Gly
        275                 280                 285

Ser Gly Gly Gly Gly Ser Asp Tyr Lys Asp Asp Asp Asp Lys Gly Thr
    290                 295                 300

Gly
305

<210> 51
<211> 315
<212> PRT

<213> Artificial Sequence

<220>
<223> Description of Artificial Sequence: Synthetic
      polypeptide

<400> 51
Met Val Pro Ala Pro Pro Lys Pro Val Arg Ile Thr Cys Pro Thr Val
1               5                   10                  15

Ala Gly Thr Tyr Pro Val Val Leu Phe Phe His Gly Phe Tyr Leu Arg
            20                  25                  30

Asn Tyr Phe Tyr Ser Asp Val Leu Asn His Ile Ala Ser His Gly Tyr
        35                  40                  45

Ile Leu Val Ala Pro Gln Leu Cys Lys Leu Leu Pro Pro Gly Gly Gln
    50                  55                  60

Val Glu Val Asp Asp Ala Gly Ser Val Ile Asn Trp Ala Ser Glu Asn
65                  70                  75                  80

Leu Lys Ala His Leu Pro Thr Ser Val Asn Ala Asn Gly Lys Tyr Thr
                85                  90                  95

Ser Leu Val Gly His Ser Arg Gly Gly Lys Thr Ala Phe Ala Val Ala
            100                 105                 110

Leu Gly His Ala Ala Thr Leu Asp Pro Ser Ile Thr Phe Ser Ala Leu
        115                 120                 125

Ile Gly Ile Asp Pro Val Ala Gly Thr Asn Lys Tyr Ile Arg Thr Asp
    130                 135                 140

Pro His Ile Leu Thr Tyr Lys Pro Glu Ser Phe Glu Leu Asp Ile Pro
145                 150                 155                 160

Val Ala Val Val Gly Thr Gly Leu Gly Pro Lys Trp Asn Asn Val Met
                165                 170                 175

Pro Pro Cys Ala Pro Thr Asp Leu Asn His Glu Glu Phe Tyr Lys Glu
            180                 185                 190

Cys Lys Ala Thr Lys Ala His Phe Val Ala Ala Asp Tyr Gly His Met
        195                 200                 205

Asp Met Leu Asp Asp Asp Leu Pro Gly Phe Val Gly Phe Met Ala Gly
    210                 215                 220

Cys Met Cys Lys Asn Gly Gln Arg Lys Lys Ser Glu Met Arg Ser Phe
225                 230                 235                 240

Val Gly Gly Ile Val Val Ala Phe Leu Lys Tyr Ser Leu Trp Gly Glu
                245                 250                 255

Lys Ala Glu Ile Arg Leu Ile Val Lys Asp Pro Ser Val Ser Pro Ala
            260                 265                 270

Lys Leu Asp Pro Ser Pro Glu Leu Glu Glu Ala Ser Gly Ile Phe Val
        275                 280                 285

Gly Thr Gly Glu Asn Leu Tyr Phe Gln Gly Ser Gly Gly Gly Gly Ser
    290                 295                 300

Asp Tyr Lys Asp Asp Asp Asp Lys Gly Thr Gly
305                 310                 315

<210> 52
<211> 301
<212> PRT
<213> Artificial Sequence

<220>
<223> Description of Artificial Sequence: Synthetic
      polypeptide

<400> 52
Met Val Pro Ala Thr Pro Val Glu Glu Gly Asp Tyr Pro Val Val Met
1               5                   10                  15

Leu Leu His Gly Tyr Leu Leu Tyr Asn Ser Phe Tyr Ser Gln Leu Met
            20                  25                  30

Leu His Val Ser Ser His Gly Phe Ile Leu Ile Ala Pro Gln Leu Tyr
        35                  40                  45

Ser Ile Ala Gly Pro Asp Thr Met Asp Glu Ile Lys Ser Thr Ala Glu
    50                  55                  60

Ile Met Asp Trp Leu Ser Val Gly Leu Asn His Phe Leu Pro Ala Gln
65                  70                  75                  80

Val Thr Pro Asn Leu Ser Lys Phe Ala Leu Ser Gly His Ser Arg Gly
                85                  90                  95

Gly Lys Thr Ala Phe Ala Val Ala Leu Lys Lys Phe Gly Tyr Ser Ser
            100                 105                 110

Asn Leu Lys Ile Ser Thr Leu Ile Gly Ile Asp Pro Val Asp Gly Thr

115                 120                 125

Gly Lys Gly Lys Gln Thr Pro Pro Pro Val Leu Ala Tyr Leu Pro Asn
    130                 135                 140

Ser Phe Asp Leu Asp Lys Thr Pro Ile Leu Val Ile Gly Ser Gly Leu
145                 150                 155                 160

Gly Glu Thr Ala Arg Asn Pro Leu Phe Pro Pro Cys Ala Pro Pro Gly
                165                 170                 175

Val Asn His Arg Glu Phe Phe Arg Glu Cys Gln Gly Pro Ala Trp His
            180                 185                 190

Phe Val Ala Lys Asp Tyr Gly His Leu Asp Met Leu Asp Asp Asp Thr
        195                 200                 205

Lys Gly Ile Arg Gly Lys Ser Ser Tyr Cys Leu Cys Lys Asn Gly Glu
    210                 215                 220

Glu Arg Arg Pro Met Arg Arg Phe Val Gly Gly Leu Val Val Ser Phe
225                 230                 235                 240

Leu Lys Ala Tyr Leu Glu Gly Asp Asp Arg Glu Leu Val Lys Ile Lys
                245                 250                 255

Asp Gly Cys His Glu Asp Val Pro Val Glu Ile Gln Glu Phe Glu Val
            260                 265                 270

Ile Met Gly Thr Gly Glu Asn Leu Tyr Phe Gln Gly Ser Gly Gly Gly
        275                 280                 285

Gly Ser Asp Tyr Lys Asp Asp Asp Asp Lys Gly Thr Gly
    290                 295                 300

<210> 53
<211> 534
<212> PRT
<213> Artificial Sequence

<220>
<223> Description of Artificial Sequence: Synthetic
      polypeptide

<400> 53
Met Val Pro Ser His Lys Lys Lys Asn Val Ile Phe Phe Val Thr Asp
1               5                   10                  15

Gly Met Gly Pro Ala Ser Leu Ser Met Ala Arg Ser Phe Asn Gln His
            20                  25                  30

Val Asn Asp Leu Pro Ile Asp Asp Ile Leu Thr Leu Asp Glu His Phe
        35                  40                  45

Ile Gly Ser Ser Arg Thr Arg Ser Ser Asp Ser Leu Val Thr Asp Ser
    50                  55                  60

Ala Ala Gly Ala Thr Ala Phe Ala Cys Ala Leu Lys Ser Tyr Asn Gly
65                  70                  75                  80

Ala Ile Gly Val Asp Pro His His Arg Pro Cys Gly Thr Val Leu Glu
                85                  90                  95

Ala Ala Lys Leu Ala Gly Tyr Leu Thr Gly Leu Val Val Thr Thr Arg
            100                 105                 110

Ile Thr Asp Ala Thr Pro Ala Ser Phe Ser Ser His Val Asp Tyr Arg
        115                 120                 125

Trp Gln Glu Asp Leu Ile Ala Thr His Gln Leu Gly Glu Tyr Pro Leu
    130                 135                 140

Gly Arg Val Val Asp Leu Leu Met Gly Gly Gly Arg Ser His Phe Tyr
145                 150                 155                 160

Pro Gln Gly Glu Lys Ala Ser Pro Tyr Gly His His Gly Ala Arg Lys
                165                 170                 175

Asp Gly Arg Asp Leu Ile Asp Glu Ala Gln Ser Asn Gly Trp Gln Tyr
            180                 185                 190

Val Gly Asp Arg Lys Asn Phe Asp Ser Leu Leu Lys Ser His Gly Glu
        195                 200                 205

Asn Val Thr Leu Pro Phe Leu Gly Leu Phe Ala Asp Asn Asp Ile Pro
    210                 215                 220

Phe Glu Ile Asp Arg Asp Glu Lys Glu Tyr Pro Ser Leu Lys Glu Gln
225                 230                 235                 240

Val Lys Val Ala Leu Gly Ala Leu Glu Lys Ala Ser Asn Glu Asp Lys
                245                 250                 255

Asp Ser Asn Gly Phe Phe Leu Met Val Glu Gly Ser Arg Ile Asp His
            260                 265                 270

Ala Gly His Gln Asn Asp Pro Ala Ser Gln Val Arg Glu Val Leu Ala

        275                 280                 285

Phe Asp Glu Ala Phe Gln Tyr Val Leu Glu Phe Ala Glu Asn Ser Asp
    290                 295                 300

Thr Glu Thr Val Leu Val Ser Thr Ser Asp His Glu Thr Gly Gly Leu
305                 310                 315                 320

Val Thr Ser Arg Gln Val Thr Ala Ser Tyr Pro Gln Tyr Val Trp Tyr
                325                 330                 335

Pro Gln Val Leu Ala Asn Ala Thr His Ser Gly Glu Phe Leu Lys Arg
            340                 345                 350

Lys Leu Val Asp Phe Val His Glu His Lys Gly Ala Ser Ser Lys Ile
        355                 360                 365

Glu Asn Phe Ile Lys His Glu Ile Leu Glu Lys Asp Leu Gly Ile Tyr
    370                 375                 380

Asp Tyr Thr Asp Ser Asp Leu Glu Thr Leu Ile His Leu Asp Pro Arg
385                 390                 395                 400

Thr Asn Ala Ile Gln Asp Lys Leu Asn Asp Met Val Ser Phe Arg Ala
                405                 410                 415

Gln Ile Gly Trp Thr Thr His Gly His Ser Ala Val Asp Val Asn Ile
            420                 425                 430

Tyr Ala Tyr Ala Asn Lys Lys Ala Thr Trp Ser Tyr Val Leu Asn Asn
        435                 440                 445

Leu Gln Gly Asn His Glu Asn Thr Glu Val Gly Gln Phe Leu Glu Asn
    450                 455                 460

Phe Leu Glu Leu Asn Leu Asn Glu Val Thr Asp Leu Ile Arg Asp Thr
465                 470                 475                 480

Lys His Thr Ser Asp Phe Asp Ala Thr Glu Ile Ala Ser Glu Val Gln
                485                 490                 495

His Tyr Asp Glu Tyr Tyr His Glu Leu Thr Asn Gly Thr Gly Glu Asn
            500                 505                 510

Leu Tyr Phe Gln Gly Ser Gly Gly Gly Gly Ser Asp Tyr Lys Asp Asp
        515                 520                 525

Asp Asp Lys Gly Thr Gly
    530

<210> 54
<211> 396
<212> PRT
<213> Artificial Sequence

<220>
<223> Description of Artificial Sequence: Synthetic
      polypeptide

<400> 54
Met Val Pro His Lys Phe Thr Gly Val Asn Ala Lys Phe Gln Gln Pro
1               5                   10                  15

Ala Leu Arg Asn Leu Ser Pro Val Val Val Glu Arg Glu Arg Glu Glu
            20                  25                  30

Phe Val Gly Phe Phe Pro Gln Ile Val Arg Asp Leu Thr Glu Asp Gly
        35                  40                  45

Ile Gly His Pro Glu Val Gly Asp Ala Val Ala Arg Leu Lys Glu Val
    50                  55                  60

Leu Gln Tyr Asn Ala Pro Gly Gly Lys Cys Asn Arg Gly Leu Thr Val
65                  70                  75                  80

Val Ala Ala Tyr Arg Glu Leu Ser Gly Pro Gly Gln Lys Asp Ala Glu
                85                  90                  95

Ser Leu Arg Cys Ala Leu Ala Val Gly Trp Cys Ile Glu Leu Phe Gln
            100                 105                 110

Ala Phe Phe Leu Val Trp Asp Asp Ile Met Asp Gln Ser Leu Thr Arg
        115                 120                 125

Arg Gly Gln Leu Cys Trp Tyr Lys Lys Glu Gly Val Gly Leu Asp Ala
    130                 135                 140

Ile Asn Asp Ser Phe Leu Leu Glu Ser Ser Val Tyr Arg Val Leu Lys
145                 150                 155                 160

Lys Tyr Cys Arg Gln Arg Pro Tyr Tyr Val His Leu Leu Glu Leu Phe
                165                 170                 175

Leu Gln Thr Ala Tyr Gln Thr Glu Leu Gly Gln Met Leu Asp Leu Ile
            180                 185                 190

Thr Ala Pro Val Ser Lys Val Asp Leu Ser His Phe Ser Glu Glu Arg

195                 200                 205

Tyr Lys Ala Ile Val Lys Tyr Lys Thr Ala Phe Tyr Ser Phe Tyr Leu
    210                 215                 220

Pro Val Ala Ala Ala Met Tyr Met Val Gly Ile Asp Ser Lys Glu Glu
225                 230                 235                 240

His Glu Asn Ala Lys Ala Ile Leu Leu Glu Met Gly Glu Tyr Phe Gln
                245                 250                 255

Ile Gln Asp Asp Tyr Leu Asp Cys Phe Gly Asp Pro Ala Leu Thr Gly
            260                 265                 270

Lys Val Gly Thr Asp Ile Gln Asp Asn Lys Cys Ser Trp Leu Val Val
        275                 280                 285

Gln Cys Leu Gln Arg Val Thr Pro Glu Gln Arg Gln Leu Leu Glu Asp
    290                 295                 300

Asn Tyr Gly Arg Lys Glu Pro Glu Lys Val Ala Lys Val Lys Glu Leu
305                 310                 315                 320

Tyr Glu Ala Val Gly Met Arg Ala Ala Phe Gln Gln Tyr Glu Glu Ser
                325                 330                 335

Ser Tyr Arg Arg Leu Gln Glu Leu Ile Glu Lys His Ser Asn Arg Leu
            340                 345                 350

Pro Lys Glu Ile Phe Leu Gly Leu Ala Gln Lys Ile Tyr Lys Arg Gln
        355                 360                 365

Lys Gly Thr Gly Glu Asn Leu Tyr Phe Gln Gly Ser Gly Gly Gly Gly
    370                 375                 380

Ser Asp Tyr Lys Asp Asp Asp Asp Lys Gly Thr Gly
385                 390                 395

<210> 55
<211> 27
<212> DNA
<213> Artificial Sequence

<220>
<223> Description of Artificial Sequence: Synthetic
      primer

<400> 55
gtgctaggta actaacgttt gattttt

<210> 56
<211> 20
<212> DNA
<213> Artificial Sequence

<220>
<223> Description of Artificial Sequence: Synthetic
      primer

<400> 56
tgggttcata tctggacgtt                                                   20


<210> 57
<211> 23
<212> DNA
<213> Artificial Sequence

<220>
<223> Description of Artificial Sequence: Synthetic
      primer

<400> 57
ggaaggggac gtaggtacat aaa                                               23


<210> 58
<211> 23
<212> DNA
<213> Artificial Sequence

<220>
<223> Description of Artificial Sequence: Synthetic
      primer

<400> 58
ttagaacgtg ttttgttccc aat                                               23


<210> 59
<211> 20
<212> DNA
<213> Artificial Sequence

<220>
<223> Description of Artificial Sequence: Synthetic
      primer

<400> 59
ccgaactgag gttgggttta                                                   20


<210> 60
<211> 20
<212> DNA
<213> Artificial Sequence

<220>
<223> Description of Artificial Sequence: Synthetic
      primer

<400> 60

gggggagcga ataggattag                                                    20

<210> 61
<211> 21
<212> DNA
<213> Artificial Sequence

<220>
<223> Description of Artificial Sequence: Synthetic
      primer

<400> 61
gcaacttcag ctgtttgacc t                                                  21

<210> 62
<211> 23
<212> DNA
<213> Artificial Sequence

<220>
<223> Description of Artificial Sequence: Synthetic
      primer

<400> 62
aaatttaacg taacgatgag ttg                                                23

<210> 63
<211> 23
<212> DNA
<213> Artificial Sequence

<220>
<223> Description of Artificial Sequence: Synthetic
      primer

<400> 63
ttagaacgtg ttttgttccc aat                                                23

<210> 64
<211> 24
<212> DNA
<213> Artificial Sequence

<220>
<223> Description of Artificial Sequence: Synthetic
      primer

<400> 64
tggtacaaga ggatttttgt tgtt                                               24

<210> 65
<211> 23
<212> DNA
<213> Artificial Sequence

<220>
<223> Description of Artificial Sequence: Synthetic
      primer

<400> 65
aaatttaacg taacgatgag ttg                                              23


<210> 66
<211> 22
<212> DNA
<213> Artificial Sequence

<220>
<223> Description of Artificial Sequence: Synthetic
      primer

<400> 66
cgttcttctg agaaatggct ta                                               22


<210> 67
<211> 20
<212> DNA
<213> Artificial Sequence

<220>
<223> Description of Artificial Sequence: Synthetic
      primer

<400> 67
attagcatca gagccgcatt                                                  20


<210> 68
<211> 20
<212> DNA
<213> Artificial Sequence

<220>
<223> Description of Artificial Sequence: Synthetic
      primer

<400> 68
ctcgcctatc ggctaacaag                                                  20


<210> 69
<211> 20
<212> DNA
<213> Artificial Sequence

<220>
<223> Description of Artificial Sequence: Synthetic
      primer

<400> 69
   cacaagaagc aaccccttga                                                 20


<210> 70
<211> 21
<212> DNA
<213> Artificial Sequence

<220>

<223> Description of Artificial Sequence: Synthetic
      primer

<400> 70
cgacggaata aaggtgtacg a                                                21


<210> 71
<211> 24
<212> DNA
<213> Artificial Sequence

<220>
<223> Description of Artificial Sequence: Synthetic
      primer

<400> 71
tggtacaaga ggatttttgt tgtt                                             24


<210> 72
<211> 20
<212> DNA
<213> Artificial Sequence

<220>
<223> Description of Artificial Sequence: Synthetic
      primer

<400> 72
tcagcaattg cagcgtaata                                                  20


<210> 73
<211> 22
<212> DNA
<213> Artificial Sequence

<220>
<223> Description of Artificial Sequence: Synthetic
      primer

<400> 73
gacgttcttg acgttttgtt tg                                               22


<210> 74
<211> 1718
<212> DNA
<213> Artificial Sequence

<220>
<223> Description of Artificial Sequence: Synthetic
      polynucleotide

<400> 74
atggtaccaa gacgttcagg taactataat cctagccgtt gggacgtaaa tttcattcaa      60

tcttattatc tgattataaa gaagataaac acgttattag agcttctgaa ttagtaacac     120

ttgttaagat ggaattagaa aaagaaacag atcaaatccg tcaattagaa ttaattgacg     180

atttacaacg tatgggttta tctgatcatt tccaaaacga atttaaagaa atcttatcaa     240

gtatttactt agatcatcat tattacaaaa atccatttcc aaaagaagag cgtgatttat 300

actcaactag cttagctttt cgtttattac gtgaacacgg ttttcaagta gcacaagaag 360

tttttgattc attcaaaaat gaagagggtg aatttaagga gagcttatct gacgatactc 420

gtggcttatt acaattatat gaagcatcat tcttattaac agagggtgaa acaaccttag 480

aaagtgcacg cgaatttgct acaaaatttt tagaagaaaa agttaacgaa ggtggcgttg 540

atggtgactt attaacaaga attgcttact cattagatat tcccttacat tggcgcatta 600

aacgtcctaa tgccccagtt tggattgaat ggtatcgtaa acgtccagat atgaacccag 660

tggttttaga attagcaatt ttagacttaa acattgtaca agctcaattt caagaggaat 720

taaaagagtc ttttcgctgg tggcgtaata ctggttttgt tgagaaatta ccatttgcac 780

gtgatcgttt agttgaatgt tacttttgga acactggtat tattgaacca cgtcaacacg 840

catcagctcg tattatgatg ggtaaagtaa atgcattaat tacagtaatt gatgacatct 900

atgatgttta tggaacactt gaagaattag aacaattcac tgatttaatt cgcagatggg 960

acataaactc aatagatcaa ttaccagatt atatgcaatt atgttttctt gcattaaaca 1020

atttcgttga tgacacttca tacgatgtta tgaaagaaaa gggtgttaat gttattcctt 1080

acttacgtca atcttgggta gaccttgcag acaaatatat ggtagaagca cgttggttct 1140

acggtggcca taaaccatca ttagaagaat acttagaaaa ttcttggcaa tctatctcag 1200

gtccatgtat gttaactcat atattctttc gtgtaacaga tagctttact aaagaaactg 1260

ttgattctct ttacaaatat catgatttag ttagatggtc atcattcgtg cttcgtcttg 1320

ctgacgactt aggtacaagc gttgaagaag tatctcgtgg tgatgtgcca aaatctttac 1380

aatgctacat gagtgattat aacgctagtg aggctgaagc acgtaaacac gtaaaatggt 1440

taattgcaga agtatggaaa aagatgaatg cagaacgtgt ttctaaagat agtccttttg 1500

gtaaagattt tataggttgt gctgttgatt taggtcgtat ggctcaatta atgtatcaca 1560

atggagatgg tcatggtact caacacccta ttattcatca acaaatgaca cgtactttat 1620

ttgaaccatt cgctggtacc ggtgaaaact tatactttca aggctcaggt ggcggtggaa 1680

gtgattacaa agatgatgat gataaaggaa ccggttaa 1718

<210> 75
<211> 1698
<212> DNA
<213> Artificial Sequence

<220>
<223> Description of Artificial Sequence: Synthetic
      polynucleotide

<400> 75
atggtaccaa gacgtactgg tggctatcaa cctacacttt gggatttttc aacaattcaa 60

ttatttgata gtgaatataa agaagaaaaa catcttatgc gtgctgctgg tatgattgct 120

caagtgaaca tgttacttca agaagaagta gacagcatcc aacgtcttga attaattgat 180

gacttacgtc gtttaggtat atcttgccac tttgatcgtg aaattgtaga gattttaaac 240

agtaaatact acaccaacaa tgaaattgat gaatcagatt tatacagtac agcacttaga 300

ttcaaacttt tacgtcaata tgattttagc gttagccaag aagtttttga ttgttttaaa 360

aatgacaaag gtacagattt caaaccatca ttagttgacg atacacgtgg cttattacaa 420

ttatatgaag catcattttt atcagctcag ggtgaagaaa ctttacattt agcacgtgat 480

tttgctacta aattcttaca taaaagagtt ttagtagata aagatatcaa tttattatct 540

agtatcgagc gtgctttaga attaccaaca cactggcgtg tacaaatgcc taacgctaga 600

tcattcatcg acgcatataa aagaagacca gacatgaacc ctacagtatt agagttagca 660

aaacttgact ttaacatggt tcaagcacag ttccaacaag aattaaaaga agccagtcgc 720

tggtggaact ctacaggatt agtacatgaa ttaccatttg tacgtgatcg tattgtggaa 780

tgttattatt ggactactgg tgtagtagaa cgtcgtgaac acggttacga acgtattatg 840

ttaacaaaaa ttaacgcttt agttacaaca atcgatgatg tttttgacat ttatggtact 900

ttagaagaat tacaactttt tacaactgct attcaaagat gggacattga gtctatgaaa 960

caacttccac cctatatgca aatctgctac ttagctttat tcaacttcgt aaatgagatg 1020

gcttacgata cattacgtga taaaggtttt aatagtactc catatttacg caaagcctgg 1080

gtagacttag tagaaagcta cttaattgaa gctaaatggt attatatggg tcacaaacca 1140

agtttagaag agtacatgaa aaactcatgg atttctatcg gaggtattcc aattttatca 1200

catttattct ttcgtttaac agacagtatc gaagaagaag acgctgaatc aatgcataaa 1260

tatcacgata tagtacgtgc ctcttgtact attttacgtt tagctgatga tatgggtaca 1320

tcattagatg aagttgaacg tggcgatgtt cctaaatctg tacaatgcta tatgaatgag 1380

aaaaacgcct ctgaagaaga agcacgtgaa catgttcgta gtttaattga tcagacatgg 1440

aagatgatga ataaagaaat gatgacttca tcattttcaa aatacttcgt acaagtgtct 1500

gcaaatcttg ctcgtatggc acaatggata tatcaacatg aaagtgatgg tttcggtatg 1560

caacactctt tagttaacaa aatgcttcgt ggtttacttt ttgaccgtta tgaaggtacc 1620

ggtgaaaact tatactttca aggctcaggt ggcggtggaa gtgattacaa agatgatgat 1680

gataaaggaa ccggttaa 1698

<210> 76
<211> 1788
<212> DNA
<213> Artificial Sequence

<220>
<223> Description of Artificial Sequence: Synthetic
      polynucleotide

<400> 76
atggtaccac gtcgcatggg tgattttcat tcaaacttat gggatgatga tgtaattcaa      60

tctttaccca cagcttacga agaaaaatct tatcttgaac gtgctgagaa gttaattgga     120

gaagttgaaa atatgttcaa cagtatgagt ttagaagatg gtgaacttat gagtccatta     180

aatgatttaa ttcaacgcct ttggattgtt gattctttag gtagattagg tatccatcgt     240

cactttaaag atgagattaa aagtgcttta gattatgttt acagttactg gggtgaaaac     300

ggaataggtt gtggtcgtga aagtgctgta actgatttaa acagtacagc tttaggcttt     360

cgtacacttc gtttacacgg ttatccagtt tcatctgatg tatttaaagc atttaaaggt     420

caaaatggtc aattcagttg ttcagaaaat atccaaacag atgaagaaat tcgtggtgtt     480

cttaacttat ttagagccag tttaatagcc ttccctggtg agaaaataat ggacgaagct     540

gaaatcttct ctacaaaata cttaaaggaa gcattacaaa agatcccagt tagttcatta     600

tcacgtgaaa tcggtgatgt acttgaatat ggatggcata catacttacc acgtttagaa     660

gcacgtaact atattcatgt tttcggacaa gatacagaga atacaaaaag ttatgtaaaa     720

tcaaagaaac ttttagaatt agctaaatta gaatttaaca tttttcagag cttacaaaaa     780

cgtgaattag aaagccttgt tcgttggtgg aaagaatctg gatttcctga aatgacattc     840

tgtagacaca gacacgtgga atattacaca cttgcatcat gtattgcatt cgaacctcag     900

catagtggtt ttcgtttagg ttttgctaaa acatgtcacc ttataacagt tttagatgac     960

atgtatgaca ctttcggcac cgtagacgaa ttagagttat ttacagcaac tatgaaacgt    1020

tgggacccaa gttcaattga ctgccttcca gaatacatga aaggagttta cattgctgtg    1080

tatgatacag ttaatgaaat ggctcgtgaa gctgaggaag ctcaaggtcg cgatacactt    1140

acatacgctc gtgaggcctg ggaggcttat atagattctt atatgcaaga agctcgctgg    1200

attgctactg gatacttacc ttctttcgat gaatattatg aaaatggtaa ggtttcatgt    1260

ggtcaccgta tatctgcttt acaaccaatt cttactatgg atattccatt tccagatcac    1320

attttaaagg aagttgactt tccttctaaa cttaatgact tagcttgtgc tatcttacgc    1380

cttcgcggtg atactcgttg ttacaaagca gaccgtgcac gtggtgaaga ggctagttct    1440

atttcttgtt atatgaaaga taatccaggt gtttctgaag aagatgcctt agatcatatt    1500

aacgcaatga tcagtgatgt tattaagggc ttaaactggg aattacttaa acccgacatt    1560

aacgtaccta tttctgctaa gaaacatgct ttcgacattg ctcgtgcttt tcactacggt    1620

tataaatatc gtgatggcta ttcagttgct aatgttgaaa caaaatcttt agttacacgt    1680

actttacttg aatcagttcc attaggtacc ggtgaaaact tatactttca aggctcaggt    1740

ggcggtggaa gtgattacaa agatgatgat gataaaggaa ccggttaa 1788

<210> 77
<211> 1773
<212> DNA
<213> Artificial Sequence

<220>
<223> Description of Artificial Sequence: Synthetic
      polynucleotide

<400> 77
atggtaccac gtagagttgg taattatcat tctaatcttt gggatgatga ttttatacaa 60

agtttaattt ctacacctta cggtgctcct gactaccgtg aacgcgctga tcgtcttatt 120

ggtgaagtaa aagatattat gtttaatttc aaatctttag aggatggtgg taatgactta 180

ttacaacgtt tacttttagt tgatgacgta gaacgtttag gcattgatcg tcatttcaaa 240

aaggaaatta agactgcatt agattatgta aatagttatt ggaatgaaaa aggaattggt 300

tgtggtcgtg agtctgtagt tacagactta aattcaactg ctttaggcct tcgtacctta 360

agattacatg gttatactgt tagctctgac gttttaaatg tttttaaaga taaaaatggt 420

caattttcat ctacagctaa tattcaaatt gaaggtgaaa ttcgtggtgt tttaaatctt 480

tttcgtgcct ctcttgtagc ttttccaggt gagaaagtga tggatgaggc tgaaactttt 540

tcaacaaaat atcttcgtga agcattacag aaaattcctg ccagttcaat tttatcatta 600

gaaatacgtg atgtattaga atatggatgg catactaatt taccacgttt agaagcacgt 660

aattacatgg atgttttcgg tcagcacacc aagaacaaaa atgcagccga aaaattactt 720

gaattagcaa aattagagtt caatatcttt cacagcttac aagaacgtga attaaagcac 780

gtttcaagat ggtggaaaga ctctggtagt ccagagatga ctttctgtcg ccaccgccat 840

gtggaatatt atgctttagc ttcttgtatt gctttcgaac cccagcacag tggtttccgt 900

ttaggtttta ctaaaatgag tcatttaatc acagtgttag atgatatgta tgatgtattc 960

ggtacagttg atgaattaga gttatttacc gccactatta aacgttggga cccttctgct 1020

atggaatgtt taccagagta catgaaaggt gtttacatga tggtttatca tacagttaac 1080

gaaatggctc gtgtggcaga aaaggctcaa ggtagagaca cattaaacta tgctcgtcaa 1140

gcctgggaag catgttttga ctcttatatg caagaagcaa aatggattgc aacaggttac 1200

ttacctacat tcgaggaata tttagaaaat ggtaaagtga gttcagcaca tcgtccttgt 1260

gcattacaac ctattttaac tcttgatatt ccatttcccg atcatattct taaagaagtg 1320

gatttcccaa gcaaacttaa tgacttaatt tgtattatct tacgtcttag aggagacaca 1380

cgttgctata aagcagaccg tgcccgtggt gaagaagcat catcaatatc ttgttatatg 1440

aaagataacc caggtttaac tgaagaagat gctttaaacc acattaactt tatgattcgt 1500

gacgcaatcc gcgaattaaa ctgggagtta cttaaaccag ataatagtgt tccaattact 1560

tcaaagaaac atgcttttga tatttcacgt gtgtggcacc acggataccg ttatcgtgat 1620

ggttacagct ttgcaaacgt ggaaactaaa agtcttgtaa tgcgtactgt aatagaacca 1680

gtaccattag gtaccggtga aaacttatac tttcaaggct caggtggcgg tggaagtgat 1740

tacaaagatg atgatgataa aggaaccggt taa 1773

<210> 78
<211> 1710
<212> DNA
<213> Artificial Sequence

<220>
<223> Description of Artificial Sequence: Synthetic
      polynucleotide

<400> 78
atggtaccac gtcgttcagg agattatcaa ccaagtttat gggactttaa ttacattcaa 60

tctttaaaca caccttacaa agaacaacgt cattttaatc gtcaagctga gttaattatg 120

caagttcgta tgttattaaa ggtaaaaatg gaagcaattc aacaattaga gttaatagat 180

gatttacagt acttaggatt atcatatttc tttcaagacg aaattaaaca aatcttaagc 240

tctattcaca atgaacctcg ttattttcat aataatgacc tttatttcac tgctttaggt 300

tttagaattt tacgtcaaca tggttttaat gtttcagaag acgtatttga ctgctttaaa 360

atcgaaaaat gttctgactt taatgctaac ttagctcagg acacaaaggg tatgttacaa 420

ttatatgaag ctagtttctt attaagagaa ggagaagata cacttgaatt agctcgtcgt 480

tttagtacac gttctttacg tgaaaaattt gatgaaggtg gtgacgagat agatgaagat 540

ttaagtagtt ggattcgtca ttctttagat ttaccattac actggcgtgt tcaaggttta 600

gaagctcgtt ggtttttaga tgcctatgct cgtcgtccag atatgaaccc tcttattttc 660

aaattagcta aattaaattt taacattgtt caagctacat accaagaaga attaaaagac 720

atctctcgtt ggtggaacag tagttgttta gcagagaaat tacccttcgt tcgcgatcgt 780

attgtagaat gtttcttctg ggctattgct gctttcgaac cacaccaata ctcatatcaa 840

cgtaaaatgg ccgctgtaat tattacattt attactatta ttgatgatgt ttacgatgta 900

tatggtacta ttgaagaatt agagttatta acagatatga ttcgtagatg ggataataag 960

agtattagtc aacttcctta ctatatgcaa gtttgttatt tagctcttta taacttcgta 1020

agtgaacgcg catacgacat cttaaaagat caacacttta acagtattcc ataccttcaa 1080

agaagttggg tttcattagt tgagggatac ttaaaagaag catattggta ctataacggt 1140

tacaaaccaa gtcttgaaga atatcttaat aatgcaaaaa ttagtattag tgcacccacc 1200

attatttcac aattatactt tactttagca aatagtatcg acgaaactgc cattgaaagt 1260

ttataccaat atcacaacat tttatactta tcaggtacta tcttacgttt agctgatgat 1320

ttaggaactt cacaacatga attagaacgt ggtgatgttc ccaaagctat tcaatgttat 1380

atgaatgata caaatgcatc agaaagagaa gctgtagaac atgttaaatt tcttattcgt 1440

gaagcctgga aagaaatgaa tacagttact accgcatcag attgtccttt tacagacgat 1500

cttgttgccg ccgcagctaa tttagctcgt gctgctcaat tcatttactt agatggtgat 1560

ggtcatggtg tacaacatag cgaaattcat cagcaaatgg gcggtcttct tttcaaccca 1620

tacgttggta ccggtgaaaa cttatacttt caaggctcag gtggcggtgg aagtgattac 1680

aaagatgatg atgataaagg aaccggttaa 1710

<210> 79
<211> 1785
<212> DNA
<213> Artificial Sequence

<220>
<223> Description of Artificial Sequence: Synthetic
      polynucleotide

<400> 79
atggtaccac gcagaattgg tgattaccat agtaacattt gggatgatga ttttatccag 60

tcactttcta ccccttatgg tgaaccatct taccaagaaa gagctgaacg tcttattgta 120

gaagtgaaaa agattttcaa cagtatgtac ttagatgacg gtcgtttaat gagttctttt 180

aatgacttaa tgcaacgttt atggattgta gactcagtag aacgtttagg tattgcccgt 240

cacttcaaaa atgaaattac atctgccctt gactatgttt ttcgttattg ggaagaaaac 300

ggtataggtt gtggtcgtga ttctattgta actgacttaa atagcacagc tttaggtttt 360

cgtacacttc gtttacacgg ttatacagtt tctccagagg ttttaaaagc atttcaagat 420

caaaatggtc aattcgtttg ttcaccagga caaacagaag gtgaaattcg ttcagtttta 480

aatttatatc gtgcaagttt aattgccttt ccaggcgaaa aagttatgga agaagcagaa 540

atcttctcta ctcgctattt aaaagaagct cttcaaaaga ttccagttag cgcattatca 600

caagaaatca aatttgttat ggaatatgga tggcatacaa atttacctag attagaagca 660

cgtaactata ttgatacttt agaaaaggat acatcagctt ggttaaacaa aaatgcaggt 720

aaaaagttat tagaattagc taaattagaa tttaacatct ttaactcatt acaacaaaaa 780

gaattacaat acttacttcg ctggtggaaa gaatctgact tacctaaatt aacctttgca 840

cgtcatagac acgttgaatt ttacacatta gcttcttgta ttgctattga tcccaaacat 900

tcagcattcc gtttaggatt cgctaaaatg tgtcacttag ttacagttct tgacgatatt 960

tatgatactt tcggtactat tgatgaactt gagttattta cttctgcaat taaacgttgg 1020

aatagttctg aaattgaaca cttaccagaa tatatgaaat gcgtgtatat ggttgttttt 1080

gaaactgtta atgaattaac tcgtgaagct gagaaaacac aaggacgtaa cactttaaac 1140

tatgttcgta aagcatggga agcatatttt gattcttata tggaggaagc aaagtggatc 1200

tcaaacggat atttaccaat gtttgaagaa taccacgaaa atggtaaagt gtcatctgca 1260

taccgtgtag caacattaca accaatttta actttaaacg cttggttacc cgactacatt 1320

cttaaaggaa ttgatttccc aagtcgtttt aacgatttag ctagttcatt cttacgttta 1380

cgtggcgata ctcgctgtta caaagctgac cgtgatcgtg gtgaagaagc tagctgcatt 1440

tcttgttaca tgaaagataa tccaggttct accgaagaag atgcacttaa tcacattaac 1500

gctatggtaa atgacatcat taaagaatta aactgggaat tattacgcag taatgataat 1560

attcctatgt tagctaaaaa gcacgctttt gatattactc gtgcacttca ccacttatac 1620

atttatcgcg atggtttcag tgttgctaat aaagaaacta aaaagttagt tatggagaca 1680

ttacttgaat caatgttatt tggtaccggt gaaaacttat actttcaagg ctcaggtggc 1740

ggtggaagtg attacaaaga tgatgatgat aaaggaaccg gttaa 1785

<210> 80
<211> 2487
<212> DNA
<213> Artificial Sequence

<220>
<223> Description of Artificial Sequence: Synthetic
      polynucleotide

<400> 80
atggtaccac aatctgctga aaagaacgac tctttatcaa gttctacatt agttaagaga 60

gaatttccac ccggtttctg gaaagacgac ttaatcgaca gtttaacttc aagtcacaaa 120

gtagctgcta gcgatgaaaa acgtatcgaa accttaattt cagaaattaa gaatatgttt 180

cgttgtatgg gttatggtga gacaaatcca tcagcttatg atactgcttg ggtagctcgc 240

atcccagcag ttgatggatc agataatcct cactttccag agactgtgga atggatctta 300

caaaatcaat taaaagatgg ttcttggggt gaaggttttt acttccttgc ttatgatcgc 360

attttagcca ctttagcttg tattatcaca cttacacttt ggcgtactgg agaaacacaa 420

gtacagaaag gtatcgaatt tttccgcact caagcaggta aaatggaaga tgaagcagat 480

tcacaccgtc caagtggttt tgagattgta tttcctgcta tgttaaaaga ggctaagatt 540

ttaggcttag atttacctta tgatcttcct tttcttaaac aaattattga aaagagagaa 600

gctaagttaa aacgtattcc tacagatgtt ttatatgctt taccaactac tttactttat 660

tcattagaag gtttacaaga aatagtagac tggcaaaaaa tcatgaaatt acaaagtaaa 720

gatggtagtt tcttatcttc tcctgcctca acagcagcag tatttatgag aacaggtaac 780

aaaaagtgtt tagatttctt aaatttcgtg cttaaaaagt tcggtaatca tgttccatgc 840

cactatcctt tagacctttt tgagcgtctt tgggcagttg atactgttga aagattaggt      900

attgaccgtc attttaaaga agaaataaaa gaggctttag actatgtgta ttcacactgg      960

gacgaacgtg gtattggttg ggctcgtgaa aaccccgttc cagatattga cgatacagca     1020

atgggtcttc gtattttacg tcttcatggt tacaatgtta gcagcgatgt tcttaaaaca     1080

tttcgtgatg aaaatggtga gttcttttgc tttttaggac aaacacaaag aggtgtgact     1140

gatatgttaa atgttaatcg ttgtagccat gtatctttcc ctggtgaaac tataatggaa     1200

gaggcaaaat tatgtactga acgttactta cgcaacgcat tagaaaatgt agacgctttt     1260

gataagtggg catttaagaa aaacattcgt ggtgaggtag aatatgctct taaatatcct     1320

tggcataaat caatgccacg tttagaagca cgttcatata ttgaaaatta cggtccagat     1380

gatgtttggt taggtaaaac tgtttatatg atgccttaca tttcaaatga aaagtactta     1440

gagttagcta aacttgattt taacaaagtt cagtcaatcc accagacaga acttcaagac     1500

ttacgccgtt ggtggaaaag ttctggtttt acagatttaa actttacaag agaacgtgtt     1560

actgaaattt acttttcacc tgcatctttt atcttcgaac cagaatttag taaatgtcgt     1620

gaggtttata caaaaacttc taattttact gtaattttag acgatttata tgacgctcat     1680

ggctctttag atgacttaaa actttttaca gagagtgtta aacgttggga tttatcttta     1740

gttgaccaaa tgccccagca gatgaaaatc tgttttgtag gtttctataa tacattcaac     1800

gatattgcta aagaaggtag agaacgtcaa ggtcgtgatg ttttaggtta tattcaaaac     1860

gtatggaaag tacaacttga agcatatact aaagaagcag aatggtcaga agcaaaatat     1920

gttcctagtt ttaacgaata cattgaaaat gcttcagttt caattgcctt aggtacagta     1980

gtacttatca gtgctttatt taccggagaa gttttaacag atgaagtttt atctaaaatt     2040

gaccgtgaaa gtagattctt acagttaatg ggcttaactg gacgtttagt aaatgatact     2100

aaaacatatc aagctgagcg tggtcaaggt gaagttgcta gtgcaattca atgttatatg     2160

aaagaccacc ctaaaattag tgaagaagaa gcattacaac atgtatattc tgtaatggaa     2220

aatgcattag aagaattaaa tcgtgagttc gttaacaaca aaattccaga catctataaa     2280

cgtcttgttt tcgaaactgc acgtataatg caattatttt acatgcaagg tgatggttta     2340

acattaagtc acgatatgga aattaaagag cacgtaaaga attgtttatt ccagccagta     2400

gctggtaccg gtgaaaactt atactttcaa ggctcaggtg gcggtggaag tgattacaaa     2460

gatgatgatg ataaaggaac cggttaa                                         2487

<210> 81
<211> 2496
<212> DNA
<213> Artificial Sequence

<220>

<223> Description of Artificial Sequence: Synthetic
      polynucleotide

<400> 81
atggtaccat cttcatcaac aggcacttca aaagtagtaa gcgaaacatc ttcaactatt      60

gtagacgata ttccacgtct ttcagcaaat tatcatggtg atttatggca tcacaacgta     120

attcagactt tagaaacacc atttagagaa agttcaactt atcaagagcg tgcagatgaa     180

ttagtagtga aaatcaaaga tatgttcaat gcattaggtg acggtgacat ctcaccttca     240

gcttatgata ctgcatgggt agctcgtgtt gctaccattt cttctgatgg tagcgaaaaa     300

ccacgttttc ctcaagctct taattgggtt tttaacaatc aattacaaga tggatcatgg     360

ggtattgaat cacattttag tttatgcgat cgtttactta atactacaaa ttcagttatt     420

gctttatcag tatggaaaac tggtcactca caggttcaac aaggtgccga atttattgct     480

gaaaatttac gtcttttaaa tgaagaagac gaattaagtc ctgattttca aattatcttc     540

ccagctttat tacagaaagc caaggcttta ggaatcaatt taccctatga tttaccattc     600

atcaaatatc ttagtacaac acgcgaagct cgtttaacag atgtgtcagc tgctgctgac     660

aacataccag ccaatatgct taatgcactt gaaggtttag aagaagtgat tgattggaat     720

aaaatcatgc gttttcaatc taaagatggt tcatttttat cttctccagc tagtacagcc     780

tgtgttttaa tgaatacagg tgatgaaaaa tgtttcacat tcttaaataa cttattagat     840

aaattcggcg gttgtgttcc atgtatgtat agcattgatt tattagaacg tttatcttta     900

gtggacaaca ttgaacactt aggtattggt cgtcacttta aacaagaaat caaaggtgca     960

ttagattatg tatatcgtca ttggtctgaa cgcggtatcg gttggggtag agactcttta    1020

gttccagatt taaacaccac agctttaggt ttacgcacat taagaatgca cggttataac    1080

gtgtctagtg atgtacttaa caatttcaaa gacgaaaatg gtcgtttctt tagtagtgct    1140

ggtcaaacac acgtagagtt acgttctgtt gtaaatcttt ttcgcgcctc agatttagcc    1200

tttccagacg aacgtgcaat ggatgatgct cgtaaattcg cagaaccata tttacgtgaa    1260

gcattagcta caaaaatatc aacaaataca aagttattca aagaaattga atatgttgtt    1320

gaataccctt ggcacatgtc aattccacgt ttagaagctc gtagttatat tgacagttat    1380

gatgataatt atgtatggca acgtaagact ttatatcgta tgccatcatt aagtaattca    1440

aaatgtttag aacttgctaa attagatttc aatattgttc aatctttaca ccaagaagaa    1500

cttaaacttt taactcgttg gtggaaagaa tctggtatgg cagacataaa tttcacccgc    1560

catcgtgtag ctgaagttta cttttctagt gctacatttg agccagaata tagtgctact    1620

cgtattgcat tcacaaaaat tggttgctta caagtacttt tcgatgatat ggctgacatt    1680

ttcgccactt tagatgagtt aaaaagtttt actgaaggtg ttaaacgctg ggacacatca    1740

ttattacatg aaattcccga atgtatgcaa acttgtttta aagtatggtt taaacttatg    1800

gaagaagtaa acaacgacgt agtaaaagtt caaggaagag atatgttagc acatattcgt 1860

aaaccctggg aattatactt taattgttat gttcaagaac gtgaatggtt agaagctggt 1920

tatattccta cattcgaaga atatcttaaa acttatgcta ttagtgtagg ccttggtcct 1980

tgtaccttac aacctattct tttaatgggt gagttagtta aagatgatgt agtagaaaaa 2040

gttcattacc cttctaacat gttcgaatta gtttctttaa gctggcgttt aactaatgat 2100

accaaaacat atcaagcaga aaaagtacgc ggtcaacaag ctagtggcat tgcctgttat 2160

atgaaagaca atccaggtgc tactgaagaa gatgctatta aacacatttg tcgtgttgtt 2220

gatcgtgcat taaaagaagc aagtttcgaa tatttcaagc cttcaaatga cattcctatg 2280

ggttgtaaat cttttatctt taacttacgt ttatgtgtac aaattttcta taaattcatt 2340

gatggttatg gtatcgcaaa cgaagaaatt aaggactaca ttcgtaaggt ttatattgat 2400

ccaattcaag ttggtaccgg tgaaaactta tactttcaag gctcaggtgg cggtggaagt 2460

gattacaaag atgatgatga taaaggaacc ggttaa 2496

<210> 82
<211> 1191
<212> DNA
<213> Artificial Sequence

<220>
<223> Description of Artificial Sequence: Synthetic
      polynucleotide

<400> 82
atggtaccac acaagttcac aggtgttaac gctaaattcc agcaaccagc attaagaaat 60

ttatctccag tggtagttga gcgcgaacgt gaggaatttg taggattctt tccacaaatt 120

gttcgtgact taactgaaga tggtattggt catccagaag taggtgacgc tgtagctcgt 180

cttaaagaag tattacaata caacgcacct ggtggtaaat gcaatagagg tttaacagtt 240

gttgcagctt accgtgaact ttctggacca ggtcaaaaag acgctgaaag tcttcgttgt 300

gctttagcag taggatggtg tattgaatta ttccaagcct ttttcttagt tgctgacgat 360

ataatggacc agtcattaac tagacgtggt caattatgtt ggtacaagaa agaaggtgtt 420

ggtttagatg caataaatga ttcttttctt ttagaaagct ctgtgtatcg cgttcttaaa 480

aagtattgcc gtcaacgtcc atattatgta catttattag agctttttct tcaaacagct 540

taccaaacag aattaggaca aatgttagat ttaatcactg ctcctgtatc taaggtagat 600

ttaagccatt tctcagaaga acgttacaaa gctattgtta agtataaaac tgctttctat 660

tcattctatt taccagttgc agcagctatg tatatggttg gtatagattc taaagaagaa 720

catgaaaacg caaaagctat tttacttgag atgggtgaat acttccaaat tcaagatgat 780

tatttagatt gttttggcga tcctgcttta acaggtaaag taggtactga tattcaagat 840

aacaaatgtt catggttagt tgtgcaatgc ttacaaagag taacaccaga acaacgtcaa 900

cttttagaag ataattacgg tcgtaaagaa ccagaaaaag ttgctaaagt taaagaatta 960

tatgaggctg taggtatgag agccgccttt caacaatacg aagaaagtag ttaccgtcgt 1020

cttcaagagt taattgagaa acattctaat cgtttaccaa aagaaatttt cttaggttta 1080

gctcagaaaa tatacaaacg tcaaaaaggt accggtgaaa acttatactt tcaaggctca 1140

ggtggcggtg gaagtgatta caaagatgat gatgataaag gaaccggtta a 1191

<210> 83
<211> 1728
<212> DNA
<213> Artificial Sequence

<220>
<223> Description of Artificial Sequence: Synthetic
      polynucleotide

<400> 83
atggtaccat cattaactga agaaaaacca attcgcccaa tcgcaaactt tcctccaagc 60

atttggggag atcaattctt aatttacgaa aaacaagtag aacaaggtgt tgaacagatt 120

gttaacgacc ttaagaaaga agtgcgccaa cttttaaaag aggctttaga tattccaatg 180

aaacacgcaa accttttaaa acttattgac gaaattcaac gtcttggtat tccatatcac 240

tttgaacgtg aaattgatca tgcattacaa tgtatctatg aaacttatgg tgataattgg 300

aatggtgatc gttcttcatt atggttccgt ttaatgcgta aacaaggtta ttatgttaca 360

tgtgacgtgt ttaacaatta caaagataaa aatggtgcat ttaaacaatc tttagctaat 420

gatgttgaag gtttattaga attatatgaa gctacttcaa tgcgtgttcc aggtgaaatt 480

attcttgaag atgcattagg ttttacacgt tctcgtttat ctattatgac aaaagacgca 540

tttagtacaa atcctgcttt atttactgaa attcagcgtg cccttaaaca gcctttatgg 600

aaacgtttac caagaattga agctgctcaa tatattccat tttatcaaca acaagattct 660

cacaataaga cattacttaa attagccaaa ttagaattta atcttttaca atcattacat 720

aaagaagaat taagtcatgt gtgtaaatgg tggaaagcat ttgatattaa gaagaatgct 780

ccatgtttac gtgatagaat tgtagagtgt tacttttggg gccttggtag tggttacgag 840

ccacaatatt cacgtgctcg tgtattcttt acaaaagctg ttgcagttat tactttaatt 900

gacgatacct atgatgcata cggaacctat gaggagctta aaattttcac tgaagctgta 960

gaacgttggt ctataacttg tttagatact ttaccagaat atatgaaacc catctacaaa 1020

ttattcatgg acacatacac tgaaatggaa gaatttttag caaaagaagg tcgcacagac 1080

ctttttaact gtggtaaaga atttgttaaa gagtttgttc gtaacttaat ggtagaagct 1140

aagtgggcta atgaaggtca cattcctact acagaagagc acgatccagt agtaataatt 1200

acaggtggag caaacttact taccacaact tgttacttag gtatgtctga catttttaca 1260

aaagaatcag tagagtgggc agtatctgca ccaccattat tccgttattc tggcatactt 1320

ggtcgtcgtc ttaatgattt aatgactcat aaagctgaac aagagcgtaa acactcatca 1380

agtagtttag aaagctatat gaaggaatat aacgttaacg aagagtatgc tcaaacactt 1440

atttacaaag aggttgaaga cgtttggaag gacattaacc gtgaatactt aacaactaaa 1500

aacattccac gtcctctttt aatggctgta atatacttat gtcaattctt agaagtacaa 1560

tacgctggaa aagataactt tacacgtatg ggtgatgaat ataaacactt aataaagagt 1620

ttattagttt atcctatgtc aataggtacc ggtgaaaact tatactttca aggctcaggt 1680

ggcggtggaa gtgattacaa agatgatgat gataaaggaa ccggttaa 1728

<210> 84
<211> 2541
<212> DNA
<213> Artificial Sequence

<220>
<223> Description of Artificial Sequence: Synthetic
      polynucleotide

<400> 84
atggtaccag caggtgtatc agctgtgtca aaagtttctt cattagtatg tgacttaagt 60

agtactagcg gcttaattcg tagaactgca aatcctcacc ctaatgtatg gggttatgac 120

ttagttcatt ctttaaaatc tccatatatt gatagtagct atcgtgaacg tgctgaagtg 180

cttgtaagtg aaataaaagc tatgttaaat ccagcaatta ctggagatgg tgaatcaatg 240

attacacctt cagcttatga cactgcttgg gttgcacgtg taccagcaat tgatggtagc 300

gcacgtccac aatttccaca aacagtagat tggattttaa agaatcaatt aaaagatggt 360

tcttggggta ttcaatcaca ctttttactt tcagaccgtt tattagctac tcttagctgt 420

gttttagttt tacttaaatg gaatgttggt gatttacagg ttgagcaagg tattgagttt 480

attaagtcaa accttgaatt agtaaaagat gaaactgatc aagattcttt agtgactgat 540

tttgagatta ttttccctag cttacttcgt gaggcccaaa gtttacgttt aggtcttcca 600

tacgatttac cttacatcca cttattacaa acaaaacgtc aggaacgttt agcaaaatta 660

agccgtgaag aaatatatgc agttccaagt ccacttttat attctttaga gggtattcaa 720

gatattgttg agtgggaacg tattatggaa gtacaatctc aggatggatc atttttaagt 780

tctccagcat caaccgcatg tgtttttatg catacaggtg acgctaagtg tttagaattt 840

cttaacagtg taatgattaa gtttggtaat tttgtaccat gcctttatcc tgtagattta 900

ttagaacgtt tacttatagt agataatata gttcgtcttg gtatttaccg tcacttcgaa 960

aaagaaatta aagaagcatt agattatgta tatcgccatt ggaatgaacg tggtattggt 1020

tggggtcgtt taaatccaat tgctgactta gaaacaactg ctttaggttt tcgtttatta 1080

cgtttacacc gttataatgt atctccagca atctttgata atttcaaaga tgccaatggc 1140

aaattcattt gtagcactgg tcagtttaat aaggatgtgg cttcaatgtt aaacttatac 1200

cgtgcatcac aattagcatt cccaggcgaa aacattttag atgaagctaa atcttttgcc 1260

accaaatact tacgtgaagc ccttgaaaaa tctgaaactt catcagcttg gaacaataaa 1320

cagaatttaa gtcaagaaat caagtatgca ttaaaaactt catggcacgc ttctgtacca 1380

cgtgttgaag caaaacgtta ttgtcaagtt tatcgtcctg attacgctcg tattgctaag 1440

tgtgtataca aattaccata cgttaacaac gaaaaattct tagaattagg taaattagat 1500

tttaacatca ttcaatcaat tcatcaagaa gaaatgaaaa atgtgacaag ttggtttcgt 1560

gattctggct taccattatt tactttcgct cgcgaacgtc ctttagaatt ttacttctta 1620

gttgctgctg gtacttatga acctcaatat gctaaatgtc gtttcttatt cacaaaagta 1680

gcttgtcttc aaacagtatt agacgatatg tacgatactt acggtacttt agacgaatta 1740

aaacttttta ccgaggctgt gcgtcgttgg gatttatctt ttacagaaaa tttacctgac 1800

tatatgaaat tatgttatca aatctattat gacatcgttc atgaagtggc ttgggaagct 1860

gaaaaagaac aaggtagaga attagtgtca ttcttccgta aaggctggga agactactta 1920

ttaggttact atgaagaagc agaatggtta gcagcagaat acgttccaac attagatgaa 1980

tacattaaaa acggtattac atcaatcggc caacgtatct tattactttc aggtgtgtta 2040

attatggatg gccaactttt atcacaagaa gcattagaaa aagttgatta ccctggtcgt 2100

cgtgttttaa ctgagttaaa ctcacttatt agccgtttag ctgacgacac taaaacttat 2160

aaagcagaaa aagctcgtgg agaattagcc tcatcaattg aatgctacat gaaagatcat 2220

cctgaatgta cagaagaaga agccttagac cacatttatt ctattcttga accagccgta 2280

aaagaattaa ctcgtgaatt tcttaaacca gacgacgttc catttgcttg taaaaagatg 2340

ttattcgaag aaactcgtgt tacaatggtg atctttaaag atggtgatgg ttttggtgta 2400

tctaagttag aagttaaaga tcacatcaaa gaatgcttaa ttgaaccatt accattaggt 2460

accggtgaaa acttatactt tcaaggctca ggtggcggtg gaagtgatta caaagatgat 2520

gatgataaag gaaccggtta a 2541

<210> 85
<211> 933
<212> DNA
<213> Artificial Sequence

<220>
<223> Description of Artificial Sequence: Synthetic
      polynucleotide

<400> 85
atggtaccaa ctatgatgaa tatgaatttt aagtactgtc acaagattat gaagaaacat      60

tcaaaatcat tcagttatgc ttttgactta ttaccagaag accaacgtaa agctgtttgg     120

gcaatttacg ccgtgtgccg caaaattgat gattctattg atgtatatgg tgatattcaa     180

ttcttaaatc agattaaaga agacatacaa agtattgaaa aatatccata cgaacatcat     240

cattttcaat ctgacagacg tattatgatg gccttacagc atgttgctca gcataaaaac     300

attgcatttc aatcattcta caatttaatt gacacagtat ataaagatca acactttaca     360

atgtttgaaa cagatgctga actttttggt tattgttacg gtgtagctgg tactgtgggt     420

gaagttttaa ctcctatatt atctgatcac gaaacacatc aaacttatga cgttgcccgt     480

cgtttaggag agtcattaca gttaatcaat attcttagag atgtaggtga agactttgac     540

aacgaacgta tttacttctc taaacaacgt ttaaaacaat acgaagtaga tattgcagaa     600

gtgtaccaaa atggtgtaaa caatcactat attgatttat gggaatatta cgctgcaatt     660

gctgaaaagg attttcaaga tgttatggac caaattaaag ttttctctat tgaagctcag     720

ccaattattg agttagctgc acgtatttat atcgaaattt tagatgaagt acgtcaagct     780

aactacacat tacatgaacg tgtttttgta gataaacgta aaaaggctaa actttttcac     840

gaaaataaag gtaccggtga aaacttatac tttcaaggct caggtggcgg tggaagtgat     900

tacaaagatg atgatgataa aggaaccggt taa                                  933


<210> 86
<211> 1596
<212> DNA
<213> Artificial Sequence

<220>
<223> Description of Artificial Sequence: Synthetic
      polynucleotide

<400> 86
atggtaccaa aaattgctgt tattggtgct ggtgttaccg gattagctgc tgctgctcgt      60

attgctagcc aaggtcatga agttacaatc ttcgaaaaaa acaataatgt aggtggtcgt     120

atgaatcaat taaaaaaaga tggttttaca ttcgatatgg gacctacaat tgttatgatg     180

ccagatgtat ataaagatgt atttactgct tgcggtaaaa actatgaaga ttatatagag     240

ttacgtcaac ttcgttacat ttatgacgta tatttcgatc acgatgatcg tattactgtt     300

ccaactgatt tagctgaatt acaacaaatg ttagaatcaa ttgaacctgg tagtacacac     360

ggatttatgt catttttaac agatgtgtac aagaaatatg aaatcgctcg cagatatttc     420

ttagaacgta cttaccgtaa accatcagac ttctacaata tgacctcttt agtacaaggt     480

gctaaactta aaactttaaa tcacgctgat caacttatcg aacactacat tgataacgaa     540

aagattcaaa aacttttagc attccaaact ctttatatcg gcattgatcc aaagcgtggt     600

cctagtttat atagtattat tcctatgatt gaaatgatgt tcggtgtaca ttttatcaaa 660

ggtggtatgt atggtatggc tcaaggatta gctcaactta acaaagattt aggtgttaat 720

attgaattaa atgctgaaat tgaacaaatc attatcgatc ctaaattcaa acgcgcagat 780

gcaattaaag ttaatggtga cattcgcaaa tttgataaga ttttatgtac tgctgacttt 840

ccttcagttg ccgaatcact tatgccagat ttcgcaccta tcaaaaagta ccctccacat 900

aaaattgcag atttagatta ttcttgttca gcttttctta tgtatattgg tattgacatc 960

gacgtaactg accaagttcg tttacataac gtaattttta gcgacgattt tcgtggaaat 1020

attgaagaaa ttttcgaagg tcgcttaagt tacgacccat caatctatgt ttatgtacca 1080

gctgtagccg ataaatcttt agctcctgaa ggtaaaacag gcatttatgt gttaatgcct 1140

actcctgaac ttaaaacagg atcaggtatt gactggtcag atgaggcttt aactcaacaa 1200

attaaagaaa tcatttatcg taaattagca acaattgaag tatttgaaga cattaaatca 1260

cacattgtat cagaaacaat ttttactcct aatgactttg aacaaaccta tcacgctaaa 1320

tttggttctg ctttcggttt aatgcccacc ttagcacaat ctaattatta cagacctcaa 1380

aatgtgtcac gtgattataa agacttatat ttcgcaggtg catcaacaca tccaggtgct 1440

ggagttccaa ttgtattaac aagtgccaag ataacagtag acgaaatgat taaagatatt 1500

gagcgtggtg tgggtaccgg tgaaaactta tactttcaag gctcaggtgg cggtggaagt 1560

gattacaaag atgatgatga taaaggaacc ggttaa 1596

<210> 87
<211> 978
<212> DNA
<213> Artificial Sequence

<220>
<223> Description of Artificial Sequence: Synthetic
      polynucleotide

<400> 87
atggtaccag catttgactt cgatggttac atgcttcgta aagctaaatc tgtaaataaa 60

gctcttgaag ctgcagtaca aatgaaagaa ccattaaaaa ttcatgaaag tatgcgttat 120

tctttattag ctggtggtaa acgtgtacgt ccaatgttat gtattgcagc ttgtgaatta 180

gttggtggtg acgaaagtac tgctatgcct gctgcttgcg ctgtagaaat gattcatact 240

atgagtttaa tgcatgatga tttaccatgt atggataatg acgatttacg tcgtggtaaa 300

ccaacaaacc acatggcatt tggtgaaagt gtagcagtat tagcaggtga tgcattatta 360

tcttttgctt ttgaacatgt agcagcagca acaaaaggtg ctcctccaga acgtattgtt 420

agagttttag gtgaacttgc agtttctatt ggttcagaag gtttagttgc tggacaagta 480

gttgacgttt gttctgaagg tatggctgag gttggtttag atcatttaga atttattcat 540

caccacaaaa ctgctgcttt attacaaggt tctgtagtat taggtgcaat attaggtggt 600

ggaaaagaag aagaggtagc aaaacttcgt aaattcgcta actgcattgg tttacttttc 660

caagtagtag atgatattct tgatgtaaca aaatcatcta aagaattagg taaaacagca 720

ggtaaagatt tagttgctga taaaactact tatccaaaat taatcggtgt tgagaaaagt 780

aaagagttcg cagaccgttt aaatcgtgaa gctcaagaac aacttcttca ttttcatcca 840

catagagcag cacctttaat cgctttagca aactatattg cttatcgtga taatggtacc 900

ggtgaaaatt tatattttca aggttcaggt ggcggaggtt ctgattataa agatgatgat 960

gataaaggaa ccggttaa 978

<210> 88
<211> 891
<212> DNA
<213> Artificial Sequence

<220>
<223> Description of Artificial Sequence: Synthetic
      polynucleotide

<400> 88
atggtaccaa gtcaacctta ctgggcagca attgaggcag atattgaacg ttacttaaaa 60

aaatcaatta caattcgtcc accagaaact gtatttggtc caatgcacca cttaactttt 120

gctgcaccag ctacagctgc tagtacttta tgtttagcag catgtgaact tgtaggtggt 180

gatcgtagtc aagctatggc tgcagcagca gcaatccatc ttgttcatgc agctgcttat 240

gtacatgaac atttaccatt aactgatggt agtcgtccag taagtaaacc agctatccaa 300

cataaatatg gtccaaatgt agaattactt acaggtgacg gtattgtacc atttggtttt 360

gaattattag caggttctgt tgatccagca cgtacagatg atccagaccg tattttacgt 420

gtaataattg aaataagtcg tgctggtggt ccagaaggta tgattagtgg tttacatcgt 480

gaagaagaga ttgtagatgg taatacttct cttgatttta ttgaatacgt ttgcaaaaaa 540

aaatatggtg aaatgcacgc atgtggtgct gcatgcggtg caattttagg tggtgcagct 600

gaagaagaaa ttcaaaaact tcgtaacttc ggattatatc aaggaacttt acgtggtatg 660

atggagatga aaaactcaca ccaacttatt gacgaaaata tcattggcaa acttaaagaa 720

ttagctttag aagaattagg tggatttcat ggtaaaaatg ctgaattaat gtctagttta 780

gtagcagaac catcattata tgctgctggt accggtgaaa atttatactt tcaaggttct 840

ggtggtggtg gcagtgatta taaagacgat gatgacaaag gaaccggtta a 891

<210> 89
<211> 1080
<212> DNA
<213> Artificial Sequence

<220>
<223> Description of Artificial Sequence: Synthetic
      polynucleotide

<400> 89
atggtaccac ttttatctaa caaattaaga gagatggttt tagcagaagt tcctaaatta      60

gcatctgctg ctgaatattt ctttaaacgt ggtgttcagg gtaaacaatt ccgttcaaca     120

attttattat taatggcaac agctcttgac gttcgtgttc cagaagcatt aattggtgaa     180

tctactgata ttgtaacatc tgaattacgt gtacgtcaac gtggcattgc tgaaattaca     240

gaaatgattc atgtagcatc acttcttcac gatgacgttc ttgacgatgc tgatactcgt     300

cgtggtgttg gtagtcttaa tgttgtaatg ggaaacaaaa tgtcagtttt agcaggtgac     360

ttcttacttt ctcgtgcttg tggtgctctt gcagctctta aaaacacaga agttgtagca     420

ttattagcta cagcagtaga acacttagtt actggtgaga caatggaaat aacttcatca     480

actgaacaac gttattctat ggattactac atgcagaaaa cttattacaa aactgcttca     540

ttaatttcaa attcatgtaa agcagttgct gtattaacag gtcaaacagc tgaagttgca     600

gtattagctt ttgaatatgg tcgtaattta ggtttagctt tccagttaat tgacgacatt     660

ttagatttca caggcacatc tgctagttta ggaaaaggtt ctttatcaga tatacgtcat     720

ggtgttatta ctgctcctat cttatttgca atggaagaat ttcctcaatt aagagaagta     780

gtagatcaag tagaaaaaga tccaagaaat gtagacatag ctttagaata tttaggtaaa     840

agtaaaggta ttcaacgtgc tcgtgaatta gcaatggaac acgcaaattt agctgctgca     900

gctattggtt ctttacctga aacagataac gaagatgtta aacgttcacg tcgtgcttta     960

attgatttaa cacacagagt aattacacgt aacaaaggta ccggtgagaa tttatacttt    1020

caaggtagtg gtggaggagg tagtgactat aaagatgatg acgataaagg aaccggttaa    1080


<210> 90
<211> 1092
<212> DNA
<213> Artificial Sequence

<220>
<223> Description of Artificial Sequence: Synthetic
      polynucleotide

<400> 90
atggtaccag tagtttctga acgtttaaga cattctgtaa caactggtat tccagcatta      60

aaaacagcag ctgaatattt ctttcgtcgt ggtatcgaag gaaaacgttt aagacctaca     120

ttagcattat taatgagtag tgctttatca ccagctgctc catcaccaga gtatttacaa     180

gttgatacaa gacctgctgc agaacaccct catgaaatgc gtcgtcgtca acaacgttta     240

gctgaaattg cagaattaat ccatgtagct tcattacttc acgatgatgt tattgatgac     300

gcacaaacac gtcgtggtgt tttaagttta aatacatctg ttggtaataa aacagctatc 360

ttagcaggtg atttcttatt agctcgtgca tctgtaacat tagctagttt aagaaactct 420

gaaattgtag aattaatgtc acaggtttta gaacacttag tatctggtga aattatgcaa 480

atgactgcta cttcagaaca acttttagat ttagaacatt atttagcaaa aacatattgt 540

aaaactgctt cattaatggc taatagttct cgttctgttg cagttcttgc aggtgcagct 600

cctgaagttt gtgatatggc atggtcatac ggtcgtcatt taggtattgc tttccaagta 660

gttgacgatt tattagattt aacaggttca tcttctgttt taggtaaacc tgctttaaac 720

gatatgcgtt ctggtttagc aacagcacca gtattattcg ctgcacaaga agaacctgca 780

ttacaggctc ttatattacg tcgttttaaa cacgacggtg acgtaacaaa agcaatgtca 840

ttaattgaac gtacacaagg cttacgtcgt gctgaagaac ttgcagcaca acacgcaaaa 900

gctgctgctg atatgattcg ttgcttacct acagctcaat cagaccatgc agaaattgct 960

cgtgaagcat taattcaaat tacacatcgt gttttaacac gtaaaaaagg taccggtgaa 1020

aacttatact ttcaaggttc tggtggtggt ggatcagatt ataaagatga tgatgacaaa 1080

ggaaccggtt aa 1092

<210> 91
<211> 987
<212> DNA
<213> Artificial Sequence

<220>
<223> Description of Artificial Sequence: Synthetic
      polynucleotide

<400> 91
atggtaccag attttccaca acaattagaa gcatgtgtta aacaagcaaa tcaagcatta 60

tcacgtttca tcgcaccact tccattccaa aatactcctg ttgttgaaac aatgcaatat 120

ggtgcattat taggaggtaa aagattaaga ccatttcttg tatatgcaac aggtcacatg 180

tttggagtat ctactaacac attagatgct ccagctgctg cagttgaatg tattcatgca 240

tatagtttaa ttcatgatga tttacctgca atggatgatg atgacttaag aagaggttta 300

cctacatgtc atgttaaatt tggtgaagct aatgctattt tagctggcga tgcacttcaa 360

actcttgcat tcagtatttt atcagatgct gatatgccag aagtttcaga tcgtgatcgt 420

atttctatga tatctgaatt agcttctgct agtggtattg ctggtatgtg cggtggccaa 480

gctcttgatt tagacgcaga aggaaaacac gttcctttag atgctttaga gcgtatacat 540

cgtcacaaaa caggagcttt aattagagct gctgttcgtc ttggtgcttt atcagctgga 600

gacaaaggtc gtcgtgcttt accagtttta gacaaatacg ctgaaagtat tggtttagct 660

tttcaagttc aggatgatat cttagatgtt gtaggtgata ctgctacttt aggtaaacgt 720

caaggtgctg atcaacagtt aggcaaatct acatacccag cacttttagg tttagaacaa 780

gctcgtaaaa aagcaagaga cttaattgac gatgctcgtc aaagtcttaa acaattagca 840

gaacaatcac ttgatacaag tgctttagaa gcattagcag attacattat tcaacgtaat 900

aaaggtaccg gtgaaaattt atattttcaa ggttctggtg gtggaggttc agactataaa 960

gatgacgatg ataaaggaac cggttaa 987

<210> 92
<211> 1242
<212> DNA
<213> Artificial Sequence

<220>
<223> Description of Artificial Sequence: Synthetic
      polynucleotide

<400> 92
atggtaccaa gtgttagttg ttgttgtaga aatttaggaa aaactatcaa aaaagctatt 60

ccaagtcacc acttacattt acgttcttta ggtggtagtt tatatagaag acgtattcaa 120

tcatcttcaa tggaaacaga cttaaaatct acattcttaa atgtttattc agttcttaaa 180

tcagatttat tacacgaccc atcatttgaa tttacaaatg aaagtcgttt atgggtagat 240

agaatgcttg attataatgt tcgtggcggt aaacttaatc gtggtctttc tgtagtagac 300

tctttcaaat tacttaaaca aggtaatgat ttaactgaac aagaagtttt cttatcttgt 360

gcattaggtt ggtgtattga gtggttacag gcttactttt tagttcttga tgatattatg 420

gataattcag ttacacgtcg tggtcaacct tgttggtttc gtgtaccaca agttggtatg 480

gtagctatta atgatggcat tcttcttcgt aaccatattc atcgtattct taaaaaacac 540

ttccgtgata aaccatatta tgtagattta gttgaccttt tcaatgaagt agagttacaa 600

actgcatgtg gacaaatgat tgatttaatc acaacatttg aaggtgaaaa agacttagct 660

aaatatagtt tatcaattca ccgtcgtatt gttcaataca aaactgcata ttactcattc 720

tatttaccag ttgcatgtgc tcttttaatg gctggcgaaa atttagaaaa ccacattgat 780

gttaaaaatg tattagtaga tatgggtatt tactttcaag ttcaggatga ttatttagac 840

tgttttgctg atcctgaaac attaggtaaa attggcactg atattgagga ctttaaatgt 900

tcttggttag ttgtaaaagc attagaacgt tgtagtgaag aacaaacaaa aattctttac 960

gaaaactatg gcaaacctga tccatctaat gttgctaaag taaaagattt atacaaagaa 1020

ttagatttag aaggcgtttt catggaatat gaatctaaat catacgagaa attaactggt 1080

gctatcgaag gtcaccaatc taaagcaatt caagctgttc ttaaatcttt cttagcaaaa 1140

atctataaac gtcaaaaagg taccggtgaa aacttatact ttcaaggtag tggtggcggt 1200

ggtagtgatt ataaagatga tgatgataaa ggaaccggtt aa 1242

<210> 93
<211> 1116
<212> DNA
<213> Artificial Sequence

<220>
<223> Description of Artificial Sequence: Synthetic
      polynucleotide

<400> 93
atggtaccag ctgatcttaa atcaacattc ttagatgttt attcagtatt aaaaagtgat      60

ttattacaag atccatcttt tgaatttaca cacgaaagtc gtcaatggtt agaacgtatg     120

ttagattata atgttcgtgg aggcaaatta aacagaggtt taagtgtagt agacagttac     180

aaacttttaa aacaaggtca agacttaaca gaaaaagaaa catttttatc ttgtgcttta     240

ggttggtgta ttgaatggtt acaagcatac ttcttagttt tagacgatat tatggataat     300

tctgtaacta gacgtggtca accatgttgg tttcgtaaac caaaagtagg tatgattgct     360

attaatgatg gaatacttct tcgtaaccac attcatcgta ttcttaaaaa acactttcgt     420

gaaatgcctt attatgtaga ccttgtagac ttatttaacg aagtagaatt tcaaacagct     480

tgtggtcaaa tgattgactt aattacaaca tttgatggtg aaaaagacct ttcaaaatat     540

tcacttcaga ttcaccgtcg tattgttgag tacaaaacag catactactc tttctattta     600

cctgtagcat gtgctttact tatggcaggt gaaaatttag aaaatcacac agatgttaaa     660

actgtattag ttgatatggg tatctatttc caagttcaag atgattattt agattgcttc     720

gctgatccag aaacattagg taaaattggt acagatattg aagactttaa atgtagttgg     780

ttagtagtaa aagcattaga acgttgtagt gaagaacaaa caaaaattct ttacgaaaat     840

tatggaaaag ctgaaccttc aaatgtagct aaagttaaag cattatacaa agaattagat     900

ttagagggtg catttatgga atatgaaaaa gaatcatacg agaaacttac aaaacttatt     960

gaagcacatc aatcaaaagc tattcaagca gttcttaaat ctttcttagc taaaatttat    1020

aaacgtcaaa aaggtaccgg tgaaaactta tactttcaag gctctggagg tggtggttca    1080

gactataaag atgatgatga taaaggaacc ggttaa                              1116

<210> 94
<211> 1170
<212> DNA
<213> Artificial Sequence

<220>
<223> Description of Artificial Sequence: Synthetic
      polynucleotide

<400> 94
atggtaccaa gtggcgaacc tactccaaaa aaaatgaaag caacatacgt tcacgaccgt      60

gaaaacttta caaaagtata cgaaactctt cgtgacgaat tacttaacga tgattgtctt     120

agtccagctg gttcacctca ggctcaagct gctcaagagt ggtttaaaga agttaatgat 180

tataatgttc ctggtggaaa acttaaccgt ggtatggctg tatatgacgt tttagcttca 240

gttaaaggtc cagatggttt aagtgaagac gaagtattta aagctaacgc tcttggttgg 300

tgtattgagt ggttacaagc atttttctta gttgctgatg atataatgga tggttcaatt 360

acacgtcgtg gccaaccttg ttggtacaaa caacctaaag ttggtatgat tgcttgtaat 420

gattacatct tattagaatg ctgtatttac tcaattctta aaagacattt tagaggtcac 480

gctgcatacg ctcaacttat ggaccttttc catgaaacta cattccagac ttcacacggt 540

caattattag atttaacaac agcacctatc ggttctgtag acttatcaaa atatacagaa 600

gataattacc ttcgtattgt aacatataaa actgcatact attcttttta tttacctgta 660

gcatgtggta tggtattagc tggcattaca gatccagctg cttttgatct tgcaaaaaat 720

atttgtgttg aaatgggtca atatttccag attcaagacg attatttaga ttgctatggt 780

gaccctgagg ttattggtaa aatcggtaca gacatagaag acaacaaatg tagttggtta 840

gtttgcacag ctcttaaaat cgcaacagaa gaacaaaaag aggttataaa agctaattat 900

ggtcacaaag aggctgaatc agtagcagca attaaagcat tatacgttga attaggtatt 960

gaacaacgtt ttaaagacta tgaagctgca tcatacgcaa aattagaagg tacaattagt 1020

gaacaaactt tattacctaa agcagtattt acttctttat tagctaaaat ctataaaaga 1080

aaaaaaggta ccggtgagaa cttatacttt caaggtagtg gaggtggtgg ttcagactat 1140

aaagatgatg atgataaagg aaccggttaa 1170

<210> 95
<211> 636
<212> DNA
<213> Artificial Sequence

<220>
<223> Description of Artificial Sequence: Synthetic
      polynucleotide

<400> 95
atggtaccac aaactgaaca tgttatctta ttaaacgctc aaggtgttcc tacaggtaca 60

ttagaaaaat atgctgcaca cactgctgat actcgtttac acttagcttt ctcatcttgg 120

ttattcaatg ctaaaggtca acttttagtt acaagacgtg cattaagtaa aaaagcatgg 180

cctggtgttt ggactaactc agtttgtggt catccacaat taggtgaaag taatgaagat 240

gcagttatac gtcgttgcag atatgaatta ggtgttgaaa taactccacc agaatcaatt 300

tatccagatt tccgttatcg tgcaactgat cctagtggta tcgttgaaaa cgaagtatgt 360

cctgtttttg ctgcacgtac aacaagtgca ttacaaatta atgatgatga agtaatggat 420

tatcaatggt gtgacttagc tgatgtttta catggtattg atgcaacacc atgggcattt 480

tcaccatgga tggtaatgca agcaacaaat cgtgaagcac gtaaaagatt aagtgctttt 540

acacagttaa aaggtaccgg tgaaaactta tactttcaag gtagtggagg tggtggttct 600

gactataaag atgacgatga taaaggaacc ggttaa 636

<210> 96
<211> 969
<212> DNA
<213> Artificial Sequence

<220>
<223> Description of Artificial Sequence: Synthetic
      polynucleotide

<400> 96
atggtaccac ttcgtagttt attaagaggt ttaacacaca ttcctcgtgt taatagtgct 60

cagcaacctt cttgcgctca cgctcgtctt caatttaaac ttcgttctat gcaattatta 120

gcagaaaacc gtacagatca catgcgtggt gcttctacat gggcaggtgg tcagtctcaa 180

gatgaattaa tgcttaaaga tgaatgtatc ttagtagatg ctgatgataa cattactggt 240

cacgcttcta aattagaatg tcacaaattt cttccacatc aaccagctgg attattacac 300

cgtgcttttt ctgtatttct tttcgacgat caaggtcgtt tacttttaca acaacgtgct 360

cgtagtaaaa ttacatttcc atctgtatgg gctaatacat gttgtagtca tccattacat 420

ggtcaaacac cagatgaagt agatcaacaa tcacaagtag cagacggaac tgtaccaggt 480

gcaaaagctg ctgcaatcag aaaattagaa catgaattag gtattccagc tcaccaatta 540

ccagcatcag cttttcgttt cttaacacgt cttcactatt gtgcagctga cgttcaacct 600

gcagcaacac aatctgcatt atggggtgaa cacgaaatgg attacatttt attcattaga 660

gctaatgtta cacttgctcc taatcctgac gaagtagatg aggtacgtta tgtaactcaa 720

gaagaattaa gacaaatgat gcaaccagat aatggtttac aatggtcacc atggttccgt 780

attattgcag caagattttt agaacgttgg tgggctgatt tagatgctgc attaaataca 840

gataaacatg aagactgggg aacagttcat cacattaacg aagctggtac cggtgaaaac 900

ttatactttc aaggatcagg aggcggtgga agtgattata aagatgatga tgataaagga 960

accggttaa 969

<210> 97
<211> 1725
<212> DNA
<213> Artificial Sequence

<220>
<223> Description of Artificial Sequence: Synthetic
      polynucleotide

<400> 97

atggtaccaa gaagatcagg caattataac ccaacagcat gggacttcaa ttatatccaa 60

tcattagaca atcaatacaa aaaagaacgt tactctactc gtcacgctga attaacagtt 120

caagttaaaa aattattaga agaagaaatg gaagctgttc aaaaacttga acttatagag 180

gatcttaaaa acttaggcat ttcttaccca tttaaagata atattcaaca aatcttaaat 240

caaatttaca atgaacacaa atgttgtcac aactcagaag ttgaagaaaa agacctttat 300

ttcactgctt tacgttttag attattacgt caacaaggtt ttgaagtaag tcaagaagta 360

tttgatcact ttaaaaacga aaaaggtaca gattttaaac ctaatttagc agatgatact 420

aaaggattat tacaattata tgaagcatca ttcttattac gtgaagcaga agacacatta 480

gaacttgctc gtcaattctc tactaaactt ttacaaaaaa aagttgatga aaacggtgac 540

gataaaattg aagataactt attactttgg attagacgta gtttagaatt accattacat 600

tggcgtgtac aaagattaga agctcgtggc tttttagatg cttacgttcg tagacctgat 660

atgaatccta ttgtatttga attagcaaaa ttagacttta acattactca agcaacacaa 720

caagaagaac ttaaagattt atcaagatgg tggaatagta ctggcttagc tgaaaaactt 780

ccttttgctc gtgatcgtgt agttgaatca tatttctggg ctatgggtac ttttgaacca 840

catcaatacg gataccaacg tgaattagtt gctaaaatca ttgcacttgc tacagttgta 900

gacgatgttt acgatgtata tggtacttta gaggaattag aactttttac tgatgctatt 960

cgtcgttggg accgtgaatc tattgaccaa ttaccatatt acatgcaatt atgttttctt 1020

actgtaaaca actttgtttt tgagttagct cacgacgtat taaaagataa atcattcaat 1080

tgtttacctc atttacaaag atcatggtta gatttagctg aagcatacct tgtagaagca 1140

aaatggtatc atagtcgtta tacaccttct ttagaagaat atcttaatat tgctcgtgtt 1200

tcagtaacat gtccaactat tgtttctcaa atgtattttg cattaccaat tccaatcgaa 1260

aaacctgtaa ttgagatcat gtacaaatat cacgatatct tatacttatc aggtatgtta 1320

ttacgtttac cagatgactt aggaacagca tcattcgaac ttaaacgtgg tgatgtacaa 1380

aaagcagttc aatgttatat gaaagaacgt aatgttcctg aaaatgaagc tcgtgaacat 1440

gttaaattct taattcgtga ggcttctaaa caaattaata cagcaatggc aacagactgt 1500

ccatttacag aagattttgc agttgcagca gcaaacttag gtcgtgtagc aaattttgta 1560

tatgttgatg gtgatggttt tggagtacaa cacagtaaaa tctatgagca aattggtaca 1620

cttatgtttg aaccatatcc aggtaccggt gaaaacttat actttcaagg tagtggtggt 1680

ggaggttctg attacaaaga cgatgatgat aaaggaaccg gttaa 1725

<210> 98
<211> 1722
<212> DNA
<213> Artificial Sequence

<220>
<223> Description of Artificial Sequence: Synthetic
      polynucleotide

<400> 98
atggtaccaa gaagaagtgg aaactataaa cctacaatgt gggattttca atttattcaa      60

agtgtaaata atctttacgc tggtgataaa tacatggaac gtttcgatga agtaaaaaaa     120

gaaatgaaaa aaaacttaat gatgatggtt gagggtttaa tagaggaatt agatgttaaa     180

ttagaattaa tagataattt agaaagatta ggtgttagtt atcatttcaa aaatgaaata     240

atgcaaatcc ttaaatctgt acaccagcaa atcacttgtc gtgataattc attatactct     300

actgcattaa aatttcgttt attacgtcaa cacggattcc acattagtca agacatcttt     360

aacgatttta aagatatgaa tggcaatgtt aaacaaagta tctgtaacga tactaaaggt     420

ttattagaac tttatgaagc atctttctta tctactgaat gtgaaacaac acttaaaaac     480

ttcactgaag cacacttaaa aaattatgtt tatattaacc actcatgtgg agatcaatac     540

aataacataa tgatggaatt agttgttcac gctttagaat taccacgtca ctggatgatg     600

cctcgtttag agacacgttg gtatatatca atttatgaac gtatgcctaa tgctaatcca     660

cttttacttg aacttgctaa attagacttc aatattgttc aagctacaca ccaacaagac     720

ttaaaatcat tatcacgttg gtggaaaaac atgtgtttag ctgaaaaatt atcattttct     780

cgtaaccgtt tagtagaaaa tcttttctgg gcagttggaa ctaatttgga accacaacac     840

agttatttcc gtcgtttaat cactaaaatc attgtttttg ttggtattat tgatgatatt     900

tatgatgttt acggcaaact tgatgagtta gaattattca ctttagctgt acaacgttgg     960

gatacaaaag caatggaaga cttaccatat tacatgcaag tttgttattt agctttaatt    1020

aatacaacaa atgatgttgc ttatgaagtt cttcgtaaac ataacattaa tgtattacca    1080

tacttaacta aatcttggac agacttatgt aaatcatatt tacaagaagc tcgttggtac    1140

tacaatggtt acaaaccttc attagaggaa tatatggata atggttggat tagtatagca    1200

gttcctatgg tattagcaca tgcacttttc ttagttacag atccaattac aaaagaagca    1260

ttagaatcat taacaaacta tcctgatatt attcgttgct cagctacaat attccgttta    1320

aatgatgatc ttggtacaag ttcagatgaa ttaaaacgtg gagatgtacc aaaatcaatt    1380

caatgctata tgaacgaaaa aggcgtttca gaggaagaag ctcgtgaaca tattcgtttc    1440

ttaatcaaag aaacatggaa attcatgaac actgcacacc ataaagagaa aagtttattt    1500

tgtgagacat ttgtagaaat tgcaaaaaat attgcaacaa cagctcattg tatgtactta    1560

aaaggtgatt ctcacggtat tcaaaacact gatgttaaaa actcaataag taatatactt    1620

ttccatccaa ttattatcgg taccggtgaa aacctttact ttcaaggttc aggtggtggc    1680

ggttcagact ataaagatga cgatgataaa ggaaccggtt aa                        1722

<210> 99
<211> 1737
<212> DNA
<213> Artificial Sequence

<220>
<223> Description of Artificial Sequence: Synthetic
      polynucleotide

<400> 99
atggtaccaa gacgtagtgg aaattatgag ccatctgcat gggacttcaa ttacttacaa      60

tctcttaata attatcacca taaagaagaa cgttacttac gtcgtcaagc tgatttaatt     120

gaaaaagtaa aaatgattct taaagaagag aaaatggaag cattacagca attagaactt     180

atagacgatc ttcgtaattt aggtctttca tattgttttg atgatcaaat taatcatatt     240

cttacaacaa tttacaacca acattcttgt ttccattatc acgaagctgc aacaagtgaa     300

gaagctaact tatatttcac agctttaggt ttccgtttac ttcgtgaaca cggattcaaa     360

gtatcacaag aagtatttga ccgtttcaaa aatgaaaaag gtacagattt tcgtccagat     420

ttagtagatg atactcaagg tttattacaa ctttatgaag catctttcct tcttcgtgaa     480

ggtgaagaca ctttagaatt tgcacgtcaa tttgctacta aatttcttca aaaaaaagtt     540

gaggagaaaa tgatagaaga ggaaaatctt ttatcttgga ctttacattc acttgaatta     600

ccattacatt ggcgtataca acgtttagaa gctaaatggt ttttagatgc ttatgctagt     660

cgtcctgata tgaatccaat aatctttgaa ttagcaaaat tagaatttaa cattgctcag     720

gcacttcaac aagaagaact taaagattta tcaagatggt ggaacgatac tggtattgct     780

gaaaaattac ctttcgctcg tgatagaatc gttgaatctc attattgggc aattggtact     840

ttagaacctt atcaataccg ttatcagcgt tcattaattg caaaaatcat tgctttaact     900

acagttgttg atgatgtata tgatgtttac ggtacattag acgaattaca gttatttact     960

gatgcaattc gtcgttggga cattgaaagt ataaatcaat taccttctta tatgcaatta    1020

tgttatttag ctatttataa tttcgtatca gaattagctt atgatatttt cagagataaa    1080

ggttttaatt ctttaccata tttacacaaa agttggcttg acttagttga ggcttacttt    1140

caagaagcaa aatggtatca ttctggctac acaccatcat tagaacaata cttaaatatc    1200

gctcaaattt ctgtagcaag tccagctata ttaagtcaaa tttactttac tatggctggt    1260

tcaattgata aaccagtaat cgaatcaatg tacaaatata gacacatttt aaacttatct    1320

ggtatattac ttagattacc agatgactta ggtactgcta gtgatgaatt aggtcgtggt    1380

gatttagcaa aagcaatgca atgttacatg aaagagcgta acgtttctga agaagaagct    1440

cgtgatcatg tacgtttctt aaatcgtgag gtttcaaaac aaatgaatcc tgctcgtgct    1500

gctgatgatt gtccattcac tgatgatttt gtagtagctg ctgctaattt aggaagagtt    1560

gcagatttca tgtatgttga aggcgatggt ttaggtttac aatacccagc tatccaccaa 1620

cacatggcag aacttttatt tcacccttac gcaggtaccg gtgaaaactt atactttcaa 1680

ggttcaggtg gtggaggttc tgactataaa gatgatgatg ataaaggaac cggttaa 1737

<210> 100
<211> 1737
<212> DNA
<213> Artificial Sequence

<220>
<223> Description of Artificial Sequence: Synthetic
      polynucleotide

<400> 100
atggtaccaa gaagatcagg aaattatcaa cctagtgcat gggattttaa ctatatccaa 60

tctcttaata acaaccattc taaagaagaa cgtcacttag agcgtaaagc aaaacttatt 120

gaagaagtaa aaatgttatt agagcaagaa atggctgctg tacaacaatt agagcttatt 180

gaagacctta aaaacttagg tttatcttac ttattccaag atgaaatcaa aataatcctt 240

aattctattt acaatcatca taaatgtttt cataataatc acgaacaatg tattcacgtt 300

aatagtgact tatactttgt tgcattaggc ttccgtttat ttcgtcaaca tggtttcaaa 360

gtttctcaag aggtttttga ctgttttaaa aacgaagaag gatcagactt tagtgctaac 420

ttagcagatg atactaaagg tttacttcaa ttatacgagg cttcatattt agttacagaa 480

gatgaagaca cattagaaat ggcacgtcaa ttttcaacta aaatcttaca aaaaaaagta 540

gaagagaaaa tgattgagaa agagaactta ttaagttgga ctttacatag tttagaatta 600

ccacttcact ggcgtattca acgtttagaa gcaaaatggt tccttgatgc ttatgctagt 660

cgtccagata tgaatccaat tatttttgaa ttagctaaat tagagtttaa cattgctcaa 720

gcattacaac aagaagaatt aaaagattta agtagatggt ggaatgatac aggcattgct 780

gaaaaattac cttttgctcg tgatagaata gtagagagtc attactgggc aattggtact 840

ttagaacctt atcaatatag atatcaacgt tcattaattg ctaaaattat tgctttaaca 900

acagttgttg atgacgttta cgacgtatat ggaactttag atgaattaca gttatttaca 960

gacgctattc gtcgttggga tattgaatct attaatcaat taccaagtta tatgcaatta 1020

tgctatttag ctatttataa ctttgtttct gaattagcat acgatatttt tcgtgacaaa 1080

ggattcaatt ctttacctta ccttcataaa tcatggttag atttagtaga agcatacttt 1140

gttgaagcta aatggtttca tgatggttat actccaactc ttgaagaata tttaaataac 1200

tcaaaaatta ctattatatg tcctgctatt gttagtgaaa tctacttcgc attcgctaat 1260

tcaattgata aaacagaagt tgaatcaatc tacaaatatc acgatatttt atatttatca 1320

ggaatgcttg cacgtttacc agacgactta ggtacttcat catttgaaat gaaaagaggt 1380

gatgttgcta aagctattca atgttacatg aaagaacata atgcttcaga ggaagaagct 1440

cgtgaacaca ttcgtttctt aatgcgtgaa gcatggaaac acatgaatac tgctgcagct 1500

gctgatgact gtccatttga atctgattta gtagtaggtg ctgcatcatt aggtagagtt 1560

gcaaactttg tatatgttga aggtgacggt tttggtgtac aacattcaaa aatacatcaa 1620

caaatggctg aattactttt ttatccatat caaggtaccg gtgaaaactt atactttcaa 1680

ggtagtggag gtggtggtag tgactataaa gacgatgacg ataaaggaac cggttaa 1737

<210> 101
<211> 1707
<212> DNA
<213> Artificial Sequence

<220>
<223> Description of Artificial Sequence: Synthetic
      polynucleotide

<400> 101
atggtaccaa gaagaagtgc taattatcaa gcaagtattt gggatgataa tttcattcaa 60

agtcttgcat ctccttatgc aggagaaaaa tatgcagaaa aagcagaaaa acttaaaaca 120

gaagttaaaa ctatgattga tcaaacaaga gatgaactta aacaattaga acttattgat 180

aacttacaac gtttaggtat atgtcatcac tttcaagacc ttacaaaaaa aattttacaa 240

aaaatttatg gagaagaacg taacggagat caccaacatt acaaagaaaa aggcttacat 300

tttacagcat tacgtttccg tattttacgt caggacggtt atcatgttcc acaagatgta 360

ttttcatcat ttatgaataa agctggtgac tttgaagaat ctttaagtaa agacacaaaa 420

ggtttagtta gtttatatga ggcttcttac ttatcaatgg aaggtgaaac tattttagat 480

atggcaaaag acttttcatc tcaccattta cataaaatgg ttgaagatgc tactgacaaa 540

cgtgtagcta atcaaattat ccattctctt gaaatgccac ttcacagacg tgttcaaaaa 600

cttgaagcaa tttggtttat tcaattctac gaatgcggct ctgatgctaa tccaacttta 660

gtagaattag caaaattaga tttcaacatg gttcaggcaa cataccaaga agaattaaaa 720

cgtttatcac gttggtatga agaaacaggc ttacaagaga aactttcatt cgctcgtcac 780

cgtcttgctg aagcattctt atggtctatg ggtattattc cagaaggaca ctttggttat 840

ggtcgtatgc acttaatgaa aattggtgct tacattacat tacttgatga tatttatgat 900

gtttatggta ctttagaaga acttcaagta ttaacagaaa ttattgaacg ttgggatatt 960

aacttattag atcaattacc tgaatacatg caaatcttct ttttatacat gtttaattct 1020

acaaatgaac ttgcttatga aattttacgt gatcaaggta tcaatgtaat atcaaactta 1080

aaaggattat gggtagagtt atctcagtgt tactttaaag aagctacttg gttccataac 1140

ggttacacac caacaactga agaatatctt aatgttgctt gtatttctgc tagtggtcct 1200

gttattttat tttcaggtta ctttactact actaatccta ttaataaaca cgaattacaa 1260

tctttagaac gtcacgcaca ttcattatct atgatattac gtttagctga tgatttaggt 1320

acatcaagtg atgaaatgaa acgtggagat gtaccaaaag ctattcaatg ttttatgaat 1380

gacactggtt gttgtgaaga agaagcacgt caacacgtaa aaagattaat agatgctgaa 1440

tggaaaaaaa tgaacaaaga catcttaatg gagaaaccat ttaaaaattt ttgtccaact 1500

gctatgaatt taggtcgtat ttctatgagt ttttatgaac acggagatgg ttatggaggt 1560

cctcactctg atacaaaaaa aaaaatggta tctttatttg tacaaccaat gaatattact 1620

attggtaccg gtgaaaacct ttattttcaa ggttctggtg gtggcggttc agattataaa 1680

gatgatgacg acaaaggaac cggttaa 1707

<210> 102
<211> 1716
<212> DNA
<213> Artificial Sequence

<220>
<223> Description of Artificial Sequence: Synthetic
      polynucleotide

<400> 102
atggtaccaa gacgttcagc taactatcaa cctagtattt ggaaccacga ttacattgaa 60

tcacttcgta tcgaatatgt tggtgaaaca tgtacacgtc aaattaacgt tttaaaagaa 120

caagttcgta tgatgttaca caaagttgtt aatccattag aacaattaga attaattgaa 180

attttacaac gtttaggttt aagttaccat ttcgaagaag aaataaaacg tattttagat 240

ggtgtttaca ataacgatca tggtggtgat acatggaaag cagaaaacct ttatgcaaca 300

gctcttaaat tccgtctttt acgtcagcac ggttattctg tttctcaaga agttttcaac 360

tcttttaaag atgagcgtgg cagtttcaaa gcatgtttat gtgaagatac taaaggtatg 420

ttatcacttt atgaagcatc tttctttctt attgaaggtg aaaacatttt agaggaagct 480

agagacttta gtacaaaaca tcttgaagaa tatgtaaaac aaaataaaga gaaaaactta 540

gctactttag ttaatcactc attagaattt ccattacatt ggcgtatgcc tcgtttagaa 600

gctcgttggt tcatcaatat ctatcgtcat aatcaagatg taaatccaat ccttttagaa 660

tttgctgaac ttgacttcaa tattgtacaa gctgctcacc aagcagattt aaaacaagta 720

tcaacatggt ggaaatcaac tggtttagta gaaaatcttt cattcgctcg tgatcgtcct 780

gtagaaaact tcttttggac agttggtctt attttccaac cacaattcgg ttattgtcgt 840

agaatgttta ctaaagtatt cgcattaatt actacaattg atgacgtata tgatgtatat 900

ggtactttag atgaattaga acttttcaca gacgttgttg aaagatggga tattaatgca 960

atggatcaat tacctgatta tatgaaaatt tgctttttaa cattacacaa tagtgttaac 1020

gaaatggcat tagacactat gaaagaacaa cgttttcaca tcattaaata ccttaaaaaa 1080

gcatgggttg atctttgtcg ttattactta gttgaagcta aatggtatag taataaatat 1140

agaccttctt tacaagaata cattgaaaat gcatggattt caattggtgc tccaactatt 1200

ttagttcatg catatttctt cgttacaaat ccaattacaa aagaagcatt agactgttta 1260

gaagaatatc caaacattat tcgttggagt agtattattg cacgtttagc tgatgattta 1320

ggtacttcaa cagacgaatt aaaacgtggt gacgtaccaa aagcaattca atgttatatg 1380

aatgaaacag gtgcttcaga agaaggtgct cgtgagtaca ttaaatactt aatttctgct 1440

acttggaaaa aaatgaacaa agatagagca gcatcaagtc cattttcaca tatcttcatt 1500

gaaattgctc ttaatttagc acgtatggca caatgtttat atcaacacgg tgacggccac 1560

ggtttaggta accgtgaaac aaaagatcgt atactttcat tacttattca accaattcca 1620

ttaaacaaag atggtaccgg tgagaactta tactttcaag gctcaggtgg tggtggttct 1680

gattacaaag atgatgatga taaaggaacc ggttaa 1716

<210> 103
<211> 1800
<212> DNA
<213> Artificial Sequence

<220>
<223> Description of Artificial Sequence: Synthetic
      polynucleotide

<400> 103
atggtaccaa gaagaattgg agactatcac tcaaacttat ggaatgatga cttcattcaa 60

tcattaacaa caccatacgg tgctccatca tatattgaac gtgctgatag attaatatct 120

gaagtaaaag aaatgtttaa tagaatgtgt atggaagatg gtgagttaat gtctccatta 180

aatgatctta ttcaaagatt atggactgtt gatagtgttg aacgtttagg tatagatcgt 240

cacttcaaaa atgaaataaa agctagttta gattatgtat actcatactg gaacgaaaaa 300

ggtatcggtt gtggtcgtca atcagtagtt acagatttaa actctactgc tcttggatta 360

agaattttac gtcaacatgg ttacacagtt tcaagtgaag ttttaaaagt ttttgaagaa 420

gaaaacggtc aatttgcttg ttcaccttca cagactgagg gcgaaattcg ttcattctta 480

aacttatatc gtgcttcatt aattgctttt cctggtgaaa aagtaatgga agaagctcaa 540

atcttttcta gtcgttactt aaaagaagca gttcagaaaa ttccagtttc aggtttatct 600

cgtgaaatag gcgatgtttt agaatatggt tggcacacaa acttacctcg ttgggaagct 660

cgtaactata tggacgtatt cggtcaagac acaaatacat cattcaacaa aaacaaaatg 720

caatatatga atacagagaa aattcttcaa ttagtaaaat tagagtttaa tatctttcat 780

tcattacaac aacgtgaatt acaatgttta ttacgttggt ggaaagaaag tggtcttcca 840

caattaacat ttgcacgtca ccgtcacgtt gaattttaca ctttagcttc ttgtattgca 900

tgtgaaccaa aacacagtgc atttcgttta ggttttgcaa aaatgtgtca cttagtaaca 960

gttttagatg atgtatatga cacatttggc aaaatggatg aattagaact ttttactgca 1020

gctgttaaac gttgggactt atcagaaact gagcgtttac ctgagtatat gaaaggttta 1080

tatgttgtag ttttcgagac tgttaatgaa ttagcacaag aagcagagaa aactcaagga 1140

cgtaatacat taaattacgt tcgtaaagca tgggaagcat acttcgatag ttatatgaaa 1200

gaagcagaat ggatctcaac aggctattta ccaacattcg aagagtattg tgaaaacggt 1260

aaagtatcaa gtgcatatag agttgctgca cttcaaccta ttttaacatt agatgtacaa 1320

cttccagatg acatcttaaa aggtattgat tttccatctc gtttcaatga tttagcatct 1380

tcatttcttc gtttacgtgg agatactaga tgttacgagg ctgatcgtgc tcgtggtgaa 1440

gaagcaagtt gtatttcttg ttacatgaaa gacaatccag gttcaactga agaagatgca 1500

ttaaatcaca ttaatgctat gataaatgat attattcgtg aattaaactg ggaatttctt 1560

aaaccagact caaatatccc aatgccagct cgtaaacatg ctttcgatat tacaagagct 1620

ttacatcact tatatattta tcgtgacggt ttttctgttg ctaacaaaga gactaaaaat 1680

cttgttgaga aaactttatt agaatcaatg ttattcggta ccggtgagaa cctttatttt 1740

caaggttcag gtggtggtgg ttcagattat aaagacgatg atgataaagg aaccggttaa 1800

<210> 104
<211> 1731
<212> DNA
<213> Artificial Sequence

<220>
<223> Description of Artificial Sequence: Synthetic
      polynucleotide

<400> 104
atggtaccaa gaagatcagc taattatcaa cctagtcgtt gggatcatca tcacctttta 60

agtgtagaaa acaaattcgc taaagataaa cgtgtaagag aacgtgactt acttaaagaa 120

aaagttcgta aaatgttaaa tgacgaacag aaaacttact tagatcaatt agaatttatt 180

gacgatcttc aaaaattagg tgttagttat cacttcgaag cagaaataga taatatactt 240

acaagttcat acaaaaaaga tcgtacaaat atacaagaaa gtgatttaca cgcaactgca 300

ttagagtttc gtctttttcg tcaacacggt tttaacgttt cagaagatgt atttgatgta 360

tttatggaaa attgtggtaa attcgaccgt gatgacattt atggtttaat ttcattatat 420

gaagctagtt atctttctac taaacttgac aaaaatcttc aaatctttat ccgtccattt 480

gctactcaac aattacgtga ttttgtagat actcacagta atgaagattt cggttcatgt 540

gatatggtag aaatagttgt tcaagcatta gacatgccat actattggca aatgcgtcgt 600

ttatctacac gttggtatat tgatgtttat ggtaaaagac aaaattacaa aaacttagta 660

gttgttgaat ttgcaaaaat tgatttcaat attgttcaag ctattcacca ggaagaactt 720

aaaaatgtat catcttggtg gatggaaact ggtttaggta aacaacttta ttttgctcgt 780

gatcgtattg tagagaacta tttttggaca attggtcaaa ttcaagaacc tcaatatgga 840

tatgttagac aaacaatgac taaaatcaat gctttattaa caacaattga tgatatttat 900

gatatatacg gtacattaga agaattacag ttattcacag ttgcatttga gaattgggac 960

ataaatcgtt tagacgaatt accagaatat atgcgtttat gtttcttagt tatctataac 1020

gaagtaaata gtatagcatg tgaaattctt agaacaaaaa atattaacgt tattcctttc 1080

ttaaaaaaat cttggactga tgtaagtaaa gcatacttag ttgaagctaa atggtataaa 1140

tcaggccata aaccaaattt agaagagtat atgcaaaatg cacgtatttc tatttcttca 1200

ccaacaatct ttgttcactt ttattgtgta ttttcagacc aattatctat tcaagtttta 1260

gaaactttat cacaacacca acaaaatgtt gtaagatgta gttcttctgt tttccgttta 1320

gctaatgact tagtaacttc tccagatgaa ttagctagag gtgatgtttg taaatcaatt 1380

caatgttata tgtcagaaac tggtgcaagt gaagataaag ctagatcaca cgttcgtcaa 1440

atgattaatg atttatggga cgaaatgaat tacgagaaaa tggcacattc aagtagtatc 1500

ttacatcatg attttatgga gacagtaatc aatttagcta gaatgtctca atgtatgtac 1560

caatatggtg acggacacgg ttctccagaa aaagctaaaa ttgtagatcg tgtaatgagt 1620

ttacttttca accctattcc tttagatggt accggtgaga atttatattt tcaaggctct 1680

ggaggtggtg gttcagatta taaagatgat gacgacaaag gaaccggtta a 1731

<210> 105
<211> 1713
<212> DNA
<213> Artificial Sequence

<220>
<223> Description of Artificial Sequence: Synthetic
      polynucleotide

<400> 105
atggtaccaa gaagaagtgc aaactatcaa ccttcattat ggcaacatga atacttatta 60

tcattaggca acacttatgt taaagaagat aatgttgaaa gagtaactct tttaaaacaa 120

gaagtttcta aaatgttaaa cgaaacagaa ggtttacttg aacaacttga attaattgac 180

actttacaaa gattaggtgt ttcttatcat tttgaacagg agattaaaaa aacattaact 240

aatgttcatg ttaaaaacgt acgtgctcat aaaaatcgta ttgatcgtaa ccgttggggc 300

gatttatatg caactgcatt agaatttcgt ttattacgtc aacatggttt ttctattgct 360

caagacgttt ttgatggtaa tattggtgtt gacttagacg acaaagacat taaaggtatt 420

ttaagtttat acgaagctag ttacttatca acacgtattg atacaaaact taaagaatca 480

atctattaca caacaaaacg tttaagaaaa ttcgtagagg taaacaaaaa cgaaactaaa 540

agttacactc ttcgtcgtat ggttattcac gcacttgaga tgccttatca ccgtcgtgtt 600

ggtcgtcttg aagctcgttg gtatatcgag gtatatggag aaagacacga catgaatcct 660

attttattag aattagctaa attagatttt aactttgttc aggctatcca ccaagacgaa 720

ttaaaatcat tatctagttg gtggtctaaa acaggattaa caaaacattt agactttgtt 780

cgtgatcgta ttacagaggg ttacttcagt agtgtaggtg ttatgtatga accagaattt 840

gcatatcatc gtcaaatgct tacaaaagta tttatgctta ttacaactat tgatgacatc 900

tatgacattt acggtacact tgaagaatta caattattca caactatcgt tgaaaaatgg 960

gatgttaatc gtttagaaga acttcctaac tatatgaaat tatgcttctt atgtttagtt 1020

aacgaaataa atcaaattgg atattttgta ttaagagata aaggttttaa tgtaattcct 1080

tatcttaaag agtcttgggc tgacatgtgt actacatttc ttaaagaagc taaatggtac 1140

aaatcaggtt ataaaccaaa ttttgaagag tatatgcaaa atggctggat ttcatcatca 1200

gttccaacta ttcttttaca cttattttgt ttattaagtg accaaacttt agacattctt 1260

ggttcttata atcacagtgt tgttcgtagt tcagcaacaa ttttacgtct tgcaaatgat 1320

ttagctactt cttcagaaga attagcaaga ggagatacaa tgaaatcagt tcaatgtcac 1380

atgcatgaaa ctggtgcttc agaagctgaa tcaagagctt acattcaagg tattattggc 1440

gtagcttggg atgaccttaa tatggagaaa aaatcatgtc gtttacacca gggattctta 1500

gaagcagcag caaatttagg acgtgtagca caatgcgtat atcaatatgg agacggtcac 1560

ggttgtccag ataaagcaaa aacagtaaat catgttcgta gtttattagt tcacccatta 1620

ccattaaacg gtaccggtga aaacctttat tttcaaggta gtggtggagg tggttctgat 1680

tataaagacg acgatgacaa aggaaccggt taa 1713

<210> 106
<211> 1752
<212> DNA
<213> Artificial Sequence

<220>
<223> Description of Artificial Sequence: Synthetic
      polynucleotide

<400> 106
atggtaccag cttctccacc tgctcatcgt tcatctaaag cagcagacga agagttacca 60

aaagcatctt ctacattcca tccatctctt tggggttcat ttttcttaac atatcagcca 120

cctacagctc cacaacgtgc aaatatgaaa gaacgtgctg aagttcttcg tgaacgtgtt 180

cgtaaagtat taaaaggttc aacaacagat caattacctg aaacagttaa cttaattctt 240

acattacaaa gacttggttt aggttattac tatgaaaatg aaattgacaa attacttcat 300

caaatttact ctaattcaga ttataacgta aaagacttaa acttagtttc tcaacgtttt 360

tacttacttc gtaaaaacgg ttatgacgta ccttctgatg ttttcttatc ttttaaaact 420

gaagaaggtg gtttcgcttg tgctgcagct gacacacgtt cacttttaag tttatacaat 480

gctgcttacc ttcgtaaaca tggtgaagaa gtattagatg aagcaatttc atcaacacgt 540

ttaagattac aagacttatt aggtcgttta ttacctgaat caccattcgc taaagaagta 600

tcaagttcac ttcgtacacc tttattccgt cgtgtaggta ttttagaagc tcgtaactat 660

attccaatct atgaaactga agctacaaga aatgaagctg tattagagct tgctaaactt 720

aacttcaatt tacaacagct tgatttctgt gaagaattaa aacattgtag tgcatggtgg 780

aatgagatga ttgctaaaag taaattaact tttgtacgtg accgtatagt tgaagaatac 840

ttttggatga atggtgcatg ttatgatcca ccatattcat taagtcgtat tattcttaca 900

aaaatcactg gtttaattac tattattgat gatatgttcg atactcatgg tacaacagag 960

gattgcatga aattcgcaga agcatttggt cgttgggatg aatcagcaat tcatcttctt 1020

ccagaataca tgaaagattt ttacatttta atgttagaaa ctttccagtc atttgaagat 1080

gcacttggtc cagaaaaatc ataccgtgta ttatacttaa aacaagcaat ggaacgttta 1140

gtagagttat attctaaaga aatcaaatgg cgtgatgacg attatgttcc aacaatgtca 1200

gaacatttac aagttagtgc tgaaacaatt gctacaattg ctttaacttg ctctgcttat 1260

gctggtatgg gtgatatgtc tattcgtaaa gaaacatttg aatgggcatt atctttccct 1320

caattcatta gaacttttgg ttcatttgta cgtttatcaa atgatgttgt atcaacaaaa 1380

cgtgaacaaa ctaaagatca ttcaccttca acagttcact gttatatgaa agaacacggt 1440

acaactatgg acgatgcttg tgaaaaaatc aaagaattaa ttgaggactc atggaaagac 1500

atgttagaac aatctttagc tcttaaaggc ttacctaaag tagtacctca attagttttt 1560

gatttctctc gtactacaga taacatgtat cgtgaccgtg atgctttaac atcatcagaa 1620

gcattaaaag aaatgataca gttattattc gtagaaccta tacctgaagg taccggtgag 1680

aatctttatt ttcaaggatc aggtggtgga ggctcagatt acaaagatga cgacgataaa 1740

ggaaccggtt aa 1752

<210> 107
<211> 1761
<212> DNA
<213> Artificial Sequence

<220>
<223> Description of Artificial Sequence: Synthetic
      polynucleotide

<400> 107
atggtaccag aggctttagg aaattttgat tatgagagtt atactaattt tacaaaatta      60

ccatcatcac aatggggtga tcaattcctt aaattttcta tagcagattc tgacttcgat     120

gtattagaaa gagaaataga agtattaaaa ccaaaagtaa gagagaacat ttttgtttca     180

tcaagtactg ataaagatgc aatgaaaaaa acaattttaa gtattcattt cttagatagt     240

ttaggtttat cttatcactt cgaaaaagaa atagaggaga gtttaaaaca tgctttcgag     300

aaaattgaag accttattgc tgatgaaaat aaacttcata caataagtac aattttccgt     360

gtattccgta catacggcta ttatatgtct tctgatgtat tcaaaatttt caaaggagac     420

gatggtaaat tcaaagaaag tttaattgaa gacgttaaag gtatgctttc tttttatgaa     480

gctgttcatt ttggaacaac tactgatcac attttagacg aagctcttag ttttacatta     540

aaccacttag agtcacttgc aacaggccgt cgtgcatcac caccacatat tagtaaatta     600

atccaaaatg ctttacatat tcctcaacat cgtaacatcc aggcattagt agctcgtgaa     660

tacattagtt tttacgaaca cgaagaagat cacgatgaaa cattattaaa attagctaaa     720

ttaaacttta aattcttaca acttcactat tttcaagaat taaaaacaat tacaatgtgg     780

tggactaaat tagatcatac atctaattta ccaccaaatt ttcgtgaacg tacagttgaa     840

acatggtttg cagctttaat gatgtatttc gaaccacaat ttagtttagg tcgtattatg     900

agtgcaaaat tatatttagt aattactttc ttagatgacg catgtgatac atacggatca     960

atatctgaag tagagtcatt agctgattgt ttagaacgtt gggacccaga ttatatggaa    1020

aatttacaag gtcacatgaa aacagcattc aaattcgtta tgtatttatt caaagaatac    1080

gaagaaattt tacgttcaca aggccgttca ttcgtattag agaaaatgat tgaggagttt    1140

aaaattatcg cacgtaaaaa cttagaactt gtaaaatggg ctcgtggtgg tcacgttcct    1200

tcttttgacg aatatataga gagtggtggt gctgagattg gtacttatgc tacaatcgct    1260

tgttcaatta tgggtcttgg tgaaattggt aaaaaagaag catttgagtg gttaatctct    1320

cgtcctaaac ttgttcgtat tttaggtgct aaaacacgtt taatggatga tatcgcagac    1380

tttgaagaag acatggaaaa aggctataca gctaatgcac ttaactatta tatgaatgaa    1440

cacggagtaa ctaaagaaga agctagtcgt gaacttgaga aaatgaatgg tgatatgaac    1500

aaaattgtaa acgaagaatg tcttaaaatt acaactatgc cacgtcgtat cttaatgcaa    1560

agtgttaact acgctcgtag tttagatgta ttatacacag ctgatgatgt atataaccac    1620

cgtgaaggca aacttaaaga atatatgaga ttacttttag tagatccaat tttacttggt    1680

accggtgaaa atctttattt tcaaggttca ggtggtggtg gttctgatta taaagatgat    1740

gacgataaag gaaccggtta a                                              1761

<210> 108

<211> 1662
<212> DNA
<213> Artificial Sequence

<220>
<223> Description of Artificial Sequence: Synthetic
      polynucleotide

<400> 108
atggtaccag agagtcaaac aacattcaaa tacgaatcat tagcatttac aaaacttagt      60

cactgtcaat ggacagacta ttttcttagt gttccaattg atgaaagtga attagatgtt     120

attactcgtg aaattgatat tcttaaacca gaagttatgg agttattaag tagtcaagga     180

gatgatgaaa caagtaaaag aaaagttctt cttattcagt tattactttc tttaggttta     240

gcattccact ttgaaaatga gattaaaaac atacttgaac acgcatttcg taaaatagat     300

gatataactg gtgacgaaaa agacttatca acaattagta ttatgttccg tgttttccgt     360

acttatggac acaatcttcc aagtagtgtt tttaaacgtt tcacaggtga tgatggtaaa     420

tttcagcaaa gtttaacaga agacgcaaaa ggtattttaa gtttatatga agctgcacat     480

ttaggtacta ctacagatta cattttagat gaagctctta aattcacatc tagtcactta     540

aaaagtttac ttgctggtgg tacatgtcgt cctcacatct tacgtttaat ccgtaataca     600

ttatacttac cacaacgttg gaacatggaa gctgttatcg ctcgtgaata catatcattt     660

tacgagcagg aagaagatca cgataaaatg cttttacgtc ttgcaaaact taactttaaa     720

cttcttcaat tacactacat taaagagctt aaaagtttca ttaaatggtg gatggaactt     780

ggtttaactt ctaaatggcc ttctcaattt cgtgaacgta ttgttgaagc atggttagct     840

ggattaatga tgtattttga accacagttc tcaggtggtc gtgttattgc tgcaaaattc     900

aactatttac ttacaatatt agacgacgca tgtgaccact atttttctat tcacgaatta     960

acacgtttag ttgcatgtgt agaacgttgg tcaccagatg gtattgacac attagaagat    1020

atttcacgtt ctgtattcaa attaatgtta gatgttttcg acgatattgg taaaggtgta    1080

cgttcagaag gttctagtta ccacttaaaa gaaatgttag aggaattaaa cactttagtt    1140

cgtgctaatt tagatttagt taaatgggct cgtggaatac aaacagctgg taaagaggct    1200

tatgaatggg ttcgttcacg tccacgttta atcaaatctt tagcagctaa aggtagactt    1260

atggatgata ttacagactt tgactcagat atgagtaatg gattcgcagc taatgctatt    1320

aactactata tgaaacaatt tgttgttaca aaagaagaag ctattcttga atgtcaacgt    1380

atgattgtag acattaacaa aactattaat gaagagttat taaaaactac ttcagttcca    1440

ggtcgtgtat taaaacaagc tcttaacttt ggccgtttat tagaattatt atatacaaaa    1500

tctgacgata tttacaattg ttctgaaggc aaacttaaag aatacattgt aactctttta    1560

attgatccta taagacttgg taccggtgaa aacttatact ttcaaggttc aggcggtggt    1620

ggtagtgatt acaaagatga tgatgacaaa ggaaccggtt aa                    1662

<210> 109
<211> 1503
<212> DNA
<213> Artificial Sequence

<220>
<223> Description of Artificial Sequence: Synthetic
      polynucleotide

<400> 109
atggtaccag agagtcaaac aaaattcgac tacgaatcat tagcttttac aaaattatca      60

cattcacaat ggactgatta ctttttatca gtacctatag acgactctga acttgacgca     120

attactcgtg aaatcgacat tatcaaacct gaagttcgta aattactttc aagtaaaggt     180

gatgatgaaa cttctaaacg taaagtatta cttatccaaa gtttattatc attaggttta     240

gcatttcatt ttgaaaacga aattaaagat attttagaag atgcatttag acgtattgat     300

gacattacag gtgatgaaaa cgacttaagt actattagta ttatgttccg tgtattccgt     360

acatacggtc acaatttacc aagtagtgtt tttaaacgtt tcactggtga tgacggtaaa     420

tttgaacgtt ctttaactga agatgctaaa ggaattttat cattatatga agctgcacat     480

ttaggaacaa ctactgatta tattcttgat gaagcattag aatttacttc atcacactta     540

aaatctttac ttgttggtgg tatgtgtcgt ccacatattt tacgtcttat tagaaatact     600

ttatatcttc cacaacgttg gaatatggaa gcagtaattg caagagaata cattagtttt     660

tatgaacaag aagaagatca cgataaaatg ttacttcgtt tagctaaatt aaatttcaaa     720

ttacttcaat tacactacat taaagagtta aaaacattca ttaaatggtg gatggaatta     780

ggacttacat caaaatggcc ttctcaattt cgtgaacgta ttgttgaagc atggttagct     840

ggtcttatga tgtattttga accacagttt tctggaggtc gtgtaatagc tgctaaattc     900

aattacttat taacaatttt agatgatgca tgtgatcact atttctcaat tccagaatta     960

actcgtttag ttgattgcgt agaaagatgg aatcatgatg gtatacatac tttagaagac    1020

atctcacgta tcatctttaa acttgcatta gatgtatttg atgatattgg tcgtggtgtt    1080

cgttctaaag gttgttctta ttacttaaaa gaaatgttag aagagttaaa aatcttagtt    1140

cgtgcaaact tagatttagt taaatgggct cgtggtaatc aattacctag ttttgaagaa    1200

cacgttgagg taggtggtat tgctcttaca acatacgcaa ctttaatgta ctcttttgtt    1260

ggcatgggtg aagcagtagg taaagaagca tacgaatggg tacgttctcg tccacgttta    1320

atcaaaagtt tagcagcaaa aggtcgtctt atggacgata ttactgattt cgaagtaaaa    1380

attatcaact tatttttcga ccttctttta tttgtattcg gtaccggtga aaacttatat    1440

ttccagggta gtggtggagg aggttcagac tacaaagatg acgatgacaa aggaaccggt    1500

taa                                                                 1503

<210> 110
<211> 1725
<212> DNA
<213> Artificial Sequence

<220>
<223> Description of Artificial Sequence: Synthetic
      polynucleotide

<400> 110
atggtaccag cagctttcac agcaaatgca gttgacatgc gtccaccagt tattacaatt      60

cacccacgtt caaaagatat tttctctcaa ttttctttag atgataaatt acaaaaacaa     120

tacgctcaag gaatcgaagc tcttaaagaa gaagctcgtt ctatgcttat ggctgcaaaa     180

tctgctaaag taatgatctt aattgataca cttgaacgtt taggattagg ttatcacttt     240

gaaaaagaaa ttgaagagaa attagaagct atttacaaaa aagaggatgg tgacgattat     300

gatcttttta caactgcttt aagattccgt ttacttagac aacaccaacg tcgtgtacca     360

tgttctgttt ttgacaaatt tatgaataaa gagggtaaat tcgaagaaga accattaatt     420

tcagatgttg aaggtcttct ttcattatat gacgctgctt atttacagat tcacggtgaa     480

cacattttac aagaggcttt aattttcact acacatcatt taactcgtat tgaaccacaa     540

ttagatgatc actctccttt aaaattaaaa ttaaaccgtg ctttagaatt tcctttttac     600

agagaaatcc ctataatcta tgcacatttt tacatttcag tatatgaacg tgacgattct     660

cgtgatgaag tattattaaa aatggctaaa ttatcttata atttcttaca aaacttatac     720

aaaaaagaat taagtcaact ttctcgttgg tggaacaaat tagaacttat tcctaattta     780

ccttatattc gtgattctgt agctggagct tatttatggg ctgttgcttt atatttcgaa     840

cctcaatatt cagacgttcg tatggcaatt gctaaactta tccaaattgc agcagctgta     900

gatgatactt acgataatta tgctactata cgtgaagctc aattattaac agaagcatta     960

gaacgtttaa atgtacacga aattgacaca ttaccagatt atatgaaaat tgtttatcgt    1020

tttgtaatgt catggagtga agatttcgaa cgtgatgcta caattaaaga acagatgtta    1080

gctacacctt atttcaaagc tgaaatgaaa aaacttggtc gtgcttataa tcaagaactt    1140

aaatgggtta tggaacgtca attacctagt ttcgaagaat acatgaaaaa ctctgaaatc    1200

acttctggtg tttacattat gtttactgta attagtcctt acttaaatag tgcaacacaa    1260

aaaaacattg actggttatt atcacaacct cgtttagcat cttcaactgc aattgttatg    1320

cgttgttgta atgatttagg ctctaatcaa cgtgaatcta aaggaggaga agttatgaca    1380

tctttagatt gctatatgaa acaacacggt gctagtaaac aagaaacaat ttctaaattc    1440

aaacttatta tcgaagatga atggaaaaac ttaaatgaag aatgggctgc aacaacatgt    1500

cttccaaaag ttatggtaga aatttttcgt aactatgcac gtattgcagg cttttgctac 1560

aaaaataacg gtgatgctta tacatctcca aaaattgtac aacaatgttt tgacgcttta 1620

tttgtaaatc cattaagaat tggtaccggt gagaatttat actttcaagg ctcaggtgga 1680

ggtggtagtg attataaaga tgatgatgat aaaggaaccg gttaa 1725

<210> 111
<211> 1818
<212> DNA
<213> Artificial Sequence

<220>
<223> Description of Artificial Sequence: Synthetic
      polynucleotide

<400> 111
atggtaccag aatttagagt tcatttacag gctgataatg aacagaaaat attccagaac 60

caaatgaaac ctgaacctga agcatcatat cttattaatc aacgtagatc agctaattac 120

aaacctaata tttggaaaaa tgacttttta gatcaaagtt taattagtaa atacgacggt 180

gatgaatatc gtaaattaag tgagaaatta atcgaggaag taaaaattta tatatctgct 240

gagacaatgg acttagtagc taaattagaa cttattgatt ctgttcgtaa attaggttta 300

gctaatcttt ttgaaaaaga aattaaagaa gcattagatt ctatcgcagc tattgagtca 360

gataatttag gtactcgtga tgacttatat ggtactgctt tacactttaa aattttacgt 420

caacatggtt ataaagtttc tcaagatatt tttggtcgtt tcatggatga aaaaggtaca 480

ttagaaaatc atcacttcgc tcacttaaaa ggtatgttag aattatttga agcatctaat 540

ttaggttttg aaggtgaaga tattttagat gaagcaaaag catcacttac attagctctt 600

cgtgatagtg gtcatatttg ttatccagat tctaacttaa gtcgtgatgt agtacactca 660

ttagaattac ctagtcaccg tcgtgttcaa tggtttgatg ttaaatggca aattaatgct 720

tatgaaaaag atatttgtag agttaatgca actcttttag aattagcaaa attaaatttt 780

aacgtagtac aagcacaact tcaaaaaaac ttacgtgaag catctcgttg gtgggctaac 840

ttaggtttcg ctgataactt aaaattcgct cgtgatcgtt tagttgaatg tttttcttgc 900

gcagtaggcg tagcatttga acctgaacac tcttcttttc gtatctgttt aacaaaagtt 960

attaatttag ttttaataat tgatgacgta tacgacatat atggaagtga agaagaatta 1020

aaacacttta caaatgctgt tgatcgttgg gattctcgtg aaacagaaca attaccagaa 1080

tgtatgaaaa tgtgctttca agttttatac aatactacat gtgaaattgc tcgtgaaatt 1140

gaagaagaaa atggatggaa tcaagtttta cctcaattaa ctaaagtatg ggctgatttt 1200

tgtaaagcat tattagtaga agctgaatgg tacaataaaa gtcacatccc aactttagaa 1260

gaatatcttc gtaatggctg tatttcatca agtgtttctg tattattagt acattctttc 1320

tttagtatta cacatgaagg tacaaaagaa atggcagatt tcttacacaa aaacgaagac 1380

ttattataca acatctcatt aattgtacgt ttaaacaacg acttaggtac aagtgcagct 1440

gaacaagaac gtggtgattc accatcatct attgtatgtt acatgcgtga agttaatgct 1500

agtgaagaaa cagctcgtaa aaatataaaa ggaatgatcg acaatgcttg gaaaaaagtt 1560

aatggtaaat gttttacaac taatcaagtt ccttttcttt cttcttttat gaataacgct 1620

actaatatgg ctcgtgtagc tcattcatta tataaagacg gagacggttt tggcgatcag 1680

gaaaaaggtc cacgtactca catcttatct ttattattcc aaccattagt taacggtacc 1740

ggtgaaaact tatactttca aggttctggt ggtggtggtt ctgactacaa agatgacgat 1800

gacaaaggaa ccggttaa 1818

<210> 112
<211> 1773
<212> DNA
<213> Artificial Sequence

<220>
<223> Description of Artificial Sequence: Synthetic
      polynucleotide

<400> 112
atggtaccaa gtagtaatgt atcagctatt cctaattctt ttgaattaat tcgtcgttca 60

gctcaatttc aggcttctgt atggggtgat tactttttat cttatcactc tttaccacct 120

gagaaaggta ataaagtaat ggaaaaacaa actgaagaac ttaaagagga aatcaaaatg 180

gaattagttt ctactactaa agatgaacca gagaaattac gtttaattga ccttattcaa 240

cgtttaggtg tatgttatca ctttgaaaat gaaattaaca acattttaca acaattacac 300

cacattacta ttacttctga gaaaaacggt gacgataatc cttataacat gactttatgt 360

ttccgtttat tacgtcaaca aggttacaat gtatctagtg aaccttttga tcgttttcgt 420

ggcaaatggg aatcttctta tgataacaat gtagaagaac ttttatcatt atatgaagca 480

tctcaattaa gaatgcaagg tgaagaagca ttagatgaag cattctgttt tgcaactgca 540

caattagaag ctattgttca agatcctact acagatccaa tggttgcagc agaaatcaga 600

caagcattaa aatggccaat gtacaaaaac ttacctcgtt taaaagctcg tcatcatatt 660

ggtttatatt ctgagaaacc atggcgtaat gagtcattac ttaatttcgc aaaaatggac 720

ttcaataaac ttcaaaattt acatcaaact gaaattgcat atatttctaa atggtgggac 780

gattacggct ttgcagaaaa actttctttc gcacgtaatc gtattgttga aggctatttc 840

ttcgcattag gtatcttttt cgaacctcaa cttttaacag cacgtcttat aatgacaaaa 900

gtaatcgcta ttggttctat gttagatgac atttatgatg tttatggtac ttttgaagag 960

ttaaaacttt taacattagc tttagaacgt tgggataaat cagaaacaaa acaattacct 1020

aattacatga aaatgtacta cgaagcatta ttagatgttt ttgaagaaat tgagcaagaa 1080

atgtcacaaa aagaaactga aacaacacca tactgtattc atcacatgaa agaagctact 1140

aaagaacttg gacgtgtatt tttagttgaa gcaacttggt gtaaagaagg ttatactcct 1200

aaagtagagg aatacttaga cattgcttta atttcttttg gtcataaatt acttatggta 1260

actgctttat taggtatggg ttctcacatg gctacacaac aaattgtaca atggattaca 1320

tctatgccaa atatcttaaa agcatctgca gtaatatgtc gtttaatgaa tgacattgta 1380

tctcataaat ttgaacaaga acgtggtcat gttgcttctg ctatcgaatg ctacatggaa 1440

caaaaccacc ttagtgaata tgaagcatta attgctcttc gtaaacaaat tgatgattta 1500

tggaaagaca tggtagaaaa ttactgtgca gtaatcacag aagacgaagt acctcgtggt 1560

gttttaatgc gtgttttaaa tcttacacgt ttattcaatg ttatttacaa agacggtgat 1620

ggatacacac aaagtcatgg tagtacaaaa gctcacatta aaagtctttt agttgatagt 1680

gtacctcttg gtaccggtga aaatctttac tttcaaggtt caggtggagg tggttctgat 1740

tataaagatg atgatgacaa aggaaccggt taa 1773

<210> 113
<211> 1770
<212> DNA
<213> Artificial Sequence

<220>
<223> Description of Artificial Sequence: Synthetic
      polynucleotide

<400> 113
atggtaccaa aagacatgag tattccatta ttagcagctg tatcttctag tacagaagaa 60

acagtacgtc ctatcgcaga ttttcatcca acactttggg gtaatcattt tcttaaatct 120

gctgctgacg tagaaactat tgatgcagca acacaagagc aacacgctgc attaaaacaa 180

gaagtacgtc gtatgattac tacaacagca aataaacttg cacaaaaact tcacatgatt 240

gatgctgtac aacgtttagg tgttgcttat cattttgaaa aagaaattga agacgaatta 300

ggtaaagtaa gtcacgattt agattcagat gatttatacg ttgtatcttt acgttttcgt 360

ttattccgtc aacaaggtgt aaaaattagt tgcgatgttt tcgacaaatt caaagatgac 420

gaaggaaaat tcaaagagtc tcttattaac gatattagag gaatgttatc attatacgaa 480

gcagcttact tagctattag aggtgaagat attttagacg aagcaattgt tttcacaact 540

actcacttaa aaagtgttat ctctattagt gatcattcac atgctaatag taatttagct 600

gaacaaatac gtcatagttt acaaattcca cttcgtaaag ctgctgcaag attagaagca 660

cgttatttct tagatattta ctctcgtgat gatttacatg atgaaacatt acttaaattc 720

gctaaacttg actttaacat tcttcaagct gcacaccaaa aagaagctag tattatgact 780

cgttggtgga acgatttagg ttttcctaaa aaagttcctt atgctcgtga ccgtattata 840

gaaacttata tttggatgtt attaggagtt tcatacgaac ctaatttagc atttggaaga 900

atttttgcaa gtaaagtagt atgtatgatt acaacaattg atgatacatt tgatgcttat 960

ggtacatttg aagagttaac attattcact gaagctgtta cacgttggga tattggttta 1020

attgacacat tacctgaata tatgaaattc attgtaaaag ctcttttaga catttaccgt 1080

gaagctgaag aagaattagc taaagaaggt agatcatacg gtattccata cgctaaacaa 1140

atgatgcaag agttaatcat tttatacttt actgaggcta aatggttata caaaggttac 1200

gttcctacat ttgacgaata caaaagtgta gctttacgtt ctattggtct tagaacatta 1260

gcagtagctt catttgtaga tttaggtgac tttattgcta caaaagacaa ttttgaatgt 1320

attcttaaaa atgcaaaaag tttaaaagct actgaaacaa ttggccgttt aatggatgat 1380

atagctggtt acaaatttga acagaaacgt ggtcataacc catctgctgt tgagtgttac 1440

aaaaatcaac acggagtatc agaagaagaa gcagttaaag agcttttatt agaagttgca 1500

aacagttgga aagatattaa cgaggaactt ttaaatccaa ctacagttcc attacctatg 1560

ttacagcgtt tattatattt tgctcgttca ggtcacttca tctatgatga tggacatgat 1620

cgttatacac attctttaat gatgaaaaga caagttgcac ttttattaac tgaaccttta 1680

gctattggta ccggtgaaaa cttatacttt caaggttcag gtggtggtgg atctgattat 1740

aaagatgatg atgacaaagg aaccggttaa 1770

<210> 114
<211> 1830
<212> DNA
<213> Artificial Sequence

<220>
<223> Description of Artificial Sequence: Synthetic
      polynucleotide

<400> 114
atggtaccag atttagctgt tgagattgca atggacttag ctgttgatga cgttgagcgt 60

cgtgtaggtg actatcatag taacctttgg gatgatgatt ttattcagag tttatcaaca 120

ccatacggcg catcatcata tcgtgaacgt gctgaaagat tagtaggaga agttaaagaa 180

atgtttactt ctatttctat cgaagatggt gaacttacat ctgatttatt acaacgttta 240

tggatggtag ataatgtaga gcgtttaggc atttcacgtc atttcgagaa cgaaataaaa 300

gcagctattg attatgttta ttcatattgg agtgacaaag gtattgtacg tggtcgtgat 360

tcagctgttc ctgacttaaa tagtattgct ttaggttttc gtacattacg tttacacggt 420

tacacagtta gtagtgatgt atttaaagtt ttccaagatc gtaaaggtga atttgcttgc 480

agtgcaattc caactgaagg agatattaaa ggagttttaa acttacttcg tgcaagttat 540

attgcattcc ctggtgaaaa agtaatggaa aaagctcaaa cttttgcagc aacatacctt      600

aaagaagcat tacagaaaat tcaagtaagt agtttaagtc gtgaaatcga atatgttctt      660

gaatacggtt ggttaactaa ctttcctcgt ttagaagcac gtaactatat tgacgtattc      720

ggtgaagaaa tttgtccata cttcaaaaaa ccatgtatta tggttgacaa acttttagaa      780

ttagcaaaat tagaatttaa cttatttcac agtcttcaac aaacagagtt aaaacatgtt      840

agtcgttggt ggaaagatag tggtttctct caattaacat ttacaagaca ccgtcatgtt      900

gagttttata cattagctag ttgtatagca attgaaccaa aacacagtgc ttttcgtctt      960

ggttttgcta aagtttgtta tttaggtata gttttagatg atatttatga cacatttggt     1020

aaaatgaaag aattagaact ttttactgca gcaatcaaac gttgggaccc ttctactaca     1080

gaatgcttac ctgaatacat gaaaggtgtt tatatggctt tttacaattg tgttaatgaa     1140

ttagcacttc aagcagagaa aacacaaggt cgtgatatgt taaactatgc acgtaaagca     1200

tgggaagctc tttttgatgc atttttagaa gaagcaaaat ggatctcttc tggctattta     1260

ccaacattcg aagaatactt agaaaatggt aaagtatctt ttggttatcg tgctgctaca     1320

ttacaaccaa ttttaacatt agatattcct ttacctttac atattttaca acagattgat     1380

tttccaagtc gttttaatga tttagcttca tctattttac gtttaagagg tgatatctgt     1440

ggttaccaag ctgaacgtag tcgtggtgaa gaagcatcat caatttcatg ttatatgaaa     1500

gataatccag gttctactga agaagatgca ttatctcaca ttaatgcaat gatctcagac     1560

aatattaacg aattaaactg ggaactttta aaaccaaatt caaatgtacc aatttcatca     1620

aaaaaacatg catttgacat tcttcgtgct ttctatcact tatacaaata tcgtgatggc     1680

ttctctatcg caaaaattga aactaaaaat cttgtaatgc gtacagtttt agaacctgta     1740

ccaatgggta ccggtgaaaa cttatacttt cagggttctg gtggaggtgg ttcagactat     1800

aaagatgatg atgataaagg aaccggttaa                                      1830

<210> 115
<211> 2511
<212> DNA
<213> Artificial Sequence

<220>
<223> Description of Artificial Sequence: Synthetic
      polynucleotide

<400> 115
atggtaccaa caagtgtatc agtagaatca ggaacagtat cttgtttatc atcaaacaac       60

ttaattagac gtacagctaa tccacatcct aacatttggg gatatgattt tgttcactca      120

cttaaatcac catatacaca cgactcatca tatcgtgaac gtgctgagac tttaatttca      180

gaaataaaag ttatgcttgg aggtggtgaa ttaatgatga ctccatcagc ttatgataca      240

gcatgggtag ctcgtgttcc atcaattgac ggtagtgctt gtccacaatt tccacaaact 300

gttgaatgga ttcttaaaaa ccaattaaaa gatggtagtt ggggaactga atctcacttc 360

ttacttagtg acagattatt agctacatta agttgtgtat tagcattatt aaaatggaaa 420

gtagctgatg ttcaagtaga gcaaggtatt gagtttatca aacgtaattt acaagctatt 480

aaagacgaac gtgatcaaga cagtttagta actgatttcg agattatttt cccatcactt 540

ttaaaagagg ctcaatcttt aaacttaggc ttaccttatg atttaccata tattagatta 600

ttacaaacaa aacgtcaaga acgtcttgct aacttaagta tggataaaat tcacggtggt 660

actttattat catctttaga gggcattcaa gatatagttg aatgggaaac aattatggat 720

gtacaatctc aagatggttc tttcttatca tcaccagctt ctacagcatg tgtattcatg 780

catacaggag atatgaaatg tttagatttc ttaaacaacg tattaactaa atttggtagt 840

agtgttcctt gtttataccc tgtagattta ttagaacgtc ttttaattgt agataatgta 900

gagcgtcttg gtattgaccg tcattttgaa aaagaaatca aagaggcttt agattatgtt 960

tatcgtcatt ggaacgatcg tggtattggt tggggtcgtt tatcacctat cgcagactta 1020

gaaacaacag ctttaggttt tcgtttactt cgtcttcatc gttacaatgt ttctcctgta 1080

gtattagaca atttcaaaga cgcagatggc gagttcttct gcagtacagg tcaatttaac 1140

aaagatgttg caagtatgtt atctttatac cgtgcttctc aattagcttt ccctgaagaa 1200

tcaattttag atgaagctaa atcattctca acacaatatc ttcgtgaagc attagaaaaa 1260

tcagaaacat tttcttcttg gaatcatcgt cagagtttat cagaagaaat taaatatgct 1320

ttaaaaacat catggcacgc ttcagttcct cgtgttgaag caaaacgtta ttgtcaggtt 1380

taccgtcaag actatgctca tttagcaaaa tcagtttata aacttcctaa agtaaataat 1440

gagaaaattc ttgaattagc aaaattagat tttaacatta ttcaatctat ccatcaaaaa 1500

gaaatgaaaa atgttacatc atggtttcgt gattcaggct taccactttt cacatttgct 1560

cgtgaaagac ctttagagtt ttacttttta atcgctggtg gaacatacga acctcaatac 1620

gcaaaatgta gattcttatt tacaaaagta gcttgtttac aaactgtttt agacgatatg 1680

tacgatactt acggtacacc atcagagtta aaattattta ctgaggcagt tcgtcgttgg 1740

gatttatcat tcacagaaaa cttacctgat tatatgaaat tatgctacaa aatttactat 1800

gatattgttc atgaagttgc ttgggaagta gaaaaagaac agggacgtga gcttgtttca 1860

tttttccgta aaggttggga agactatctt ttaggttatt atgaagaagc tgaatggtta 1920

gctgctgaat acgttcctac tttagatgaa tacattaaaa acggtattac atctattggt 1980

caacgtattt tacttttatc aggtgtactt attatggaag gtcaactttt atcacaagaa 2040

gctcttgaaa aagtagatta tccaggtcgt cgtgttttaa cagaattaaa cagtttaatt 2100

agtcgtttag cagacgatac taaaacatac aaagcagaaa aagctcgtgg tgaacttgct 2160

agtagtattg aatgttatat gaaagaccac cctggttgtc aagaagaaga agcattaaac 2220

catatttatg gcattttaga accagctgtt aaagaattaa ctcgtgagtt tcttaaagca 2280

gatcacgtac cattcccttg caaaaaaatg ttatttgatg aaacaagagt tacaatggta 2340

attttcaaag atggtgatgg tttcggtatt tctaaattag aagtaaaaga ccacataaaa 2400

gaatgtttaa ttgagccatt accacttggt accggtgaaa atctttattt tcaaggtagt 2460

ggtggtggcg gttctgacta caaagatgac gacgataaag gaaccggtta a 2511

<210> 116
<211> 1731
<212> DNA
<213> Artificial Sequence

<220>
<223> Description of Artificial Sequence: Synthetic
      polynucleotide

<400> 116
atggtaccag gttctgaagt aaatagacct ttagcagact ttccagcaaa catttgggaa 60

gacccattaa cttctttctc aaaatctgat cttggtacag aaacatttaa agagaaacat 120

agtactttaa aagaagctgt taaagaggca tttatgagtt ctaaagctaa tccaatcgaa 180

aatatcaaat tcatagatgc attatgccgt ttaggagtat cttatcactt tgaaaaagat 240

attgtagaac aattagataa atcatttgat tgcttagatt ttccacaaat ggtacgtcaa 300

gaaggttgcg atttatatac agttggtatt atctttcaag tttttagaca atttggtttc 360

aaattaagtg ctgatgtttt tgaaaaattc aaagatgaaa atggtaaatt caaaggtcac 420

ttagtaactg atgcttatgg tatgttatca ttatacgaag ctgcacaatg gggtactcac 480

ggtgaagaca tcattgacga agctcttgct ttttctcgta gtcacttaga agaaatatct 540

agtcgtagtt caccacactt agcaattcgt attaaaaacg ctttaaaaca tccatatcat 600

aaaggtattt cacgtattga aacacgtcaa tacattagtt actatgaaga agaagaatct 660

tgtgatccaa cattattaga gttcgctaaa attgacttta acttattaca aattttacac 720

cgtgaagagt tagcttgtgt aactcgttgg catcatgaaa tggaatttaa aagtaaagta 780

acttacacac gtcatcgtat tacagaagca tatttatgga gtcttggaac atattttgaa 840

ccacaataca gtcaagctcg tgtaataact acaatggcat taatcttatt tactgcttta 900

gacgacatgt acgatgctta cggtactatg gaggagttag agttattcac agatgctatg 960

gacgaatggt taccagttgt tccagatgaa attcctattc cagattcaat gaaattcatt 1020

tacaatgtta cagttgaatt ttacgataaa ttagacgaag aattagaaaa agaaggtcgt 1080

tctggttgtg gtttccatct taaaaaaagt ttacaaaaaa cagctaatgg atatatgcaa 1140

gaagcaaaat ggcttaaaaa agattacatt gctacatttg atgagtataa agaaaatgct 1200

attttatctt caggttatta tgcattaatt gcaatgacat ttgttcgtat gactgatgtt 1260

gctaaattag atgcttttga atggttaagt agtcacccaa aaattcgtgt agcaagtgaa 1320

atcatttcac gttttacaga cgatatttca agttatgaat ttgaacacaa acgtgaacac 1380

gttgctacag gtattgattg ttatatgcaa caattcggag ttagtaaaga acgtgctgtt 1440

gaagttatgg gcaatatagt ttctgatgca tggaaagact taaatcaaga acttatgcgt 1500

cctcatgttt tcccatttcc acttcttatg cgtgttttaa atctttcaag agtaattgat 1560

gtattttatc gttaccaaga tgcatatact aatccaaaat tacttaaaga gcacattgtt 1620

tctttactta ttgaaactat tccaattggt accggtgaaa acttatactt tcaaggtagt 1680

ggtggaggtg gttctgatta taaagacgac gatgacaaag gaaccggtta a 1731

<210> 117
<211> 1773
<212> DNA
<213> Artificial Sequence

<220>
<223> Description of Artificial Sequence: Synthetic
      polynucleotide

<400> 117
atggtaccag aggcaattag agtatttggc ttaaaacttg gttcaaaatt atctattcac 60

tcacaaacaa atgcttttcc tgcattcaaa ttatctcgtt ttccattaac atctttccct 120

ggtaaacatg ctcacttaga tccattaaaa gcaacaactc atccattagc ttttgatggt 180

gaagaaaata accgtgagtt taaaaactta ggtccaagtg agtggggcca tcaatttctt 240

tctgctcatg tagatttatc tgaaatggat gcattagaac gtgaaattga agctcttaaa 300

ccaaaagtac gtgatatgtt aatatcaagt gaaagttcaa aaaaaaaaat cttatttctt 360

tatcttttag tatcattagg attagcttat cactttgaag atgaaattaa agaaagttta 420

gaggatggat tacagaaaat tgaggaaatg atggcttcag aagatgatct tcgttttaaa 480

ggcgataatg gtaaattcaa agaatgttta gcaaaagatg ctaaaggtat tttatctctt 540

tatgaggctg ctcacatggg tacaacaact gattatattc ttgatgaggc tttatcattt 600

actttaacat atatggaatc attagcagct tcaggaacat gtaaaatcaa cttatcacgt 660

cgtattagaa aagcattaga tcaacctcaa cacaaaaata tggaaataat tgtagcaatg 720

aaatacattc aattttatga agaagaggaa gattgcgata aaactttact taaatttgct 780

aaacttaact ttaaattctt acaattacac tatttacaag aacttaaaat cttatctaaa 840

tggtataaag accaagactt taaatcaaaa ttacctccat atttccgtga ccgtcttgta 900

gaatgtcatt ttgcatcatt aacatgtttt gagcctaaat atgctcgtgc acgtattttc 960

ttatctaaaa tcttcactgt tcaaattttc attgacgata cttgtgaccg ttacgcatca 1020

ttaggtgaag ttgagtcatt agctgacact atcgaacgtt gggaccctga tgatcatgct 1080

atggacggat tacctgatta tcttaaatca gtagttaaat ttgtattcaa tacatttcaa 1140

gaatttgaac gtaaatgtaa acgttcactt cgtattaact tacaagtagc aaaatgggtt 1200

aaagctggtc acttaccatc ttttgatgag tatcttgatg tagctggttt agaattagct 1260

atttcattca ctttcgctgg tatcttaatg ggcatggaaa atgtttgtaa acctgaagca 1320

tacgaatggt taaaatctcg tgacaaactt gttcgtggtg taatcacaaa agttcgttta 1380

cttaatgata tttttggcta tgaagatgat atgcgtcgtg gttatgtaac aaattcaata 1440

aactgctaca aaaaacaata tggagtaaca gaggaagaag ctattcgtaa attacatcaa 1500

atcgttgctg atggagagaa aatgatgaat gaagagttct taaaacctat taatgtacca 1560

tatcaggttc ctaaagtagt tattttagac actttacgtg cagctaatgt ttcatacgaa 1620

aaagatgacg aatttacacg tccaggcgaa caccttaaaa actgcattac atctatttac 1680

ttcgatttag gtaccggtga aaacttatac tttcaaggta gtggtggcgg tggtagtgat 1740

tacaaagatg atgatgataa aggaaccggt taa 1773

<210> 118
<211> 1128
<212> DNA
<213> Artificial Sequence

<220>
<223> Description of Artificial Sequence: Synthetic
      polynucleotide

<400> 118
atggtaccaa ctacaacatt atcatctaac cttaactcac aattcatgca ggtttacgag 60

actcttaaat cagaacttat tcatgaccca ttatttgagt tcgatgacga ttcaagacaa 120

tgggtagaac gtatgattga ttatactgta ccaggtggta aaatggttcg tggttatagt 180

gtagtagata gttatcaatt acttaaaggt gaagaactta cagaagaaga ggcattttta 240

gcttgtgcac ttggttggtg tacagaatgg tttcaagcat tcattctttt acatgatgat 300

atgatggatg gtagtcacac aagacgtggt caaccatgtt ggtttcgttt acctgaggtt 360

ggtgctgttg ctattaatga tggtgtttta cttcgtaatc acgttcaccg tattcttaaa 420

aaacattttc aaggtaaagc atattatgtt catttagttg atttattcaa tgaaactgaa 480

tttcaaacaa ttagtggaca aatgatcgac ttaattacaa cattagttgg tgaaaaagac 540

ttatctaaat attcattaag tattcatcgt cgtatcgttc aatacaaaac agcatactac 600

tcattttact taccagttgc ttgtgcttta cttatgtttg gtgaggatct tgataaacat 660

gtagaagtta aaaatgttct tgttgaaatg ggtacatatt ttcaagttca agatgattat 720

ttagattgtt ttggtgctcc agaagttatt ggcaaaattg gtactgatat tgaagacttt 780

aaatgttcat ggttagtagt taaagcatta gaattagcaa atgaagaaca gaaaaaaact 840

ttacacgaaa attatggaaa aaaagatcca gcatcagttg ctaaagttaa agaagtatac 900

cacacactta atttacaagc tgttttcgaa gattatgaag caacatcata caaaaaactt 960

attacttcta ttgaaaatca cccatctaaa gctgttcaag ctgttttaaa atctttctta 1020

ggcaaaatat acaaacgtca aaaaggtacc ggtgaaaact tatactttca aggttctggt 1080

ggcggtggaa gtgattacaa agatgatgac gataaaggaa ccggttaa 1128

<210> 119
<211> 1803
<212> DNA
<213> Artificial Sequence

<220>
<223> Description of Artificial Sequence: Synthetic
      polynucleotide

<400> 119
atggtaccaa gtcaacctta ctgggctgca attgaagcag acattgaaag atatttaaaa 60

aaatcaatta caattcgtcc accagaaact gtatttggtc ctatgcacca tttaacattt 120

gctgctcctg ctactgcagc tagtacatta tgccttgctg cttgtgaatt agttggcggt 180

gatcgtagtc aagctatggc agctgctgct gctatccatt tagttcatgc agctgcttac 240

gttcacgaac atcttccttt aacagatgga tcacgtcctg taagtaaacc tgctattcaa 300

cataaatatg gtccaaacgt tgaactttta acaggtgatg gtatcgttcc tttcggtttt 360

gagttattag caggttcagt agatccagca cgtactgatg accctgatcg tattttacgt 420

gtaattattg aaatttctcg tgctggtgga ccagaaggca tgatttctgg tttacaccgt 480

gaggaagaaa tcgtagatgg taacacatca ttagacttta tagaatatgt atgcaaaaaa 540

aaatacggtg aaatgcacgc atgtggtgca gcttgcggag ctattttagg tggagctgct 600

gaagaagaaa ttcaaaaact tcgtaacttt ggtctttatc aaggcacatt acgtggtatg 660

atggaaatga aaaatagtca tcagttaatt gacgaaaata tcattggaaa acttaaagaa 720

cttgctcttg aagaattagg tggattccac ggtaaaaacg ctgaattaat gagttcttta 780

gttgctgaac ctagtttata tgcagcttca tcaaataact taggtatcga aggtcgtttt 840

gactttgacg gttacatgct tcgtaaagca aaatctgtaa ataaagcatt agaagctgct 900

gttcaaatga aagaaccact taaaattcac gaatcaatgc gttattcatt attagctggt 960

ggtaaacgtg ttcgtccaat gttatgtatt gcagcttgtg aacttgttgg tggtgacgaa 1020

tctacagcaa tgcctgcagc atgtgctgtt gaaatgattc acacaatgtc tttaatgcat 1080

gatgaccttc catgtatgga taacgatgac ttacgtcgtg gtaaacctac aaaccacatg 1140

gcttttggtg agtctgtagc tgttcttgct ggtgatgcat tacttagttt tgcttttgaa 1200

catgttgctg ctgcaacaaa aggcgcacca cctgaacgta tcgtacgtgt attaggtgaa 1260

ttagctgtta gtattggttc agaaggactt gtagcaggtc aagttgtaga cgtttgttct 1320

gaaggcatgg ctgaagtagg attagatcat cttgaattta ttcaccatca taaaactgct 1380

gcattattac aaggttcagt tgttttaggt gcaatattag gaggcggtaa agaagaagaa 1440

gtagctaaac ttcgtaaatt tgctaactgt attggtttac ttttccaagt tgttgatgat 1500

attttagatg ttactaaaag tagtaaagag ttaggtaaaa ctgcaggtaa agacttagta 1560

gctgataaaa ctacatatcc taaacttata ggcgttgaaa aatcaaaaga atttgctgac 1620

cgtttaaatc gtgaagcaca agaacaatta ttacattttc atcctcaccg tgctgctcca 1680

ttaatcgctt tagctaacta catcgcttac cgtgataatg gtaccggtga aaacttatac 1740

ttccagggta gtggtggtgg cggatcagat tataaagatg acgatgataa aggaaccggt 1800

taa 1803

<210> 120
<211> 1371
<212> DNA
<213> Artificial Sequence

<220>
<223> Description of Artificial Sequence: Synthetic
      polynucleotide

<400> 120
atggtaccag taacagcagc acgtgcaaca ccaaaattaa gtaatagaaa attacgtgtt 60

gctgtaattg gaggcggtcc agcaggaggt gcagctgctg aaacattagc acaaggaggt 120

attgaaacaa ttcttatcga acgtaaaatg gataattgta aaccatgtgg tggtgctatt 180

ccattatgta tggtaggaga gttcaattta cctttagaca ttattgaccg tcgtgtaaca 240

aaaatgaaaa tgatctctcc ttcaaacatt gcagttgata tcggtcgtac acttaaagaa 300

cacgaatata ttggtatggt tcgtcgtgag gtacttgatg cttatcttcg tgaacgtgca 360

gaaaaatcag gtgctactgt tattaacggt ttattcttaa aaatggatca cccagaaaat 420

tgggattcac catatacact tcactacaca gagtatgatg gaaaaacagg tgctacagga 480

actaaaaaaa ctatggaagt agatgctgtt attggtgctg atggtgctaa ttctcgtgtt 540

gcaaaaagta ttgacgcagg tgattatgat tatgctattg catttcaaga acgtattcgt 600

atacctgatg agaaaatgac ttattatgag gacttagctg agatgtatgt aggtgatgat 660

gtatcaccag acttctacgg ttgggtattc ccaaaatgtg atcatgtagc tgttggtaca 720

ggtactgtaa cacataaagg tgatatcaaa aaattccagt tagctacacg taatcgtgct 780

aaagataaaa ttcttggtgg caaaataatc cgtgtagagg ctcatcctat tccagagcat 840

cctagaccac gtcgtttatc aaaacgtgtt gcattagtag gcgacgcagc aggttacgtt 900

actaaatgtt caggagaagg aatttacttc gcagctaaat ctggtcgtat gtgtgctgaa 960

gctatcgttg aaggttcaca aaatggcaaa aaaatgatag atgaaggcga tttaagaaaa 1020

tacttagaaa aatgggataa aacttactta ccaacttatc gtgttttaga tgtacttcaa 1080

aaagttttct atcgttctaa cccagctcgt gaggcttttg ttgaaatgtg taacgatgag 1140

tatgtacaga aaatgacatt tgattcttac ctttataaac gtgtagctcc tggtagtcca 1200

ttagaagata tcaaattagc tgtaaatact attggttcac ttgttcgtgc taacgcatta 1260

cgtcgtgaaa ttgagaaatt atcagtaggt accggtgaga atctttactt tcaaggatca 1320

ggtggtggtg gttctgatta taaagatgac gatgataaag gaaccggtta a 1371

<210> 121
<211> 1362
<212> DNA
<213> Artificial Sequence

<220>
<223> Description of Artificial Sequence: Synthetic
      polynucleotide

<400> 121
atggtaccag tagctgttat tggtggtggt ccaagtggcg cttgtgcagc agaaacttta 60

gcaaaaggtg gtgtagaaac tttcttactt gagcgtaaat tagataattg taaaccttgt 120

ggaggtgcaa ttccattatg tatggttgaa gaatttgatt taccaatgga aataattgac 180

cgtcgtgtta ctaaaatgaa aatgatatca ccttcaaacc gtgaagttga tgttggaaaa 240

actttatcag aaactgaatg gatcggtatg tgtcgtcgtg aagtatttga cgattactta 300

agaaaccgtg cacagaaatt aggtgctaat attgttaacg gtttattcat gcgttcagaa 360

caacaatctg cagagggtcc attcacaatt cactataatt cttatgaaga cggtagtaaa 420

atgggaaaac ctgctacttt agaagttgat atgataattg gtgcagatgg agcaaattct 480

cgtattgcaa aagagataga tgcaggtgaa tacgactacg ctatagcttt tcaagaacgt 540

attcgtattc ctgatgataa aatgaaatat tacgaaaacc ttgctgaaat gtatgtaggt 600

gatgacgtat ctcctgattt ctatggttgg gtttttccta aatatgatca cgttgctgtt 660

ggtacaggta ctgttgtaaa caaaacagct attaaacaat atcaacaggc aacacgtgac 720

agatcaaaag ttaaaacaga aggtggcaaa attatacgtg ttgaagcaca cccaattcca 780

gaacatccac gtccacgtcg ttgtaaaggt cgtgttgcat tagtaggcga cgcagctggt 840

tatgttacaa aatgttctgg cgagggcatt tactttgctg ctaaatctgg tagaatggct 900

gctgaagcta ttgtagaagg ttctgctaac ggtacaaaaa tgtgtggtga ggatgcaatt 960

cgtgtttatt tagataaatg ggatcgtaaa tattggacaa catacaaagt attagacatt 1020

ttacaaaaag tattttatcg tagtaatcca gcacgtgaag catttgttga attatgtgaa 1080

gatagttatg tacagaaaat gacatttgat tcatacttat ataaaactgt tgttccagga 1140

aacccattag acgacgtaaa attacttgtt cgtacagtat cttctatttt acgttcaaat 1200

gctttacgtt ctgttaattc taaatctgta aatgtttctt tcggctctaa agcaaatgag 1260

gaacgtgtta tggctgcagg taccggtgaa aatctttatt ttcaaggttc aggaggtggt 1320

ggttcagatt ataaagatga tgatgacaaa ggaaccggtt aa 1362

<210> 122
<211> 918
<212> DNA
<213> Artificial Sequence

<220>
<223> Description of Artificial Sequence: Synthetic
      polynucleotide

<400> 122
atggtaccag caatggcagt accattagat gtagtaatta catatccttc ttcaggtgct 60

gctgcttatc cagtacttgt tatgtataac ggtttccaag ctaaagctcc atggtatcgt 120

ggtattgtag atcatgtttc tagttggggt tacacagttg ttcaatatac aaatggtggc 180

ttatttccta ttgttgtaga tcgtgttgag ttaacttatt tagagccatt attaacttgg 240

ttagaaacac aaagtgctga tgctaaatct cctttatacg gtcgtgcaga tgtttctcgt 300

ttaggtacaa tgggtcattc acgtggtggt aaattagcag ctttacaatt tgctggacgt 360

acagatgtaa gtggttgtgt attatttgac cctgtagatg gaagtccaat gacaccagaa 420

tctgctgatt atccttcagc tacaaaagca ttagcagcag ctggtcgttc tgctggctta 480

gtaggtgcag ctattacagg ttcatgtaat ccagtaggtc aaaattaccc aaaattctgg 540

ggtgctttag ctcctggttc ttggcaaatg gtattatcac aagctggtca catgcaattt 600

gctcgtactg gtaatccatt cttagattgg tcattagacc gtttatgtgg tcgtggtaca 660

atgatgagtt cagatgttat tacatatagt gcagcattta ctgttgcttg gtttgaaggt 720

atttttcgtc ctgctcaaag tcaaatgggt atttctaatt tcaaaacttg ggctaatact 780

caagttgcag ctcgtagtat cacttttgat attaaaccta tgcaatctcc tcagggtacc 840

ggtgaaaacc tttactttca aggtagtggt ggtggaggaa gtgattataa agatgatgat 900

gacaaaggaa ccggttaa 918

<210> 123
<211> 948
<212> DNA
<213> Artificial Sequence

<220>
<223> Description of Artificial Sequence: Synthetic
      polynucleotide

<400> 123
atggtaccag caccaccaaa accagttcgt ataacttgtc caacagtagc tggcacttat 60

cctgttgttt tattctttca cggtttttat cttcgtaact atttctattc agatgtttta 120

aatcatattg ctagtcatgg ttacatctta gttgcaccac aattatgtaa acttttacct 180

ccaggtggcc aagtagaagt tgatgacgct ggttcagtta ttaactgggc ttcagagaat 240

cttaaagcac accttccaac ttctgttaat gctaatggta aatatacatc tttagttgga 300

cattcacgtg gtggcaaaac agctttcgca gttgcattag gtcacgcagc tacattagat 360

ccatcaatta cattttcagc attaattggt attgatccag tagcaggaac taacaaatac 420

attcgtacag atccacacat cttaacttat aaacctgaat catttgaatt agatattcct 480

gtagctgttg taggcactgg tcttggtcca aaatggaata acgtaatgcc tccatgcgca 540

cctacagatt taaaccacga agaattttac aaagaatgta aagctactaa agctcacttt 600

gttgctgctg attatggtca catggacatg ttagacgacg atcttccagg ttttgtaggc 660

ttcatggctg gttgtatgtg taaaaatggt caacgtaaaa aatcagaaat gcgttctttt 720

gtaggtggta tagttgtagc attcttaaaa tattctttat ggggtgaaaa agctgaaata 780

agattaattg ttaaagatcc tagtgtatct cctgctaaat tagacccatc accagaatta 840

gaagaagcat caggtatttt tgttggtacc ggtgaaaatc tttattttca aggttcaggt 900

ggaggtggtt ctgattataa agatgatgat gacaaaggaa ccggttaa 948

<210> 124
<211> 906
<212> DNA
<213> Artificial Sequence

<220>
<223> Description of Artificial Sequence: Synthetic
      polynucleotide

<400> 124
atggtaccag ctacaccagt tgaagaaggt gattatccag ttgtaatgtt attacatggc 60

taccttttat ataattcatt ttattcacaa ttaatgttac atgtatcatc tcacggtttc 120

atcttaattg ctccacaatt atactcaatt gctggtcctg atactatgga tgaaattaaa 180

agtactgctg agattatgga ctggttatca gttggtttaa atcacttttt accagctcaa 240

gttacaccta atttatctaa atttgcatta tctggtcata gtcgtggtgg taaaactgct 300

tttgctgtag cattaaaaaa atttggttat tcttcaaact taaaaattag tactttaatt 360

ggtattgatc cagtagacgg aacaggtaaa ggtaaacaaa ctccacctcc tgttttagca 420

tatttaccta atagttttga cttagacaaa acaccaattt tagtaattgg ttcaggttta 480

ggtgaaactg cacgtaatcc tttatttcct ccatgtgctc ctccaggtgt taaccaccgt 540

gagtttttcc gtgaatgtca aggtccagca tggcactttg ttgctaaaga ttatggtcat 600

ttagacatgc ttgatgatga tacaaaaggt attcgtggca aatctagtta ctgtttatgc 660

aaaaatggtg aagaacgtcg tccaatgcgt cgtttcgttg gtggtttagt tgttagtttt 720

cttaaagcat atcttgaagg tgatgatcgt gaattagtaa aaatcaaaga tggttgtcat 780

gaagatgtac ctgttgaaat tcaagaattt gaagtaatta tgggtaccgg tgaaaatctt 840

tactttcaag gttcaggcgg tggaggttca gattataaag atgatgatga caaaggaacc 900

ggttaa 906

<210> 125
<211> 1605
<212> DNA
<213> Artificial Sequence

<220>
<223> Description of Artificial Sequence: Synthetic
      polynucleotide

<400> 125
atggtaccaa gtcacaaaaa aaaaaacgta atcttcttcg taactgatgg tatgggtcct 60

gcttctcttt caatggctcg ttcatttaat caacacgtta atgatttacc aattgatgat 120

attttaacat tagatgaaca ttttattgga agttcaagaa cacgttcatc agattcactt 180

gtaactgact cagctgctgg agctacagct tttgcttgtg cacttaaatc atacaatggt 240

gctataggtg tagatccaca ccatcgtcca tgtggaactg ttttagaagc tgctaaatta 300

gcaggttatt taacaggatt agtagttact acacgtatta ctgatgctac accagctagt 360

ttctcaagtc acgtagatta tcgttggcaa gaagatttaa ttgcaacaca ccaattaggt 420

gaatatcctt taggacgtgt tgttgatctt cttatgggtg gtggtcgttc tcacttttat 480

cctcaaggtg aaaaagctag tccatacggt caccacggtg cacgtaaaga tggtcgtgat 540

ttaatcgatg aagctcaaag taatggctgg cagtatgtag gagatcgtaa aaattttgat 600

tctttactta aatcacatgg tgaaaatgtt actttaccat ttttaggttt atttgctgac 660

aacgatatcc catttgaaat tgatcgtgat gaaaaagaat atcctagttt aaaagaacaa 720

gtaaaagtag cattaggtgc tttagaaaaa gcaagtaacg aagataaaga tagtaatggt 780

ttctttttaa tggtagaagg ttctcgtatt gatcatgctg gccatcaaaa cgatcctgca 840

tctcaagtac gtgaagtatt agcatttgat gaggcttttc aatatgtatt agaatttgca 900

gaaaacagtg atacagaaac agtattagta agtacatcag atcatgaaac aggtggttta 960

gttacttcaa gacaagtaac agcatcatac ccacaatatg tatggtatcc tcaagtatta 1020

gctaacgcta cacatagtgg agagtttctt aaacgtaaat tagttgattt cgttcatgaa 1080

cacaaaggcg catcatcaaa aatagaaaac ttcataaaac acgaaattct tgaaaaagat 1140

ttaggtattt atgattatac agattctgac ttagaaacac ttattcattt agatgataac 1200

gctaatgcaa ttcaagataa acttaatgat atggtaagtt ttagagctca aattggttgg 1260

acaacacatg gtcattcagc agttgatgta aacatatatg cttacgcaaa caaaaaagct 1320

acatggtctt atgttcttaa taacttacaa ggtaatcacg aaaacacaga agttggtcaa 1380

ttcttagaga atttcttaga attaaactta aatgaagtta ctgatttaat ccgtgataca 1440

aaacatactt ctgattttga cgcaacagaa atagcaagtg aggttcaaca ctatgatgaa 1500

tattaccacg aattaacaaa tggtaccggt gaaaatcttt attttcaagg ttctggtgga 1560

ggtggcagtg attataaaga tgatgatgac aaaggaaccg gttaa 1605

<210> 126
<211> 1191
<212> DNA
<213> Artificial Sequence

<220>
<223> Description of Artificial Sequence: Synthetic
      polynucleotide

<400> 126
atggtaccac acaagttcac aggtgttaac gctaaattcc agcaaccagc attaagaaat 60

ttatctccag tggtagttga gcgcgaacgt gaggaatttg taggattctt tccacaaatt 120

gttcgtgact taactgaaga tggtattggt catccagaag taggtgacgc tgtagctcgt 180

cttaaagaag tattacaata caacgcacct ggtggtaaat gcaatagagg tttaacagtt 240

gttgcagctt accgtgaact ttctggacca ggtcaaaaag acgctgaaag tcttcgttgt 300

gctttagcag taggatggtg tattgaatta ttccaagcct ttttcttagt ttgggacgat 360

ataatggacc agtcattaac tagacgtggt caattatgtt ggtacaagaa agaaggtgtt 420

ggtttagatg caataaatga ttcttttctt ttagaaagct ctgtgtatcg cgttcttaaa 480

aagtattgcc gtcaacgtcc atattatgta catttattag agctttttct tcaaacagct 540

taccaaacag aattaggaca aatgttagat ttaatcactg ctcctgtatc taaggtagat 600

ttaagccatt tctcagaaga acgttacaaa gctattgtta agtataaaac tgctttctat 660

tcattctatt taccagttgc agcagctatg tatatggttg gtatagattc taaagaagaa 720

catgaaaacg caaaagctat tttacttgag atgggtgaat acttccaaat tcaagatgat 780

tatttagatt gttttggcga tcctgcttta acaggtaaag taggtactga tattcaagat 840

aacaaatgtt catggttagt tgtgcaatgc ttacaaagag taacaccaga acaacgtcaa 900

cttttagaag ataattacgg tcgtaaagaa ccagaaaaag ttgctaaagt taaagaatta 960

tatgaggctg taggtatgag agccgccttt caacaatacg aagaaagtag ttaccgtcgt 1020

cttcaagagt taattgagaa acattctaat cgtttaccaa aagaaatttt cttaggttta 1080

gctcagaaaa tatacaaacg tcaaaaaggt accggtgaaa acttatactt tcaaggctca 1140

ggtggcggtg gaagtgatta caaagatgat gatgataaag gaaccggtta a 1191

<210> 127
<211> 1734
<212> DNA
<213> Artificial Sequence

<220>
<223> Description of Artificial Sequence: Synthetic
      polynucleotide

<400> 127
catatggtac caagacgttc aggtaactat aatcctagcc gttgggacgt aaatttcatt 60

caatctttat tatctgatta taaagaagat aaacacgtta ttagagcttc tgaattagta 120

acacttgtta agatggaatt agaaaaagaa acagatcaaa tccgtcaatt agaattaatt 180

gacgatttac aacgtatggg tttatctgat catttccaaa acgaatttaa agaaatctta 240

tcaagtattt acttagatca tcattattac aaaaatccat ttccaaaaga agagcgtgat 300

ttatactcaa ctagcttagc ttttcgttta ttacgtgaac acggttttca agtagcacaa 360

gaagtttttg attcattcaa aaatgaagag ggtgaattta aggagagctt atctgacgat 420

actcgtggct tattacaatt atatgaagca tcattcttat taacagaggg tgaaacaacc 480

ttagaaagtg cacgcgaatt tgctacaaaa tttttagaag aaaaagttaa cgaaggtggc 540

gttgatggtg acttattaac aagaattgct tactcattag atattccctt acattggcgc 600

attaaacgtc ctaatgcccc agtttggatt gaatggtatc gtaaacgtcc agatatgaac 660

ccagtggttt tagaattagc aattttagac ttaaacattg tacaagctca atttcaagag 720

gaattaaaag agtcttttcg ctggtggcgt aatactggtt ttgttgagaa attaccattt 780

gcacgtgatc gtttagttga atgttacttt tggaacactg gtattattga accacgtcaa 840

cacgcatcag ctcgtattat gatgggtaaa gtaaatgcat taattacagt aattgatgac 900

atctatgatg tttatggaac acttgaagaa ttagaacaat tcactgattt aattcgcaga 960

tgggacataa actcaataga tcaattacca gattatatgc aattatgttt tcttgcatta 1020

aacaatttcg ttgatgacac ttcatacgat gttatgaaag aaaagggtgt taatgttatt 1080

ccttacttac gtcaatcttg ggtagacctt gcagacaaat atatggtaga agcacgttgg 1140

ttctacggtg gccataaacc atcattagaa gaatacttag aaaattcttg gcaatctatc 1200

tcaggtccat gtatgttaac tcatatattc tttcgtgtaa cagatagctt tactaaagaa 1260

actgttgatt ctctttacaa atatcatgat ttagttagat ggtcatcatt cgtgcttcgt 1320

cttgctgacg acttaggtac aagcgttgaa gaagtatctc gtggtgatgt gccaaaatct 1380

ttacaatgct acatgagtga ttataacgct agtgaggctg aagcacgtaa acacgtaaaa 1440

tggttaattg cagaagtatg gaaaaagatg aatgcagaac gtgtttctaa agatagtcct 1500

tttggtaaag attttatagg ttgtgctgtt gatttaggtc gtatggctca attaatgtat 1560

cacaatggag atggtcatgg tactcaacac cctattattc atcaacaaat gacacgtact 1620

ttatttgaac cattcgctgg taccggtgaa aacttatact ttcaaggctc aggtggcggt 1680

ggaagtgatt acaaagatga tgatgataaa ggaaccggtt aatctagact cgag 1734

<210> 128
<211> 1713
<212> DNA
<213> Artificial Sequence

<220>
<223> Description of Artificial Sequence: Synthetic
      polynucleotide

<400> 128
catatggtac caagacgtac tggtggctat caacctacac tttgggattt ttcaacaatt 60

caattatttg atagtgaata taaagaagaa aaacatctta tgcgtgctgc tggtatgatt 120

gctcaagtga acatgttact tcaagaagaa gtagacagca tccaacgtct tgaattaatt 180

gatgacttac gtcgtttagg tatatcttgc cactttgatc gtgaaattgt agagatttta 240

aacagtaaat actacaccaa caatgaaatt gatgaatcag atttatacag tacagcactt 300

agattcaaac ttttacgtca atatgatttt agcgttagcc aagaagtttt tgattgtttt 360

aaaaatgaca aaggtacaga tttcaaacca tcattagttg acgatacacg tggcttatta 420

caattatatg aagcatcatt tttatcagct cagggtgaag aaactttaca tttagcacgt 480

gattttgcta ctaaattctt acataaaaga gttttagtag ataaagatat caatttatta 540

tctagtatcg agcgtgcttt agaattacca acacactggc gtgtacaaat gcctaacgct 600

agatcattca tcgacgcata taaaagaaga ccagacatga accctacagt attagagtta 660

gcaaaacttg actttaacat ggttcaagca cagttccaac aagaattaaa agaagccagt 720

cgctggtgga actctacagg attagtacat gaattaccat ttgtacgtga tcgtattgtg 780

gaatgttatt attggactac tggtgtagta gaacgtcgtg aacacggtta cgaacgtatt 840

atgttaacaa aaattaacgc tttagttaca acaatcgatg atgtttttga catttatggt 900

actttagaag aattacaact ttttacaact gctattcaaa gatgggacat tgagtctatg 960

aaacaacttc caccctatat gcaaatctgc tacttagctt tattcaactt cgtaaatgag 1020

atggcttacg atacattacg tgataaaggt tttaatagta ctccatattt acgcaaagcc 1080

tgggtagact tagtagaaag ctacttaatt gaagctaaat ggtattatat gggtcacaaa 1140

ccaagtttag aagagtacat gaaaaactca tggatttcta tcggaggtat tccaatttta 1200

tcacatttat tctttcgttt aacagacagt atcgaagaag aagacgctga atcaatgcat 1260

aaatatcacg atatagtacg tgcctcttgt actattttac gtttagctga tgatatgggt 1320

acatcattag atgaagttga acgtggcgat gttcctaaat ctgtacaatg ctatatgaat 1380

gagaaaaacg cctctgaaga agaagcacgt gaacatgttc gtagtttaat tgatcagaca 1440

tggaagatga tgaataaaga aatgatgact tcatcatttt caaaatactt cgtacaagtg 1500

tctgcaaatc ttgctcgtat ggcacaatgg atatatcaac atgaaagtga tggtttcggt 1560

atgcaacact ctttagttaa caaaatgctt cgtggtttac tttttgaccg ttatgaaggt 1620

accggtgaaa acttatactt tcaaggctca ggtggcggtg gaagtgatta caaagatgat 1680

gatgataaag gaaccggtta atctagactc gag 1713

<210> 129
<211> 1803
<212> DNA
<213> Artificial Sequence

<220>
<223> Description of Artificial Sequence: Synthetic
      polynucleotide

<400> 129
catatggtac cacgtcgcat gggtgatttt cattcaaact tatgggatga tgatgtaatt 60

caatctttac ccacagctta cgaagaaaaa tcttatcttg aacgtgctga gaagttaatt 120

ggagaagttg aaaatatgtt caacagtatg agtttagaag atggtgaact tatgagtcca 180

ttaaatgatt taattcaacg cctttggatt gttgattctt taggtagatt aggtatccat 240

cgtcacttta aagatgagat taaaagtgct ttagattatg tttacagtta ctggggtgaa 300

aacggaatag gttgtggtcg tgaaagtgct gtaactgatt taaacagtac agctttaggc 360

tttcgtacac ttcgtttaca cggttatcca gtttcatctg atgtatttaa agcatttaaa 420

ggtcaaaatg gtcaattcag ttgttcagaa aatatccaaa cagatgaaga aattcgtggt 480

gttcttaact tatttagagc cagtttaata gccttccctg gtgagaaaat aatggacgaa 540

gctgaaatct tctctacaaa atacttaaag gaagcattac aaaagatccc agttagttca 600

ttatcacgtg aaatcggtga tgtacttgaa tatggatggc atacatactt accacgttta 660

gaagcacgta actatattca tgttttcgga caagatacag agaatacaaa aagttatgta 720

aaatcaaaga aacttttaga attagctaaa ttagaattta acatttttca gagcttacaa 780

aaacgtgaat tagaaagcct tgttcgttgg tggaaagaat ctggatttcc tgaaatgaca 840

ttctgtagac acagacacgt ggaatattac acacttgcat catgtattgc attcgaacct 900

cagcatagtg gttttcgttt aggttttgct aaaacatgtc accttataac agttttagat 960

gacatgtatg acactttcgg caccgtagac gaattagagt tatttacagc aactatgaaa 1020

cgttgggacc caagttcaat tgactgcctt ccagaataca tgaaaggagt ttacattgct 1080

gtgtatgata cagttaatga aatggctcgt gaagctgagg aagctcaagg tcgcgataca    1140

cttacatacg ctcgtgaggc ctgggaggct tatatagatt cttatatgca agaagctcgc    1200

tggattgcta ctggatactt accttctttc gatgaatatt atgaaaatgg taaggtttca    1260

tgtggtcacc gtatatctgc tttacaacca attcttacta tggatattcc atttccagat    1320

cacattttaa aggaagttga ctttccttct aaacttaatg acttagcttg tgctatctta    1380

cgccttcgcg gtgatactcg ttgttacaaa gcagaccgtg cacgtggtga agaggctagt    1440

tctatttctt gttatatgaa agataatcca ggtgtttctg aagaagatgc cttagatcat    1500

attaacgcaa tgatcagtga tgttattaag ggcttaaact gggaattact taaacccgac    1560

attaacgtac ctatttctgc taagaaacat gctttcgaca ttgctcgtgc ttttcactac    1620

ggttataaat atcgtgatgg ctattcagtt gctaatgttg aaacaaaatc tttagttaca    1680

cgtactttac ttgaatcagt tccattaggt accggtgaaa acttatactt tcaaggctca    1740

ggtggcggtg gaagtgatta caaagatgat gatgataaag gaaccggtta atctagactc    1800

gag                                                                  1803

<210> 130
<211> 1788
<212> DNA
<213> Artificial Sequence

<220>
<223> Description of Artificial Sequence: Synthetic
      polynucleotide

<400> 130
catatggtac cacgtagagt tggtaattat cattctaatc tttgggatga tgattttata      60

caaagtttaa tttctacacc ttacggtgct cctgactacc gtgaacgcgc tgatcgtctt     120

attggtgaag taaaagatat tatgtttaat ttcaaatctt tagaggatgg tggtaatgac     180

ttattacaac gtttactttt agttgatgac gtagaacgtt taggcattga tcgtcatttc     240

aaaaaggaaa ttaagactgc attagattat gtaaatagtt attggaatga aaaaggaatt     300

ggttgtggtc gtgagtctgt agttacagac ttaaattcaa ctgctttagg ccttcgtacc     360

ttaagattac atggttatac tgttagctct gacgttttaa atgtttttaa agataaaaat     420

ggtcaatttt catctacagc taatattcaa attgaaggtg aaattcgtgg tgttttaaat     480

ctttttcgtg cctctcttgt agcttttcca ggtgagaaag tgatggatga ggctgaaact     540

ttttcaacaa aatatcttcg tgaagcatta cagaaaattc ctgccagttc aattttatca     600

ttagaaatac gtgatgtatt agaatatgga tggcatacta atttaccacg tttagaagca     660

cgtaattaca tggatgtttt cggtcagcac accaagaaca aaaatgcagc cgaaaaatta     720

cttgaattag caaaattaga gttcaatatc tttcacagct tacaagaacg tgaattaaag     780

cacgtttcaa gatggtggaa agactctggt agtccagaga tgactttctg tcgccaccgc 840

catgtggaat attatgcttt agcttcttgt attgctttcg aaccccagca cagtggtttc 900

cgtttaggtt ttactaaaat gagtcattta atcacagtgt tagatgatat gtatgatgta 960

ttcggtacag ttgatgaatt agagttattt accgccacta ttaaacgttg ggacccttct 1020

gctatggaat gtttaccaga gtacatgaaa ggtgtttaca tgatggttta tcatacagtt 1080

aacgaaatgg ctcgtgtggc agaaaaggct caaggtagag acacattaaa ctatgctcgt 1140

caagcctggg aagcatgttt tgactcttat atgcaagaag caaaatggat tgcaacaggt 1200

tacttaccta cattcgagga atatttagaa aatggtaaag tgagttcagc acatcgtcct 1260

tgtgcattac aacctatttt aactcttgat attccatttc ccgatcatat tcttaaagaa 1320

gtggatttcc caagcaaact taatgactta atttgtatta tcttacgtct tagaggagac 1380

acacgttgct ataaagcaga ccgtgcccgt ggtgaagaag catcatcaat atcttgttat 1440

atgaaagata acccaggttt aactgaagaa gatgctttaa accacattaa ctttatgatt 1500

cgtgacgcaa tccgcgaatt aaactgggag ttacttaaac cagataatag tgttccaatt 1560

acttcaaaga aacatgcttt tgatatttca cgtgtgtggc accacggata ccgttatcgt 1620

gatggttaca gctttgcaaa cgtggaaact aaaagtcttg taatgcgtac tgtaatagaa 1680

ccagtaccat taggtaccgg tgaaaactta tactttcaag gctcaggtgg cggtggaagt 1740

gattacaaag atgatgatga taaaggaacc ggttaatcta gactcgag 1788

<210> 131
<211> 1725
<212> DNA
<213> Artificial Sequence

<220>
<223> Description of Artificial Sequence: Synthetic
      polynucleotide

<400> 131
catatggtac cacgtcgttc aggagattat caaccaagtt tatgggactt taattacatt 60

caatctttaa acacacctta caaagaacaa cgtcatttta atcgtcaagc tgagttaatt 120

atgcaagttc gtatgttatt aaaggtaaaa atggaagcaa ttcaacaatt agagttaata 180

gatgatttac agtacttagg attatcatat ttctttcaag acgaaattaa acaaatctta 240

agctctattc acaatgaacc tcgttatttt cataataatg acctttattt cactgcttta 300

ggttttagaa ttttacgtca acatggtttt aatgtttcag aagacgtatt tgactgcttt 360

aaaatcgaaa aatgttctga ctttaatgct aacttagctc aggacacaaa gggtatgtta 420

caattatatg aagctagttt cttattaaga gaaggagaag atacacttga attagctcgt 480

cgttttagta cacgttcttt acgtgaaaaa tttgatgaag gtggtgacga gatagatgaa 540

gatttaagta gttggattcg tcattcttta gatttaccat tacactggcg tgttcaaggt 600

ttagaagctc gttggttttt agatgcctat gctcgtcgtc cagatatgaa ccctcttatt 660

ttcaaattag ctaaattaaa ttttaacatt gttcaagcta cataccaaga agaattaaaa 720

gacatctctc gttggtggaa cagtagttgt ttagcagaga aattaccctt cgttcgcgat 780

cgtattgtag aatgtttctt ctgggctatt gctgctttcg aaccacacca atactcatat 840

caacgtaaaa tggccgctgt aattattaca tttattacta ttattgatga tgtttacgat 900

gtatatggta ctattgaaga attagagtta ttaacagata tgattcgtag atgggataat 960

aagagtatta gtcaacttcc ttactatatg caagtttgtt atttagctct ttataacttc 1020

gtaagtgaac gcgcatacga catcttaaaa gatcaacact ttaacagtat tccatacctt 1080

caaagaagtt gggtttcatt agttgaggga tacttaaaag aagcatattg gtactataac 1140

ggttacaaac caagtcttga agaatatctt aataatgcaa aaattagtat tagtgcaccc 1200

accattattt cacaattata ctttacttta gcaaatagta tcgacgaaac tgccattgaa 1260

agtttatacc aatatcacaa cattttatac ttatcaggta ctatcttacg tttagctgat 1320

gatttaggaa cttcacaaca tgaattagaa cgtggtgatg ttcccaaagc tattcaatgt 1380

tatatgaatg atacaaatgc atcagaaaga gaagctgtag aacatgttaa atttcttatt 1440

cgtgaagcct ggaaagaaat gaatacagtt actaccgcat cagattgtcc ttttacagac 1500

gatcttgttg ccgccgcagc taatttagct cgtgctgctc aattcattta cttagatggt 1560

gatggtcatg gtgtacaaca tagcgaaatt catcagcaaa tgggcggtct tctttttcaa 1620

ccatacgttg gtaccggtga aaacttatac tttcaaggct caggtggcgg tggaagtgat 1680

tacaaagatg atgatgataa aggaaccggt taatctagac tcgag 1725

<210> 132
<211> 1800
<212> DNA
<213> Artificial Sequence

<220>
<223> Description of Artificial Sequence: Synthetic
      polynucleotide

<400> 132
catatggtac cacgcagaat tggtgattac catagtaaca tttgggatga tgattttatc 60

cagtcacttt ctacccctta tggtgaacca tcttaccaag aaagagctga acgtcttatt 120

gtagaagtga aaaagatttt caacagtatg tacttagatg acggtcgttt aatgagttct 180

tttaatgact taatgcaacg tttatggatt gtagactcag tagaacgttt aggtattgcc 240

cgtcacttca aaaatgaaat tacatctgcc cttgactatg tttttcgtta ttgggaagaa 300

aacggtatag gttgtggtcg tgattctatt gtaactgact taaatagcac agctttaggt 360

tttcgtacac ttcgtttaca cggttataca gtttctccag aggttttaaa agcatttcaa 420

gatcaaaatg gtcaattcgt ttgttcacca ggacaaacag aaggtgaaat tcgttcagtt 480

ttaaatttat atcgtgcaag tttaattgcc tttccaggcg aaaaagttat ggaagaagca 540

gaaatcttct ctactcgcta tttaaaagaa gctcttcaaa agattccagt tagcgcatta 600

tcacaagaaa tcaaatttgt tatggaatat ggatggcata caaatttacc tagattagaa 660

gcacgtaact atattgatac tttagaaaag gatacatcag cttggttaaa caaaaatgca 720

ggtaaaaagt tattagaatt agctaaatta gaatttaaca tctttaactc attacaacaa 780

aaagaattac aatacttact tcgctggtgg aaagaatctg acttacctaa attaaccttt 840

gcacgtcata gacacgttga attttacaca ttagcttctt gtattgctat tgatcccaaa 900

cattcagcat tccgtttagg attcgctaaa atgtgtcact tagttacagt tcttgacgat 960

atttatgata ctttcggtac tattgatgaa cttgagttat ttacttctgc aattaaacgt 1020

tggaatagtt ctgaaattga acacttacca gaatatatga aatgcgtgta tatggttgtt 1080

tttgaaactg ttaatgaatt aactcgtgaa gctgagaaaa cacaaggacg taacacttta 1140

aactatgttc gtaaagcatg ggaagcatat tttgattctt atatggagga agcaaagtgg 1200

atctcaaacg gatatttacc aatgtttgaa gaataccacg aaaatggtaa agtgtcatct 1260

gcataccgtg tagcaacatt acaaccaatt ttaactttaa acgcttggtt acccgactac 1320

attcttaaag gaattgattt cccaagtcgt tttaacgatt tagctagttc attcttacgt 1380

ttacgtggcg atactcgctg ttacaaagct gaccgtgatc gtggtgaaga agctagctgc 1440

atttcttgtt acatgaaaga taatccaggt tctaccgaag aagatgcact taatcacatt 1500

aacgctatgg taaatgacat cattaaagaa ttaaactggg aattattacg cagtaatgat 1560

aatattccta tgttagctaa aaagcacgct tttgatatta ctcgtgcact tcaccactta 1620

tacatttatc gcgatggttt cagtgttgct aataaagaaa ctaaaaagtt agttatggag 1680

acattacttg aatcaatgtt atttggtacc ggtgaaaact tatactttca aggctcaggt 1740

ggcggtggaa gtgattacaa agatgatgat gataaaggaa ccggttaatc tagactcgag 1800

<210> 133
<211> 2502
<212> DNA
<213> Artificial Sequence

<220>
<223> Description of Artificial Sequence: Synthetic
      polynucleotide

<400> 133
catatggtac cacaatctgc tgaaaagaac gactctttat caagttctac attagttaag 60

agagaatttc cacccggttt ctggaaagac gacttaatcg acagtttaac ttcaagtcac 120

aaagtagctg ctagcgatga aaaacgtatc gaaaccttaa tttcagaaat taagaatatg 180

tttcgttgta tgggttatgg tgagacaaat ccatcagctt atgatactgc ttgggtagct 240

cgcatcccag cagttgatgg atcagataat cctcactttc cagagactgt ggaatggatc 300

ttacaaaatc aattaaaaga tggttcttgg ggtgaaggtt tttacttcct tgcttatgat 360

cgcattttag ccactttagc ttgtattatc acacttacac tttggcgtac tggagaaaca 420

caagtacaga aaggtatcga atttttccgc actcaagcag gtaaaatgga agatgaagca 480

gattcacacc gtccaagtgg ttttgagatt gtatttcctg ctatgttaaa agaggctaag 540

attttaggct tagatttacc ttatgatctt ccttttctta aacaaattat tgaaaagaga 600

gaagctaagt taaaacgtat tcctacagat gttttatatg ctttaccaac tactttactt 660

tattcattag aaggtttaca agaaatagta gactggcaaa aaatcatgaa attacaaagt 720

aaagatggta gtttcttatc ttctcctgcc tcaacagcag cagtatttat gagaacaggt 780

aacaaaaagt gtttagattt cttaaatttc gtgcttaaaa agttcggtaa tcatgttcca 840

tgccactatc ctttagacct ttttgagcgt ctttgggcag ttgatactgt tgaaagatta 900

ggtattgacc gtcattttaa agaagaaata aaagaggctt tagactatgt gtattcacac 960

tgggacgaac gtggtattgg ttgggctcgt gaaaaccccg ttccagatat tgacgataca 1020

gcaatgggtc ttcgtatttt acgtcttcat ggttacaatg ttagcagcga tgttcttaaa 1080

acatttcgtg atgaaaatgg tgagttcttt tgctttttag gacaaacaca aagaggtgtg 1140

actgatatgt taaatgttaa tcgttgtagc catgtatctt tccctggtga aactataatg 1200

gaagaggcaa aattatgtac tgaacgttac ttacgcaacg cattagaaaa tgtagacgct 1260

tttgataagt gggcatttaa gaaaaacatt cgtggtgagg tagaatatgc tcttaaatat 1320

ccttggcata aatcaatgcc acgtttagaa gcacgttcat atattgaaaa ttacggtcca 1380

gatgatgttt ggttaggtaa aactgtttat atgatgcctt acatttcaaa tgaaaagtac 1440

ttagagttag ctaaacttga ttttaacaaa gttcagtcaa tccaccagac agaacttcaa 1500

gacttacgcc gttggtggaa aagttctggt tttacagatt taaactttac aagagaacgt 1560

gttactgaaa tttacttttc acctgcatct tttatcttcg aaccagaatt tagtaaatgt 1620

cgtgaggttt atacaaaaac ttctaatttt actgtaattt tagacgattt atatgacgct 1680

catggctctt tagatgactt aaaacttttt acagagagtg ttaaacgttg ggatttatct 1740

ttagttgacc aaatgcccca gcagatgaaa atctgttttg taggtttcta taatacattc 1800

aacgatattg ctaaagaagg tagagaacgt caaggtcgtg atgttttagg ttatattcaa 1860

aacgtatgga aagtacaact tgaagcatat actaaagaag cagaatggtc agaagcaaaa 1920

tatgttccta gttttaacga atacattgaa aatgcttcag tttcaattgc cttaggtaca 1980

gtagtactta tcagtgcttt atttaccgga gaagttttaa cagatgaagt tttatctaaa 2040

attgaccgtg aaagtagatt cttacagtta atgggcttaa ctggacgttt agtaaatgat 2100

actaaaacat atcaagctga gcgtggtcaa ggtgaagttg ctagtgcaat tcaatgttat 2160

atgaaagacc accctaaaat tagtgaagaa gaagcattac aacatgtata ttctgtaatg 2220

gaaaatgcat tagaagaatt aaatcgtgag ttcgttaaca acaaaattcc agacatctat 2280

aaacgtcttg ttttcgaaac tgcacgtata atgcaattat tttacatgca aggtgatggt 2340

ttaacattaa gtcacgatat ggaaattaaa gagcacgtaa agaattgttt attccagcca 2400

gtagctggta ccggtgaaaa cttatacttt caaggctcag gtggcggtgg aagtgattac 2460

aaagatgatg atgataaagg aaccggttaa tctagactcg ag 2502

<210> 134
<211> 2511
<212> DNA
<213> Artificial Sequence

<220>
<223> Description of Artificial Sequence: Synthetic
      polynucleotide

<400> 134
catatggtac catcttcatc aacaggcact tcaaaagtag taagcgaaac atcttcaact 60

attgtagacg atattccacg tctttcagca aattatcatg gtgatttatg gcatcacaac 120

gtaattcaga ctttagaaac accatttaga gaaagttcaa cttatcaaga gcgtgcagat 180

gaattagtag tgaaaatcaa agatatgttc aatgcattag gtgacggtga catctcacct 240

tcagcttatg atactgcatg ggtagctcgt gttgctacca tttcttctga tggtagcgaa 300

aaaccacgtt ttcctcaagc tcttaattgg gtttttaaca atcaattaca agatggatca 360

tggggtattg aatcacattt tagtttatgc gatcgtttac ttaatactac aaattcagtt 420

attgctttat cagtatggaa aactggtcac tcacaggttc aacaaggtgc cgaatttatt 480

gctgaaaatt tacgtctttt aaatgaagaa gacgaattaa gtcctgattt tcaaattatc 540

ttcccagctt tattacagaa agccaaggct ttaggaatca atttacccta tgatttacca 600

ttcatcaaat atcttagtac aacacgcgaa gctcgtttaa cagatgtgtc agctgctgct 660

gacaacatac cagccaatat gcttaatgca cttgaaggtt tagaagaagt gattgattgg 720

aataaaatca tgcgttttca atctaaagat ggttcatttt tatcttctcc agctagtaca 780

gcctgtgttt taatgaatac aggtgatgaa aaatgtttca cattcttaaa taacttatta 840

gataaattcg gcggttgtgt tccatgtatg tatagcattg atttattaga acgtttatct 900

ttagtggaca acattgaaca cttaggtatt ggtcgtcact ttaaacaaga aatcaaaggt 960

gcattagatt atgtatatcg tcattggtct gaacgcggta tcggttgggg tagagactct 1020

ttagttccag atttaaacac cacagcttta ggtttacgca cattaagaat gcacggttat 1080

aacgtgtcta gtgatgtact taacaatttc aaagacgaaa atggtcgttt ctttagtagt 1140

gctggtcaaa cacacgtaga gttacgttct gttgtaaatc tttttcgcgc ctcagattta 1200

gcctttccag acgaacgtgc aatggatgat gctcgtaaat tcgcagaacc atatttacgt 1260

gaagcattag ctacaaaaat atcaacaaat acaaagttat tcaaagaaat tgaatatgtt 1320

gttgaatacc cttggcacat gtcaattcca cgtttagaag ctcgtagtta tattgacagt 1380

tatgatgata attatgtatg gcaacgtaag actttatatc gtatgccatc attaagtaat 1440

tcaaaatgtt tagaacttgc taaattagat ttcaatattg ttcaatcttt acaccaagaa 1500

gaacttaaac ttttaactcg ttggtggaaa gaatctggta tggcagacat aaatttcacc 1560

cgccatcgtg tagctgaagt ttacttttct agtgctacat ttgagccaga atatagtgct 1620

actcgtattg cattcacaaa aattggttgc ttacaagtac ttttcgatga tatggctgac 1680

attttcgcca ctttagatga gttaaaaagt tttactgaag gtgttaaacg ctgggacaca 1740

tcattattac atgaaattcc cgaatgtatg caaacttgtt ttaaagtatg gtttaaactt 1800

atggaagaag taaacaacga cgtagtaaaa gttcaaggaa gagatatgtt agcacatatt 1860

cgtaaaccct gggaattata ctttaattgt tatgttcaag aacgtgaatg gttagaagct 1920

ggttatattc ctacattcga agaatatctt aaaacttatg ctattagtgt aggccttggt 1980

ccttgtacct tacaacctat tcttttaatg ggtgagttag ttaaagatga tgtagtagaa 2040

aaagttcatt acccttctaa catgttcgaa ttagtttctt taagctggcg tttaactaat 2100

gataccaaaa catatcaagc agaaaaagta cgcggtcaac aagctagtgg cattgcctgt 2160

tatatgaaag acaatccagg tgctactgaa gaagatgcta ttaaacacat ttgtcgtgtt 2220

gttgatcgtg cattaaaaga agcaagtttc gaatatttca agccttcaaa tgacattcct 2280

atgggttgta aatcttttat ctttaactta cgtttatgtg tacaaatttt ctataaattc 2340

attgatggtt atggtatcgc aaacgaagaa attaaggact acattcgtaa ggtttatatt 2400

gatccaattc aagttggtac cggtgaaaac ttatactttc aaggctcagg tggcggtgga 2460

agtgattaca aagatgatga tgataaagga accggttaat ctagactcga g 2511

<210> 135
<211> 1206
<212> DNA
<213> Artificial Sequence

<220>
<223> Description of Artificial Sequence: Synthetic
      polynucleotide

<400> 135
catatggtac cacacaagtt cacaggtgtt aacgctaaat tccagcaacc agcattaaga 60

aatttatctc cagtggtagt tgagcgcgaa cgtgaggaat ttgtaggatt ctttccacaa 120

attgttcgtg acttaactga agatggtatt ggtcatccag aagtaggtga cgctgtagct 180

cgtcttaaag aagtattaca atacaacgca cctggtggta aatgcaatag aggtttaaca 240

gttgttgcag cttaccgtga actttctgga ccaggtcaaa aagacgctga aagtcttcgt 300

tgtgctttag cagtaggatg gtgtattgaa ttattccaag cctttttctt agttgctgac 360

gatataatgg accagtcatt aactagacgt ggtcaattat gttggtacaa gaaagaaggt 420

gttggtttag atgcaataaa tgattctttt cttttagaaa gctctgtgta tcgcgttctt 480

aaaaagtatt gccgtcaacg tccatattat gtacatttat tagagctttt tcttcaaaca 540

gcttaccaaa cagaattagg acaaatgtta gatttaatca ctgctcctgt atctaaggta 600

gatttaagcc atttctcaga agaacgttac aaagctattg ttaagtataa aactgctttc 660

tattcattct atttaccagt tgcagcagct atgtatatgg ttggtataga ttctaaagaa 720

gaacatgaaa acgcaaaagc tattttactt gagatgggtg aatacttcca aattcaagat 780

gattatttag attgttttgg cgatcctgct ttaacaggta aagtaggtac tgatattcaa 840

gataacaaat gttcatggtt agttgtgcaa tgcttacaaa gagtaacacc agaacaacgt 900

caacttttag aagataatta cggtcgtaaa gaaccagaaa aagttgctaa agttaaagaa 960

ttatatgagg ctgtaggtat gagagccgcc tttcaacaat acgaagaaag tagttaccgt 1020

cgtcttcaag agttaattga gaaacattct aatcgtttac caaaagaaat tttcttaggt 1080

ttagctcaga aaatatacaa acgtcaaaaa ggtaccggtg aaaacttata ctttcaaggc 1140

tcaggtggcg gtggaagtga ttacaaagat gatgatgata aaggaaccgg ttaatctaga 1200

ctcgag 1206

<210> 136
<211> 1743
<212> DNA
<213> Artificial Sequence

<220>
<223> Description of Artificial Sequence: Synthetic
      polynucleotide

<400> 136
catatggtac catcattaac tgaagaaaaa ccaattcgcc caatcgcaaa ctttcctcca 60

agcatttggg gagatcaatt cttaatttac gaaaaacaag tagaacaagg tgttgaacag 120

attgttaacg accttaagaa agaagtgcgc caacttttaa aagaggcttt agatattcca 180

atgaaacacg caaacctttt aaaacttatt gacgaaattc aacgtcttgg tattccatat 240

cactttgaac gtgaaattga tcatgcatta caatgtatct atgaaactta tggtgataat 300

tggaatggtg atcgttcttc attatggttc cgtttaatgc gtaaacaagg ttattatgtt 360

acatgtgacg tgtttaacaa ttacaaagat aaaaatggtg catttaaaca atctttagct 420

aatgatgttg aaggtttatt agaattatat gaagctactt caatgcgtgt tccaggtgaa 480

attattcttg aagatgcatt aggttttaca cgttctcgtt tatctattat gacaaaagac 540

gcatttagta caaatcctgc tttatttact gaaattcagc gtgcccttaa acagccttta 600

tggaaacgtt taccaagaat tgaagctgct caatatattc cattttatca acaacaagat 660

tctcacaata agacattact taaattagcc aaattagaat ttaatctttt acaatcatta 720

cataaagaag aattaagtca tgtgtgtaaa tggtggaaag catttgatat taagaagaat 780

gctccatgtt tacgtgatag aattgtagag tgttactttt ggggccttgg tagtggttac 840

gagccacaat attcacgtgc tcgtgtattc tttacaaaag ctgttgcagt tattacttta 900

attgacgata cctatgatgc atacggaacc tatgaggagc ttaaaatttt cactgaagct 960

gtagaacgtt ggtctataac ttgtttagat actttaccag aatatatgaa acccatctac 1020

aaattattca tggacacata cactgaaatg gaagaatttt tagcaaaaga aggtcgcaca 1080

gaccttttta actgtggtaa agaatttgtt aaagagtttg ttcgtaactt aatggtagaa 1140

gctaagtggg ctaatgaagg tcacattcct actacagaag agcacgatcc agtagtaata 1200

attacaggtg gagcaaactt acttaccaca acttgttact taggtatgtc tgacattttt 1260

acaaaagaat cagtagagtg ggcagtatct gcaccaccat tattccgtta ttctggcata 1320

cttggtcgtc gtcttaatga tttaatgact cataaagctg aacaagagcg taaacactca 1380

tcaagtagtt tagaaagcta tatgaaggaa tataacgtta acgaagagta tgctcaaaca 1440

cttatttaca aagaggttga agacgtttgg aaggacatta accgtgaata cttaacaact 1500

aaaaacattc cacgtcctct tttaatggct gtaatatact tatgtcaatt cttagaagta 1560

caatacgctg gaaaagataa ctttacacgt atgggtgatg aatataaaca cttaataaag 1620

agtttattag tttatcctat gtcaataggt accggtgaaa acttatactt tcaaggctca 1680

ggtggcggtg gaagtgatta caaagatgat gatgataaag gaaccggtta atctagactc 1740

gag 1743

<210> 137
<211> 2556
<212> DNA
<213> Artificial Sequence

<220>
<223> Description of Artificial Sequence: Synthetic
      polynucleotide

<400> 137
catatggtac cagcaggtgt atcagctgtg tcaaaagttt cttcattagt atgtgactta 60

agtagtacta gcggcttaat tcgtagaact gcaaatcctc accctaatgt atggggttat 120

gacttagttc attctttaaa atctccatat attgatagta gctatcgtga acgtgctgaa 180

gtgcttgtaa gtgaaataaa agctatgtta aatccagcaa ttactggaga tggtgaatca 240

atgattacac cttcagctta tgacactgct tgggttgcac gtgtaccagc aattgatggt 300

agcgcacgtc cacaatttcc acaaacagta gattggattt taaagaatca attaaaagat 360

ggttcttggg gtattcaatc acacttttta ctttcagacc gtttattagc tactcttagc 420

tgtgttttag ttttacttaa atggaatgtt ggtgatttac aggttgagca aggtattgag 480

tttattaagt caaaccttga attagtaaaa gatgaaactg atcaagattc tttagtgact 540

gattttgaga ttattttccc tagcttactt cgtgaggccc aaagtttacg tttaggtctt 600

ccatacgatt taccttacat ccacttatta caaacaaaac gtcaggaacg tttagcaaaa 660

ttaagccgtg aagaaatata tgcagttcca agtccacttt tatattcttt agagggtatt 720

caagatattg ttgagtggga acgtattatg gaagtacaat ctcaggatgg atcattttta 780

agttctccag catcaaccgc atgtgttttt atgcatacag gtgacgctaa gtgtttagaa 840

tttcttaaca gtgtaatgat taagtttggt aattttgtac catgccttta tcctgtagat 900

ttattagaac gtttacttat agtagataat atagttcgtc ttggtattta ccgtcacttc 960

gaaaaagaaa ttaaagaagc attagattat gtatatcgcc attggaatga acgtggtatt 1020

ggttggggtc gtttaaatcc aattgctgac ttagaaacaa ctgctttagg ttttcgttta 1080

ttacgtttac accgttataa tgtatctcca gcaatctttg ataatttcaa agatgccaat 1140

ggcaaattca tttgtagcac tggtcagttt aataaggatg tggcttcaat gttaaactta 1200

taccgtgcat cacaattagc attcccaggc gaaaacattt tagatgaagc taaatctttt 1260

gccaccaaat acttacgtga agcccttgaa aaatctgaaa cttcatcagc ttggaacaat 1320

aaacagaatt taagtcaaga aatcaagtat gcattaaaaa cttcatggca cgcttctgta 1380

ccacgtgttg aagcaaaacg ttattgtcaa gtttatcgtc ctgattacgc tcgtattgct 1440

aagtgtgtat acaaattacc atacgttaac aacgaaaaat tcttagaatt aggtaaatta 1500

gattttaaca tcattcaatc aattcatcaa gaagaaatga aaaatgtgac aagttggttt 1560

cgtgattctg gcttaccatt atttactttc gctcgcgaac gtcctttaga attttacttc 1620

ttagttgctg ctggtactta tgaacctcaa tatgctaaat gtcgtttctt attcacaaaa 1680

gtagcttgtc ttcaaacagt attagacgat atgtacgata cttacggtac tttagacgaa 1740

ttaaaacttt ttaccgaggc tgtgcgtcgt tgggatttat cttttacaga aaatttacct 1800

gactatatga aattatgtta tcaaatctat tatgacatcg ttcatgaagt ggcttgggaa 1860

gctgaaaaag aacaaggtag agaattagtg tcattcttcc gtaaaggctg ggaagactac 1920

ttattaggtt actatgaaga agcagaatgg ttagcagcag aatacgttcc aacattagat 1980

gaatacatta aaaacggtat tacatcaatc ggccaacgta tcttattact ttcaggtgtg 2040

ttaattatgg atggccaact tttatcacaa gaagcattag aaaaagttga ttaccctggt 2100

cgtcgtgttt taactgagtt aaactcactt attagccgtt tagctgacga cactaaaact 2160

tataaagcag aaaaagctcg tggagaatta gcctcatcaa ttgaatgcta catgaaagat 2220

catcctgaat gtacagaaga agaagcctta gaccacattt attctattct tgaaccagcc 2280

gtaaaagaat taactcgtga atttcttaaa ccagacgacg ttccatttgc ttgtaaaaag 2340

atgttattcg aagaaactcg tgttacaatg gtgatcttta aagatggtga tggttttggt 2400

gtatctaagt tagaagttaa agatcacatc aaagaatgct taattgaacc attaccatta 2460

ggtaccggtg aaaacttata ctttcaaggc tcaggtggcg gtggaagtga ttacaaagat 2520

gatgatgata aaggaaccgg ttaatctaga ctcgag 2556

<210> 138
<211> 948
<212> DNA
<213> Artificial Sequence

<220>
<223> Description of Artificial Sequence: Synthetic
      polynucleotide

<400> 138
catatggtac caactatgat gaatatgaat tttaagtact gtcacaagat tatgaagaaa 60

cattcaaaat cattcagtta tgcttttgac ttattaccag aagaccaacg taaagctgtt 120

tgggcaattt acgccgtgtg ccgcaaaatt gatgattcta ttgatgtata tggtgatatt 180

caattcttaa atcagattaa agaagacata caaagtattg aaaaatatcc atacgaacat 240

catcattttc aatctgacag acgtattatg atggccttac agcatgttgc tcagcataaa 300

aacattgcat ttcaatcatt ctacaattta attgacacag tatataaaga tcaacacttt 360

acaatgtttg aaacagatgc tgaacttttt ggttattgtt acggtgtagc tggtactgtg 420

ggtgaagttt taactcctat attatctgat cacgaaacac atcaaactta tgacgttgcc 480

cgtcgtttag gagagtcatt acagttaatc aatattctta gagatgtagg tgaagacttt 540

gacaacgaac gtatttactt ctctaaacaa cgtttaaaac aatacgaagt agatattgca 600

gaagtgtacc aaaatggtgt aaacaatcac tatattgatt tatgggaata ttacgctgca 660

attgctgaaa aggattttca agatgttatg gaccaaatta aagttttctc tattgaagct 720

cagccaatta ttgagttagc tgcacgtatt tatatcgaaa ttttagatga agtacgtcaa 780

gctaactaca cattacatga acgtgttttt gtagataaac gtaaaaaggc taaacttttt 840

cacgaaaata aaggtaccgg tgaaaactta tactttcaag gctcaggtgg cggtggaagt 900

gattacaaag atgatgatga taaaggaacc ggttaatcta gactcgag 948

<210> 139

<211> 1611
<212> DNA
<213> Artificial Sequence

<220>
<223> Description of Artificial Sequence: Synthetic
      polynucleotide

<400> 139
catatggtac caaaaattgc tgttattggt gctggtgtta ccggattagc tgctgctgct      60

cgtattgcta gccaaggtca tgaagttaca atcttcgaaa aaaacaataa tgtaggtggt     120

cgtatgaatc aattaaaaaa agatggtttt acattcgata tgggacctac aattgttatg     180

atgccagatg tatataaaga tgtatttact gcttgcggta aaaactatga agattatata     240

gagttacgtc aacttcgtta catttatgac gtatatttcg atcacgatga tcgtattact     300

gttccaactg atttagctga attacaacaa atgttagaat caattgaacc tggtagtaca     360

cacggattta tgtcattttt aacagatgtg tacaagaaat atgaaatcgc tcgcagatat     420

ttcttagaac gtacttaccg taaaccatca gacttctaca atatgacctc tttagtacaa     480

ggtgctaaac ttaaaacttt aaatcacgct gatcaactta tcgaacacta cattgataac     540

gaaaagattc aaaaactttt agcattccaa actctttata tcggcattga tccaaagcgt     600

ggtcctagtt tatatagtat tattcctatg attgaaatga tgttcggtgt acattttatc     660

aaaggtggta tgtatggtat ggctcaagga ttagctcaac ttaacaaaga tttaggtgtt     720

aatattgaat taaatgctga aattgaacaa atcattatcg atcctaaatt caaacgcgca     780

gatgcaatta aagttaatgg tgacattcgc aaatttgata agattttatg tactgctgac     840

tttccttcag ttgccgaatc acttatgcca gatttcgcac ctatcaaaaa gtaccctcca     900

cataaaattg cagatttaga ttattcttgt tcagcttttc ttatgtatat tggtattgac     960

atcgacgtaa ctgaccaagt tcgtttacat aacgtaattt ttagcgacga ttttcgtgga    1020

aatattgaag aaattttcga aggtcgctta agttacgacc catcaatcta tgtttatgta    1080

ccagctgtag ccgataaatc tttagctcct gaaggtaaaa caggcattta tgtgttaatg    1140

cctactcctg aacttaaaac aggatcaggt attgactggt cagatgaggc tttaactcaa    1200

caaattaaag aaatcattta tcgtaaatta gcaacaattg aagtatttga agacattaaa    1260

tcacacattg tatcagaaac aatttttact cctaatgact ttgaacaaac ctatcacgct    1320

aaatttggtt ctgctttcgg tttaatgccc accttagcac aatctaatta ttacagacct    1380

caaaatgtgt cacgtgatta taaagactta tatttcgcag gtgcatcaac acatccaggt    1440

gctggagttc caattgtatt aacaagtgcc aagataacag tagacgaaat gattaaagat    1500

attgagcgtg gtgtgggtac cggtgaaaac ttatactttc aaggctcagg tggcggtgga    1560

agtgattaca aagatgatga tgataaagga accggttaat ctagactcga g             1611

<210> 140
<211> 993
<212> DNA
<213> Artificial Sequence

<220>
<223> Description of Artificial Sequence: Synthetic
      polynucleotide

<400> 140
catatggtac cagcatttga cttcgatggt tacatgcttc gtaaagctaa atctgtaaat      60

aaagctcttg aagctgcagt acaaatgaaa gaaccattaa aaattcatga aagtatgcgt     120

tattctttat tagctggtgg taaacgtgta cgtccaatgt tatgtattgc agcttgtgaa     180

ttagttggtg gtgacgaaag tactgctatg cctgctgctt gcgctgtaga aatgattcat     240

actatgagtt taatgcatga tgatttacca tgtatggata atgacgattt acgtcgtggt     300

aaaccaacaa accacatggc atttggtgaa agtgtagcag tattagcagg tgatgcatta     360

ttatcttttg cttttgaaca tgtagcagca gcaacaaaag gtgctcctcc agaacgtatt     420

gttagagttt taggtgaact tgcagtttct attggttcag aaggtttagt tgctggacaa     480

gtagttgacg tttgttctga aggtatggct gaggttggtt tagatcattt agaatttatt     540

catcaccaca aaactgctgc tttattacaa ggttctgtag tattaggtgc aatattaggt     600

ggtggaaaag aagaagaggt agcaaaactt cgtaaattcg ctaactgcat tggtttactt     660

ttccaagtag tagatgatat tcttgatgta acaaaatcat ctaaagaatt aggtaaaaca     720

gcaggtaaag atttagttgc tgataaaact acttatccaa aattaatcgg tgttgagaaa     780

agtaaagagt tcgcagaccg tttaaatcgt gaagctcaag aacaacttct tcattttcat     840

ccacatagag cagcaccttt aatcgcttta gcaaactata ttgcttatcg tgataatggt     900

accggtgaaa atttatattt tcaaggttca ggtggcggag gttctgatta taaagatgat     960

gatgataaag gaaccggtta atctagactc gag                                  993

<210> 141
<211> 906
<212> DNA
<213> Artificial Sequence

<220>
<223> Description of Artificial Sequence: Synthetic
      polynucleotide

<400> 141
catatggtac caagtcaacc ttactgggca gcaattgagg cagatattga acgttactta      60

aaaaaatcaa ttacaattcg tccaccagaa actgtatttg gtccaatgca ccacttaact     120

tttgctgcac cagctacagc tgctagtact ttatgtttag cagcatgtga acttgtaggt     180

ggtgatcgta gtcaagctat ggctgcagca gcagcaatcc atcttgttca tgcagctgct     240

tatgtacatg aacatttacc attaactgat ggtagtcgtc cagtaagtaa accagctatc 300

caacataaat atggtccaaa tgtagaatta cttacaggtg acggtattgt accatttggt 360

tttgaattat tagcaggttc tgttgatcca gcacgtacag atgatccaga ccgtatttta 420

cgtgtaataa ttgaaataag tcgtgctggt ggtccagaag gtatgattag tggtttacat 480

cgtgaagaag agattgtaga tggtaatact tctcttgatt ttattgaata cgtttgcaaa 540

aaaaaatatg gtgaaatgca cgcatgtggt gctgcatgcg gtgcaatttt aggtggtgca 600

gctgaagaag aaattcaaaa acttcgtaac ttcggattat atcaaggaac tttacgtggt 660

atgatggaga tgaaaaactc acaccaactt attgacgaaa atatcattgg caaacttaaa 720

gaattagctt tagaagaatt aggtggattt catggtaaaa atgctgaatt aatgtctagt 780

ttagtagcag aaccatcatt atatgctgct ggtaccggtg aaaatttata ctttcaaggt 840

tctggtggtg gtggcagtga ttataaagac gatgatgaca aaggaaccgg ttaatctaga 900

ctcgag 906

<210> 142
<211> 1095
<212> DNA
<213> Artificial Sequence

<220>
<223> Description of Artificial Sequence: Synthetic
      polynucleotide

<400> 142
catatggtac cacttttatc taacaaatta agagagatgg ttttagcaga agttcctaaa 60

ttagcatctg ctgctgaata tttctttaaa cgtggtgttc agggtaaaca attccgttca 120

acaattttat tattaatggc aacagctctt gacgttcgtg ttccagaagc attaattggt 180

gaatctactg atattgtaac atctgaatta cgtgtacgtc aacgtggcat tgctgaaatt 240

acagaaatga ttcatgtagc atcacttctt cacgatgacg ttcttgacga tgctgatact 300

cgtcgtggtg ttggtagtct taatgttgta atgggaaaca aaatgtcagt tttagcaggt 360

gacttcttac tttctcgtgc ttgtggtgct cttgcagctc ttaaaaacac agaagttgta 420

gcattattag ctacagcagt agaacactta gttactggtg agacaatgga aataacttca 480

tcaactgaac aacgttattc tatggattac tacatgcaga aaacttatta caaaactgct 540

tcattaattt caaattcatg taaagcagtt gctgtattaa caggtcaaac agctgaagtt 600

gcagtattag cttttgaata tggtcgtaat ttaggtttag ctttccagtt aattgacgac 660

attttagatt tcacaggcac atctgctagt ttaggaaaag gttctttatc agatatacgt 720

catggtgtta ttactgctcc tatcttattt gcaatggaag aatttcctca attaagagaa 780

gtagtagatc aagtagaaaa agatccaaga aatgtagaca tagctttaga atatttaggt 840

aaaagtaaag gtattcaacg tgctcgtgaa ttagcaatgg aacacgcaaa tttagctgct 900

gcagctattg gttctttacc tgaaacagat aacgaagatg ttaaacgttc acgtcgtgct 960

ttaattgatt taacacacag agtaattaca cgtaacaaag gtaccggtga gaatttatac 1020

tttcaaggta gtggtggagg aggtagtgac tataaagatg atgacgataa aggaaccggt 1080

taatctagac tcgag 1095

<210> 143
<211> 1107
<212> DNA
<213> Artificial Sequence

<220>
<223> Description of Artificial Sequence: Synthetic
      polynucleotide

<400> 143
catatggtac cagtagtttc tgaacgttta agacattctg taacaactgg tattccagca 60

ttaaaaacag cagctgaata tttctttcgt cgtggtatcg aaggaaaacg tttaagacct 120

acattagcat tattaatgag tagtgcttta tcaccagctg ctccatcacc agagtattta 180

caagttgata caagacctgc tgcagaacac cctcatgaaa tgcgtcgtcg tcaacaacgt 240

ttagctgaaa ttgcagaatt aatccatgta gcttcattac ttcacgatga tgttattgat 300

gacgcacaaa cacgtcgtgg tgttttaagt ttaaatacat ctgttggtaa taaaacagct 360

atcttagcag gtgatttctt attagctcgt gcatctgtaa cattagctag tttaagaaac 420

tctgaaattg tagaattaat gtcacaggtt ttagaacact tagtatctgg tgaaattatg 480

caaatgactg ctacttcaga acaactttta gatttagaac attatttagc aaaaacatat 540

tgtaaaactg cttcattaat ggctaatagt tctcgttctg ttgcagttct tgcaggtgca 600

gctcctgaag tttgtgatat ggcatggtca tacggtcgtc atttaggtat tgctttccaa 660

gtagttgacg atttattaga tttaacaggt tcatcttctg ttttaggtaa acctgcttta 720

aacgatatgc gttctggttt agcaacagca ccagtattat tcgctgcaca agaagaacct 780

gcattacagg ctcttatatt acgtcgtttt aaacacgacg gtgacgtaac aaaagcaatg 840

tcattaattg aacgtacaca aggcttacgt cgtgctgaag aacttgcagc acaacacgca 900

aaagctgctg ctgatatgat tcgttgctta cctacagctc aatcagacca tgcagaaatt 960

gctcgtgaag cattaattca aattacacat cgtgttttaa cacgtaaaaa aggtaccggt 1020

gaaaacttat actttcaagg ttctggtggt ggtggatcag attataaaga tgatgatgac 1080

aaaggaaccg gttaatctag actcgag 1107

<210> 144
<211> 1002

<212> DNA
<213> Artificial Sequence

<220>
<223> Description of Artificial Sequence: Synthetic
      polynucleotide

<400> 144
catatggtac cagattttcc acaacaatta gaagcatgtg ttaaacaagc aaatcaagca      60

ttatcacgtt tcatcgcacc acttccattc caaaatactc ctgttgttga aacaatgcaa     120

tatggtgcat tattaggagg taaaagatta agaccatttc ttgtatatgc aacaggtcac     180

atgtttggag tatctactaa cacattagat gctccagctg ctgcagttga atgtattcat     240

gcatatagtt taattcatga tgatttacct gcaatggatg atgatgactt aagaagaggt     300

ttacctacat gtcatgttaa atttggtgaa gctaatgcta ttttagctgg cgatgcactt     360

caaactcttg cattcagtat tttatcagat gctgatatgc cagaagtttc agatcgtgat     420

cgtatttcta tgatatctga attagcttct gctagtggta ttgctggtat gtgcggtggc     480

caagctcttg atttagacgc agaaggaaaa cacgttcctt tagatgcttt agagcgtata     540

catcgtcaca aaacaggagc tttaattaga gctgctgttc gtcttggtgc tttatcagct     600

ggagacaaag gtcgtcgtgc tttaccagtt ttagacaaat acgctgaaag tattggttta     660

gcttttcaag ttcaggatga tatcttagat gttgtaggtg atactgctac tttaggtaaa     720

cgtcaaggtg ctgatcaaca gttaggcaaa tctacatacc cagcactttt aggtttagaa     780

caagctcgta aaaaagcaag agacttaatt gacgatgctc gtcaaagtct taaacaatta     840

gcagaacaat cacttgatac aagtgcttta gaagcattag cagattacat tattcaacgt     900

aataaaggta ccggtgaaaa tttatatttt caaggttctg gtggtggagg ttcagactat     960

aaagatgacg atgataaagg aaccggttaa tctagactcg ag                       1002


<210> 145
<211> 1257
<212> DNA
<213> Artificial Sequence

<220>
<223> Description of Artificial Sequence: Synthetic
      polynucleotide

<400> 145
catatggtac caagtgttag ttgttgttgt agaaatttag gaaaaactat caaaaaagct      60

attccaagtc accacttaca tttacgttct ttaggtggta gtttatatag aagacgtatt     120

caatcatctt caatggaaac agacttaaaa tctacattct taaatgttta ttcagttctt     180

aaatcagatt tattacacga cccatcattt gaatttacaa atgaaagtcg tttatgggta     240

gatagaatgc ttgattataa tgttcgtggc ggtaaactta atcgtggtct ttctgtagta     300

gactctttca aattacttaa acaaggtaat gatttaactg aacaagaagt tttcttatct 360

tgtgcattag gttggtgtat tgagtggtta caggcttact ttttagttct tgatgatatt 420

atggataatt cagttacacg tcgtggtcaa ccttgttggt ttcgtgtacc acaagttggt 480

atggtagcta ttaatgatgg cattcttctt cgtaaccata ttcatcgtat tcttaaaaaa 540

cacttccgtg ataaaccata ttatgtagat ttagttgacc ttttcaatga agtagagtta 600

caaactgcat gtggacaaat gattgattta atcacaacat ttgaaggtga aaaagactta 660

gctaaatata gtttatcaat tcaccgtcgt attgttcaat acaaaactgc atattactca 720

ttctatttac cagttgcatg tgctctttta atggctggcg aaaatttaga aaaccacatt 780

gatgttaaaa atgtattagt agatatgggt atttactttc aagttcagga tgattattta 840

gactgttttg ctgatcctga aacattaggt aaaattggca ctgatattga ggactttaaa 900

tgttcttggt tagttgtaaa agcattagaa cgttgtagtg aagaacaaac aaaaattctt 960

tacgaaaact atggcaaacc tgatccatct aatgttgcta aagtaaaaga tttatacaaa 1020

gaattagatt tagaaggcgt tttcatggaa tatgaatcta aatcatacga gaaattaact 1080

ggtgctatcg aaggtcacca atctaaagca attcaagctg ttcttaaatc tttcttagca 1140

aaaatctata aacgtcaaaa aggtaccggt gaaaacttat actttcaagg tagtggtggc 1200

ggtggtagtg attataaaga tgatgatgat aaaggaaccg gttaatctag actcgag 1257

<210> 146
<211> 1131
<212> DNA
<213> Artificial Sequence

<220>
<223> Description of Artificial Sequence: Synthetic
      polynucleotide

<400> 146
catatggtac cagctgatct taaatcaaca ttcttagatg tttattcagt attaaaaagt 60

gatttattac aagatccatc ttttgaattt acacacgaaa gtcgtcaatg gttagaacgt 120

atgttagatt ataatgttcg tggaggcaaa ttaaacagag gtttaagtgt agtagacagt 180

tacaaacttt taaaacaagg tcaagactta acagaaaaag aaacattttt atcttgtgct 240

ttaggttggt gtattgaatg gttacaagca tacttcttag ttttagacga tattatggat 300

aattctgtaa ctagacgtgg tcaaccatgt tggtttcgta aaccaaaagt aggtatgatt 360

gctattaatg atggaatact tcttcgtaac cacattcatc gtattcttaa aaaacacttt 420

cgtgaaatgc cttattatgt agaccttgta gacttattta acgaagtaga atttcaaaca 480

gcttgtggtc aaatgattga cttaattaca acatttgatg gtgaaaaaga cctttcaaaa 540

tattcacttc agattcaccg tcgtattgtt gagtacaaaa cagcatacta ctctttctat 600

ttacctgtag catgtgcttt acttatggca ggtgaaaatt tagaaaatca cacagatgtt 660

aaaactgtat tagttgatat gggtatctat ttccaagttc aagatgatta tttagattgc 720

ttcgctgatc cagaaacatt aggtaaaatt ggtacagata ttgaagactt taaatgtagt 780

tggttagtag taaaagcatt agaacgttgt agtgaagaac aaacaaaaat tctttacgaa 840

aattatggaa aagctgaacc ttcaaatgta gctaaagtta aagcattata caaagaatta 900

gatttagagg gtgcatttat ggaatatgaa aaagaatcat acgagaaact tacaaaactt 960

attgaagcac atcaatcaaa agctattcaa gcagttctta aatctttctt agctaaaatt 1020

tataaacgtc aaaaaggtac cggtgaaaac ttatactttc aaggctctgg aggtggtggt 1080

tcagactata aagatgatga tgataaagga accggttaat ctagactcga g 1131

<210> 147
<211> 1185
<212> DNA
<213> Artificial Sequence

<220>
<223> Description of Artificial Sequence: Synthetic
      polynucleotide

<400> 147
catatggtac caagtggcga acctactcca aaaaaaatga aagcaacata cgttcacgac 60

cgtgaaaact ttacaaaagt atacgaaact cttcgtgacg aattacttaa cgatgattgt 120

cttagtccag ctggttcacc tcaggctcaa gctgctcaag agtggtttaa agaagttaat 180

gattataatg ttcctggtgg aaaacttaac cgtggtatgg ctgtatatga cgttttagct 240

tcagttaaag gtccagatgg tttaagtgaa gacgaagtat ttaaagctaa cgctcttggt 300

tggtgtattg agtggttaca agcatttttc ttagttgctg atgatataat ggatggttca 360

attacacgtc gtggccaacc ttgttggtac aaacaaccta aagttggtat gattgcttgt 420

aatgattaca tcttattaga atgctgtatt tactcaattc ttaaaagaca ttttagaggt 480

cacgctgcat acgctcaact tatggacctt ttccatgaaa ctacattcca gacttcacac 540

ggtcaattat tagatttaac aacagcacct atcggttctg tagacttatc aaaatataca 600

gaagataatt accttcgtat tgtaacatat aaaactgcat actattcttt ttatttacct 660

gtagcatgtg gtatggtatt agctggcatt acagatccag ctgcttttga tcttgcaaaa 720

aatatttgtg ttgaaatggg tcaatatttc cagattcaag acgattattt agattgctat 780

ggtgaccctg aggttattgg taaaatcggt acagacatag aagacaacaa atgtagttgg 840

ttagtttgca cagctcttaa aatcgcaaca gaagaacaaa aagaggttat aaaagctaat 900

tatggtcaca aagaggctga atcagtagca gcaattaaag cattatacgt tgaattaggt 960

attgaacaac gttttaaaga ctatgaagct gcatcatacg caaaattaga aggtacaatt 1020

agtgaacaaa ctttattacc taaagcagta tttacttctt tattagctaa aatctataaa 1080

agaaaaaaag gtaccggtga gaacttatac tttcaaggta gtggaggtgg tggttcagac 1140

tataaagatg atgatgataa aggaaccggt taatctagac tcgag 1185

<210> 148
<211> 651
<212> DNA
<213> Artificial Sequence

<220>
<223> Description of Artificial Sequence: Synthetic
      polynucleotide

<400> 148
catatggtac cacaaactga acatgttatc ttattaaacg ctcaaggtgt tcctacaggt 60

acattagaaa aatatgctgc acacactgct gatactcgtt tacacttagc tttctcatct 120

tggttattca atgctaaagg tcaactttta gttacaagac gtgcattaag taaaaaagca 180

tggcctggtg tttggactaa ctcagtttgt ggtcatccac aattaggtga aagtaatgaa 240

gatgcagtta tacgtcgttg cagatatgaa ttaggtgttg aaataactcc accagaatca 300

atttatccag atttccgtta tcgtgcaact gatcctagtg gtatcgttga aaacgaagta 360

tgtcctgttt ttgctgcacg tacaacaagt gcattacaaa ttaatgatga tgaagtaatg 420

gattatcaat ggtgtgactt agctgatgtt ttacatggta ttgatgcaac accatgggca 480

ttttcaccat ggatggtaat gcaagcaaca aatcgtgaag cacgtaaaag attaagtgct 540

tttacacagt taaaaggtac cggtgaaaac ttatactttc aaggtagtgg aggtggtggt 600

tctgactata aagatgacga tgataaagga accggttaat ctagactcga g 651

<210> 149
<211> 984
<212> DNA
<213> Artificial Sequence

<220>
<223> Description of Artificial Sequence: Synthetic
      polynucleotide

<400> 149
catatggtac cacttcgtag tttattaaga ggtttaacac acattcctcg tgttaatagt 60

gctcagcaac cttcttgcgc tcacgctcgt cttcaattta aacttcgttc tatgcaatta 120

ttagcagaaa accgtacaga tcacatgcgt ggtgcttcta catgggcagg tggtcagtct 180

caagatgaat taatgcttaa agatgaatgt atcttagtag atgctgatga taacattact 240

ggtcacgctt ctaaattaga atgtcacaaa tttcttccac atcaaccagc tggattatta 300

caccgtgctt tttctgtatt tcttttcgac gatcaaggtc gtttactttt acaacaacgt 360

gctcgtagta aaattacatt tccatctgta tgggctaata catgttgtag tcatccatta 420

catggtcaaa caccagatga agtagatcaa caatcacaag tagcagacgg aactgtacca 480

ggtgcaaaag ctgctgcaat cagaaaatta gaacatgaat taggtattcc agctcaccaa 540

ttaccagcat cagcttttcg tttcttaaca cgtcttcact attgtgcagc tgacgttcaa 600

cctgcagcaa cacaatctgc attatggggt gaacacgaaa tggattacat tttattcatt 660

agagctaatg ttacacttgc tcctaatcct gacgaagtag atgaggtacg ttatgtaact 720

caagaagaat taagacaaat gatgcaacca gataatggtt tacaatggtc accatggttc 780

cgtattattg cagcaagatt tttagaacgt tggtgggctg atttagatgc tgcattaaat 840

acagataaac atgaagactg gggaacagtt catcacatta acgaagctgg taccggtgaa 900

aacttatact ttcaaggatc aggaggcggt ggaagtgatt ataaagatga tgatgataaa 960

ggaaccggtt aatctagact cgag 984

<210> 150
<211> 1740
<212> DNA
<213> Artificial Sequence

<220>
<223> Description of Artificial Sequence: Synthetic
      polynucleotide

<400> 150
catatggtac caagaagatc aggcaattat aacccaacag catgggactt caattatatc 60

caatcattag acaatcaata caaaaaagaa cgttactcta ctcgtcacgc tgaattaaca 120

gttcaagtta aaaaattatt agaagaagaa atggaagctg ttcaaaaact tgaacttata 180

gaggatctta aaaacttagg catttcttac ccatttaaag ataatattca acaaatctta 240

aatcaaattt acaatgaaca caaatgttgt cacaactcag aagttgaaga aaaagacctt 300

tatttcactg ctttacgttt tagattatta cgtcaacaag gttttgaagt aagtcaagaa 360

gtatttgatc actttaaaaa cgaaaaaggt acagatttta aacctaattt agcagatgat 420

actaaaggat tattacaatt atatgaagca tcattcttat tacgtgaagc agaagacaca 480

ttagaacttg ctcgtcaatt ctctactaaa cttttacaaa aaaaagttga tgaaaacggt 540

gacgataaaa ttgaagataa cttattactt tggattagac gtagtttaga attaccatta 600

cattggcgtg tacaaagatt agaagctcgt ggctttttag atgcttacgt tcgtagacct 660

gatatgaatc ctattgtatt tgaattagca aaattagact ttaacattac tcaagcaaca 720

caacaagaag aacttaaaga tttatcaaga tggtggaata gtactggctt agctgaaaaa 780

cttccttttg ctcgtgatcg tgtagttgaa tcatatttct gggctatggg tacttttgaa 840

ccacatcaat acggatacca acgtgaatta gttgctaaaa tcattgcact tgctacagtt 900

gtagacgatg tttacgatgt atatggtact ttagaggaat tagaactttt tactgatgct 960

attcgtcgtt gggaccgtga atctattgac caattaccat attacatgca attatgtttt   1020

cttactgtaa acaactttgt ttttgagtta gctcacgacg tattaaaaga taaatcattc   1080

aattgtttac ctcatttaca aagatcatgg ttagatttag ctgaagcata ccttgtagaa   1140

gcaaaatggt atcatagtcg ttatacacct tctttagaag aatatcttaa tattgctcgt   1200

gtttcagtaa catgtccaac tattgtttct caaatgtatt ttgcattacc aattccaatc   1260

gaaaaacctg taattgagat catgtacaaa tatcacgata tcttatactt atcaggtatg   1320

ttattacgtt taccagatga cttaggaaca gcatcattcg aacttaaacg tggtgatgta   1380

caaaaagcag ttcaatgtta tatgaaagaa cgtaatgttc ctgaaaatga agctcgtgaa   1440

catgttaaat tcttaattcg tgaggcttct aaacaaatta atacagcaat ggcaacagac   1500

tgtccattta cagaagattt tgcagttgca gcagcaaact taggtcgtgt agcaaatttt   1560

gtatatgttg atggtgatgg ttttggagta caacacagta aaatctatga gcaaattggt   1620

acacttatgt ttgaaccata tccaggtacc ggtgaaaact tatactttca aggtagtggt   1680

ggtggaggtt ctgattacaa agacgatgat gataaaggaa ccggttaatc tagactcgag   1740

<210> 151
<211> 1737
<212> DNA
<213> Artificial Sequence

<220>
<223> Description of Artificial Sequence: Synthetic
      polynucleotide

<400> 151
catatggtac caagaagaag tggaaactat aaacctacaa tgtgggattt tcaatttatt     60

caaagtgtaa ataatcttta cgctggtgat aaatacatgg aacgtttcga tgaagtaaaa    120

aaagaaatga aaaaaaactt aatgatgatg gttgagggtt taatagagga attagatgtt    180

aaattagaat taatagataa tttagaaaga ttaggtgtta gttatcattt caaaaatgaa    240

ataatgcaaa tccttaaatc tgtacaccag caaatcactt gtcgtgataa ttcattatac    300

tctactgcat taaaatttcg tttattacgt caacacggat tccacattag tcaagacatc    360

tttaacgatt ttaaagatat gaatggcaat gttaaacaaa gtatctgtaa cgatactaaa    420

ggtttattag aactttatga agcatctttc ttatctactg aatgtgaaac aacacttaaa    480

aacttcactg aagcacactt aaaaaattat gtttatatta accactcatg tggagatcaa    540

tacaataaca taatgatgga attagttgtt cacgctttag aattaccacg tcactggatg    600

atgcctcgtt tagagacacg ttggtatata tcaatttatg aacgtatgcc taatgctaat    660

ccacttttac ttgaacttgc taaattagac ttcaatattg ttcaagctac acaccaacaa    720

gacttaaaat cattatcacg ttggtggaaa aacatgtgtt tagctgaaaa attatcattt    780

tctcgtaacc gtttagtaga aaatcttttc tgggcagttg gaactaattt tgaaccacaa 840

cacagttatt tccgtcgttt aatcactaaa atcattgttt ttgttggtat tattgatgat 900

atttatgatg tttacggcaa acttgatgag ttagaattat tcactttagc tgtacaacgt 960

tgggatacaa aagcaatgga agacttacca tattacatgc aagtttgtta tttagcttta 1020

attaatacaa caaatgatgt tgcttatgaa gttcttcgta aacataacat taatgtatta 1080

ccatacttaa ctaaatcttg gacagactta tgtaaatcat atttacaaga agctcgttgg 1140

tactacaatg gttacaaacc ttcattagag gaatatatgg ataatggttg gattagtata 1200

gcagttccta tggtattagc acatgcactt ttcttagtta cagatccaat tacaaaagaa 1260

gcattagaat cattaacaaa ctatcctgat attattcgtt gctcagctac aatattccgt 1320

ttaaatgatg atcttggtac aagttcagat gaattaaaac gtggagatgt accaaaatca 1380

attcaatgct atatgaacga aaaaggcgtt tcagaggaag aagctcgtga acatattcgt 1440

ttcttaatca aagaaacatg gaaattcatg aacactgcac accataaaga gaaaagttta 1500

ttttgtgaga catttgtaga aattgcaaaa aatattgcaa caacagctca ttgtatgtac 1560

ttaaaaggtg attctcacgg tattcaaaac actgatgtta aaaactcaat aagtaatata 1620

cttttccatc caattattat cggtaccggt gaaaaccttt actttcaagg ttcaggtggt 1680

ggcggttcag actataaaga tgacgatgat aaaggaaccg gttaatctag actcgag 1737

<210> 152
<211> 1752
<212> DNA
<213> Artificial Sequence

<220>
<223> Description of Artificial Sequence: Synthetic
      polynucleotide

<400> 152
catatggtac caagacgtag tggaaattat gagccatctg catgggactt caattactta 60

caatctctta ataattatca ccataaagaa gaacgttact tacgtcgtca agctgattta 120

attgaaaaag taaaaatgat tcttaaagaa gagaaaatgg aagcattaca gcaattagaa 180

cttatagacg atcttcgtaa tttaggtctt tcatattgtt ttgatgatca aattaatcat 240

attcttacaa caatttacaa ccaacattct tgtttccatt atcacgaagc tgcaacaagt 300

gaagaagcta acttatattt cacagcttta ggtttccgtt tacttcgtga acacggattc 360

aaagtatcac aagaagtatt tgaccgtttc aaaaatgaaa aaggtacaga ttttcgtcca 420

gatttagtag atgatactca aggtttatta caactttatg aagcatcttt ccttcttcgt 480

gaaggtgaag acactttaga atttgcacgt caatttgcta ctaaatttct tcaaaaaaaa 540

gttgaggaga aaatgataga agaggaaaat cttttatctt ggactttaca ttcacttgaa 600

ttaccattac attggcgtat acaacgttta gaagctaaat ggtttttaga tgcttatgct 660

agtcgtcctg atatgaatcc aataatcttt gaattagcaa aattagaatt taacattgct 720

caggcacttc aacaagaaga acttaaagat ttatcaagat ggtggaacga tactggtatt 780

gctgaaaaat tacctttcgc tcgtgataga atcgttgaat ctcattattg ggcaattggt 840

actttagaac cttatcaata ccgttatcag cgttcattaa ttgcaaaaat cattgcttta 900

actacagttg ttgatgatgt atatgatgtt tacggtacat tagacgaatt acagttattt 960

actgatgcaa ttcgtcgttg ggacattgaa agtataaatc aattaccttc ttatatgcaa 1020

ttatgttatt tagctattta taatttcgta tcagaattag cttatgatat tttcagagat 1080

aaaggtttta attctttacc atatttacac aaaagttggc ttgacttagt tgaggcttac 1140

tttcaagaag caaaatggta tcattctggc tacacaccat cattagaaca atacttaaat 1200

atcgctcaaa tttctgtagc aagtccagct atattaagtc aaatttactt tactatggct 1260

ggttcaattg ataaaccagt aatcgaatca atgtacaaat atagacacat tttaaactta 1320

tctggtatat tacttagatt accagatgac ttaggtactg ctagtgatga attaggtcgt 1380

ggtgatttag caaaagcaat gcaatgttac atgaaagagc gtaacgtttc tgaagaagaa 1440

gctcgtgatc atgtacgttt cttaaatcgt gaggtttcaa aacaaatgaa tcctgctcgt 1500

gctgctgatg attgtccatt cactgatgat tttgtagtag ctgctgctaa tttaggaaga 1560

gttgcagatt tcatgtatgt tgaaggcgat ggtttaggtt tacaataccc agctatccac 1620

caacacatgg cagaactttt atttcaccct tacgcaggta ccggtgaaaa cttatacttt 1680

caaggttcag gtggtggagg ttctgactat aaagatgatg atgataaagg aaccggttaa 1740

tctagactcg ag 1752

<210> 153
<211> 1752
<212> DNA
<213> Artificial Sequence

<220>
<223> Description of Artificial Sequence: Synthetic
      polynucleotide

<400> 153
catatggtac caagaagatc aggaaattat caacctagtg catgggattt taactatatc 60

caatctctta ataacaacca ttctaaagaa gaacgtcact tagagcgtaa agcaaaactt 120

attgaagaag taaaaatgtt attagagcaa gaaatggctg ctgtacaaca attagagctt 180

attgaagacc ttaaaaactt aggtttatct tacttattcc aagatgaaat caaaataatc 240

cttaattcta tttacaatca tcataaatgt tttcataata atcacgaaca atgtattcac 300

gttaatagtg acttatactt tgttgcatta ggcttccgtt tatttcgtca acatggtttc 360

aaagtttctc aagaggtttt tgactgtttt aaaaacgaag aaggatcaga ctttagtgct 420

aacttagcag atgatactaa aggtttactt caattatacg aggcttcata tttagttaca 480

gaagatgaag acacattaga aatggcacgt caattttcaa ctaaaatctt acaaaaaaaa 540

gtagaagaga aaatgattga gaaagagaac ttattaagtt ggactttaca tagtttagaa 600

ttaccacttc actggcgtat tcaacgttta gaagcaaaat ggttccttga tgcttatgct 660

agtcgtccag atatgaatcc aattattttt gaattagcta aattagagtt taacattgct 720

caagcattac aacaagaaga attaaaagat ttaagtagat ggtggaatga tacaggcatt 780

gctgaaaaat taccttttgc tcgtgataga atagtagaga gtcattactg ggcaattggt 840

actttagaac cttatcaata tagatatcaa cgttcattaa ttgctaaaat tattgcttta 900

acaacagttg ttgatgacgt ttacgacgta tatggaactt tagatgaatt acagttattt 960

acagacgcta ttcgtcgttg ggatattgaa tctattaatc aattaccaag ttatatgcaa 1020

ttatgctatt tagctattta taactttgtt tctgaattag catacgatat ttttcgtgac 1080

aaaggattca attctttacc ttaccttcat aaatcatggt tagatttagt agaagcatac 1140

tttgttgaag ctaaatggtt tcatgatggt tatactccaa ctcttgaaga atatttaaat 1200

aactcaaaaa ttactattat atgtcctgct attgttagtg aaatctactt cgcattcgct 1260

aattcaattg ataaaacaga agttgaatca atctacaaat atcacgatat tttatattta 1320

tcaggaatgc ttgcacgttt accagacgac ttaggtactt catcatttga aatgaaaaga 1380

ggtgatgttg ctaaagctat tcaatgttac atgaaagaac ataatgcttc agaggaagaa 1440

gctcgtgaac acattcgttt cttaatgcgt gaagcatgga aacacatgaa tactgctgca 1500

gctgctgatg actgtccatt tgaatctgat ttagtagtag gtgctgcatc attaggtaga 1560

gttgcaaact ttgtatatgt tgaaggtgac ggttttggtg tacaacattc aaaaatacat 1620

caacaaatgg ctgaattact tttttatcca tatcaaggta ccggtgaaaa cttatacttt 1680

caaggtagtg gaggtggtgg tagtgactat aaagacgatg acgataaagg aaccggttaa 1740

tctagactcg ag 1752

<210> 154
<211> 1722
<212> DNA
<213> Artificial Sequence

<220>
<223> Description of Artificial Sequence: Synthetic
      polynucleotide

<400> 154
catatggtac caagaagaag tgctaattat caagcaagta tttgggatga taatttcatt 60

caaagtcttg catctcctta tgcaggagaa aaatatgcag aaaaagcaga aaaacttaaa 120

acagaagtta aaactatgat tgatcaaaca agagatgaac ttaaacaatt agaacttatt 180

gataacttac aacgtttagg tatatgtcat cactttcaag accttacaaa aaaaatttta 240

caaaaaattt atggagaaga acgtaacgga gatcaccaac attacaaaga aaaaggctta 300

cattttacag cattacgttt ccgtatttta cgtcaggacg gttatcatgt tccacaagat 360

gtattttcat catttatgaa taaagctggt gactttgaag aatctttaag taaagacaca 420

aaaggtttag ttagtttata tgaggcttct tacttatcaa tggaaggtga aactatttta 480

gatatggcaa aagacttttc atctcaccat ttacataaaa tggttgaaga tgctactgac 540

aaacgtgtag ctaatcaaat tatccattct cttgaaatgc cacttcacag acgtgttcaa 600

aaacttgaag caatttggtt tattcaattc tacgaatgcg gctctgatgc taatccaact 660

ttagtagaat tagcaaaatt agatttcaac atggttcagg caacatacca agaagaatta 720

aaacgtttat cacgttggta tgaagaaaca ggcttacaag agaaactttc attcgctcgt 780

caccgtcttg ctgaagcatt cttatggtct atgggtatta ttccagaagg acactttggt 840

tatggtcgta tgcacttaat gaaaattggt gcttacatta cattacttga tgatatttat 900

gatgtttatg gtactttaga agaacttcaa gtattaacag aaattattga acgttgggat 960

attaacttat tagatcaatt acctgaatac atgcaaatct tctttttata catgtttaat 1020

tctacaaatg aacttgctta tgaaatttta cgtgatcaag gtatcaatgt aatatcaaac 1080

ttaaaaggat tatgggtaga gttatctcag tgttacttta aagaagctac ttggttccat 1140

aacggttaca caccaacaac tgaagaatat cttaatgttg cttgtatttc tgctagtggt 1200

cctgttattt tattttcagg ttactttact actactaatc ctattaataa acacgaatta 1260

caatctttag aacgtcacgc acattcatta tctatgatat tacgtttagc tgatgattta 1320

ggtacatcaa gtgatgaaat gaaacgtgga gatgtaccaa aagctattca atgttttatg 1380

aatgacactg gttgttgtga agaagaagca cgtcaacacg taaaaagatt aatagatgct 1440

gaatggaaaa aaatgaacaa agacatctta atggagaaac catttaaaaa tttttgtcca 1500

actgctatga atttaggtcg tatttctatg agtttttatg aacacggaga tggttatgga 1560

ggtcctcact ctgatacaaa aaaaaaaatg gtatctttat ttgtacaacc aatgaatatt 1620

actattggta ccggtgaaaa cctttatttt caaggttctg gtggtggcgg ttcagattat 1680

aaagatgatg acgacaaagg aaccggttaa tctagactcg ag 1722

<210> 155
<211> 1731
<212> DNA
<213> Artificial Sequence

<220>
<223> Description of Artificial Sequence: Synthetic

polynucleotide

<400> 155
catatggtac caagacgttc agctaactat caacctagta tttggaacca cgattacatt 60

gaatcacttc gtatcgaata tgttggtgaa acatgtacac gtcaaattaa cgttttaaaa 120

gaacaagttc gtatgatgtt acacaaagtt gttaatccat tagaacaatt agaattaatt 180

gaaattttac aacgtttagg tttaagttac catttcgaag aagaaataaa acgtatttta 240

gatggtgttt acaataacga tcatggtggt gatacatgga aagcagaaaa cctttatgca 300

acagctctta aattccgtct tttacgtcag cacggttatt ctgtttctca agaagttttc 360

aactctttta aagatgagcg tggcagtttc aaagcatgtt tatgtgaaga tactaaaggt 420

atgttatcac tttatgaagc atctttcttt cttattgaag gtgaaaacat tttagaggaa 480

gctagagact ttagtacaaa acatcttgaa gaatatgtaa aacaaaataa agagaaaaac 540

ttagctactt tagttaatca ctcattagaa tttccattac attggcgtat gcctcgttta 600

gaagctcgtt ggttcatcaa tatctatcgt cataatcaag atgtaaatcc aatcctttta 660

gaatttgctg aacttgactt caatattgta caagctgctc accaagcaga tttaaaacaa 720

gtatcaacat ggtggaaatc aactggttta gtagaaaatc tttcattcgc tcgtgatcgt 780

cctgtagaaa acttcttttg gacagttggt cttattttcc aaccacaatt cggttattgt 840

cgtagaatgt ttactaaagt attcgcatta attactacaa ttgatgacgt atatgatgta 900

tatggtactt tagatgaatt agaacttttc acagacgttg ttgaaagatg ggatattaat 960

gcaatggatc aattacctga ttatatgaaa atttgctttt taacattaca caatagtgtt 1020

aacgaaatgg cattagacac tatgaaagaa caacgttttc acatcattaa ataccttaaa 1080

aaagcatggg ttgatctttg tcgttattac ttagttgaag ctaaatggta tagtaataaa 1140

tatagacctt ctttacaaga atacattgaa aatgcatgga tttcaattgg tgctccaact 1200

attttagttc atgcatattt cttcgttaca aatccaatta caaaagaagc attagactgt 1260

ttagaagaat atccaaacat tattcgttgg agtagtatta ttgcacgttt agctgatgat 1320

ttaggtactt caacagacga attaaaacgt ggtgacgtac caaaagcaat tcaatgttat 1380

atgaatgaaa caggtgcttc agaagaaggt gctcgtgagt acattaaata cttaatttct 1440

gctacttgga aaaaaatgaa caaagataga gcagcatcaa gtccattttc acatatcttc 1500

attgaaattg ctcttaattt agcacgtatg gcacaatgtt tatatcaaca cggtgacggc 1560

cacggtttag gtaaccgtga aacaaaagat cgtatacttt cattacttat tcaaccaatt 1620

ccattaaaca aagatggtac cggtgagaac ttatactttc aaggctcagg tggtggtggt 1680

tctgattaca aagatgatga tgataaagga accggttaat ctagactcga g 1731

<210> 156

<211> 1815
<212> DNA
<213> Artificial Sequence

<220>
<223> Description of Artificial Sequence: Synthetic
      polynucleotide

<400> 156
catatggtac caagaagaat tggagactat cactcaaact tatggaatga tgacttcatt      60

caatcattaa caacaccata cggtgctcca tcatatattg aacgtgctga tagattaata     120

tctgaagtaa aagaaatgtt taatagaatg tgtatggaag atggtgagtt aatgtctcca     180

ttaaatgatc ttattcaaag attatggact gttgatagtg ttgaacgttt aggtatagat     240

cgtcacttca aaaatgaaat aaaagctagt ttagattatg tatactcata ctggaacgaa     300

aaaggtatcg gttgtggtcg tcaatcagta gttacagatt taaactctac tgctcttgga     360

ttaagaattt tacgtcaaca tggttacaca gtttcaagtg aagttttaaa agtttttgaa     420

gaagaaaacg gtcaatttgc ttgttcacct tcacagactg agggcgaaat tcgttcattc     480

ttaaacttat atcgtgcttc attaattgct tttcctggtg aaaaagtaat ggaagaagct     540

caaatctttt ctagtcgtta cttaaaagaa gcagttcaga aaattccagt ttcaggttta     600

tctcgtgaaa taggcgatgt tttagaatat ggttggcaca caaacttacc tcgttgggaa     660

gctcgtaact atatggacgt attcggtcaa gacacaaata catcattcaa caaaaacaaa     720

atgcaatata tgaatacaga gaaaattctt caattagtaa aattagagtt taatatcttt     780

cattcattac aacaacgtga attacaatgt ttattacgtt ggtggaaaga aagtggtctt     840

ccacaattaa catttgcacg tcaccgtcac gttgaatttt acactttagc ttcttgtatt     900

gcatgtgaac caaaacacag tgcatttcgt ttaggttttg caaaaatgtg tcacttagta     960

acagttttag atgatgtata tgacacattt ggcaaaatgg atgaattaga actttttact    1020

gcagctgtta aacgttggga cttatcagaa actgagcgtt tacctgagta tatgaaaggt    1080

ttatatgttg tagttttcga gactgttaat gaattagcac aagaagcaga gaaaactcaa    1140

ggacgtaata cattaaatta cgttcgtaaa gcatgggaag catacttcga tagttatatg    1200

aaagaagcag aatggatctc aacaggctat ttaccaacat tcgaagagta ttgtgaaaac    1260

ggtaaagtat caagtgcata tagagttgct gcacttcaac ctattttaac attagatgta    1320

caacttccag atgacatctt aaaaggtatt gattttccat ctcgtttcaa tgatttagca    1380

tcttcatttc ttcgtttacg tggagatact agatgttacg aggctgatcg tgctcgtggt    1440

gaagaagcaa gttgtatttc ttgttacatg aaagacaatc caggttcaac tgaagaagat    1500

gcattaaatc acattaatgc tatgataaat gatattattc gtgaattaaa ctgggaattt    1560

cttaaaccag actcaaatat cccaatgcca gctcgtaaac atgctttcga tattacaaga    1620

gctttacatc acttatatat ttatcgtgac ggtttttctg ttgctaacaa agagactaaa 1680

aatcttgttg agaaaacttt attagaatca atgttattcg gtaccggtga gaacctttat 1740

tttcaaggtt caggtggtgg tggttcagat tataaagacg atgatgataa aggaaccggt 1800

taatctagac tcgag 1815

<210> 157
<211> 1746
<212> DNA
<213> Artificial Sequence

<220>
<223> Description of Artificial Sequence: Synthetic
      polynucleotide

<400> 157
catatggtac caagaagatc agctaattat caacctagtc gttgggatca tcatcacctt 60

ttaagtgtag aaaacaaatt cgctaaagat aaacgtgtaa gagaacgtga cttacttaaa 120

gaaaaagttc gtaaaatgtt aaatgacgaa cagaaaactt acttagatca attagaattt 180

attgacgatc ttcaaaaatt aggtgttagt tatcacttcg aagcagaaat agataatata 240

cttacaagtt catacaaaaa agatcgtaca aatatacaag aaagtgattt acacgcaact 300

gcattagagt ttcgtctttt tcgtcaacac ggttttaacg tttcagaaga tgtatttgat 360

gtatttatgg aaaattgtgg taaattcgac cgtgatgaca tttatggttt aatttcatta 420

tatgaagcta gttatctttc tactaaactt gacaaaaatc ttcaaatctt tatccgtcca 480

tttgctactc aacaattacg tgattttgta gatactcaca gtaatgaaga tttcggttca 540

tgtgatatgg tagaaatagt tgttcaagca ttagacatgc catactattg gcaaatgcgt 600

cgtttatcta cacgttggta tattgatgtt tatggtaaaa gacaaaatta caaaaactta 660

gtagttgttg aatttgcaaa aattgatttc aatattgttc aagctattca ccaggaagaa 720

cttaaaaatg tatcatcttg gtggatggaa actggtttag gtaaacaact ttattttgct 780

cgtgatcgta ttgtagagaa ctatttttgg acaattggtc aaattcaaga acctcaatat 840

ggatatgtta gacaaacaat gactaaaatc aatgctttat taacaacaat tgatgatatt 900

tatgatatat acggtacatt agaagaatta cagttattca cagttgcatt tgagaattgg 960

gacataaatc gtttagacga attaccagaa tatatgcgtt tatgtttctt agttatctat 1020

aacgaagtaa atagtatagc atgtgaaatt cttagaacaa aaaatattaa cgttattcct 1080

ttcttaaaaa aatcttggac tgatgtaagt aaagcatact tagttgaagc taaatggtat 1140

aaatcaggcc ataaaccaaa tttagaagag tatatgcaaa atgcacgtat ttctatttct 1200

tcaccaacaa tctttgttca cttttattgt gtattttcag accaattatc tattcaagtt 1260

ttagaaactt tatcacaaca ccaacaaaat gttgtaagat gtagttcttc tgttttccgt 1320

ttagctaatg acttagtaac ttctccagat gaattagcta gaggtgatgt ttgtaaatca 1380

attcaatgtt atatgtcaga aactggtgca agtgaagata aagctagatc acacgttcgt 1440

caaatgatta atgatttatg ggacgaaatg aattacgaga aaatggcaca ttcaagtagt 1500

atcttacatc atgattttat ggagacagta atcaatttag ctagaatgtc tcaatgtatg 1560

taccaatatg gtgacggaca cggttctcca gaaaaagcta aaattgtaga tcgtgtaatg 1620

agtttacttt tcaaccctat tcctttagat ggtaccggtg agaatttata ttttcaaggc 1680

tctggaggtg gtggttcaga ttataaagat gatgacgaca aaggaaccgg ttaatctaga 1740

ctcgag 1746

<210> 158
<211> 1728
<212> DNA
<213> Artificial Sequence

<220>
<223> Description of Artificial Sequence: Synthetic
      polynucleotide

<400> 158
catatggtac caagaagaag tgcaaactat caaccttcat tatggcaaca tgaatactta 60

ttatcattag gcaacactta tgttaaagaa gataatgttg aaagagtaac tcttttaaaa 120

caagaagttt ctaaaatgtt aaacgaaaca gaaggtttac ttgaacaact tgaattaatt 180

gacactttac aaagattagg tgtttcttat cattttgaac aggagattaa aaaaacatta 240

actaatgttc atgttaaaaa cgtacgtgct cataaaaatc gtattgatcg taaccgttgg 300

ggcgatttat atgcaactgc attagaattt cgtttattac gtcaacatgg tttttctatt 360

gctcaagacg tttttgatgg taatattggt gttgacttag acgacaaaga cattaaaggt 420

attttaagtt tatacgaagc tagttactta tcaacacgta ttgatacaaa acttaaagaa 480

tcaatctatt acacaacaaa acgtttaaga aaattcgtag aggtaaacaa aaacgaaact 540

aaaagttaca ctcttcgtcg tatggttatt cacgcacttg agatgcctta tcaccgtcgt 600

gttggtcgtc ttgaagctcg ttggtatatc gaggtatatg gagaaagaca cgacatgaat 660

cctattttat tagaattagc taaattagat tttaactttg ttcaggctat ccaccaagac 720

gaattaaaat cattatctag ttggtggtct aaaacaggat taacaaaaca tttagacttt 780

gttcgtgatc gtattacaga gggttacttc agtagtgtag gtgttatgta tgaaccagaa 840

tttgcatatc atcgtcaaat gcttacaaaa gtatttatgc ttattacaac tattgatgac 900

atctatgaca tttacggtac acttgaagaa ttacaattat tcacaactat cgttgaaaaa 960

tgggatgtta atcgtttaga agaacttcct aactatatga aattatgctt cttatgttta 1020

gttaacgaaa taaatcaaat tggatatttt gtattaagag ataaaggttt taatgtaatt 1080

ccttatctta aagagtcttg ggctgacatg tgtactacat ttcttaaaga agctaaatgg 1140

tacaaatcag gttataaacc aaattttgaa gagtatatgc aaaatggctg gatttcatca 1200

tcagttccaa ctattctttt acacttattt tgtttattaa gtgaccaaac tttagacatt 1260

cttggttctt ataatcacag tgttgttcgt agttcagcaa caattttacg tcttgcaaat 1320

gatttagcta cttcttcaga agaattagca agaggagata caatgaaatc agttcaatgt 1380

cacatgcatg aaactggtgc ttcagaagct gaatcaagag cttacattca aggtattatt 1440

ggcgtagctt gggatgacct taatatggag aaaaaatcat gtcgtttaca ccagggattc 1500

ttagaagcag cagcaaattt aggacgtgta gcacaatgcg tatatcaata tggagacggt 1560

cacggttgtc cagataaagc aaaaacagta aatcatgttc gtagtttatt agttcaccca 1620

ttaccattaa acggtaccgg tgaaaacctt tattttcaag gtagtggtgg aggtggttct 1680

gattataaag acgacgatga caaaggaacc ggttaatcta gactcgag 1728

<210> 159
<211> 1767
<212> DNA
<213> Artificial Sequence

<220>
<223> Description of Artificial Sequence: Synthetic
      polynucleotide

<400> 159
catatggtac cagcttctcc acctgctcat cgttcatcta aagcagcaga cgaagagtta 60

ccaaaagcat cttctacatt ccatccatct ctttggggtt catttttctt aacatatcag 120

ccacctacag ctccacaacg tgcaaatatg aaagaacgtg ctgaagttct tcgtgaacgt 180

gttcgtaaag tattaaaagg ttcaacaaca gatcaattac ctgaaacagt taacttaatt 240

cttacattac aaagacttgg tttaggttat tactatgaaa atgaaattga caaattactt 300

catcaaattt actctaattc agattataac gtaaaagact taaacttagt ttctcaacgt 360

ttttacttac ttcgtaaaaa cggttatgac gtaccttctg atgttttctt atcttttaaa 420

actgaagaag gtggtttcgc ttgtgctgca gctgacacac gttcactttt aagtttatac 480

aatgctgctt accttcgtaa acatggtgaa gaagtattag atgaagcaat ttcatcaaca 540

cgtttaagat tacaagactt attaggtcgt ttattacctg aatcaccatt cgctaaagaa 600

gtatcaagtt cacttcgtac acctttattc cgtcgtgtag gtattttaga agctcgtaac 660

tatattccaa tctatgaaac tgaagctaca agaaatgaag ctgtattaga gcttgctaaa 720

cttaacttca atttacaaca gcttgatttc tgtgaagaat taaaacattg tagtgcatgg 780

tggaatgaga tgattgctaa aagtaaatta acttttgtac gtgaccgtat agttgaagaa 840

tacttttgga tgaatggtgc atgttatgat ccaccatatt cattaagtcg tattattctt 900

acaaaaatca ctggtttaat tactattatt gatgatatgt tcgatactca tggtacaaca 960

gaggattgca tgaaattcgc agaagcattt ggtcgttggg atgaatcagc aattcatctt 1020

cttccagaat acatgaaaga tttttacatt ttaatgttag aaactttcca gtcatttgaa 1080

gatgcacttg gtccagaaaa atcataccgt gtattatact taaaacaagc aatggaacgt 1140

ttagtagagt tatattctaa agaaatcaaa tggcgtgatg acgattatgt tccaacaatg 1200

tcagaacatt tacaagttag tgctgaaaca attgctacaa ttgctttaac ttgctctgct 1260

tatgctggta tgggtgatat gtctattcgt aaagaaacat ttgaatgggc attatctttc 1320

cctcaattca ttagaacttt tggttcattt gtacgtttat caaatgatgt tgtatcaaca 1380

aaacgtgaac aaactaaaga tcattcacct tcaacagttc actgttatat gaaagaacac 1440

ggtacaacta tggacgatgc ttgtgaaaaa atcaaagaat taattgagga ctcatggaaa 1500

gacatgttag aacaatcttt agctcttaaa ggcttaccta aagtagtacc tcaattagtt 1560

tttgatttct ctcgtactac agataacatg tatcgtgacc gtgatgcttt aacatcatca 1620

gaagcattaa aagaaatgat acagttatta ttcgtagaac ctatacctga aggtaccggt 1680

gagaatcttt attttcaagg atcaggtggt ggaggctcag attacaaaga tgacgacgat 1740

aaaggaaccg gttaatctag actcgag 1767

<210> 160
<211> 1776
<212> DNA
<213> Artificial Sequence

<220>
<223> Description of Artificial Sequence: Synthetic
      polynucleotide

<400> 160
catatggtac cagaggcttt aggaaatttt gattatgaga gttatactaa ttttacaaaa 60

ttaccatcat cacaatgggg tgatcaattc cttaaatttt ctatagcaga ttctgacttc 120

gatgtattag aaagagaaat agaagtatta aaaccaaaag taagagagaa catttttgtt 180

tcatcaagta ctgataaaga tgcaatgaaa aaaacaattt taagtattca tttcttagat 240

agtttaggtt tatcttatca cttcgaaaaa gaaatagagg agagtttaaa acatgctttc 300

gagaaaattg aagaccttat tgctgatgaa aataaacttc atacaataag tacaattttc 360

cgtgtattcc gtacatacgg ctattatatg tcttctgatg tattcaaaat tttcaaagga 420

gacgatggta aattcaaaga aagtttaatt gaagacgtta aaggtatgct ttctttttat 480

gaagctgttc attttggaac aactactgat cacattttag acgaagctct tagttttaca 540

ttaaaccact tagagtcact tgcaacaggc cgtcgtgcat caccaccaca tattagtaaa 600

ttaatccaaa atgctttaca tattcctcaa catcgtaaca tccaggcatt agtagctcgt 660

gaatacatta gtttttacga acacgaagaa gatcacgatg aaacattatt aaaattagct 720

aaattaaact ttaaattctt acaacttcac tattttcaag aattaaaaac aattacaatg 780

tggtggacta aattagatca tacatctaat ttaccaccaa attttcgtga acgtacagtt 840

gaaacatggt ttgcagcttt aatgatgtat ttcgaaccac aatttagttt aggtcgtatt 900

atgagtgcaa aattatattt agtaattact ttcttagatg acgcatgtga tacatacgga 960

tcaatatctg aagtagagtc attagctgat tgtttagaac gttgggaccc agattatatg 1020

gaaaatttac aaggtcacat gaaaacagca ttcaaattcg ttatgtattt attcaaagaa 1080

tacgaagaaa ttttacgttc acaaggccgt tcattcgtat tagagaaaat gattgaggag 1140

tttaaaatta tcgcacgtaa aaacttagaa cttgtaaaat gggctcgtgg tggtcacgtt 1200

ccttcttttg acgaatatat agagagtggt ggtgctgaga ttggtactta tgctacaatc 1260

gcttgttcaa ttatgggtct tggtgaaatt ggtaaaaaag aagcatttga gtggttaatc 1320

tctcgtccta aacttgttcg tattttaggt gctaaaacac gtttaatgga tgatatcgca 1380

gactttgaag aagacatgga aaaaggctat acagctaatg cacttaacta ttatatgaat 1440

gaacacggag taactaaaga agaagctagt cgtgaacttg agaaaatgaa tggtgatatg 1500

aacaaaattg taaacgaaga atgtcttaaa attacaacta tgccacgtcg tatcttaatg 1560

caaagtgtta actacgctcg tagtttagat gtattataca cagctgatga tgtatataac 1620

caccgtgaag gcaaacttaa agaatatatg agattacttt tagtagatcc aattttactt 1680

ggtaccggtg aaaatcttta ttttcaaggt tcaggtggtg gtggttctga ttataaagat 1740

gatgacgata aaggaaccgg ttaatctaga ctcgag 1776

<210> 161
<211> 1677
<212> DNA
<213> Artificial Sequence

<220>
<223> Description of Artificial Sequence: Synthetic
      polynucleotide

<400> 161
catatggtac cagagagtca aacaacattc aaatacgaat cattagcatt tacaaaactt 60

agtcactgtc aatggacaga ctattttctt agtgttccaa ttgatgaaag tgaattagat 120

gttattactc gtgaaattga tattcttaaa ccagaagtta tggagttatt aagtagtcaa 180

ggagatgatg aaacaagtaa aagaaaagtt cttcttattc agttattact ttctttaggt 240

ttagcattcc actttgaaaa tgagattaaa aacatacttg aacacgcatt tcgtaaaata 300

gatgatataa ctggtgacga aaaagactta tcaacaatta gtattatgtt ccgtgttttc 360

cgtacttatg gacacaatct tccaagtagt gtttttaaac gtttcacagg tgatgatggt 420

aaatttcagc aaagtttaac agaagacgca aaaggtattt taagtttata tgaagctgca 480

catttaggta ctactacaga ttacatttta gatgaagctc ttaaattcac atctagtcac 540

ttaaaaagtt tacttgctgg tggtacatgt cgtcctcaca tcttacgttt aatccgtaat 600

acattatact taccacaacg ttggaacatg gaagctgtta tcgctcgtga atacatatca 660

ttttacgagc aggaagaaga tcacgataaa atgcttttac gtcttgcaaa acttaacttt 720

aaacttcttc aattacacta cattaaagag cttaaaagtt tcattaaatg gtggatggaa 780

cttggtttaa cttctaaatg gccttctcaa tttcgtgaac gtattgttga agcatggtta 840

gctggattaa tgatgtattt tgaaccacag ttctcaggtg gtcgtgttat tgctgcaaaa 900

ttcaactatt tacttacaat attagacgac gcatgtgacc actatttttc tattcacgaa 960

ttaacacgtt tagttgcatg tgtagaacgt tggtcaccag atggtattga cacattagaa 1020

gatatttcac gttctgtatt caaattaatg ttagatgttt tcgacgatat tggtaaaggt 1080

gtacgttcag aaggttctag ttaccactta aaagaaatgt tagaggaatt aaacacttta 1140

gttcgtgcta atttagattt agttaaatgg gctcgtggaa tacaaacagc tggtaaagag 1200

gcttatgaat gggttcgttc acgtccacgt ttaatcaaat ctttagcagc taaaggtaga 1260

cttatggatg atattacaga ctttgactca gatatgagta atggattcgc agctaatgct 1320

attaactact atatgaaaca atttgttgtt acaaaagaag aagctattct tgaatgtcaa 1380

cgtatgattg tagacattaa caaaactatt aatgaagagt tattaaaaac tacttcagtt 1440

ccaggtcgtg tattaaaaca agctcttaac tttggccgtt tattagaatt attatataca 1500

aaatctgacg atatttacaa ttgttctgaa ggcaaactta aagaatacat tgtaactctt 1560

ttaattgatc ctataagact tggtaccggt gaaaacttat actttcaagg ttcaggcggt 1620

ggtggtagtg attacaaaga tgatgatgac aaaggaaccg gttaatctag actcgag 1677

<210> 162
<211> 1518
<212> DNA
<213> Artificial Sequence

<220>
<223> Description of Artificial Sequence: Synthetic
      polynucleotide

<400> 162
catatggtac cagagagtca aacaaaattc gactacgaat cattagcttt tacaaaatta 60

tcacattcac aatggactga ttacttttta tcagtaccta tagacgactc tgaacttgac 120

gcaattactc gtgaaatcga cattatcaaa cctgaagttc gtaaattact ttcaagtaaa 180

ggtgatgatg aaacttctaa acgtaaagta ttacttatcc aaagtttatt atcattaggt 240

ttagcatttc attttgaaaa cgaaattaaa gatattttag aagatgcatt tagacgtatt 300

gatgacatta caggtgatga aaacgactta agtactatta gtattatgtt ccgtgtattc 360

cgtacatacg gtcacaattt accaagtagt gtttttaaac gtttcactgg tgatgacggt 420

aaatttgaac gttctttaac tgaagatgct aaaggaattt tatcattata tgaagctgca 480

catttaggaa caactactga ttatattctt gatgaagcat tagaatttac ttcatcacac 540

ttaaaatctt tacttgttgg tggtatgtgt cgtccacata ttttacgtct tattagaaat 600

actttatatc ttccacaacg ttggaatatg gaagcagtaa ttgcaagaga atacattagt 660

ttttatgaac aagaagaaga tcacgataaa atgttacttc gtttagctaa attaaatttc 720

aaattacttc aattacacta cattaaagag ttaaaaacat tcattaaatg gtggatggaa 780

ttaggactta catcaaaatg gccttctcaa tttcgtgaac gtattgttga agcatggtta 840

gctggtctta tgatgtattt tgaaccacag ttttctggag gtcgtgtaat agctgctaaa 900

ttcaattact tattaacaat tttagatgat gcatgtgatc actatttctc aattccagaa 960

ttaactcgtt tagttgattg cgtagaaaga tggaatcatg atggtataca tactttagaa 1020

gacatctcac gtatcatctt taaacttgca ttagatgtat ttgatgatat tggtcgtggt 1080

gttcgttcta aaggttgttc ttattactta aaagaaatgt tagaagagtt aaaaatctta 1140

gttcgtgcaa acttagattt agttaaatgg gctcgtggta atcaattacc tagttttgaa 1200

gaacacgttg aggtaggtgg tattgctctt acaacatacg caactttaat gtactctttt 1260

gttggcatgg gtgaagcagt aggtaaagaa gcatacgaat gggtacgttc tcgtccacgt 1320

ttaatcaaaa gtttagcagc aaaaggtcgt cttatggacg atattactga tttcgaagta 1380

aaaattatca acttattttt cgaccttctt ttatttgtat tcggtaccgg tgaaaactta 1440

tatttccagg gtagtggtgg aggaggttca gactacaaag atgacgatga caaaggaacc 1500

ggttaatcta gactcgag 1518

<210> 163
<211> 1740
<212> DNA
<213> Artificial Sequence

<220>
<223> Description of Artificial Sequence: Synthetic
      polynucleotide

<400> 163
catatggtac cagcagcttt cacagcaaat gcagttgaca tgcgtccacc agttattaca 60

attcacccac gttcaaaaga tattttctct caattttctt tagatgataa attacaaaaa 120

caatacgctc aaggaatcga agctcttaaa gaagaagctc gttctatgct tatggctgca 180

aaatctgcta aagtaatgat cttaattgat acacttgaac gtttaggatt aggttatcac 240

tttgaaaaag aaattgaaga gaaattagaa gctatttaca aaaaagagga tggtgacgat 300

tatgatcttt ttacaactgc tttaagattc cgtttactta gacaacacca acgtcgtgta 360

ccatgttctg tttttgacaa atttatgaat aaagagggta aattcgaaga agaaccatta 420

atttcagatg ttgaaggtct tctttcatta tatgacgctg cttatttaca gattcacggt 480

gaacacattt tacaagaggc tttaattttc actacacatc atttaactcg tattgaacca 540

caattagatg atcactctcc tttaaaatta aaattaaacc gtgctttaga atttcctttt 600

tacagagaaa tccctataat ctatgcacat ttttacattt cagtatatga acgtgacgat 660

tctcgtgatg aagtattatt aaaaatggct aaattatctt ataatttctt acaaaactta 720

tacaaaaaag aattaagtca actttctcgt tggtggaaca aattagaact tattcctaat 780

ttaccttata ttcgtgattc tgtagctgga gcttatttat gggctgttgc tttatatttc 840

gaacctcaat attcagacgt tcgtatggca attgctaaac ttatccaaat tgcagcagct 900

gtagatgata cttacgataa ttatgctact atacgtgaag ctcaattatt aacagaagca 960

ttagaacgtt taaatgtaca cgaaattgac acattaccag attatatgaa aattgtttat 1020

cgttttgtaa tgtcatggag tgaagatttc gaacgtgatg ctacaattaa agaacagatg 1080

ttagctacac cttatttcaa agctgaaatg aaaaaacttg gtcgtgctta taatcaagaa 1140

cttaaatggg ttatggaacg tcaattacct agtttcgaag aatacatgaa aaactctgaa 1200

atcacttctg gtgtttacat tatgtttact gtaattagtc cttacttaaa tagtgcaaca 1260

caaaaaaaca ttgactggtt attatcacaa cctcgtttag catcttcaac tgcaattgtt 1320

atgcgttgtt gtaatgattt aggctctaat caacgtgaat ctaaaggagg agaagttatg 1380

acatctttag attgctatat gaaacaacac ggtgctagta aacaagaaac aatttctaaa 1440

ttcaaactta ttatcgaaga tgaatggaaa aacttaaatg aagaatgggc tgcaacaaca 1500

tgtcttccaa aagttatggt agaaattttt cgtaactatg cacgtattgc aggcttttgc 1560

tacaaaaata acggtgatgc ttatacatct ccaaaaattg tacaacaatg ttttgacgct 1620

ttatttgtaa atccattaag aattggtacc ggtgagaatt tatactttca aggctcaggt 1680

ggaggtggta gtgattataa agatgatgat gataaaggaa ccggttaatc tagactcgag 1740

<210> 164
<211> 1833
<212> DNA
<213> Artificial Sequence

<220>
<223> Description of Artificial Sequence: Synthetic
      polynucleotide

<400> 164
catatggtac cagaatttag agttcattta caggctgata atgaacagaa aatattccag 60

aaccaaatga aacctgaacc tgaagcatca tatcttatta atcaacgtag atcagctaat 120

tacaaaccta atatttggaa aaatgacttt ttagatcaaa gtttaattag taaatacgac 180

ggtgatgaat atcgtaaatt aagtgagaaa ttaatcgagg aagtaaaaat ttatatatct 240

gctgagacaa tggacttagt agctaaatta gaacttattg attctgttcg taaattaggt 300

ttagctaatc tttttgaaaa agaaattaaa gaagcattag attctatcgc agctattgag 360

tcagataatt taggtactcg tgatgactta tatggtactg ctttacactt taaaatttta 420

cgtcaacatg gttataaagt ttctcaagat atttttggtc gtttcatgga tgaaaaaggt 480

acattagaaa atcatcactt cgctcactta aaaggtatgt tagaattatt tgaagcatct 540

aatttaggtt ttgaaggtga agatatttta gatgaagcaa aagcatcact tacattagct 600

cttcgtgata gtggtcatat ttgttatcca gattctaact taagtcgtga tgtagtacac 660

tcattagaat tacctagtca ccgtcgtgtt caatggtttg atgttaaatg gcaaattaat 720

gcttatgaaa aagatatttg tagagttaat gcaactcttt tagaattagc aaaattaaat 780

tttaacgtag tacaagcaca acttcaaaaa aacttacgtg aagcatctcg ttggtgggct 840

aacttaggtt tcgctgataa cttaaaattc gctcgtgatc gtttagttga atgtttttct 900

tgcgcagtag gcgtagcatt tgaacctgaa cactcttctt ttcgtatctg tttaacaaaa 960

gttattaatt tagttttaat aattgatgac gtatacgaca tatatggaag tgaagaagaa 1020

ttaaaacact ttacaaatgc tgttgatcgt tgggattctc gtgaaacaga acaattacca 1080

gaatgtatga aaatgtgctt tcaagtttta tacaatacta catgtgaaat tgctcgtgaa 1140

attgaagaag aaaatggatg gaatcaagtt ttacctcaat taactaaagt atgggctgat 1200

ttttgtaaag cattattagt agaagctgaa tggtacaata aaagtcacat cccaacttta 1260

gaagaatatc ttcgtaatgg ctgtatttca tcaagtgttt ctgtattatt agtacattct 1320

ttctttagta ttacacatga aggtacaaaa gaaatggcag atttcttaca caaaaacgaa 1380

gacttattat acaacatctc attaattgta cgtttaaaca acgacttagg tacaagtgca 1440

gctgaacaag aacgtggtga ttcaccatca tctattgtat gttacatgcg tgaagttaat 1500

gctagtgaag aaacagctcg taaaaatata aaaggaatga tcgacaatgc ttggaaaaaa 1560

gttaatggta aatgttttac aactaatcaa gttccttttc tttcttcttt tatgaataac 1620

gctactaata tggctcgtgt agctcattca ttatataaag acggagacgg ttttggcgat 1680

caggaaaaag gtccacgtac tcacatctta tctttattat tccaaccatt agttaacggt 1740

accggtgaaa acttatactt tcaaggttct ggtggtggtg gttctgacta caaagatgac 1800

gatgacaaag gaaccggtta atctagactc gag 1833

<210> 165
<211> 1788
<212> DNA
<213> Artificial Sequence

<220>
<223> Description of Artificial Sequence: Synthetic
      polynucleotide

<400> 165
catatggtac caagtagtaa tgtatcagct attcctaatt cttttgaatt aattcgtcgt      60

tcagctcaat ttcaggcttc tgtatggggt gattactttt tatcttatca ctctttacca     120

cctgagaaag gtaataaagt aatggaaaaa caaactgaag aacttaaaga ggaaatcaaa     180

atggaattag tttctactac taaagatgaa ccagagaaat tacgtttaat tgaccttatt     240

caacgtttag gtgtatgtta tcactttgaa aatgaaatta acaacatttt acaacaatta     300

caccacatta ctattacttc tgagaaaaac ggtgacgata atccttataa catgacttta     360

tgtttccgtt tattacgtca acaaggttac aatgtatcta gtgaaccttt tgatcgtttt     420

cgtggcaaat gggaatcttc ttatgataac aatgtagaag aacttttatc attatatgaa     480

gcatctcaat taagaatgca aggtgaagaa gcattagatg aagcattctg ttttgcaact     540

gcacaattag aagctattgt tcaagatcct actacagatc caatggttgc agcagaaatc     600

agacaagcat taaaatggcc aatgtacaaa aacttacctc gtttaaaagc tcgtcatcat     660

attggtttat attctgagaa accatggcgt aatgagtcat tacttaattt cgcaaaaatg     720

gacttcaata aacttcaaaa tttacatcaa actgaaattg catatatttc taaatggtgg     780

gacgattacg gctttgcaga aaaactttct ttcgcacgta atcgtattgt tgaaggctat     840

ttcttcgcat taggtatctt tttcgaacct caacttttaa cagcacgtct tataatgaca     900

aaagtaatcg ctattggttc tatgttagat gacatttatg atgtttatgg tacttttgaa     960

gagttaaaac ttttaacatt agctttagaa cgttgggata aatcagaaac aaaacaatta    1020

cctaattaca tgaaaatgta ctacgaagca ttattagatg tttttgaaga aattgagcaa    1080

gaaatgtcac aaaaagaaac tgaaacaaca ccatactgta ttcatcacat gaaagaagct    1140

actaaagaac ttggacgtgt atttttagtt gaagcaactt ggtgtaaaga aggttatact    1200

cctaaagtag aggaatactt agacattgct ttaatttctt ttggtcataa attacttatg    1260

gtaactgctt tattaggtat gggttctcac atggctacac aacaaattgt acaatggatt    1320

acatctatgc caaatatctt aaaagcatct gcagtaatat gtcgtttaat gaatgacatt    1380

gtatctcata aatttgaaca agaacgtggt catgttgctt ctgctatcga atgctacatg    1440

gaacaaaacc accttagtga atatgaagca ttaattgctc ttcgtaaaca aattgatgat    1500

ttatggaaag acatggtaga aaattactgt gcagtaatca cagaagacga agtacctcgt    1560

ggtgttttaa tgcgtgtttt aaatcttaca cgtttattca atgttattta caaagacggt    1620

gatggataca cacaaagtca tggtagtaca aaagctcaca ttaaaagtct tttagttgat    1680

agtgtacctc ttggtaccgg tgaaaatctt tactttcaag gttcaggtgg aggtggttct    1740

gattataaag atgatgatga caaaggaacc ggttaatcta gactcgag 1788

<210> 166
<211> 1785
<212> DNA
<213> Artificial Sequence

<220>
<223> Description of Artificial Sequence: Synthetic
      polynucleotide

<400> 166
catatggtac caaaagacat gagtattcca ttattagcag ctgtatcttc tagtacagaa 60

gaaacagtac gtcctatcgc agattttcat ccaacacttt ggggtaatca ttttcttaaa 120

tctgctgctg acgtagaaac tattgatgca gcaacacaag agcaacacgc tgcattaaaa 180

caagaagtac gtcgtatgat tactacaaca gcaaataaac ttgcacaaaa acttcacatg 240

attgatgctg tacaacgttt aggtgttgct tatcattttg aaaaagaaat tgaagacgaa 300

ttaggtaaag taagtcacga tttagattca gatgatttat acgttgtatc tttacgtttt 360

cgtttattcc gtcaacaagg tgtaaaaatt agttgcgatg ttttcgacaa attcaaagat 420

gacgaaggaa aattcaaaga gtctcttatt aacgatatta gaggaatgtt atcattatac 480

gaagcagctt acttagctat tagaggtgaa gatattttag acgaagcaat tgttttcaca 540

actactcact taaaaagtgt tatctctatt agtgatcatt cacatgctaa tagtaattta 600

gctgaacaaa tacgtcatag tttacaaatt ccacttcgta aagctgctgc aagattagaa 660

gcacgttatt tcttagatat ttactctcgt gatgatttac atgatgaaac attacttaaa 720

ttcgctaaac ttgactttaa cattcttcaa gctgcacacc aaaaagaagc tagtattatg 780

actcgttggt ggaacgattt aggttttcct aaaaaagttc cttatgctcg tgaccgtatt 840

atagaaactt atatttggat gttattagga gtttcatacg aacctaattt agcatttgga 900

agaatttttg caagtaaagt agtatgtatg attacaacaa ttgatgatac atttgatgct 960

tatggtacat ttgaagagtt aacattattc actgaagctg ttacacgttg ggatattggt 1020

ttaattgaca cattacctga atatatgaaa ttcattgtaa aagctctttt agacatttac 1080

cgtgaagctg aagaagaatt agctaaagaa ggtagatcat acggtattcc atacgctaaa 1140

caaatgatgc aagagttaat cattttatac tttactgagg ctaaatggtt atacaaaggt 1200

tacgttccta catttgacga atacaaaagt gtagctttac gttctattgg tcttagaaca 1260

ttagcagtag cttcatttgt agatttaggt gactttattg ctacaaaaga caattttgaa 1320

tgtattctta aaaatgcaaa aagtttaaaa gctactgaaa caattggccg tttaatggat 1380

gatatagctg gttacaaatt tgaacagaaa cgtggtcata acccatctgc tgttgagtgt 1440

tacaaaaatc aacacggagt atcagaagaa gaagcagtta aagagctttt attagaagtt 1500

gcaaacagtt ggaaagatat taacgaggaa cttttaaatc caactacagt tccattacct 1560

atgttacagc gtttattata ttttgctcgt tcaggtcact tcatctatga tgatggacat 1620

gatcgttata cacattcttt aatgatgaaa agacaagttg cacttttatt aactgaacct 1680

ttagctattg gtaccggtga aaacttatac tttcaaggtt caggtggtgg tggatctgat 1740

tataaagatg atgatgacaa aggaaccggt taatctagac tcgag 1785

<210> 167
<211> 1845
<212> DNA
<213> Artificial Sequence

<220>
<223> Description of Artificial Sequence: Synthetic
      polynucleotide

<400> 167
catatggtac cagatttagc tgttgagatt gcaatggact tagctgttga tgacgttgag 60

cgtcgtgtag gtgactatca tagtaacctt tgggatgatg attttattca gagtttatca 120

acaccatacg gcgcatcatc atatcgtgaa cgtgctgaaa gattagtagg agaagttaaa 180

gaaatgttta cttctatttc tatcgaagat ggtgaactta catctgattt attacaacgt 240

ttatggatgg tagataatgt agagcgttta ggcatttcac gtcatttcga gaacgaaata 300

aaagcagcta ttgattatgt ttattcatat tggagtgaca aaggtattgt acgtggtcgt 360

gattcagctg ttcctgactt aaatagtatt gctttaggtt ttcgtacatt acgtttacac 420

ggttacacag ttagtagtga tgtatttaaa gttttccaag atcgtaaagg tgaatttgct 480

tgcagtgcaa ttccaactga aggagatatt aaaggagttt taaacttact tcgtgcaagt 540

tatattgcat tccctggtga aaaagtaatg gaaaaagctc aaacttttgc agcaacatac 600

cttaaagaag cattacagaa aattcaagta agtagtttaa gtcgtgaaat cgaatatgtt 660

cttgaatacg gttggttaac taactttcct cgtttagaag cacgtaacta tattgacgta 720

ttcggtgaag aaatttgtcc atacttcaaa aaaccatgta ttatggttga caaactttta 780

gaattagcaa aattagaatt taacttattt cacagtcttc aacaaacaga gttaaaacat 840

gttagtcgtt ggtggaaaga tagtggtttc tctcaattaa catttacaag acaccgtcat 900

gttgagtttt atacattagc tagttgtata gcaattgaac caaaacacag tgcttttcgt 960

cttggttttg ctaaagtttg ttatttaggt atagttttag atgatattta tgacacattt 1020

ggtaaaatga aagaattaga actttttact gcagcaatca aacgttggga cccttctact 1080

acagaatgct tacctgaata catgaaaggt gtttatatgg ctttttacaa ttgtgttaat 1140

gaattagcac ttcaagcaga gaaaacacaa ggtcgtgata tgttaaacta tgcacgtaaa 1200

gcatgggaag ctctttttga tgcattttta gaagaagcaa aatggatctc ttctggctat 1260

ttaccaacat tcgaagaata cttagaaaat ggtaaagtat cttttggtta tcgtgctgct 1320

acattacaac caattttaac attagatatt cctttacctt tacatatttt acaacagatt 1380

gattttccaa gtcgttttaa tgatttagct tcatctattt tacgtttaag aggtgatatc 1440

tgtggttacc aagctgaacg tagtcgtggt gaagaagcat catcaatttc atgttatatg 1500

aaagataatc caggttctac tgaagaagat gcattatctc acattaatgc aatgatctca 1560

gacaatatta acgaattaaa ctgggaactt ttaaaaccaa attcaaatgt accaatttca 1620

tcaaaaaaac atgcatttga cattcttcgt gctttctatc acttatacaa atatcgtgat 1680

ggcttctcta tcgcaaaaat tgaaactaaa aatcttgtaa tgcgtacagt tttagaacct 1740

gtaccaatgg gtaccggtga aaacttatac tttcagggtt ctggtggagg tggttcagac 1800

tataaagatg atgatgataa aggaaccggt taatctagac tcgag 1845

<210> 168
<211> 2526
<212> DNA
<213> Artificial Sequence

<220>
<223> Description of Artificial Sequence: Synthetic
      polynucleotide

<400> 168
catatggtac caacaagtgt atcagtagaa tcaggaacag tatcttgttt atcatcaaac 60

aacttaatta gacgtacagc taatccacat cctaacattt ggggatatga ttttgttcac 120

tcacttaaat caccatatac acacgactca tcatatcgtg aacgtgctga gactttaatt 180

tcagaaataa aagttatgct tggaggtggt gaattaatga tgactccatc agcttatgat 240

acagcatggg tagctcgtgt tccatcaatt gacggtagtg cttgtccaca atttccacaa 300

actgttgaat ggattcttaa aaaccaatta aaagatggta gttggggaac tgaatctcac 360

ttcttactta gtgacagatt attagctaca ttaagttgtg tattagcatt attaaaatgg 420

aaagtagctg atgttcaagt agagcaaggt attgagttta tcaaacgtaa tttacaagct 480

attaaagacg aacgtgatca agacagttta gtaactgatt tcgagattat tttcccatca 540

cttttaaaag aggctcaatc tttaaactta ggcttacctt atgatttacc atatattaga 600

ttattacaaa caaaacgtca agaacgtctt gctaacttaa gtatggataa aattcacggt 660

ggtactttat tatcatcttt agagggcatt caagatatag ttgaatggga aacaattatg 720

gatgtacaat ctcaagatgg ttctttctta tcatcaccag cttctacagc atgtgtattc 780

atgcatacag gagatatgaa atgtttagat ttcttaaaca acgtattaac taaatttggt 840

agtagtgttc cttgtttata ccctgtagat ttattagaac gtcttttaat tgtagataat 900

gtagagcgtc ttggtattga ccgtcatttt gaaaaagaaa tcaaagaggc tttagattat 960

gtttatcgtc attggaacga tcgtggtatt ggttggggtc gtttatcacc tatcgcagac 1020

ttagaaacaa cagctttagg ttttcgttta cttcgtcttc atcgttacaa tgtttctcct 1080

gtagtattag acaatttcaa agacgcagat ggcgagttct tctgcagtac aggtcaattt 1140

aacaaagatg ttgcaagtat gttatcttta taccgtgctt ctcaattagc tttccctgaa 1200

gaatcaattt tagatgaagc taaatcattc tcaacacaat atcttcgtga agcattagaa 1260

aaatcagaaa cattttcttc ttggaatcat cgtcagagtt tatcagaaga aattaaatat 1320

gctttaaaaa catcatggca cgcttcagtt cctcgtgttg aagcaaaacg ttattgtcag 1380

gtttaccgtc aagactatgc tcatttagca aaatcagttt ataaacttcc taaagtaaat 1440

aatgagaaaa ttcttgaatt agcaaaatta gattttaaca ttattcaatc tatccatcaa 1500

aaagaaatga aaaatgttac atcatggttt cgtgattcag gcttaccact tttcacattt 1560

gctcgtgaaa gacctttaga gttttacttt ttaatcgctg gtggaacata cgaacctcaa 1620

tacgcaaaat gtagattctt atttacaaaa gtagcttgtt tacaaactgt tttagacgat 1680

atgtacgata cttacggtac accatcagag ttaaaattat ttactgaggc agttcgtcgt 1740

tgggatttat cattcacaga aaacttacct gattatatga aattatgcta caaaatttac 1800

tatgatattg ttcatgaagt tgcttgggaa gtagaaaaag aacagggacg tgagcttgtt 1860

tcatttttcc gtaaaggttg ggaagactat cttttaggtt attatgaaga agctgaatgg 1920

ttagctgctg aatacgttcc tactttagat gaatacatta aaaacggtat tacatctatt 1980

ggtcaacgta ttttactttt atcaggtgta cttattatgg aaggtcaact tttatcacaa 2040

gaagctcttg aaaaagtaga ttatccaggt cgtcgtgttt taacagaatt aaacagttta 2100

attagtcgtt tagcagacga tactaaaaca tacaaagcag aaaaagctcg tggtgaactt 2160

gctagtagta ttgaatgtta tatgaaagac caccctggtt gtcaagaaga agaagcatta 2220

aaccatattt atggcatttt agaaccagct gttaaagaat taactcgtga gtttcttaaa 2280

gcagatcacg taccattccc ttgcaaaaaa atgttatttg atgaaacaag agttacaatg 2340

gtaattttca aagatggtga tggtttcggt atttctaaat tagaagtaaa agaccacata 2400

aaagaatgtt taattgagcc attaccactt ggtaccggtg aaaatcttta ttttcaaggt 2460

agtggtggtg gcggttctga ctacaaagat gacgacgata aaggaaccgg ttaatctaga 2520

ctcgag 2526

<210> 169
<211> 1746
<212> DNA
<213> Artificial Sequence

<220>
<223> Description of Artificial Sequence: Synthetic

polynucleotide

<400> 169
catatggtac caggttctga agtaaataga cctttagcag actttccagc aaacatttgg 60

gaagacccat taacttcttt ctcaaaatct gatcttggta cagaaacatt taaagagaaa 120

catagtactt taaaagaagc tgttaaagag gcatttatga gttctaaagc taatccaatc 180

gaaaatatca aattcataga tgcattatgc cgtttaggag tatcttatca ctttgaaaaa 240

gatattgtag aacaattaga taaatcattt gattgcttag attttccaca aatggtacgt 300

caagaaggtt gcgatttata tacagttggt attatctttc aagtttttag acaatttggt 360

ttcaaattaa gtgctgatgt ttttgaaaaa ttcaaagatg aaaatggtaa attcaaaggt 420

cacttagtaa ctgatgctta tggtatgtta tcattatacg aagctgcaca atggggtact 480

cacggtgaag acatcattga cgaagctctt gctttttctc gtagtcactt agaagaaata 540

tctagtcgta gttcaccaca cttagcaatt cgtattaaaa acgctttaaa acatccatat 600

cataaaggta tttcacgtat tgaaacacgt caatacatta gttactatga agaagaagaa 660

tcttgtgatc caacattatt agagttcgct aaaattgact ttaacttatt acaaatttta 720

caccgtgaag agttagcttg tgtaactcgt tggcatcatg aaatggaatt taaaagtaaa 780

gtaacttaca cacgtcatcg tattacagaa gcatatttat ggagtcttgg aacatatttt 840

gaaccacaat acagtcaagc tcgtgtaata actacaatgg cattaatctt atttactgct 900

ttagacgaca tgtacgatgc ttacggtact atggaggagt tagagttatt cacagatgct 960

atggacgaat ggttaccagt tgttccagat gaaattccta ttccagattc aatgaaattc 1020

atttacaatg ttacagttga attttacgat aaattagacg aagaattaga aaaagaaggt 1080

cgttctggtt gtggtttcca tcttaaaaaa agtttacaaa aaacagctaa tggatatatg 1140

caagaagcaa aatggcttaa aaaagattac attgctacat ttgatgagta taaagaaaat 1200

gctattttat cttcaggtta ttatgcatta attgcaatga catttgttcg tatgactgat 1260

gttgctaaat tagatgcttt tgaatggtta agtagtcacc caaaaattcg tgtagcaagt 1320

gaaatcattt cacgttttac agacgatatt tcaagttatg aatttgaaca caaacgtgaa 1380

cacgttgcta caggtattga ttgttatatg caacaattcg gagttagtaa agaacgtgct 1440

gttgaagtta tgggcaatat agtttctgat gcatggaaag acttaaatca agaacttatg 1500

cgtcctcatg ttttcccatt tccacttctt atgcgtgttt taaatctttc aagagtaatt 1560

gatgtatttt atcgttacca agatgcatat actaatccaa aattacttaa agagcacatt 1620

gtttctttac ttattgaaac tattccaatt ggtaccggtg aaaacttata ctttcaaggt 1680

agtggtggag gtggttctga ttataaagac gacgatgaca aaggaaccgg ttaatctaga 1740

ctcgag 1746

<210> 170
<211> 1788
<212> DNA
<213> Artificial Sequence

<220>
<223> Description of Artificial Sequence: Synthetic
      polynucleotide

<400> 170
catatggtac cagaggcaat tagagtattt ggcttaaaac ttggttcaaa attatctatt      60

cactcacaaa caaatgcttt tcctgcattc aaattatctc gttttccatt aacatctttc     120

cctggtaaac atgctcactt agatccatta aaagcaacaa ctcatccatt agcttttgat     180

ggtgaagaaa ataaccgtga gtttaaaaac ttaggtccaa gtgagtgggg ccatcaattt     240

ctttctgctc atgtagattt atctgaaatg gatgcattag aacgtgaaat tgaagctctt     300

aaaccaaaag tacgtgatat gttaatatca agtgaaagtt caaaaaaaaa aatcttattt     360

ctttatcttt tagtatcatt aggattagct tatcactttg aagatgaaat taaagaaagt     420

ttagaggatg gattacagaa aattgaggaa atgatggctt cagaagatga tcttcgtttt     480

aaaggcgata atggtaaatt caaagaatgt ttagcaaaag atgctaaagg tattttatct     540

ctttatgagg ctgctcacat gggtacaaca actgattata ttcttgatga ggctttatca     600

tttactttaa catatatgga atcattagca gcttcaggaa catgtaaaat caacttatca     660

cgtcgtatta gaaaagcatt agatcaacct caacacaaaa atatggaaat aattgtagca     720

atgaaataca ttcaatttta tgaagaagag gaagattgcg ataaaacttt acttaaattt     780

gctaaactta actttaaatt cttacaatta cactatttac aagaacttaa aatcttatct     840

aaatggtata aagaccaaga ctttaaatca aaattacctc catatttccg tgaccgtctt     900

gtagaatgtc attttgcatc attaacatgt tttgagccta aatatgctcg tgcacgtatt     960

ttcttatcta aaatcttcac tgttcaaatt ttcattgacg atacttgtga ccgttacgca    1020

tcattaggtg aagttgagtc attagctgac actatcgaac gttgggaccc tgatgatcat    1080

gctatggacg gattacctga ttatcttaaa tcagtagtta aatttgtatt caatacattt    1140

caagaatttg aacgtaaatg taaacgttca cttcgtatta acttacaagt agcaaaatgg    1200

gttaaagctg gtcacttacc atcttttgat gagtatcttg atgtagctgg tttagaatta    1260

gctatttcat tcactttcgc tggtatctta atgggcatgg aaaatgtttg taaacctgaa    1320

gcatacgaat ggttaaaatc tcgtgacaaa cttgttcgtg gtgtaatcac aaaagttcgt    1380

ttacttaatg atattttggg ctatgaagat gatatgcgtc gtggttatgt aacaaattca    1440

ataaactgct acaaaaaaca atatggagta acagaggaag aagctattcg taaattacat    1500

caaatcgttg ctgatggaga gaaaatgatg aatgaagagt tcttaaaacc tattaatgta    1560

ccatatcagg ttcctaaagt agttatttta gacactttac gtgcagctaa tgtttcatac 1620

gaaaaagatg acgaatttac acgtccaggc gaacacctta aaaactgcat tacatctatt 1680

tacttcgatt taggtaccgg tgaaaactta tactttcaag gtagtggtgg cggtggtagt 1740

gattacaaag atgatgatga taaaggaacc ggttaatcta gactcgag 1788

<210> 171
<211> 1143
<212> DNA
<213> Artificial Sequence

<220>
<223> Description of Artificial Sequence: Synthetic
      polynucleotide

<400> 171
catatggtac caactacaac attatcatct aaccttaact cacaattcat gcaggtttac 60

gagactctta aatcagaact tattcatgac ccattatttg agttcgatga cgattcaaga 120

caatgggtag aacgtatgat tgattatact gtaccaggtg gtaaaatggt tcgtggttat 180

agtgtagtag atagttatca attacttaaa ggtgaagaac ttacagaaga agaggcattt 240

ttagcttgtg cacttggttg gtgtacagaa tggtttcaag cattcattct tttacatgat 300

gatatgatgg atggtagtca cacaagacgt ggtcaaccat gttggtttcg tttacctgag 360

gttggtgctg ttgctattaa tgatggtgtt ttacttcgta atcacgttca ccgtattctt 420

aaaaaacatt ttcaaggtaa agcatattat gttcatttag ttgatttatt caatgaaact 480

gaatttcaaa caattagtgg acaaatgatc gacttaatta caacattagt tggtgaaaaa 540

gacttatcta aatattcatt aagtattcat cgtcgtatcg ttcaatacaa aacagcatac 600

tactcatttt acttaccagt tgcttgtgct ttacttatgt ttggtgagga tcttgataaa 660

catgtagaag ttaaaaatgt tcttgttgaa atgggtacat attttcaagt tcaagatgat 720

tatttagatt gttttggtgc tccagaagtt attggcaaaa ttggtactga tattgaagac 780

tttaaatgtt catggttagt agttaaagca ttagaattag caaatgaaga acagaaaaaa 840

actttacacg aaaattatgg aaaaaaagat ccagcatcag ttgctaaagt taaagaagta 900

taccacacac ttaatttaca agctgttttc gaagattatg aagcaacatc atacaaaaaa 960

cttattactt ctattgaaaa tcacccatct aaagctgttc aagctgtttt aaaatctttc 1020

ttaggcaaaa tatacaaacg tcaaaaaggt accggtgaaa acttatactt tcaaggttct 1080

ggtggcggtg gaagtgatta caaagatgat gacgataaag gaaccggtta atctagactc 1140

gag 1143

<210> 172
<211> 1818
<212> DNA

<213> Artificial Sequence

<220>
<223> Description of Artificial Sequence: Synthetic
      polynucleotide

<400> 172
catatggtac caagtcaacc ttactgggct gcaattgaag cagacattga aagatattta      60

aaaaaatcaa ttacaattcg tccaccagaa actgtatttg gtcctatgca ccatttaaca     120

tttgctgctc ctgctactgc agctagtaca ttatgccttg ctgcttgtga attagttggc     180

ggtgatcgta gtcaagctat ggcagctgct gctgctatcc atttagttca tgcagctgct     240

tacgttcacg aacatcttcc tttaacagat ggatcacgtc ctgtaagtaa acctgctatt     300

caacataaat atggtccaaa cgttgaactt ttaacaggtg atggtatcgt tcctttcggt     360

tttgagttat tagcaggttc agtagatcca gcacgtactg atgaccctga tcgtatttta     420

cgtgtaatta ttgaaatttc tcgtgctggt ggaccagaag gcatgatttc tggtttacac     480

cgtgaggaag aaatcgtaga tggtaacaca tcattagact ttatagaata tgtatgcaaa     540

aaaaaatacg gtgaaatgca cgcatgtggt gcagcttgcg gagctatttt aggtggagct     600

gctgaagaag aaattcaaaa acttcgtaac tttggtcttt atcaaggcac attacgtggt     660

atgatggaaa tgaaaaatag tcatcagtta attgacgaaa atatcattgg aaaacttaaa     720

gaacttgctc ttgaagaatt aggtggattc cacggtaaaa acgctgaatt aatgagttct     780

ttagttgctg aacctagttt atatgcagct tcatcaaata acttaggtat cgaaggtcgt     840

tttgactttg acggttacat gcttcgtaaa gcaaaatctg taaataaagc attagaagct     900

gctgttcaaa tgaaagaacc acttaaaatt cacgaatcaa tgcgttattc attattagct     960

ggtggtaaac gtgttcgtcc aatgttatgt attgcagctt gtgaacttgt tggtggtgac    1020

gaatctacag caatgcctgc agcatgtgct gttgaaatga ttcacacaat gtctttaatg    1080

catgatgacc ttccatgtat ggataacgat gacttacgtc gtggtaaacc tacaaaccac    1140

atggcttttg gtgagtctgt agctgttctt gctggtgatg cattacttag ttttgctttt    1200

gaacatgttg ctgctgcaac aaaaggcgca ccacctgaac gtatcgtacg tgtattaggt    1260

gaattagctg ttagtattgg ttcagaagga cttgtagcag gtcaagttgt agacgtttgt    1320

tctgaaggca tggctgaagt aggattagat catcttgaat ttattcacca tcataaaact    1380

gctgcattat tacaaggttc agttgtttta ggtgcaatat taggaggcgg taaagaagaa    1440

gaagtagcta aacttcgtaa atttgctaac tgtattggtt tacttttcca agttgttgat    1500

gatattttag atgttactaa aagtagtaaa gagttaggta aaactgcagg taaagactta    1560

gtagctgata aaactacata tcctaaactt ataggcgttg aaaaatcaaa agaatttgct    1620

gaccgtttaa atcgtgaagc acaagaacaa ttattacatt ttcatcctca ccgtgctgct    1680

ccattaatcg ctttagctaa ctacatcgct taccgtgata atggtaccgg tgaaaactta 1740

tacttccagg gtagtggtgg tggcggatca gattataaag atgacgatga taaaggaacc 1800

ggttaatcta gactcgag 1818

<210> 173
<211> 1386
<212> DNA
<213> Artificial Sequence

<220>
<223> Description of Artificial Sequence: Synthetic
      polynucleotide

<400> 173
catatggtac cagtaacagc agcacgtgca acaccaaaat taagtaatag aaaattacgt 60

gttgctgtaa ttggaggcgg tccagcagga ggtgcagctg ctgaaacatt agcacaagga 120

ggtattgaaa caattcttat cgaacgtaaa atggataatt gtaaaccatg tggtggtgct 180

attccattat gtatggtagg agagttcaat ttacctttag acattattga ccgtcgtgta 240

acaaaaatga aaatgatctc tccttcaaac attgcagttg atatcggtcg tacacttaaa 300

gaacacgaat atattggtat ggttcgtcgt gaggtacttg atgcttatct tcgtgaacgt 360

gcagaaaaat caggtgctac tgttattaac ggtttattct taaaaatgga tcacccagaa 420

aattgggatt caccatatac acttcactac acagagtatg atggaaaaac aggtgctaca 480

ggaactaaaa aaactatgga agtagatgct gttattggtg ctgatggtgc taattctcgt 540

gttgcaaaaa gtattgacgc aggtgattat gattatgcta ttgcatttca agaacgtatt 600

cgtatacctg atgagaaaat gacttattat gaggacttag ctgagatgta tgtaggtgat 660

gatgtatcac cagacttcta cggttgggta ttcccaaaat gtgatcatgt agctgttggt 720

acaggtactg taacacataa aggtgatatc aaaaaattcc agttagctac acgtaatcgt 780

gctaaagata aaattcttgg tggcaaaata atccgtgtag aggctcatcc tattccagag 840

catcctagac cacgtcgttt atcaaaacgt gttgcattag taggcgacgc agcaggttac 900

gttactaaat gttcaggaga aggaatttac ttcgcagcta aatctggtcg tatgtgtgct 960

gaagctatcg ttgaaggttc acaaaatggc aaaaaaatga tagatgaagg cgatttaaga 1020

aaatacttag aaaaatggga taaaacttac ttaccaactt atcgtgtttt agatgtactt 1080

caaaaagttt tctatcgttc taacccagct cgtgaggctt ttgttgaaat gtgtaacgat 1140

gagtatgtac agaaaatgac atttgattct tacctttata aacgtgtagc tcctggtagt 1200

ccattagaag atatcaaatt agctgtaaat actattggtt cacttgttcg tgctaacgca 1260

ttacgtcgtg aaattgagaa attatcagta ggtaccggtg agaatcttta ctttcaagga 1320

tcaggtggtg gtggttctga ttataaagat gacgatgata aaggaaccgg ttaatctaga 1380

ctcgag                                                            1386

<210> 174
<211> 1377
<212> DNA
<213> Artificial Sequence

<220>
<223> Description of Artificial Sequence: Synthetic
      polynucleotide

<400> 174
catatggtac cagtagctgt tattggtggt ggtccaagtg gcgcttgtgc agcagaaact      60

ttagcaaaag gtggtgtaga aactttctta cttgagcgta aattagataa ttgtaaacct     120

tgtggaggtg caattccatt atgtatggtt gaagaatttg atttaccaat ggaaataatt     180

gaccgtcgtg ttactaaaat gaaaatgata tcaccttcaa accgtgaagt tgatgttgga     240

aaaactttat cagaaactga atggatcggt atgtgtcgtc gtgaagtatt tgacgattac     300

ttaagaaacc gtgcacagaa attaggtgct aatattgtta acggtttatt catgcgttca     360

gaacaacaat ctgcagaggg tccattcaca attcactata attcttatga agacggtagt     420

aaaatgggaa aacctgctac tttagaagtt gatatgataa ttggtgcaga tggagcaaat     480

tctcgtattg caaaagagat agatgcaggt gaatacgact acgctatagc ttttcaagaa     540

cgtattcgta ttcctgatga taaaatgaaa tattacgaaa accttgctga aatgtatgta     600

ggtgatgacg tatctcctga tttctatggt tgggtttttc ctaaatatga tcacgttgct     660

gttggtacag gtactgttgt aaacaaaaca gctattaaac aatatcaaca ggcaacacgt     720

gacagatcaa aagttaaaac agaaggtggc aaaattatac gtgttgaagc acacccaatt     780

ccagaacatc cacgtccacg tcgttgtaaa ggtcgtgttg cattagtagg cgacgcagct     840

ggttatgtta caaaatgttc tggcgagggc atttactttg ctgctaaatc tggtagaatg     900

gctgctgaag ctattgtaga aggttctgct aacggtacaa aaatgtgtgg tgaggatgca     960

attcgtgttt atttagataa atgggatcgt aaatattgga caacatacaa agtattagac    1020

attttacaaa aagtatttta tcgtagtaat ccagcacgtg aagcatttgt tgaattatgt    1080

gaagatagtt atgtacagaa aatgacattt gattcatact tatataaaac tgttgttcca    1140

ggaaacccat tagacgacgt aaaattactt gttcgtacag tatcttctat tttacgttca    1200

aatgctttac gttctgttaa ttctaaatct gtaaatgttt ctttcggctc taaagcaaat    1260

gaggaacgtg ttatggctgc aggtaccggt gaaaatcttt attttcaagg ttcaggaggt    1320

ggtggttcag attataaaga tgatgatgac aaaggaaccg gttaatctag actcgag       1377

<210> 175
<211> 933
<212> DNA

<213> Artificial Sequence

<220>
<223> Description of Artificial Sequence: Synthetic
      polynucleotide

<400> 175
catatggtac cagcaatggc agtaccatta gatgtagtaa ttacatatcc ttcttcaggt      60

gctgctgctt atccagtact tgttatgtat aacggtttcc aagctaaagc tccatggtat     120

cgtggtattg tagatcatgt ttctagttgg ggttacacag ttgttcaata tacaaatggt     180

ggcttatttc ctattgttgt agatcgtgtt gagttaactt atttagagcc attattaact     240

tggttagaaa cacaaagtgc tgatgctaaa tctcctttat acggtcgtgc agatgtttct     300

cgtttaggta caatgggtca ttcacgtggt ggtaaattag cagctttaca atttgctgga     360

cgtacagatg taagtggttg tgtattattt gaccctgtag atggaagtcc aatgacacca     420

gaatctgctg attatccttc agctacaaaa gcattagcag cagctggtcg ttctgctggc     480

ttagtaggtg cagctattac aggttcatgt aatccagtag gtcaaaatta cccaaaattc     540

tggggtgctt tagctcctgg ttcttggcaa atggtattat cacaagctgg tcacatgcaa     600

tttgctcgta ctggtaatcc attcttagat tggtcattag accgtttatg tggtcgtggt     660

acaatgatga gttcagatgt tattacatat agtgcagcat ttactgttgc ttggtttgaa     720

ggtatttttc gtcctgctca aagtcaaatg ggtatttcta atttcaaaac ttgggctaat     780

actcaagttg cagctcgtag tatcactttt gatattaaac ctatgcaatc tcctcagggt     840

accggtgaaa acctttactt tcaaggtagt ggtggtggag gaagtgatta taaagatgat     900

gatgacaaag gaaccggtta atctagactc gag                                  933


<210> 176
<211> 963
<212> DNA
<213> Artificial Sequence

<220>
<223> Description of Artificial Sequence: Synthetic
      polynucleotide

<400> 176
catatggtac cagcaccacc aaaaccagtt cgtataactt gtccaacagt agctggcact      60

tatcctgttg ttttattctt tcacggtttt tatcttcgta actatttcta ttcagatgtt     120

ttaaatcata ttgctagtca tggttacatc ttagttgcac cacaattatg taaactttta     180

cctccaggtg gccaagtaga agttgatgac gctggttcag ttattaactg ggcttcagag     240

aatcttaaag cacaccttcc aacttctgtt aatgctaatg gtaaatatac atctttagtt     300

ggacattcac gtggtggcaa aacagctttc gcagttgcat taggtcacgc agctacatta     360

gatccatcaa ttacattttc agcattaatt ggtattgatc cagtagcagg aactaacaaa     420

tacattcgta cagatccaca catcttaact tataaacctg aatcatttga attagatatt 480

cctgtagctg ttgtaggcac tggtcttggt ccaaaatgga ataacgtaat gcctccatgc 540

gcacctacag atttaaacca cgaagaattt tacaaagaat gtaaagctac taaagctcac 600

tttgttgctg ctgattatgg tcacatggac atgttagacg acgatcttcc aggttttgta 660

ggcttcatgg ctggttgtat gtgtaaaaat ggtcaacgta aaaaatcaga aatgcgttct 720

tttgtaggtg gtatagttgt agcattctta aaatattctt tatggggtga aaaagctgaa 780

ataagattaa ttgttaaaga tcctagtgta tctcctgcta aattagaccc atcaccagaa 840

ttagaagaag catcaggtat ttttgttggt accggtgaaa atctttattt tcaaggttca 900

ggtggaggtg gttctgatta taaagatgat gatgacaaag gaaccggtta atctagactc 960

gag 963

<210> 177
<211> 921
<212> DNA
<213> Artificial Sequence

<220>
<223> Description of Artificial Sequence: Synthetic
      polynucleotide

<400> 177
catatggtac cagctacacc agttgaagaa ggtgattatc cagttgtaat gttattacat 60

ggctaccttt tatataattc attttattca caattaatgt tacatgtatc atctcacggt 120

ttcatcttaa ttgctccaca attatactca attgctggtc ctgatactat ggatgaaatt 180

aaaagtactg ctgagattat ggactggtta tcagttggtt taaatcactt tttaccagct 240

caagttacac ctaatttatc taaatttgca ttatctggtc atagtcgtgg tggtaaaact 300

gcttttgctg tagcattaaa aaaatttggt tattcttcaa acttaaaaat tagtacttta 360

attggtattg atccagtaga cggaacaggt aaaggtaaac aaactccacc tcctgtttta 420

gcatatttac ctaatagttt tgacttagac aaaacaccaa ttttagtaat tggttcaggt 480

ttaggtgaaa ctgcacgtaa tcctttattt cctccatgtg ctcctccagg tgttaaccac 540

cgtgagtttt tccgtgaatg tcaaggtcca gcatggcact ttgttgctaa agattatggt 600

catttagaca tgcttgatga tgatacaaaa ggtattcgtg gcaaatctag ttactgttta 660

tgcaaaaatg gtgaagaacg tcgtccaatg cgtcgtttcg ttggtggttt agttgttagt 720

tttcttaaag catatcttga aggtgatgat cgtgaattag taaaaatcaa agatggttgt 780

catgaagatg tacctgttga aattcaagaa tttgaagtaa ttatgggtac cggtgaaaat 840

ctttactttc aaggttcagg cggtggaggt tcagattata aagatgatga tgacaaagga 900

accggttaat ctagactcga g 921

<210> 178
<211> 1620
<212> DNA
<213> Artificial Sequence

<220>
<223> Description of Artificial Sequence: Synthetic
      polynucleotide

<400> 178
catatggtac caagtcacaa aaaaaaaaac gtaatcttct tcgtaactga tggtatgggt      60

cctgcttctc tttcaatggc tcgttcattt aatcaacacg ttaatgattt accaattgat     120

gatattttaa cattagatga acattttatt ggaagttcaa gaacacgttc atcagattca     180

cttgtaactg actcagctgc tggagctaca gcttttgctt gtgcacttaa atcatacaat     240

ggtgctatag gtgtagatcc acaccatcgt ccatgtggaa ctgttttaga agctgctaaa     300

ttagcaggtt atttaacagg attagtagtt actacacgta ttactgatgc tacaccagct     360

agtttctcaa gtcacgtaga ttatcgttgg caagaagatt taattgcaac acaccaatta     420

ggtgaatatc ctttaggacg tgttgttgat cttcttatgg gtggtggtcg ttctcacttt     480

tatcctcaag gtgaaaaagc tagtccatac ggtcaccacg gtgcacgtaa agatggtcgt     540

gatttaatcg atgaagctca aagtaatggc tggcagtatg taggagatcg taaaaatttt     600

gattctttac ttaaatcaca tggtgaaaat gttactttac catttttagg tttatttgct     660

gacaacgata tcccatttga aattgatcgt gatgaaaaag aatatcctag tttaaaagaa     720

caagtaaaag tagcattagg tgctttagaa aaagcaagta acgaagataa agatagtaat     780

ggtttctttt taatggtaga aggttctcgt attgatcatg ctggccatca aaacgatcct     840

gcatctcaag tacgtgaagt attagcattt gatgaggctt ttcaatatgt attagaattt     900

gcagaaaaca gtgatacaga aacagtatta gtaagtacat cagatcatga aacaggtggt     960

ttagttactt caagacaagt aacagcatca tacccacaat atgtatggta tcctcaagta    1020

ttagctaacg ctacacatag tggagagttt cttaaacgta aattagttga tttcgttcat    1080

gaacacaaag gcgcatcatc aaaaatagaa aacttcataa aacacgaaat tcttgaaaaa    1140

gatttaggta tttatgatta tacagattct gacttagaaa cacttattca tttagatgat    1200

aacgctaatg caattcaaga taaacttaat gatatggtaa gttttagagc tcaaattggt    1260

tggacaacac atggtcattc agcagttgat gtaaacatat atgcttacgc aaacaaaaaa    1320

gctacatggt cttatgttct taataactta caaggtaatc acgaaaacac agaagttggt    1380

caattcttag agaatttctt agaattaaac ttaaatgaag ttactgattt aatccgtgat    1440

acaaaacata cttctgattt tgacgcaaca gaaatagcaa gtgaggttca acactatgat    1500

gaatattacc acgaattaac aaatggtacc ggtgaaaatc tttattttca aggttctggt    1560

ggaggtggca gtgattataa agatgatgat gacaaaggaa ccggttaatc tagactcgag 1620

<210> 179
<211> 1206
<212> DNA
<213> Artificial Sequence

<220>
<223> Description of Artificial Sequence: Synthetic
      polynucleotide

<400> 179
catatggtac cacacaagtt cacaggtgtt aacgctaaat tccagcaacc agcattaaga 60

aatttatctc cagtggtagt tgagcgcgaa cgtgaggaat ttgtaggatt ctttccacaa 120

attgttcgtg acttaactga agatggtatt ggtcatccag aagtaggtga cgctgtagct 180

cgtcttaaag aagtattaca atacaacgca cctggtggta aatgcaatag aggtttaaca 240

gttgttgcag cttaccgtga actttctgga ccaggtcaaa aagacgctga aagtcttcgt 300

tgtgctttag cagtaggatg gtgtattgaa ttattccaag cctttttctt agtttgggac 360

gatataatgg accagtcatt aactagacgt ggtcaattat gttggtacaa gaaagaaggt 420

gttggtttag atgcaataaa tgattctttt cttttagaaa gctctgtgta tcgcgttctt 480

aaaaagtatt gccgtcaacg tccatattat gtacatttat tagagctttt tcttcaaaca 540

gcttaccaaa cagaattagg acaaatgtta gatttaatca ctgctcctgt atctaaggta 600

gatttaagcc atttctcaga agaacgttac aaagctattg ttaagtataa aactgctttc 660

tattcattct atttaccagt tgcagcagct atgtatatgg ttggtataga ttctaaagaa 720

gaacatgaaa acgcaaaagc tattttactt gagatgggtg aatacttcca aattcaagat 780

gattatttag attgttttgg cgatcctgct ttaacaggta aagtaggtac tgatattcaa 840

gataacaaat gttcatggtt agttgtgcaa tgcttacaaa gagtaacacc agaacaacgt 900

caacttttag aagataatta cggtcgtaaa gaaccagaaa aagttgctaa agttaaagaa 960

ttatatgagg ctgtaggtat gagagccgcc tttcaacaat acgaagaaag tagttaccgt 1020

cgtcttcaag agttaattga gaaacattct aatcgtttac caaaagaaat tttcttaggt 1080

ttagctcaga aaatatacaa acgtcaaaaa ggtaccggtg aaaacttata ctttcaaggc 1140

tcaggtggcg gtggaagtga ttacaaagat gatgatgata aaggaaccgg ttaatctaga 1200

ctcgag 1206

<210> 180
<211> 977
<212> DNA
<213> Artificial Sequence

<220>

<223> Description of Artificial Sequence: Synthetic
      polynucleotide

<400> 180
atggtaccag catttgactt cgatggttac atgcttcgta aagctaaatc tgtaaataaa      60

gctttgaagc tgcagtacaa atgaaagaac cattaaaaat tcatgaaagt atgcgttatt     120

ctttattagc tggtggtaaa cgtgtacgtc caatgttatg tattgcagct tgtgaattag     180

ttggtggtga cgaaagtact gctatgcctg ctgcttgcgc tgtagaaatg attcatacta     240

tgagtttaat gcatgatgat ttaccatgta tggataatga cgatttacgt cgtggtaaac     300

caacaaacca catggcattt ggtgaaagtg tagcagtatt agcaggtgat gcattattat     360

cttttgcttt tgaacatgta gcagcagcaa caaaaggtgc tcctccagaa cgtattgtta     420

gagttttagg tgaacttgca gtttctattg gttcagaagg tttagttgct ggacaagtag     480

ttgacgtttg ttctgaaggt atggctgagg ttggtttaga tcatttagaa tttattcatc     540

accacaaaac tgctgcttta ttacaaggtt ctgtagtatt aggtgcaata ttaggtggtg     600

gaaaagaaga agaggtagca aaacttcgta aattcgctaa ctgcattggt ttacttttcc     660

aagtagtaga tgatattctt gatgtaacaa aatcatctaa agaattaggt aaaacagcag     720

gtaaagattt agttgctgat aaaactactt atccaaaatt aatcggtgtt gagaaaagta     780

aagagttcgc agaccgttta aatcgtgaag ctcaagaaca acttcttcat tttcatccac     840

atagagcagc acctttaatc gctttagcaa actatattgc ttatcgtgat aatggtaccg     900

gtgaaaattt atattttcaa ggttcaggtg gcggaggttc tgattataaa gatgatgatg     960

ataaaggaac cggttaa                                                    977

<210> 181
<211> 891
<212> DNA
<213> Artificial Sequence

<220>
<223> Description of Artificial Sequence: Synthetic
      polynucleotide

<400> 181
atggtaccaa gtcaacctta ctgggcagca attgaggcag atattgaacg ttacttaaaa      60

aaatcaatta caattcgtcc accagaaact gtatttggtc caatgcacca cttaactttt     120

gctgcaccag ctacagctgc tagtacttta tgtttagcag catgtgaact tgtaggtggt     180

gatcgtagtc aagctatggc tgcagcagca gcaatccatc ttgttcatgc agctgcttat     240

gtacatgaac atttaccatt aactgatggt agtcgtccag taagtaaacc agctatccaa     300

cataaatatg gtccaaatgt agaattactt acaggtgacg gtattgtacc atttggtttt     360

gaattattag caggttctgt tgatccagca cgtacagatg atccagaccg tattttacgt     420

gtaataattg aaataagtcg tgctggtggt ccagaaggta tgattagtgg tttacatcgt 480

gaagaagaga ttgtagatgg taatacttct cttgatttta ttgaatacgt ttgcaaaaaa 540

aaatatggtg aaatgcacgc atgtggtgct gcatgcggtg caattttagg tggtgcagct 600

gaagaagaaa ttcaaaaact tcgtaacttc ggattatatc aaggaacttt acgtggtatg 660

atggagatga aaaactcaca ccaacttatt gacgaaaata tcattggcaa acttaaagaa 720

ttagctttag aagaattagg tggatttcat ggtaaaaatg ctgaattaat gtctagttta 780

gtagcagaac catcattata tgctgctggt accggtgaaa atttatactt tcaaggttct 840

ggtggtggtg gcagtgatta taaagacgat gatgacaaag gaaccggtta a 891

<210> 182
<211> 1080
<212> DNA
<213> Artificial Sequence

<220>
<223> Description of Artificial Sequence: Synthetic
      polynucleotide

<400> 182
atggtaccac ttttatctaa caaattaaga gagatggttt tagcagaagt tcctaaatta 60

gcatctgctg ctgaatattt ctttaaacgt ggtgttcagg gtaaacaatt ccgttcaaca 120

attttattat taatggcaac agctcttgac gttcgtgttc cagaagcatt aattggtgaa 180

tctactgata ttgtaacatc tgaattacgt gtacgtcaac gtggcattgc tgaaattaca 240

gaaatgattc atgtagcatc acttcttcac gatgacgttc ttgacgatgc tgatactcgt 300

cgtggtgttg gtagtcttaa tgttgtaatg ggaaacaaaa tgtcagtttt agcaggtgac 360

ttcttacttt ctcgtgcttg tggtgctctt gcagctctta aaaacacaga agttgtagca 420

ttattagcta cagcagtaga acacttagtt actggtgaga caatggaaat aacttcatca 480

actgaacaac gttattctat ggattactac atgcagaaaa cttattacaa aactgcttca 540

ttaatttcaa attcatgtaa agcagttgct gtattaacag gtcaaacagc tgaagttgca 600

gtattagctt ttgaatatgg tcgtaattta ggtttagctt tccagttaat tgacgacatt 660

ttagatttca caggcacatc tgctagttta ggaaaaggtt ctttatcaga tatacgtcat 720

ggtgttatta ctgctcctat cttatttgca atggaagaat ttcctcaatt aagagaagta 780

gtagatcaag tagaaaaaga tccaagaaat gtagacatag ctttagaata tttaggtaaa 840

agtaaaggta ttcaacgtgc tcgtgaatta gcaatggaac acgcaaattt agctgctgca 900

gctattggtt ctttacctga aacagataac gaagatgtta aacgttcacg tcgtgcttta 960

attgatttaa cacacagagt aattacacgt aacaaaggta ccggtgagaa tttatacttt 1020

caaggtagtg gtggaggagg tagtgactat aaagatgatg acgataaagg aaccggttaa 1080

<210> 183
<211> 1092
<212> DNA
<213> Artificial Sequence

<220>
<223> Description of Artificial Sequence: Synthetic
      polynucleotide

<400> 183
atggtaccag tagtttctga acgtttaaga cattctgtaa caactggtat tccagcatta      60

aaaacagcag ctgaatattt ctttcgtcgt ggtatcgaag gaaaacgttt aagacctaca     120

ttagcattat taatgagtag tgctttatca ccagctgctc catcaccaga gtatttacaa     180

gttgatacaa gacctgctgc agaacaccct catgaaatgc gtcgtcgtca acaacgttta     240

gctgaaattg cagaattaat ccatgtagct tcattacttc acgatgatgt tattgatgac     300

gcacaaacac gtcgtggtgt tttaagttta aatacatctg ttggtaataa aacagctatc     360

ttagcaggtg atttcttatt agctcgtgca tctgtaacat tagctagttt aagaaactct     420

gaaattgtag aattaatgtc acaggtttta gaacacttag tatctggtga aattatgcaa     480

atgactgcta cttcagaaca acttttagat ttagaacatt atttagcaaa aacatattgt     540

aaaactgctt cattaatggc taatagttct cgttctgttg cagttcttgc aggtgcagct     600

cctgaagttt gtgatatggc atggtcatac ggtcgtcatt taggtattgc tttccaagta     660

gttgacgatt tattagattt aacaggttca tcttctgttt taggtaaacc tgctttaaac     720

gatatgcgtt ctggtttagc aacagcacca gtattattcg ctgcacaaga agaacctgca     780

ttacaggctc ttatattacg tcgttttaaa cacgacggtg acgtaacaaa agcaatgtca     840

ttaattgaac gtacacaagg cttacgtcgt gctgaagaac ttgcagcaca acacgcaaaa     900

gctgctgctg atatgattcg ttgcttacct acagctcaat cagaccatgc agaaattgct     960

cgtgaagcat taattcaaat tacacatcgt gttttaacac gtaaaaaagg taccggtgaa    1020

aacttatact ttcaaggttc tggtggtggt ggatcagatt ataaagatga tgatgacaaa    1080

ggaaccggtt aa                                                        1092

<210> 184
<211> 1128
<212> DNA
<213> Artificial Sequence

<220>
<223> Description of Artificial Sequence: Synthetic
      polynucleotide

<400> 184
atggtaccaa ctacaacatt atcatctaac cttaactcac aattcatgca ggtttacgag      60

actcttaaat cagaacttat tcatgaccca ttatttgagt tcgatgacga ttcaagacaa     120

tgggtagaac gtatgattga ttatactgta ccaggtggta aaatggttcg tggttatagt 180

gtagtagata gttatcaatt acttaaaggt gaagaactta cagaagaaga ggcattttta 240

gcttgtgcac ttggttggtg tacagaatgg tttcaagcat tcattctttt acatgatgat 300

atgatggatg gtagtcacac aagacgtggt caaccatgtt ggtttcgttt acctgaggtt 360

ggtgctgttg ctattaatga tggtgtttta cttcgtaatc acgttcaccg tattcttaaa 420

aaacattttc aaggtaaagc atattatgtt catttagttg atttattcaa tgaaactgaa 480

tttcaaacaa ttagtggaca aatgatcgac ttaattacaa cattagttgg tgaaaaagac 540

ttatctaaat attcattaag tattcatcgt cgtatcgttc aatacaaaac agcatactac 600

tcattttact taccagttgc ttgtgcttta cttatgtttg gtgaggatct tgataaacat 660

gtagaagtta aaaatgttct tgttgaaatg ggtacatatt ttcaagttca agatgattat 720

ttagattgtt ttggtgctcc agaagttatt ggcaaaattg gtactgatat tgaagacttt 780

aaatgttcat ggttagtagt taaagcatta gaattagcaa atgaagaaca gaaaaaaact 840

ttacacgaaa attatggaaa aaaagatcca gcatcagttg ctaaagttaa agaagtatac 900

cacacactta atttacaagc tgttttcgaa gattatgaag caacatcata caaaaaactt 960

attacttcta ttgaaaatca cccatctaaa gctgttcaag ctgttttaaa atctttctta 1020

ggcaaaatat acaaacgtca aaaaggtacc ggtgaaaact tatactttca aggttctggt 1080

ggcggtggaa gtgattacaa agatgatgac gataaaggaa ccggttaa 1128

<210> 185
<211> 1803
<212> DNA
<213> Artificial Sequence

<220>
<223> Description of Artificial Sequence: Synthetic
      polynucleotide

<400> 185
atggtaccaa gtcaacctta ctgggctgca attgaagcag acattgaaag atatttaaaa 60

aaatcaatta caattcgtcc accagaaact gtatttggtc ctatgcacca tttaacattt 120

gctgctcctg ctactgcagc tagtacatta tgccttgctg cttgtgaatt agttggcggt 180

gatcgtagtc aagctatggc agctgctgct gctatccatt tagttcatgc agctgcttac 240

gttcacgaac atcttccttt aacagatgga tcacgtcctg taagtaaacc tgctattcaa 300

cataaatatg gtccaaacgt tgaactttta acaggtgatg gtatcgttcc tttcggtttt 360

gagttattag caggttcagt agatccagca cgtactgatg accctgatcg tattttacgt 420

gtaattattg aaatttctcg tgctggtgga ccagaaggca tgatttctgg tttacaccgt 480

gaggaagaaa tcgtagatgg taacacatca ttagacttta tagaatatgt atgcaaaaaa 540

aaatacggtg aaatgcacgc atgtggtgca gcttgcggag ctattttagg tggagctgct 600

gaagaagaaa ttcaaaaact tcgtaacttt ggtctttatc aaggcacatt acgtggtatg 660

atggaaatga aaaatagtca tcagttaatt gacgaaaata tcattggaaa acttaaagaa 720

cttgctcttg aagaattagg tggattccac ggtaaaaacg ctgaattaat gagttcttta 780

gttgctgaac ctagtttata tgcagcttca tcaaataact taggtatcga aggtcgtttt 840

gactttgacg gttacatgct tcgtaaagca aaatctgtaa ataaagcatt agaagctgct 900

gttcaaatga aagaaccact taaaattcac gaatcaatgc gttattcatt attagctggt 960

ggtaaacgtg ttcgtccaat gttatgtatt gcagcttgtg aacttgttgg tggtgacgaa 1020

tctacagcaa tgcctgcagc atgtgctgtt gaaatgattc acacaatgtc tttaatgcat 1080

gatgaccttc catgtatgga taacgatgac ttacgtcgtg gtaaacctac aaaccacatg 1140

gcttttggtg agtctgtagc tgttcttgct ggtgatgcat tacttagttt tgcttttgaa 1200

catgttgctg ctgcaacaaa aggcgcacca cctgaacgta tcgtacgtgt attaggtgaa 1260

ttagctgtta gtattggttc agaaggactt gtagcaggtc aagttgtaga cgtttgttct 1320

gaaggcatgg ctgaagtagg attagatcat cttgaattta ttcaccatca taaaactgct 1380

gcattattac aaggttcagt tgttttaggt gcaatattag gaggcggtaa agaagaagaa 1440

gtagctaaac ttcgtaaatt tgctaactgt attggtttac ttttccaagt tgttgatgat 1500

attttagatg ttactaaaag tagtaaagag ttaggtaaaa ctgcaggtaa agacttagta 1560

gctgataaaa ctacatatcc taaacttata ggcgttgaaa aatcaaaaga atttgctgac 1620

cgtttaaatc gtgaagcaca agaacaatta ttacattttc atcctcaccg tgctgctcca 1680

ttaatcgctt tagctaacta catcgcttac cgtgataatg gtaccggtga aaacttatac 1740

ttccagggta gtggtggtgg cggatcagat tataaagatg acgatgataa aggaaccggt 1800

taa 1803

<210> 186
<211> 1191
<212> DNA
<213> Artificial Sequence

<220>
<223> Description of Artificial Sequence: Synthetic
      polynucleotide

<400> 186
atggtaccac acaagttcac aggtgttaac gctaaattcc agcaaccagc attaagaaat 60

ttatctccag tggtagttga gcgcgaacgt gaggaatttg taggattctt tccacaaatt 120

gttcgtgact taactgaaga tggtattggt catccagaag taggtgacgc tgtagctcgt 180

cttaaagaag tattacaata caacgcacct ggtggtaaat gcaatagagg tttaacagtt 240

gttgcagctt accgtgaact ttctggacca ggtcaaaaag acgctgaaag tcttcgttgt 300

gctttagcag taggatggtg tattgaatta ttccaagcct ttttcttagt ttgggacgat 360

ataatggacc agtcattaac tagacgtggt caattatgtt ggtacaagaa agaaggtgtt 420

ggtttagatg caataaatga ttcttttctt ttagaaagct ctgtgtatcg cgttcttaaa 480

aagtattgcc gtcaacgtcc atattatgta catttattag agctttttct tcaaacagct 540

taccaaacag aattaggaca aatgttagat ttaatcactg ctcctgtatc taaggtagat 600

ttaagccatt tctcagaaga acgttacaaa gctattgtta agtataaaac tgctttctat 660

tcattctatt taccagttgc agcagctatg tatatggttg gtatagattc taaagaagaa 720

catgaaaacg caaaagctat tttacttgag atgggtgaat acttccaaat tcaagatgat 780

tatttagatt gttttggcga tcctgcttta acaggtaaag taggtactga tattcaagat 840

aacaaatgtt catggttagt tgtgcaatgc ttacaaagag taacaccaga acaacgtcaa 900

cttttagaag ataattacgg tcgtaaagaa ccagaaaaag ttgctaaagt taaagaatta 960

tatgaggctg taggtatgag agccgccttt caacaatacg aagaaagtag ttaccgtcgt 1020

cttcaagagt taattgagaa acattctaat cgtttaccaa aagaaatttt cttaggttta 1080

gctcagaaaa tatacaaacg tcaaaaaggt accggtgaaa acttatactt tcaaggctca 1140

ggtggcggtg gaagtgatta caaagatgat gatgataaag gaaccggtta a 1191

<210> 187
<211> 1191
<212> DNA
<213> Artificial Sequence

<220>
<223> Description of Artificial Sequence: Synthetic
      polynucleotide

<400> 187
atggtaccac acaagttcac aggtgttaac gctaaattcc agcaaccagc attaagaaat 60

ttatctccag tggtagttga gcgcgaacgt gaggaatttg taggattctt tccacaaatt 120

gttcgtgact taactgaaga tggtattggt catccagaag taggtgacgc tgtagctcgt 180

cttaaagaag tattacaata caacgcacct ggtggtaaat gcaatagagg tttaacagtt 240

gttgcagctt accgtgaact ttctggacca ggtcaaaaag acgctgaaag tcttcgttgt 300

gctttagcag taggatggtg tattgaatta ttccaagcct ttttcttagt tgctgacgat 360

ataatggacc agtcattaac tagacgtggt caattatgtt ggtacaagaa agaaggtgtt 420

ggtttagatg caataaatga ttcttttctt ttagaaagct ctgtgtatcg cgttcttaaa 480

aagtattgcc gtcaacgtcc atattatgta catttattag agctttttct tcaaacagct 540

taccaaacag aattaggaca aatgttagat ttaatcactg ctcctgtatc taaggtagat 600

ttaagccatt tctcagaaga acgttacaaa gctattgtta agtataaaac tgctttctat 660

tcattctatt taccagttgc agcagctatg tatatggttg gtatagattc taaagaagaa 720

catgaaaacg caaaagctat tttacttgag atgggtgaat acttccaaat tcaagatgat 780

tatttagatt gttttggcga tcctgcttta acaggtaaag taggtactga tattcaagat 840

aacaaatgtt catggttagt tgtgcaatgc ttacaaagag taacaccaga acaacgtcaa 900

cttttagaag ataattacgg tcgtaaagaa ccagaaaaag ttgctaaagt taaagaatta 960

tatgaggctg taggtatgag agccgccttt caacaatacg aagaaagtag ttaccgtcgt 1020

cttcaagagt taattgagaa acattctaat cgtttaccaa aagaaatttt cttaggttta 1080

gctcagaaaa tatacaaacg tcaaaaaggt accggtgaaa acttatactt tcaaggctca 1140

ggtggcggtg gaagtgatta caaagatgat gatgataaag gaaccggtta a 1191

<210> 188
<211> 987
<212> DNA
<213> Artificial Sequence

<220>
<223> Description of Artificial Sequence: Synthetic
      polynucleotide

<400> 188
atggtaccag attttccaca acaattagaa gcatgtgtta aacaagcaaa tcaagcatta 60

tcacgtttca tcgcaccact tccattccaa aatactcctg ttgttgaaac aatgcaatat 120

ggtgcattat taggaggtaa aagattaaga ccatttcttg tatatgcaac aggtcacatg 180

tttggagtat ctactaacac attagatgct ccagctgctg cagttgaatg tattcatgca 240

tatagtttaa ttcatgatga tttacctgca atggatgatg atgacttaag aagaggttta 300

cctacatgtc atgttaaatt tggtgaagct aatgctattt tagctggcga tgcacttcaa 360

actcttgcat tcagtatttt atcagatgct gatatgccag aagtttcaga tcgtgatcgt 420

atttctatga tatctgaatt agcttctgct agtggtattg ctggtatgtg cggtggccaa 480

gctcttgatt tagacgcaga aggaaaacac gttcctttag atgctttaga gcgtatacat 540

cgtcacaaaa caggagcttt aattagagct gctgttcgtc ttggtgcttt atcagctgga 600

gacaaaggtc gtcgtgcttt accagtttta gacaaatacg ctgaaagtat tggtttagct 660

tttcaagttc aggatgatat cttagatgtt gtaggtgata ctgctacttt aggtaaacgt 720

caaggtgctg atcaacagtt aggcaaatct acatacccag cacttttagg tttagaacaa 780

gctcgtaaaa aagcaagaga cttaattgac gatgctcgtc aaagtcttaa acaattagca 840

gaacaatcac ttgatacaag tgctttagaa gcattagcag attacattat tcaacgtaat 900

aaaggtaccg gtgaaaattt atattttcaa ggttctggtg gtggaggttc agactataaa 960

gatgacgatg ataaaggaac cggttaa                                          987


<210> 189
<211> 1242
<212> DNA
<213> Artificial Sequence

<220>
<223> Description of Artificial Sequence: Synthetic
      polynucleotide

<400> 189
atggtaccaa gtgttagttg ttgttgtaga aatttaggaa aaactatcaa aaaagctatt      60

ccaagtcacc acttacattt acgttcttta ggtggtagtt tatatagaag acgtattcaa     120

tcatcttcaa tggaaacaga cttaaaatct acattcttaa atgtttattc agttcttaaa     180

tcagatttat tacacgaccc atcatttgaa tttacaaatg aaagtcgttt atgggtagat     240

agaatgcttg attataatgt tcgtggcggt aaacttaatc gtggtctttc tgtagtagac     300

tctttcaaat tacttaaaca aggtaatgat ttaactgaac aagaagtttt cttatcttgt     360

gcattaggtt ggtgtattga gtggttacag gcttactttt tagttcttga tgatattatg     420

gataattcag ttacacgtcg tggtcaacct tgttggtttc gtgtaccaca agttggtatg     480

gtagctatta atgatggcat tcttcttcgt aaccatattc atcgtattct taaaaaacac     540

ttccgtgata aaccatatta tgtagattta gttgaccttt tcaatgaagt agagttacaa     600

actgcatgtg gacaaatgat tgatttaatc acaacatttg aaggtgaaaa agacttagct     660

aaatatagtt tatcaattca ccgtcgtatt gttcaataca aaactgcata ttactcattc     720

tatttaccag ttgcatgtgc tcttttaatg gctggcgaaa atttagaaaa ccacattgat     780

gttaaaaatg tattagtaga tatgggtatt tactttcaag ttcaggatga ttatttagac     840

tgttttgctg atcctgaaac attaggtaaa attggcactg atattgagga ctttaaatgt     900

tcttggttag ttgtaaaagc attagaacgt tgtagtgaag aacaaacaaa aattctttac     960

gaaaactatg gcaaacctga tccatctaat gttgctaaag taaaagattt atacaaagaa    1020

ttagatttag aaggcgtttt catggaatat gaatctaaat catacgagaa attaactggt    1080

gctatcgaag gtcaccaatc taaagcaatt caagctgttc ttaaatcttt cttagcaaaa    1140

atctataaac gtcaaaaagg taccggtgaa aacttatact ttcaaggtag tggtggcggt    1200

ggtagtgatt ataaagatga tgatgataaa ggaaccggtt aa                       1242


<210> 190
<211> 1116
<212> DNA
<213> Artificial Sequence

<220>

<223> Description of Artificial Sequence: Synthetic
      polynucleotide

<400> 190
atggtaccag ctgatcttaa atcaacattc ttagatgttt attcagtatt aaaaagtgat      60

ttattacaag atccatcttt tgaatttaca cacgaaagtc gtcaatggtt agaacgtatg     120

ttagattata atgttcgtgg aggcaaatta aacagaggtt taagtgtagt agacagttac     180

aaacttttaa aacaaggtca agacttaaca gaaaaagaaa catttttatc ttgtgcttta     240

ggttggtgta ttgaatggtt acaagcatac ttcttagttt tagacgatat tatggataat     300

tctgtaacta gacgtggtca accatgttgg tttcgtaaac caaaagtagg tatgattgct     360

attaatgatg gaatacttct tcgtaaccac attcatcgta ttcttaaaaa acactttcgt     420

gaaatgcctt attatgtaga ccttgtagac ttatttaacg aagtagaatt tcaaacagct     480

tgtggtcaaa tgattgactt aattacaaca tttgatggtg aaaaagacct ttcaaaatat     540

tcacttcaga ttcaccgtcg tattgttgag tacaaaacag catactactc tttctattta     600

cctgtagcat gtgctttact tatggcaggt gaaaatttag aaaatcacac agatgttaaa     660

actgtattag ttgatatggg tatctatttc caagttcaag atgattattt agattgcttc     720

gctgatccag aaacattagg taaaattggt acagatattg aagactttaa atgtagttgg     780

ttagtagtaa aagcattaga acgttgtagt gaagaacaaa caaaaattct ttacgaaaat     840

tatggaaaag ctgaaccttc aaatgtagct aaagttaaag cattatacaa agaattagat     900

ttagagggtg catttatgga atatgaaaaa gaatcatacg agaaacttac aaaacttatt     960

gaagcacatc aatcaaaagc tattcaagca gttcttaaat ctttcttagc taaaatttat    1020

aaacgtcaaa aaggtaccgg tgaaaactta tactttcaag gctctggagg tggtggttca    1080

gactataaag atgatgatga taaaggaacc ggttaa                              1116


<210> 191
<211> 1170
<212> DNA
<213> Artificial Sequence

<220>
<223> Description of Artificial Sequence: Synthetic
      polynucleotide

<400> 191
atggtaccaa gtggcgaacc tactccaaaa aaaatgaaag caacatacgt tcacgaccgt      60

gaaaacttta caaaagtata cgaaactctt cgtgacgaat tacttaacga tgattgtctt     120

agtccagctg gttcacctca ggctcaagct gctcaagagt ggtttaaaga agttaatgat     180

tataatgttc ctggtggaaa acttaaccgt ggtatggctg tatatgacgt tttagcttca     240

gttaaaggtc cagatggttt aagtgaagac gaagtattta aagctaacgc tcttggttgg     300

tgtattgagt ggttacaagc atttttctta gttgctgatg atataatgga tggttcaatt 360

acacgtcgtg gccaaccttg ttggtacaaa caacctaaag ttggtatgat tgcttgtaat 420

gattacatct tattagaatg ctgtatttac tcaattctta aaagacattt tagaggtcac 480

gctgcatacg ctcaacttat ggaccttttc catgaaacta cattccagac ttcacacggt 540

caattattag atttaacaac agcacctatc ggttctgtag acttatcaaa atatacagaa 600

gataattacc ttcgtattgt aacatataaa actgcatact attcttttta tttacctgta 660

gcatgtggta tggtattagc tggcattaca gatccagctg cttttgatct tgcaaaaaat 720

atttgtgttg aaatgggtca atatttccag attcaagacg attatttaga ttgctatggt 780

gaccctgagg ttattggtaa aatcggtaca gacatagaag acaacaaatg tagttggtta 840

gtttgcacag ctcttaaaat cgcaacagaa gaacaaaaag aggttataaa agctaattat 900

ggtcacaaag aggctgaatc agtagcagca attaaagcat tatacgttga attaggtatt 960

gaacaacgtt ttaaagacta tgaagctgca tcatacgcaa aattagaagg tacaattagt 1020

gaacaaactt tattacctaa agcagtattt acttctttat tagctaaaat ctataaaaga 1080

aaaaaaggta ccggtgagaa cttatacttt caaggtagtg gaggtggtgg ttcagactat 1140

aaagatgatg atgataaagg aaccggttaa 1170

<210> 192
<211> 1371
<212> DNA
<213> Artificial Sequence

<220>
<223> Description of Artificial Sequence: Synthetic
      polynucleotide

<400> 192
atggtaccag taacagcagc acgtgcaaca ccaaaattaa gtaatagaaa attacgtgtt 60

gctgtaattg gaggcggtcc agcaggaggt gcagctgctg aaacattagc acaaggaggt 120

attgaaacaa ttcttatcga acgtaaaatg gataattgta aaccatgtgg tggtgctatt 180

ccattatgta tggtaggaga gttcaattta cctttagaca ttattgaccg tcgtgtaaca 240

aaaatgaaaa tgatctctcc ttcaaacatt gcagttgata tcggtcgtac acttaaagaa 300

cacgaatata ttggtatggt tcgtcgtgag gtacttgatg cttatcttcg tgaacgtgca 360

gaaaaatcag gtgctactgt tattaacggt ttattcttaa aaatggatca cccagaaaat 420

tgggattcac catatacact tcactacaca gagtatgatg gaaaaacagg tgctacagga 480

actaaaaaaa ctatggaagt agatgctgtt attggtgctg atggtgctaa ttctcgtgtt 540

gcaaaaagta ttgacgcagg tgattatgat tatgctattg catttcaaga acgtattcgt 600

atacctgatg agaaaatgac ttattatgag gacttagctg agatgtatgt aggtgatgat 660

gtatcaccag acttctacgg ttgggtattc ccaaaatgtg atcatgtagc tgttggtaca 720

ggtactgtaa cacataaagg tgatatcaaa aaattccagt tagctacacg taatcgtgct 780

aaagataaaa ttcttggtgg caaaataatc cgtgtagagg ctcatcctat tccagagcat 840

cctagaccac gtcgtttatc aaaacgtgtt gcattagtag gcgacgcagc aggttacgtt 900

actaaatgtt caggagaagg aatttacttc gcagctaaat ctggtcgtat gtgtgctgaa 960

gctatcgttg aaggttcaca aaatggcaaa aaaatgatag atgaaggcga tttaagaaaa 1020

tacttagaaa aatgggataa aacttactta ccaacttatc gtgttttaga tgtacttcaa 1080

aaagttttct atcgttctaa cccagctcgt gaggcttttg ttgaaatgtg taacgatgag 1140

tatgtacaga aaatgacatt tgattcttac ctttataaac gtgtagctcc tggtagtcca 1200

ttagaagata tcaaattagc tgtaaatact attggttcac ttgttcgtgc taacgcatta 1260

cgtcgtgaaa ttgagaaatt atcagtaggt accggtgaga atctttactt tcaaggatca 1320

ggtggtggtg gttctgatta taaagatgac gatgataaag gaaccggtta a 1371

<210> 193
<211> 1362
<212> DNA
<213> Artificial Sequence

<220>
<223> Description of Artificial Sequence: Synthetic
      polynucleotide

<400> 193
atggtaccag tagctgttat tggtggtggt ccaagtggcg cttgtgcagc agaaacttta 60

gcaaaaggtg gtgtagaaac tttcttactt gagcgtaaat tagataattg taaaccttgt 120

ggaggtgcaa ttccattatg tatggttgaa gaatttgatt taccaatgga aataattgac 180

cgtcgtgtta ctaaaatgaa aatgatatca ccttcaaacc gtgaagttga tgttggaaaa 240

actttatcag aaactgaatg gatcggtatg tgtcgtcgtg aagtatttga cgattactta 300

agaaaccgtg cacagaaatt aggtgctaat attgttaacg gtttattcat gcgttcagaa 360

caacaatctg cagagggtcc attcacaatt cactataatt cttatgaaga cggtagtaaa 420

atgggaaaac ctgctacttt agaagttgat atgataattg gtgcagatgg agcaaattct 480

cgtattgcaa aagagataga tgcaggtgaa tacgactacg ctatagcttt tcaagaacgt 540

attcgtattc ctgatgataa aatgaaatat tacgaaaacc ttgctgaaat gtatgtaggt 600

gatgacgtat ctcctgattt ctatggttgg gtttttccta aatatgatca cgttgctgtt 660

ggtacaggta ctgttgtaaa caaaacagct attaaacaat atcaacaggc aacacgtgac 720

agatcaaaag ttaaaacaga aggtggcaaa attatacgtg ttgaagcaca cccaattcca 780

gaacatccac gtccacgtcg ttgtaaaggt cgtgttgcat tagtaggcga cgcagctggt 840

tatgttacaa aatgttctgg cgagggcatt tactttgctg ctaaatctgg tagaatggct 900

gctgaagcta ttgtagaagg ttctgctaac ggtacaaaaa tgtgtggtga ggatgcaatt 960

cgtgtttatt tagataaatg ggatcgtaaa tattggacaa catacaaagt attagacatt 1020

ttacaaaaag tattttatcg tagtaatcca gcacgtgaag catttgttga attatgtgaa 1080

gatagttatg tacagaaaat gacatttgat tcatacttat ataaaactgt tgttccagga 1140

aacccattag acgacgtaaa attacttgtt cgtacagtat cttctatttt acgttcaaat 1200

gctttacgtt ctgttaattc taaatctgta aatgtttctt tcggctctaa agcaaatgag 1260

gaacgtgtta tggctgcagg taccggtgaa aatctttatt ttcaaggttc aggaggtggt 1320

ggttcagatt ataaagatga tgatgacaaa ggaaccggtt aa 1362

<210> 194
<211> 918
<212> DNA
<213> Artificial Sequence

<220>
<223> Description of Artificial Sequence: Synthetic
      polynucleotide

<400> 194
atggtaccag caatggcagt accattagat gtagtaatta catatccttc ttcaggtgct 60

gctgcttatc cagtacttgt tatgtataac ggtttccaag ctaaagctcc atggtatcgt 120

ggtattgtag atcatgtttc tagttggggt tacacagttg ttcaatatac aaatggtggc 180

ttatttccta ttgttgtaga tcgtgttgag ttaacttatt tagagccatt attaacttgg 240

ttagaaacac aaagtgctga tgctaaatct cctttatacg gtcgtgcaga tgtttctcgt 300

ttaggtacaa tgggtcattc acgtggtggt aaattagcag ctttacaatt tgctggacgt 360

acagatgtaa gtggttgtgt attatttgac cctgtagatg gaagtccaat gacaccagaa 420

tctgctgatt atccttcagc tacaaaagca ttagcagcag ctggtcgttc tgctggctta 480

gtaggtgcag ctattacagg ttcatgtaat ccagtaggtc aaaattaccc aaaattctgg 540

ggtgctttag ctcctggttc ttggcaaatg gtattatcac aagctggtca catgcaattt 600

gctcgtactg gtaatccatt cttagattgg tcattagacc gtttatgtgg tcgtggtaca 660

atgatgagtt cagatgttat tacatatagt gcagcattta ctgttgcttg gtttgaaggt 720

atttttcgtc ctgctcaaag tcaaatgggt atttctaatt tcaaaacttg ggctaatact 780

caagttgcag ctcgtagtat cacttttgat attaaaccta tgcaatctcc tcagggtacc 840

ggtgaaaacc tttactttca aggtagtggt ggtggaggaa gtgattataa agatgatgat 900

gacaaaggaa ccggttaa 918

<210> 195

<211> 948
<212> DNA
<213> Artificial Sequence

<220>
<223> Description of Artificial Sequence: Synthetic
      polynucleotide

<400> 195
atggtaccag caccaccaaa accagttcgt ataacttgtc caacagtagc tggcacttat      60

cctgttgttt tattctttca cggtttttat cttcgtaact atttctattc agatgtttta     120

aatcatattg ctagtcatgg ttacatctta gttgcaccac aattatgtaa acttttacct     180

ccaggtggcc aagtagaagt tgatgacgct ggttcagtta ttaactgggc ttcagagaat     240

cttaaagcac accttccaac ttctgttaat gctaatggta aatatacatc tttagttgga     300

cattcacgtg gtggcaaaac agctttcgca gttgcattag gtcacgcagc tacattagat     360

ccatcaatta cattttcagc attaattggt attgatccag tagcaggaac taacaaatac     420

attcgtacag atccacacat cttaacttat aaacctgaat catttgaatt agatattcct     480

gtagctgttg taggcactgg tcttggtcca aaatggaata acgtaatgcc tccatgcgca     540

cctacagatt taaaccacga agaattttac aaagaatgta aagctactaa agctcacttt     600

gttgctgctg attatggtca catggacatg ttagacgacg atcttccagg ttttgtaggc     660

ttcatggctg gttgtatgtg taaaaatggt caacgtaaaa aatcagaaat gcgttctttt     720

gtaggtggta tagttgtagc attcttaaaa tattctttat ggggtgaaaa agctgaaata     780

agattaattg ttaaagatcc tagtgtatct cctgctaaat tagacccatc accagaatta     840

gaagaagcat caggtatttt tgttggtacc ggtgaaaatc tttattttca aggttcaggt     900

ggaggtggtt ctgattataa agatgatgat gacaaaggaa ccggttaa                  948


<210> 196
<211> 906
<212> DNA
<213> Artificial Sequence

<220>
<223> Description of Artificial Sequence: Synthetic
      polynucleotide

<400> 196
atggtaccag ctacaccagt tgaagaaggt gattatccag ttgtaatgtt attacatggc      60

taccttttat ataattcatt ttattcacaa ttaatgttac atgtatcatc tcacggtttc     120

atcttaattg ctccacaatt atactcaatt gctggtcctg atactatgga tgaaattaaa     180

agtactgctg agattatgga ctggttatca gttggtttaa atcacttttt accagctcaa     240

gttacaccta atttatctaa atttgcatta tctggtcata gtcgtggtgg taaaactgct     300

tttgctgtag cattaaaaaa atttggttat tcttcaaact taaaaattag tactttaatt     360

ggtattgatc cagtagacgg aacaggtaaa ggtaaacaaa ctccacctcc tgttttagca 420

tatttaccta atagttttga cttagacaaa acaccaattt tagtaattgg ttcaggttta 480

ggtgaaactg cacgtaatcc tttatttcct ccatgtgctc ctccaggtgt taaccaccgt 540

gagtttttcc gtgaatgtca aggtccagca tggcactttg ttgctaaaga ttatggtcat 600

ttagacatgc ttgatgatga tacaaaaggt attcgtggca aatctagtta ctgtttatgc 660

aaaaatggtg aagaacgtcg tccaatgcgt cgtttcgttg gtggtttagt tgttagtttt 720

cttaaagcat atcttgaagg tgatgatcgt gaattagtaa aaatcaaaga tggttgtcat 780

gaagatgtac ctgttgaaat tcaagaattt gaagtaatta tgggtaccgg tgaaaatctt 840

tactttcaag gttcaggcgg tggaggttca gattataaag atgatgatga caaaggaacc 900

ggttaa 906

<210> 197
<211> 1047
<212> DNA
<213> Artificial Sequence

<220>
<223> Description of Artificial Sequence: Synthetic
      polynucleotide

<400> 197
atggtaccag ctgctgctgc acctgctgag acaatgaata aatctgcagc tggcgctgaa 60

gtaccagagg ctttcacatc agtttttcaa ccaggtaaat tagcagttga agcaattcaa 120

gtagatgaaa atgcagctcc tactccacct attcctgttt taatagttgc tccaaaagat 180

gctggtacat atccagttgc tatgttatta cacggatttt tcttacataa tcacttttat 240

gaacacttat tacgtcacgt tgcatctcat ggctttatca ttgttgctcc acaattttct 300

attagtatta ttccatcagg agatgctgaa gacatcgctg ctgctgcaaa agtagcagat 360

tggttacctg acggattacc aagtgtttta ccaaaaggtg ttgaaccaga gttatcaaaa 420

cttgctttag ctggacacag tcgtggtggt cacacagctt tttctttagc tttaggtcac 480

gctaaaacac aattaacttt cagtgcatta attggtttag atcctgttgc tggaacaggt 540

aaatcatctc aattacaacc aaaaattctt acttatgagc caagttcatt tggtatggct 600

atgccagttt tagttattgg tacaggttta ggagaagaaa aaaaaaacat tttctttcct 660

ccatgtgctc ctaaagacgt aaaccatgca gaattttatc gtgaatgtag accaccatgt 720

tactattttg taactaaaga ttatggccat cttgatatgt tagatgatga cgctccaaaa 780

tttatcacat gtgtttgtaa agacggtaat ggatgtaaag gaaaaatgcg tcgttgtgta 840

gctggcatca tggttgcttt cttaaacgct gctttaggtg aaaaagacgc agatttagaa 900

gctattttac gtgatccagc agttgctcct acaacattag acccagttga acaccgtgtt 960

gctggtaccg gtgagaattt atacttccag ggatctggtg gtggtggcag tgattataaa 1020

gatgatgatg ataaaggaac cggttaa 1047

<210> 198
<211> 1605
<212> DNA
<213> Artificial Sequence

<220>
<223> Description of Artificial Sequence: Synthetic
      polynucleotide

<400> 198
atggtaccaa gtcacaaaaa aaaaaacgta atcttcttcg taactgatgg tatgggtcct 60

gcttctcttt caatggctcg ttcatttaat caacacgtta atgatttacc aattgatgat 120

attttaacat tagatgaaca ttttattgga agttcaagaa cacgttcatc agattcactt 180

gtaactgact cagctgctgg agctacagct tttgcttgtg cacttaaatc atacaatggt 240

gctataggtg tagatccaca ccatcgtcca tgtggaactg ttttagaagc tgctaaatta 300

gcaggttatt taacaggatt agtagttact acacgtatta ctgatgctac accagctagt 360

ttctcaagtc acgtagatta tcgttggcaa gaagatttaa ttgcaacaca ccaattaggt 420

gaatatcctt taggacgtgt tgttgatctt cttatgggtg gtggtcgttc tcacttttat 480

cctcaaggtg aaaaagctag tccatacggt caccacggtg cacgtaaaga tggtcgtgat 540

ttaatcgatg aagctcaaag taatggctgg cagtatgtag gagatcgtaa aaattttgat 600

tctttactta aatcacatgg tgaaaatgtt actttaccat ttttaggttt atttgctgac 660

aacgatatcc catttgaaat tgatcgtgat gaaaaagaat atcctagttt aaaagaacaa 720

gtaaaagtag cattaggtgc tttagaaaaa gcaagtaacg aagataaaga tagtaatggt 780

ttctttttaa tggtagaagg ttctcgtatt gatcatgctg gccatcaaaa cgatcctgca 840

tctcaagtac gtgaagtatt agcatttgat gaggcttttc aatatgtatt agaatttgca 900

gaaaacagtg atacagaaac agtattagta agtacatcag atcatgaaac aggtggttta 960

gttacttcaa gacaagtaac agcatcatac ccacaatatg tatggtatcc tcaagtatta 1020

gctaacgcta cacatagtgg agagtttctt aaacgtaaat tagttgattt cgttcatgaa 1080

cacaaaggcg catcatcaaa aatagaaaac ttcataaaac acgaaattct tgaaaaagat 1140

ttaggtattt atgattatac agattctgac ttagaaacac ttattcattt agatgataac 1200

gctaatgcaa ttcaagataa acttaatgat atggtaagtt ttagagctca aattggttgg 1260

acaacacatg gtcattcagc agttgatgta aacatatatg cttacgcaaa caaaaaagct 1320

acatggtctt atgttcttaa taacttacaa ggtaatcacg aaaacacaga agttggtcaa 1380

ttcttagaga atttcttaga attaaactta aatgaagtta ctgatttaat ccgtgataca 1440

aaacatactt ctgattttga cgcaacagaa atagcaagtg aggttcaaca ctatgatgaa 1500

tattaccacg aattaacaaa tggtaccggt gaaaatcttt attttcaagg ttctggtgga 1560

ggtggcagtg attataaaga tgatgatgac aaaggaaccg gttaa 1605

<210> 199
<211> 1008
<212> DNA
<213> Artificial Sequence

<220>
<223> Description of Artificial Sequence: Synthetic
      polynucleotide

<400> 199
atggtaccag ctttatacga cattattaac tatttctacg gttcaaactc taaattcaac 60

cgtattacat ggggttttaa atcaccaact ttcatcaaat ggagaattac tgatttcatt 120

ttaatcatcg ttttaattgt tcttttcttc gtaacttctc aagcagagcc attccatcgt 180

caattttatc ttaacgacat gactatccaa catccttttg cagaacatga acgtgtaact 240

aatattcaac ttggtttata ttcaacagta attcctttat cagttattat cattgttgct 300

ttaattagta catgtccacc taaatacaaa ttatacaaca cttgggtttc aagtattggt 360

ttacttttat cagttttaat cacatctttt gttacaaaca tcgttaaaaa ctggtttgga 420

cgtttacgtc ctgacttctt agatcgttgc caaccagcta acgatacacc taaagataaa 480

ttagtttcta ttgaggtttg tactacagac aatttagacc gtttagctga cggttttcgt 540

acaacacctt ctggtcattc ttcaatctca tttgctggtt tattctattt aacattattt 600

cttttaggtc aatctcaggc aaataatggt aaaacatctt catggcgtac aatgatcagt 660

tttatacctt ggttaatggc ttgttatatc gctttaagtc gtacacaaga ctaccgtcat 720

catttcattg acgtatttgt tggtagttgc ttaggcttaa ttatcgcaat ttggcaatac 780

ttccgtttat tcccttggtt cggtggtaac caagcaaatg attcatttaa caaccgtatt 840

atgattgaag agattaaacg taaagaggaa attaaacaag atgaaaataa ctaccgtcgt 900

atttctgata tttctactaa tgtaggtacc ggtgaaaacc tttactttca aggttcaggt 960

ggcggcggtt cagattataa agatgatgac gacaaaggaa ccggttaa 1008

<210> 200
<211> 1023
<212> DNA
<213> Artificial Sequence

<220>
<223> Description of Artificial Sequence: Synthetic
      polynucleotide

<400> 200

catatggtac cagctttata cgacattatt aactatttct acggttcaaa ctctaaattc 60

aaccgtatta catggggttt taaatcacca actttcatca aatggagaat tactgatttc 120

attttaatca tcgttttaat tgttcttttc ttcgtaactt ctcaagcaga gccattccat 180

cgtcaatttt atcttaacga catgactatc caacatcctt ttgcagaaca tgaacgtgta 240

actaatattc aacttggttt atattcaaca gtaattcctt tatcagttat tatcattgtt 300

gctttaatta gtacatgtcc acctaaatac aaattataca acacttgggt ttcaagtatt 360

ggtttacttt tatcagtttt aatcacatct tttgttacaa acatcgttaa aaactggttt 420

ggacgtttac gtcctgactt cttagatcgt tgccaaccag ctaacgatac acctaaagat 480

aaattagttt ctattgaggt ttgtactaca gacaatttag accgtttagc tgacggtttt 540

cgtacaacac cttctggtca ttcttcaatc tcatttgctg gtttattcta tttaacatta 600

tttcttttag gtcaatctca ggcaaataat ggtaaaacat cttcatggcg tacaatgatc 660

agttttatac cttggttaat ggcttgttat atcgctttaa gtcgtacaca agactaccgt 720

catcatttca ttgacgtatt tgttggtagt tgcttaggct taattatcgc aatttggcaa 780

tacttccgtt tattcccttg gttcggtggt aaccaagcaa atgattcatt taacaaccgt 840

attatgattg aagagattaa acgtaaagag gaaattaaac aagatgaaaa taactaccgt 900

cgtatttctg atatttctac taatgtaggt accggtgaaa acctttactt tcaaggttca 960

ggtggcggcg gttcagatta taaagatgat gacgacaaag gaaccggtta atctagactc 1020

gag 1023

<210> 201
<211> 906
<212> DNA
<213> Artificial Sequence

<220>
<223> Description of Artificial Sequence: Synthetic
      polynucleotide

<400> 201
catatggtac caagtcaacc ttactgggca gcaattgagg cagatattga acgttactta 60

aaaaaatcaa ttacaattcg tccaccagaa actgtatttg gtccaatgca ccacttaact 120

tttgctgcac cagctacagc tgctagtact ttatgtttag cagcatgtga acttgtaggt 180

ggtgatcgta gtcaagctat ggctgcagca gcagcaatcc atcttgttca tgcagctgct 240

tatgtacatg aacatttacc attaactgat ggtagtcgtc cagtaagtaa accagctatc 300

caacataaat atggtccaaa tgtagaatta cttacaggtg acggtattgt accatttggt 360

tttgaattat tagcaggttc tgttgatcca gcacgtacag atgatccaga ccgtatttta 420

cgtgtaataa ttgaaataag tcgtgctggt ggtccagaag gtatgattag tggtttacat 480

cgtgaagaag agattgtaga tggtaatact tctcttgatt ttattgaata cgtttgcaaa 540

aaaaaatatg gtgaaatgca cgcatgtggt gctgcatgcg gtgcaatttt aggtggtgca 600

gctgaagaag aaattcaaaa acttcgtaac ttcggattat atcaaggaac tttacgtggt 660

atgatggaga tgaaaaactc acaccaactt attgacgaaa atatcattgg caaacttaaa 720

gaattagctt tagaagaatt aggtggattt catggtaaaa atgctgaatt aatgtctagt 780

ttagtagcag aaccatcatt atatgctgct ggtaccggtg aaaatttata ctttcaaggt 840

tctggtggtg gtggcagtga ttataaagac gatgatgaca aaggaaccgg ttaatctaga 900

ctcgag 906

<210> 202
<211> 1095
<212> DNA
<213> Artificial Sequence

<220>
<223> Description of Artificial Sequence: Synthetic
      polynucleotide

<400> 202
catatggtac cacttttatc taacaaatta agagagatgg ttttagcaga agttcctaaa 60

ttagcatctg ctgctgaata tttctttaaa cgtggtgttc agggtaaaca attccgttca 120

acaattttat tattaatggc aacagctctt gacgttcgtg ttccagaagc attaattggt 180

gaatctactg atattgtaac atctgaatta cgtgtacgtc aacgtggcat tgctgaaatt 240

acagaaatga ttcatgtagc atcacttctt cacgatgacg ttcttgacga tgctgatact 300

cgtcgtggtg ttggtagtct taatgttgta atgggaaaca aaatgtcagt tttagcaggt 360

gacttcttac tttctcgtgc ttgtggtgct cttgcagctc ttaaaaacac agaagttgta 420

gcattattag ctacagcagt agaacactta gttactggtg agacaatgga aataacttca 480

tcaactgaac aacgttattc tatggattac tacatgcaga aaacttatta caaaactgct 540

tcattaattt caaattcatg taaagcagtt gctgtattaa caggtcaaac agctgaagtt 600

gcagtattag cttttgaata tggtcgtaat ttaggtttag ctttccagtt aattgacgac 660

attttagatt tcacaggcac atctgctagt ttaggaaaag gttctttatc agatatacgt 720

catggtgtta ttactgctcc tatcttattt gcaatggaag aatttcctca attaagagaa 780

gtagtagatc aagtagaaaa agatccaaga aatgtagaca tagctttaga atatttaggt 840

aaaagtaaag gtattcaacg tgctcgtgaa ttagcaatgg aacacgcaaa tttagctgct 900

gcagctattg gttctttacc tgaaacagat aacgaagatg ttaaacgttc acgtcgtgct 960

ttaattgatt taacacacag agtaattaca cgtaacaaag gtaccggtga gaatttatac 1020

tttcaaggta gtggtggagg aggtagtgac tataaagatg atgacgataa aggaaccggt 1080

taatctagac tcgag                                                    1095

<210> 203
<211> 1107
<212> DNA
<213> Artificial Sequence

<220>
<223> Description of Artificial Sequence: Synthetic
      polynucleotide

<400> 203
catatggtac cagtagtttc tgaacgttta agacattctg taacaactgg tattccagca      60

ttaaaaacag cagctgaata tttctttcgt cgtggtatcg aaggaaaacg tttaagacct     120

acattagcat tattaatgag tagtgcttta tcaccagctg ctccatcacc agagtattta     180

caagttgata caagacctgc tgcagaacac cctcatgaaa tgcgtcgtcg tcaacaacgt     240

ttagctgaaa ttgcagaatt aatccatgta gcttcattac ttcacgatga tgttattgat     300

gacgcacaaa cacgtcgtgg tgttttaagt ttaaatacat ctgttggtaa taaaacagct     360

atcttagcag gtgatttctt attagctcgt gcatctgtaa cattagctag tttaagaaac     420

tctgaaattg tagaattaat gtcacaggtt ttagaacact tagtatctgg tgaaattatg     480

caaatgactg ctacttcaga acaactttta gatttagaac attatttagc aaaaacatat     540

tgtaaaactg cttcattaat ggctaatagt tctcgttctg ttgcagttct tgcaggtgca     600

gctcctgaag tttgtgatat ggcatggtca tacggtcgtc atttaggtat tgctttccaa     660

gtagttgacg atttattaga tttaacaggt tcatcttctg ttttaggtaa acctgcttta     720

aacgatatgc gttctggttt agcaacagca ccagtattat tcgctgcaca agaagaacct     780

gcattacagg ctcttatatt acgtcgtttt aaacacgacg gtgacgtaac aaaagcaatg     840

tcattaattg aacgtacaca aggcttacgt cgtgctgaag aacttgcagc acaacacgca     900

aaagctgctg ctgatatgat tcgttgctta cctacagctc aatcagacca tgcagaaatt     960

gctcgtgaag cattaattca aattacacat cgtgttttaa cacgtaaaaa aggtaccggt    1020

gaaaacttat actttcaagg ttctggtggt ggtggatcag attataaaga tgatgatgac    1080

aaaggaaccg gttaatctag actcgag                                        1107

<210> 204
<211> 1143
<212> DNA
<213> Artificial Sequence

<220>
<223> Description of Artificial Sequence: Synthetic
      polynucleotide

<400> 204
catatggtac caactacaac attatcatct aaccttaact cacaattcat gcaggtttac      60

gagactctta aatcagaact tattcatgac ccattatttg agttcgatga cgattcaaga 120

caatgggtag aacgtatgat tgattatact gtaccaggtg gtaaaatggt tcgtggttat 180

agtgtagtag atagttatca attacttaaa ggtgaagaac ttacagaaga agaggcattt 240

ttagcttgtg cacttggttg gtgtacagaa tggtttcaag cattcattct tttacatgat 300

gatatgatgg atggtagtca cacaagacgt ggtcaaccat gttggtttcg tttacctgag 360

gttggtgctg ttgctattaa tgatggtgtt ttacttcgta atcacgttca ccgtattctt 420

aaaaaacatt ttcaaggtaa agcatattat gttcatttag ttgatttatt caatgaaact 480

gaatttcaaa caattagtgg acaaatgatc gacttaatta caacattagt tggtgaaaaa 540

gacttatcta aatattcatt aagtattcat cgtcgtatcg ttcaatacaa aacagcatac 600

tactcatttt acttaccagt tgcttgtgct ttacttatgt ttggtgagga tcttgataaa 660

catgtagaag ttaaaaatgt tcttgttgaa atgggtacat attttcaagt tcaagatgat 720

tatttagatt gttttggtgc tccagaagtt attggcaaaa ttggtactga tattgaagac 780

tttaaatgtt catggttagt agttaaagca ttagaattag caaatgaaga acagaaaaaa 840

actttacacg aaaattatgg aaaaaaagat ccagcatcag ttgctaaagt taaagaagta 900

taccacacac ttaatttaca agctgttttc gaagattatg aagcaacatc atacaaaaaa 960

cttattactt ctattgaaaa tcacccatct aaagctgttc aagctgtttt aaaatctttc 1020

ttaggcaaaa tatacaaacg tcaaaaaggt accggtgaaa acttatactt tcaaggttct 1080

ggtggcggtg gaagtgatta caaagatgat gacgataaag gaaccggtta atctagactc 1140

gag 1143

<210> 205
<211> 1818
<212> DNA
<213> Artificial Sequence

<220>
<223> Description of Artificial Sequence: Synthetic
      polynucleotide

<400> 205
catatggtac caagtcaacc ttactgggct gcaattgaag cagacattga aagatattta 60

aaaaaatcaa ttacaattcg tccaccagaa actgtatttg gtcctatgca ccatttaaca 120

tttgctgctc ctgctactgc agctagtaca ttatgccttg ctgcttgtga attagttggc 180

ggtgatcgta gtcaagctat ggcagctgct gctgctatcc atttagttca tgcagctgct 240

tacgttcacg aacatcttcc tttaacagat ggatcacgtc ctgtaagtaa acctgctatt 300

caacataaat atggtccaaa cgttgaactt ttaacaggtg atggtatcgt tcctttcggt 360

tttgagttat tagcaggttc agtagatcca gcacgtactg atgaccctga tcgtatttta 420

cgtgtaatta ttgaaatttc tcgtgctggt ggaccagaag gcatgatttc tggtttacac 480

cgtgaggaag aaatcgtaga tggtaacaca tcattagact ttatagaata tgtatgcaaa 540

aaaaaatacg gtgaaatgca cgcatgtggt gcagcttgcg gagctatttt aggtggagct 600

gctgaagaag aaattcaaaa acttcgtaac tttggtcttt atcaaggcac attacgtggt 660

atgatggaaa tgaaaaatag tcatcagtta attgacgaaa atatcattgg aaaacttaaa 720

gaacttgctc ttgaagaatt aggtggattc cacggtaaaa acgctgaatt aatgagttct 780

ttagttgctg aacctagttt atatgcagct tcatcaaata acttaggtat cgaaggtcgt 840

tttgactttg acggttacat gcttcgtaaa gcaaaatctg taaataaagc attagaagct 900

gctgttcaaa tgaaagaacc acttaaaatt cacgaatcaa tgcgttattc attattagct 960

ggtggtaaac gtgttcgtcc aatgttatgt attgcagctt gtgaacttgt tggtggtgac 1020

gaatctacag caatgcctgc agcatgtgct gttgaaatga ttcacacaat gtctttaatg 1080

catgatgacc ttccatgtat ggataacgat gacttacgtc gtggtaaacc tacaaaccac 1140

atggcttttg gtgagtctgt agctgttctt gctggtgatg cattacttag ttttgctttt 1200

gaacatgttg ctgctgcaac aaaaggcgca ccacctgaac gtatcgtacg tgtattaggt 1260

gaattagctg ttagtattgg ttcagaagga cttgtagcag gtcaagttgt agacgtttgt 1320

tctgaaggca tggctgaagt aggattagat catcttgaat ttattcacca tcataaaact 1380

gctgcattat tacaaggttc agttgtttta ggtgcaatat taggaggcgg taaagaagaa 1440

gaagtagcta aacttcgtaa atttgctaac tgtattggtt tacttttcca agttgttgat 1500

gatattttag atgttactaa aagtagtaaa gagttaggta aaactgcagg taaagactta 1560

gtagctgata aaactacata tcctaaactt ataggcgttg aaaaatcaaa agaatttgct 1620

gaccgtttaa atcgtgaagc acaagaacaa ttattacatt ttcatcctca ccgtgctgct 1680

ccattaatcg ctttagctaa ctacatcgct taccgtgata atggtaccgg tgaaaactta 1740

tacttccagg gtagtggtgg tggcggatca gattataaag atgacgatga taaaggaacc 1800

ggttaatcta gactcgag 1818

<210> 206
<211> 1206
<212> DNA
<213> Artificial Sequence

<220>
<223> Description of Artificial Sequence: Synthetic
      polynucleotide

<400> 206
catatggtac cacacaagtt cacaggtgtt aacgctaaat tccagcaacc agcattaaga 60

aatttatctc cagtggtagt tgagcgcgaa cgtgaggaat ttgtaggatt ctttccacaa 120

attgttcgtg acttaactga agatggtatt ggtcatccag aagtaggtga cgctgtagct 180

cgtcttaaag aagtattaca atacaacgca cctggtggta aatgcaatag aggtttaaca 240

gttgttgcag cttaccgtga actttctgga ccaggtcaaa aagacgctga aagtcttcgt 300

tgtgctttag cagtaggatg gtgtattgaa ttattccaag cctttttctt agtttgggac 360

gatataatgg accagtcatt aactagacgt ggtcaattat gttggtacaa gaaagaaggt 420

gttggtttag atgcaataaa tgattctttt cttttagaaa gctctgtgta tcgcgttctt 480

aaaaagtatt gccgtcaacg tccatattat gtacatttat tagagctttt tcttcaaaca 540

gcttaccaaa cagaattagg acaaatgtta gatttaatca ctgctcctgt atctaaggta 600

gatttaagcc atttctcaga agaacgttac aaagctattg ttaagtataa aactgctttc 660

tattcattct atttaccagt tgcagcagct atgtatatgg ttggtataga ttctaaagaa 720

gaacatgaaa acgcaaaagc tattttactt gagatgggtg aatacttcca aattcaagat 780

gattatttag attgttttgg cgatcctgct ttaacaggta aagtaggtac tgatattcaa 840

gataacaaat gttcatggtt agttgtgcaa tgcttacaaa gagtaacacc agaacaacgt 900

caacttttag aagataatta cggtcgtaaa gaaccagaaa aagttgctaa agttaaagaa 960

ttatatgagg ctgtaggtat gagagccgcc tttcaacaat acgaagaaag tagttaccgt 1020

cgtcttcaag agttaattga gaaacattct aatcgtttac caaaagaaat tttcttaggt 1080

ttagctcaga aaatatacaa acgtcaaaaa ggtaccggtg aaaacttata ctttcaaggc 1140

tcaggtggcg gtggaagtga ttacaaagat gatgatgata aaggaaccgg ttaatctaga 1200

ctcgag 1206

<210> 207
<211> 1206
<212> DNA
<213> Artificial Sequence

<220>
<223> Description of Artificial Sequence: Synthetic
      polynucleotide

<400> 207
catatggtac cacacaagtt cacaggtgtt aacgctaaat tccagcaacc agcattaaga 60

aatttatctc cagtggtagt tgagcgcgaa cgtgaggaat ttgtaggatt ctttccacaa 120

attgttcgtg acttaactga agatggtatt ggtcatccag aagtaggtga cgctgtagct 180

cgtcttaaag aagtattaca atacaacgca cctggtggta aatgcaatag aggtttaaca 240

gttgttgcag cttaccgtga actttctgga ccaggtcaaa aagacgctga aagtcttcgt 300

tgtgctttag cagtaggatg gtgtattgaa ttattccaag cctttttctt agttgctgac 360

gatataatgg accagtcatt aactagacgt ggtcaattat gttggtacaa gaaagaaggt 420

gttggtttag atgcaataaa tgattctttt cttttagaaa gctctgtgta tcgcgttctt 480

aaaaagtatt gccgtcaacg tccatattat gtacatttat tagagctttt tcttcaaaca 540

gcttaccaaa cagaattagg acaaatgtta gatttaatca ctgctcctgt atctaaggta 600

gatttaagcc atttctcaga agaacgttac aaagctattg ttaagtataa aactgctttc 660

tattcattct atttaccagt tgcagcagct atgtatatgg ttggtataga ttctaaagaa 720

gaacatgaaa acgcaaaagc tattttactt gagatgggtg aatacttcca aattcaagat 780

gattatttag attgttttgg cgatcctgct ttaacaggta aagtaggtac tgatattcaa 840

gataacaaat gttcatggtt agttgtgcaa tgcttacaaa gagtaacacc agaacaacgt 900

caacttttag aagataatta cggtcgtaaa gaaccagaaa aagttgctaa agttaaagaa 960

ttatatgagg ctgtaggtat gagagccgcc tttcaacaat acgaagaaag tagttaccgt 1020

cgtcttcaag agttaattga gaaacattct aatcgtttac caaaagaaat tttcttaggt 1080

ttagctcaga aaatatacaa acgtcaaaaa ggtaccggtg aaaacttata ctttcaaggc 1140

tcaggtggcg gtggaagtga ttacaaagat gatgatgata aaggaaccgg ttaatctaga 1200

ctcgag 1206

<210> 208
<211> 1062
<212> DNA
<213> Artificial Sequence

<220>
<223> Description of Artificial Sequence: Synthetic
      polynucleotide

<400> 208
catatggtac cagctgctgc tgcacctgct gagacaatga ataaatctgc agctggcgct 60

gaagtaccag aggctttcac atcagttttt caaccaggta aattagcagt tgaagcaatt 120

caagtagatg aaaatgcagc tcctactcca cctattcctg ttttaatagt tgctccaaaa 180

gatgctggta catatccagt tgctatgtta ttacacggat ttttcttaca taatcacttt 240

tatgaacact tattacgtca cgttgcatct catggcttta tcattgttgc tccacaattt 300

tctattagta ttattccatc aggagatgct gaagacatcg ctgctgctgc aaaagtagca 360

gattggttac ctgacggatt accaagtgtt ttaccaaaag gtgttgaacc agagttatca 420

aaacttgctt tagctggaca cagtcgtggt ggtcacacag ctttttcttt agctttaggt 480

cacgctaaaa cacaattaac tttcagtgca ttaattggtt tagatcctgt tgctggaaca 540

ggtaaatcat ctcaattaca accaaaaatt cttacttatg agccaagttc atttggtatg 600

gctatgccag ttttagttat tggtacaggt ttaggagaag aaaaaaaaaa catttctttt 660

cctccatgtg ctcctaaaga cgtaaaccat gcagaatttt atcgtgaatg tagaccacca 720

tgttactatt ttgtaactaa agattatggc catcttgata tgttagatga tgacgctcca 780

aaatttatca catgtgtttg taaagacggt aatggatgta aaggaaaaat gcgtcgttgt 840

gtagctggca tcatggttgc tttcttaaac gctgctttag gtgaaaaaga cgcagattta 900

gaagctattt tacgtgatcc agcagttgct cctacaacat tagacccagt tgaacaccgt 960

gttgctggta ccggtgagaa tttatacttc cagggatctg gtggtggtgg cagtgattat 1020

aaagatgatg atgataaagg aaccggttaa tctagactcg ag 1062

<210> 209
<211> 6
<212> PRT
<213> Artificial Sequence

<220>
<223> Description of Artificial Sequence: Synthetic
      6x His tag

<400> 209
His His His His His His
1               5